# EUROPEAN PATENT APPLICATION

(11) **EP 4 269 405 A1**
(43) Date of publication of application: **01.11.2023**
(21) Application number: 21909465.3
(22) Date of filing: 22.12.2021
(51) Int. Cl.: C07D 403/04, C07D 471/04, A61K 31/517, A61P 35/00

(54) **PREPARATION AND APPLICATION METHOD OF HETEROCYCLIC COMPOUND AS KRAS INHIBITOR**

(30) Priority: 22.12.2020 WO PCT/CN2020/138417; 30.09.2021 CN 202111157323
(71) Applicant: Shanghai Kechow Pharma, Inc., Shanghai 201203 (CN)
(72) Inventor: TIAN, Hongqi, Shanghai 201203 (CN); HUANG, Gongchao, Shanghai 201203 (CN); GAO, Xuguang, Shanghai 201203 (CN); XU, Haijiang, Shanghai 201203 (CN); WANG, Xingkai, Shanghai 201203 (CN)
(74) Representative: Pearl Cohen Zedek Latzer Baratz UK LLP
(86) International application number: PCT/CN2021/140466
(87) International publication number: WO 2022/135470

(57) **Abstract**

Disclosed is a compound of formula (I) or a pharmaceutically acceptable salt, prodrug, tautomer or stereoisomer, and solvate thereof. The compound can be applied to treatment of cancers and inflammations of mammals. Further disclosed are a preparation method for the compound of formula (I) and a pharmaceutical composition containing the compound.

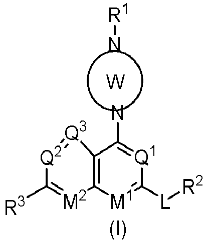

## Description

### Technical Field

The present invention relates to some novel heterocyclic compounds or pharmaceutically acceptable salts thereof, which can be used for the treatment or prevention of a wide variety of cancers. The present invention further relates to pharmaceutical compositions comprising such compounds and salts thereof, intermediates in the preparation of the compounds, and methods for treating various cancers by using the compounds and salts thereof.

### Background Art

In 1982, Weinberg and Barbacid isolated, for the first time, a transforming gene from a human bladder cancer cell line, which could make NIH 3T3 cells undergo malignant transformation; whereas, DNA extracted from normal human tissues had no such effects. Subsequently, Santos and Parada found that the above-mentioned transforming gene was not a new gene, but a human homologous gene of the ras gene of Harvery murine sarcoma virus, named H2ras. In the same year, Krontiris found a homologue of Kirsten murine sarcoma virus gene in human lung cancer cells, named K-ras. Another similar gene, called N2ras, was a gene similar to ras and was found when human neuroblastoma DNA infected NIH 3T3 cells. This gene is irrelevant to viruses.

Ras gene is quite conservative in evolution and widely exists in various eukaryotes such as mammals, fruit flies, fungi, nematodes, and yeasts, suggesting that it has important physiological functions. The mammalian ras gene family has three members, i.e., H-ras, K-ras, and N-ras, wherein the fourth exon of K-ras has two variants, A and B. Various ras genes have similar structures, all of which are composed of four exons, which are distributed on DNA with a full length of about 30 kb. The encoded product thereof is a protein with a relative molecular weight of 21,000 and is thus called P21 protein. It has been demonstrated that H-ras is located on the short arm of human chromosome 11 (11p15.1-p15.3), K-ras is located on the short arm of human chromosome 12 (12p1.1-pter), and N-ras is located on the short arm of human chromosome 1 (1p22-p32). Except for the variation of the fourth exon of K-ras, the P21-encoding sequence of each ras gene is evenly distributed on four exons, and the sequence and size of introns are very different, so the whole gene is also very different. For example, human K-ras is 35 kb long and N-ras is 3 kb long. Since there are two exons 4, K-ras can be spliced in two ways, but the content of mRNA encoding K-ras-B is high. Except K-ras-B which contains 188 amino acids, the other two ras proteins both contain 189 amino acids.

Ras(P21) protein, which is located on the inner side of the cell membrane, plays an important role in transmitting cell growth and differentiation signals. It belongs to guanosine triphosphate (GTP) binding protein (a coupling factor of cell information transmission), which regulates information transmission by mutual transformation between GTP and guanosine diphosphate (GDP). P21 has a strong affinity with GTP and GDP and a weak GTPase activity. Under normal circumstances, the binding between P21 and GDP is in an inactive state. When the growth differentiation factor outside the cell transmits a signal to P21 on the inner side of the cell membrane, the activity of binding P21 to GTP can be enhanced, making the binding of P21 and GTP be in an activated state, causing the signal system to be open. Due to the GTPase activity, P21 can hydrolyze GTP into GDP. After P21 is bound with GDP, P21 become inactivated and the signal system is closed. Under normal circumstances, the GTPase activity of P21 is very weak. After it is bound with GTPase-activating protein (GAP), the hydrolysis rate thereof can be increased by 10,000 times, causing P21 to be deactivated. After P21 is bound with GDP, guanosine nucleotide releasing protein (GNRP) can be activated, and GNRP makes P21 release GDP and bind GTP Therefore, by the mutual transformation between GTP and GDP, P21 can be regulated to turn on and off the signal system in a controlled way, thereby completing the process of transmitting growth and differentiation signals into cells.

More than 115 cancer patients are accompanied by ras gene mutations, which mostly occur in G12, G13, and Q61 residues. The mutations lead to GAP protein mediation failure and ras signal is in a continuously activated state. The present invention designs and synthesizes a range of chemical molecules, which have strong biological activity of inhibiting ras, and provides a method for treating related cancers by inhibiting H-ras, K-ras, or N-ras.

### Summary of the Invention

The present invention provides compounds capable of regulating G12C mutant KRAS, HRAS and/or NRAS proteins, including stereoisomers, pharmaceutically acceptable salts, tautomers and prodrugs thereof. Further provided is a method of using such compounds to treat various diseases or conditions, such as cancer.

In one aspect of the present invention, there is provided a compound of formula (I) or a pharmaceutically acceptable salt, solvate, tautomer, stereoisomer or prodrug thereof, wherein the compound of formula (I) is: 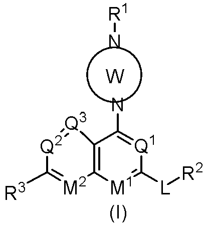 wherein:
ring W is a 4- to 12-membered saturated or partially saturated monocyclic, bridged cyclic, or spirocyclic ring, wherein the saturated or partially saturated monocyclic ring is optionally additionally substituted with one or more R⁴,
wherein
R⁴ is selected from: oxo, alkyl, alkenyl, alkynyl, cycloalkyl, aryl, heteroaryl, heterocyclyl, cyano, nitro, -C(O)OR⁵, or -C(O)N(R⁵)₂, among which the alkyl is unsubstituted or substituted with one or more of cyano, halo, -OR⁵, -N(R⁵)₂, or heteroaryl, wherein each R⁵ is independently hydrogen or alkyl;
R¹ is -L¹-T,
wherein
L¹ is -O-, -S-, -NR^{a}-, -C(O)-, -SO₂-, -SO-, -C(=NR^{a})-, -C(O)-O-, -OC(O)-, -C(O)-NR^{a}-, or -NR^{a}C(O)-,
T is -CR^{a}=CR^{b}R^{c}, -C=CR^{b}, alkyl, cycloalkyl, aryl, heteroaryl, or heterocyclyl, and each of the alkyl, cycloalkyl, aryl, heteroaryl, or heterocyclyl is unsubstituted or substituted with one or more of oxo, halogen, hydroxyl, alkyl, haloalkyl, hydroxyalkyl, alkoxy, CN, nitro, or NR^{x}R^{y};
   wherein
   R^{a} is hydrogen, deuterium, cyano, halogen, hydroxyl, alkyl, haloalkyl, hydroxyalkyl, aryl, heteroaryl, or heterocyclyl;
   R^{b} and R^{c} are each independently hydrogen, deuterium, cyano, halogen, -C(O)OR^{x}, alkyl, cycloalkyl, aryl, heteroaryl, or heterocyclyl, among which each of the alkyl, cycloalkyl, aryl, heteroaryl, or heterocyclyl is unsubstituted or substituted with one or two of oxo; halogen; hydroxyl; alkyl; haloalkyl; hydroxyalkyl; alkoxy; CN; nitro; NR^{x}R^{y}; aryl which is unsubstituted or substituted with alkyl, hydroxyl, or halogen; heteroaryl which is unsubstituted or substituted with alkyl, hydroxyl, or halogen; or heterocyclyl which is unsubstituted or substituted with alkyl, hydroxyl, or halogen,
   or when T is -CR^{a}=CR^{b}R^{c}, R^{a} and R^{b}, or R^{a} and R^{c}, together with the carbon atom to which they are attached, form an unsaturated 5- to 8-membered ring which is unsubstituted or substituted with one or two of oxo, hydroxyl, halogen, alkyl, hydroxyalkyl, haloalkyl, or alkoxy;
R^{x} and R^{y} are each independently hydrogen or alkyl;
Q¹, Q², and Q³ are each independently N or CR¹¹, and M¹ and M² are each independently N or CR¹², provided that at least one of Q¹ and M¹ is N;
wherein
R¹¹ and R¹² are each independently hydrogen, halogen, cyano, nitro, alkyl, alkenyl, alkynyl, cycloalkyl, aryl, heteroaryl or heterocyclyl, -OR^{d}, -C(O)R^{d}, -CO₂R^{d}, -CONR^{d}R^{e}, or -NR^{d}R^{e}, wherein the alkyl, alkenyl, alkynyl, cycloalkyl, aryl, heteroaryl, and heterocyclyl are each independently substituted with one or more of oxo, halogen, hydroxyl, alkoxy, alkyl, cycloalkyl, nitro, cyano, and -NR^{d}R^{e}, wherein R^{d} and R^{e} are each independently hydrogen, alkyl, C₃-C₆ cycloalkyl, hydroxyalkyl, haloalkyl, and alkoxyalkyl;
L is a single bond, -O-, -S-, -NR^{a}-, -O-CH₂-, -S-CH₂-, -NR^{a}-CH₂-, -CH₂-O-, -CH₂-S-, -CH₂-NR^{a}-, - C(O)-, -SO₂-, -SO-, -C(O)-O-, -OC(O)-, -C(O)-NR^{a}-, or -NR^{a}C(O)-;
R² is alkyl, alkenyl, alkynyl, cycloalkyl, aryl, heteroaryl, or heterocyclyl, wherein the alkyl, alkenyl, alkynyl, cycloalkyl, aryl, heteroaryl, and heterocyclyl are each independently unsubstituted or substituted with one or more of halogen, cyano, alkyl, haloalkyl, hydroxyalkyl, alkoxyalkyl, oxo, -OR^{d}, -C(O)R^{d}, - CO₂R^{d}, -CONR^{d}R^{e}, -NR^{d}R^{e}, cycloalkyl, cycloalkylalkyl, aryl, heteroaryl, and heterocyclyl, wherein R^{d} and R^{e} are each independently hydrogen, alkyl, hydroxyalkyl, haloalkyl, and alkoxyalkyl;
R³ is cycloalkyl, heterocyclyl, aryl, or heteroaryl, provided that when M¹, Q¹, and Q² are all N, R³ is non-aromatic fused bicyclic heterocyclyl, R³ is unsubstituted or substituted with one or more of the following groups: oxo, halogen, cyano, -OR^{d}, -C(O)R^{d}, -CO₂R^{d}, -CONR^{d}R^{e}, -NR^{d}COR^{e}, -NR^{d}R^{e}, - S(O)₂NR^{d}R^{e}, alkyl, alkenyl, alkynyl, cycloalkyl, aryl, heteroaryl, and heterocyclyl, wherein the alkyl, alkenyl, alkynyl, cycloalkyl, aryl, heteroaryl, and heterocyclyl are each independently substituted with halogen, alkyl, cyano, carbamoyl, alkoxy, hydroxyl, cycloalkyl, and heteroaryl, wherein R^{d} and R^{e} are each independently hydrogen, alkyl, C₃-C₆ cycloalkyl, hydroxyalkyl, haloalkyl, alkoxyalkyl, alkenyl, or cycloalkyl.

In some embodiments, there is provided a compound of formula (I) or a pharmaceutically acceptable salt, solvate, tautomer, stereoisomer or prodrug thereof, wherein the compound of formula (I) is: 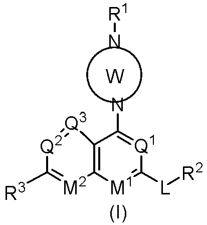 wherein:
ring W is a 4- to 12-membered saturated or partially saturated monocyclic, bridged cyclic, or spirocyclic ring, wherein the saturated or partially saturated monocyclic ring is optionally additionally substituted with one or more R⁴,
wherein
R⁴ is selected from: oxo, alkyl, alkenyl, alkynyl, cycloalkyl, aryl, heteroaryl, heterocyclyl, cyano, nitro, -C(O)OR⁵, or -C(O)N(R⁵)₂, among which the alkyl is unsubstituted or substituted with one or more of cyano, halo, -OR⁵, -N(R⁵)₂, or heteroaryl, wherein each R⁵ is independently hydrogen or alkyl;
R¹ is -L'-T,
wherein
L¹ is -O-, -S-, -NR^{a}-, -C(O)-, -SO₂-, -SO-, -C(=NR^{a})-, -C(O)-O-, -OC(O)-, -C(O)-NR^{a}-, or -NR^{a}C(O)-,
T is -CR^{a}=CR^{b}R^{c}, -C=CR^{b}, alkyl, cycloalkyl, aryl, heteroaryl, or heterocyclyl, and each of the alkyl, cycloalkyl, aryl, heteroaryl, or heterocyclyl is unsubstituted or substituted with one or more of oxo, halogen, hydroxyl, alkyl, haloalkyl, hydroxyalkyl, alkoxy, CN, nitro, or NR^{x}R^{y};
   wherein
   R^{a} is hydrogen, deuterium, cyano, halogen, hydroxyl, alkyl, haloalkyl, hydroxyalkyl, aryl, heteroaryl, or heterocyclyl;
   R^{b} and R^{c} are each independently hydrogen, deuterium, cyano, halogen, -C(O)OR^{x}, alkyl, cycloalkyl, aryl, heteroaryl, or heterocyclyl, among which each of the alkyl, cycloalkyl, aryl, heteroaryl, or heterocyclyl is unsubstituted or substituted with one or two of oxo; halogen; hydroxyl; alkyl; haloalkyl; hydroxyalkyl; alkoxy; CN; nitro; NR^{x}R^{y}; aryl which is unsubstituted or substituted with alkyl, hydroxyl, or halogen; heteroaryl which is unsubstituted or substituted with alkyl, hydroxyl, or halogen; or heterocyclyl which is unsubstituted or substituted with alkyl, hydroxyl, or halogen,
   or when T is -CR^{a}=CR^{b}R^{c}, R^{a} and R^{b}, or R^{a} and R^{c}, together with the carbon atom to which they are attached, form an unsaturated 5- to 8-membered ring which is unsubstituted or substituted with one or two of oxo, hydroxyl, halogen, alkyl, hydroxyalkyl, haloalkyl, or alkoxy;
R^{x} and R^{y} are each independently hydrogen or alkyl;
Q¹, Q², and Q³ are each independently N or CR¹¹, and M¹ and M² are each independently N or CR¹², provided that at least one of Q¹ and M¹ is N;
wherein
R¹¹ and R¹² are each independently hydrogen, halogen, cyano, nitro, alkyl, alkenyl, alkynyl, cycloalkyl, aryl, heteroaryl or heterocyclyl, -OR^{d}, -C(O)R^{d}, -CO₂R^{d}, -CONR^{d}R^{e}, or -NR^{d}R^{e}, wherein the alkyl, alkenyl, alkynyl, cycloalkyl, aryl, heteroaryl, and heterocyclyl are each independently substituted with one or more of oxo, halogen, hydroxyl, alkoxy, alkyl, cycloalkyl, nitro, cyano, and -NR^{d}R^{e}, wherein R^{d} and R^{e} are each independently hydrogen, alkyl, C₃-C₆ cycloalkyl, hydroxyalkyl, haloalkyl, and alkoxyalkyl;
L is a single bond, -O-, -S-, -NR^{a}-, -O-CH₂-, -S-CH₂-, -NR^{a}-CH₂-, -CH₂-O-, -CH₂-S-, -CH₂-NR^{a}-, - C(O)-, -SO₂-, -SO-, -C(O)-O-, -OC(O)-, -C(O)-NR^{a}-, or -NR^{a}C(O)-;
R² is alkyl, alkenyl, alkynyl, cycloalkyl, aryl, heteroaryl, or heterocyclyl, wherein the alkyl, alkenyl, alkynyl, cycloalkyl, aryl, heteroaryl, and heterocyclyl are each independently unsubstituted or substituted with one or more of halogen, cyano, alkyl, haloalkyl, hydroxyalkyl, alkoxyalkyl, oxo, -OR^{d}, -C(O)R^{d}, - CO₂R^{d}, -CONR^{d}R^{e}, -NR^{d}R^{e}, -CH₂ NR^{d}R^{e}, cycloalkyl, cycloalkylalkyl, aryl, heteroaryl, and heterocyclyl, wherein R^{d} and R^{e} are each independently hydrogen, alkyl, hydroxyalkyl, haloalkyl, and alkoxyalkyl;
R³ is non-aromatic fused bicyclic heterocyclyl, R³ is unsubstituted or substituted with one or more of the following groups: oxo, halogen, cyano, -OR^{d}, -C(O)R^{d}, -CO₂R^{d}, -CONR^{d}R^{e}, -NR^{d}COR^{e}, -NR^{d}R^{e}, - S(O)₂NR^{d}R^{e}, alkyl, alkenyl, alkynyl, cycloalkyl, aryl, heteroaryl, and heterocyclyl, wherein the alkyl, alkenyl, alkynyl, cycloalkyl, aryl, heteroaryl, and heterocyclyl are each independently substituted with halogen, alkyl, cyano, carbamoyl, alkoxy, hydroxyl, cycloalkyl, and heteroaryl, wherein R^{d} and R^{e} are each independently hydrogen, alkyl, hydroxyalkyl, haloalkyl, alkoxyalkyl, alkenyl, or cycloalkyl.

In some embodiments, L' is -C(O)- or -SO₂-.

In some embodiments, L' is -C(=NR^{a})-, wherein R^{a} is H, CN, or hydroxyl.

In some embodiments, T is -CR^{a}=CR^{b}R^{c}, -C=CR^{b}, alkyl, or heterocyclyl, wherein R^{a} and R^{b} are as defined in formula (I).

In some embodiments, T is -CR^{a}=CR^{b}R^{c} or -C=CR^{b}, wherein R^{a} is hydrogen, deuterium, cyano, halogen, hydroxyl, or alkyl, R^{b} and R^{c} are each independently hydrogen; halogen; unsubstituted alkyl; alkyl substituted with hydroxyl, halogen, NR^{x}R^{y} or heterocyclyl; unsubstituted aryl or heteroaryl; aryl or heteroaryl substituted with alkyl, hydroxyl or halogen, wherein R^{x} and R^{y} are each independently hydrogen or alkyl. Preferably, in the above embodiments, the aryl is phenyl, which is unsubstituted or substituted with one or two of halogen, hydroxyl, or C₁₋₃ alkyl. Preferably, in the above embodiments, the heteroaryl is thiazolyl, oxazolyl, pyridinyl, or pyrimidinyl, which is unsubstituted or substituted with one or two of halogen, hydroxyl, or C₁₋₃ alkyl.

In some embodiments, T is -CR^{a}=CR^{b}R^{c}, wherein R^{a} and R^{b}, or R^{a} and R^{c}, together with the carbon atom to which they are attached, form an unsaturated 5- to 8-membered ring which is unsubstituted or substituted with one or two of hydroxyl, halogen, alkyl, hydroxyalkyl, haloalkyl, or alkoxy. In some embodiments, T is -CR^{a}=CR^{b}R^{c}, wherein R^{a} and R^{b}, or R^{a} and R^{c}, together with the carbon atom to which they are attached, form an unsaturated 5-, 6-, 7-, or 8-membered carbocyclic ring which is unsubstituted or substituted with one or two of hydroxyl, halogen, alkyl, hydroxyalkyl, haloalkyl, or alkoxy. Preferably, the unsaturated 5-, 6-, 7-, or 8-membered carbocyclic ring is a cyclopentene ring, a cyclohexene ring, a cycloheptene ring, or a cyclooctene ring.

In some embodiments, T is alkyl, which is unsubstituted or substituted with halogen, hydroxyl, NR^{x}R^{y}, CN, haloalkyl, hydroxyalkyl, alkoxy, or heterocyclyl, wherein R^{x} and R^{y} are each independently hydrogen or alkyl. Preferably, the heterocyclyl in the above embodiments is 4- to 8-membered heterocyclyl containing one or two heteroatoms selected from oxygen, nitrogen and sulfur, e.g., azetidine, pyrrolidine, piperidinyl, and morpholinyl.

In some embodiments, T is heterocyclyl, which is unsubstituted or substituted with halogen, hydroxyl, NR^{x}R^{y}, CN, alkyl, haloalkyl, hydroxyalkyl, or alkoxy, wherein R^{x} and R^{y} are each independently hydrogen or alkyl. Preferably, T is a 3- to 8-membered heterocyclic ring containing one heteroatom selected from oxygen, nitrogen and sulfur, e.g., unsubstituted or methyl-substituted propylene oxide.

In some embodiments, L' is -C(O)- or -SO₂-, and T is -CH=CH₂.

In some embodiments, L is -O-CH₂- or -O-.

In some embodiments, L is -O-CH₂-, and R² is heterocyclyl, which is unsubstituted or substituted with one or more of halogen and alkyl. Preferably, L is -O-CH₂-, and R² is heterocyclyl, wherein the heterocyclyl is a 4- to 8-membered monocyclic ring containing 1, 2, or 3 heteroatoms selected from oxygen, nitrogen and sulfur, and the heterocyclyl is unsubstituted or substituted with one or more of halogen and alkyl. More preferably, the heterocyclyl is azetidinyl, pyrrolidinyl, or piperidinyl, and the ring is unsubstituted or substituted with one or two halogens or alkyl groups. In a further preferred embodiment, L-R² is

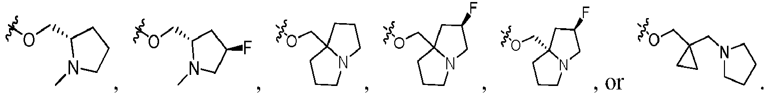

In some embodiments, R³ is aryl, and the aryl is phenyl or naphthyl which is unsubstituted or substituted with 1, 2, or 3 substituents of halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; - NR^{d}COR^{e} in which R^{d} and R^{e} are each independently hydrogen or alkyl; alkyl which is unsubstituted or substituted with halogen, cycloalkyl, hydroxyl or alkoxy; cycloalkyl which is unsubstituted or substituted with alkyl, cyano or carbamoyl; alkynyl; -NR^{d}R^{e} in which R^{d} and R^{e} are each independently hydrogen or alkyl; or heteroaryl.

In some embodiments, R³ is partially hydrogenated naphthyl which is unsubstituted or substituted with hydroxyl, alkyl, hydroxyalkyl, haloalkyl or halogen. Preferably, R³ is 1,2,3,4-tetrahydronaphthalenyl, which is unsubstituted or substituted with hydroxyl, alkyl, hydroxyalkyl, haloalkyl, halogen, amino, alkylamino, or dialkylamino.

In some embodiments, R³ is heteroaryl which is unsubstituted or substituted with 1, 2, or 3 substituents of oxo, halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; -NR^{d}COR^{e} in which R^{d} and R^{e} are each independently hydrogen, alkyl, or alkenyl; alkyl which is unsubstituted or substituted with halogen, cycloalkyl, hydroxyl, or alkoxy; cycloalkyl which is unsubstituted or substituted with alkyl, cyano or carbamoyl; alkynyl; or -NR^{d}R^{e} in which R^{d} and R^{e} are each independently hydrogen or alkyl. Preferably, the above heteroaryl is monocyclic heteroaryl, such as thiophene, thiazole, pyrazole, pyridine, or pyrimidine, which is unsubstituted or substituted as described above. Preferably, the above heteroaryl is bicyclic heteroaryl, such as 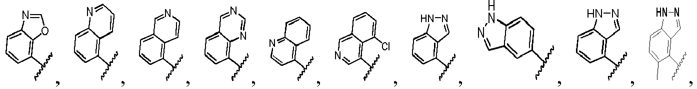 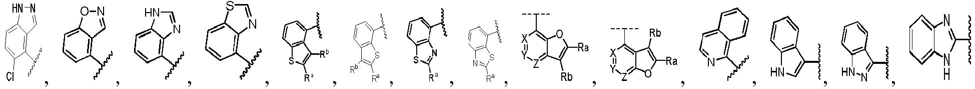 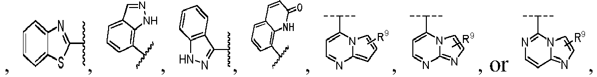 in which R^{a} and R^{b} are independently hydrogen, halogen, or alkyl, or R^{a} and R^{b} are connected to form a substituted or unsubstituted C₃-C₆ cycloalkyl, wherein the heteroaryl is unsubstituted or substituted as described above.

In some embodiments, R³ is heterocyclyl, preferably non-aromatic fused bicyclic heterocyclyl, which is unsubstituted or substituted with 1, 2, or 3 substituents of oxo, halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; -NR^{d}COR^{e} in which R^{d} and R^{e} are each independently hydrogen, alkyl, or alkenyl; alkyl which is unsubstituted or substituted with halogen, cycloalkyl, hydroxyl, or alkoxy; cycloalkyl which is unsubstituted or substituted with alkyl, cyano or carbamoyl; alkynyl; or -NR^{d}R^{e} in which R^{d} and R^{e} are each independently hydrogen or alkyl. In other embodiments, R³ is non-aromatic fused bicyclic heterocyclyl, which is unsubstituted or substituted with 1, 2, or 3 substituents of oxo, halogen; hydroxyl, alkoxy, and alkyl; preferably, the substituent is oxo, halogen, hydroxyl, methoxy, or methyl. In a further embodiment, R³ is non-aromatic fused bicyclic heterocyclyl, which is 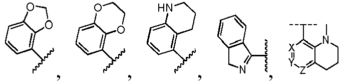 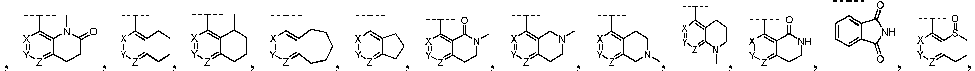 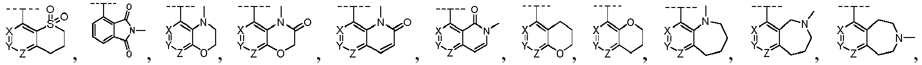 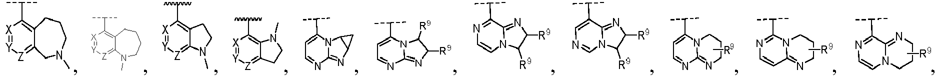 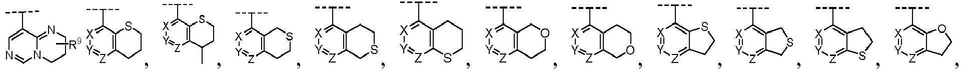 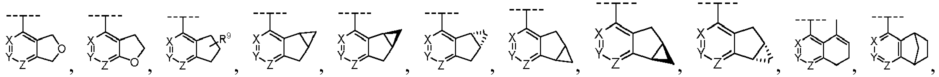 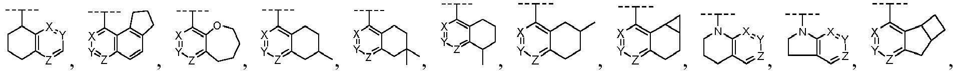 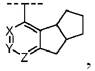 which is unsubstituted or substituted with 1, 2, or 3 substituents of oxo, halogen; hydroxyl, alkoxy, and alkyl; preferably, the substituent is oxo, halogen, hydroxyl, methoxy, or methyl, wherein X, Y, and Z are each independently N or CR⁹ in which R⁹ is hydrogen, hydroxyl, cyano, alkyl, haloalkyl, halogen, hydroxyalkyl, alkoxyalkyl, or alkylsulfonyl. In some embodiments, the compound of formula (I) is 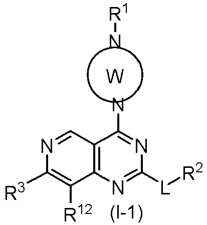 wherein: R³ is preferably 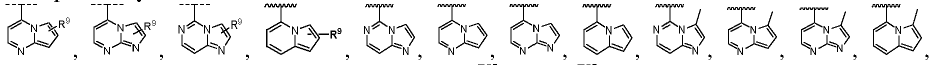 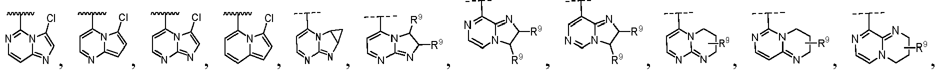 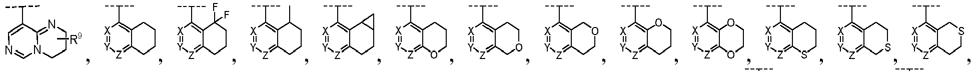 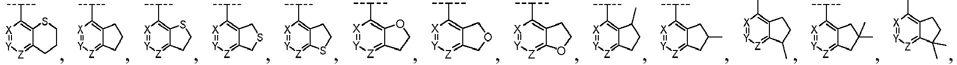 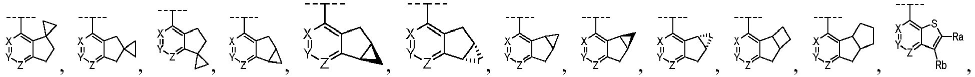 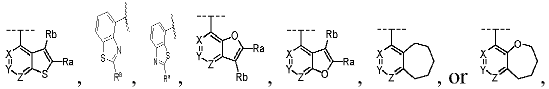 wherein X, Y, and Z are selected from N or CR⁹, and R^{a} and R^{b} are independently hydrogen, halogen, or alkyl, or R^{a} and R^{b} are connected to form a substituted or unsubstituted C₃-C₆ cycloalkyl. and the remaining variables are as defined for formula (I). In some embodiments, the compound of formula (I) is 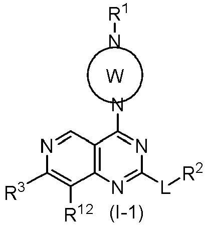 wherein L-R² is 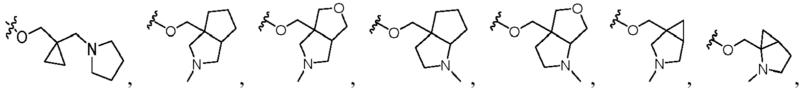 or 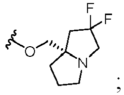 and or R³ is preferably

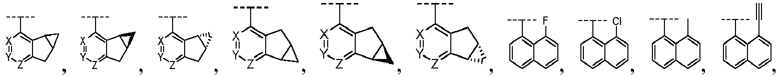

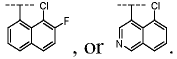

In some embodiments, the compound of formula (I) is as represented by formula (I-2), (I-3), (I-4), (I-5), and (I-6): 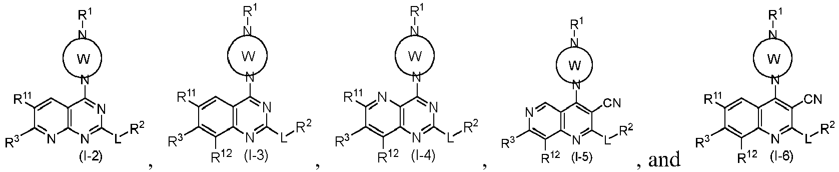 wherein:
R³ is preferably 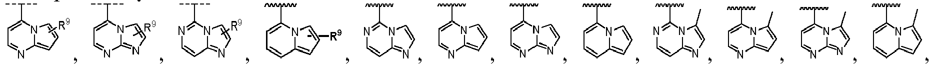 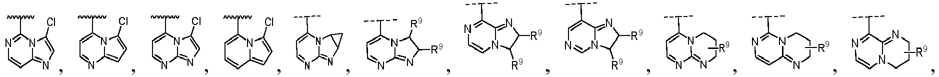 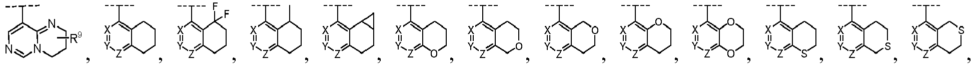 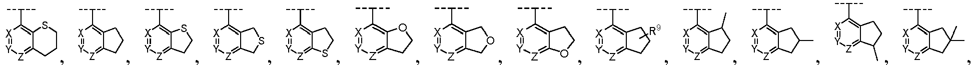 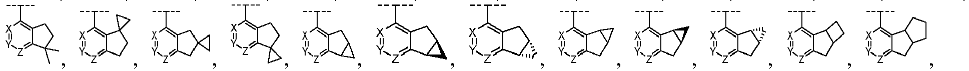 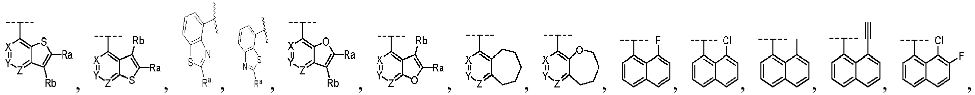 or 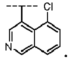 R^{a} and R^{b} are independently hydrogen, halogen, or alkyl, or R^{a} and R^{b} are connected to form a substituted or unsubstituted C₃-C₆ cycloalkyl.

In some embodiments, R'-W is 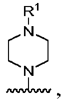 wherein the piperazine ring is optionally additionally substituted with one or more R⁴, and R⁴ is as defined in formula (I). In some embodiments, R⁴ is C₁₋C₃ alkyl, wherein the alkyl group is unsubstituted or substituted with cyano. In some embodiments, R'-W in the compound of formula (I) is

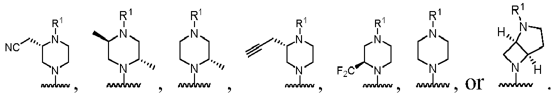

In some embodiments, R¹ is the group of: 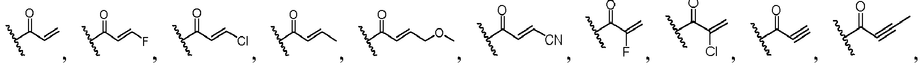 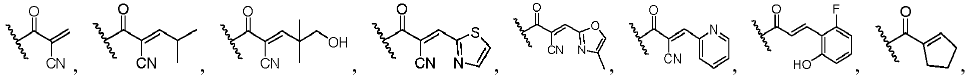 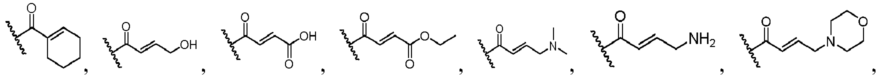 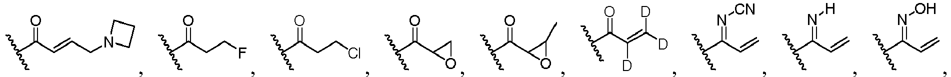 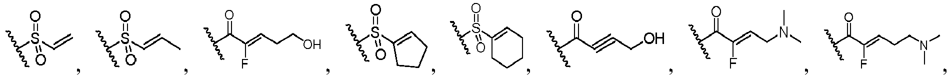 or

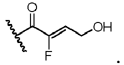

In a preferred embodiment, R'-W is 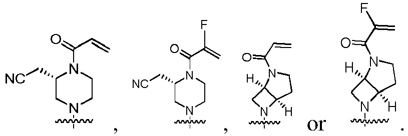 In some embodiments, L-R² is 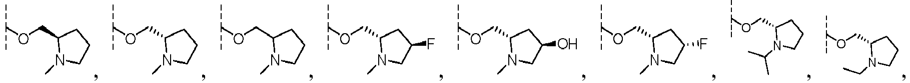 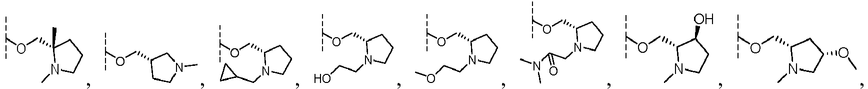 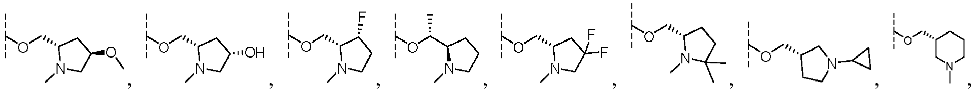 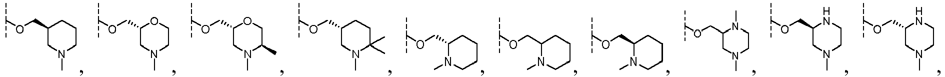 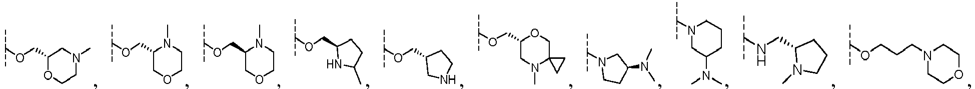 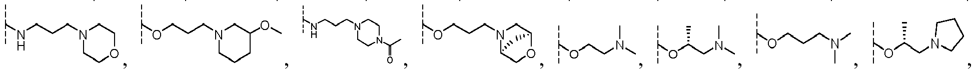 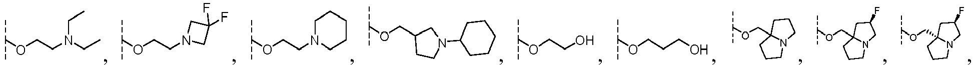 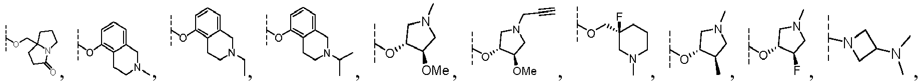 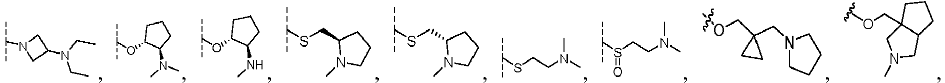 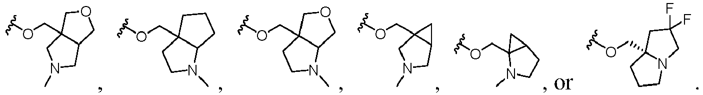 In a preferred embodiment, L-R² is 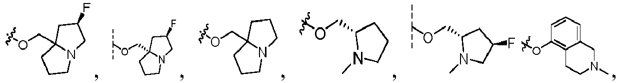 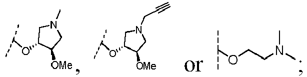 more preferably 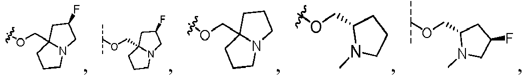 or 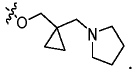 In some embodiments, R³ is 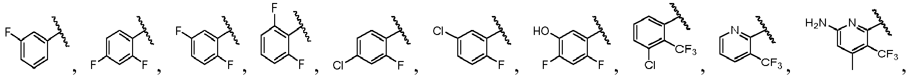 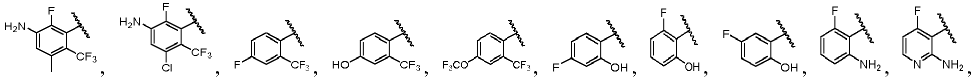 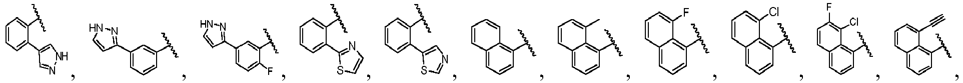 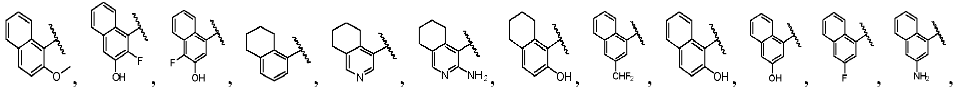 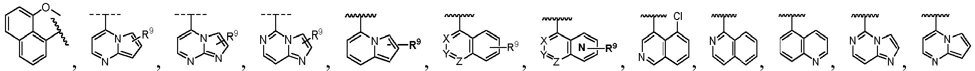 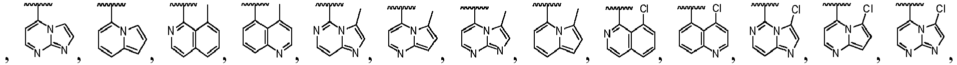 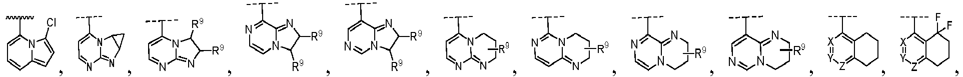 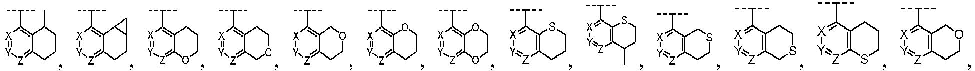 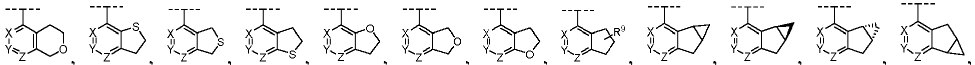 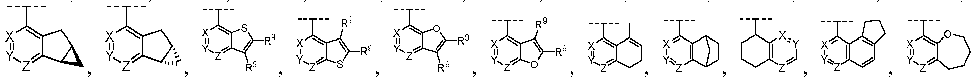 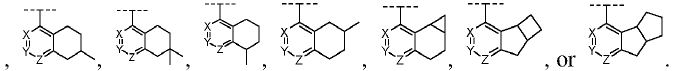 In a preferred embodiment, R³ is

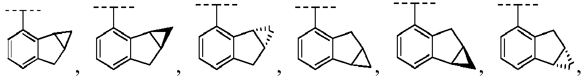

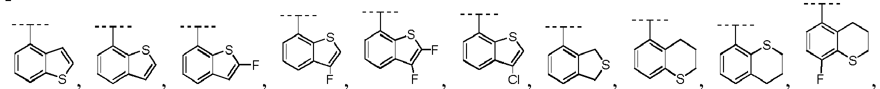

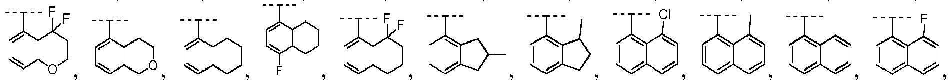

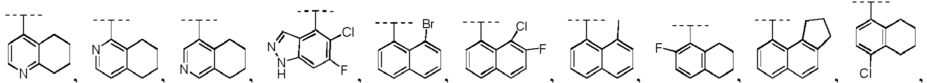

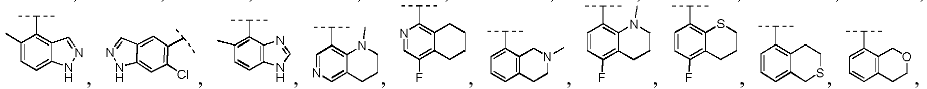

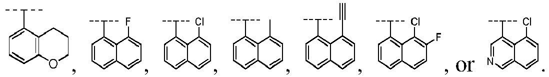

In some embodiments, R¹¹ is hydrogen, nitro, hydroxyl, halogen, cyano, alkyl, haloalkyl, alkoxy, or alkoxyalkyl; preferably hydrogen, halogen, cyano, trifluoromethyl, or nitro.In a preferred embodiment, R¹¹ is F.

In some embodiments, R¹² is hydrogen, hydroxyl, halogen, C₁-C₆ alkyl, C₃-C₆ cycloalkyl, C₃-C₆ cycloalkyloxy, heterocyclyl, C₁-C₆ haloalkyl, aryl, or heteroaryl, wherein the aryl and heteroaryl are each unsubstituted or substituted with one or more of C₁-C₃ alkyl, halogen, C₁-C₃ haloalkyl, and C₃-C₆ cycloalkyl.In a preferred embodiment, R¹² is F or cyclopropyloxy.

In some embodiments, the compound of formula (I) is 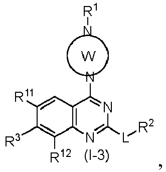 wherein R'-W is 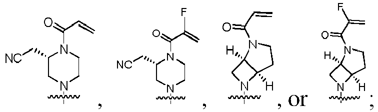 R³ is 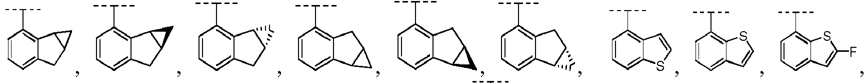 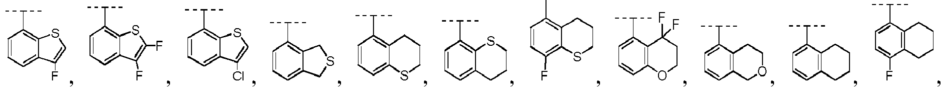 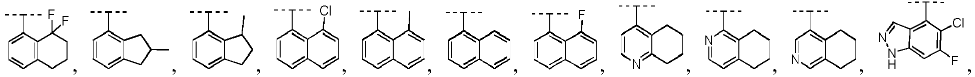 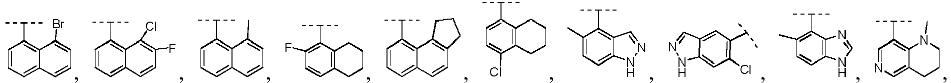 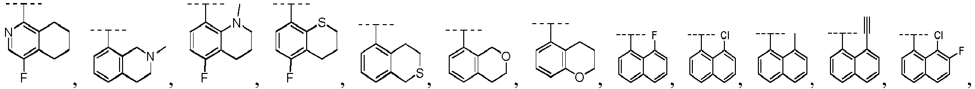 or 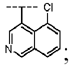 and L-R² is 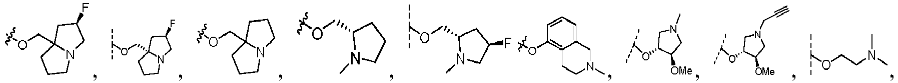 or 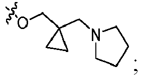 more preferably 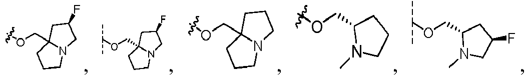 or 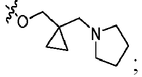 R¹¹ and R¹² are each independently hydrogen, hydroxyl, alkyl, C₃-C₆ cycloalkyloxy, or halogen. In some embodiments, the compound of formula (I) is

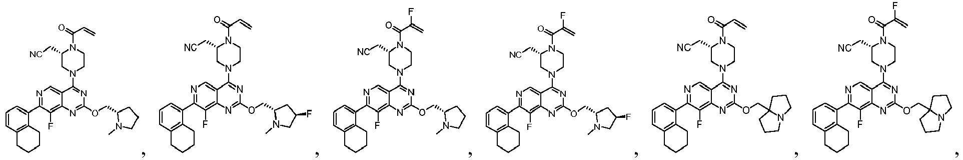

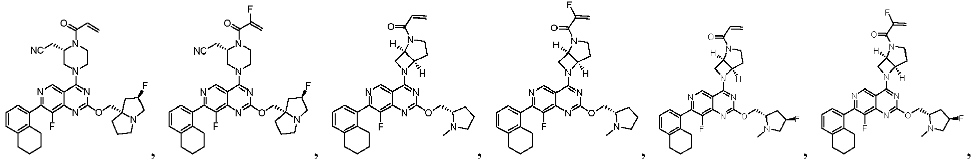

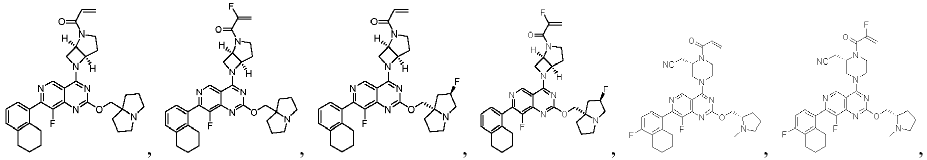

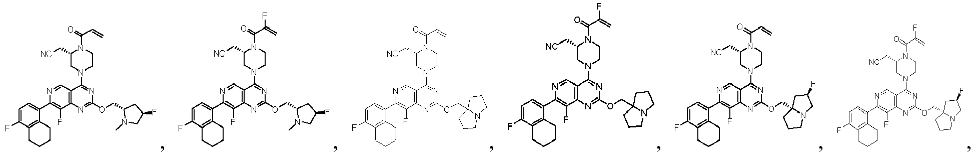

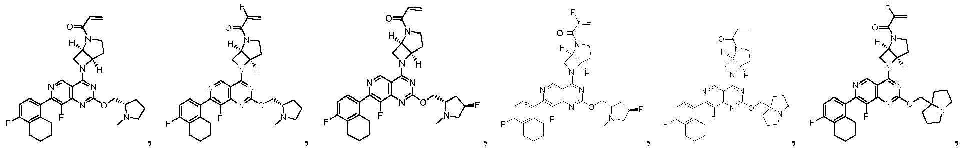

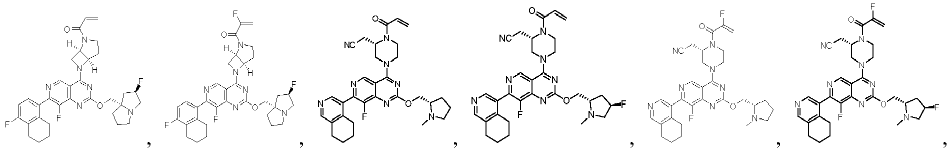

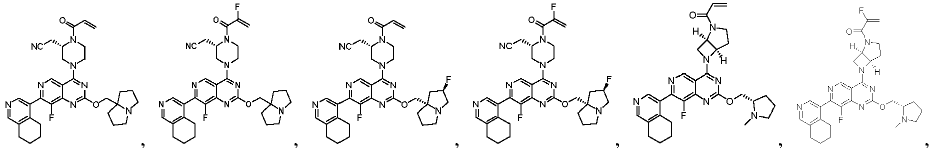

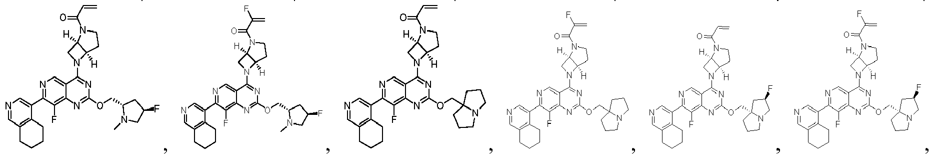

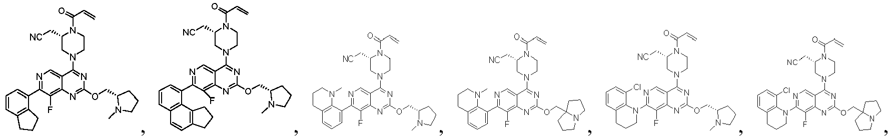

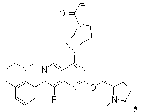

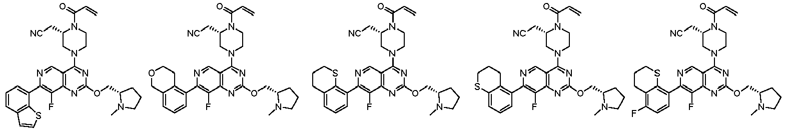

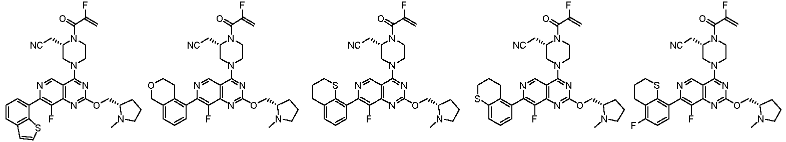

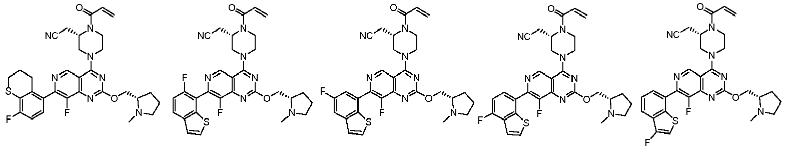

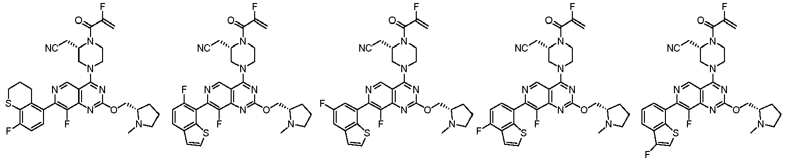

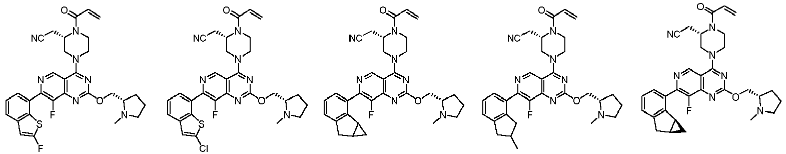

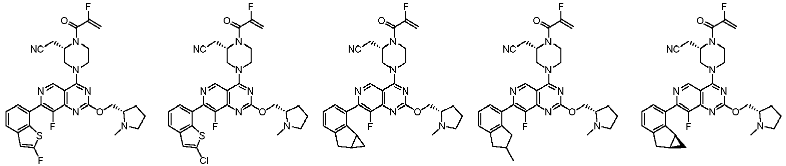

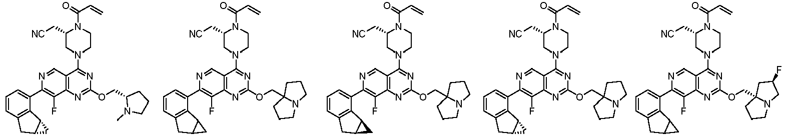

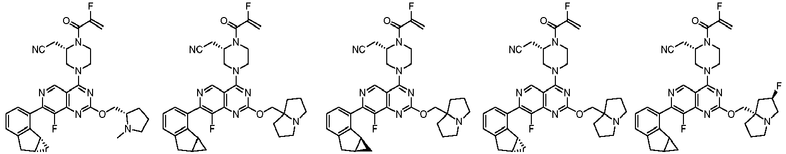

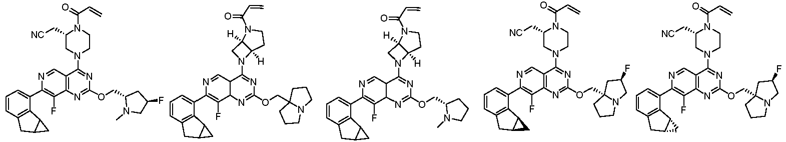

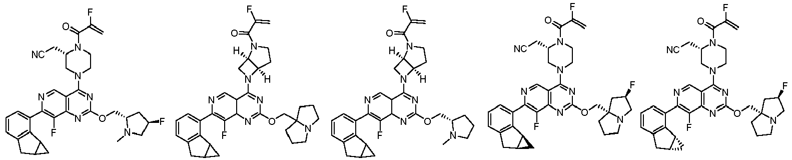

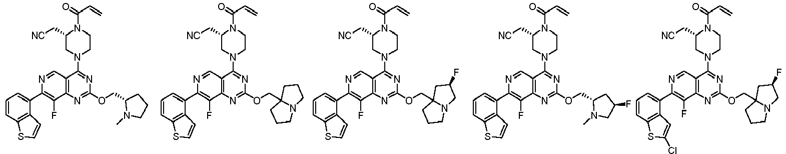

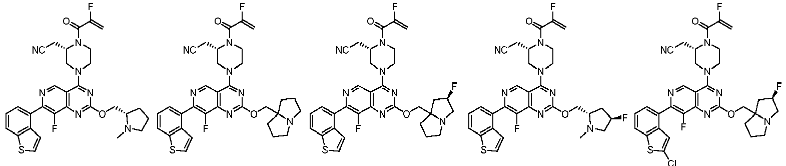

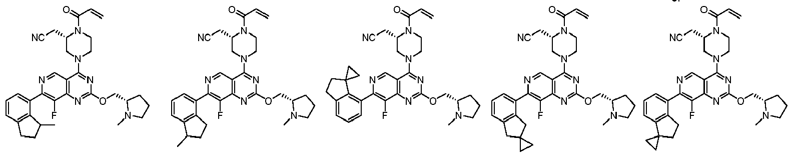

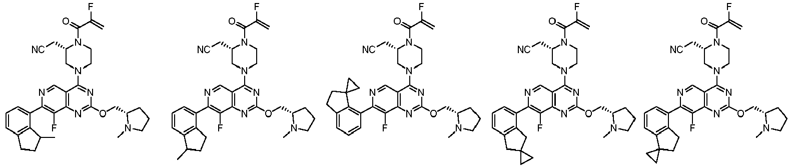

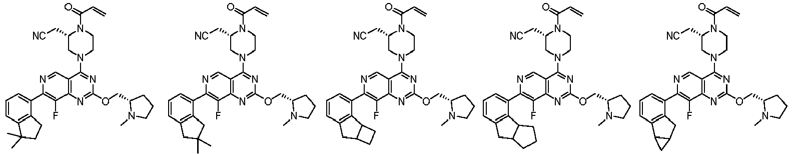

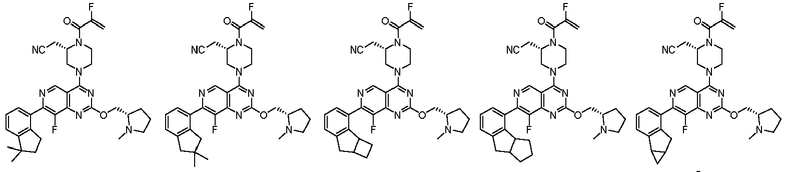

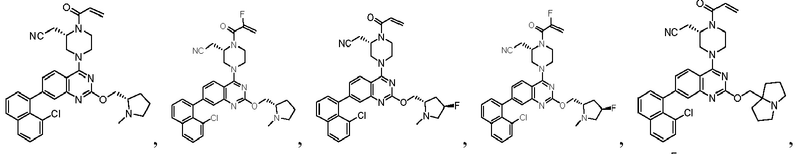

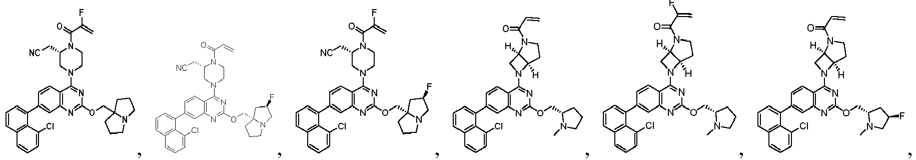

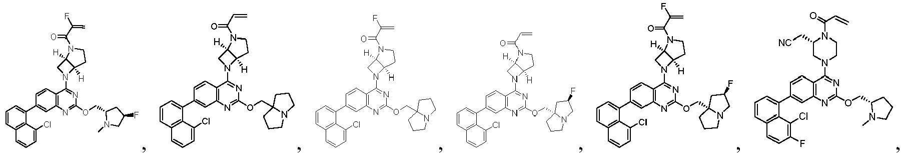

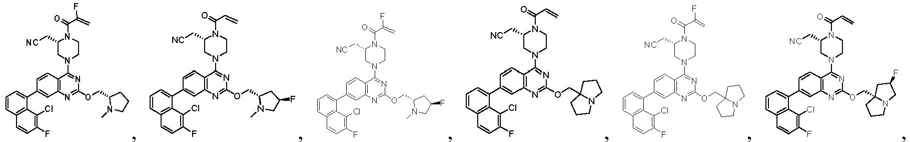

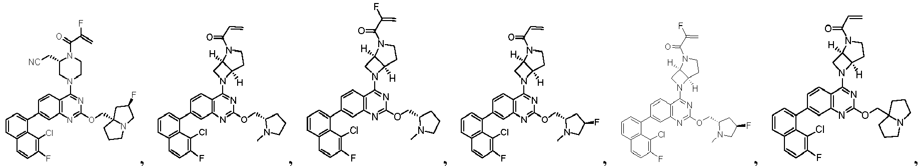

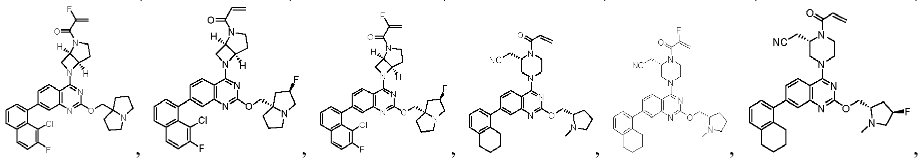

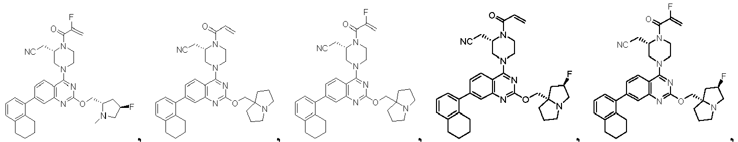

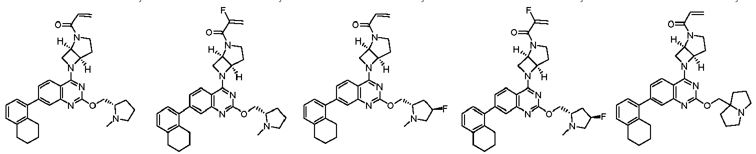

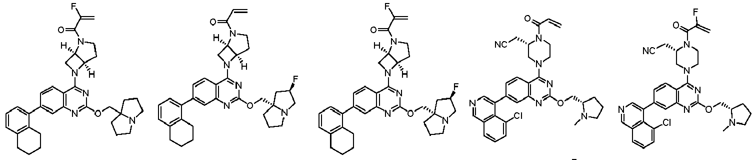

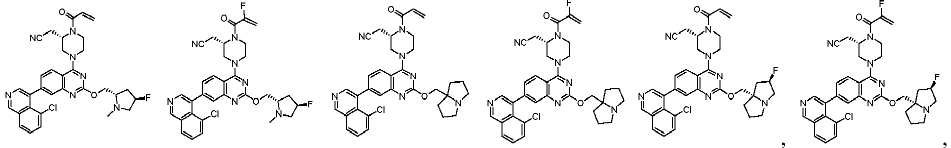

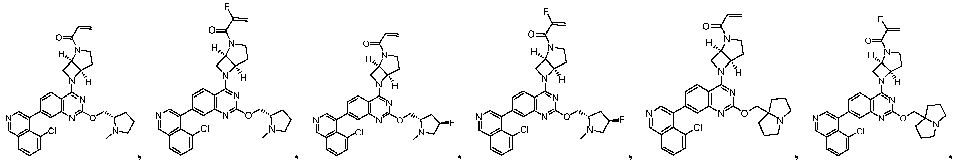

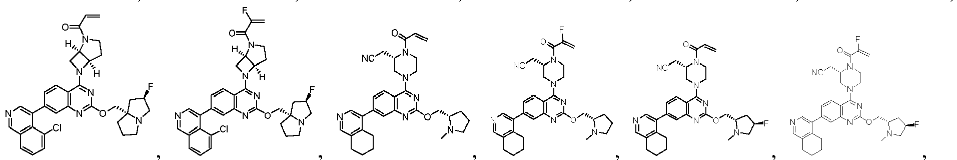

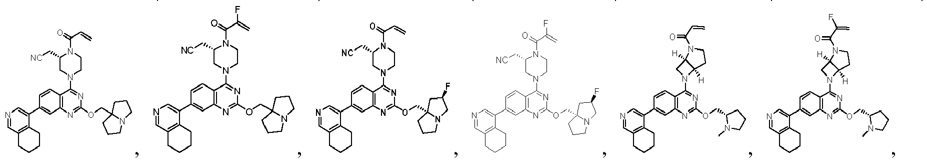

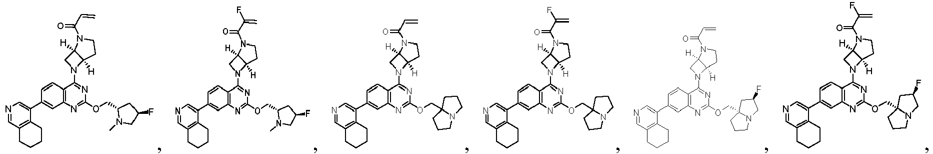

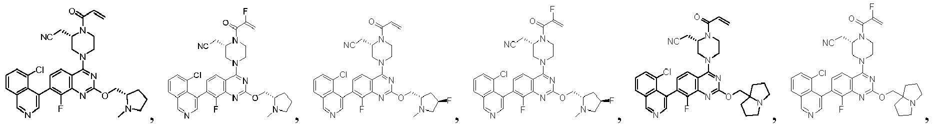

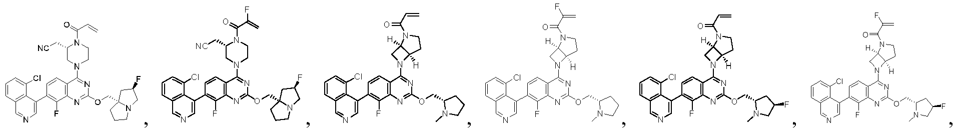

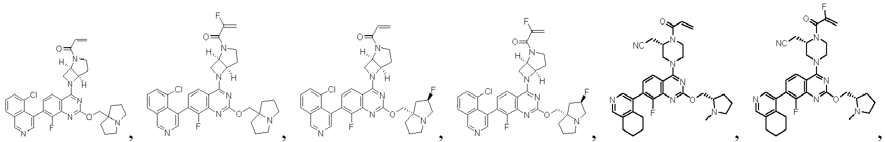

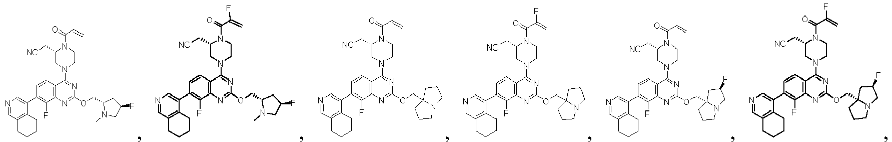

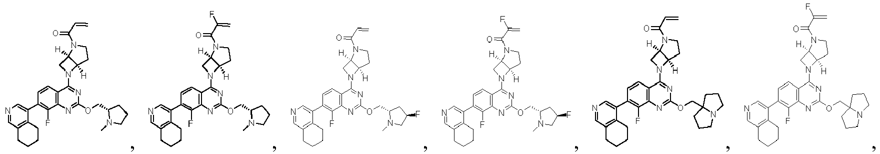

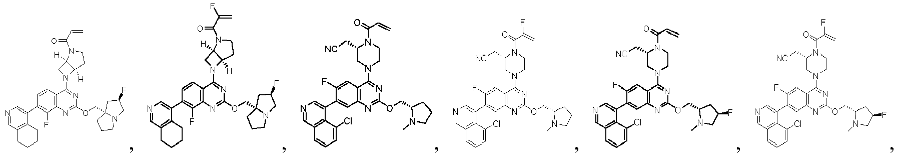

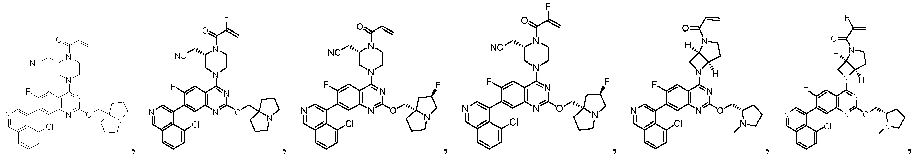

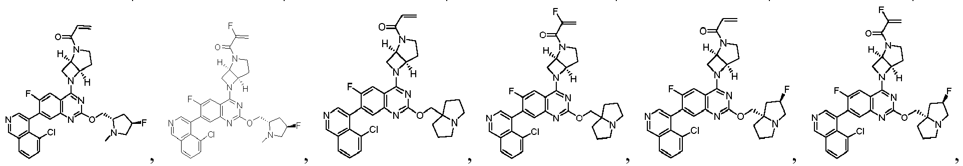

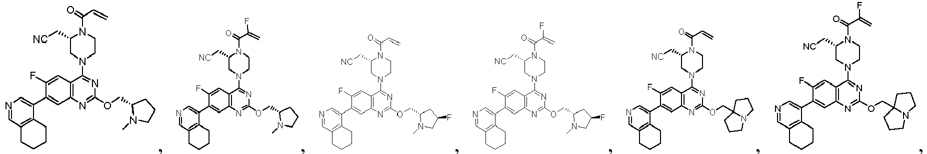

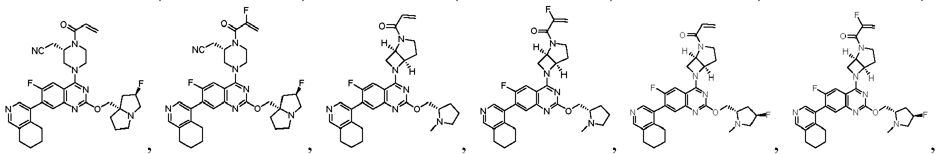

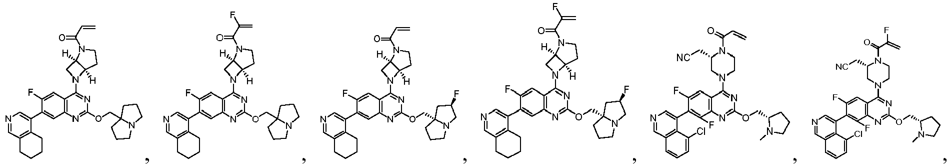

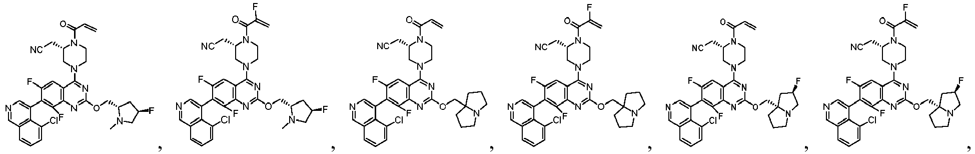

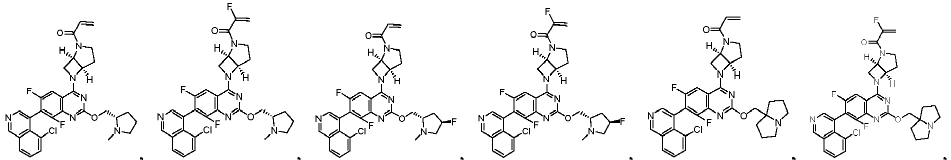

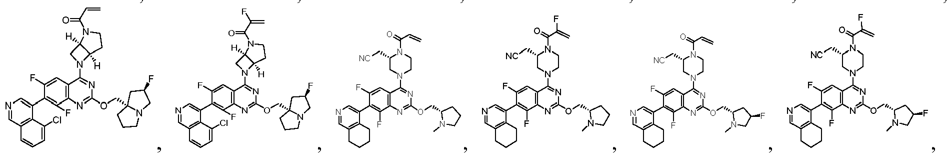

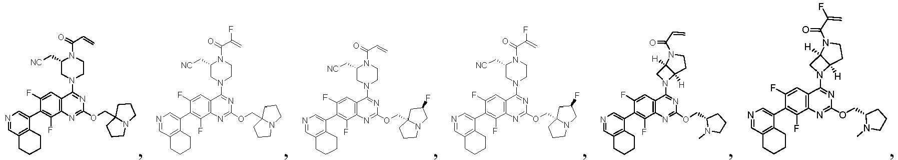

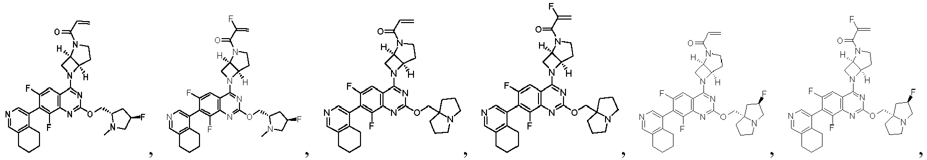

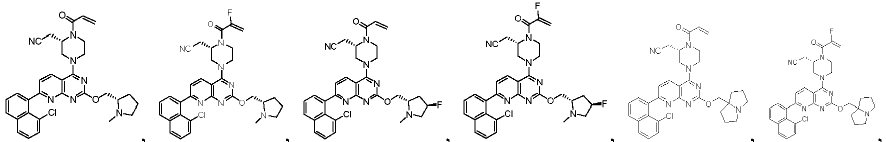

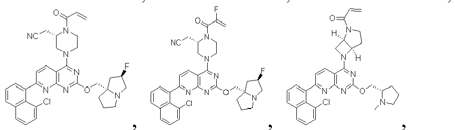

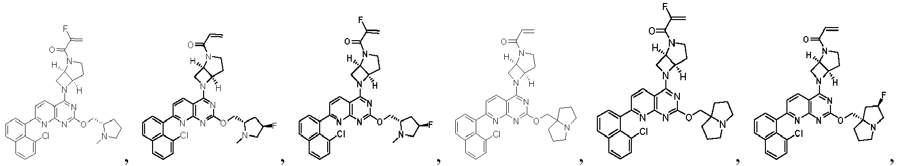

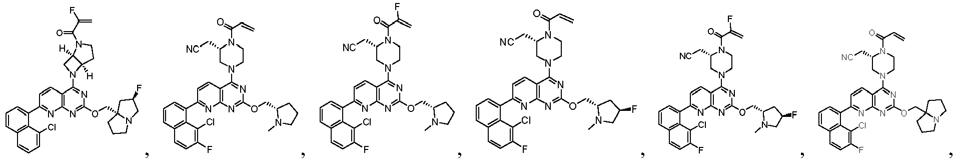

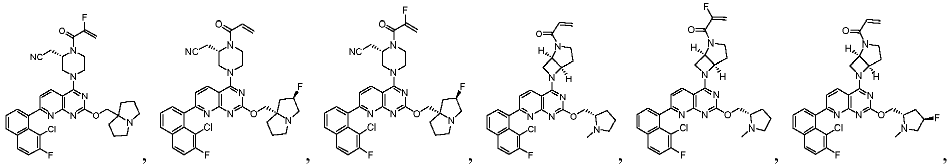

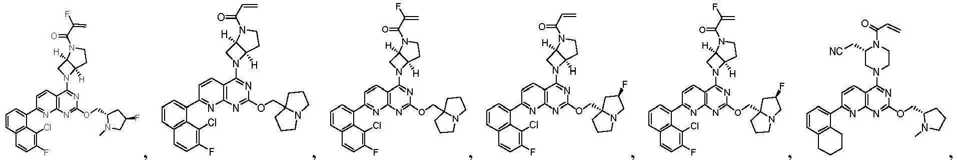

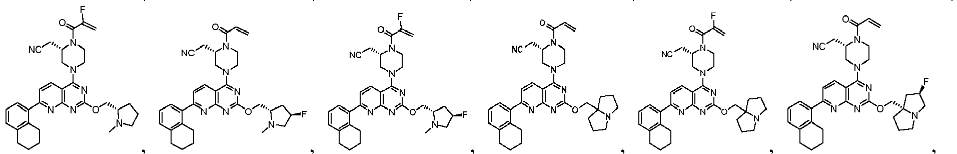

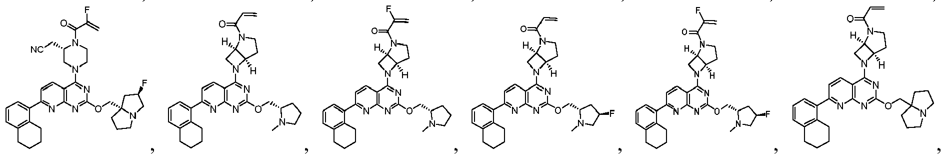

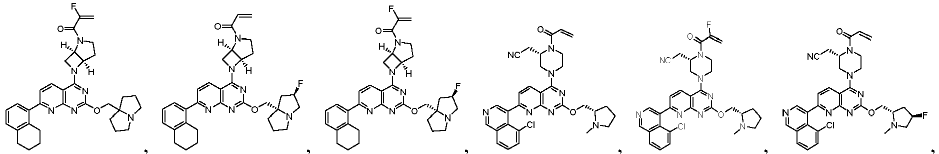

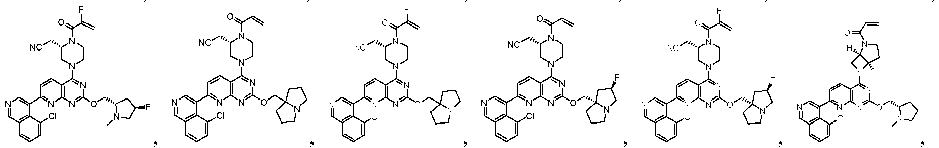

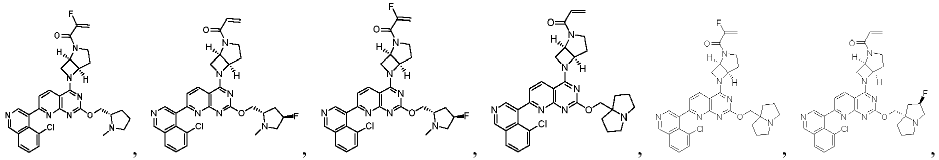

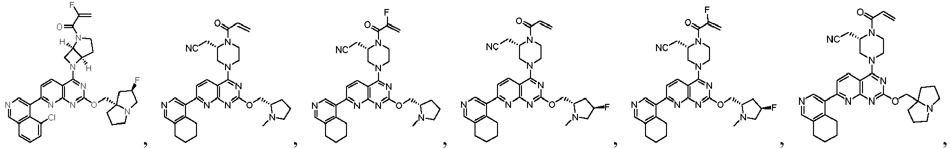

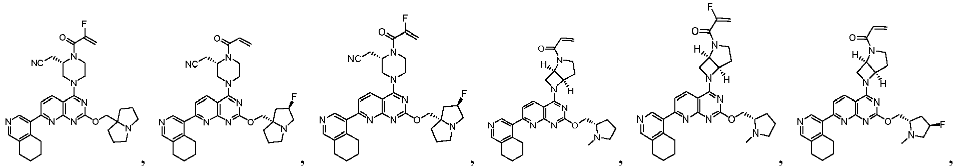

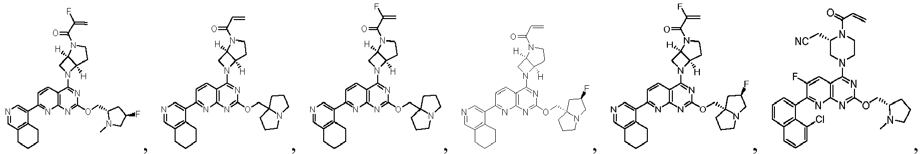

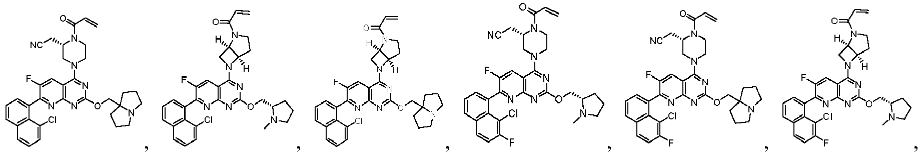

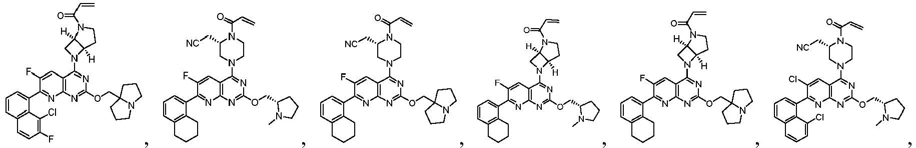

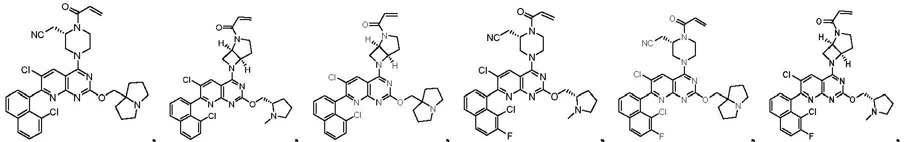

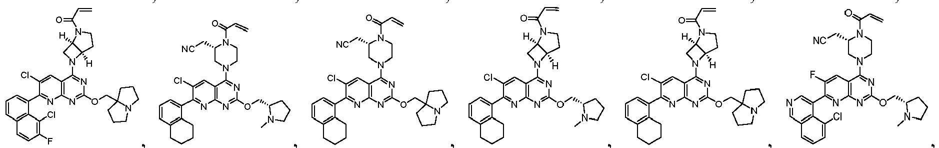

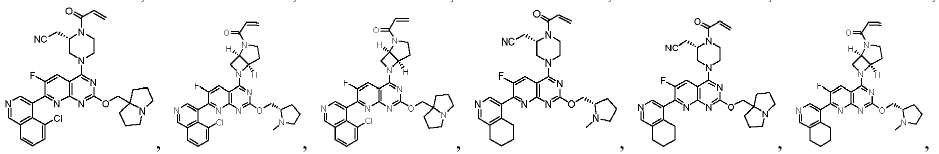

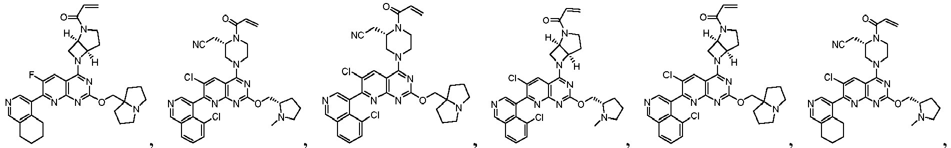

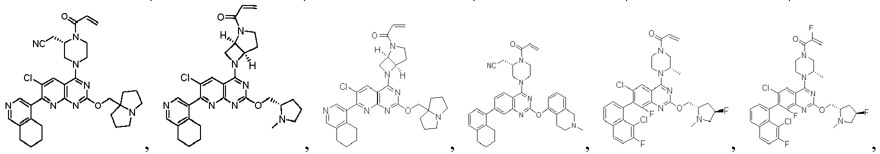

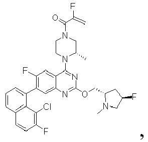

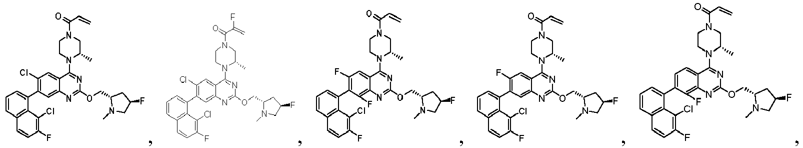

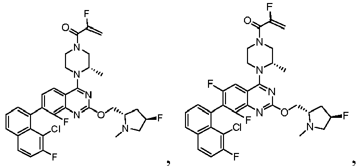

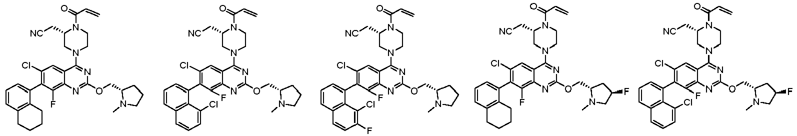

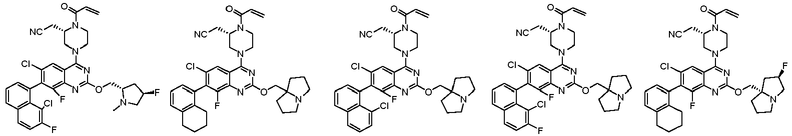

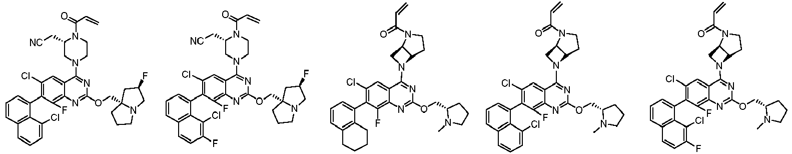

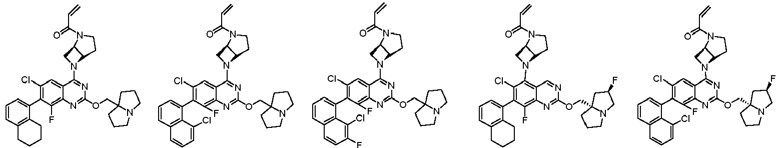

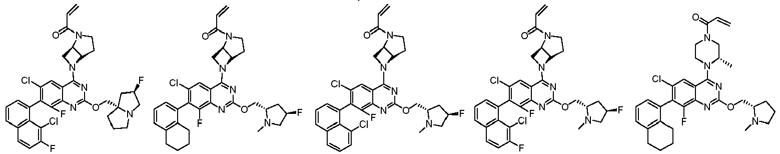

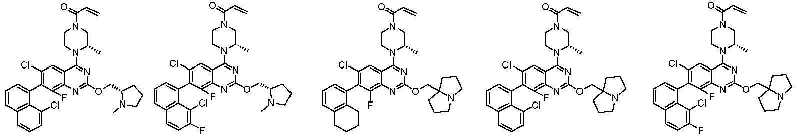

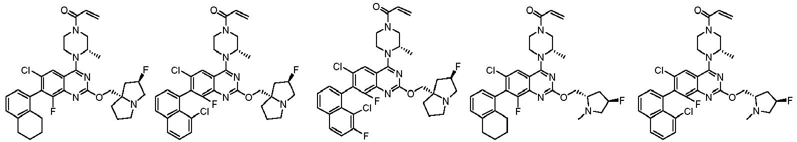

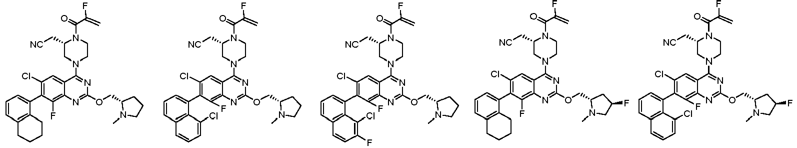

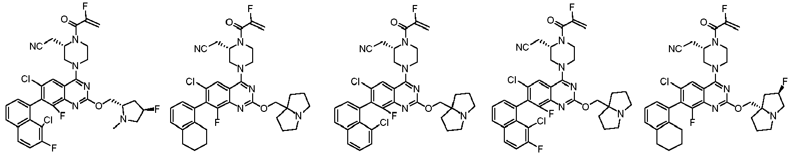

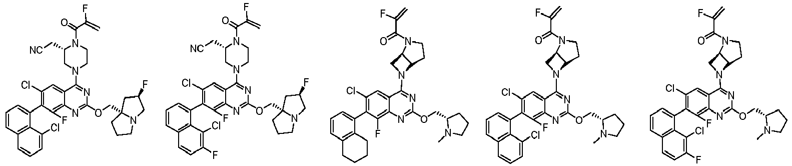

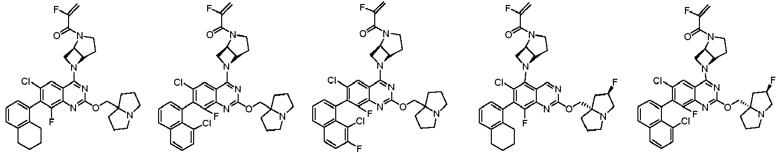

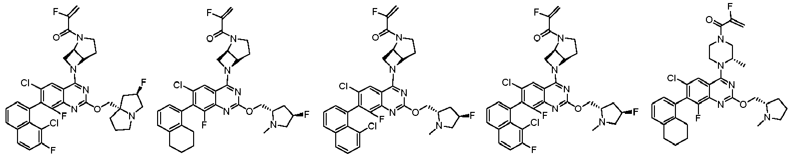

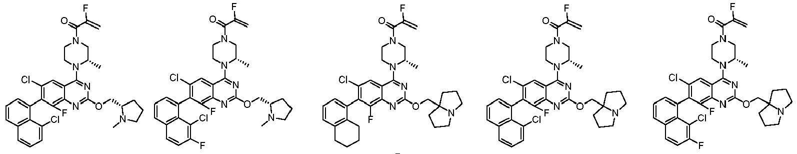

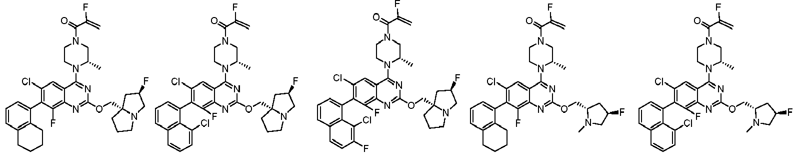

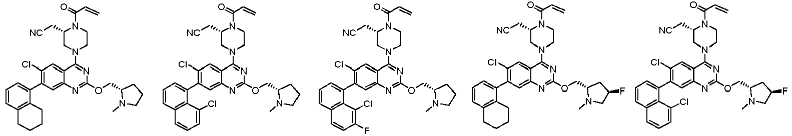

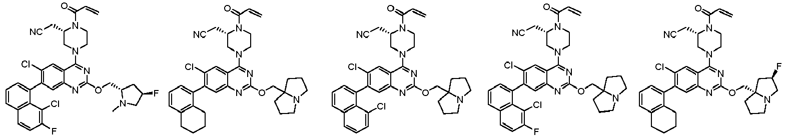

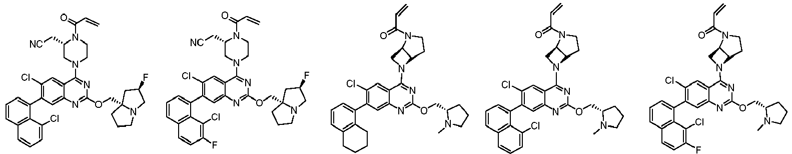

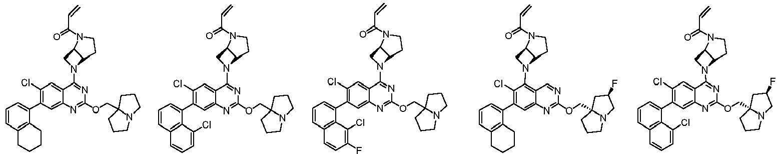

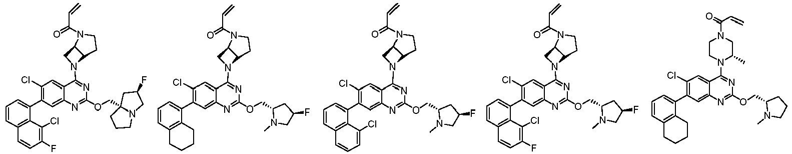

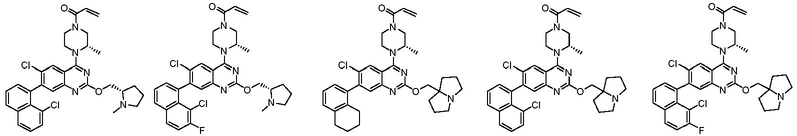

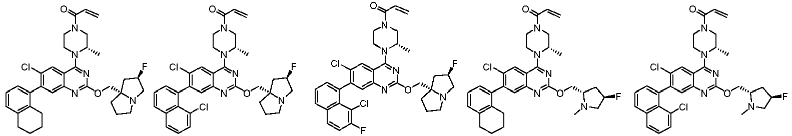

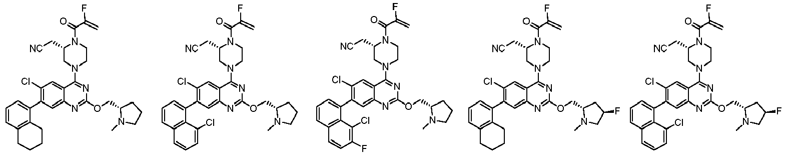

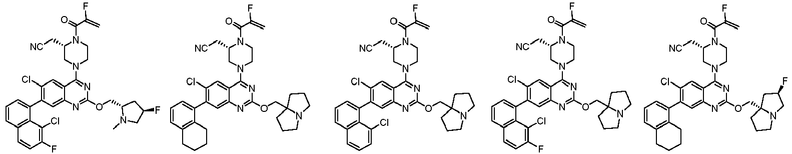

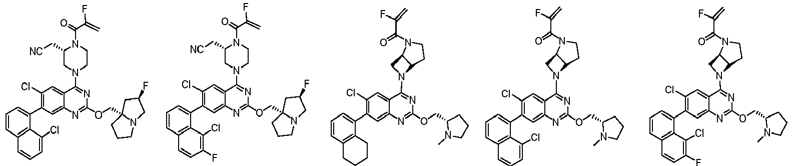

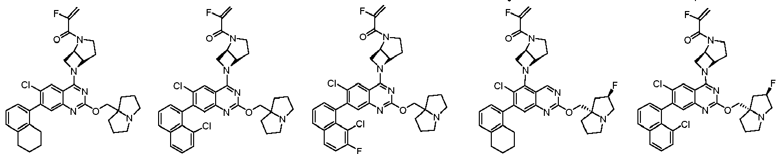

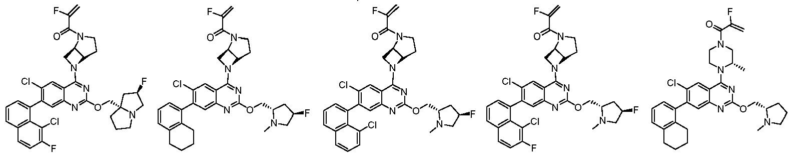

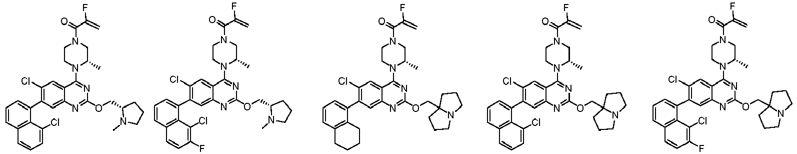

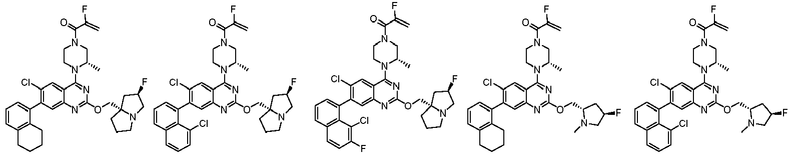

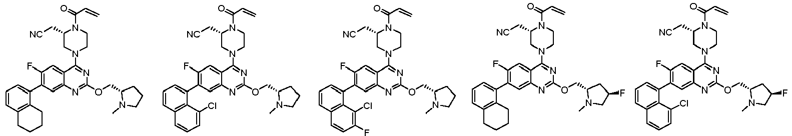

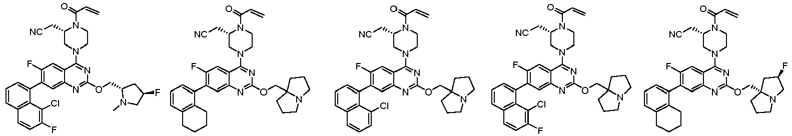

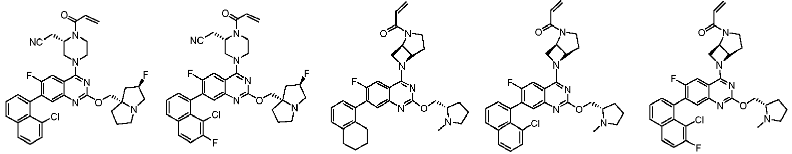

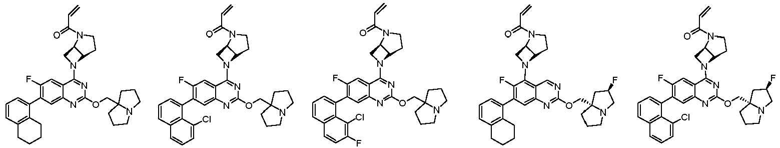

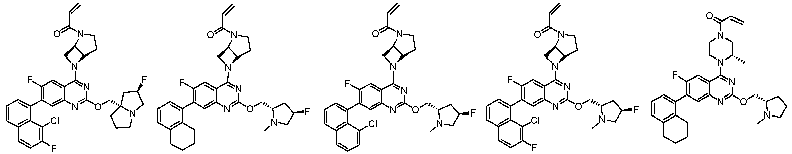

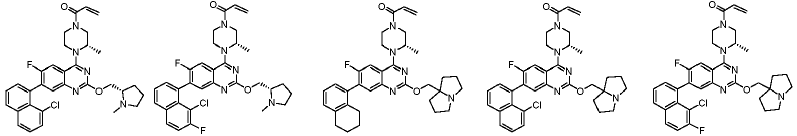

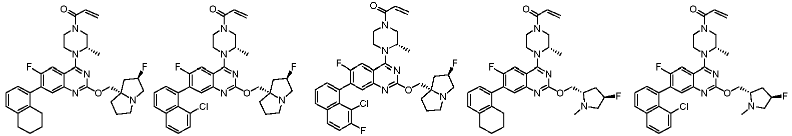

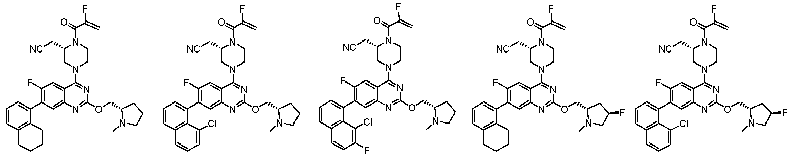

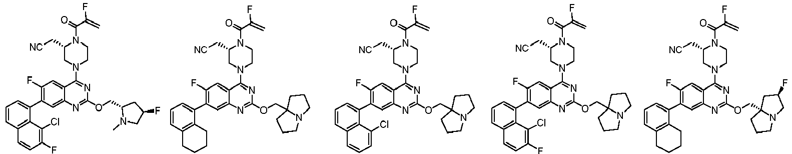

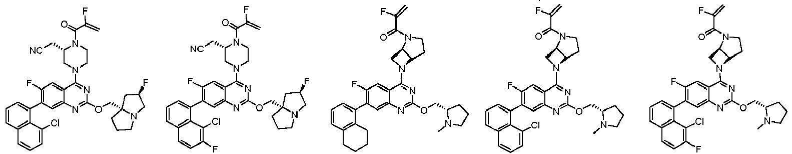

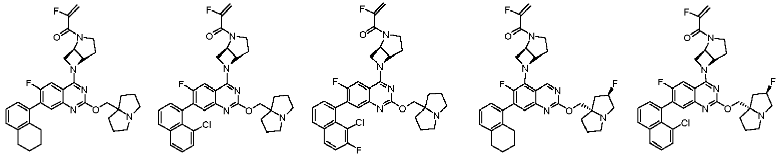

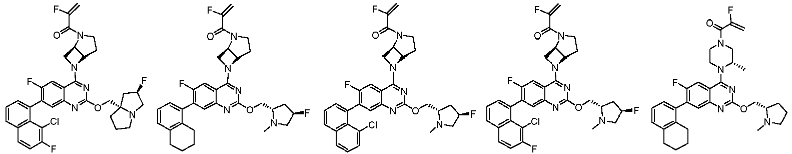

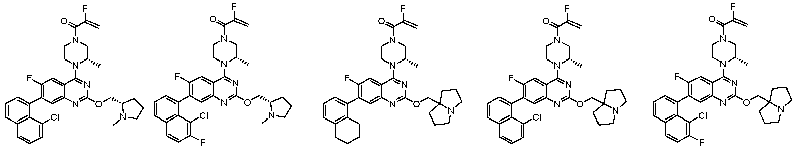

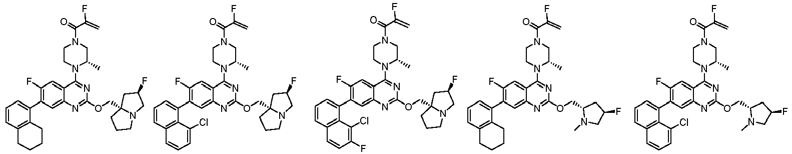

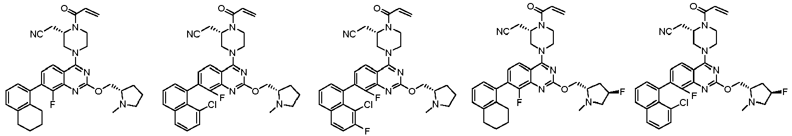

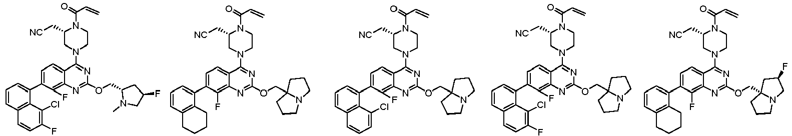

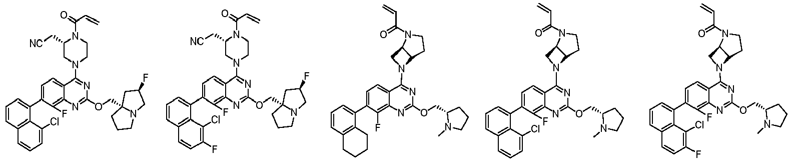

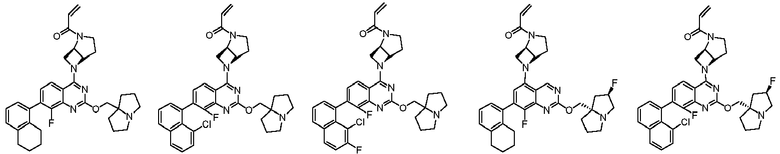

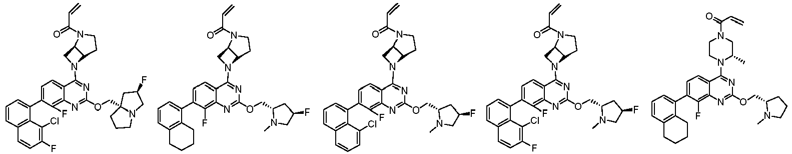

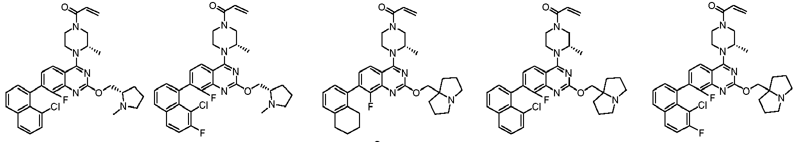

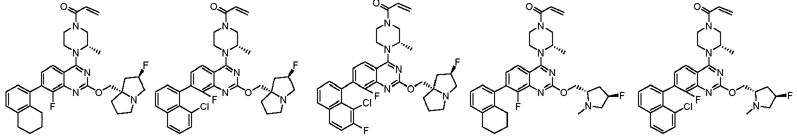

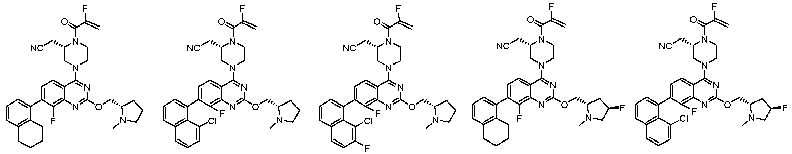

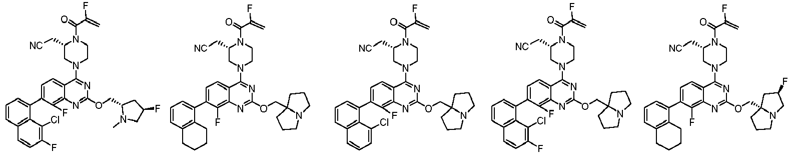

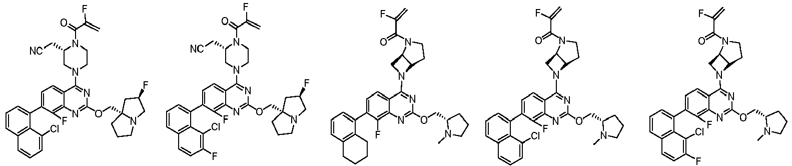

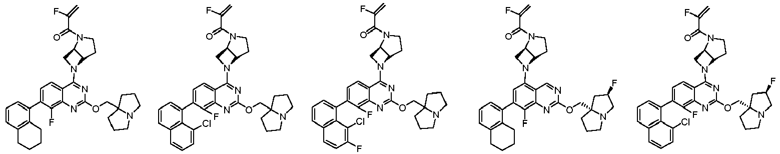

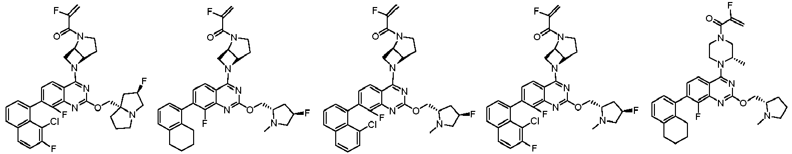

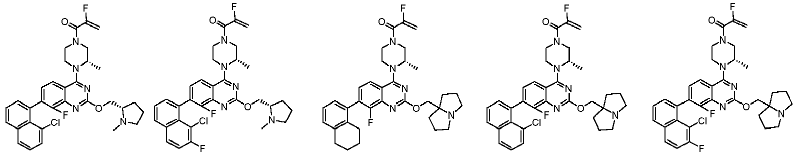

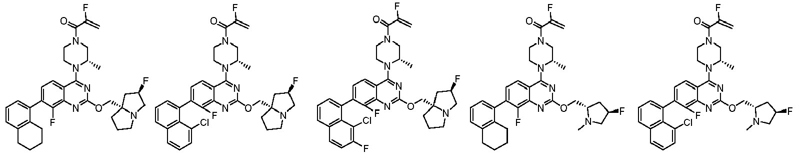

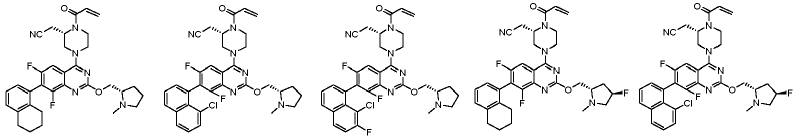

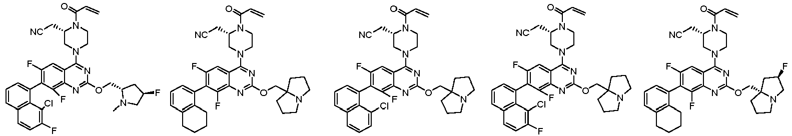

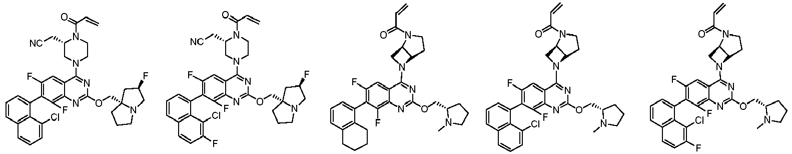

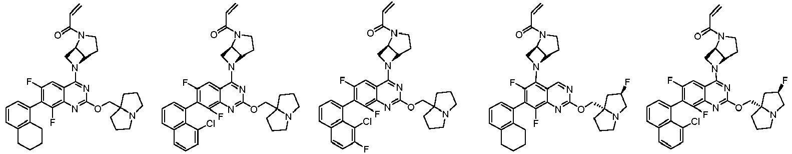

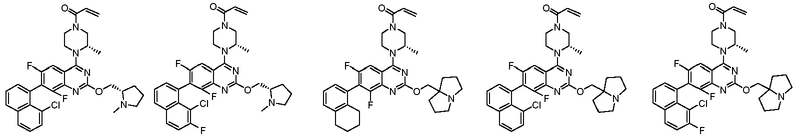

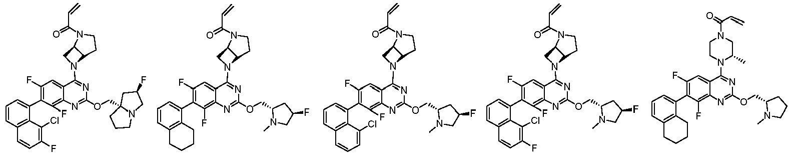

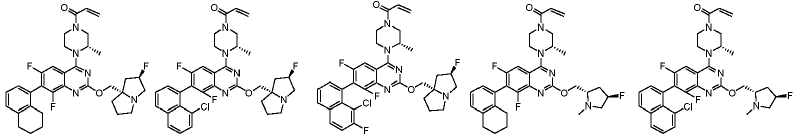

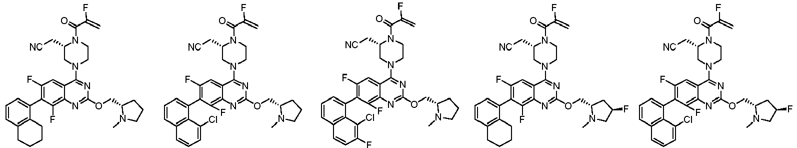

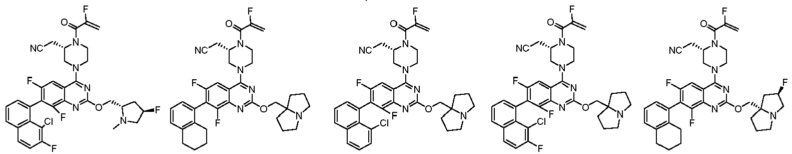

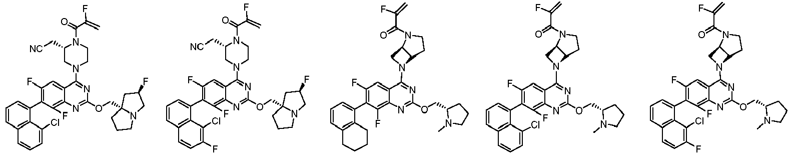

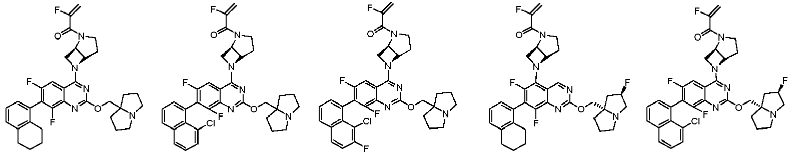

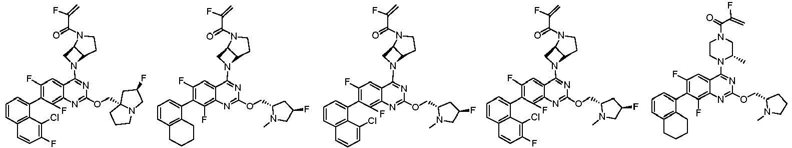

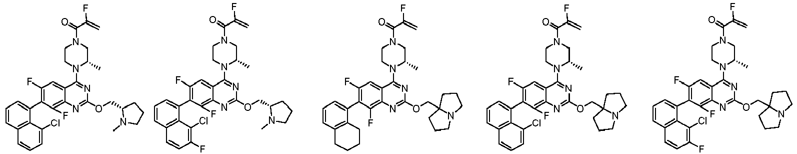

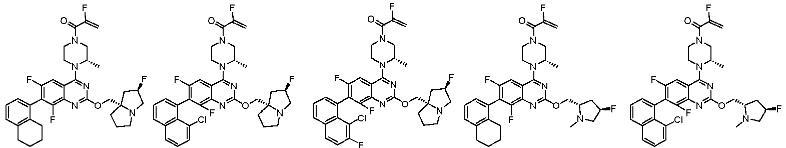

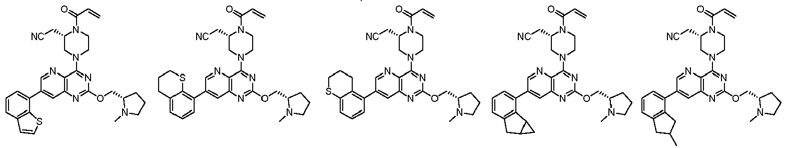

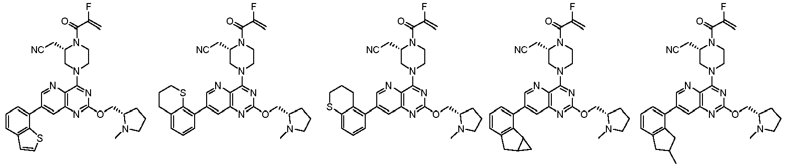

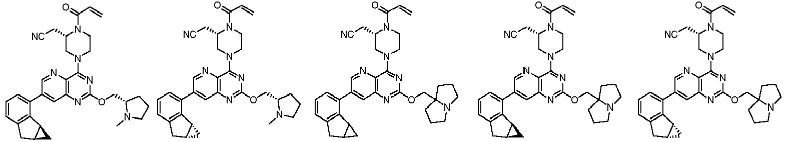

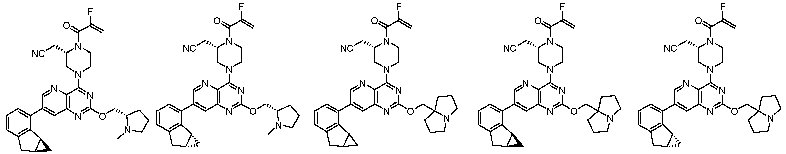

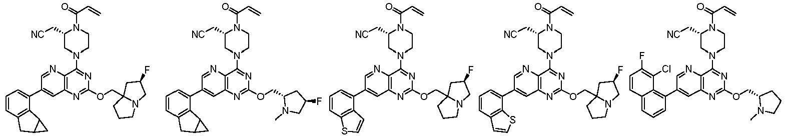

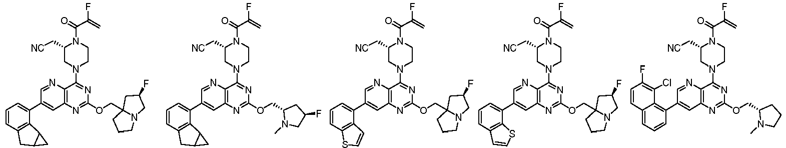

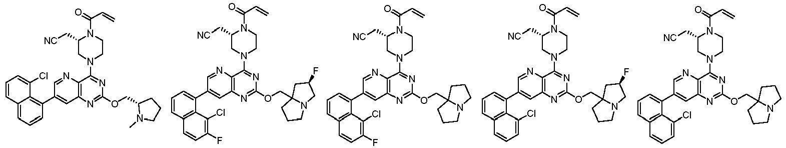

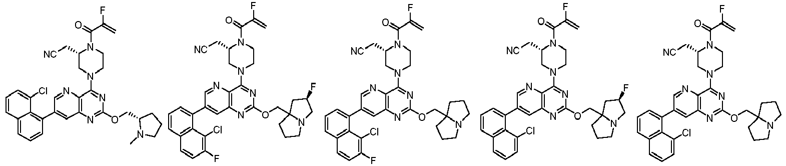

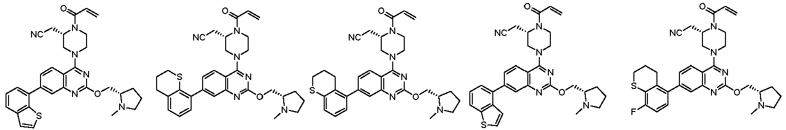

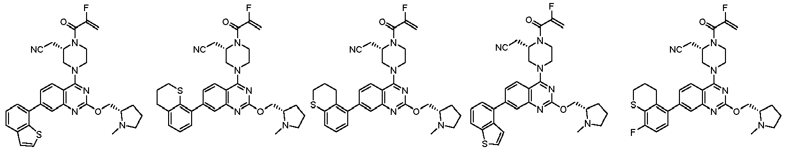

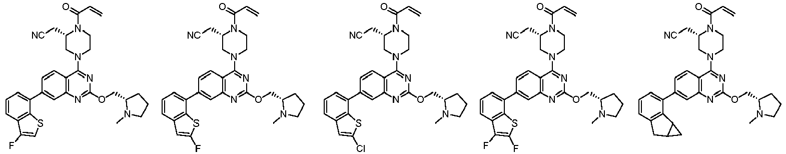

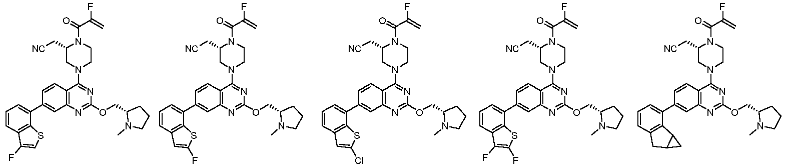

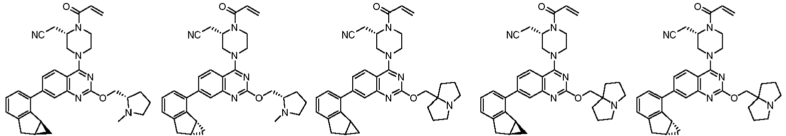

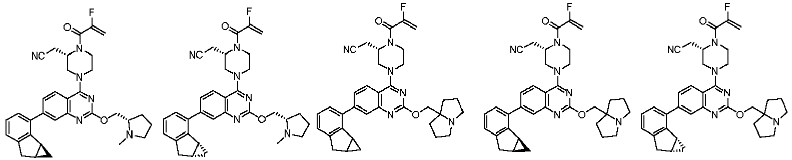

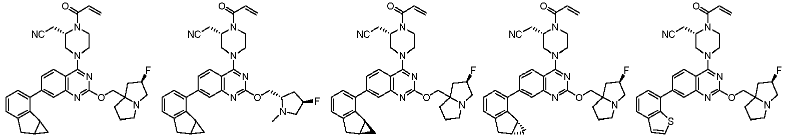

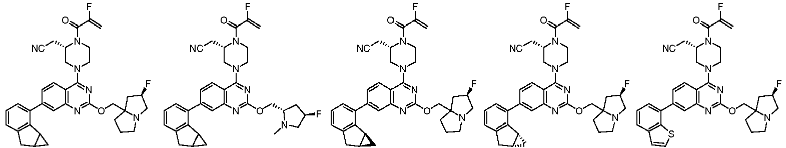

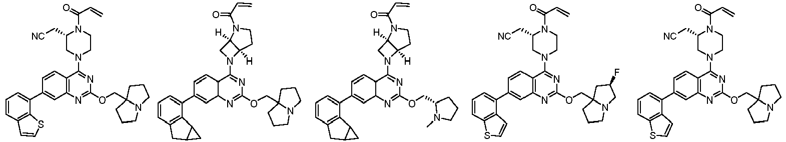

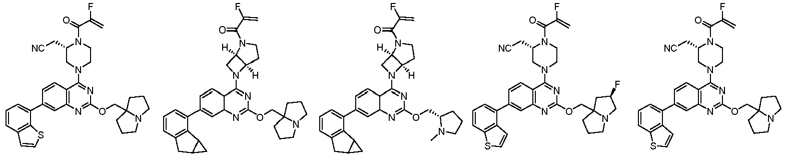

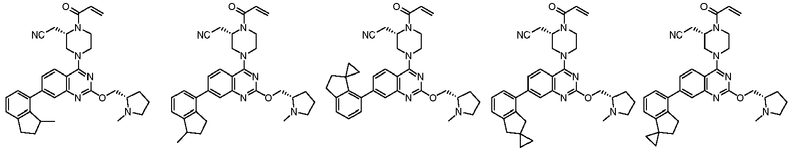

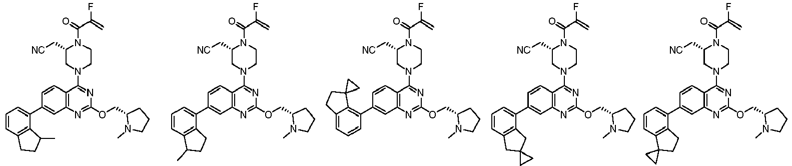

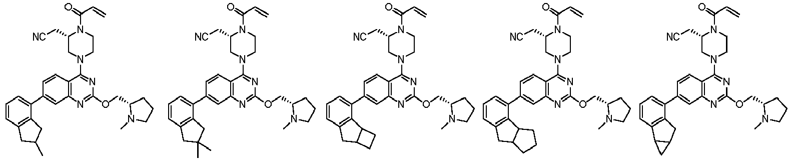

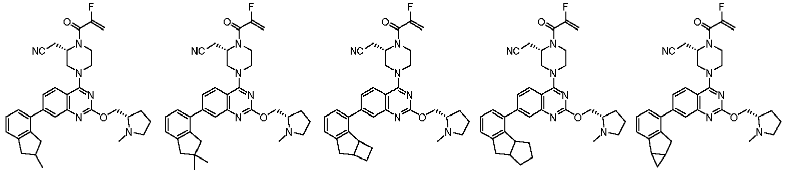

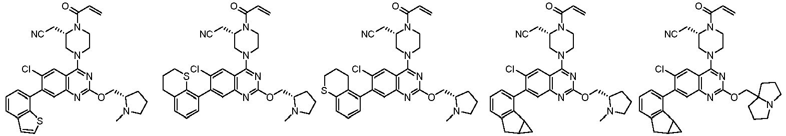

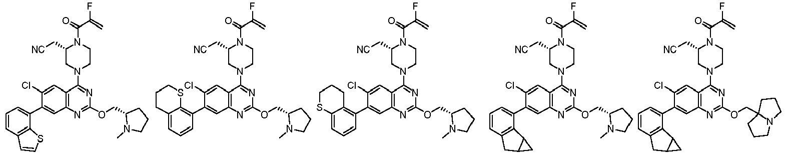

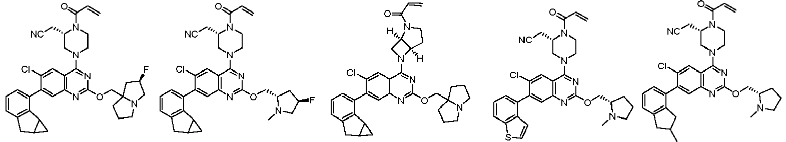

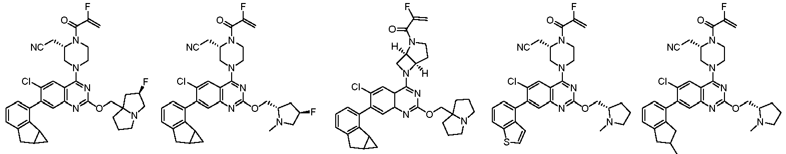

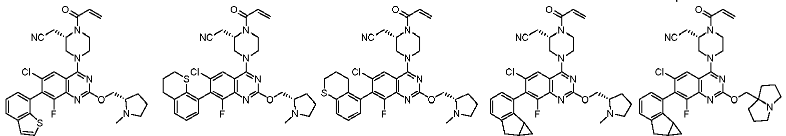

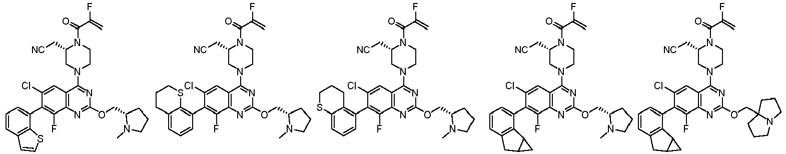

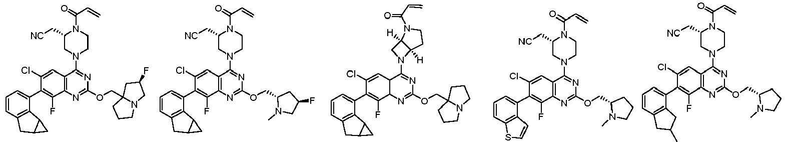

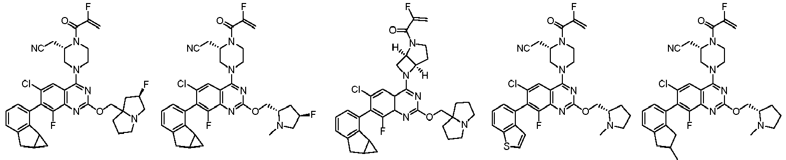

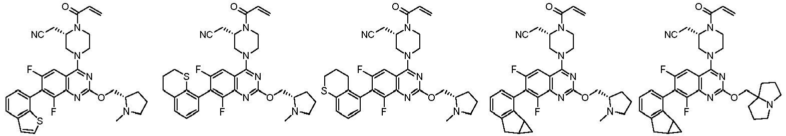

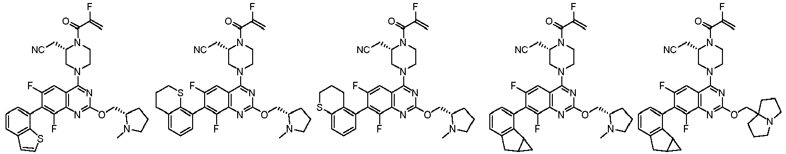

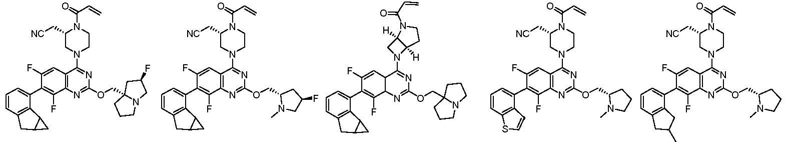

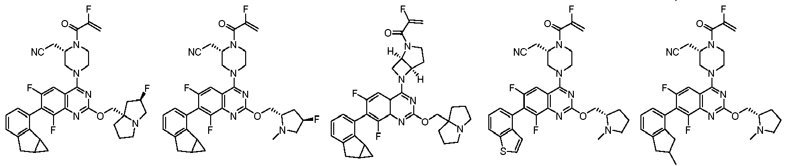

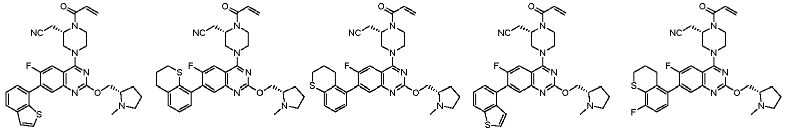

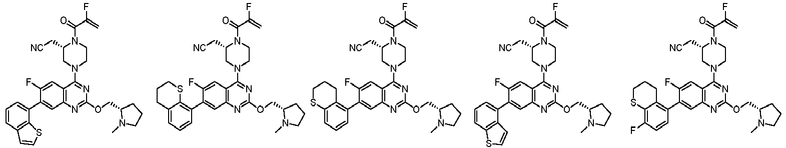

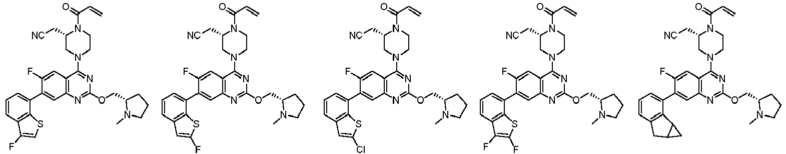

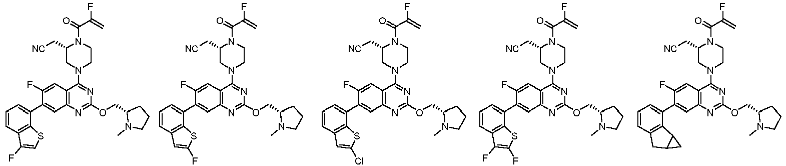

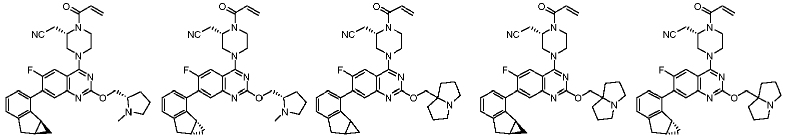

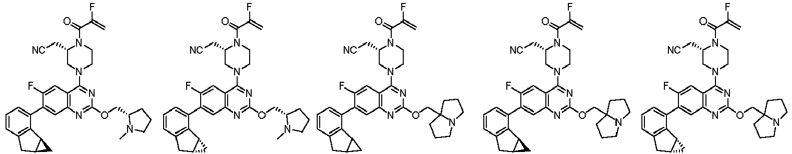

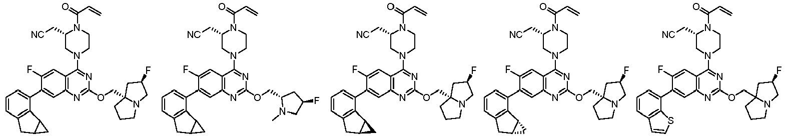

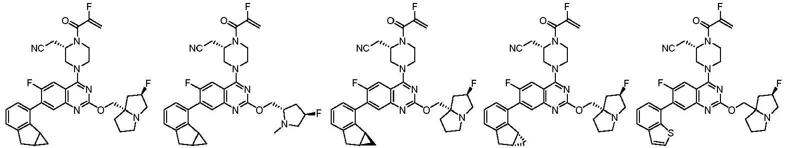

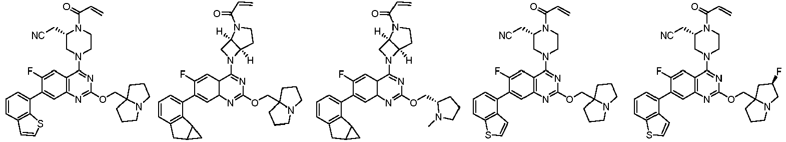

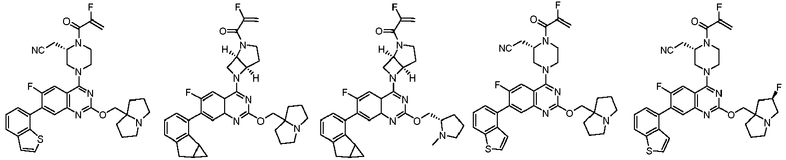

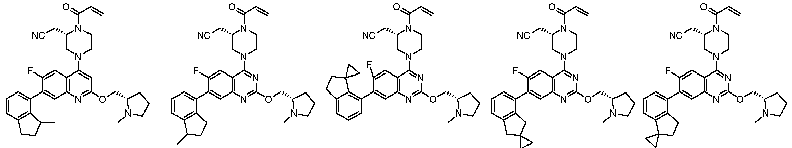

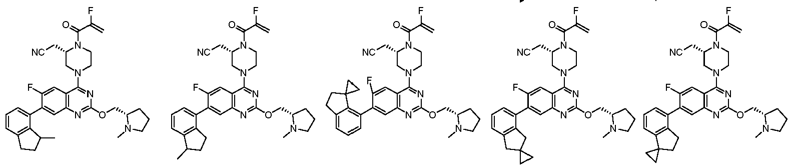

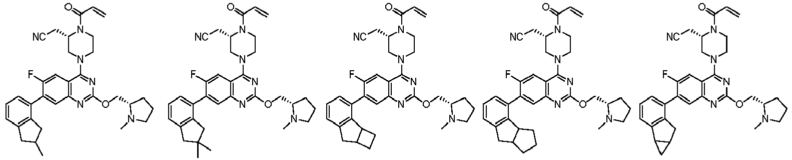

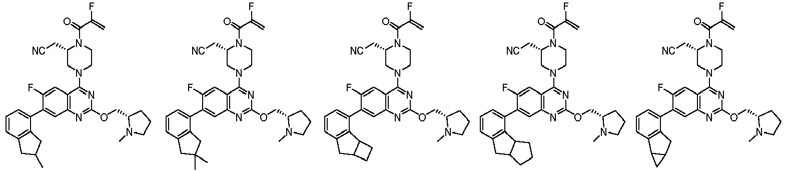

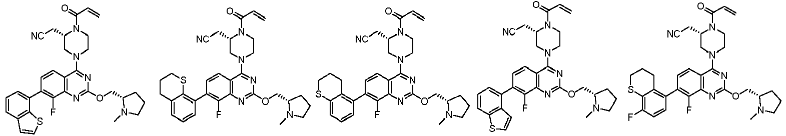

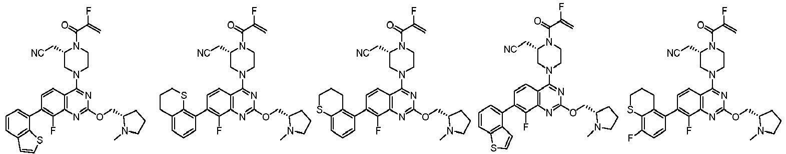

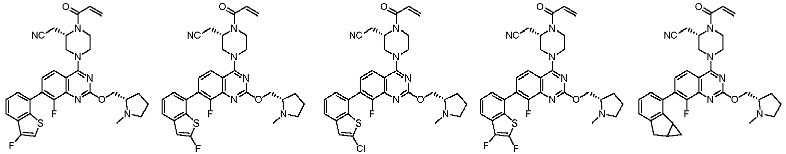

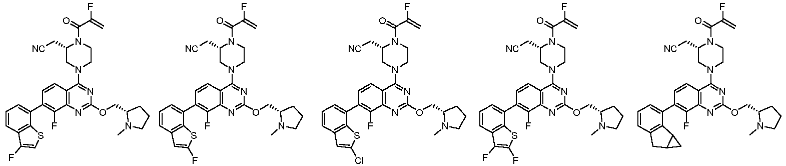

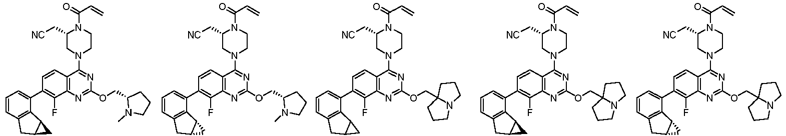

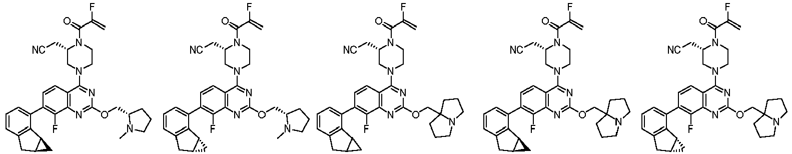

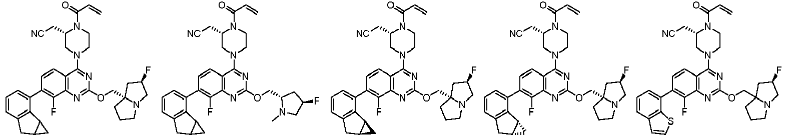

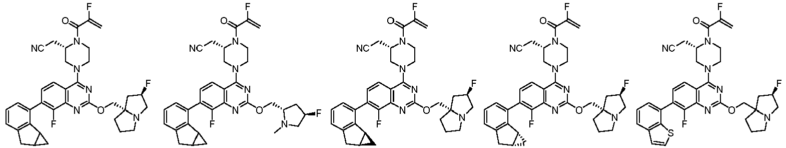

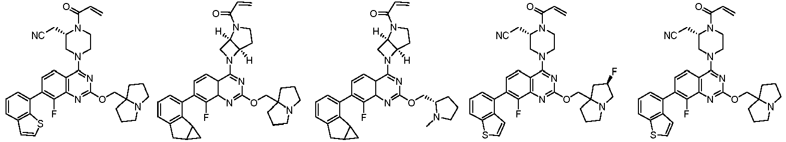

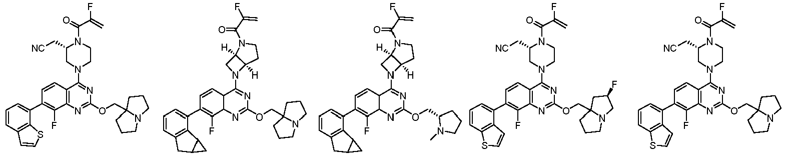

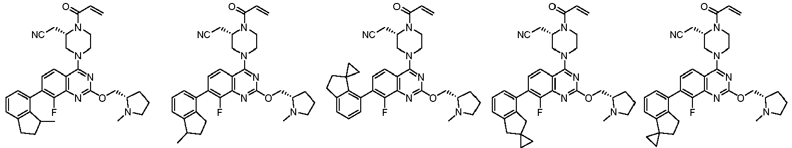

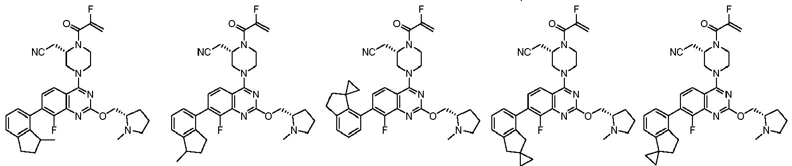

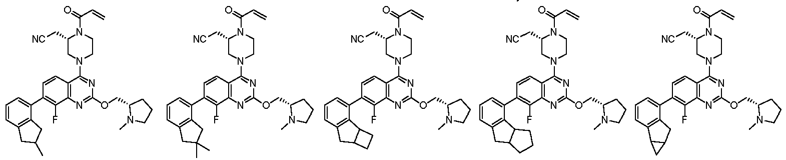

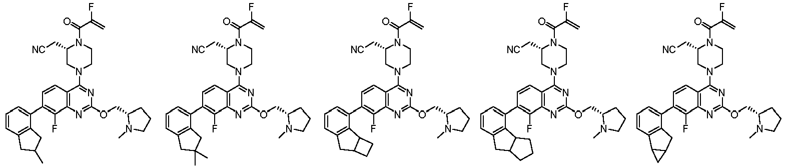

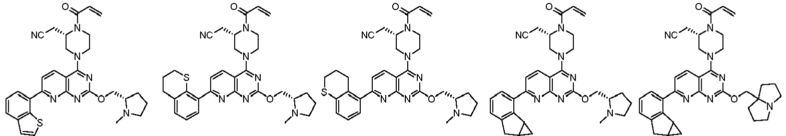

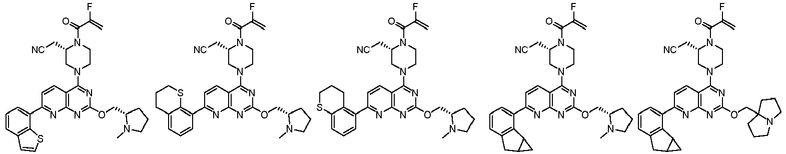

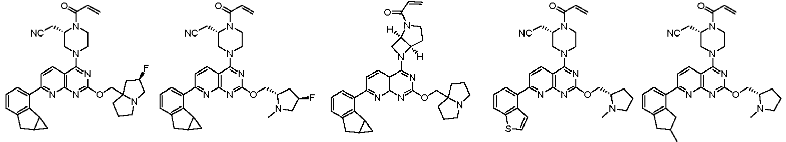

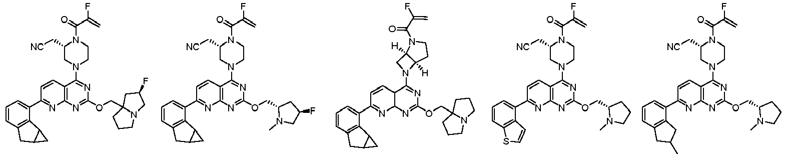

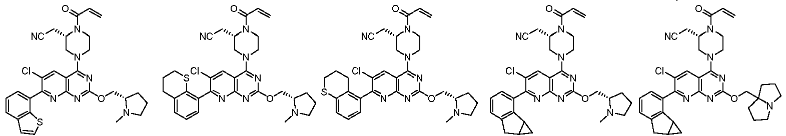

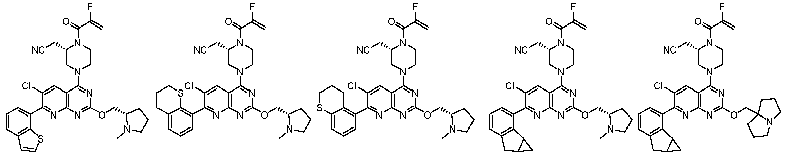

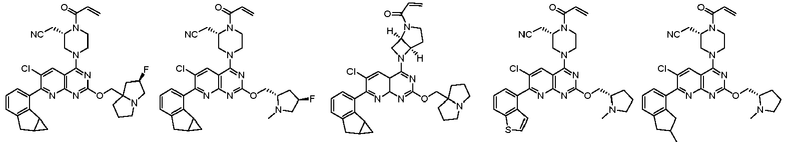

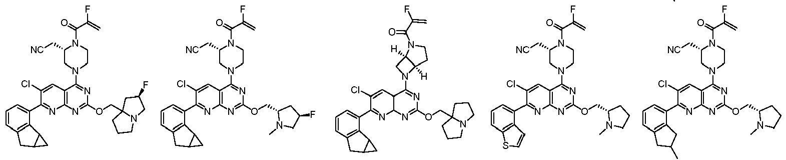

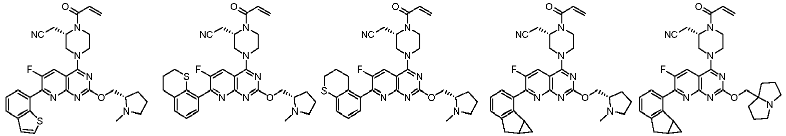

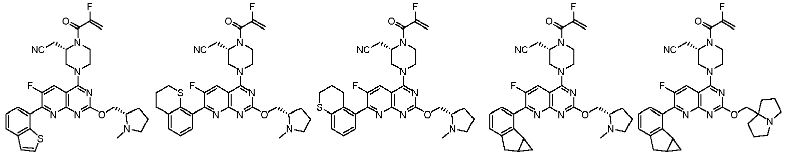

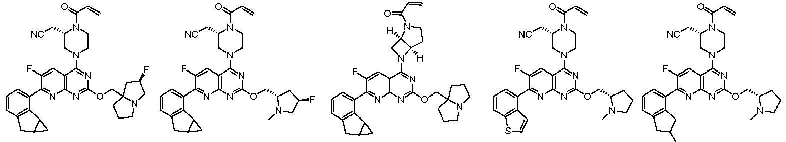

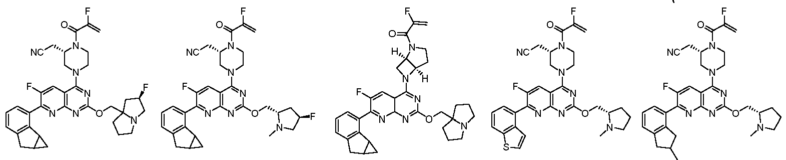

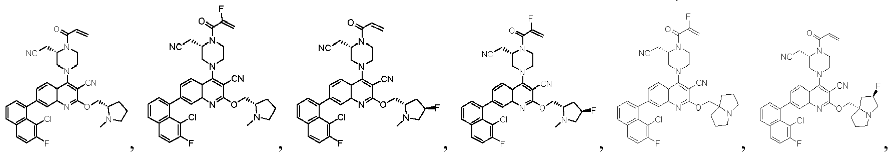

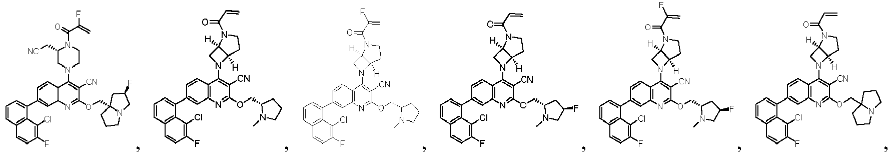

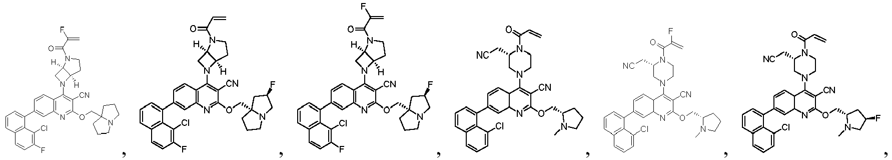

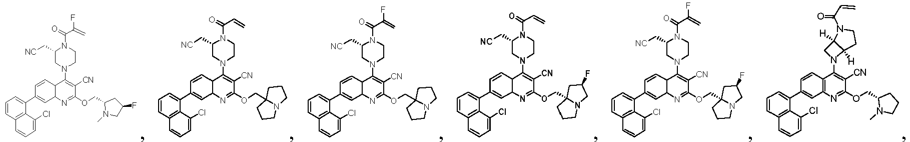

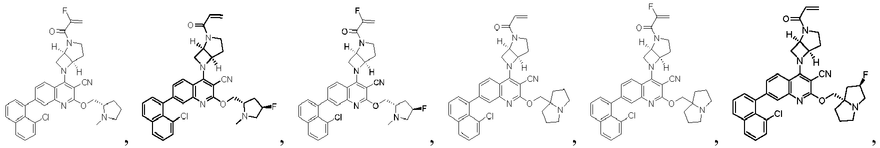

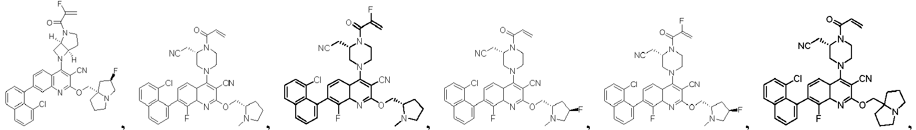

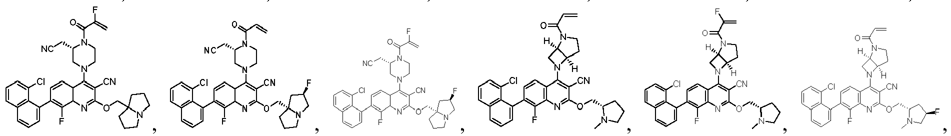

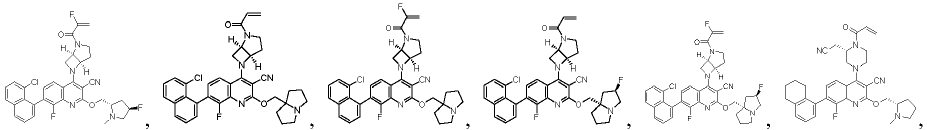

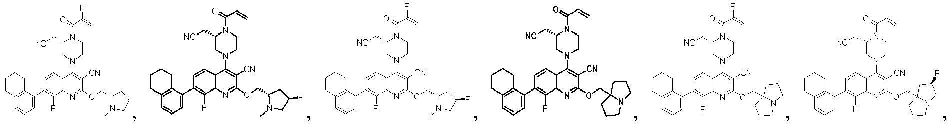

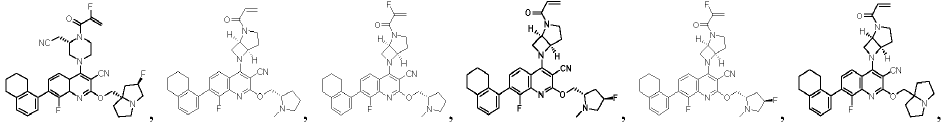

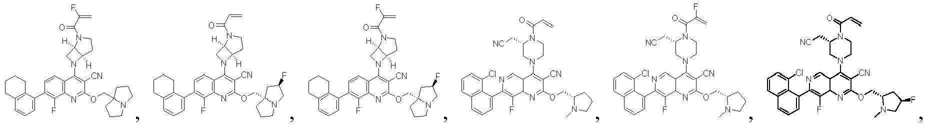

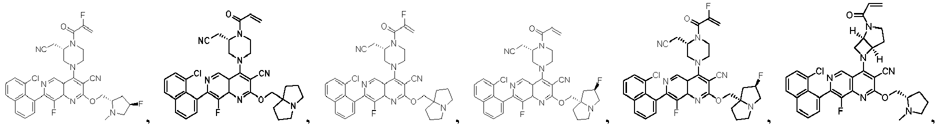

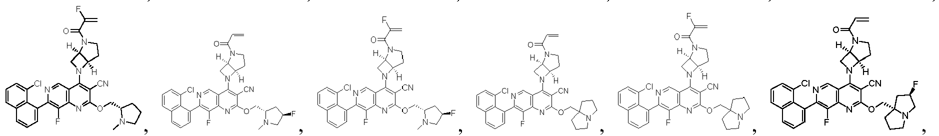

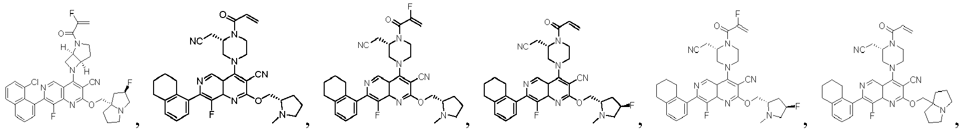

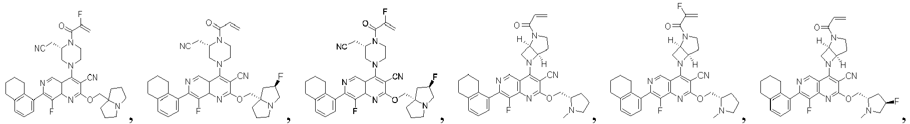

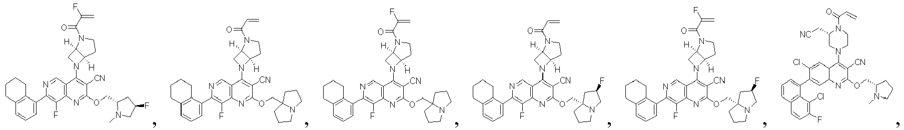

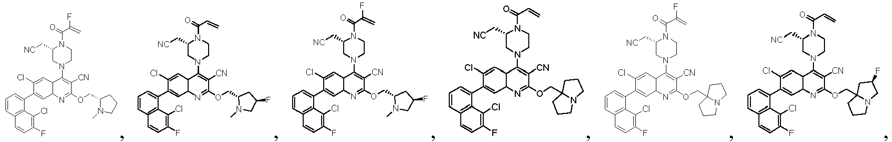

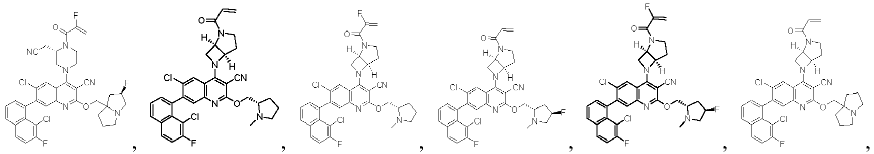

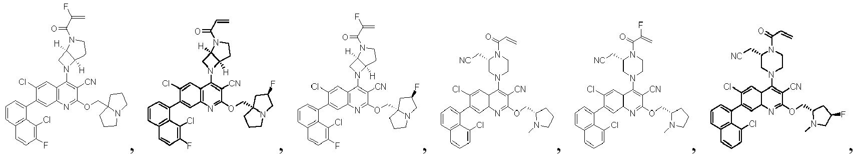

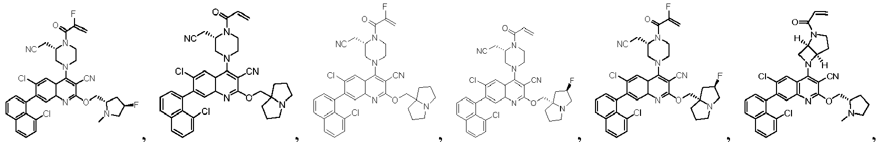

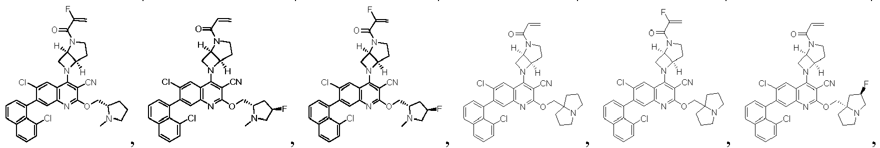

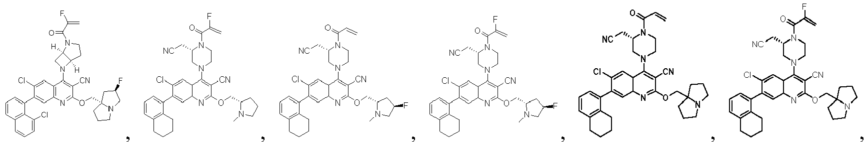

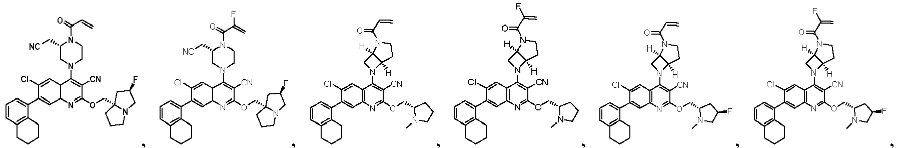

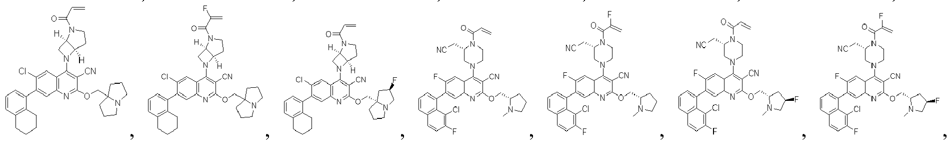

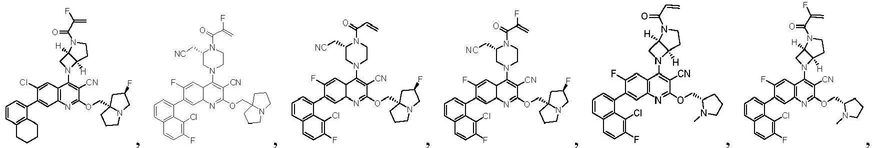

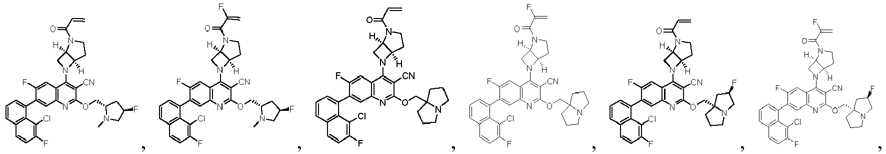

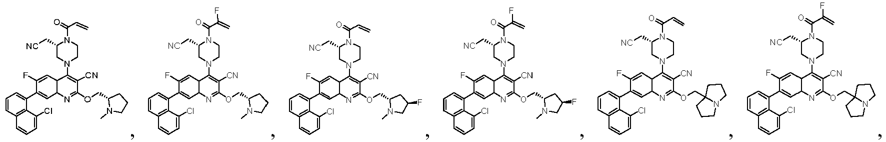

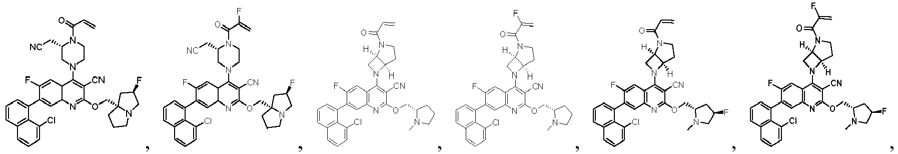

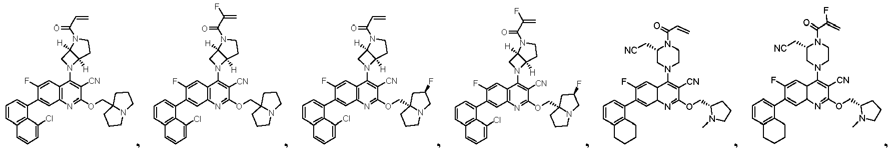

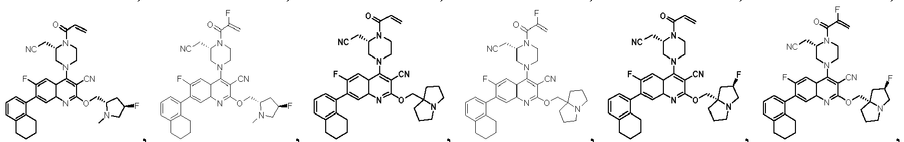

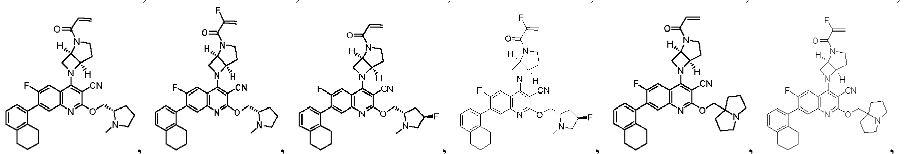

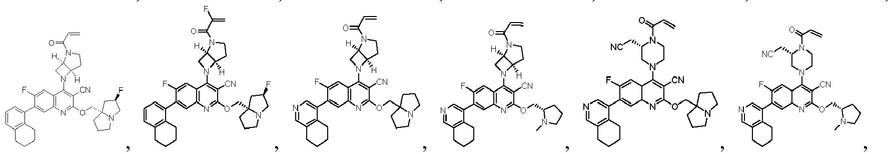

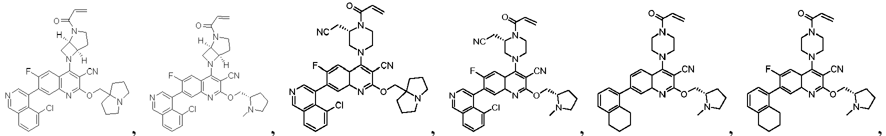

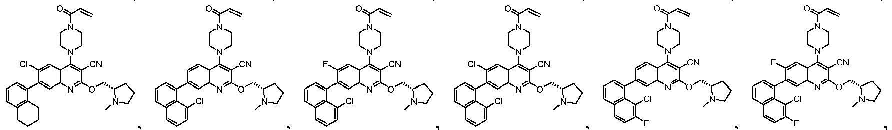

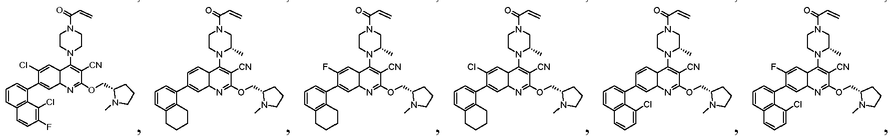

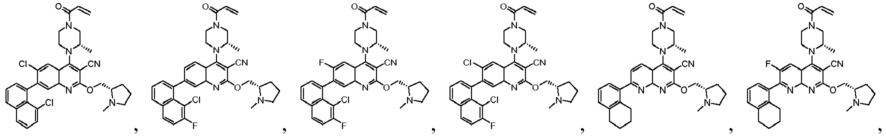

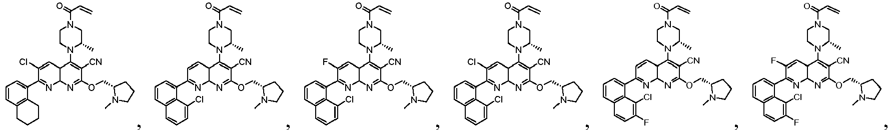

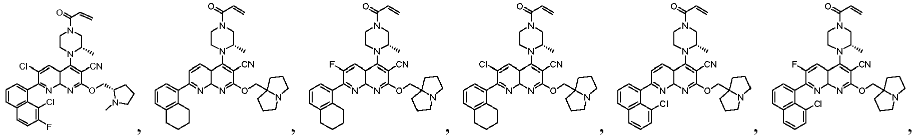

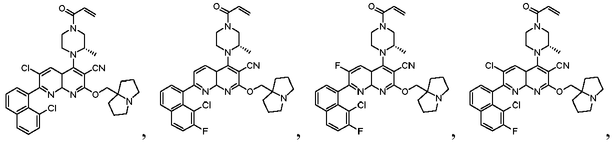

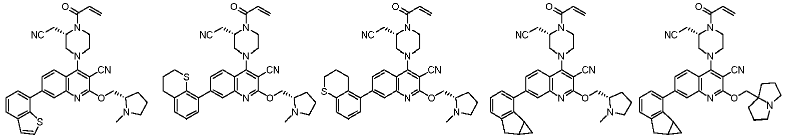

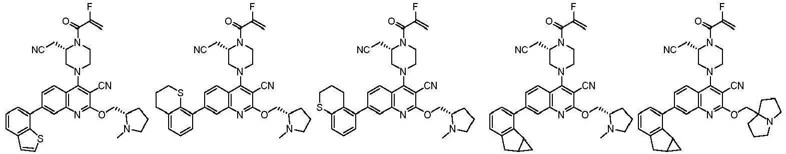

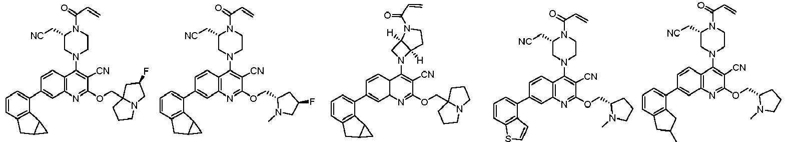

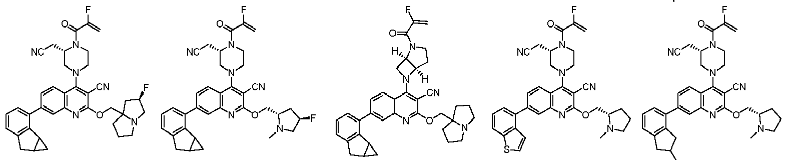

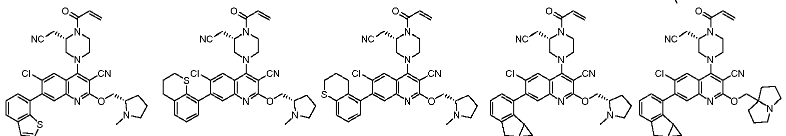

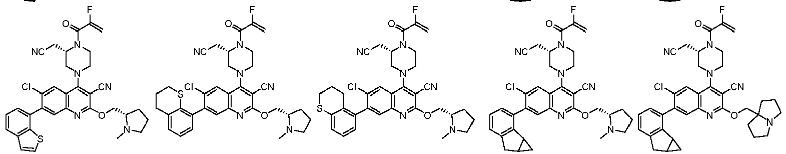

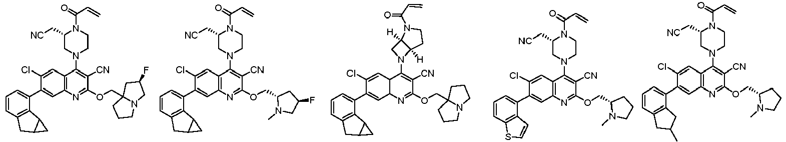

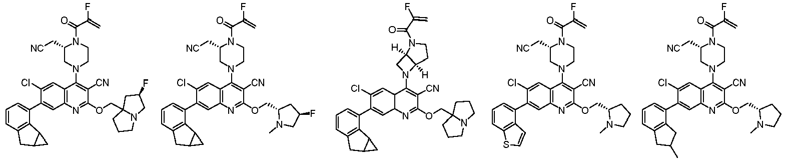

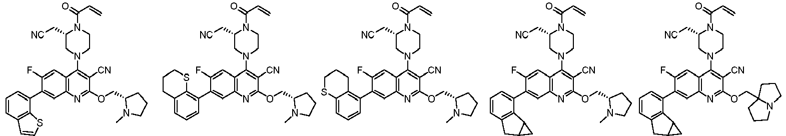

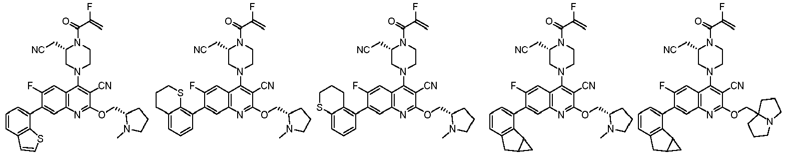

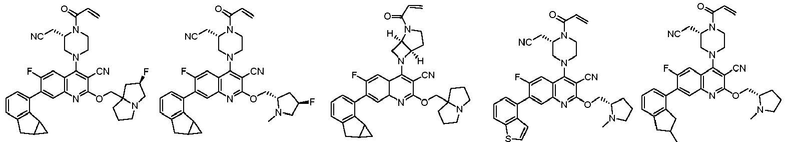

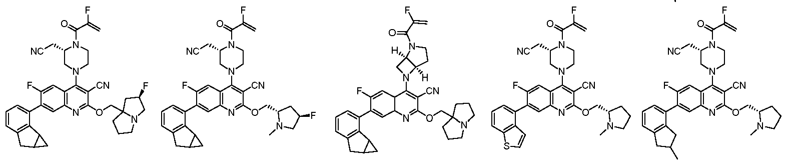

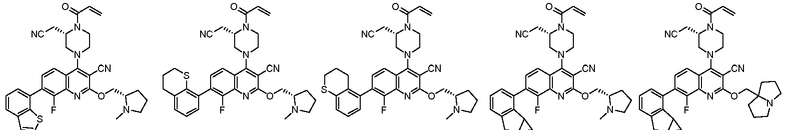

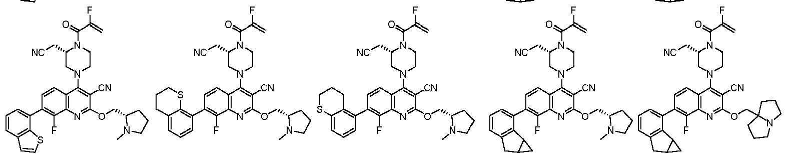

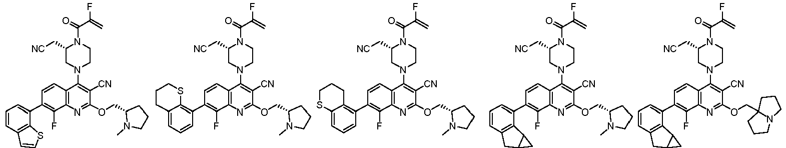

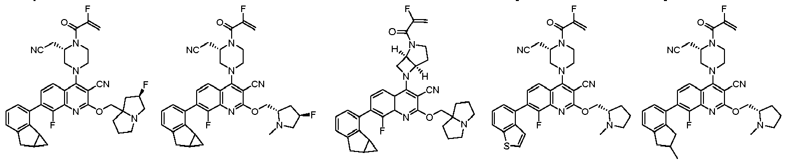

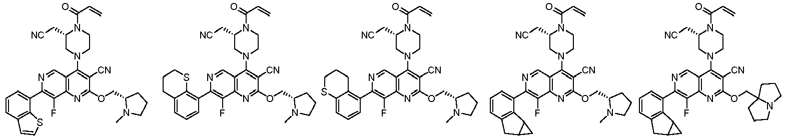

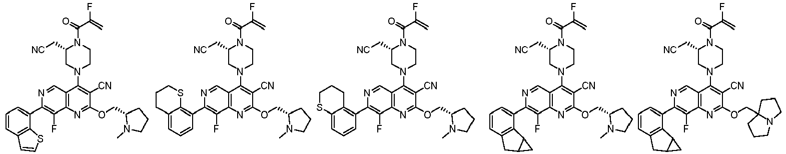

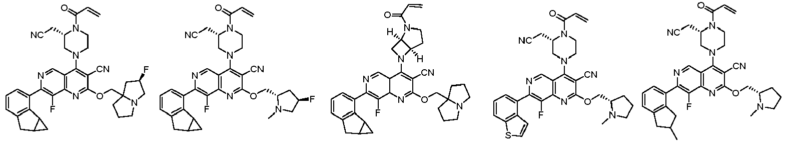

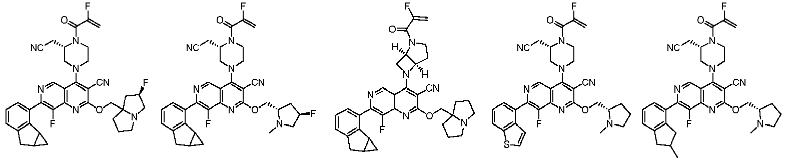

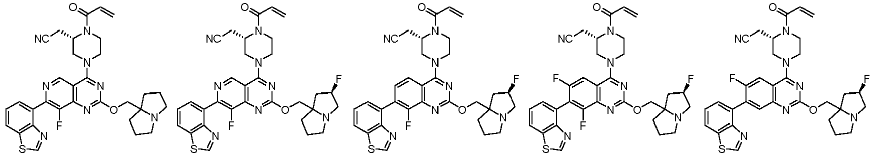

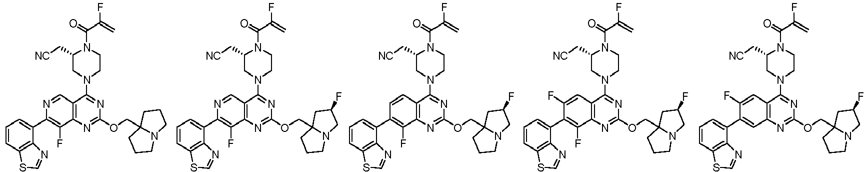

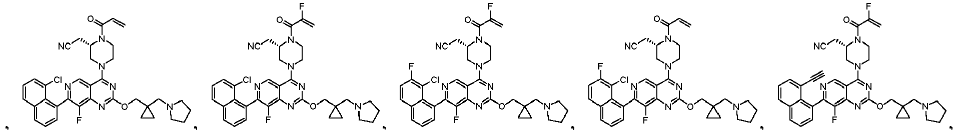

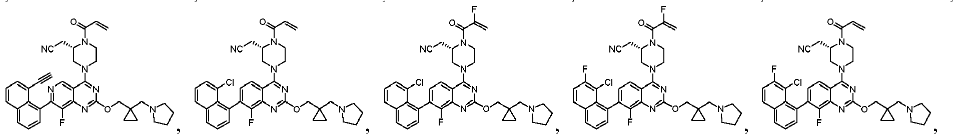

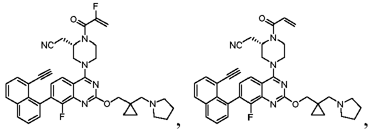

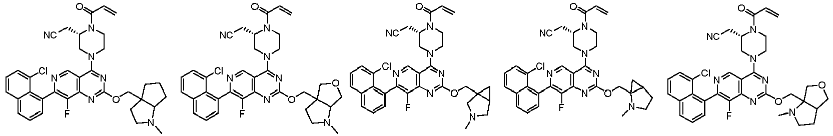

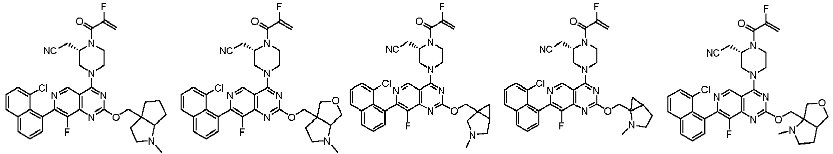

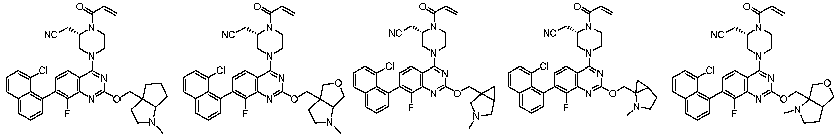

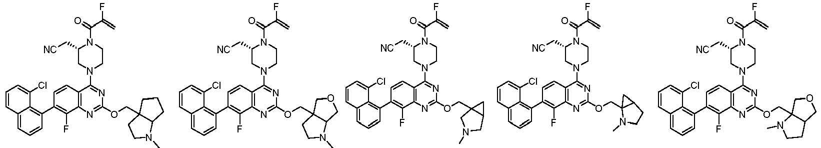

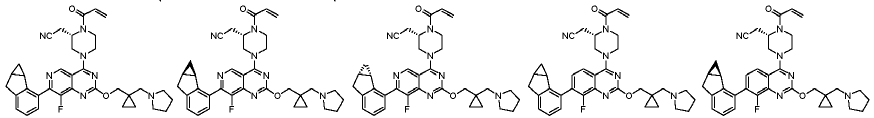

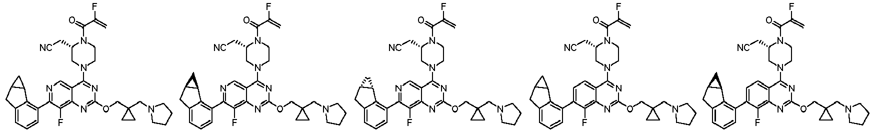

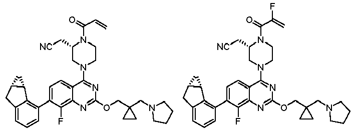

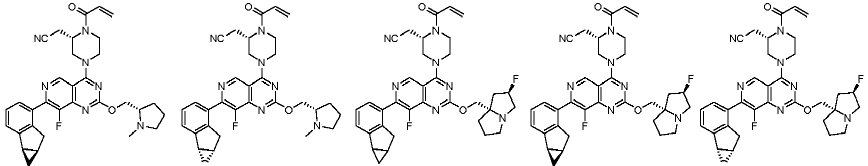

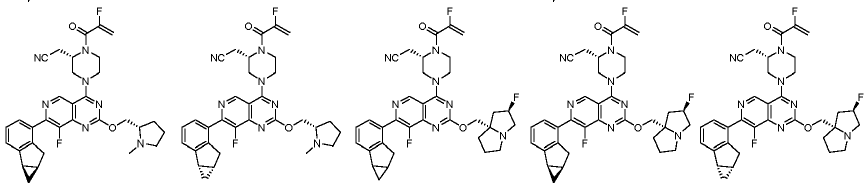

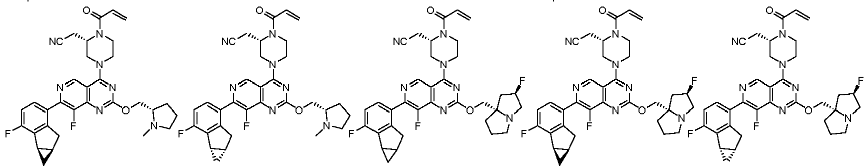

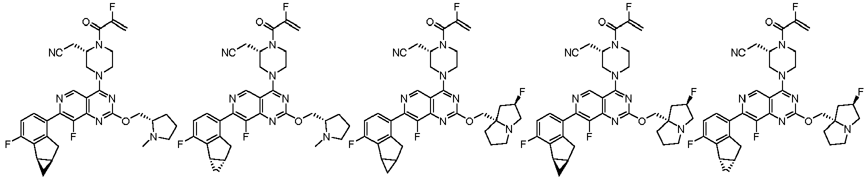

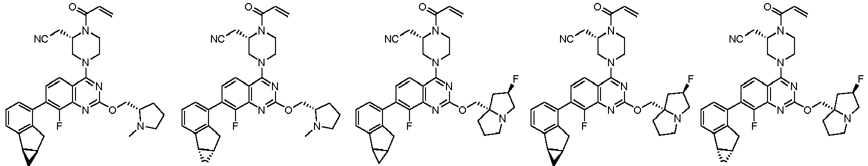

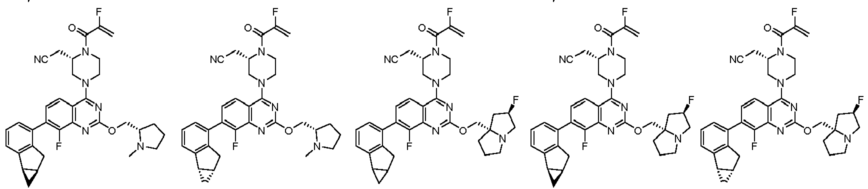

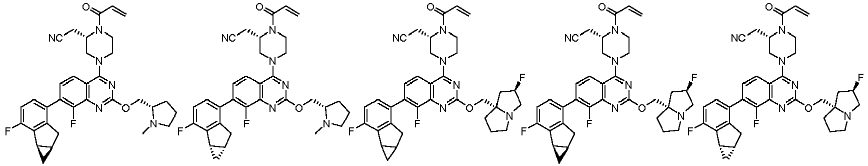

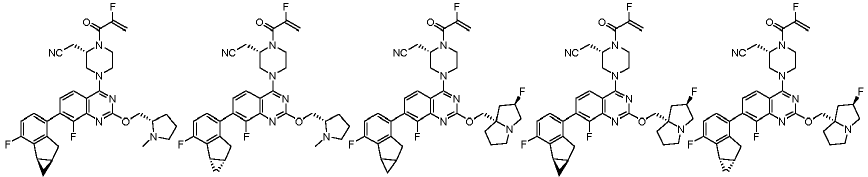

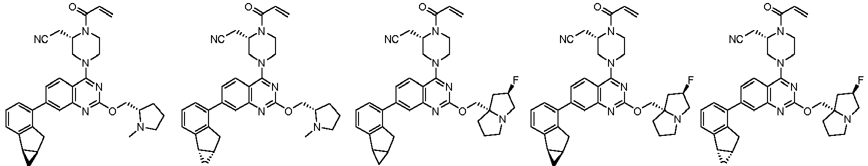

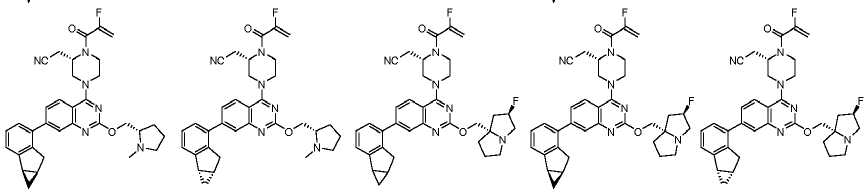

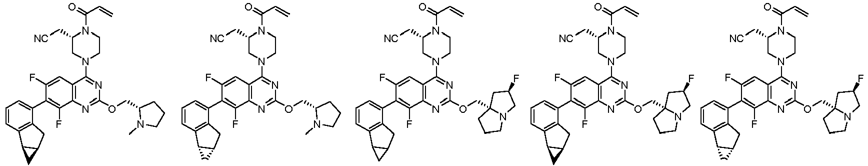

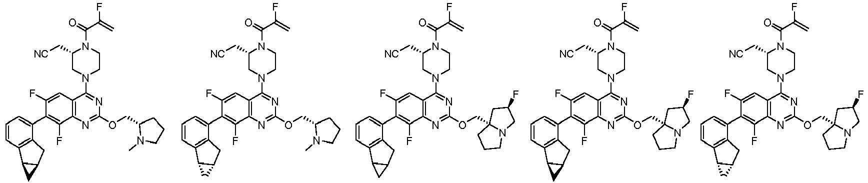

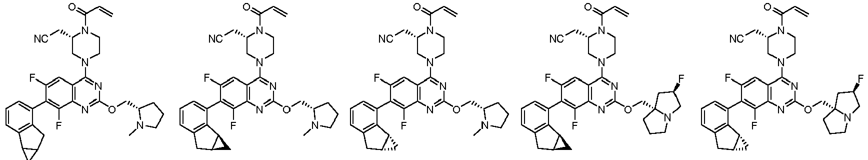

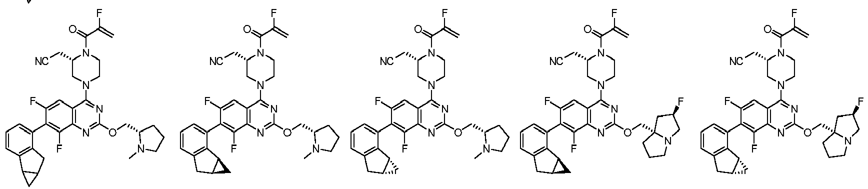

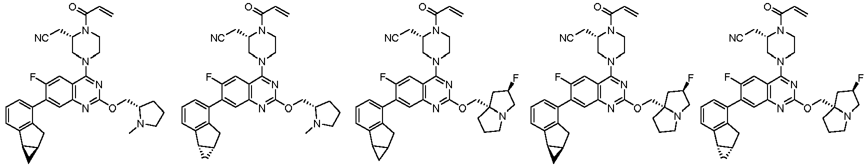

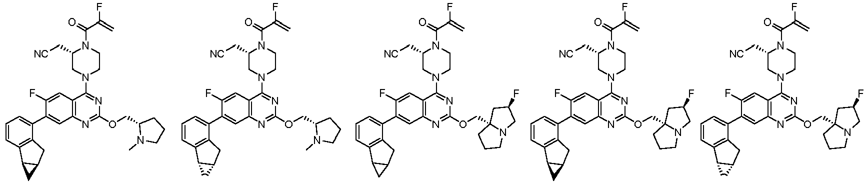

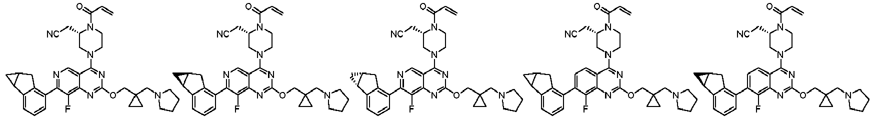

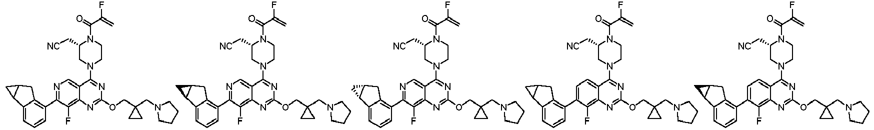

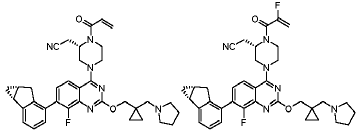

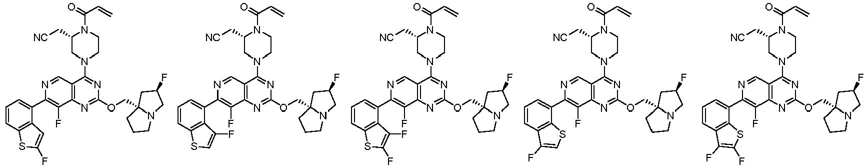

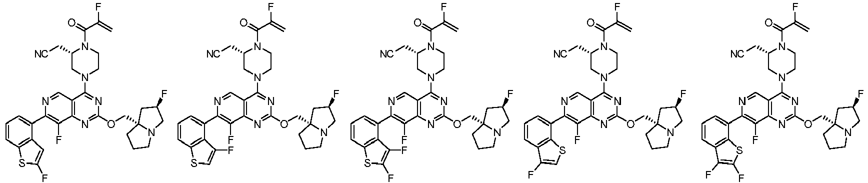

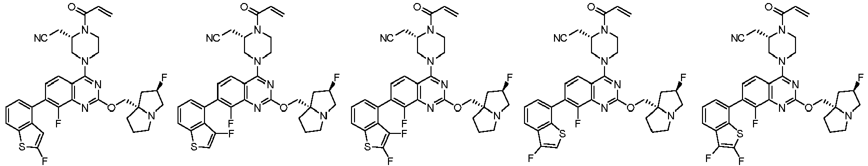

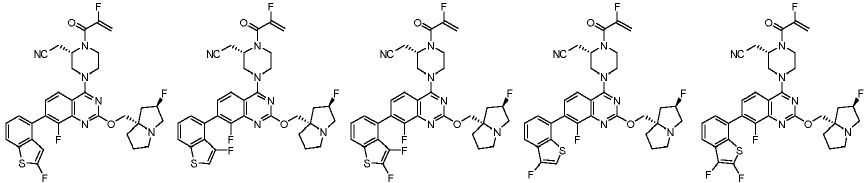

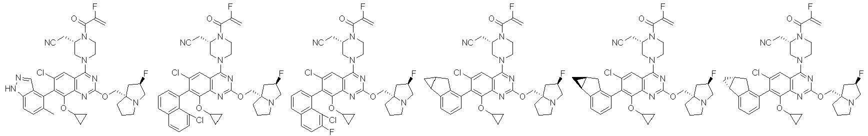

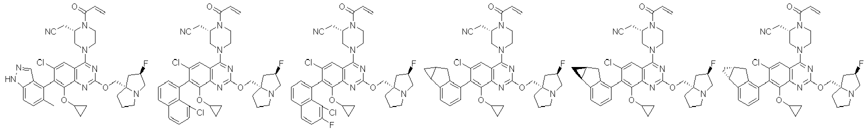

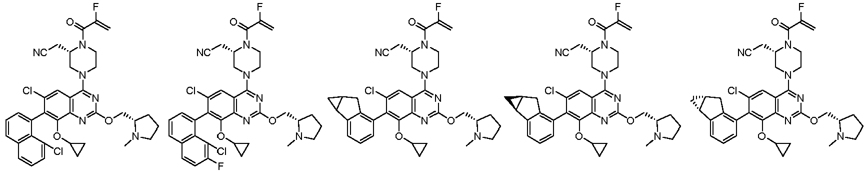

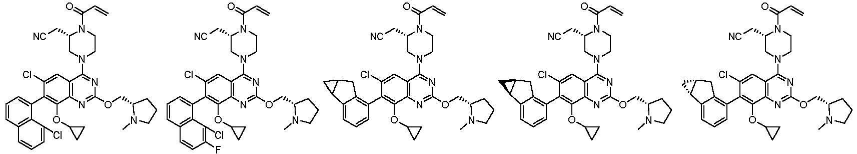

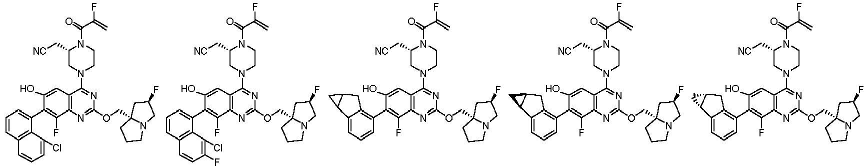

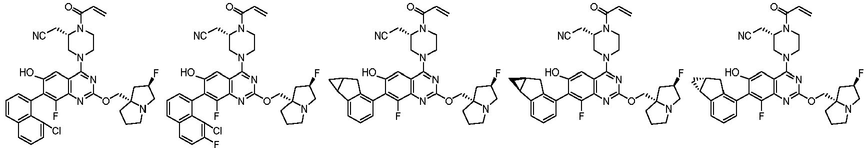

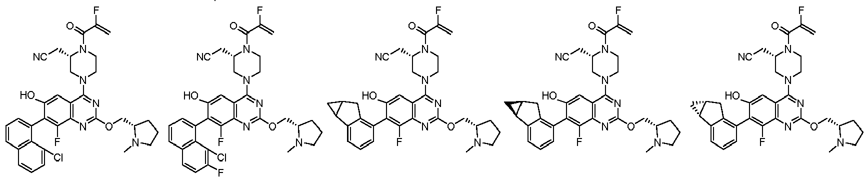

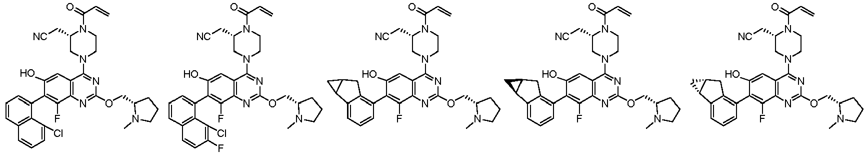

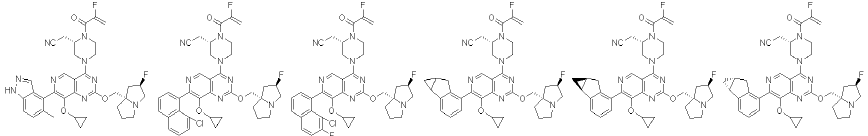

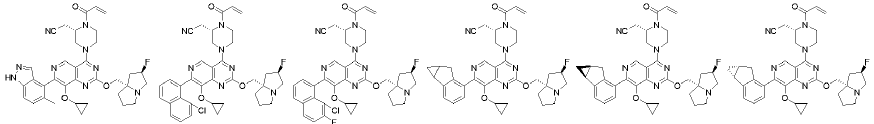

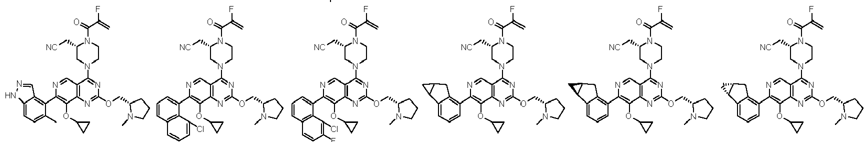

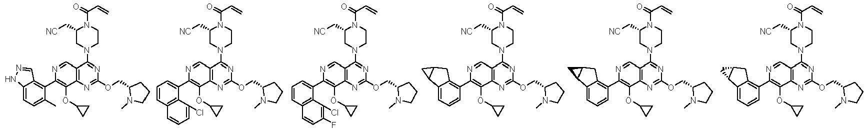

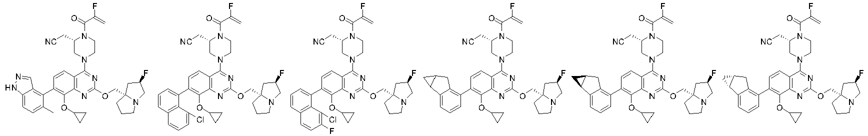

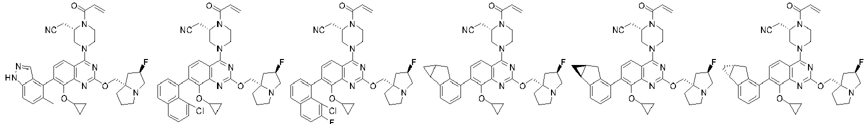

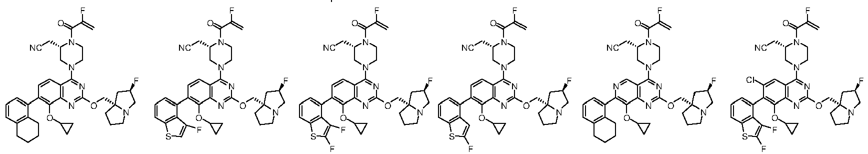

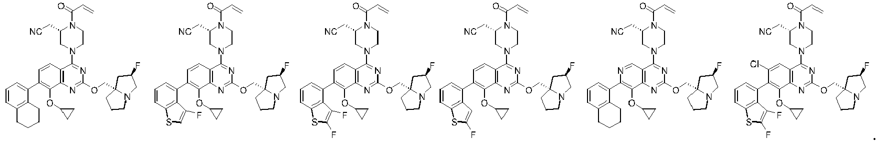

In another aspect of the present invention, there is provided an exemplary method for preparing the compound of formula (I) or a pharmaceutically acceptable salt, tautomer or stereoisomer thereof:

### Preparation method 1:

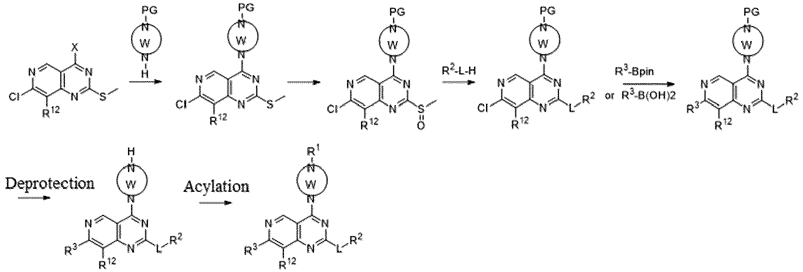

wherein R¹, R², R³, L, and W are as defined hereinbefore. PG is an amino-protecting group, such as Boc- or Cbz, and X is Cl, OTf, etc. The first step is a substitution reaction occurring under alkaline condition (such as triethylamine or diisopropylethylamine); the second step is an oxidation reaction occurring under oxidant (such as m-chloroperoxybenzoic acid) condition to obtain an intermediate sulfoxide; the third step is a substitution reaction of the intermediate sulfoxide under alkaline condition (triethylamine, sodium hydride, sodium tert-butoxide, etc.) to obtain the target intermediate; the fourth step is a Suzuki coupling reaction, whereby a coupling reaction with R³-Bpin or R³-B(OH)₂ occurs to obtain an intermediate; the fifth step is to remove the protecting group (such as BOC); and the sixth step is to react with a corresponding acid or acyl chloride to obtain the target compound.

### Preparation method 2:

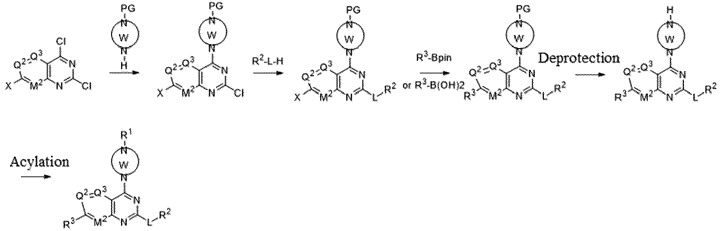

wherein R¹, R², R³, Q², Q³, M², L, and W are as defined hereinbefore. X is Cl, Br, or I. PG is an amino-protecting group, such as Boc- or Cbz. The first step is a substitution reaction occurring under alkaline condition (such as triethylamine or diisopropylethylamine); the second step is a substitution reaction occurring under alkaline condition (triethylamine, sodium hydride, sodium tert-butoxide, etc.) to obtain the target intermediate; the third step is a Suzuki coupling reaction, whereby a coupling reaction with R³-Bpin or R³-B(OH)₂ occurs to obtain an intermediate; the fourth step is to remove the protecting group (such as BOC); and the fifth step is to react with a corresponding acid or acyl chloride to obtain the target compound.

### Preparation method 3:

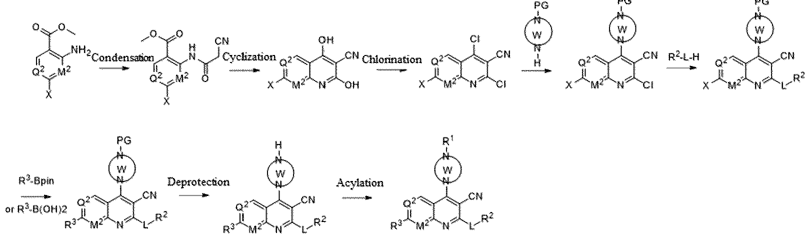

wherein R¹, R², R³, Q², M², L, and W are as defined hereinbefore. X is Cl, Br, or I. PG is an amino-protecting group, such as Boc- or Cbz. The first step is a condensation reaction occurring under condensation agent (such as EDCI, HOBT, or HATU) condition; the second step is a cyclization reaction under alkaline condition (such as sodium hydride, sodium methoxide, or sodium ethoxide); the third step is a chlorination reaction under phosphorus oxychloride condition; the fourth step is a substitution reaction occurring under alkaline condition (such as triethylamine or diisopropylethylamine); the fifth step is a substitution reaction occurring under alkaline condition (triethylamine, sodium hydride, sodium tert-butoxide, etc.) to obtain the target intermediate; the sixth step is a Suzuki coupling reaction, whereby a coupling reaction with R³-Bpin or R³-B(OH)₂ occurs to obtain an intermediate; the seventh step is to remove the protecting group (such as BOC); and the eighth step is to react with a corresponding acid or acyl chloride to obtain the target compound.

### Preparation method 4:

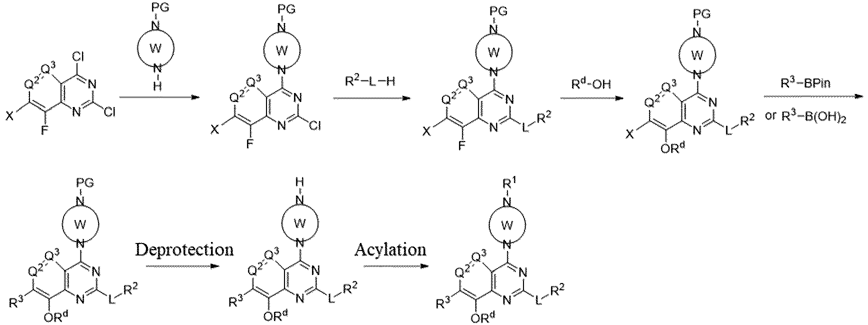

wherein R¹, R², R³, Q², Q³, R^{d}, L, and W are as defined hereinbefore. X is Cl, Br, or I. PG is an amino-protecting group, such as Boc- or Cbz. The first step is a substitution reaction occurring under alkaline condition (such as triethylamine or diisopropylethylamine); the second step is a substitution reaction occurring under alkaline condition (triethylamine, sodium hydride, sodium tert-butoxide, etc.) to obtain the target intermediate; the third step is a substitution reaction occurring under strong alkaline conditions (sodium cyanide, sodium tert-butoxide, potassium tert-butoxide, potassium hexamethyldisilazide, etc.); the fourth step is a Suzuki coupling reaction, whereby the halogenated intermediate undergoes a coupling reaction with R³-Bpin or R³-B(OH)₂ to obtain an intermediate; the fifth step is to remove the protecting group (such as BOC); and the sixth step is to react with a corresponding acid or acyl chloride to obtain the target compound.

Other general synthesis methods are provided in the examples. It would be obvious to those of ordinary skill in the art that the compound of formula (I) can be prepared according to one or more methods or in other means known in this technology. Obviously, in general, when following the general route described herein, it is necessary to use diversely substituted starting materials and/or protecting groups to obtain the desired compounds. Various substituents can also be added at different points in the synthesis route to prepare the desired compounds.

The present invention relates to a pharmaceutical composition of the compound of formula (I) or a pharmaceutically acceptable salt, prodrug and solvate thereof.

In yet another aspect of the present invention, there is provided a method of using the compound or pharmaceutical composition of the present invention to treat a disease condition, including but not limited to conditions (such as cancer) associated with G12 KRAS, HRAS or NRAS mutations. The cancer is pancreatic cancer, lung cancer, colorectal cancer, etc., which are mediated by G12C mutation.

The present invention relates to a compound of formula (I), which has good physical and chemical properties and safety and toxicity parameters and can be used for the treatment of cancer and inflammation in a mammal.

In other examples, a method for inhibiting the proliferation of a cell population is further provided, which comprises bringing the cell population into contact with any one of the compounds with structure (I).

Other embodiments relate to a pharmaceutical composition. The pharmaceutical composition comprises any one (or more) of the aforementioned compounds and a pharmaceutically acceptable carrier. In some embodiments, the pharmaceutical composition is formulated for oral administration. In other embodiments, the pharmaceutical composition is formulated for injection. In more embodiments, the pharmaceutical composition comprises the compound disclosed herein and another therapeutic agent (e.g., an anticancer agent). Non-limiting examples of such therapeutic agents are described hereinafter.

Suitable routes of administration include but are not limited to oral, intravenous, rectal, aerosol, parenteral, ocular, pulmonary, mucosal, percutaneous, vaginal, auricular, nasal and local administrations. In addition, by way of example only, parenteral delivery includes intramuscular, subcutaneous, intravenous, and intramedullary injections, as well as intrathecal, direct intraventricular, intraperitoneal, endolymphangial and intranasal injections.

### Detailed Description of Embodiments

Unless otherwise indicated, the entire disclosure of the present invention is defined by the following terms:
The term "prodrug" refers to any derivative that can be converted into the corresponding active pharmaceutical compound in an organism. The prodrug of the compound described herein can easily undergo chemical changes under physiological conditions and is thus transformed into the compound of the present invention. In addition, the prodrug can be converted into the compound of the present invention *in vivo* by a chemical or biochemical method.
Unless otherwise specified, the term "pharmaceutically acceptable salt" includes salts of acidic groups (e.g., but not limited to, potassium salt, sodium salt, magnesium salt, calcium salt, etc.) or salts of basic groups (e.g., but not limited to, formate, acetate, citrate, tartrate, methanesulfonate, malate or sulfate, hydrochloride, phosphate, nitrate, and carbonate) that can be present in the compound of the present invention.
The term "solvate" refers to a complex molecular compound formed by solute molecules or ions attracting adjacent solvent molecules via intermolecular forces such as Coulomb force, van der Waals force, charge transfer force and hydrogen bond in a solution. In one embodiment, the solvent is water, that is, the compound of the present invention forms a hydrate.

The compound of the present invention or the pharmaceutically acceptable salt thereof may contain one or more asymmetric centers, and can thus produce enantiomers, diastereomers and other stereoisomeric forms. As for the absolute stereochemical configuration of amino acids, it is defined as (R)- or (S)-configuration or as (D)- or (L)-configuration. The present invention is intended to include all such possible isomers, as well as racemic and optically pure forms thereof. Optically active (+) and (-), (R)- and (S)-, or (D)- and (L)-isomers can be obtained by chiral synthesis or chiral preparation, or by resolution using conventional techniques such as chromatography and fractional crystallization. Conventional techniques for preparing/separating individual enantiomers include chiral synthesis from suitable optically pure precursors and resolution of racemates (or racemates of salts or derivatives) using, for example, chiral high-pressure liquid chromatography (HPLC). The present invention provides pure isomers and isomer mixtures, a preparation method therefor, the use thereof, and compositions comprising same. For the sake of simplicity, it will be referred to as the compound of formula (I) hereinafter, which refers to both pure optical isomers and, if appropriate, mixtures of isomers at various ratios.

The compound of the present invention may be present in a specific. Unless otherwise specified, the term "tautomer" or "tautomeric form" means that at room temperature, isomers of different functional groups are in dynamic equilibrium and can quickly transform into each other. If tautomers are possible (such as in a solution), the chemical equilibrium of tautomers can be achieved. For example, proton tautomers (also referred to as prototropic tautomers) include mutual transformation by proton migration, such as keto-enol isomerization and imine-enamine isomerization. Valencetautomers include mutual transformation by recombination of some bonding electrons.

The alkyl, alkenyl, alkynyl, and cycloalkyl moieties can be each independently optionally substituted with one or more groups selected from hydroxyl, oxo, halogen, cyano, nitro, trifluoromethyl, azido, amino, carboxyl, and mercapto.

Saturated or unsaturated hydrocarbon groups, such as alkyl, alkanediyl or alkenyl, including those bonded with heteroatoms, such as alkoxy, can all be individually linear or branched.

The term "optional" or "optionally" means that the subsequently described event or condition possibly, but not necessarily, occurs, and the description includes the case where the event or condition occurs and the case where the event or condition does not occur.

The term "substituted" means that any one or more hydrogen atoms on a specific atom are replaced with a substituent, which may include heavy hydrogen and hydrogen variants, as long as the valence state of the specific atom is normal and the substituted compound is stable. When the substituent is oxygen (i.e., =O), it is meant that two hydrogen atoms are replaced. Substitution with oxygen does not occur on aromatic groups. The term "optionally substituted" refers to either substituted or unsubstituted. Unless otherwise specified, the type and number of substituents may be arbitrary on the basis of being achievable in chemistry.

When any variable (such as R) appears more than once in the composition or structure of a compound, the definition thereoof in each case is independent. Therefore, for example, if one group is substituted with 0-2 **R**, the group can be optionally substituted with at most two R, and R in each case has an independent option. In addition, a combination of substituents and/or variants thereof is allowed only if such a combination produces a stable compound.

When one variable is selected from a single bond, it means that the two groups connected thereto are directly connected. For example, when L in Ar-L-R represents a single bond, it means that the structure is actually **Ar-R**. When a substituent is vacant, it means that the substituent does not exist. For example, when L is vacant in Ar-L-R, Ar-L-R means that the structure is actually Ar.

Unless otherwise specified, the term "hetero" means a heteroatom or a heteroatom group (i.e., a heteroatom-containing radical), including atoms other than carbon (C) and hydrogen (H) and radicals containing these heteroatoms, such as including oxygen (O), nitrogen (N), sulfur (S), silicon (Si), germanium (Ge), aluminium (Al), boron (B), -O-, -S-, -C(=O)O-, -C(=O)-, -C(=S)-, -S(=O), and -S(=O)2-, as well as optionally substituted -C(=O)N(H)-, -N(H)-, -C(=NH)-, -S(=O)2N(H)-, or -S(=O)N(H)-.

Unless otherwise specified, the term "ring" means substituted or unsubstituted cycloalkyl, heterocycloalkyl, cycloalkenyl, heterocycloalkenyl, cycloalkynyl, heterocycloalkynyl, aryl, or heteroaryl. The ring includes both monocyclic rings and bicyclic or polycyclic systems such as spiro, fused and bridged cyclic rings. The number of atoms on a ring is usually defined as the number of the members of the ring. For example, a "5-7-membered ring" refers to 5-7 atoms arranged in a circle. Unless otherwise specified, the ring optionally contains 1-3 heteroatoms. Therefore, the term "5-7-membered ring" includes, for example, phenyl, pyridinyl, and piperidinyl; on the other hand, the term "5-7-membered heterocycloalkyl" includes pyridinyl and piperidinyl, but does not include phenyl. The term "ring" also includes a ring system containing at least one ring, wherein each "ring" independently conforms to the above definition.

Unless otherwise specified, the term "heteroalkyl", by itself or in combination with another term, represents a stable linear or branched alkyl radical or its composition which consists of a certain number of carbon atoms and at least one heteroatom or heteroatom radical. In some embodiments, the heteroatom is selected from B, O, N, and S, wherein the nitrogen and sulfur atoms are optionally oxidized and the nitrogen heteroatom is optionally quaternized. In other embodiments, the heteroatom radical is selected from -C(=O)O-, -C(=O)-, -C(=S)-, -S(=O), -S(=O)2-, -C(=O)N(H)-, -N(H)-, -C(=NH)-, -S(=O)2N(H)-, and -S(=O)N(H)-. In some embodiments, the heteroalkyl is C₁-C₆ heteroalkyl; and in other embodiments, the heteroalkyl is C₁-C₃ heteroalkyl. The heteroatom or heteroatom radical can be located in any internal position of the heteroalkyl, including the position at which the alkyl is connected to the remainder of the molecule, but the terms "alkoxy", "alkylamino" and "alkylthio" (or thioalkoxy) are customary expressions and refer to alkyl groups that are connected to the remainder of the molecule via an oxygen atom, amino, or a sulfur atom, respectively. Examples of heteroalkyl include, but are not limited to, -OCH₃, -OCH₂CH₃, -OCH₂CH₂CH₃, -OCH₂(CH₃)₂, -CH₂-CH₂-O-CH₃, -NHCH₃, -N(CH₃)₂, -NHCH₂CH₃, -N(CH₃)(CH₂CH₃), - CH₂-CH₂-NH-CH₃, -CH₂-CH₂-N(CH₃)-CH₃, -SCH₃, -SCH₂CH₃, -SCH₂CH₂CH₃, -SCH₂(CH₃)₂, -CH₂-SCH₂-CH₃, -CH₂-CH₂, -S(=O)-CH₃, -CH₂-CH₂-S(=O)₂-CH₃, -CH=CH-O-CH₃, -CH₂-CH=N-OCH₃, and - CH=CHNCCH₃)-CH₃. At most two heteroatoms can be continuous, e.g., in -CH₂-NH-OCH₃.

Unless otherwise specified, the term "heterocycloalkyl", respectively by itself or in combination with other terms, means cyclized "heteroalkyl", which includes monocyclic, bicyclic and tricyclic systems, wherein the bicyclic and tricyclic systems include spiro, bicyclic and bridged cyclic rings. In addition, in terms of "heterocycloalkyl", the heteroatom can occupy the position at which the heterocycloalkyl is connected to the remainder of the molecule. In some embodiments, the heterocycloalkyl is 4- to 6-membered heterocycloalkyl; and in other embodiments, the heterocycloalkyl is 5- to 6-membered heterocycloalkyl. Examples of heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolyl, isothiazolyl, 1,2-oxazinyl, 1,2-thiazinyl, hexahydropyridazinyl, homopiperazinyl, homopiperidinyl, or oxepanyl.

The term "alkoxy" represents the above-mentioned alkyl with a specific number of carbon atoms connected by an oxygen bridge, and unless otherwise specified, C₁-C₆ alkoxy includes C₁, C₂, C₃, C₄, C₅, and C₆ alkoxy. In some embodiments, the alkoxy is C₁-C₃ alkoxy. Examples of alkoxy include, but are not limited to, methoxy, ethoxy, n-propoxy, isopropoxy, n-butoxy, sec-butoxy, tert-butoxy, n-pentoxy, and pentoxy.

Unless otherwise specified, the term "aryl" in the present invention represents a polyunsaturated carbocyclic system, which can be a monocyclic, bicyclic or polycyclic system, in which at least one ring is aromatic, and the rings in the bicyclic and polycyclic systems are fused together. It may be monosubstituted or polysubstituted, and may be monovalent, divalent or multivalent. In some embodiments, the aryl is C₆-C₁₂ aryl; and in other embodiments, the aryl is C₆-C₁₀ aryl. Examples of aryl include, but are not limited to, phenyl and naphthyl (including 1-naphthyl, 2-naphthyl, etc.). The substituents of any of the above aryl ring systems are selected from the acceptable substituents described in the present invention.

Unless otherwise specified, the term "heteroaryl" in the present invention refers to aryl containing 1, 2, 3, or 4 heteroatoms independently selected from B, N, O, and S, which may be a monocyclic, bicyclic or tricyclic system, in which the nitrogen atom may be substituted or unsubstituted (i.e., N or NR, where R is H or other substituents as defined herein), and optionally quaternized, and the nitrogen and sulfur heteroatoms may be optionally oxidized (i.e., NO and S(O)p, wherein p is 1 or 2). The heteroaryl group can be attached to the remainder of the molecule via a heteroatom. In some embodiments, the heteroaryl is 5- to 10-membered heteroaryl; and in other embodiments, the heteroaryl is 5- to 6-membered heteroaryl. Examples of the heteroaryl include, but are not limited to, pyrrolyl (including pyrrolyl, 2-pyrrolyl, 3-pyrrolyl, etc.), pyrazolyl (including 2-pyrazolyl, 3-pyrazolyl, etc.), imidazolyl (including imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H-1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), benzothiazolyl (including 5-benzothiazolyl, etc.), purinyl, benzimidazolyl (including 2-benzimidazolyl, etc.), indolyl (including 5-indolyl, etc.), isoquinolyl (including 1-isoquinolyl, 5-isoquinolyl, etc.), quinoxalinyl (including 2-quinoxalinyl, 5-quinoxalinyl, etc.), quinolyl (including 3-quinolyl, 6-quinolyl, etc.), pyrazinyl, purinyl, and benzoxazolyl. The substituents of any of the above heteroaryl ring systems are selected from the acceptable substituents described in the present invention.

### Synthesis

All suitable solvents commonly used in organic reactions can be used in the following steps of the preparation method of the present invention, e.g., but not limited to, aliphatic and aromatic, optional hydrocarbons or halogenated hydrocarbons (e.g., pentane, hexane, heptane, cyclohexane, petroleum ether, gasoline, volatile oils, benzene, toluene, xylene, dichloromethane, dichloroethane, chloroform, carbon tetrachloride, chlorobenzene and o-dichlorobenzene), aliphatic and aromatic, optional alcohols (e.g., methanol, ethanol, propanol, isopropanol, tert-butanol, and ethylene glycol), ethers (e.g., diethyl ether, dibutyl ether, ethylene glycol dimethyl ether, diethylene glycol dimethyl ether, tetrahydrofuran and dioxane), esters (e.g., methyl acetate or ethyl acetate), nitriles (e.g., acetonitrile or propionitrile), ketones (e.g., acetone and butanone), amides (e.g., dimethylformamide, dimethylacetamide, and N-methylpyrrolidone), dimethyl sulfoxide, tetramethylene sulfone, hexamethylphosphoryl triamine, N,N-dimethylpropylene urea (DMPU), etc.

The following abbreviations are used in the present invention: DCM stands for dichloromethane; CHCl₃ stands for trichloromethane; EA stands for ethyl acetate; THF stands for tetrahydrofuran; MeCN stands for acetonitrile; MeOH stands for methanol; EtOH stands for ethanol; i-PrOH stands for isopropanol; PE stands for petroleum ether; toulene stands for methylbenzene; DMSO stands for dimethyl sulfoxide; DMF stands for N,N-dimethylformamide; DMA stands for N,N-dimethylacetamide; CDCl₃ stands for deuterated chloroform; D₂O stands for heavy water; (CD₃)₂SO stands for deuterated DMSO; CD₃OD stands for deuterated methanol; CuI stands for cuprous iodide; DIPEA stands for diisopropylethylamine; TEA stands for triethylamine; K₂CO₃ stands for potassium carbonate; Cs₂CO₃ stands for cesium carbonate; Na₂CO₃ stands for sodium carbonate; NaHCO₃ stands for sodium bicarbonate; NaOH stands for sodium hydroxide; KOH stands for potassium hydroxide; LiHMDS stands for potassium hexamethyldisilazide; CDI stands for 1,1'-carbonyl imidazole; MS stands for mass spectrometry; NMR stands for nuclear magnetic resonance; TFA stands for trifluoroacetic acid; BINAP stands for (2R,3S)-2,2'-diphenylphosphine-1,1'-binaphthyl; BOC stands for tert-butoxycarbonyl; Cbz stands for benzyloxycarbonyl; DBU stands for bicyclo-1,5-diaza-5-undecene; DCC stands for 1,3-dicyclohexylcarbodiimide; DCE stands for 1,2-dichloroethane; DMAP stands for 4-dimethylaminopyridine; dppf stands for bis(diphenylphosphino)ferrocene; LiAlH4 stands for lithium aluminium hydride; LDA stands for lithium diisopropylamide; m-CPBA stands for m-chloroperoxybenzoic acid; MTM stands for dimethyl sulfide; NBS stands for N-bromosuccinimide; NCS stands for N-chlorosuccinimide; NIS stands for N-iodosuccinimide; PCC stands for pyridinium dichromate; TBAF stands for tetrabutylamine fluoride; THP stands for tetrahydropyranyl; TMEDA stands for tetramethylethylene diamine; TMS stands for trimethylsilyl;TMP stands for 2,2,6,6-tetramethylpiperidine;Ts stands for p-toluenesulfonyl; Pd(PPh₃)₄ stands for tetrakis(triphenylphosphine)palladium; PdCl₂(dppf) stands for 1,1'-bis(diphenylphosphino)ferrocene palladium(II) dichloride; Pd₂(dba)₃ stands for tris(dibenzylideneacetone)dipalladium; HOBT stands for 1-hydroxybenzotriazole; HATU stands for 2-(7-oxidobenzotriazole)-N,N,N',N'-tetramethyluronium hexafluorophosphate; TBTU stands for O-benzotriazole-N,N,N',N'-tetramethyluronium tetrafluoroborate; Tf₂O stands for trifluoroacetic anhydride; Pd(OAc)₂ stands for palladium diacetate; RuPhos stands for 2-dicyclohexylphosphino-2',6'-diisopropoxy-1,1'-biphenyl; Pd(PPh₃)₂Cl₂ stands for bis(triphenylphosphine)palladium(II) dichloride; Sphos stands for 3,2-dicyclohexylphosphino-2',6'-dimethoxybiphenyl; XantPhos stands for 4,5-bis(diphenylphosphino)-9,9-dimethylxanthene; MeONa stands for sodium methoxide; n-BuLi stands for n-butyl lithium; t-BuONa stands for sodium tert-butoxide; t-BuOK stands for potassium tert-butoxide; KSCN stands for potassium thiocyanate; CuBr stands for cuprous bromide; NaNO₂ stands for sodium nitrite; urea stands for carbamide; POCl₃ stands for phosphorus oxychloride; BBr₃ stands for boron tribromide; NH₄Cl stands for ammonium chloride; MeI stands for iodomethane; NMP stands for N-methylpyrrolidone; K₃PO₄ stands for potassium phosphate; column chromatography stands for column chromatography separation; Ac stands for acetyl; Bn stands for benzyl; Fmoc stands for fluorenylmethyloxycarbonyl; Cy stands for cyclohexyl; Tf stands for trifluoromethylsulfonyl; and PDC stands for pyridine dichromate.

### Synthesis Examples:

### Preparation of intermediate:

### Synthesis of 4,4,5,5-tetramethyl-2-(5,6,7,8-tetrahydronaphthalen-1-yl)-1,3,2-dioxaborane

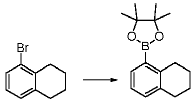

The compound 5-bromo-1,2,3,4-tetralin (5.00 g, 23.69 mmol) and 4,4,4',4',5,5,5',5'-octamethyl-2,2'-bis(1,3,2-dioxaborane) (12.03 g, 47.37 mmol) was dissolved in anhydrous 1,4-dioxane (50 mL), potassium acetate (6.97 g, 71.07 mmol) and Pd(dppf)Cl₂ (1.73 g, 2.37 mmol) were added, and after displacement with nitrogen, the mixture was heated to 100°C and reacted under stirring for 16 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by column chromatography to obtain a light yellow oil. (5.0 g, yield: 82%). ¹H NMR (400MHz, CDCl₃) δ 7.58 (d,J = 6.9Hz, 1H), 7.11 (s, 1H), 7.08 (d,J = 7.3Hz, 1H), 3.03 (t,J = 5.9Hz, 2H), 2.77 (t,J = 5.8Hz, 2H), 1.78 (dd,J = 7.1, 4.3Hz, 4H), 1.34 (s, 12H).

### Synthesis of intermediate 2-(4-fluoro-5,6,7,8-tetrahydronaphthalen-1-yl)-4,4,5,5-tetramethyl-1,3,2-dioxaborane:

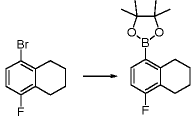

The compound 5-bromo-8-fluoro-1,2,3,4-tetralin (2.00 g, 8.73 mmol) and 4,4,4',4',5,5,5',5'-octamethyl-2,2'-bis(1,3,2-dioxaborane) (4.43 g, 17.46 mmol) was dissolved in anhydrous 1,4-dioxane (30 mL), potassium acetate (2.57 g, 26.19 mmol) and Pd(dppf)Cl₂ (0.64 g, 0.87 mmol) were added, and after displacement with nitrogen, the mixture was heated to 100°C and reacted under stirring for 16 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by column chromatography to obtain a light yellow oil. (2.1 g, yield: 87%). ¹H NMR (600MHz, CDCl₃) δ 7.63 - 7.54 (m, 1H), 6.80 (t,J = 8.8Hz, 1H), 3.03 (s, 2H), 2.71 (s, 2H), 1.82 - 1.72 (m, 4H), 1.33 (s, 12H).

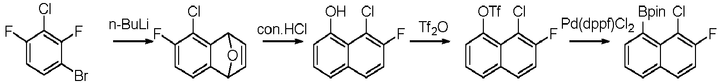

### Step 1: Synthesis of 5-chloro-6-fluoro-1,4-dihydro-1,4-epoxynaphthalene

The compound 1-bromo-3-chloro-2,4-difluorobenzene (5.0 g, 21.98 mmol) and furan (2.99 g, 43.97 mmol) were dissolved in anhydrous toluene (50 mL); in a nitrogen atmosphere, after the reaction liquid was cooled to -15°C, n-BuLi (10.6 mL, 26.38 mmol) was added dropwise to the reaction liquid, and after the dropwise addition was complete, the reaction liquid was slowly heated to room temperature and reacted under stirring for 12 h; and after the reaction was complete, the reaction was quenched with saturated ammonium chloride and extracted with methyl tert-butyl ether, and the organic phase was washed with a saturated aqueous NaCl solution, dried with anhydrous sodium sulfate, and concentrated to obtain a brown oil, which was directly used for the next step. (4.3 g, yield: 100%).

### Step 2: Synthesis of 8-chloro-7-fluoronaphthalen-1-ol

The crude compound obtained from the previous step (synthesis of 8-chloro-7-fluoronaphthalen-1-ol), i.e., 5-chloro-6-fluoro-1,4-dihydro-1,4-epoxynaphthalene, (4.3 g, 21.98 mmol) was dissolved in ethanol (10 ml) and concentrated hydrochloric acid (8 mL), and the mixture was heated to 80°C and reacted under stirring for 4 h. After the reaction was complete, the reaction liquid was cooled to room temperature, diluted with water, extracted with ethyl acetate, the organic phase was washed with a saturated aqueous NaCl solution, dried with anhydrous sodium sulfate, and concentrated to obtain a brown oil, the brown oil was placed in a refrigerator for 24 h to precipitate out a solid, which was diluted with petroleum ether, filtered, washed with petroleum ether, and dried to obtain an off-white solid. 1.3 g, yield: 30%. ¹H NMR (400MHz, CDCl₃) δ 7.91 (s, 1H), 7.75 (dd,J = 9.1, 5.6Hz, 1H), 7.44 - 7.34 (m, 2H), 7.30 (d,J = 8.7Hz, 1H), 7.08 (d,J = 7.1Hz, 1H).

### Step 3: Synthesis of 8-chloro-7-fluoronaphthalen-1-yl trifluoromethanesulfonate

The compound 8-chloro-7-fluoronaphthalen-1-ol (1.0 g, 5.08 mmol) was dissolved in anhydrous dichloromethane (10 mL), DIEA (3.94 g, 30.51 mmol) and a molecular sieve (1 g) were added, the mixture was stirred for 10 min at room temperature and then cooled to -40°C, and trifluoromethanesulfonic anhydride (1.86 g, 6.61 mmol) was added dropwise to the reaction liquid; after a stirred reaction was carried out for 20 min, the reaction was quenched with water and extracted with dichloromethane, and the organic phase was washed with a saturated aqueous NaCl solution, dried with anhydrous sodium sulfate, concentrated and separated by column chromatography to obtain a yellow solid. (1.65 g, yield: 98.8%). ¹H NMR (400MHz, CDCl₃) δ 7.90 (d,J = 8.1Hz, 1H), 7.84 (dd,J = 9.0, 5.4Hz, 1H), 7.59 (d,J = 7.7Hz, 1H), 7.51 (s, 1H), 7.44 (s, 1H).

### Step 4: Synthesis of 2-(8-chloro-7-fluoronaphthalen-1-yl)-4,4,5,5-tetramethyl-1,3,2-dioxaborane

The compound 8-chloro-7-fluoronaphthalen-1-yl trifluoromethanesulfonate (1.65 g, 5.02 mmol) and pinacol borate (2.53 g, 10.04 mmol) were dissolved in anhydrous DMF (20 mL), potassium acetate (2.44 g, 24.85 mmol) and Pd(dppf)Cl₂ (366 mg, 0.50 mmol) were added, and after displacement with nitrogen, a stirred reaction was carried out in a nitrogen atmosphere for 12 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by column chromatography to obtain an off-white solid. (1.25 g, yield: 82%). ¹H NMR (400MHz, CDCl₃) δ 7.83 (t,J = 10.4Hz, 1H), 7.75 (dd,J = 9.0, 5.5Hz, 1H), 7.70 (d,J = 6.8 Hz, 1H), 7.50 - 7.44 (m, 1H), 7.32 (t,J = 8.7Hz, 1H), 1.45 (s, 12H).

### Synthesis of intermediate (tetrahydro-1H-oyrrolizin-7a(5H)-yl)methanol;

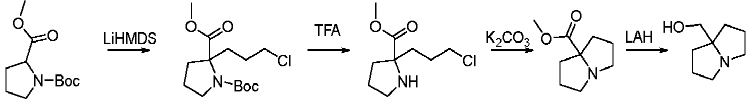

### Step 1: Synthesis of 1-tert-butyl 2-methyl 2-(3-chloropropyl)pyrrolidine-1,2-dicarboxylate:

1-tert-butyl 2-methyl 2-methylpyrrolidine-1,2-dicarboxylate (5.8 g, 25.3 mmol) was dissolved in tetrahydrofuran (25 mL) and cooled to -78°C, LiHMDS (1 M/L, 37.9 mmol) was added dropwise, and after 30 min, 1-bromo-3-chloropropane (19.9 g, 126 mmol) was added; and the mixture was reacted at room temperature for 2 h, and the reaction was then quenched by adding a saturated ammonium chloride aqueous solution, extracted with ethyl acetate, concentrated and then purified by column chromatography (petroleum ether/ethyl acetate = 5/1) to obtain a transparent oil. (5.1 g, yield: 65.9%). ¹H NMR (400 MHz, CDCl₃) δ 3.83 - 3.28 (m, 7H), 2.39 - 1.68 (m, 8H), 1.43 (d, *J =* 13.1 Hz, 9H).

### Step 2: Synthesis of methyl 2-(3-chloropropyl)pyrrolidine-2-carboxylate_{:}

1-(tert-butyl) 2-methyl 2-(3-chloropropyl)pyrrolidine-1,2-dicarboxylate (1 g, 3.27 mmol) was dissolved in dichloromethane (10 mL), trifluoroacetic acid (5 mL) was added, and the mixture was reacted at room temperature for 1 h, concentrated to dryness, and directly used for the next step of reaction.

### Step 3: Synthesis of methyl tetrahydro-1H-pyrrolizine-7a(5H)-carboxylate:

2-(3-chloropropyl) methyl pyrrolidine-2-carboxylate (670 mg, 3.27 mmol) was dissolved in methanol (10 mL), potassium carbonate (1.35 g, 9.81 mmol), potassium iodide (670 mg, 0.327 mmol) was added, the mixture was reacted at room temperature for 2 h, the solid was filtered out, and the filtrate was concentrated and then purified by column chromatography (petroleum ether/ethyl acetate = 5/1) to obtain a transparent oil. (400 mg, yield: 72.5%). ¹H NMR (400 MHz, CDCl₃) δ 3.72 (s, 3H), 3.21 - 3.11 (m, 2H), 2.64 (d, *J =* 10.2 Hz, 2H), 2.38 - 2.24 (m, 2H), 1.86 - 1.76 (m, 4H), 1.72 - 1.66 (m, 2H).

### Step 4: Synthesis of (tetrahydro-1H-pyrrolizin-7a(5H)-yl)methanol:

Methyl tetrahydro-1H-pyrrolizine-7a(5H)-carboxylate (400 mg, 2.37 mmol) was dissolved in tetrahydrofuran (10 mL), lithium aluminium tetrahydride (270 mg, 7.10 mmol) was added in portions under ice bath condition, after 1 h, TLC (petroleum ether/ethyl acetate = 10/1) detected that the reaction was complete, sodium sulfate decahydrate was added, the solid was filtered out, and the filtrate was concentrated to obtain a transparent oil. (290 mg, yield: 87%). ¹H NMR (400 MHz, MeOD) δ 3.36 - 3.28 (m, 2H), 2.96 (dt, *J =* 10.4, 6.1 Hz, 2H), 2.64 (ddd, *J =* 10.5, 7.3, 6.0 Hz, 2H), 1.97 - 1.81 (m, 4H), 1.73 (dt, *J =* 12.6, 6.8 Hz, 2H), 1.64 - 1.52 (m, 2H).

### Synthesis of intermediate tert-butyl (1R,5R)2,6-diazabicyclo[3.2.0]heptane-2-carboxylate:

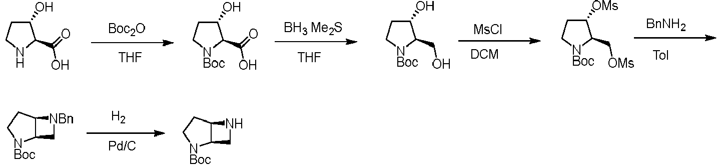

### Step 1: Synthesis of (2S,3S)-1-(tert-butoxycarbonyl)-3-hydroxypyrrolidine-2-carboxylic acid:

(2S,3S)-3-hydroxypyrrolidine-2-carboxylic acid (1.31 g, 10 mmol) was dissolved in tetrahydrofuran (20 mL) and water (10 mL), sodium hydroxide (0.80 g, 20 mmol) and Boc anhydride (3.30 g, 15 mmol) were added, the mixture was stirred at room temperature for 15 h and extracted with ethyl acetate, and the aqueous layer was adjusted to pH = 2.0 with 1N hydrochloric acid, extracted with ethyl acetate, and concentrated to obtain a white solid. (1.5 g, yield: 65%). ¹H NMR (400 MHz, CDCl₃)δ 4.82 (s, 1H), 4.25 (s, 1H), 3.62 (q, *J =* 9.3 Hz, 1H), 3.48 (s, 1H), 2.12 (dd, *J =* 8.9, 4.5 Hz, 1H), 1.94 (ddd, *J =* 10.0, 6.7, 3.3 Hz, 1H), 1.51 (s, 9H).

### Step 2: Synthesis of tert-butyl (2R,3S)-3-hydroxy-2-(hydroxymethyl)pyrrolidine-1-carboxylate:

(2S,3S)-1-(tert-butoxycarbonyl)-3-hydroxypyrrolidine-2-carboxylic acid (1.5 g, 6.5 mmol) was dissolved in tetrahydrofuran (20 mL), borane dimethyl sulfide (2 M/L, 14.3 mmol) was added, the mixture was heated to reflux for 3 h and cooled to room temperature, methanol was added dropwise to quench the reaction, and after concentration, the reaction product was purified by column chromatography (petroleum ether/ethyl acetate = 1/1) to obtain a transparent oil. (1.2 g, yield: 85.7%). 1H NMR (400 MHz, MeOD) δ 4.42 - 4.21 (m, 1H), 3.66 (d, J = 8.8 Hz, 2H), 3.52 - 3.35 (m, 3H), 2.19 - 2.04 (m, 1H), 1.90 - 1.74 (m, 1H), 1.47 (s, 9H).

### Step 3: Synthesis of tert-butyl (2R,3S)3-(methylsulfonyloxy)-2-((methylsulfonyloxy)methyl)pyrrolidine-1-carboxylate:

Tert-butyl (2R,3S)-3-hydroxy-2-(hydroxymethyl)pyrrolidine-1-carboxylate (1.16 g, 5.3 mmol) was dissolved in dichloromethane (20 mL), triethylamine (2.26 g, 22.4 mmol) and methylsulfonyl chloride (1.83 g, 16 mmol) were added under ice bath condition, the mixture was reacted at room temperature for 2 h, ice water was added, and the mixture was extracted with dichloromethane, dried with anhydrous sodium sulfate, filtered, concentrated, and then directly used for the next step of reaction.

### Step 4: Synthesis of tert-butyl (1R,5R)-6-benzyl-2,6-diazabicyclo[3.2.0]heptane-2-carboxylate:

Tert-butyl (2R,3S)3-(methylsulfonyloxy)-2-((methylsulfonyloxy)methyl)pyrrolidine-1 -carboxylate (2.0 g, 5.3 mmol) was dissolved in toluene (20 mL), benzylamine (1.71 g, 16 mmol) was added, the mixture was heated to 110°C, reacted for 15 h, and cooled to room temperature, the solid was filtered out, and the filtrate was concentrated and then purified by column chromatography (petroleum ether/ethyl acetate = 1/1) to obtain a light yellow oil. (890 mg, yield: 58%). ¹H NMR (400 MHz, MeOD) δ 7.42 - 7.18 (m, 5H), 4.31 - 4.15 (m, 1H), 3.99 (d, *J =* 5.0 Hz, 1H), 3.67 (d, *J =* 14.7 Hz, 4H), 3.18 (dd, *J =* 6.4, 4.3 Hz, 2H), 1.69 - 1.53 (m, 2H), 1.44 (d, *J =* 15.2 Hz, 9H).

### Step 5: Synthesis of tert-butyl (1R,5R)2,6-diazabicyclo[3.2.0]heptane-2-carboxylate:

Tert-butyl (1R,5R)-6-benzyl-2,6-diazabicyclo[3.2.0]heptane-2-carboxylate (145 mg, 0.5 mmol) was dissolved in methanol (20 mL), palladium on carbon (10%, 100 mg) was added, the mixture was reacted under the pressure of a hydrogen balloon for 20 h and filtered, and the filtrate was concentrated to obtain a transparent solid. (90 mg, yield: 90.3%). ¹H NMR (400 MHz, MeOD) δ 4.11 (dd, *J =* 10.7, 6.0 Hz, 1H), 3.92 (s, 1H), 3.72 (td, *J =* 10.8, 6.9 Hz, 2H), 3.44 - 3.33 (m, 2H), 2.07 (tt, *J =* 16.0, 7.9 Hz, 2H), 1.47 (d, *J* = 4.2 Hz, 12H).

### Example 1: Synthesis of 2-((S)-1-acryloyl-4-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)piperazin-2-yl)acetonitrile

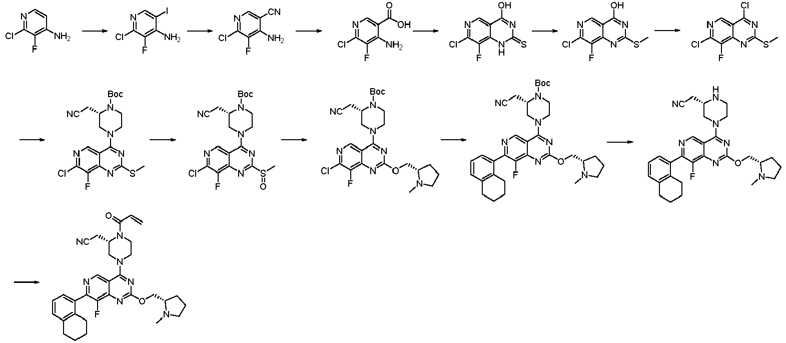

### Step 1: 2-chloro-3-fluoro-5-iodopyridine-4-amine

The compound 2-chloro-3-fluoropyridine-4-amine (4.22 g, 28.80 mmol) was dissolved in acetonitrile (50 mL), NIS (7.77 g, 34.55 mmol) and p-methylbenzenesulfonic acid (248 mg, 1.44 mmol) were then added, and the mixture was heated to 70°C and reacted under stirring for 16 h. After the reaction was complete, the reaction liquid was cooled to room temperature and diluted with water, whereby a solid precipitated out, which was filtered out and washed with a saturated sodium thiosulfate aqueous solution and with water and dried in vacuo to obtain the target compound, which was directly used in the next step. (7.5 g, yield: 98%). ¹H NMR (400MHz, CDCl₃) δ 8.17 (s, 1H), 4.83 (s, 2H).

### Step 2: Synthesis of 4-amino-6-chloro-5-fluoronicotinonitrile

The compound 2-chloro-3-fluoro-5-iodopyridine-4-amine (7.7 g, 28.26 mmol) and Zn(CN)₂ (4.32 g, 36.74 mmol) were dissolved in anhydrous DMF (150 mL), Pd(PPh₃)₄ (1.63 g, 1.41 mmol) and a 4A molecular sieve (2.5 g) were then added, and after displacement with nitrogen, the mixture was heated to 100°C in a nitrogen atmosphere and reacted under stirring for 3 h. After the reaction was complete, the reaction product was filtered to remove solids, the solution was cooled to room temperature, 300 mL of water was added to dilute the reaction liquid, whereby a solid precipitated out, and after filtration, the solid was washed with water and dried in vacuo to obtain a crude product, which was directly used in the next step. (4.85 g, yield: 100%). ¹H NMR (400MHz, DMSO) δ 8.20 (s, 1H), 7.66 (s, 2H).

### Step 3: Synthesis of 4-amino-6-chloro-5-fluoronicotinic acid

The compound 4-amino-6-chloro-5-fluoronicotinonitrile (4.85 g, 28.26 mmol) was dissolved in 50% H₂SO₄ (50 mL), and the mixture was heated to 120° C and reacted under stirring for 6 h. After the reaction was complete, the reaction product was cooled to room temperature, the reaction liquid was slowly poured onto crushed ice, whereby a solid precipitated out, and after filtration, the solid was washed with water. The solid was dissolved with ethyl acetate and washed by adding a saturated sodium carbonate aqueous solution, an aqueous phase was collected, the aqueous phase was adjusted to pH 2-3 with 10% hydrochloric acid, whereby a solid precipitated out, and after filtration, the solid was dried in vacuo to obtain an off-white solid. (4.62 g, yield: 85.8%). ¹H NMR (400MHz, DMSO) δ 8.36 (s, 1H), 7.59 (s, 2H).

### Step 4: Synthesis of 7-chloro-8-fluoro-4-hydroxypyridino[4,3-d]pyrimidine-2(1H)-thione

The compound 4-amino-6-chloro-5-fluoronicotinic acid was added to a reaction flask, POCl₃ (50 mL) was then added, and the mixture was heated to 90°C and reacted under stirring for 4 h. After the reaction was complete, the reaction product was cooled to room temperature, the reaction liquid was concentrated to obtain an oil, and the oil was dissolved in anhydrous tetrahydrofuran (20 mL), then added dropwise to ammonium thiocyanate (3.67 g, 48.28 mmol) in tetrahydrofuran (80 mL), and reacted under stirring at room temperature for 24 h. After the reaction was complete, the reaction liquid was diluted with water and extracted with ethyl acetate, and the organic phase was washed with a saturated aqueous NaCl solution, dried with anhydrous sodium sulfate and concentrated to obtain a yellow solid. 10 ml of ethyl acetate was then added for pulping and filtered to obtain a light yellow solid. (4.52 g, yield: 80.8%). ¹H NMR (400MHz, DMSO) δ 13.29 (s, 1H), 12.85 (s, 1H), 8.64 (s, 1H).

### Step 5: Synthesis of 7-chloro-8-fluoro-2-(methylthio)pyridino[4,3-d]pyrimidin-4-ol

The compound 7-chloro-8-fluoro-4-hydroxypyridino[4,3-d]pyrimidine-2(1H)-thione (4.52 g, 19.51 mmol) was dissolved in anhydrous DMF (50 mL), sodium methoxide (1.06 g, 19.51 mmol) was then added, the mixture was stirred at room temperature for 10 min, iodomethane (2.77 g, 1.21 mL, 19.51 mmol) was added dropwise, and the mixture was reacted at room temperature under stirring for 2 h. After the reaction was complete, the reaction liquid was diluted by adding cold water, whereby a solid precipitated out, and after filtration, the solid was washed with water and dried in vacuo to obtain a yellow solid. (3.0 g, yield: 66%). ¹H NMR (400MHz, DMSO) δ 13.24 (s, 1H), 8.81 (s, 1H), 2.62 (s, 3H).

### Step 6: Synthesis of 4,7-dichloro-8-fluoro-2-(methylthio)pyridino[4,3-d]pyrimidine

The compound 7-chloro-8-fluoro-2-(methylthio)pyridino[4,3-d]pyrimidin-4-ol (420 mg, 1.71 mmol) was dissolved in phosphorus oxychloride (4 mL), DIEA (442 mg, 3.42 mmol) was then added, and the mixture was heated to 90°C and reacted for 3 h. After the reaction was complete, the reaction product was cooled to room temperature and concentrated to remove excess phosphorus oxychloride. The product was then dissolved in ethyl acetate and washed sequentially with a saturated aqueous NaCl solution and water, and the organic phase was dried with anhydrous sodium sulfate and concentrated to obtain a crude product, which was directly used for the next step. (450 mg, yield: 100%).

### Step 7: Synthesis of tert-butyl (S)-4-(7-chloro-8-fluoro-2-(methylthio)pyridino[4,3-d]pyrimidin-4-yl)-2-(cyanomethyl)piperazine-1-carboxylate

The compound 4,7-dichloro-8-fluoro-2-(methylthio)pyridino[4,3-d]pyrimidine (450 mg, 1.71 mmol) was dissolved in anhydrous DMF (10 mL), and DIEA (1.10 g, 8.55 mmol) and (S)-2-(piperazin-2-yl)acetonitrile dihydrochloride (339 mg, 1.71 mmol) were added under ice-water bath cooling condition; and after stirring for 10 min under ice-water bath cooling condition, di-tert butyl dicarbonate (747 mg, 3.42 mmol) was added, and the mixture was reacted at room temperature under stirring for 16 h. After the reaction was complete, the reaction liquid was diluted by adding 100 mL of cold water under stirring and extracted with ethyl acetate, and the organic phase was washed with a saturated aqueous NaCl solution, dried with anhydrous sodium sulfate, concentrated and separated by column chromatography to obtain an off-white solid. (710 mg, yield: 91.6%). ¹H NMR (600MHz, CDCl₃) δ 8.80 (s, 1H), 4.62 (s, 1H), 4.45 (dd,J = 13.9, 3.5Hz, 1H), 4.28 (d,J = 12.8 Hz, 1H), 4.08 (s, 1H), 3.84 (s, 1H), 3.66 (d,J = 8.6Hz, 1H), 3.39 (s, 1H), 2.87 - 2.74 (m, 1H), 2.69 (dd,J = 16.8, 5.9Hz, 1H), 2.64 (s, 3H), 1.51 (s, 9H).

### Step 8: Synthesis of tert-butyl (2S)-4-(7-chloro-8-fluoro-2-(methylsulfinyl)pyridino[4,3-d]pyrimidin-4-yl)-2-(cyanomethyl)piperazine-1-carboxylate

The compound tert-butyl (S)-4-(7-chloro-8-fluoro-2-(methylthio)pyridino[4,3-d]pyrimidin-4-yl)-2-(cyanomethyl)piperazine-1-carboxylate (700 mg, 1.55 mmol) was dissolved in dichloromethane (10 mL), 85% m-chloroperoxybenzoic acid (378 mg, 1.86 mmol) was added under ice-water bath cooling condition, and the mixture was reacted under stirring and ice-water bath cooling conditions for 30 min. After the reaction was complete, the reaction was quenched by using a saturated sodium thiosulfate solution and extracted with dichloromethane, and the organic phase was washed with saturated sodium bicarbonate and a table salt aqueous solution, dried with anhydrous sodium sulfate and concentrated to obtain a crude product, which was directly used for the next step. (725 mg, yield: 100%).

### Step 9: Synthesis of tert-butyl (S)-4-(7-chloro-8-fluoro-2-(((S)-1-methylpyrrolidin-2-yl)methoxyl)pyridino[4,3-d]pyrimidin-4-yl)-2-(cyanomethyl)piperazine-1-carboxylate

The compound tert-butyl (2S)-4-(7-chloro-8-fluoro-2-(methylsulfinyl)pyridino[4,3-d]pyrimidin-4-yl)-2-(cyanomethyl)piperazine-1-carboxylate (725 mg, 1.55 mmol) was dissolved in anhydrous toluene (10 mL), (S)-(1-methylpyrrolidin-2-yl)methanol (0.31 g, 2.71 mmol) was then added, sodium tert-butoxide (0.30 g, 3.09 mmol) was added under ice-water bath cooling condition, and the mixture was reacted under stirring and ice-water bath cooling conditions for 30 min. After the reaction was complete, the reaction was quenched with cold water and extracted with dichloromethane, and the organic phase was washed with a saturated aqueous NaCl solution, dried with anhydrous sodium sulfate, concentrated and separated by column chromatography to obtain an off-white solid. (510 mg, yield: 63%).

### Step 10: Synthesis of tert-butyl (S)-2-(cyanomethyl)-4-(8-fluoro-2-(((S)-1-methylpyrrolidin-2-yl)methoxyl-7-(5,6,7,8-tetrahydronaphthalen-1-yl)pyridino[4,3-d]pyrimidin-4-yl)piperazine-1-carboxylate

The compound tert-butyl (S)-4-(7-chloro-8-fluoro-2-(((S)-1-methylpyrrolidin-2-yl)methoxy)pyridino[4,3-d]pyrimidin-4-yl)-2-(cyanomethyl)piperazine-1-carboxylate (40 mg, 0.08 mmol) and 4,4,5,5-tetramethyl-2-(5,6,7,8-tetrahydronaphthalen-1-yl)-1,3,2-dioxaborane (30 mg, 0.12 mmol) were dissolved in 1,4-dioxane/water = 5/1 (3 mL), cesium carbonate (76 mg, 0.23 mmol) and Pd(PPh₃)₄ (45 mg, 0.04 mmol) were added, and after displacement with nitrogen, the mixture was heated to 95°C in a nitrogen atmosphere and reacted under stirring for 1 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by TLC to obtain an off-white solid. (20 mg, yield: 42%).

### Step 11: Synthesis of 2-((S)-4-(8-fluoro-2-(((S)-1-methylypyrrolidin-2-yl)methoxy)-7-(5,6,7,8-tetrahydronaphthalen-1-yl)pyridino[4,3-d]pyrimidin-4-yl)piperazin-2-yl)acetonitrile

The compound tert-butyl (S)-2-(cyanomethyl)-4-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)piperazine-1-carboxylate (20 mg, 0.03mmoL) was dissolved in dichloromethane (3 mL), trifluoroacetic acid (1 mL) was added, and the mixture was reacted at room temperature under stirring for 1 h. After the reaction was complete, the reaction liquid was concentrated, then dissolved in dichloromethane, adjusted to pH 8-9 with a saturated sodium carbonate solution and extracted with dichloromethane, and the organic phase was dried with anhydrous sodium sulfate and concentrated to obtain a crude product, which was directly used for the next step. (14 mg, yield: 98%).

### Step 12: Synthesis of 2-((S)-1-acryloyl-4-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)piperazin-2-yl)acetonitrile

The compound 2-((S)-4-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)piperazin-2-yl)acetonitrile (14 mg, 0.03 mmol) was dissolved in dichloromethane (5 mL), DIEA (5 mg, 0.03 mmol) and acryloyl chloride (3 mg, 0.03 mmol) were added under ice-water bath cooling condition, and the mixture was reacted under stirring and ice-water bath cooling conditions for 5 min. After the reaction was complete, the reaction was quenched with saturated sodium carbonate and extracted with dichloromethane, and the organic phase was dried with anhydrous sodium sulfate, concentrated, separated and purified by TLC to obtain an off-white solid. (10 mg, yield: 62%). ¹H NMR (400 MHz, CDCl₃) δ 9.10 (s, 1H), 7.26 - 7.21 (dd, *m*, 3H), 6.62- 6.57 (m, 1H), 6.48 - 6.31 (m, 1H), 5.83 (dd, *J =* 19.7, 11.2 Hz, 1H), 5.02 (s, 2H), 4.62- 4.58 (m, 1H), 4.49 - 4.44 (m, 3H), 4.10 (d, *J =* 12.0 Hz, 1H), 3.87 - 3.83 (m, 2H), 3.65 (dd, *J =* 13.5, 6.8 Hz, 1H), 3.57 - 3.51 (m, 2H), 3.34-3.31 (m, 1H), 3.14 - 2.95 (m, 2H), 2.87 - 2.83 (m, 6H), 2.75 - 2.71 (m, 1H), 2.62 (t, *J=* 5.7 Hz, 3H), 2.30 - 2.25 (m, 2H), 2.16 - 2.11 (m, 2H). MS m/z: 570.75 [M+H]⁺

### Example 2: Synthesis of (S)-2-(1-acryloyl-4-(8-fluoro-2-((tetrahydro-1H-pyrrolizin-7a(5H)-yl)methoxy)-7-(5,6,7,8-tetrahydronanhthalen-1-yl)pyridino[4,3-d]pyrminidin-4-yl)piperazin-2-yl)acetonitrile

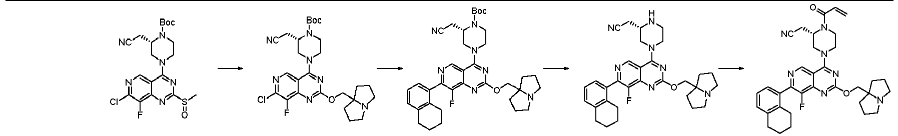

### Step 1: Synthesis of tert-butyl (S)-4-(7-chloro-8-fluoro-2-((tetrahydro-1H-pyrrolizin-7a(5H)-yl)methoxy)pyridino[4,3-d]pyrimidin-4-yl)-2-(cyanomethyl)piperazine-1-carboxylate

The compound tert-butyl (2S)-4-(7-chloro-8-fluoro-2-(methylsulfinyl)pyridino[4,3-d]pyrimidin-4-yl)-2-(cyanomethyl)piperazine-1-carboxylate (100 mg, 0.21 mmol) was dissolved in anhydrous toluene (3 mL), and (tetrahydro-1H-pyrrolizin-7a(5H)-yl)methanol (46 mg, 0.32 mmol) and sodium tert-butoxide (31 mg, 0.32 mmol) were added under ice-water bath cooling condition and reacted under stirring and ice-water bath cooling conditions for 3 h; and after the reaction was complete, the reaction was quenched with cold water and extracted with dichloromethane, and the organic phase was washed with a saturated aqueous NaCl solution, dried with anhydrous sodium sulfate and separated by column chromatography to obtain an off-white solid. (55 mg, yield: 47%). ¹H NMR (400MHz, CDCl₃) δ 8.82 (s, 1H), 4.75 (dt,J = 23.5, 12.9Hz, 3H), 4.58 (s, 1H), 4.31 (d,J = 11.7Hz, 1H), 4.18 - 3.88 (m, 4H), 3.82 (t,J = 10.1Hz, 1H), 3.37 (s, 1H), 3.23 (dd,J = 16.7, 8.9Hz, 1H), 3.01 (s, 2H), 2.80 (dd,J = 16.7, 3.9Hz, 1H), 2.55 - 2.37 (m, 2H), 2.34 - 2.21 (m, 3H), 2.15 (dt,J = 13.5, 6.8Hz, 2H), 2.02 (dd,J = 11.3, 6.9Hz, 2H), 1.49 (s, 9H).

### Step 2: Synthesis of tert-butyl (S)-2-(cyanomethyl)-4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazine-1-carboxylate

The compound tert-butyl (S)-4-(7-chloro-8-fluoro-2-((tetrahydro-1H-pyrrolizin-7a(5H)-yl)methoxy)pyridino[4,3-d]pyrimidin-4-yl)-2-(cyanomethyl)piperazine-1-carboxylate (57 mg, 0.10 mmol) and 4,4,5,5-tetramethyl-2-(5,6,7,8-tetrahydronaphthalen-1-yl)-1,3,2-dioxaborane (33 mg, 0.13 mmol) were dissolved in 1,4-dioxane/water = 5/1 (3 mL), cesium carbonate (102 mg, 0.31 mmol) and Pd(PPh₃)₄ (60 mg, 0.05 mmol) were added, and after displacement with nitrogen, the mixture was heated to 95°C in a nitrogen atmosphere and reacted under stirring for 1 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by TLC to obtain an off-white solid. (38 mg, yield: 57%). ¹H NMR (400 MHz, CDCl₃) δ 9.09 (s, 1H), 7.26 - 7.21 (m, 3H), 4.82 (s, 2H), 4.73 - 4.57 (m, 2H), 4.41 - 4.38 (m, 1H), 3.98 - 3.92 (m, 4H), 3.87 -3.82 (m, 1H), 3.47 - 3.40 (m, 2H), 3.23 - 3.20 (m, 1H), 3.00 (s, 2H), 2.87 (t, *J* = 6.2 Hz, 2H), 2.63 - 2.61 (m, 2H), 2.54 - 2.38 (m, 2H), 2.38 - 2.20 (m, 2H), 2.14 (s, 2H), 2.03 - 1.98 (m, 2H), 1.81 (d, *J* = 6.1 Hz, 2H), 1.73 - 1.71(m, 2H), 1.50 (s, 9H).

### Step 3: Synthesis of (S)-2-(4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazin-2-yl)acetonitrile

The compound tert-butyl (S)-2-(cyanomethyl)-4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazine-1-carboxylate (38 mg, 0.06 mmol) was dissolved in dichloromethane (3 mL), trifluoroacetic acid (1 mL) was then added, and the mixture was reacted at room temperature under stirring for 1 h. After the reaction was complete, the reaction liquid was concentrated, then dissolved in dichloromethane, adjusted to pH 8-9 with saturated sodium carbonate and extracted with dichloromethane, and the organic phase was dried with anhydrous sodium sulfate and concentrated to obtain a crude product, which was directly used for the next step. (32 mg, yield: 100%).

### Step 4: Synthesis of (S)-2-(1-acryloyl-4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazin-2-yl)acetonitrile

The compound (S)-2-(4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazin-2-yl)acetonitrile (15 mg, 0.03 mmol) was dissolved in dichloromethane (3 mL), DIEA (5 mg, 0.033 mmol) and acryloyl chloride (3 mg, 0.03 mmol) were added under ice-water bath cooling condition, and the mixture was reacted under stirring and ice-water bath cooling conditions for 5 min. After the reaction was complete, the reaction was quenched with saturated sodium carbonate and extracted with dichloromethane, and the organic phase was dried with anhydrous sodium sulfate, concentrated, separated and purified by TLC to obtain an off-white solid. (8 mg, yield: 49%). ¹H NMR (400 MHz, CDCl₃) δ 9.13 (s, 1H), 7.21 (d, *J =* 3.8 Hz, 3H), 6.56 (s, 1H), 6.38 (d, *J* = 15.4 Hz, 1H), 5.82 (d, *J =* 9.6 Hz, 1H), 4.82 (s, 2H), 4.62 (d, *J =* 14.6 Hz, 1H), 4.47 (d, *J =* 11.3 Hz, 1H), 4.25 - 4.22 (m, 1H), 3.93 (s, 4H), 3.66 (d, *J =* 4.6 Hz, 1H), 3.35 (dd, *J =* 16.9, 7.6 Hz, 1H), 3.15 - 2.94 (m, 4H), 2.87 (t, *J =* 6.1 Hz, 2H), 2.63 (d, *J =* 5.9 Hz, 2H), 2.45 (ddd, *J =* 26.5, 13.0, 6.6 Hz, 2H), 2.28 (dd, *J*= 16.8, 8.4 Hz, 2H), 2.18 - 2.11 (m, 2H), 2.06 - 1.99 (m, 2H), 1.81 (d, *J =* 6.4 Hz, 2H), 1.73 (d, *J =* 6.2 Hz, 2H). MS m/z: 596.68 [M+H]⁺

### Example 3: Synthesis of (S)-2-(4-(8-fluoro-2-((tetrahydro-1H-pyrrolizin-7a(5H)-yl)methoxy)-7-5.6.7,8-tetrahydronanhthalen-1-yl)pyridino[4,3-d]pyrimidin-4-yl)-1-(2-fluoroacryloyl)piperazin-2-yl)acetonitrile

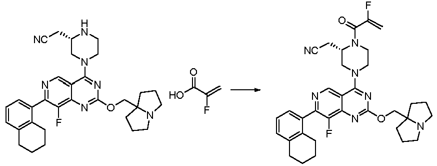

The compound (S)-2-(4-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)piperazin-2-yl)acetonitrile (15 mg, 0.03 mmol) and 2-fluoroacrylic acid (4 mg, 0.04 mmol) were dissolved in dichloromethane (3 mL), HATU (16 mg, 0.04 mmol) was added, the mixture was cooled to 0-10°C in an ice-water bath, DIEA (6 mg, 0.04 mmol) was then added, and the mixture was reacted at 0-10°C under stirring for 4 h. After the reaction was complete, the reaction liquid was diluted with a saturated sodium bicarbonate aqueous solution and extracted with dichloromethane, and the organic phase was dried with anhydrous sodium sulfate, concentrated, separated and purified by TLC to obtain an off-white solid. (8 mg, yield: 47%). ¹H NMR (400 MHz, CDCl₃) δ 9.14 (s, 1H), 7.26 - 7.22 (m, 3H), 5.60 - 5.38(m, 1H), 5.28 (dd, J = 16.8Hz, 1H), , 4.82 (s, 2H), 4.62 (d, *J* = 14.6 Hz, 1H), 4.47 (d, *J=* 11.3 Hz, 1H), 4.25 - 4.22 (m, 1H), 3.93 (s, 4H), 3.66 (d, *J =* 4.6 Hz, 1H), 3.35 (dd, *J =* 16.9, 7.6 Hz, 1H), 3.15 - 2.94 (m, 4H), 2.87 (t, *J =* 6.1 Hz, 2H), 2.63 (d, *J*= 5.9 Hz, 2H), 2.45 (ddd, *J =* 26.5, 13.0, 6.6 Hz, 2H), 2.28 (dd, *J* = 16.8, 8.4 Hz, 2H), 2.18 - 2.11 (m, 2H), 2.06 - 1.99 (m, 2H), 1.81 (d, *J =* 6.4 Hz, 2H), 1.73 (d, *J =* 6.2 Hz, 2H)MS m/z: 614.6 [M+H]⁺

The compounds of Examples 4-48 were prepared by preparation method 1

| Ex. | Compound name | Structural formula | m/z: ES⁺[M+H] |
|---|---|---|---|
| 4 | 2-((S)-4-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)-1-(2-fluoroacryloyl)piperazin-2-yl)acetonitrile | 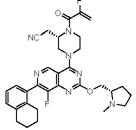 | 588.2 |
| 5 | 2-((S)-1-acryloyl-4-(8-fluoro-2-(((2S,4R)-4-fluoro-1-methylpyrrolidin-2-yl)methoxy)-7-(5,6,7,8-tetrahydronaphthalen-1-yl)pyridino[4,3-d]pyrimidin-4-yl)piperazin-2-yl)acetonitrile | 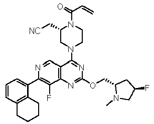 | 588.2 |
| 6 | 2-((S)-4-((8-fluoro-2-(((2S,4R)-4-fluoro-1-methylpyrrolidin-2-yl)methoxy)-7-(5,6,7,8-tetrahydronaphthalen-1-yl)pyridino[4,3-d]pyrimidin-4-yl)-1-(2-fluoroacryloyl)piperazin-2-yl)acetonitrile | 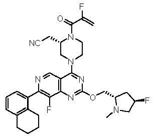 | 606.2 |
| 7 | 2-((S)-1-acryloyl-4-(8-fluoro-2-(((2R,7aS)-2-fluorotetrahydro-1H-pyrrolizin-7a(5H)-yl)methoxy)-7-(5,6,7,8-tetrahydronaphthalen-1-yl)pyridino[4,3-d]pyrimidin-4-yl)piperazin-2-yl)acetonitrile | 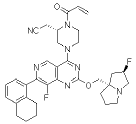 | 614.7 |
| 8 | 2-((S)-4-(8-fluoro-2-(((2R,7aS)-2-fluorotetrahydro-1H-pyrrolizin-7a(5H)-yl)methoxy)-7-(5,6,7,8-tetrahydronaphthalen-1-yl)pyridino[4,3-d]pyrimidin-4-yl)-1-(2-fluoroacryloyl)piperazin-2-yl)acetonitrile | 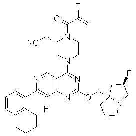 | 632.7 |
| 9 | 1-((1R,SR)-6-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 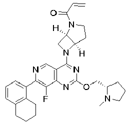 | 543 |
| 10 | 2-fluoro-1-((1R,SR)-6-(8-fluoro-2-(((S)-1-methylpyrrolidin-2-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 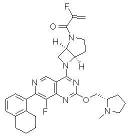 | 561.6 |
| 11 | 1-((1R,5R)-6-(8-fluoro-2-(((2S,4R)-4-fluoro-1-methylpyrrolidin-2-yl)methoxy)-7-(5,6,7,8-tetrahydronaphthalen-1-yl)pyridino[4,3-d]pyrimidin-4-yl-2,6-diazabicyclo[3.2.0]hept-2-yl)prop-2-en-1-one | 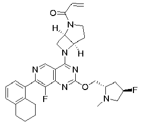 | 561.6 |
| 12 | 2-fluoro-1-(((1R,SR)-6-(8-fluoro-2-((((2S,4R)-4-fluoro-1-methylpyrrolidin-2-yl)methoxy)-7-(5 , 6,7,8-tetrahydronaphthalen-1-yl)pyridino[4,3-d]pyrimidin-4-yl)-2,6-diazabicyclo[3.2.0]hept-2-yl)prop-2-en-1-one | 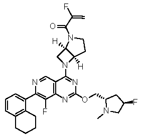 | 579.6 |
| 13 | 1-((1R,SR)-6-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 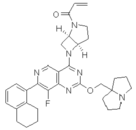 | 569.3 |
| 14 | 2-fluoro-1-((1R,SR)-6-(8-fluoro-2-((tetrahydro-1H-pyrrolizin-7a(5H)-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 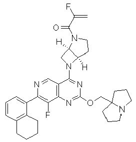 | 587.7 |
| 15 | 1-(((1R,SR)-6-(8-fluoro-2-((((2R,7aS)-2-fluorotetrahydro-1H-pyrrolizin-7a(5H)-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 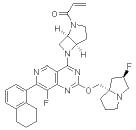 | 587.7 |
| 16 | 2-fluoro-1-(((1R,SR)-6-(8-fluoro-2-((((2R,7aS)-2-fluorotetrahydro-1H-pyrrolizin-7a(5H)-yl)methoxy)-7-(5,6,7,8-tetrahydronaphthalen-1-yl)pyridino[4,3-d]pyrimidin-4-yl)-2,6-diazabicyclo[3.2.0]hept-2-yl)prop-2-en-1-one | 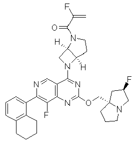 | 605.6 |
| 17 | 2-((S)-1-acryloyl-4-(8-fluoro-7-(4-fluoro-5,6,7,8-tetrahydronaphthalen-1-yl)-2-(((S)-1-methylpyrrolidin-2-yl)methoxy)pyridino[4,3-d]pyrimidin-4-yl)piperazin-2-yl)acetonitrile | 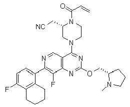 | 588 |
| 18 | 2-((S)-4-((8-fluoro-7-(4-fluoro-5,6,7,8-tetrahydronaphthalen-1-yl)-2-(((S)-1-methylpyrrolidin-2-yl)methoxy)pyridino[4,3-d]pyrimidin-4-yl)-1-(2-fluoroacryloyl)piperazin-2-yl)acetonitrile | 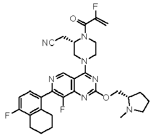 | 606 |
| 19 | 2-((S)-1-acryloyl-4-(8-fluoro-2-((((2S,4R)-4-fluoro-1-methylpyrrolidin-2-yl)methoxy)-7-(4-fluoro-5,6,7,8-tetrahydronaphthalen-1-yl)pyridino[4,3-d]pyrimidin-4-yl)piperazin-2-yl)acetonitrile | 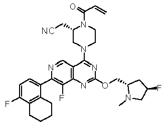 | 606 |
| 20 | 2-((S)-4-(8-fluoro-2-((((2S,4R)-4-fluoro-1-methylpyrrolidin-2-yl)methoxy)-7-(4-fluoro-5,6,7,8-tetrahydronaphthalen-1-yl)pyridino[4,3-d]pyrimidin-4-yl)-1-(2-fluoroacryloyl)piperazin-2-yl)acetonitrile | 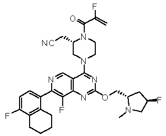 | 624 |
| 21 | (S)-2-(1-acryloyl-4-(8-fluoro-7-(4-fluoro-5,6,7,8-tetrahydronaphthalen-1-yl)-2-(((tetrahydro-1H-pyrrolizin-7a(5H)-yl)methoxy)pyridino[4,3-d]pyrimidin-4-yl)piperazin-2-yl)acetonitrile | 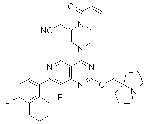 | 614 |
| 22 | (S)-2-(4-(8-fluoro-7-(4-fluoro-5,6,7,8-tetrahydronaphthalen-1-yl)-2-((tetrahydro-1H-pyrrolizin-7a(SH))-yl)methoxy)pyridino[4,3-d]pyrimidin-4-yl)-1-(2-fluoroacryloyl)piperazin-2-yl)acetonitrile | 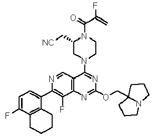 | |
| 23 | 2-((2S)-1-acryloyl-4-(8-fluoro-7-(4-fluoro-5,6,7,8-tetrahydronaphthalen-1-yl)-2-((((2R,7aS)-2-fluorotetrahydro-1H-pyrrolizin-7a(5H)-yl)methoxy)pyridino[4,3-d]pyrimidin-4-yl)piperazin-2-yl)acetonitrile | 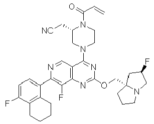 | 632 |
| 24 | 2-((2S)-4-(8-fluoro-7-(4-fluoro-5,6,7,8-tetrahydronaphthalen-1-yl)-2-(((((2R,7aS)-2-fluorotetrahydro-1H-pyrrolizin-7a(5H)-yl)methoxy)pyridino[4,3-d]pyrimidin-4-yl)-1-(2-fluoroacryloyl)piperazin-2-yl)acetonitrile | 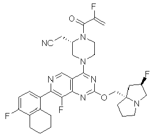 | 650 |
| 25 | 1-((1R,5R)-6-(8-fluoro-7-(4-fluoro-5,6,7,8-tetrahydronaphthalen-1-yl)-2-((((S)-1-methylpyrrolidin-2-yl)methoxy)pyridino[4,3-d]pyrimidin-4-yl)-2,6-diazabicyclo[3.2.0]hept-2-yl)prop-2-en-1-one | 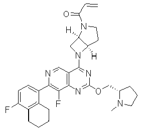 | 561.6 |
| 26 | 2-fluoro-1-(((1R,SR)-6-(8-fluoro-7-(4-fluoro-5,6,7,8-tetrahydronaphthalen-1-yl)-2-(((S)-1-methylpyrrolidin-2-yl)methoxy)pyridino[4,3-d]pyrimidin-4-yl)-2,6-diazabicyclo[3.2.0]hept-2-yl)prop-2-en-1-one | 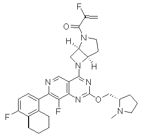 | 579.6 |
| 27 | 1-((1R,5R)-6-(8-fluoro-2-((((2S,4R)-4-fluoro-1-methylpyrrolidin-2-yl)methoxy)-7-(4-fluoro-5,6,7,8-tetrahydronaphthalen-1-yl)pyridino[4,3-d]pyrimidin-4-yl)-2,6-diazabicyclo[3.2.0]hept-2-yl)prop-2-en-1-one | 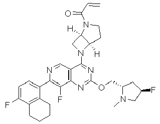 | 579.2 |
| 28 | 2-fluoro-1-(((1R,SR)-6-(8-fluoro-2-(((2S,4R)-4-fluoro-1-methylpyrrolidin-2-yl)methoxy)-7-(4-fluoro-5,6,7,8-tetrahydronaphthalen-1-yl)pyridino[4,3-d]pyrimidin-4-yl)-2,6-diazabicyclo[3.2.0]hept-2-yl)prop-2-en-1-one | 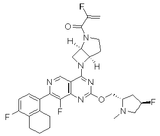 | 597.2 |
| 29 | 1-(((1R,5R)-6-(8-fluoro-7-(4-fluoro-5,6,7,8-tetrahydronaphthalen-1-yl)-2-((tetrahydro-1H-pyrrolizin-7a(5H)-yl)methoxy)pyridino[4,3-d]pyrimidin-4-yl)-2,6-diazabicyclo[3.2.0]hept-2-yl)prop-2-en-1-one | 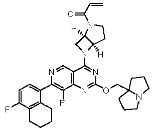 | 587 |
| 30 | 2-fluoro-1-(((1R,SR)-6-(8-fluoro-7-(4-fluoro-5,6,7,8-tetrahydronaphthalen-1-yl)-2-((tetrahydro-1H-pyrrolizin-7a(5H)-yl)methoxy)pyridino[4,3-d]pyrimidin-4-yl)-2,6-diazabicyclo[3.2.0]hept-2-yl)prop-2-en-1-one | 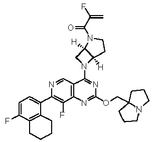 | 605 |
| 31 | 1-(((1R,SR)-6-(8-fluoro-7-(4-fluoro-5,6,7,8-tetrahydronaphthalen-1-yl)-2-((((2R,7aS)-2-fluorotetrahydro-1H-pyrrolizin-7a(5H)-yl)methoxy)pyridino[4,3-d]pyrimidin-4-yl)-2,6-diazabicyclo[3.2.0]hept-2-yl)prop-2-en-1-one | 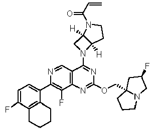 | 605.6 |
| 32 | 2-fluoro-1-(((1R,5R)-6-(8-fluoro-7-(4-fluoro-5,6,7,8-tetrahydronaphthalen-1-yl)-2-((((2R,7aS)-2-fluorotetrahydro-1H-pyrrolizin-7a(5H)-yl)methoxy)pyridino[4,3-d]pyrimidin-4-yl)-2,6-diazabicyclo[3.2.0]hept-2-yl)prop-2-en-1-one | 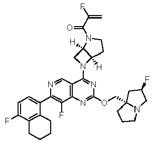 | 623.2 |
| 33 | 2-((S)-1-acryloyl-4-(8-fluoro-2-(((S)-1-methylpyrrolidin-2-yl)methoxy)-7-(5,6,7,8-tetrahydroisoquinoline-4-yl)pyridino[4,3-d]pyrimidin-4-yl)piperazin-2-yl)acetonitrile | 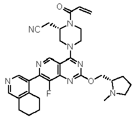 | 571.3 |
| 34 | 2-((S)-4-(8-fluoro-2-(((S)-1-methylpyrrolidin-2-yl)methoxy)-7-(5,6,7,8-tetrahydroisoquinolin-4-yl)pyridino[4,3-d]pyrimidin-4-yl)-1-(2-fluoroacryloyl)piperazin-2-yl)acetonitrile | 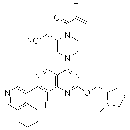 | 589 |
| 35 | 2-((S)-1-acryloyl-4-(8-fluoro-2-((((2S,4R)-4-fluoro-1-methylpyrrolidin-2-yl)methoxy)-7-(5,6,7,8-tetrahydroisoquinolin-4-yl)pyridino[4,3-d]pyrimidin-4-yl)piperazin-2-yl)acetonitrile | 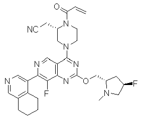 | 589 |
| 36 | 2-((S)-4-(8-fluoro-2-((((2S,4R)-4-fluoro-1-methylpyrrolidin-2-yl)methoxy)-7-(5,6,7,8-tetrahydroisoquinolin-4-yl)pyridino [4,3-d]pyrimidin-4-yl)-1-(2-fluoroacryloyl)piperazin-2-yl)acetonitrile | 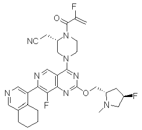 | 607 |
| 37 | (S)-2-(1-acryloyl-4-(8-fluoro-2-((tetrahydro-1H-pyrrolizin-7a(5H)-yl)methoxy)-7-(5,6,7,8-tetrahydroisoquinolin-4-yl)pyridino [4,3-d]pyrimidin-4-yl)piperazin-2-yl)acetonitrile | 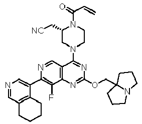 | 597.3 |
| 38 | (S)-2-(4-(8-fluoro-2-((tetrahydro- 1H-pyrrolizin-7a(5H)-yl)methoxy)-7-(5,6,7,8-tetrahydroisoquinolin-4-yl)pyridino [4,3-d]pyrimidin-4-yl)-1-(2-fluoroacryloyl)piperazin-2-yl)acetonitrile | 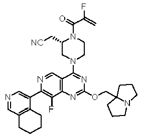 | 615 |
| 39 | 2-((S)-1-acryloyl-4-(8-fluoro-2-((((2R,7aS)-2-fluorotetrahydro-1H-pyrrolizin-7a(5H)-yl)methoxy)-7-(5,6,7,8-tetrahydroisoquinolin-4-yl)pyridino[4,3-d]pyrimidin-4-yl)piperazin-2-yl)acetonitrile | 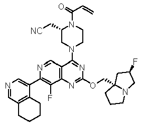 | 615 |
| 40 | 2-((S)-4-(8-fluoro-2-((((2R,7aS)-2-fluorotetrahydro-1H-pyrrolizin-7a(5H)-yl)methoxy)-7-(5,6,7,8-tetrahydroisoquinolin-4-yl)pyridino[4,3-d]pyrimidin-4-yl)-1-(2-fluoroacryloyl)piperazin-2-yl)acetonitrile | 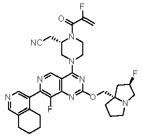 | 633 |
| 41 | 1-((1R,SR)-6-(8-fluoro-2-((((S)-1-methylpyrrolidin-2-yl)methoxy)-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 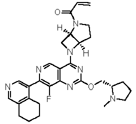 | 544.6 |
| 42 | 2-fluoro-1-(((1R,SR)-6-(8-fluoro-2-(((S)-1-methylpyrrolidin-2-yl)methoxy)-7]-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 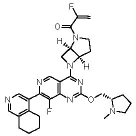 | 562 |
| 43 | 1-((1R,5R)-6-(8-fluoro-2-((((2S,4R)-4-fluoro-1-methylpyrrolidin-2-yl)methoxy)-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 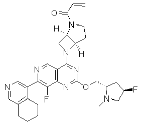 | 562 |
| 44 | 2-fluoro-1-(((1R,SR)-6-(8-fluoro-2-((((2S,4R)-4-fluoro-1-methylpyrrolidin-2-yl)methoxy)-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 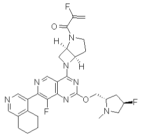 | 580 |
| 45 | 1-((1R,SR)-6-(8-fluoro-2-((tetrahydro-1H-pyrrolizin-7a(5H)-yl)methoxy)-7-(5,6,7,8-tetrahydroisoquinoline-4-yl)pyridino[4,3-d]pyrimidin-4-yl)-2,6-diazabicyclo[3.2.0]hept-2-yl)prop-2-en-1-one | 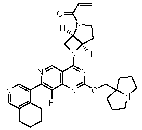 | 570 |
| 46 | 2-fluoro-1-(((1R,SR)-6-(8-fluoro-2-((tetrahydro-1H-pyrrolizin-7a(5H)-yl)methoxy))-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 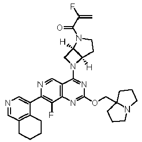 | 588 |
| 47 | 1-(((1R,SR)-6-(8-fluoro-2-((((2R,7aS)-2-fluorotetrahydro-1H-pyrrolizin-7a(5H)-yl)methoxy)-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 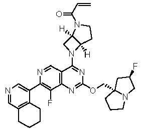 | 588 |
| 48 | 2-fluoro-1-(((1R,SR)-6-(8-fluoro-2-((((2R,7aS)-2-fluorotetrahydro-1H-pyrrolizin-7a(5H)-yl)methoxy)-7-(5,6,7,8-tetrahydroisoquinolin-4-yl)pyridino[4,3-d]pyrimidin-4-yl)-2,6-diazabicyclo[3.2.0]hept-2-yl)prop-2-en-1-one | 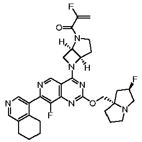 | 606 |

### Example 49: Synthesis of 2-((S)-1-acryloyl-4-(8-fluoro-2-(((S)-1-methylpyrrolidin-2-yl)methoxy)-7-(thiochroman-8-yl)pyridino[4,3-d]pyrimidin-4-yl)piperazin-2-yl)acetonitrile

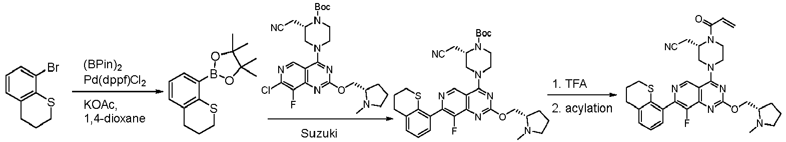

### Step 1: Synthesis of 4,4,5,5-tetramethyl-2-(thiochroman-8-yl)-1,3,2-dioxolane

8-bromo-thiochroman (114 mg, 0.5 mmol), bis(pinacolato)diboron (279 mg, 1.1 mmol), Pd(dppf)Cl₂ (37 mg, 0.05 mmol) and potassium acetate (147 mg, 1.5 mmol) were added to a round-bottomed flask. After displacement with nitrogen, anhydrous 1,4-dioxane (5 mL) which had been deoxidized in advance was added. The resulting suspension was stirred at 100°C for 16 h. After cooling to room temperature, the reaction mixture was extracted with water (20 mL) and methyl tert-butyl ether (20 mL). After layering, the aqueous phase was extracted with methyl tert-butyl ether. The combined organic phases were washed with a saturated aqueous NaCl solution (50 mL), dried with sodium sulfate and spin-dried in vacuo. The remaining dark brown oil was purified by column chromatography (silica gel, ethyl acetate : petroleum ether = 1 : 40) to obtain a colorless viscous material. (42 mg, 0.152 mmol, yield: 30%)

### Step 2: Synthesis of tert-butyl (S)-2-(cyanomethyl)-4-(8-fluoro-2-((((S)-1-methylpyrrolidin-2-yl)methoxy)-7-(thiocyano-8)pyridinyl[4,3-d]pyrimidin-4-yl)piperazine-1-carboxylate

Tert-butyl (S)-4-(7-chloro-8-fluoro-2-(((S)-1-methylpyrrolidin-2-yl)methoxy)pyridino[4,3-d]pyrimidin-4-yl)-2-(cyanomethyl)piperazine-1-carboxylate (52 mg, 0.1 mmol), 4,4,5,5-tetramethyl-2-(thiochroman-8-yl)-1,3,2-dioxolane(42 mg, 0.15 mmol), tetrakis(triphenylphosphine)palladium (46 mg, 0.4 mmol) and cesium carbonate (98 mg, 0.3 mmol) were added to a round-bottomed flask. After displacement with nitrogen, 1,4-dioxane (2 mL) and water (0.4 mL) which had been deoxidized in advance was added. The reaction liquid was stirred at 100°C for 3 h. Ethyl acetate (15 mL) and water (15 mL) were added. After shaking and layering, the aqueous phase was extracted with ethyl acetate (10 mL × 2). The combined organic phases were washed with a saturated aqueous NaCl solution (10 mL), dried with sodium sulfate and spin-dried in vacuo. The remaining brown viscous material was purified by prep-TLC (silica gel, methanol : dichloromethane = 1 : 9) to obtain a light yellow solid. (32 mg, 0.05 mmol, yield: 50%) MS m/z: 634.7 [M+H]⁺.

### Step 3: Synthesis of 2-((S)-4-(8-fluoro-2-(((S)-1-methylpyrrolidin-2-yl)methoxyl-7-(thiochroman-8-yl)pyridino[4,3-d]pyrimidin-4-yl)piperazin-2-yl)acetonitrile

Trifluoroacetic acid (1 mL) was added dropwise to a solution of tert-butyl (S)-2-(cyanomethyl)-4-(8-fluoro-2-((((S)-1-methylpyrrolidin-2-yl)methoxy)-7-(thiocyano-8)pyridinyl[4,3-d]pyrimidin-4-yl)piperazine-1-carboxylate (32 mg, 0.05 mmol) in dichloromethane (3 mL) at room temperature. The resulting solution was stirred at room temperature for 1 h. Dichloromethane (7 mL) was added and concentration in vacuo was performed. Dichloromethane (5 mL) was added to the resulting residue, concentration was performed again, and this process was repeated once. The resulting yellow solid was directly used for the next step.

### Step 4: Synthesis of 2-((S)-1-acryloyl-4-(8-fluoro-2-(((S)-1-methylpyrrolidin-2-yl)methoxy)-7-(thiochroman-8-yl)pyridino[4,3-d]pyrimidin-4-yl)piperazin-2-yl)acetonitrile

A solution of acryloyl chloride (0.01 mL, 0.122 mmol) in dichloromethane (1 mL) was added dropwise to a solution of 2-((S)-4-(8-fluoro-2-(((S)-1-methylpyrrolidin-2-yl)methoxy)-7-(thiochroman-8-yl)pyridino[4,3-d]pyrimidin-4-yl)piperazin-2-yl)acetonitrile and triethylamine (0.05 mL, 0.35 mmol) in dichloromethane (2 mL) at room temperature. The reaction liquid was stirred at room temperature for 15 min. Dichloromethane (20 mL) and a saturated sodium carbonate aqueous solution (20 mL) were added, and after shaking and separation, the aqueous phase was extracted with dichloromethane (10 mL). The organic phases were combined, washed with a saturated aqueous NaCl solution (10 mL), dried with sodium sulfate and subjected to rotary evaporation to obtain a viscous material. Purification by prep-TLC (methanol : dichloromethane = 1 : 8) gave a white solid (17 mg, overall yield over two steps: 57%). MS m/z: 588.6 [M+H]⁺. ¹H NMR (400 MHz, CDCl3) δ 9.11 (s, 1H), 7.27 (t, *J =* 7.7 Hz, 1H), 7.16-7.12 (m, 2H), 6.66-6.56 (m, 1H), 6.42 (d, *J =* 16 Hz, 1H), 5.86 (d, *J =* 12 Hz, 1H), 5.10-4.98 (m, 2H), 4.65 - 4.60 (m, 1H), 4.52-4.40 (m, 2H), 4.10 - 4.07 (m, 1H), 3.85 - 3.81 (m, 1H), 3.72 - 3.25 (m, 4H), 3.06-2.78 (m, 10 H), 2.32 - 1.98 (m, 3H), 1.80-1.55 (m, 3H).

### Example 50: Synthesis of 2-((S)-1-acryloyl-4-(8-fluoro-7-(isochroman-5-yl)-2-(((S)-1-methylpyrrolidin-2-yl)methoxy)pyridino[4,3-d]pyrimidin-4-yl)piperazin-2-yl)acetonitrile

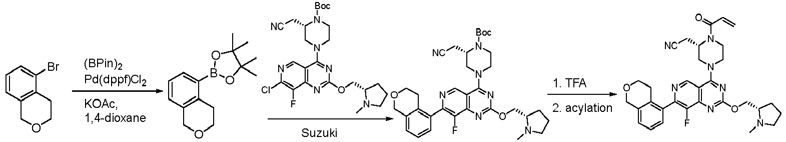

### Step 1: Synthesis of 2-(isochroman-5-yl)-4,4,5,5-tetramethyl-1,3,2-dioxolane

5-bromoisochroman (198 mg, 0.93 mmol), bis(pinacolato)diboron (709 mg, 2.8 mmol), Pd(dppf)Cl₂ (102 mg, 0.14 mmol) and potassium acetate (274 mg, 2.8 mmol) were added to a round-bottomed flask. After displacement with nitrogen, anhydrous 1,4-dioxane (10 mL) which had been deoxidized in advance was added. The resulting suspension was stirred at 100°C for 16 h. After cooling to room temperature, the reaction mixture was extracted with water (20 mL) and methyl tert-butyl ether (20 mL). After layering, the aqueous phase was extracted with methyl tert-butyl ether. The combined organic phases were washed with a saturated aqueous NaCl solution (50 mL), dried with sodium sulfate and spin-dried in vacuo. The remaining dark brown oil was purified by column chromatography (silica gel, ethyl acetate : petroleum ether = 1 : 30) to obtain a colorless oil. (196 mg, 0.754 mmol, yield: 81%)

### Step 2: Synthesis of tert-butyl (S)-2-(cyanomethyl)-4-(8-fluoro-7-(isochroman-5-yl)-2-(((S)-1-methylpyrrolidin-2-yl)methoxy)pyridino[4,3-d]pyrimidin-4-yl)piperazine-1-carboxylate

Tert-butyl (S)-4-(7-chloro-8-fluoro-2-(((S)-1-methylpyrrolidin-2-yl)methoxy)pyridino[4,3-d]pyrimidin-4-yl)-2-(cyanomethyl)piperazine-1-carboxylate (55 mg, 0.106 mmol), 2-(isochroman-5-yl)-4,4,5,5-tetramethyl-1,3,2-dioxolane(52 mg, 0.2 mmol), tetrakis(triphenylphosphine)palladium (49 mg, 0.0424 mmol) and cesium carbonate (104 mg, 0.32 mmol) were added to a round-bottomed flask. After displacement with nitrogen, 1,4-dioxane (2 mL) and water (0.4 mL) which had been deoxidized in advance was added. The reaction liquid was stirred at 100°C for 3 h. Ethyl acetate (15 mL) and water (15 mL) were added. After shaking and layering, the aqueous phase was extracted with ethyl acetate (10 mL × 2). The combined organic phases were washed with a saturated aqueous NaCl solution (10 mL), dried with sodium sulfate and spin-dried in vacuo. The remaining brown viscous material was purified by prep-TLC (silica gel, methanol : dichloromethane = 1 : 9) to obtain a light yellow powder. (16 mg, 0.0259 mmol, yield: 24%) MS m/z: 618.7 [M+H]⁺.

### Step 3: Synthesis of 2-((S)-4-(8-fluoro-7-(isochroman-5-yl)-2-((S)-1-methylpyrrolidin-2-yl)methoxy)pyridino[4,3-d]pyrimidin-4-yl)piperazin-2-yl)acetonitrile

Trifluoroacetic acid (1 mL) was added dropwise to a solution of tert-butyl (S)-2-(cyanomethyl)-4-(8-fluoro-7-(isochroman-5-yl)-2-(((S)-1-methylpyrrolidin-2-yl)methoxy)pyridino[4,3-d]pyrimidin-4-yl)piperazine-1-carboxylate (16 mg, 0.0259 mmol) in dichloromethane (3 mL) at room temperature. The resulting solution was stirred at room temperature for 1 h. Dichloromethane (7 mL) was added and concentration in vacuo was performed. Dichloromethane (5 mL) was added to the resulting residue, concentration was performed again, and this process was repeated once. The resulting yellow solid was directly used for the next step.

### Step 4: Synthesis of 2-((S)-1-acryloyl-4-(8-fluoro-7-(isochroman-5-yl)-2-(((S)-1-methylpyrrolidin-2-yl)methoxy)pyridino[4,3-d]pyrimidin-4-yl)piperazin-2-yl)acetonitrile

A solution of acryloyl chloride (0.01 mL, 0.122 mmol) in dichloromethane (1 mL) was added dropwise to a solution of 2-((S)-4-(8-fluoro-7-(isochroman-5-yl)-2-((S)-1-methylpyrrolidin-2-yl)methoxy)pyridino[4,3-d]pyrimidin-4-yl)piperazin-2-yl)acetonitrile and triethylamine (0.06 mL, 0.42 mmol) in dichloromethane (2 mL) at room temperature. The reaction liquid was stirred at room temperature for 15 min. Dichloromethane (20 mL) and a saturated sodium carbonate aqueous solution (20 mL) were added, and after shaking and separation, the aqueous phase was extracted with dichloromethane (10 mL). The organic phases were combined, washed with a saturated aqueous NaCl solution (10 mL), dried with sodium sulfate and subjected to rotary evaporation to obtain a viscous material. Purification by prep-TLC (methanol : dichloromethane = 1 : 9) gave a white solid (3 mg, 0.0052 mmol, overall yield over two steps: 20%). MS m/z: 572.7 [M+H]⁺. ¹H NMR (400 MHz, CDCl3) δ 9.11 (s, 1H), 7.34-7.32 (m, 2H), 7.13 (d, *J=* 4.0 Hz, 1H), 6.66-6.56 (m, 1H), 6.42 (d, *J=* 16 Hz, 1H), 5.86 (d, *J=* 12 Hz, 1H), 5.10-4.98 (m, 2H), 4.89 (s, 2H), 4.56-4.40 (m, 3H), 4.10 - 4.14 (m, 1H), 3.94 (t, *J =* 8.0 Hz, 2 H), 3.85 - 3.81 (m, 1H), 3.72 - 3.25 (m, 2H), 3.06-3.00 (m, 1H), 2.80-2.52 (m, 7 H), 2.32 - 1.98 (m, 3H), 1.80-1.55 (m, 3H).

### Example 51: Synthesis of 2-((S)-1-acryloyl-4-(7-(benzo[b]thien-7-yl)-8-fluoro-2-(((S)-1-methylpyrrolidin-2-yl)methoxy)pyridino[4.3-d]pyrimidin-4-yl)piperazin-2-yl)acetonitrile

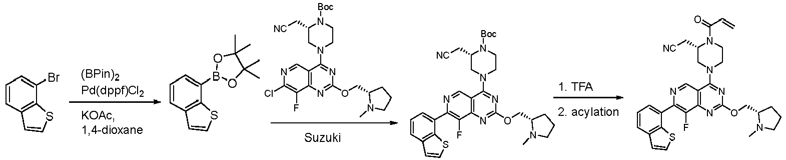

### Step 1: Synthesis of 2-(benzothien-7-yl)-4,4,5,5-tetramethyl-1,3,2-dioxolane

7-bromobenzo[b]thiophene (150 mg, 0.7 mmol), bis(pinacolato)diboron (533 mg, 2.1 mmol), Pd(dppf)Cl₂(102 mg, 0.14 mmol) and potassium acetate (206 mg, 2.1 mmol) were added to a round-bottomed flask. After displacement with nitrogen, anhydrous 1,4-dioxane (5 mL) which had been deoxidized in advance was added. The resulting suspension was stirred at 100°C for 16 h. After cooling to room temperature, the reaction mixture was extracted with water (20 mL) and methyl tert-butyl ether (20 mL). After layering, the aqueous phase was extracted with methyl tert-butyl ether. The combined organic phases were washed with a saturated aqueous NaCl solution (50 mL), dried with sodium sulfate and spin-dried in vacuo. The remaining dark brown oil was purified by column chromatography (silica gel, ethyl acetate : petroleum ether = 1 : 40) to obtain a colorless viscous material. (160 mg, 0.615 mmol, yield: 99%)

### Step 2: Synthesis of tert-butyl (S)-4-(7-(benzothien-7-yl)-8-fluoro-2-(((S)-1-methylpyrrolidin-2-yl)methoxyl)pyridino[4,3-d]pyrimidin-4-yl)-2-(cyanomethyl)piperazine-1-carboxylate

Tert-butyl (S)-4-(7-chloro-8-fluoro-2-(((S)-1-methylpyrrolidin-2-yl)methoxy)pyridino[4,3-d]pyrimidin-4-yl)-2-(cyanomethyl)piperazine-1-carboxylate (26 mg, 0.05 mmol), 2-(benzothien-7-yl)-4,4,5,5-tetramethyl-1,3,2-dioxolane (26 mg, 0.1 mmol), tetrakis(triphenylphosphine)palladium (23 mg, 0.02 mmol) and cesium carbonate (29 mg, 0.15 mmol) were added to a round-bottomed flask. After displacement with nitrogen, 1,4-dioxane (2 mL) and water (0.4 mL) which had been deoxidized in advance was added. The reaction liquid was stirred at 100°C for 3 h. Ethyl acetate (15 mL) and water (15 mL) were added. After shaking and layering, the aqueous phase was extracted with ethyl acetate (10 mL × 2). The combined organic phases were washed with a saturated aqueous NaCl solution (10 mL), dried with sodium sulfate and spin-dried in vacuo. The remaining brown viscous material was purified by prep-TLC (silica gel, methanol : dichloromethane = 1 : 9) to obtain a light yellow solid. (16 mg, 0.0259 mmol, yield: 52%) MS m/z: 618.7 [M+H]⁺.

### Step 3: Synthesis of 2-((S)-4-(7-(benzo[b]thien-7-yl)-8-fluoro-2-(((S)-1-methylpyrrolidin-2-yl)methoxy)pyridino[4,3-d]pyrimidin-4-yl)piperazin-2-yl)acetonitrile

Trifluoroacetic acid (1 mL) was added dropwise to a solution of tert-butyl (S)-4-(7-(benzothien-7-yl)-8-fluoro-2-(((S)-1-methylpyrrolidin-2-yl)methoxy)pyridino[4,3-d]pyrimidin-4-yl)-2-(cyanomethyl)piperazine-1-carboxylate (16 mg, 0.0259 mmol) in dichloromethane (3 mL) at room temperature. The resulting solution was stirred at room temperature for 1 h. Dichloromethane (10 mL) was added and concentration in vacuo was performed. Dichloromethane (5 mL) was added to the resulting residue, concentration was performed again, and this process was repeated once. The resulting yellow solid was directly used for the next step.

### Step 4: Synthesis of 2-((S)-1-acryloyl-4-(7-(benzo[b]thien-7-yl)-8-fluoro-2-(((S)-1-methylpyrrolidin-2-yl)methoxy)pyridino[4,3-d]pyrimidin-4-yl)piperazin-2-yl)acetonitrile

A solution of acryloyl chloride (0.01 mL, 0.122 mmol) in dichloromethane (1 mL) was added dropwise to a solution of 2-((S)-4-(7-(benzo[b]thien-7-yl)-8-fluoro-2-(((S)-1-methylpyrrolidin-2-yl)methoxy)pyridino[4,3-d]pyrimidin-4-yl)piperazin-2-yl)acetonitrile and triethylamine (0.06 mL, 0.42 mmol) in dichloromethane (2 mL) at room temperature. The reaction liquid was stirred at room temperature for 15 min. Dichloromethane (20 mL) and a saturated sodium carbonate aqueous solution (20 mL) were added, and after shaking and separation, the aqueous phase was extracted with dichloromethane (10 mL). The organic phases were combined, washed with a saturated aqueous NaCl solution (10 mL), dried with sodium sulfate and subjected to rotary evaporation to obtain a viscous material. Purification by prep-TLC (methanol : dichloromethane = 1 : 8) gave a light yellow powder (10 mg, 0.0175 mmol, overall yield over two steps: 67%). MS m/z: 572.6 [M+H]⁺. ¹H NMR (400 MHz, CDCl3) δ 9.24 (s, 1H), 8.12 (d, *J* = 8.0 Hz, 1H), 7.97 (d, *J =* 8.0 Hz, 1H), 7.57-7.53 (m, 2H), 7.45 (d, *J* = 4.0 Hz, 1H), 6.66 -6.56 (m, 1H), 6.40 (d, *J =* 16 Hz, 1H), 5.86 (d, *J =* 12 Hz, 1H), 5.10-4.98 (m, 2H), 4.67-4.65 (m, 1H), 4.57-4.49 (m, 2H), 4.10 - 4.14 (m, 1H), 3.89 - 3.85 (m, 1H), 3.70 - 3.30 (m, 2H), 2.86 (s, 3H), 2.75-2.65 (m, 1H), 2.32 - 1.70 (m, 8H).

### Example 52: Synthesis of 2-((2S)-1-acryloyl-4-(8-fluoro-2-(((S)-1-methylpyrrolidin-2-yl)methoxy)-7-(1,1a,6,6a-tetrahydrocyclonropa[a]inden-2-yl)pyridino[4,3-d]pyrimidin-4-yl)piperazin-2-yl)acetonitrile

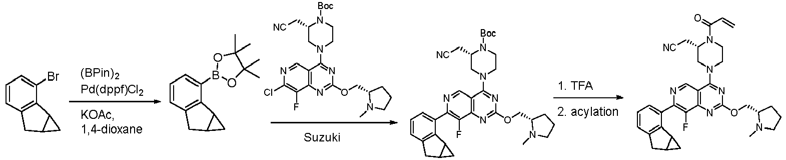

### Step 1: Synthesis of 4,4,5,5-tetramethyl-2-(1,1a,6,6a-tetrahydrocyclopropa[a]inden-2-yl)-1,3,2= dioxolane

2-Bromo-1,1a,6,6a-tetrahydrocyclopropa[a]indene (180 mg, 0.86 mmol), bis(pinacolato)diboron (655 mg, 2.58 mmol), Pd(dppf)Cl₂(126 mg, 0.172 mmol) and potassium acetate (253 mg, 2.58 mmol) were added to a round-bottomed flask. After displacement with nitrogen, anhydrous 1,4-dioxane (6 mL) which had been deoxidized in advance was added. The resulting suspension was stirred at 100°C for 16 h. After cooling to room temperature, the reaction mixture was extracted with water (20 mL) and methyl tert-butyl ether (20 mL). After layering, the aqueous phase was extracted with methyl tert-butyl ether. The combined organic phases were washed with a saturated aqueous NaCl solution (50 mL), dried with sodium sulfate and spin-dried in vacuo. The remaining dark brown oil was purified by column chromatography (silica gel, ethyl acetate : petroleum ether = 1 : 40) to obtain a colorless viscous material. (101 mg, 0.395 mmol, yield: 46%)

### Step 2: Synthesis of tert-butyl (2S)-2-(cyanomethyl)-4-(8-fluoro-2-((((S)-1-methylpyrrolidin-2-yl)methoxy)-7-(1,1a,6,6a-tetrahydrocyclopropa[a]inden-2-yl)pyridino[4,3-d]pyrimidin-4-yl)piperazine-1-carboxylate

Tert-butyl (S)-4-(7-chloro-8-fluoro-2-(((S)-1-methylpyrrolidin-2-yl)methoxy)pyridino[4,3-d]pyrimidin-4-yl)-2-(cyanomethyl)piperazine-1-carboxylate (30 mg, 0.058 mmol), 4,4,5,5-tetramethyl-2-(1,1a,6,6a-tetrahydrocyclopropa[a]inden-2-yl)-1,3,2-dioxolane(26 mg, 0.104 mmol), tetrakis(triphenylphosphine)palladium (27 mg, 0.023 mmol) and cesium carbonate (57 mg, 0.174 mmol) were added to a round-bottomed flask. After displacement with nitrogen, 1,4-dioxane (2 mL) and water (0.4 mL) which had been deoxidized in advance was added. The reaction liquid was stirred at 100°C for 3 h. Ethyl acetate (15 mL) and water (15 mL) were added. After shaking and layering, the aqueous phase was extracted with ethyl acetate (10 mL × 2). The combined organic phases were washed with a saturated aqueous NaCl solution (10 mL), dried with sodium sulfate and spin-dried in vacuo. The remaining brown viscous material was purified by prep-TLC (silica gel, methanol : dichloromethane = 1 : 9) to obtain a light yellow solid. (15 mg, 0.0244 mmol, yield: 42%) MS m/z: 614.7 [M+H]⁺.

### Step 3: Synthesis of 2-((2S)-4-(8-fluoro-2-(((S)-1-methylpyrrolidin-2-yl)methoxy)-7-(1,1a,6,6a-tetrahydrocyclopropa[a]inden-2-yl)pyridino[4,3-d]pyrimidin-4-yl)piperazin-2-yl)acetonitrile

Trifluoroacetic acid (1 mL) was added dropwise to a solution of tert-butyl (2S)-2-(cyanomethyl)-4-(8-fluoro-2-((((S)-1-methylpyrrolidin-2-yl)methoxy)-7-(1,1a,6,6a-tetrahydrocyclopropa[a]inden-2-yl)pyridino[4,3-d]pyrimidin-4-yl)piperazine-1-carboxylate (15 mg, 0.0244 mmol) in dichloromethane (3 mL) at room temperature. The resulting solution was stirred at room temperature for 1 h. Dichloromethane (10 mL) was added and concentration in vacuo was performed. Dichloromethane (5 mL) was added to the resulting residue, concentration was performed again, and this process was repeated once. The resulting yellow solid was directly used for the next step.

### Step 4: Synthesis of 2-((2S)-1-acryloyl-4-(8-fluoro-2-(((S)-1-methylpyrrolidin-2-yl)methoxy)-7-(1,1a,6,6a-tetrahydrocyclopropa[a]inden-2-yl)pyridino[4,3-d]pyrimidin-4-yl)piperazin-2-yl)acetonitrile

A solution of acryloyl chloride (0.005 mL, 0.061 mmol) in dichloromethane (1 mL) was added dropwise to a solution of 2-((2S)-4-(8-fluoro-2-(((S)-1-methylpyrrolidin-2-yl)methoxy)-7-(1,1a,6,6a-tetrahydrocyclopropa[a]inden-2-yl)pyridino[4,3-d]pyrimidin-4-yl)piperazin-2-yl)acetonitrile and triethylamine (0.03 mL, 0.21 mmol) in dichloromethane (2 mL) at room temperature. The reaction liquid was stirred at room temperature for 15 min. Dichloromethane (20 mL) and a saturated sodium carbonate aqueous solution (20 mL) were added, and after shaking and separation, the aqueous phase was extracted with dichloromethane (10 mL). The organic phases were combined, washed with a saturated aqueous NaCl solution (10 mL), dried with sodium sulfate and subjected to rotary evaporation to obtain a viscous material. Purification by prep-TLC (methanol: dichloromethane = 1 : 8) gave a light yellow powder (10 mg, 0.0176 mmol, overall yield over two steps: 72%). MS m/z: 568.6 [M+H]⁺. ¹H NMR (400 MHz, CDCl3) δ 9.09 (s, 1H), 7.42 (d, *J* = 8.0 Hz, 1H), 7.37-7.35 (m, 2H), 6.66 -6.56 (m, 1H), 6.42 (d, *J =* 16 Hz, 1H), 5.86 (d, *J* = 12 Hz, 1H), 5.04-4.98 (m, 2H), 4.61 - 4.45 (m, 3H), 4.11 - 4.07 (m, 1H), 3.81 - 3.76 (m, 1H), 3.60 - 3.38 (m, 1H), 3.34-3.24 (m, 2 H), 3.06-2.97 (m, 2H), 2.83 (s, 3H), 2.75-2.70 (m, 1H), 2.48-2.44 (m, 1H), 2.32 - 1.80 (m, 8H), 1.10-1.04 (m, 1H), 0.15-0.11 (m, 1H).

The compounds of Examples 53-72 were prepared by preparation method 1

| Ex. | Compound name | Structural formula | m/z: ES⁺[M+H] |
|---|---|---|---|
| 53 | 2-((S)-4-(8-fluoro-2-(((S)-1-methylpyrrolidin-2-yl)methoxy)-7-(thiochroman-8-yl)pyridino[4,3-d]pyrimidin-4-yl)-1-(2-fluoroacryloyl)piperazin-2-yl)acetonitrile | 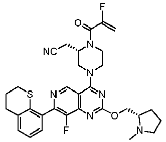 | 606.6 |
| 54 | 2-((S)-1-acryloyl-4-(8-fluoro-7-(5-fluorothiochroman-8-yl)-2-(((S)-1-methylpyrrolidin-2-yl)methoxy)pyridino[4,3-d]pyrimidin-4-yl)piperazin-2-yl)acetonitrile | 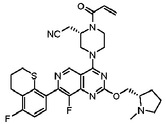 | 606.6 |
| 55 | 2-((2S)-4-(8-fluoro-2-(((S)-1-methylpyrrolidin-2-yl)methoxy)-7-(1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 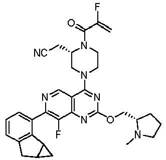 | 586.7 |
| 56 | 2-((S)-1-acryloyl-4-(8-fluoro-2-(((S)-1-methylpyrrolidin-2-yl)methoxy)-7-((1aS,6aS)-1,1a,6,6a-tetrahydrocyclopropa[a]inden-2-yl)pyridino[4,3-d]pyrimidin-4-yl)piperazin-2-yl)acetonitrile | 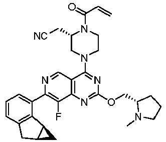 | 568.7 |
| 57 | 2-((S)-4-(8-fluoro-2-(((S)-1-methylpyrrolidin-2-yl)methoxy)-7-((1aS,6aS)-1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 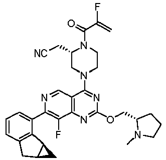 | 586.7 |
| 58 | 2-((S)-1-acryloyl-4-(8-fluoro-2-(((S)-1-methylpyrrolidin-2-yl)methoxy)-7-((1aR,6aR)-1,1a,6,6a-tetrahydrocyclopropa[a]inden-2-yl)pyridino[4,3-d]pyrimidin-4-yl)piperazin-2-yl)acetonitrile | 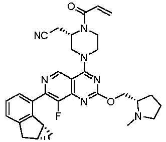 | 568.7 |
| 59 | 2-((S)-4-(8-fluoro-2-(((S)-1-methylpyrrolidin-2-yl)methoxy)-7-((1aR,6aR)-1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 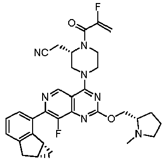 | 586.7 |
| 60 | 2-((2S)-1-acryloyl-4-(8-fluoro-7-(2-methyl-2,3-dihydro-1H-inden-4-yl)-2-(((S)-1-methylpyrrolidin-2-yl)methoxy)pyridino[4,3-d]pyrimidin-4-yl)piperazin-2-yl)acetonitrile | 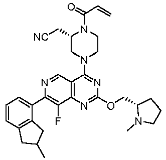 | 570.3 |
| 61 | 2-((2S)-1-acryloyl-4-(8-fluoro-2-((tetrahydro-1H-pyrrolizin-7a(5H-yl)methoxy)-7-(1,1a,6,6a-tetrahydrocyclopropa[a]inden-2-yl)pyridino[4,3-d]pyrimidin-4-yl)piperazin-2-yl)acetonitrile | 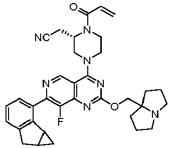 | 594.3 |
| 62 | 2-((2S)-4-(8-fluoro-2-((tetrahydro-1H-pyrrolizin-7a(5H)-yl)methoxy)-7-(1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 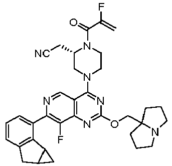 | 612.3 |
| 63 | 2-((S)-1-acryloyl-4-(8-fluoro-2-((tetrahydro-1H-pyrrolizin-7a(5H)-yl)methoxy)-7-((1aS,6aS)-1,1a,6,6a-tetrahydrocyclopropa[a]inden-2-yl)pyridino[4,3-d]pyrimidin-4-yl)piperazin-2-yl)acetonitrile | 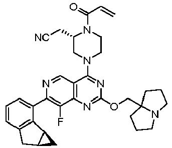 | 594.3 |
| 64 | 2-((S)-4-(8-fluoro-2-((tetrahydro-1H-pyrrolizin-7a(5H)-yl)methoxy)-7-((1aS,6aS)-1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 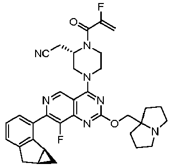 | 612.3 |
| 65 | 2-((S)-1-acryloyl-4-(8-fluoro-2-((tetrahydro-1H-pyrrolizin-7a(5H-yl)methoxy)-7-((1aR,6aR)-1,1a,6,6a-tetrahydrocyclopropa[a]inden-2-yl)pyridino[4,3-d]pyrimidin-4-yl)piperazin-2-yl)acetonitrile | 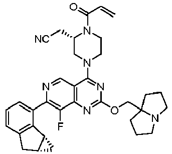 | 594.3 |
| 66 | 2-((S)-4-(8-fluoro-2-((tetrahydro-1H-pyrrolizin-7a(5H)-yl)methoxy)-7-((1aR,6aR)-1,1a,6,6a-tetrahydrocyclopropa[a]inden-2-yl)pyridino[4,3-d]pyrimidin-4-yl)-1-(2-fluoroacryloyl)piperazin-2-yl)acetonitrile | 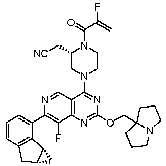 | 612.3 |
| 67 | 2-(((2S)-1-acryloyl-4-(8-fluoro-2-((((2R,7aS)-2-fluorotetrahydro-1H-pyrrolizin-7a(5H)-yl)methoxy)-7-(1,1a,6,6a-tetrahydrocyclopropa[a]indan-2-yl)pyridinyl[4,3-d]pyrimidin-4-yl)piperazin-2-yl)acetonitrile | 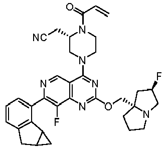 | 612.3 |
| 68 | 2-((2S)-4-(8-fluoro-2-((((2R,7aS)-2-fluorotetrahydro-1H-pyrrolizine-7a(5H)-yl)methoxy)-7-(1,1a,6,6a-tetrahydrocyclopropa[a]indan-2-yl)pyridinyl[4,3-d]pyrimidin-4-yl)-1-(2-fluoroacryloyl)piperazin-2-yl)acetonitrile | 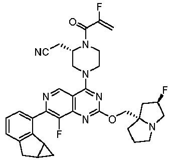 | 630.3 |
| 69 | 2-((S)-1-acryloyl-4-(8-fluoro-2-((((2R,7aS)-2-fluorotetrahydro-1H-pyrrolizin-7a(5H)-yl)methoxy)-7-(((1aS,6aS)-1,1a,6,6a-tetrahydrocyclopropa[a]indan-2-yl)pyridinyl[4,3-d]pyrimidin-4-yl)piperazin-2-yl)acetonitrile | 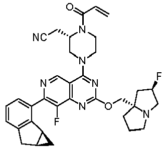 | 612.3 |
| 70 | 2-((S)-4-(8-fluoro-2-((((2R,7aS)-2-fluorotetrahydro-1H-pyrrolizin-7a(5H)-yl)methoxy)-7-((1aS,6aS)-1,1a,6,6a-tetrahydrocyclopropa[a]indan-2-yl)pyridinyl[4,3-d]pyrimidin-4-yl)-1-(2-fluoroacryloyl)piperazin-2-yl)acetonitrile | 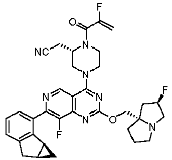 | 630.3 |
| 71 | 2-((S)-1-acryloyl-4-(8-fluoro-2-((((2R,7aS)-2-fluorotetrahydro-1H-pyrrolizin-7a(5H)-yl)methoxy)-7-(((1aR,6aR)-1,1a,6,6a-tetrahydrocyclopropa[a]indan-2-yl)pyridinyl[4,3-d]pyrimidin-4-yl)piperazin-2-yl)acetonitrile | 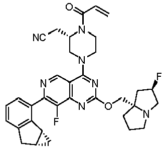 | 612.3 |
| 72 | 2-((S)-4-(8-fluoro-2-((((2R,7aS)-2-fluorotetrahydro-1H-pyrrolizine-7a(5H)-yl)methoxy)-7-((1aR,6aR)-1,1a,6,6a-tetrahydrocyclopropa[a]indan-2-yl)pyridinyl[4,3-d]pyrimidin-4-yl)-1-(2-fluoroacryloyl)piperazin-2-yl)acetonitrile | 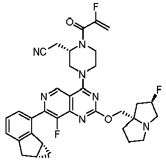 | 630.3 |

### Example 73: Synthesis of 2-((S)-1-acryloyl-4-(7-(8-chloronaphthalen-1-yl)-2-(((S)-1-methylpyrrolidin-2-yl)methoxy)quinazolin-4-yl)piperazin-2-yl)acetonitrile

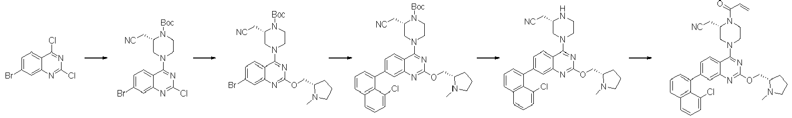

### Step 1: Synthesis of tert-butyl (S)-4-(7-bromo-2-chloroquinazolin-4-yl)-2-(cyanomethyl)piperazine-1-carboxylate

The compound 7-bromo-2,4-dichloroquinazoline (200 mg, 0.72 mmol) was dissolved in DMF (5 mL), and DIEA (465 mg, 3.60 mmol) and (S)-2-(piperazin-2-yl)acetonitrile dihydrochloride (142 mg, 0.72 mmol) were then added and reacted at room temperature under stirring for 30 min. Di-tert butyl dicarbonate (472 mg, 2.16 mmol) was then added, and the mixture was heated to 60° C and reacted under stirring for 16 h. After the reaction was complete, the reaction liquid was diluted by adding 20 mL of cold water under stirring and extracted with ethyl acetate, and the organic phase was washed with a saturated aqueous NaCl solution, dried with anhydrous sodium sulfate, concentrated and separated by column chromatography to obtain an off-white solid. (200 mg, yield: 60%). ¹H NMR (400 MHz, CDCl₃) δ 8.06 (d, *J =* 1.9 Hz, 1H), 7.78 (d, *J =* 8.9 Hz, 1H), 7.60 (dd, *J* = 8.9, 1.9 Hz, 1H), 4.68 (s, 1H), 4.37 (d, *J =* 13.3 Hz, 1H), 4.27 (d, *J =* 11.7 Hz, 1H), 4.15 (d, *J* = 7.1 Hz, 1H), 3.71 (dd, *J =* 13.6, 3.7 Hz, 1H), 3.59 - 3.48 (m, 1H), 3.42 (s, 1H), 2.87 (s, 1H), 2.75 (s, 1H), 1.54 (s, 9H).

### Step 2: Synthesis of tert-butyl (S)-4-(7-bromo-2-(((S)-1-methylpyrrolidin-2-yl)methoxy)quinazolin-4-yl)-2-(cyanomethyl)piperazine-1-carboxylate

The compound (S)-(1-methylpyrrolidin-2-yl)methanol (149 mg, 1.29 mmol) was dissolved in anhydrous tetrahydrofuran (5 mL), 60% NaH (52 mg, 1.29 mmol) was added under ice-water bath cooling condition and reacted at room temperature under stirring for 20 min, and tert-butyl (S)-4-(7-bromo-2-chloroquinazolin-4-yl)-2-(cyanomethyl)piperazine-1-carboxylate (200 mg, 0.43 mmol) was added and reacted at room temperature under stirring for 1 h. After the reaction was complete, the reaction was quenched with cold water, extracted with ethyl acetate, and the organic phase was washed with a saturated aqueous NaCl solution, dried with anhydrous sodium sulfate, concentrated and separated by column chromatography to obtain an off-white solid. (200 mg, yield: 85%)

### Step 3: Synthesis of tert-butyl (S)-4-(7-(8-chloronaphthalen-1-yl)-2-(((S)-1-methylpyrrolidin-2-yl)methoxy)quinazolin-4-yl)-2-cyanomethyl)piperazine-1-carboxylate

The compound tert-butyl (S)-4-(7-bromo-2-(((S)-1-methylpyrrolidin-2-yl)methoxy)quinazolin-4-yl)-2-(cyanomethyl)piperazine-1-carboxylate (30 mg, 0.06 mmol) and 2-(8-chloronaphthalen-1-yl)-4,4,5,5-tetramethyl-1,3,2-dioxaborane (19 mg, 0.07) were dissolved in dioxane/water = 5/1 (3 mL), cesium carbonate (54 mg, 0.17 mmol) and Pd(PPh₃)₄ (30 mg, 0.03 mmol) were then added, and after displacement with nitrogen, the mixture was heated to 90°C and reacted under stirring for 1 h. After the reaction was complete, the reaction product was cooled to room temperature, the reaction liquid was diluted by adding water and extracted with ethyl acetate, and the organic phase was dried with anhydrous sodium sulfate, concentrated, separated and purified by TLC to obtain an off-white solid. (28 mg, yield: 80%).

¹H NMR (300MHz, CDCl₃) δ 7.96 (dd,J = 8.2, 1.1Hz, 1H), 7.90 (dd,J = 8.1, 1.1Hz, 1H), 7.82 (d,J = 8.5Hz, 1H), 7.75 (t,J = 1.7Hz, 1H), 7.60 - 7.52 (m, 2H), 7.45 (dd,J = 5.6, 2.7Hz, 1H), 7.33 (dd,J = 8.5, 1.6Hz, 1H), 7.23 (d,J = 7.6Hz, 1H), 4.86 (d,J = 5.8Hz, 1H), 4.71 (s, 1H), 4.57 (d,J = 11.5Hz, 1H), 4.47 - 4.25 (m, 2H), 4.15 (s, 1H), 3.64 - 3.22 (m, 4H), 3.01 - 2.55 (m, 6H), 2.25 (d,J = 6.3Hz, 1H), 2.18 - 1.89 (m, 3H), 1.54 (s, 9H).

### Step 4: Synthesis of 2-((S)-4-(7-(8-chloronaphthalen-1-yl)-2-(((S)-1-methylpyrrolidin-2-yl)methoxyquinazolin-4-yl)piperazin-2-yl)acetonitrile

The compound tert-butyl (S)-4-(7-(8-chloronaphthalen-1-yl)-2-(((S)-1-methylpyrrolidin-2-yl)methoxy)quinazolin-4-yl)-2-cyanomethyl)piperazine-1-carboxylate (28 mg, 0.04 mmol) was dissolved in dichloromethane (3 mL), trifluoroacetic acid (1 mL) was then added, and the mixture was reacted at room temperature under stirring for 1 h. After the reaction was complete, the reaction liquid was concentrated, then dissolved in dichloromethane, adjusted to pH 8-9 with a saturated sodium carbonate aqueous solution and extracted with dichloromethane, and the organic phase was dried with anhydrous sodium sulfate and concentrated to obtain a crude product, which was directly used for the next step. (23 mg, yield: 100%).

### Step 5: Synthesis of 2-((S)-1-acryloyl-4-(7-(8-chloronaphthalen-1-yl)-2-(((S)-1-methylpyrrolidin-2-yl)methoxy)quinazolin-4-yl)piperazin-2-yl)acetonitrile

The compound 2-((S)-4-(7-(8-chloronaphthalen-1-yl)-2-(((S)-1-methylpyrrolidin-2-yl)methoxy)quinazolin-4-yl)piperazin-2-yl)acetonitrile (23 mg, 0.04 mmol) was dissolved in anhydrous dichloromethane (5 mL), DIEA (8 mg, 0.06 mmol) and acryloyl chloride (5 mg, 0.05 mmol) were added under ice-water bath cooling condition, and the mixture was reacted under stirring and ice-water bath cooling conditions for 5 min. After the reaction was complete, the reaction was quenched with a saturated sodium carbonate aqueous solution and extracted with dichloromethane, and the organic phase was dried with anhydrous sodium sulfate, concentrated and separated by TLC to obtain an off-white solid. ¹H NMR (300 MHz, CDCl₃) δ 7.96 - 7.74 (m, 4H), 7.54 (d, *J =* 7.1 Hz, 2H), 7.43 (d, *J =* 4.9 Hz, 2H), 7.34 (d, *J =* 8.6 Hz, 1H), 6.70 - 6.61 (m, 1H), 6.49 - 6.32 (m, 1H), 5.84 (d, *J =* 10.5 Hz, 1H), 5.42-5.34 (m, 1H), 5.13 - 5.06 (m, 1H), 4.71 - 4.67 (m, 1H), 4.55 - 4.31 (m, 2H), 3.89 - 3.43 (m, 4H), 3.10 -2.88 (m, 6H), 2.33 (s, 2H), 2.21 - 1.96 (m, 4H). MS m/z: 581.58 [M+H]⁺

### Example 74: Synthesis of 2-((2S)-1-acryloyl-4-(6-chloro-8-fluoro-2-((((S)-1-methylpyrrolidin-2-yl)methoxy)-7-[5.6.7,8-tetrahydronaphthalen-1-ylquinazolin-4-ylpiperazin-2-ylacetonitrile

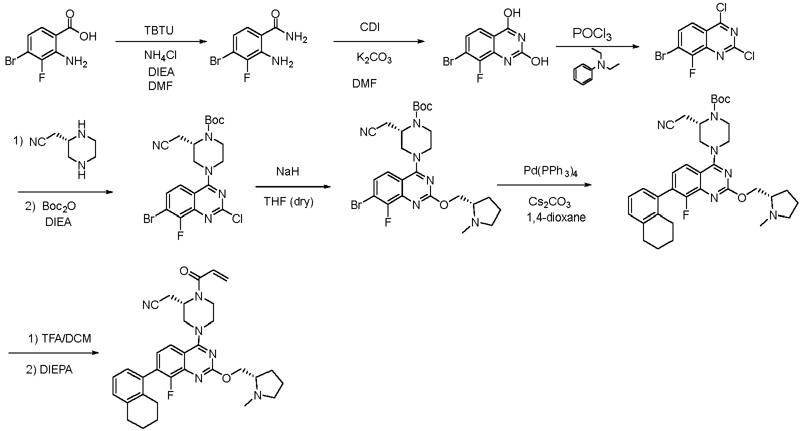

### Step 1: Synthesis of compound 2-amino-3-fluoro-4-bromobenzamide

The compound 2-amino-3-fluoro-4-bromobenzoic acid (5.0 g, 20 mmol) was dissolved in 50 ml of DMF, stirred and dissolved, TBTU (16.0 g, 50 mmol), NH₄Cl(27.0 g, 50 mmol), and DIEA (14 ml, 80 mmol) were added in one portion at room temperature, and the reaction system was stirred at room temperature for 3 h. After the reaction was complete, about 300 ml of water was added to the reaction system, and a large amount of solid was precipitated. When no more solid was precipitated, suction filtration gave a light yellow solid, which was directly used for the next step. (3.7 g, yield: 74%). ¹H NMR (400MHz, DMSO) δ 7.92 (s, 2H), 7.66 (s, 1H), 6.94 (s, 1H), 6.25 (s, 2H).

### Step 2: Synthesis of compound 7-bromo-8-fluoroqiuinazolin-2,4-diol

The compound 2-amino-4-bromo-5-chlorobenzamide (3.6 g, 14.4 mmol) was dissolved in 40 ml of DMF, stirred and dissolved, CDI (9.3 g, 57.7 mmol) and K₂CO₃ (8.0 g, 50 mmol) were added then dropwise in one portion at room temperature, and the reaction system was heated to 80°C and stirred overnight. After the reaction was complete, about 300 ml of water was added to the reaction system, and a large amount of solid was precipitated. When no more solid was precipitated, suction filtration gave a light yellow solid, which was directly used for the next step. (3.9 g, yield: 90 %). ¹H NMR (400MHz, DMSO) δ 11.34 (s,1H), 11.40 (s, 1H), 8.12 (s, 1H), 7.93 (s, 1H).

### Step 3: Synthesis of compound 7-bromo-2,4-dichloro-8-fluoroqinazoline

The compound 7-bromo-6-chloroquinazolin-2,4-diol (3.9 g, 14 mmol) was dissolved in 50 ml of POCl₃, and about 5 ml of N,N-diethylaniline was added at room temperature. The reaction system was heated to 110°C and stirred overnight. After the reaction was complete, the reaction system was placed under reduced pressure to remove the solvent to obtain a crude product. The crude product was separated by column chromatography (petroleum ether) to obtain a yellow solid. (2.62 g, 60%). ¹H NMR (400MHz, CDCl₃) δ 8.12 (s, 1H), 7.93 (s, 1H).

### Step 4: Synthesis of tert-butyl (S)-4-(7-bromo-2-chloro-8-fluoroquinazolin-4-yl)-2-(cyanomethyl)piperazine-1-carboxylate

The compound 7-bromo-2,4,6-trichloroquinazoline (312 mg, 1 mmol) and DIEA (0.6 ml, 3.5 mmol) were dissolved in 10 ml of DMF, and 2-cyanopiperazine (198 mg, 1 mmol) was added in portions under ice-water bath condition. The reaction was stirred and returned to room temperature, the reaction was monitored complete by TLC, and BoczO (0.6 ml, 2.5 mmol) was then added, and the reaction was stirred at room temperature for 2 h; and after the reaction was complete, about 70 ml of a saturated sodium chloride solution was added to the reaction system, and extraction was performed with ethyl acetate for separation. After the organic phase was dried with anhydrous sodium sulfate, the solvent was removed under reduced pressure to obtain a light yellow solid which was directly used for the next reaction. (450 mg, yield 90%). ¹H NMR (400MHz, CDCl₃) *δ* 8.14 (s, 1H), 7.95 (s, 1H), 3.5 (m, 1H), 3.38-3.13 (m, 4H), 3.02-2.98 (m, 2H), 2.73 (m, 1H), 2.48 (m, 1H), 1.42 (s, 9H).

### Step 5: Synthesis of tert-butyl (S)-4-(7-bromo-8-fluoro-2-((((S)-1-methylpyrrolidin-2-yl)methoxy)quinazolin-4-yl)-2-(cyanomethyl)piperazine-1-carboxylate

(S)-(1-methylpyrrolidin-2-yl)methanol (173 mg, 1.5 mmol) was dissolved in ultradry THF (15 mL), 60% sodium hydride (36 mg, 1.5 mmol) was added under ice-water bath condition and stirred 30 min, the compound tert-butyl (S)-4-(7-bromo-2,6-dichloroquinazolin-4-yl)-2-(cyanomethyl)piperazine-1-carboxylate (250 mg, 0.5 mmol) was then added, and the reaction system was stirred at room temperature for one hour. After the reaction was complete, water was added to the reaction system to quench the reaction, ethyl acetate was used for extraction and separation, and the organic phase was then dried with anhydrous sodium sulfate. The solvent was removed under reduced pressure to obtain a crude product, which was separated by column chromatography to obtain a light yellow solid. (133 mg, yield: 50%).

### Step 6: Synthesis of tert-butyl (S)-4-(6-chloro-2-(((S)-1-methylpyrrolidin-2-yl)methoxy)-7-(5,6,7,8-tetrahydronaphthalen-1-yl)quinazolin-4-yl)-2-(cyanomethyl)piperazine-1-carboxylic acid

The compound tert-butyl (S)-4-(7-bromo-6-chloro-2-((((S)-1-methylpyrrolidin-2-yl)methoxy)quinazolin-4-yl)-2-(cyanomethyl)piperazine-1-carboxylate (64 mg, 0.11 mmol), 4,4,5,5-tetramethyl-2-(5,6,7,8-tetrahydronaphthalen-1-yl)-1,3,2-dioxaborane (43 mg, 0.17 mmol) and Cs₂CO₃ (72 mg, 0.22 mmol) were dissolved in a hybrid solvent of 1,4-dioxane and water (4 ml/1 ml), and after displacement with nitrogen, a Pd(PPh₃)₄ catalyst (13 mg, 0.01 mmol) was added. The reaction system was stirred at 90°C for 1 h. After the reaction was complete, the reaction system was extracted with ethyl acetate and separated, the organic phase was dried, and the solvent was then removed under reduced pressure to obtain a crude product. The crude product was separated by column chromatography to obtain a light yellow solid (50 mg, yield: 80%).

### Step 7: Synthesis of 2-((2S)-1-acryloyl-4-(6-chloro-8-fluoro-2-((((S)-1-methylpyrrolidin-2-yl)methoxy)-7-(5,6,7,8-tetrahydronaphthalen-1-ylquinazolin-4-ylpiperazin-2-ylacetonitrile

The compound tert-butyl (S)-4-(6-chloro-2-(((S)-1-methylpyrrolidin-2-yl)methoxy)-7-(5,6,7,8-tetrahydronaphthalen-1-yl)quinazolin-4-yl)-2-(cyanomethyl)piperazine-1-carboxylic acid (31 mg, 0.1 mmol) was dissolved in a hybrid solvent of DCM and CF₃COOH (3 ml/1 ml) and stirred at room temperature for 1 h; after the reaction was complete, the organic solvent was removed from the reaction system under reduced pressure, dichloromethane was added for dissolution, the system was then spin-dried again, and the same operation was repeated again. The resulting crude product, which was directly used for the next reaction, was dissolved in 3 ml of ultradry dichloromethane, triethylamine (0.1 ml, 0.5 mmol) and acryloyl chloride (0.05 ml, 0.2 mmol) were added, and the reaction system was stirred at room temperature for 1 h. After the reaction was complete, the reaction system was spin-dried, ethyl acetate was added for dissolution, the organic phase was neutralized with a saturated sodium carbonate solution, extracted and separated, the organic phase was then dried with anhydrous sodium sulfate, the solvent was removed under reduced pressure to obtain a crude product, and the crude product was separated by PLC to obtain an off-white solid. (10 mg, yield: 34%) ¹H NMR (400MHz, CDCl₃) *δ* 8.29 (s, 1H), 8.23 (s, 1H), 7.27 (m,1H), 7.21 (m,1H),7.02 (m, 1H), 6.62 (m, 1H), 6.04 (m, 1H), 5.58 (m, 1H), 3.65-3.40 (m, 3H),3.38-3.13 (m, 4H), 3.02-2.98 (m, 2H), 2.85 (m, 1H), 2.75-2.70 (m, 5H), 2.50-2.30 (m, 3H), 2.26 (s, 3H), 1.74-1.41 (m, 8H). MS m/z: 585.27 [M+H]⁺

### Example 75: Synthesis of 2-((S)-1-acryloyl-4-(6-chloro-2-(((S)-1-methylpyrrolidin-2-yl)methoxy)-7-(5,6,7,8-tetrahydronaphthalen-1-yl)quinazolin-4-yl)piperazin-2-yl)acetonitrile

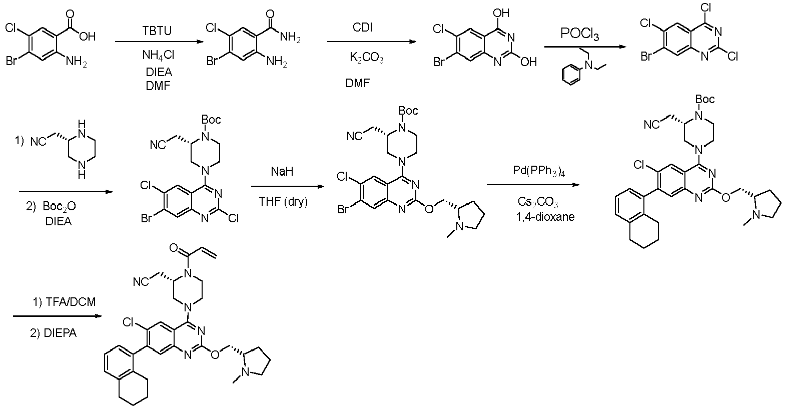

### Step 1: Synthesis of compound 2-amino-4-bromo-5-chlorobenzamide

The compound 2-amino-4-bromo-5-chlorobenzoic acid (5.0 g, 20 mmol) was dissolved in 50 ml of DMF, stirred and dissolved, TBTU (16.0 g, 50 mmol), NH₄Cl(27.0 g, 50 mmol), and DIEA (14 ml, 80 mmol) were added in one portion at room temperature, and the reaction system was stirred at room temperature for 3 h. After the reaction was complete, about 300 ml of water was added to the reaction system, and a large amount of solid was precipitated. When no more solid was precipitated, suction filtration gave a light yellow solid, which was directly used for the next step. (3.6 g, yield: 72%). ¹H NMR (400MHz, DMSO) δ 7.90 (s, 2H), 7.68 (s, 1H), 6.96 (s, 1H), 6.27 (s, 2H).

### Step 2: Synthesis of compound 7-bromo-6-chloroquinazolin-2,4-diol

The compound 2-amino-4-bromo-5-chlorobenzamide (3.6 g, 14.4 mmol) was dissolved in 40 ml of DMF, stirred and dissolved, CDI (9.3 g, 57.7 mmol) and K₂CO₃ (8.0 g, 50 mmol) were added then dropwise in one portion at room temperature, and the reaction system was heated to 80°C and stirred overnight. After the reaction was complete, about 300 ml of water was added to the reaction system, and a large amount of solid was precipitated. When no more solid was precipitated, suction filtration gave a light yellow solid, which was directly used for the next step. (3.9 g, yield: 90 %). ¹H NMR (400MHz, DMSO) δ 11.34 (s,1H), 11.40 (s, 1H), 8.12 (s, 1H), 7.93 (s, 1H).

### Step 3: Synthesis of compound 7-bromo-2,4,6-trichloroquinazoline

The compound 7-bromo-6-chloroquinazolin-2,4-diol (3.9 g, 14 mmol) was dissolved in 50 ml of POCl₃, and about 5 ml of N,N-diethylaniline was added at room temperature. The reaction system was heated to 110°C and stirred overnight. After the reaction was complete, the reaction system was placed under reduced pressure to remove the solvent to obtain a crude product. The crude product was separated by column chromatography (petroleum ether) to obtain a yellow solid. (2.62 g, 60%). ¹H NMR (400MHz, CDCl₃) δ 8.12 (s, 1H), 7.93 (s, 1H).

### Step 4: Synthesis of compound tert-butyl (S)-4-(7-bromo-2,6-dichloroquinazolin-4-yl)-2-(cyanomethyl)piperazine-1-carboxylate

The compound 7-bromo-2,4,6-trichloroquinazoline (312 mg, 1 mmol) and DIEA (0.6 ml, 3.5 mmol) were dissolved in 10 ml of DMF, and 2-cyanopiperazine (198 mg, 1 mmol) was added in portions under ice-water bath condition. The reaction was stirred and returned to room temperature, the reaction was monitored complete by TLC, and BoczO (0.6 ml, 2.5 mmol) was then added, and the reaction was stirred at room temperature for 2 h; and after the reaction was complete, about 70 ml of a saturated sodium chloride solution was added to the reaction system, and extraction was performed with ethyl acetate for separation. After the organic phase was dried with anhydrous sodium sulfate, the solvent was removed under reduced pressure to obtain a light yellow solid which was directly used for the next reaction. (450 mg, yield 90%). ¹H NMR (400MHz, CDCl₃) *δ* 8.14 (s, 1H), 7.95 (s, 1H), 3.5 (m, 1H), 3.38-3.13 (m, 4H), 3.02-2.98 (m, 2H), 2.73 (m, 1H), 2.48 (m, 1H), 1.42 (s, 9H).

### Step 5: Synthesis of tert-butyl (S)-4-(7-bromo-6-chloro-2-((((S)-1-methylpyrrolidin-2-yl)methoxy)quinazolin-4-yl)-2-(cvanomethyl)piperazine-1-carboxylate

(S)-(1-methylpyrrolidin-2-yl)methanol (173 mg, 1.5 mmol) was dissolved in ultradry THF (15 mL), 60% sodium hydride (36 mg, 1.5 mmol) was added under ice-water bath condition and stirred 30 min, the compound tert-butyl (S)-4-(7-bromo-2,6-dichloroquinazolin-4-yl)-2-(cyanomethyl)piperazine-1-carboxylate (250 mg, 0.5 mmol) was then added, and the reaction system was stirred at room temperature for one hour. After the reaction was complete, water was added to the reaction system to quench the reaction, ethyl acetate was used for extraction and separation, and the organic phase was then dried with anhydrous sodium sulfate. The solvent was removed under reduced pressure to obtain a crude product, which was separated by column chromatography to obtain a light yellow solid. (150 mg, yield: 52%).

### Step 6: Synthesis of tert-butyl (S)-4-(6-chloro-2-(((S)-1-methylpyrrolidin-2-yl)methoxy)-7-(5,6,7,8-tetrahydronaphthalen-1-yl)quinazolin-4-yl)-2-(cyanomethyl)piperazine-1-carboxylic acid

The compound tert-butyl (S)-4-(7-bromo-6-chloro-2-((((S)-1-methylpyrrolidin-2-yl)methoxy)quinazolin-4-yl)-2-(cyanomethyl)piperazine-1-carboxylate (64 mg, 0.11 mmol), 4,4,5,5-tetramethyl-2-(5,6,7,8-tetrahydronaphthalen-1-yl)-1,3,2-dioxaborane (43 mg, 0.17 mmol) and Cs₂CO₃ (72 mg, 0.22 mmol) were dissolved in a hybrid solvent of 1,4-dioxane and water (4 ml/1 ml), and after displacement with nitrogen, a Pd(PPh₃)₄ catalyst (13 mg, 0.01 mmol) was added. The reaction system was stirred at 90°C for 1 h. After the reaction was complete, the reaction system was extracted with ethyl acetate and separated, the organic phase was dried, and the solvent was then removed under reduced pressure to obtain a crude product. The crude product was separated by column chromatography to obtain a light yellow solid (62 mg, yield: 89%).

### Step 7: Synthesis of 2-((S)-1-acryloyl-4-(6-chloro-2-(((S)-1-methylpyrrolidin-2-yl)methoxy)-7-(5,6,7,8-tetrahydronaphthalen-1-yl)quinazolin-4-yl)piperazin-2-yl)acetonitrile

The compound tert-butyl (S)-4-(6-chloro-2-(((S)-1-methylpyrrolidin-2-yl)methoxy)-7-(5,6,7,8-tetrahydronaphthalen-1-yl)quinazolin-4-yl)-2-(cyanomethyl)piperazine-1-carboxylic acid (62 mg, 0.1 mmol) was dissolved in a hybrid solvent of DCM and CF₃COOH (3 ml/1 ml) and stirred at room temperature for 1 h; after the reaction was complete, the organic solvent was removed from the reaction system under reduced pressure, dichloromethane was added for dissolution, the system was then spin-dried again, and the same operation was repeated again. The resulting crude product, which was directly used for the next reaction, was dissolved in 3 ml of ultradry dichloromethane, triethylamine (0.1 ml, 0.5 mmol) and acryloyl chloride (0.05 ml, 0.2 mmol) were added, and the reaction system was stirred at room temperature for 1 h. After the reaction was complete, the reaction system was spin-dried, ethyl acetate was added for dissolution, the organic phase was neutralized with a saturated sodium carbonate solution, extracted and separated, the organic phase was then dried with anhydrous sodium sulfate, the solvent was removed under reduced pressure to obtain a crude product, and the crude product was separated by PLC to obtain an off-white solid. (20 mg, yield: 34%) ¹H NMR (400MHz, CDCl₃) *δ* 8.29 (s, 1H), 8.23 (s, 1H), 7.27 (m,1H), 7.21 (m,1H),7.02 (m, 1H), 6.62 (m, 1H), 6.04 (m, 1H), 5.58 (m, 1H), 3.65-3.40 (m, 3H),3.38-3.13 (m, 4H), 3.02-2.98 (m, 2H), 2.85 (m, 1H), 2.75-2.70 (m, 5H), 2.50-2.30 (m, 3H), 2.26 (s, 3H), 1.74-1.41 (m, 8H). MS m/z: 585.27 [M+H]⁺

### Example 76: Synthesis of 1-(4-(6-chloro-8-fluoro-2-(((S)-1-methylpyrrolidin-2-yl)methoxy)-7-(5,6,7,8-tetrahydronaphthalen-1-yl)quinazolin-4-yl)piperazin-1-yl)prop-2-en-1-one

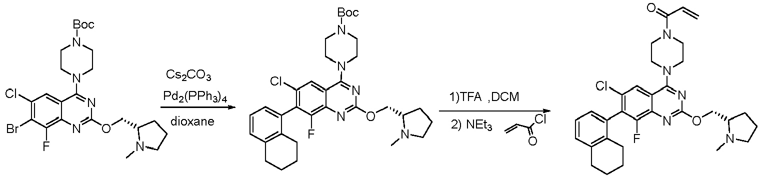

### Step 1: Synthesis of tert-butyl 4-(6-chloro-8-fluoro-2-(((S)-1-methylpyrrolidin-2-yl)methoxy)-7-(5,6,7,8-tetrahydronaphthalen-1-yl)quinazolin-4-yl)piperazine-1-carboxylate

The compound tert-butyl (S)-4-(7-bromo-6-chloro-8-fluoro-2-((1-methylpyrrolidin-2-yl)methoxy)quinazolin-4-yl)piperazine-1-carboxylate (56 mg, 0.11 mmol), 4,4,5,5-tetramethyl-2-(5,6,7,8-tetrahydronaphthalen-1-yl)-1,3,2-dioxaborane (43 mg, 0.17 mmol) and Cs2CO3 (72 mg, 0.22 mmol) was dissolved in a hybrid solvent of 1,4-dioxane and water (4 ml/1 ml), and after displacement with nitrogen, a Pd(PPh3)4 catalyst (13 mg, 0.01 mmol) was added. The reaction system was stirred at 90°C for 1 h. After the reaction was complete, the reaction system was extracted with ethyl acetate and separated, the organic phase was dried, and the solvent was then removed under reduced pressure to obtain a crude product. The crude product was separated by column chromatography to obtain a light yellow solid (33 mg, yield: 50%).

### Step 2: Synthesis of 1-(4-(6-chloro-8-fluoro-2-(((S)-1-methylpyrrolidin-2-yl)methoxy)-7-(5,6,7,8-tetrahydronaphthalen-1-yl)quinazolin-4-yl)piperazin-1-yl)prop-2-en-1-one

The compound tert-butyl 4-(6-chloro-8-fluoro-2-(((S)-1-methylpyrrolidin-2-yl)methoxy)-7-(5,6,7,8-tetrahydronaphthalen-1-yl)quinazolin-4-yl)piperazine-1-carboxylate (33 mg, 0.05 mmol) was dissolved in a hybrid solvent of DCM and CF3COOH (3 ml/1 ml) and stirred at room temperature for 1 h; after the reaction was complete, the organic solvent was removed from the reaction system under reduced pressure, dichloromethane was added for dissolution, the system was then spin-dried again, and the same operation was repeated again. The resulting crude product, which was directly used for the next reaction, was dissolved in 3 ml of ultradry dichloromethane, triethylamine (0.1 ml, 0.5 mmol) and acryloyl chloride (0.05 ml, 0.2 mmol) were added, and the reaction system was stirred at room temperature for 1 h. After the reaction was complete, the reaction system was spin-dried, ethyl acetate was added for dissolution, the organic phase was neutralized with a saturated sodium carbonate solution, extracted and separated, the organic phase was then dried with anhydrous sodium sulfate, the solvent was removed under reduced pressure to obtain a crude product, and the crude product was separated by PLC to obtain an off-white solid. (8 mg, yield: 28 %) ¹H NMR (400MHz, CDCl₃) δ 8.00 (s, 1H), 7.27 (m,1H), 7.11 (m,1H),7.02 (m, 1H), 6.62 (m, 1H), 6.04 (m, 1H), 5.58 (m, 1H), 3.65-3.40 (m, 2H),3.38-3.13 (m, 4H), 2.85 (m, 1H), 2.75-2.70 (m, 5H), 2.50-2.30 (m, 3H), 2.26 (s, 3H), 1.74-1.41 (m, 8H). MS m/z: 585.27 [M+H]+

### Example 77: Synthesis of 2-((S)-1-acryloyl-4-(2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile

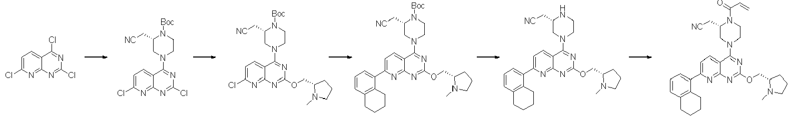

### Step 1: Synthesis of tert-butyl (S)-2-(cyanomethyl)-4-(2,7-dichloropyridino[2,3-d]pyrimidin-4-yl)piperazine-1-carboxylate

The compound 2,4,7-trichloropyridino[2,3-d]pyrimidine (200 mg, 0.85 mmol) was dissolved in anhydrous DMF (5 mL), DIEA (552 mg, 4.27 mmol) and (S)-2-(piperazin-2-yl)acetonitrile dihydrochloride (169 mg, 0.85 mmol) were added under ice-water bath cooling condition, the mixture was reacted under stirring and ice-water bath cooling conditions for 10 min, di-tert butyl dicarbonate (372 g, 1.70 mmol) was then added, and the mixture was heated to 40° C and reacted under stirring for 3 h. After the reaction was complete, the reaction product was cooled to room temperature, the reaction liquid was diluted by adding a saturated aqueous NaCl solution and extracted with ethyl acetate, and the organic phase was washed with a saturated aqueous NaCl solution, dried with anhydrous sodium sulfate, concentrated and separated by column chromatography to obtain a light yellow solid. (335 mg, yield: 93%). ¹H NMR (400MHz, CDCl₃) δ 8.18 (d,J = 8.6Hz, 1H), 7.34 (d,J = 8.6Hz, 1H), 4.65 (s, 1H), 4.47 (dd,J = 13.9, 3.0Hz, 1H), 4.30 (d,J = 12.0Hz, 1H), 4.12 (d,J = 7.1Hz, 1H), 3.82 (d,J = 12.2Hz, 1H), 3.69 (s, 1H), 3.54 (s, 1H), 3.02 - 2.87 (m, 1H), 2.80 (d,J = 13.8Hz, 1H), 1.52 (s, 9H).

### Step 2: Synthesis of tert-butyl (S)-4-(7-chloro-2-(((S)-1-methylpyrrolidin-2-yl)methoxy)pyridino[2,3-d]pyrimidin-4-yl)-2-(cyanomethyl)piperazine-1-carboxylate

The compound tert-butyl (S)-2-(cyanomethyl)-4-(2,7-dichloropyridino[2,3-d]pyrimidin-4-yl)piperazine-1-carboxylate (100 mg, 0.24 mmol) and (S)-(1-methylpyrrolidin-2-yl)methanol (82 mg, 0.71 mmol) was dissolved in anhydrous 1,4-dioxane (5 mL), DIEA (92 mg, 0.71 mmol) was added, and the mixture was heated to 80°C and reacted under stirring for 16 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated, separated and purified by TLC to obtain an off-white solid. (70 mg, yield: 59%). ¹H NMR (400 MHz, CDCl₃) δ 8.14 (d, *J* = 8.6 Hz, 1H), 7.26 (d,J = 8.6Hz, 1H), 5.04 (s, 1H), 4.82 (s, 1H), 4.61 (s, 1H), 4.45 (d, *J* = 13.9 Hz, 1H), 4.21 (d, *J* = 12.6 Hz, 1H), 4.07 (s, 1H), 3.73 (d, *J* = 10.6 Hz, 2H), 3.64 - 3.47 (m, 2H), 3.45 - 3.25 (m, 2H), 2.95 (s, 3H), 2.86 - 2.61 (m, 3H), 2.28 (s, 2H), 2.10 (d, *J* = 21.3 Hz, 2H), 1.51 (s, 9H).

### Step 3: Synthesis of tert-butyl (S)-2-(cyanomethyl)-4-(2-(((S)-1-methylpyrrolidin-2-yl)methoxy)-7-(5,6,7,8-tetrahydronaphthalen-1-yl)pyridino[2,3-d]pyrimidin-4-yl)piperazine-1-carboxylate

The compound tert-butyl (S)-4-(7-chloro-2-(((S)-1-methylpyrrolidin-2-yl)methoxy)pyridino[2,3-d]pyrimidin-4-yl)-2-(cyanomethyl)piperazine-1-carboxylate (30 mg, 0.06 mmol) and 4,4,5,5-tetramethyl-2-(5,6,7,8-tetrahydronaphthalen-1-yl)-1,3,2-dioxaborane (19 mg, 0.07 mmol) were dissolved in 1,4-dioxane/water = 5/1 (3 mL), cesium carbonate (59 mg, 0.18 mmol) and Pd(PPh₃)₄ (35 mg, 0.03 mmol) were added, and after displacement with nitrogen, the mixture was heated to 95°C in a nitrogen atmosphere and reacted under stirring for 1 h. After the reaction was complete, the reaction liquid was cooled to room temperature, diluted with water and extracted with ethyl acetate, and the organic phase was washed with a saturated aqueous NaCl solution, dried with anhydrous sodium sulfate, concentrated and separated by TLC to obtain an off-white solid. (27 mg, yield: 75%). MS m/z: 598.84 [M+H]⁺.

### Step 4: Synthesis of 2-((S)-4-(2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile

The compound tert-butyl (S)-2-(cyanomethyl)-4-(2-(((S)-1-methylpyrrolidin-2-yl)methoxy)-7-(5,6,7,8-tetrahydronaphthalen-1-yl)pyridino[2,3-d]pyrimidin-4-yl)piperazine-1-carboxylate (27 mg, 0.04 mmol) was dissolved in dichloromethane (3 mL), trifluoroacetic acid (1 mL) was added, and the mixture was reacted at room temperature under stirring for 1 h. After the reaction was complete, the reaction liquid was concentrated, a saturated sodium carbonate aqueous solution and dichloromethane were added, the mixture was extracted with dichloromethane, and the organic phase was dried with anhydrous sodium sulfate and concentrated to obtain a crude product, which was directly used for the next step.

### Step 5: Synthesis of 2-((S)-1-acryloyl-4-(2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile

The compound 2-((S)-4-(2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile (22 mg, 0.04 mmol) was dissolved in dichloromethane (5 mL), and DIEA (8 mg, 0.06 mmol) and acryloyl chloride (5 mg, 0.05 mmol) were added under ice-water bath cooling condition and reacted under stirring and ice-water bath cooling conditions for 5 min. After the reaction was complete, the reaction was quenched by adding saturated sodium carbonate and extracted with dichloromethane, and the organic phase was dried with anhydrous sodium sulfate, concentrated, separated and purified by TLC to obtain an off-white solid. (18 mg, yield: 74%). ¹H NMR (400 MHz, CDCl₃) δ 8.24 (d, *J* = 8.4 Hz, 1H), 7.39 (d, *J* = 8.4 Hz, 1H), 7.22- 7.16 (m, 3H), 6.59 (s, 1H), 6.41 (d, *J* = 16.3 Hz, 1H), 5.84 (d, *J* = 11.7 Hz, 1H), 5.02 (s, 2H), 4.84 (d, *J* = 11.2 Hz, 1H), 4.47 (d, *J* = 14.0 Hz, 1H),4.37 - 4.34 (m, 1H), 3.84 (d, *J* = 10.3 Hz, 1H), 3.77 - 3.49 (m, 4H), 3.08 (ddd, *J* = 16.9, 11.4, 7.7 Hz, 2H), 2.97 (s, 3H), 2.89 - 2.73 (m, 6H), 2.42 - 2.17 (m, 4H), 2.09 - 2.03 (m, 4H).MS m/z: 552.64 [M+H]⁺.

The compounds of Examples 78-614 were prepared by preparation method 2

| Ex. | Compound name | Structural formula | m/z: ES⁺[M+H] |
|---|---|---|---|
| 78 | 2-((S)-4-(7-(8-chloronaphthalen-1-yl)-2-((((S)-1-methylpyrrolidin-2-yl)methoxy)quinazolin-4-yl)-1-(2-fluoroacryloyl)piperazin-2-yl)acetonitrile | 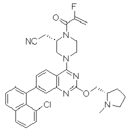 | 599.2 |
| 79 | 2-((S)-1-acryloyl-4-(7-(8-chloronaphthalen-1-yl)-2-((((2S,4R)-4-fluoro-1-methylpyrrolidin-2-yl]methoxy))quinazolin-4-yl)piperazin-2-yl)acetonitrile | 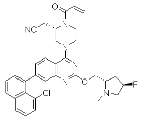 | 599.2 |
| 80 | 2-((S)-4-(7-(8-chloronaphthalen-1-yl)-2-((((2S,4R)-4-fluoro-1-methylpyrrolidin-2--2-yl)methoxy)quinazolin-4-yl)-1-(2-fluoroacryloyl)piperazin-2-yl)acetonitrile | 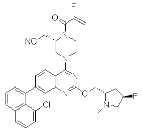 | 617.2 |
| 81 | (S)-2-(1-acryloyl-4-(7-(8-chloronaphthalen-1-yl)-2-((tetrahydro-1H-pyrrolizin-7a(5H)-yl)methoxy)quinazolin-4-yl piperazin-2-yl)acetonitrile | 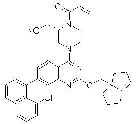 | 607 |
| 82 | (S)-2-(4-(7-(8-chloronaphthalen-1-yl)-2-((tetrahydro-1H-pyrrolizin-7a(5H)-yl)methoxy)quinazolin-4-yl)-1-(2-fluoroacryloyl)piperazin-2-yl)acetonitrile | 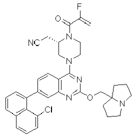 | 625.2 |
| 83 | 2-((S)-1-acryloyl-4-(7-(8-chloronaphthalen-1-yl)-2-(((((2R,7aS)-2-fluorotetrahydro-1H-pyrrolizin-7a(5H)-yl)methoxy)quinazolin-4-yl)piperazin-2-yl)acetonitrile | 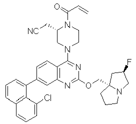 | 625.2 |
| 84 | 2-((S)-4-(7-(8-chloronaphthalen-1-yl)-2-(((((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 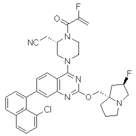 | 643.2 |
| 85 | 1-((1R,5R)-6-(7-(8-chloronaphthalen-1-yl)-2-(((S)-1-methylpyrrolidin-2-yl)methoxy)quinazolin-4-yl)-2,6-diazabicyclo[3.2.0]hept-2-yl)prop-2-en-1-one | 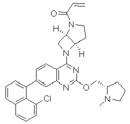 | 554.2 |
| 86 | 1-((1R,5R)-6-(7-(8-chloronaphthalen-1-yl)-2-(((S)-1-methylpyrrolidin-2-yl)methoxy)quinazolin-4-yl)-2,6-diazabicyclo[3.2.0]hept-2-yl)-2-fluoroprop-2-en-1-one | 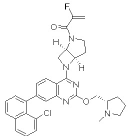 | 572.2 |
| 87 | 1-((1R,SR)-6-(7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 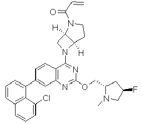 | 572.2 |
| 88 | 1-((1R,SR)-6-(7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)-2-fluoroprop-2-en-1 -one | 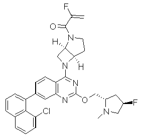 | 590.2 |
| 89 | 1-((1R,SR)-6-(7-(8-chloronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 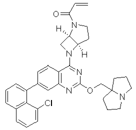 | 580.2 |
| 90 | 1-((1R,SR)-6-(7-(8-chloronaphthalen-1-yl)-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 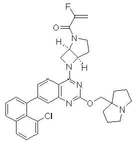 | 598.2 |
| 91 | 1-(((1R,5R)-6-(7-(8-chloronaphthalen-1-yl)-2-((((2R,7aS)-2-fluorotetrahydro-1H-pyrrolizin-7a(5H)-yl]methoxy)quinazolin-4-yl)-2,6-diazabicyclo[3.2.0]hept-2-yl)prop-2-en-1-one | 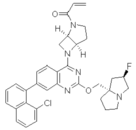 | 598.2 |
| 92 | 1-(((1R,5R)-6-(7-(8-chloronaphthalen-1-yl)-2-((((2R,7aS)-2-fluorotetrahydro-1H-pyrrolizin-7a(5H)-yl]methoxy)quinazolin-4-yl)-2,6-diazabicyclo[3.2.0]hept-2-yl)-2-fluoroprop-2-en-1-one | 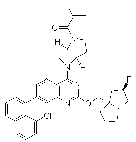 | 616.2 |
| 93 | 2-((S)-1-acryloyl-4-(7-(8-chloro-7-fluoronaphthalen-1-yl)-2-(((S)-1 -methylpyrrolidin-2-yl)methoxy)quinazolin-4-yl)piperazin-2-yl)acetonitrile | 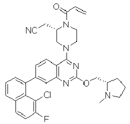 | 599.2 |
| 94 | 2-((S)-4-(7-(8-chloro-7-fluoronaphthalen-1-yl)-2-(((S)-1-methylpyrrolidin-2-yl)methoxy)quinazolin-4-yl)-1- (2-fluoroacryloyl)piperazin-2-yl)acetonitrile | 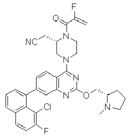 | 617.2 |
| 95 | 2-((S)-1-acryloyl-4-(7-(8-chloro-7-fluoronaphthalen-1-yl)-2-((((2S,4R)-4-fluoro-1-methylpyrrolidin-2-yl)methoxy)quinazolin-4-yl)piperazin-2-yl)acetonitrile | 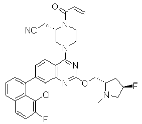 | 617.2 |
| 96 | 2-((S)-4-(7-(8-chloro-7-fluoronaphthalen-1-yl)-2-((((2S,4R)-4-fluoro-1-methylpyrrolidin-2-yl)methoxy)quinazolin-4-yl)-1-(2-fluoroacryloyl)piperazin-2-yl)acetonitrile | 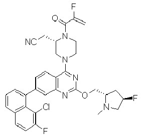 | 635 |
| 97 | (S)-2-(1-acryloyl-4-(7-(8-chloro-7-fluoronaphthalen-1-yl)-2-((tetrahydro-1H-pyrrolizin-7 a(5H)-yl)methoxy)quinazolin-4-yl)piperazin-2-yl)acetonitrile | 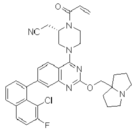 | 625.2 |
| 98 | (S)-2-(4-(7-(8-chloro-7-fluoronaphthalen-1-yl)-2-((tetrahydro-1H-pyrrolizin-7a(5H)-yl)methoxy)quinazolin-4-yl)-1-(2-fluoroacryloyl)piperazin-2-yl)acetonitrile | 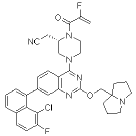 | 643 |
| 99 | 2-((S)-1-acryloyl-4-(7-(8-chloro-7-fluoronaphthalen-1-yl)-2-((((2R,7aS)-2-fluorotetrahydro-1H-pyrrolizin-7a(5H)-yl)methoxy)quinazolin-4-yl)piperazin-2-yl)acetonitrile | 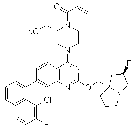 | 643 |
| 100 | 2-((S)-4-(7-(8-chloro-7-fluoronaphthalen-1-yl)-2-(((((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 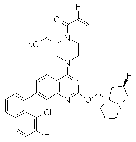 | 661 |
| 101 | 1-((1R,5R)-6-(7-(8-chloro-7-fluoronaphthalen-1-yl)-2-(((S)-1 -methylpyrrolidin-2-yl)methoxy)quinazolin-4-yl)-2,6-diazabicyclo[3.2.0]hept-2-yl)prop-2-en-1-one | 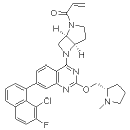 | 572.2 |
| 102 | 1-((1R,5R)-6-(7-(8-chloro-7-fluoronaphthalen-1-yl)-2-(((S)-1 -methylpyrrolidin-2-yl)methoxy)quinazolin-4-yl)-2,6-diazabicyclo[3.2.0]hept-2-yl)-2-fluoroprop-2-en-1-one | 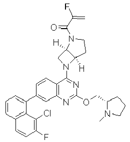 | 590.2 |
| 103 | 1-((1R,SR)-6-(7-(8-chloro-7-fluoronaphthalen-1-yl)-2-((((2S,4R)-4-fluoro-1-methylpyrrolidin-2-yl))methoxy)quinazolin-4-yl)-2,6-diazabicyclo[3.2.0]hept-2-yl)prop-2-en-1-one | 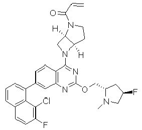 | 590.2 |
| 104 | 1-((1R,SR)-6-(7-(8-chloro-7-fluoronaphthalen-1-yl)-2-((((2S,4R)-4-fluoro-1-methylpyrrolidin-2-yl))methoxy)quinazolin-4-yl)-2,6-diazabicyclo[3.2.0]hept-2-yl)-2-fluoroprop-2-en-1-one | 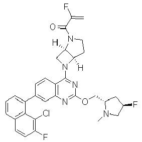 | 608.2 |
| 105 | 1-((1R,SR)-6-(7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 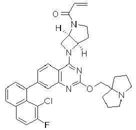 | 598.23 |
| 106 | 1-((1R,SR)-6-(7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 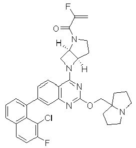 | 616.2 |
| 107 | 1-(((1R,SR)-6-(7-(8-chloro-7-fluoronaphthalen-1-yl)-2-((((2R,7aS)-2-fluorotetrahydro-1H-pyrrolizin-7a(5H] -yl)methoxy)quinazolin-4-yl)-2,6-diazabicyclo[3.2.0]hept-2-yl)prop-2-en-1-one | 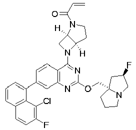 | 616.2 |
| 108 | 1-(((1R,SR)-6-(7-(8-chloro-7-fluoronaphthalen-1-yl)-2-((((2R,7aS)-2-fluorotetrahydro-1H-pyrrolizin-7a(5H]- yl)methoxy)quinazolin-4-yl)-2,6-diazabicyclo[3.2.0]hept-2-yl)-2-fluoroprop-2-en-1-one | 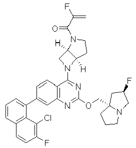 | 634.2 |
| 109 | (S)-2-(1-acryloyl-4-(2-((2-methyl-1,2,3,4-tetrahydroisoquinolin-5-yl)oxy)-7-(5,6,7,8-tetrahydronaphthalen-1-yl)quinazolin-4-ylpiperazin-2-yl)acetonitrile | 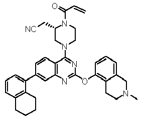 | 599.6 |
| 110 | 2-((S)-1-acryloyl-4-(2-(((S)-1-methylpyrrolidin-2-yl)methoxy)-7-(5,6,7,8-tetrahydronaphthalen-1-yl)quinazolin-4-yl)piperazin-2-yl)acetonitrile | 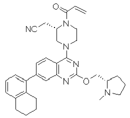 | 551.3 |
| 111 | 2-((S)-1-(2-fluoroacryloyl)-4-(2-((((S)-1-methylpyrrolidin-2-yl)methoxy)-7-(5,6,7,8-tetrahydronaphthalen-1-yl)quinazolin-4-yl)piperazin-2-yl)acetonitrile | 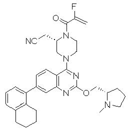 | 569.3 |
| 112 | 2-((S)-1-acryloyl-4-(2-((((2S,4R)-4-fluoro-1-methylpyrrolidin-2-yl)methoxy)-7-(5,6,7,8-tetrahydronaphthalen-1 -yl)quinazolin-4-ylpiperazin-2-yl)acetonitrile | 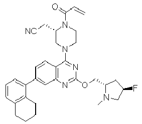 | 569.3 |
| 113 | 2-((S)-4-(2-(((((2S,4R)-4-fluoro-1-methylpyrrolidin-2-yl)methoxy)-7-(5,6,7,8-tetrahydronaphthalen-1)-yl)quinazolin-4-yl)-1-(2-fluoroacryloyl)piperazin-2-yl)acetonitrile | 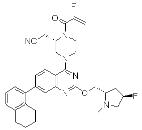 | 587 |
| 114 | (S)-2-(1-acryloyl-4-(2-((tetrahydro-1H-pyrrolizin-7a(5H)-yl)methoxy)-7-(5,6,7,8-tetrahydronaphthalen-1-yl)quinazolin-4-yl)piperazin-2-yl)acetonitrile | 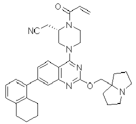 | 577.3 |
| 115 | (S)-2-(1-(2-fluoroacryloyl)-4-(2-((tetrahydro-1H-pyrrolizin-7a(5H)-yl)methoxy)-7-(5,6,7,8-tetrahydronaphthalen-1 -yl)quinazolin-4-ylpiperazin-2-yl)acetonitrile | 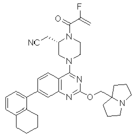 | 595.3 |
| 116 | 2-((2S)-1-acryloyl-4-(2-(((((2R,7aS)-2-fluorotetrahydro-1H-pyrrolizin-7a(5H)-yl)methoxy)-7-(5,6,7,8-tetrahydronaphthalen-1-ylquinazolin-4-ylpiperazin-2-ylacetonitrile | 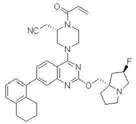 | 595.3 |
| 117 | 2-((2S)-1-(2-fluoroacryloyl)-4-(2-(((((2R,7aS)-2-fluorotetrahydro-1H-pyrrolizin-7a(5H)-yl)methoxy)-7-(5,6,7,8-tetrahydronaphthalen-1-yl)quinazolin-4-yl)piperazin-2-yl)acetonitrile | 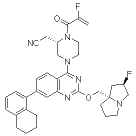 | 613.3 |
| 118 | 1-((1R,SR)-6-(2-((((S)-1-methylpyrrolidin-2-yl)methoxy)-7]-(5,6,7,8-tetrahydronaphthalen-1-yl)quinazolin-4-yl)-2,6-diazabicyclo[3.2.0]hept-2-yl)prop-2-en-1-one | 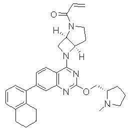 | 524.3 |
| 119 | 2-fluoro-1-(((1R,SR)-6-(2-((((S)-1-methylpyrrolidin-2-yl)methoxy)-7]-(5,6,7,8-tetrahydronaphthalen-1)-yl)quinazolin-4-yl)-2,6-diazabicyclo[3.2.0]hept-2-yl)prop-2-en-1 -one | 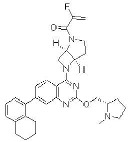 | 542.3 |
| 120 | 1-((1R,SR)-6-(2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 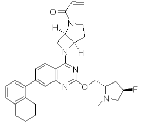 | 542.4 |
| 121 | 2-fluoro-1-(((1R,SR)-6-(2-((((2S,4R)-4-fluoro-1-methylpyrrolidin-2-yl)methoxy)-7)((5,6,7,8-tetrahydronaphthalen-1-yl)quinazolin-4-yl)-2,6-diazabicyclo[3.2.0]hept-2-yl)prop-2-en-1-one | 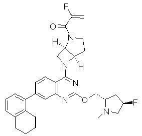 | 560 |
| 122 | 1-((1R,5R)-6-(2-(((tetrahydro-1H-pyrrolizin-7a(5H)-yl)methoxy)-7)((5,6,7,8-tetrahydronaphthalen-1-yl)quinazolin-4-yl)-2,6-diazabicyclo[3.2.0]hept-2-yl)prop-2-en-1-one | 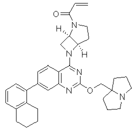 | 550 |
| 123 | 2-fluoro-1-(((1R,5R)-6-(2-((tetrahydro-1H-pyrrolizin-7a(5H)-yl)methoxy))-7-(5,6,7,8-tetrahydronaphthalen-1-yl)quinazolin-4-yl)-2,6-diazabicyclo[3.2.0]hept-2-yl)prop-2-en-1 -one | 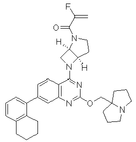 | 568 |
| 124 | 1-(((1R,SR)-6-(2-(((((2R,7aS)-2-fluorotetrahydro-1H-pyrrolizin-7a(5H)-yl)methoxy)-7)((tetrahydronaphthalen-1-yl)quinazolin-4-yl)-2,6-diazabicyclo[3.2.0]hept-2-yl)prop-2-en-1-one | 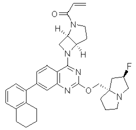 | 568 |
| 125 | 2-fluoro-1-(((1R,5R)-6-(2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)-2,6-diazabicyclo[3.2.0]hept-2-yl)prop-2-en-1-one | 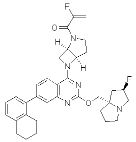 | 586 |
| 126 | 2-((S)-1-acryloyl-4-(7-(5-chloroisoquinolin-4-yl)-2-((((S)-1-methylpyrrolidin-2-yl)methoxy)quinazolin-4-yl]) piperazin-2-yl)acetonitrile | 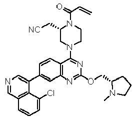 | 582.2 |
| 127 | 2-((S)-4-(7-(5-chloroisoquinolin-4-yl)-2-((((S)-1-methylpyrrolidin-2-yl)methoxy)quinazolin-4-yl)-1-(2-fluoroacryloyl)piperazin-2-yl)acetonitrile | 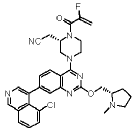 | 600.2 |
| 128 | 2-((S)-1-acryloyl-4-(7-(5-chloroisoquinolin-4-yl)-2-((((2S,4R)-4-fluoro-1-methylpyrrolidin-2-yl)methoxy))quinazolin-4-yl)piperazin-2-yl)acetonitrile | 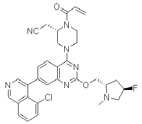 | 600 |
| 129 | 2-((S)-4-(7-(5-chloroisoquinolin-4-yl)-2-((((2S,4R)-4-fluoro-1-methylpyrrolidin-2--2-yl)methoxy)quinazolin-4-yl)-1-(2-fluoroacryloyl)piperazin-2-yl)acetonitrile | 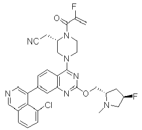 | 618 |
| 130 | (S)-2-(1-acryloyl-4-(7-(5-chloroisoquinolin-4-yl)-2-((tetrahydro-1H-pyrrolizin-7a(5H)-yl)methoxy)quinazolin-4-yl)piperazin-2-yl)acetonitrile | 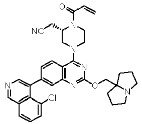 | 608 |
| 131 | (S)-2-(4-(7-(5-chloroisoquinolin-4-yl)-2-((tetrahydro-1H-pyrrolizin-7a(5H)-yl)methoxy)quinazolin-4-yl)-1-(2-fluoroacryloyl)piperazin-2-yl)acetonitrile | 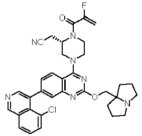 | 626 |
| 132 | 2-((2S)-1-acryloyl-4-(7-(5-chloroisoquinolin-4-yl)-2-((((2R,7aS)-2-fluorotetrahydro-1H-pyrrolizin-7a(5H)-yl)methoxy)quinazolin-4-yl)piperazin-2-yl)acetonitrile | 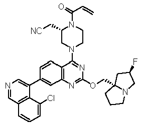 | 626 |
| 133 | 2-((2S)-4-(7-(5-chloroisoquinolin-4-yl)-2-(((((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 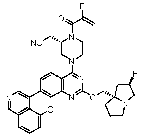 | 644 |
| 134 | 1-((1R,5R)-6-(7-(5-chloroisoquinolin-4-yl)-2-((((S)-1-methylpyrrolidin-2-yl)methoxy)quinazolin-4-yl]-2,6-diazabicyclo[3.2.0]hept-2-yl)prop-2-en-1-one | 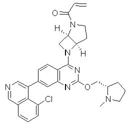 | 555 |
| 135 | 1-((1R,5R)-6-(7-(5-chloroisoquinolin-4-yl)-2-((((S)-1-methylpyrrolidin-2-yl)methoxy)quinazolin-4-yl]-2,6-diazabicyclo[3.2.0]hept-2-yl)-2-fluoroprop-2-en-1-one | 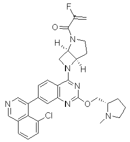 | 572 |
| 136 | 1-((1R,SR)-6-(7-(5-chloroisoquinolin-4-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 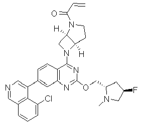 | 573 |
| 137 | 1-((1R,SR)-6-(7-(5-chloroisoquinolin-4-yl)-2-((((2S,4R)-4-fluoro-1-methylpyrrolidin-2-yl)methoxy)quinazolin-4-yl)-2,6-diazabicyclo[3.2.0]hept-2-yl)-2-fluoroprop-2-en-1-one | 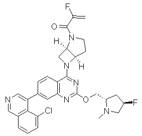 | 591 |
| 138 | 1-(((1R,SR)-6-(7-(5-chloroisoquinolin-4-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 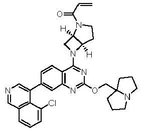 | 581 |
| 139 | 1-(((1R,SR)-6-(7-(5-chloroisoquinolin-4-yl)-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 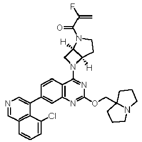 | 599 |
| 140 | 1-((1R,SR)-6-(7-(5-chloroisoquinolin-4-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 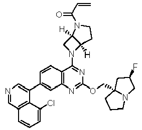 | 599 |
| 141 | 1-((1R,SR)-6-(7-(5-chloroisoquinolin-4-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)-2-fluoroprop-2-en-1-one | 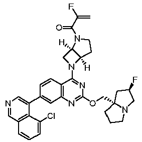 | 617 |
| 142 | 2-((S)-1-acryloyl-4-(2-((((S)-1-methylpyrrolidin-2-yl)methoxy)-7-(5,6,7,8-tetrahydroisoquinolin-4-yl)quinazolin-4-yl)piperazin-2-yl)acetonitrile | 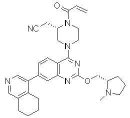 | 552 |
| 143 | 2-((S)-1-(2-fluoroacryloyl)-4-(2-((((S)-1-methylpyrrolidin-2-yl)methoxy)-7-(5,6,7,8-tetrahydroisoquinoline-4-yl)quinazolin-4-yl)piperazin-2-yl)acetonitrile | 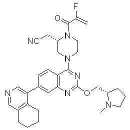 | 570 |
| 144 | 2-((S)-1-acryloyl-4-(2-((((2S,4R)-4-fluoro-1-methylpyrrolidin-2-yl)methoxy)-7-(5,6,7,8-tetrahydroisoquinolin-4-yl)quinazolin-4-ylpiperazin-2-yl)acetonitrile | 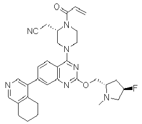 | 570 |
| 145 | 2-((S)-4-(2-(((((2S,4R)-4-fluoro-1-methylpyrrolidin-2-yl)methoxy))-7-(5,6,7,8-tetrahydroisoquinolin-4-yl)quinazolin-4-yl)-1-(2-fluoroacryloyl)piperazin-2-yl)acetonitrile | 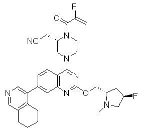 | 588 |
| 146 | (S)-2-(1-acryloyl-4-(2-((tetrahydro-1H-pyrrolizin-7a(5H)-yl)methoxy)-7-(5,6,7,8-tetrahydroisoquinolin-4-yl)quinazolin-4-yl)piperazin-2-yl)acetonitrile | 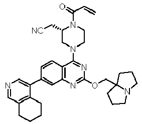 | 578 |
| 147 | (S)-2-(1-(2-fluoroacryloyl)-4-(2-((tetrahydro-1H-pyrrolizin-7a(5H)-yl)methoxy)-7-(5,6,7,8-tetrahydroisoquinolin-4-yl)quinazolin-4-ylpiperazin-2-yl)acetonitrile | 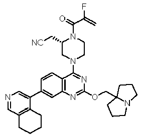 | 596 |
| 148 | 2-((2S)-1-acryloyl-4-(2-(((((2R,7aS)-2-fluorotetrahydro-1H-pyrrolizin-7a(5H)-yl]methoxy)-7)-(5,6,7,8-tetrahydroisoquinolin-4-yl)quinazolin-4-ylpiperazin-2-yl)acetonitrile | 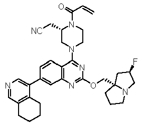 | 596 |
| 149 | 2-((2S)-1-(2-fluoroacryloyl)-4-(2-(((((2R,7aS)-2-fluorotetrahydro-1H-pyrrolizin-7a(5H)-yl)methoxy)-7-(5,6,7,8-tetrahydroisoquinolin-4-yl)quinazolin-4-yl)piperazin-2-yl)acetonitrile | 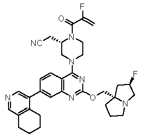 | 614 |
| 150 | 1-((1R,5R)-6-(2-((((S)-1-methylpyrrolidin-2-yl)methoxy)-7)-(5,6,7,8-tetrahydroisoquinolin-4-yl)quinazolin-4-yl)-2,6-diazabicyclo[3.2.0]hept-2-yl)prop-2-en-1-one | 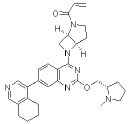 | 525 |
| 151 | 2-fluoro-1-(((1R,SR)-6-(2-((((S)-1-methylpyrrolidin-2-yl)methoxy]-7)-(5,6,7,8-tetrahydroisoquinolin-4-yl)quinazolin-4-yl)-2,6-diazabicyclo[3.2.0]hept-2-yl)prop-2-en-1 -one | 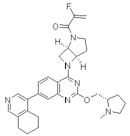 | 543 |
| 152 | 1-(((1R,5R)-6-(2-((((2S,4R)-4-fluoro-1-methylpyrrolidin-2-yl)methoxy)-7)((5,6,7,8-tetrahydroisoquinoline-4-yl)quinazolin-4-yl)-2,6-diazabicyclo[3.2.0]hept-2-yl)prop-2-en-1-one | 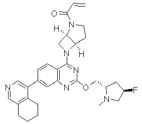 | 543 |
| 153 | 2-fluoro-1-(((1R,5R)-6-(2-((((2S,4R)-4-fluoro-1-methylpyrrolidin-2-yl)methoxy)-7)((5,6,7,8-tetrahydroisoquinolin-4-yl)quinazolin-4-yl)-2,6-diazabicyclo[3.2.0]hept-2-yl)prop-2-en-1-one | 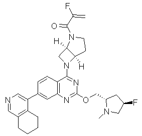 | 561 |
| 154 | 1-((1R,5R)-6-(2-(((tetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 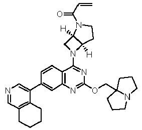 | 551 |
| 155 | 2-fluoro-1-(((1R,5R)-6-(2-((tetrahydro-1H-pyrrolizin-7a(5H)-yl)methoxy))-7-(5,6,7,8-tetrahydroisoquinolin-4-yl)quinazolin-4-yl)-2,6-diazabicyclo[3.2.0]hept-2-yl)prop-2-en-1-one | 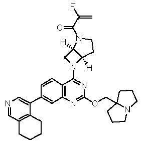 | 569 |
| 156 | 1-(((1R,SR)-6-(2-(((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 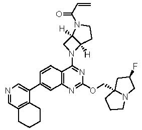 | 569.3 |
| 157 | 2-fluoro-1-(((1R,SR)-6-(2-(((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 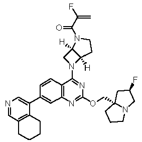 | 587 |
| 158 | 2-((S)-1-acryloyl-4-(7-(5-chloroisoquinolin-4-yl)-8-fluoro-2-(((S)-1-methylpyrrolidin-2-yl)methoxy)quinazolin-4-yl)piperazin-2-yl)acetonitrile | 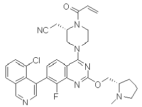 | 600.2 |
| 159 | 2-((S)-4-(7-(5-chloroisoquinolin-4-yl)-8-fluoro-2-((((S)-1-methylpyrrolidin-2-yl)methoxy)quinazolin-4-yl)-1-(2-fluoroacryloyl)piperazin-2-yl)acetonitrile | 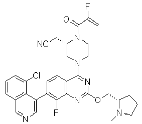 | 618.2 |
| 160 | 2-((S)-1-acryloyl-4-(7-(5-chloroisoquinolin-4-yl)-8-fluoro-2-((((2S,4R)-4-fluoro-1-methylpyrrolidin-2-(yl)methoxy)quinazolin-4-yl)piperazin-2-yl)acetonitrile | 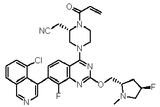 | 618.2 |
| 161 | 2-((S)-4-(7-(5-chloroisoquinolin-4-yl)-8-fluoro-2-((((2S,4R)-4-fluoro-1-methylpyrrolidin-2-yl)methoxy)quinazolin-4-yl)-1-(2-fluoroacryloyl)piperazin-2-yl)acetonitrile | 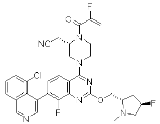 | 636.2 |
| 162 | (S)-2-(1-acryloyl-4-(7-(5-chloroisoquinolin-4-yl)-8-fluoro-2-((tetrahydro-1H-pyrrolizin-7a(5H)-yl)methoxy)quinazolin-4-yl)piperazin-2-yl)acetonitrile | 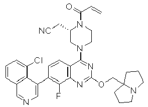 | 626.2 |
| 163 | (S)-2-(4-(7-(5-chloroisoquinolin-4-yl)-8-fluoro-2-((tetrahydro-1H-pyrrolizin-7a(5H)-yl)methoxy)quinazolin-4-yl)-1-(2-fluoroacryloyl)piperazin-2-yl)acetonitrile | 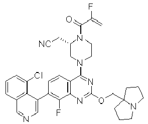 | 644 |
| 164 | 2-((2S)-1-acryloyl-4-(7-(5-chloroisoquinolin-4-yl)-8-fluoro-2-((((2R,7aS)-2-fluorotetrahydro-1H-pyrrolizin-7a(5H))-yl)methoxy)quinazolin-4-yl)piperazin-2-yl)acetonitrile | 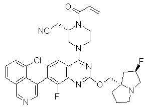 | 644 |
| 165 | 2-((2S)-4-(7-(5-chloroisoquinolin-4-yl)-8-fluoro-2-((((2R,7aS)-2-fluorotetrahydro-1H-pyrrolizin-7a(5H)-yl]methoxy)quinazolin-4-yl)-1-(2-fluoroacryloyl)piperazin-2-yl)acetonitrile | 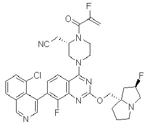 | 662 |
| 166 | 1-((1R,SR)-6-(7-(5-chloroisoquinolin-4-yl)-8-fluoro-2-(((S)-1-methylpyrrolidin-2-yl)methoxy)quinazolin-4-yl)-2,6-diazabicyclo[3.2.0]hept-2-yl)prop-2-en-1-one | 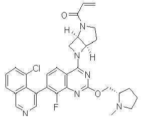 | 573 |
| 167 | 1-((1R,SR)-6-(7-(5-chloroisoquinolin-4-yl)-8-fluoro-2-(((S)-1-methylpyrrolidin-2-yl)methoxy)quinazolin-4-yl)-2,6-diazabicyclo[3.2.0]hept-2-yl)-2-fluoroprop-2-en-1-one | 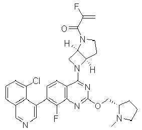 | 591.2 |
| 168 | 1-(((1R,5R)-6-(7-(5-chloroisoquinolin-4-yl)-8-fluoro-2-((((2S,4R)-4-fluoro-1-methylpyrrolidin-2-yl))methoxy)quinazolin-4-yl)-2,6-diazabicyclo[3.2.0]hept-2-yl)prop-2-en-1-one | 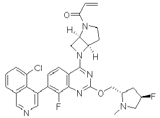 | 591 |
| 169 | 1-(((1R,5R)-6-(7-(5-chloroisoquinolin-4-yl)-8-fluoro-2-((((2S,4R)-4-fluoro-1-methylpyrrolidin-2-yl))methoxy)quinazolin-4-yl)-2,6-diazabicyclo[3.2.0]hept-2-yl)-2-fluoroprop-2-en-1-one | 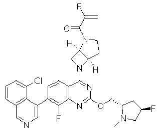 | 609 |
| 170 | 1-(((1R,5R)-6-(7-(5-chloroisoquinolin-4-yl)-8)-fluoro-2-((tetrahydro-1H-pyrrolizin-7a(5H)-yl)methoxy)quinazolin-4-yl)-2,6-diazabicyclo[3.2.0]hept-2-yl)prop-2-en-1-one | 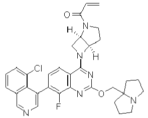 | 599 |
| 171 | 1-(((1R,5R)-6-(7-(5-chloroisoquinolin-4-yl)-8)-fluoro-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 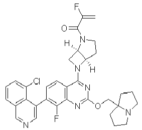 | 617 |
| 172 | 1-(((1R,5R)-6-(7-(5-chloroisoquinolin-4-yl)-8)-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 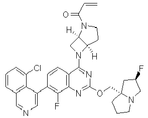 | 617 |
| 173 | 1-(((1R,5R)-6-(7-(5-chloroisoquinolin-4-yl)-8)-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)-2-fluoroprop-2-en-1-one | 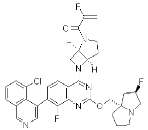 | 635 |
| 174 | 2-((S)-1-acryloyl-4-(8-fluoro-2-(((S)-1-methylpyrrolidin-2-yl)methoxy)-7-(5,6,7,8-tetrahydroisoquinoline-4-yl)quinazolin-4-yl)piperazin-2-yl)acetonitrile | 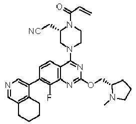 | 570 |
| 175 | 2-((S)-4-(8-fluoro-2-(((S)-1-methylpyrrolidin-2-yl)methoxy)-7-(5,6,7,8-tetrahydroisoquinolin-4-yl))quinazolin-4-yl)-1-(2-fluoroacryloyl)piperazin-2-yl)acetonitrile | 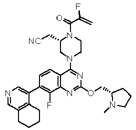 | 588 |
| 176 | 2-((S)-1-acryloyl-4-(8-fluoro-2-((((2S,4R)-4-fluoro-1-methylpyrrolidin-2-yl)methoxy)-7-(5,6,7,8-tetrahydroisoquinolin-4-yl)quinazolin-4-ylpiperazin-2-yl)acetonitrile | 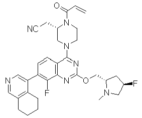 | 588 |
| 177 | 2-((S)-4-(8-fluoro-2-((((2S,4R)-4-fluoro-1-methylpyrrolidin-2-yl)methoxy)-7-(5,6,7,8-tetrahydroisoquinolin-4-yl)quinazolin-4-yl)-1-(2-fluoroacryloyl)piperazin-2-yl)acetonitrile | 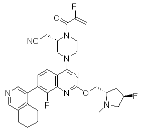 | 606 |
| 178 | (S)-2-(1-acryloyl-4-(8-fluoro-2-((tetrahydro-1H-pyrrolizin-7a(5H)-yl)methoxy)-7-(5,6,7,8-tetrahydroisoquinolin-4-yl)quinazolin-4-ylpiperazin-2-yl)acetonitrile | 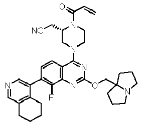 | 596 |
| 179 | (S)-2-(4-(8-fluoro-2-((tetrahydro- 1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 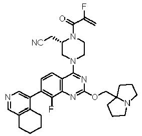 | 614 |
| 180 | 2-((2S)-1-acryloyl-4-(8-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)piperazin-2-yl)acetonitrile | 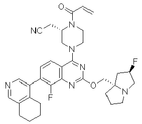 | 614 |
| 181 | 2-((2S)-4-(8-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 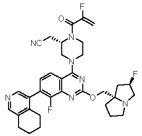 | 632.4 |
| 182 | 1-((1R,SR)-6-(8-fluoro-2-((((S)-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 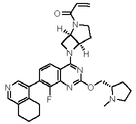 | 543.2 |
| 183 | 2-fluoro-1-(((1R,5R)-6-(8-fluoro-2-(((S)-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 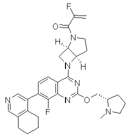 | 561 |
| 184 | 1-((1R,5R)-6-(8-fluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 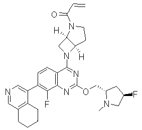 | 561 |
| 185 | 2-fluoro-1-(((1R,5R)-6-(8-fluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 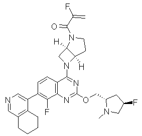 | 579 |
| 186 | 1-((1R,5R)-6-(8-fluoro-2-((tetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 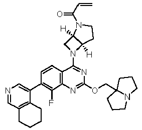 | 569 |
| 187 | 2-fluoro-1-(((1R,SR)-6-(8-fluoro-2-((tetrahydro-1H-pyrrolizin-7a(5H)-yl)methoxy))-7-(5,6,7,8-tetrahydroisoquinolin-4-yl)quinazolin-4-yl)-2,6-diazabicyclo[3.2.0]hept-2-yl)prop-2-en-1-one | 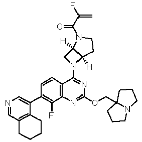 | 587 |
| 188 | 1-(((1R,SR)-6-(8-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 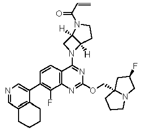 | 587 |
| 189 | 2-fluoro-1-(((1R,5R)-6-(8-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 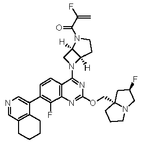 | 605 |
| 190 | 2-((S)-1-acryloyl-4-(7-(5-chloroisoquinolin-4-yl)-6-fluoro-2-(((S)-1-methylpyrrolidin-2-yl)methoxy)quinazolin-4-yl)piperazin-2-yl)acetonitrile | 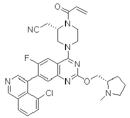 | 600.2 |
| 191 | 2-((S)-4-(7-(5-chloroisoquinolin-4-yl)-6-fluoro-2-((((S)-1-methylpyrrolidin-2-yl)methoxy)quinazolin-4-yl)-1-(2-fluoroacryloyl)piperazin-2-yl)acetonitrile | 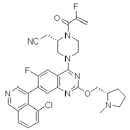 | 618.2 |
| 192 | 2-((S)-1-acryloyl-4-(7-(5-chloroisoquinolin-4-yl)-6-fluoro-2-((((2S,4R)-4-fluoro-1-methylpyrrolidin-2-(yl)methoxy)quinazolin-4-yl)piperazin-2-yl)acetonitrile | 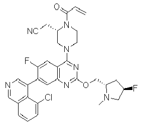 | 618.2 |
| 193 | 2-((S)-4-(7-(5-chloroisoquinolin-4-yl)-6-fluoro-2-((((2S,4R)-4-fluoro-1-methylpyrrolidin-2-yl)methoxy)quinazolin-4-yl)-1-(2-fluoroacryloyl)piperazin-2-yl)acetonitrile | 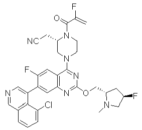 | 636.2 |
| 194 | (S)-2-(1-acryloyl-4-(7-(5-chloroisoquinolin-4-yl)-6-fluoro-2-((tetrahydro-1H-pyrrolizin-7a(5H)-yl)methoxy)quinazolin-4-yl)piperazin-2-yl)acetonitrile | 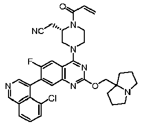 | 626.2 |
| 195 | (S)-2-(4-(7-(5-chloroisoquinolin-4-yl)-6-fluoro-2-((tetrahydro-1H-pyrrolizin-7a(5H)-yl)methoxy)quinazolin-4-yl)-1-(2-fluoroacryloyl)piperazin-2-yl)acetonitrile | 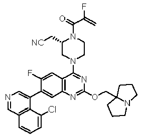 | 644.3 |
| 196 | 2-((2S)-1-acryloyl-4-(7-(5-chloroisoquinolin-4-yl)-6-fluoro-2-((((2R,7aS)-2-fluorotetrahydro-1H-pyrrolizin-7a(5H))-yl)methoxy)quinazolin-4-yl)piperazin-2-yl)acetonitrile | 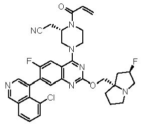 | 644.3 |
| 197 | 2-((2S)-4-(7-(5-chloroisoquinolin-4-yl)-6-fluoro-2-((((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 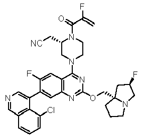 | 662.3 |
| 198 | 1-((1R,SR)-6-(7-(5-chloroisoquinolin-4-yl)-6-fluoro-2-(((S)-1-methylpyrrolidin-2-yl)methoxy)quinazolin-4-yl)-2,6-diazabicyclo[3.2.0]hept-2-yl)prop-2-en-1-one | 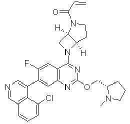 | 573.2 |
| 199 | 1-((1R,SR)-6-(7-(5-chloroisoquinolin-4-yl)-6-fluoro-2-(((S)-1-methylpyrrolidin-2-yl)methoxy)quinazolin-4-yl)-2,6-diazabicyclo[3.2.0]hept-2-yl)-2-fluoroprop-2-en-1-one | 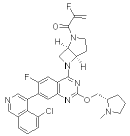 | 591.2 |
| 200 | 1-(((1R,5R)-6-(7-(5-chloroisoquinolin-4-yl)-6-fluoro-2-((((2S,4R)-4-fluoro-1-methylpyrrolidin-2-yl))methoxy)quinazolin-4-yl)-2,6-diazabicyclo[3.2.0]hept-2-yl)prop-2-en-1-one | 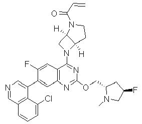 | 591 |
| 201 | 1-(((1R,5R)-6-(7-(5-chloroisoquinolin-4-yl)-6-fluoro-2-((((2S,4R)-4-fluoro-1-methylpyrrolidin-2-yl))methoxy)quinazolin-4-yl)-2,6-diazabicyclo[3.2.0]hept-2-yl)-2-fluoroprop-2-en-1-one | 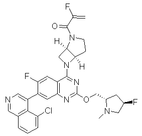 | 609 |
| 202 | 1-((1R,SR)-6-(7-(5-chloroisoquinolin-4-yl)-6-fluoro-2-((tetrahydro-1H-pyrrolizin-7a(5H)-yl)methoxy)quinazolin-4-yl)-2,6-diazabicyclo[3.2.0]hept-2-yl)prop-2-en-1-one | 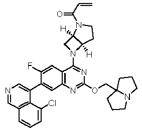 | 599.2 |
| 203 | 1-((1R,SR)-6-(7-(5-chloroisoquinolin-4-yl)-6-fluoro-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 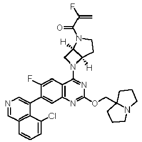 | 617.2 |
| 204 | 1-(((1R,5R)-6-(7-(5-chloroisoquinolin-4-yl)-6-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 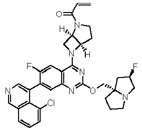 | 617.2 |
| 205 | 1-(((1R,5R)-6-(7-(5-chloroisoquinolin-4-yl)-6-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)-2-fluoroprop-2-en-1-one | 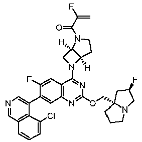 | 635 |
| 206 | 2-((S)-1-acryloyl-4-(6-fluoro-2-(((S)-1-methylpyrrolidin-2-yl)methoxy)-7-(5,6,7,8-tetrahydroisoquinoline-4-yl)quinazolin-4-yl)piperazin-2-yl)acetonitrile | 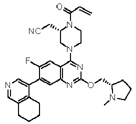 | 570 |
| 207 | 2-((S)-4-(6-fluoro-2-(((S)-1-methylpyrrolidin-2-yl)methoxy)-7-(5,6,7,8-tetrahydroisoquinolin-4-yl)quinazolin-4-yl)-1-(2-fluoroacryloyl)piperazin-2-yl)acetonitrile | 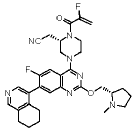 | 588 |
| 208 | 2-((S)-1-acryloyl-4-(6-fluoro-2-((((2S,4R)-4-fluoro-1-methylpyrrolidin-2-yl)methoxy)-7-(5,6,7,8-tetrahydroisoquinolin-4-yl)quinazolin-4-ylpiperazin-2-vl)acetonitrile | 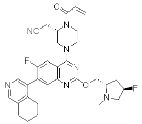 | 588 |
| 209 | 2-((S)-4-(6-fluoro-2-((((2S,4R)-4-fluoro-1-methylpyrrolidin-2-yl)methoxy)-7-(5,6,7,8-tetrahydroisoquinolin-4-yl)quinazolin-4-yl)-1-(2-fluoroacryloyl)piperazin-2-yl)acetonitrile | 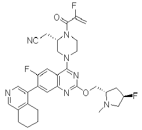 | 606 |
| 210 | (S)-2-(1-acryloyl-4-(6-fluoro-2-((tetrahydro-1H-pyrrolizin-7a(5H)-yl)methoxy)-7-(5,6,7,8-tetrahydroisoquinolin-4-yl)quinazolin-4-ylpiperazin-2-yl)acetonitrile | 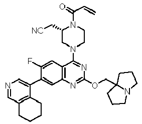 | 596 |
| 211 | (S)-2-(4-(6-fluoro-2-((tetrahydro- 1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 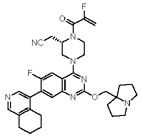 | 614 |
| 212 | 2-((S)-1-acryloyl-4-(6-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)piperazin-2-yl)acetonitrile | 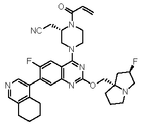 | 614 |
| 213 | 2-((2S)-4-(6-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 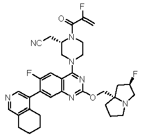 | 632 |
| 214 | 1-((1R,SR)-6-(6-fluoro-2-((((S)-1-methylpyrrolidin-2-yl]methoxy)-7-(5,6,7,8-tetrahydroisoquinolin-4-yl)quinazolin-4-yl)-2,6-diazabicyclo[3.2.0]hept-2-yl)prop-2-en-1-one | 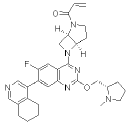 | 543 |
| 215 | 2-fluoro-1-(((1R,SR)-6-(6-fluoro-2-((((S)-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 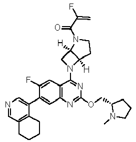 | 561 |
| 216 | 1-((1R,5R)-6-(6-fluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 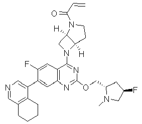 | 561 |
| 217 | 2-fluoro-1-(((1R,5R)-6-(6-fluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 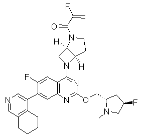 | 579 |
| 218 | 1-((1R,5R)-6-(6-fluoro-2-((tetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 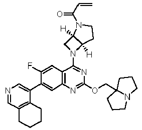 | 569 |
| 219 | 2-fluoro-1-(((1R,SR)-6-(6-fluoro-2-((tetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 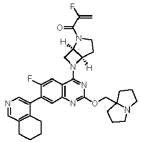 | 587 |
| 220 | 1-(((1R,5R)-6-(6-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 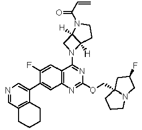 | 587 |
| 221 | 2-fluoro-1-(((1R,5R)-6-(6-fluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 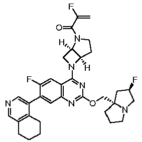 | 605 |
| 222 | 2-((2S)-1-acryloyl-4-(7-(5-chloroisoquinolin-4-yl)-6,8-difluoro-2-((((S)-1-methylpyrrolidin-2-yl)methoxy)quinazolin-4-yl)piperazin-2-yl)acetonitrile | 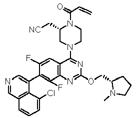 | 618 |
| 223 | 2-((2S)-4-(7-(5-chloroisoquinolin-4-yl)-6,8-difluoro-2-((((S)-1-methylpyrrolidin-2-yl)methoxy)quinazolin-4-yl)-1-(2-fluoroacryloyl)piperazin-2-yl)acetonitrile | 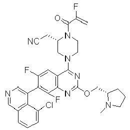 | 636 |
| 224 | 2-((2S)-1-acryloyl-4-(7-(5-chloroisoquinolin-4-yl)-6,8-difluoro-2-((((2S,4R)-4-fluoro-1-methylpyrrolidon-2-yl)methoxy)quinazolin-4-ylpiperazin-2-yl)acetonitrile | 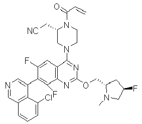 | 636 |
| 225 | 2-((2S)-4-(7-(5-chloroisoquinolin-4-yl)-6, 8-difluoro-2-((((2S,4R)-4-fluoro-1-methylpyrrolidin-2-yl)methoxy)quinazolin-4-yl)-1-(2-fluoroacryloyl)piperazin-2-yl)acetonitrile | 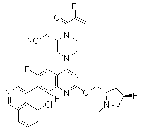 | 654 |
| 226 | 2-((2S)-1-acryloyl-4-(7-(5-chloroisoquinolin-4-yl)-6,8-difluoro-2-((tetrahydro-1H-pyrrolizin-7a(5H)-yl)methoxy)quinazolin-4-yl)piperazin-2-yl)acetonitrile | 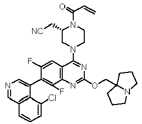 | 644 |
| 227 | 2-((2S)-4-(7-(5-chloroisoquinolin-4-yl)-6,8-difluoro-2-((tetrahydro-1H-pyrrolizin-7a(5H)-yl)methoxy)quinazolin-4-yl)-1-(2-fluoroacryloyl)piperazin-2-yl)acetonitrile | 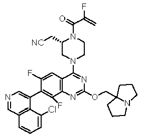 | 662 |
| 228 | 2-((2S)-1-acryloyl-4-(7-(5-chloroisoquinolin-4-yl)-6,8-difluoro-2-((((2R,7aS)-2-fluorotetrahydro-1H-pyrrolizin-7a(5H)-yl)methoxy)quinazolin-4-yl)piperazin-2-yl)acetonitrile | 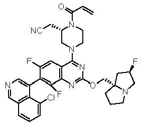 | 662 |
| 229 | 2-((2S)-4-(7-(5-chloroisoquinolin-4-yl)-6,8-difluoro-2-((((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 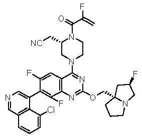 | 680 |
| 230 | 1-((1R,SR)-6-(7-(5-chloroisoquinolin-4-yl)-6,8-difluoro-2-(((S)-1-methylpyrrolidin-2-yl)methoxy)quinazolin-4-yl)-2,6-diazabicyclo[3.2.0]hept-2-yl)prop-2-en-1-one | 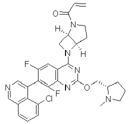 | 591 |
| 231 | 1-((1R,SR)-6-(7-(5-chloroisoquinolin-4-yl)-6,8-difluoro-2-(((S)-1-methylpyrrolidin-2-yl)methoxy)quinazolin-4-yl)-2,6-diazabicyclo[3.2.0]hept-2-yl)-2-fluoroprop-2-en-1-one | 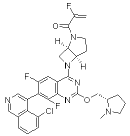 | 609 |
| 232 | 1-(((1R,SR)-6-(7-(5-chloroisoquinolin-4-yl)-6,8-difluoro-2-((((2S,4R)-4-fluoro-1 -methylpyrrolidin-2-yl)methoxy)quinazolin-4-yl)-2,6-diazabicyclo[3.2.0]hept-2-yl)prop-2-en-1-one | 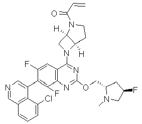 | 609 |
| 233 | 1-(((1R,SR)-6-(7-(5-chloroisoquinolin-4-yl)-6,8-difluoro-2-((((2S,4R)-4-fluoro-1-methylpyrrolidin-2-yl)methoxy)quinazolin-4-yl)-2,6-diazabicyclo[3.2.0]hept-2-yl)-2-fluoroprop-2-en-1-one | 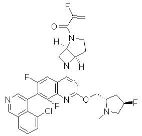 | 627 |
| 234 | 1-(((1R,SR)-6-(7-(5-chloroisoquinolin-4-yl)-6,8-difluoro-2-((tetrahydro-1H-pyrrolizin-7a(5H)-yl)methoxy)quinazolin-4-yl)-2,6-diazabicyclo[3.2.0]hept-2-yl)prop-2-en-1-one | 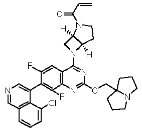 | 617 |
| 235 | 1-(((1R,SR)-6-(7-(5-chloroisoquinolin-4-yl)-6,8-difluoro-2-((tetrahydro-1H-pyrrolizin-7a(5H)-yl)methoxy)quinazolin-4-yl)-2,6-diazabicyclo[3.2.0]hept-2-yl)-2-fluoroprop-2-en-1-one | 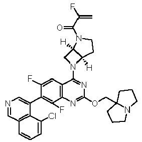 | 635 |
| 236 | 1-(((1R,SR)-6-(7-(5-chloroisoquinolin-4-yl)-6,8-difluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 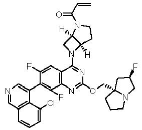 | 635 |
| 237 | 1-(((1R,SR)-6-(7-(5-chloroisoquinolin-4-yl)-6,8-difluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)-2-fluoroprop-2-en-1-one | 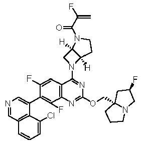 | 653 |
| 238 | 2-((2S)-1-acryloyl-4-(6,8-difluoro-2-((((S)-1-methylpyrrolidin-2-yl)methoxy)-7-(5,6,7,8-tetrahydroisoquinolin-4-yl)quinazolin-4-ylpiperazin-2-yl)acetonitrile | 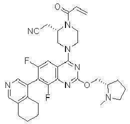 | 588 |
| 239 | 2-((2S)-4-(6,8-difluoro-2-(((S)-1-methylpyrrolidin-2-yl)methoxy)-7-(5,6,7,8-tetrahydroisoquinolin-4-yl)quinazolin-4-yl)-1-(2-fluoroacryloyl)piperazin-2-yl)acetonitrile | 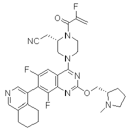 | 606 |
| 240 | 2-((2S)-1-acryloyl-4-(6,8-difluoro-2-((((2S,4R)-4-fluoro- 1-methylpyrrolidin-2-yl)methoxy)-7-(5,6,7,8-tetrahydroisoquinolin-4-yl)quinazolin-4-yl)piperazin-2-yl)acetonitrile | 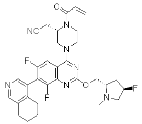 | 606 |
| 241 | 2-((2S)-4-(6,8-difluoro-2-((((2S,4R)-4-fluoro-1-methylpyrrolidin-2-yl)methoxy)-7-(5,6,7,8-tetrahydroisoquinolin-4-yl)quinazolin-4-yl)-1-(2-fluoroacryloyl)piperazin-2-yl)acetonitrile | 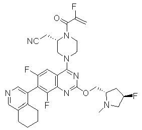 | 624 |
| 242 | 2-((2S)-1-acryloyl-4-(6,8-difluoro-2-(((tetrahydro-1H-pyrrolizin-7a(5H)-yl)methoxy)-7)((5,6,7,8-tetrahydroisoquinolin-4-yl)quinazolin-4-ylpiperazin-2-yl)acetonitrile | 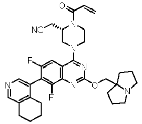 | 614 |
| 243 | 2-((2S)-4-(6,8-difluoro-2-((tetrahydro-1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 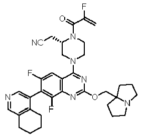 | 632 |
| 244 | 2-((2S)-1-acryloyl-4-(6,8-difluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)piperazin-2-yl)acetonitrile | 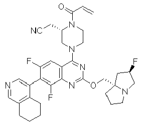 | 632 |
| 245 | 2-((2S)-4-(6,8-difluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-1-(2-fluoroacryloyl)piperazin-2-yl)acetonitrile | 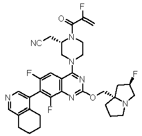 | 650 |
| 246 | 1-((1R,SR)-6-(6,8-difluoro-2-(((S)-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 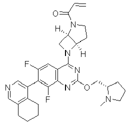 | 561 |
| 247 | 1-((1R,5R)-6-(6,8-difluoro-2-((((S)-1-methylpyrrolidin-2-yl)methoxy)-7-(5,6,7,8-tetrahydroisoquinoline-4-yl)quinazolin-4-yl)-2,6-diazabicyclo[3.2.0]hept-2-yl)-2-fluoroprop-2-en-1-one | 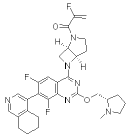 | 579 |
| 248 | 1-((1R,5R)-6-(6,8-difluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)prop-2-en-1-one | 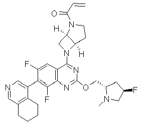 | 579 |
| 249 | 1-((1R,5R)-6-(6,8-difluoro-2-((((2S,4R)-4-fluoro-1-methylpyrrolidin-2-yl)methoxy)-7-(5,6,7,8-tetrahydroisoquinolin-4-yl)quinazolin-4-yl)-2,6-diazabicyclo[3.2.0]hept-2-yl)-2-fluoroprop-2-en-1-one | 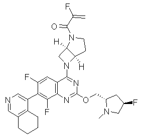 | 597 |
| 250 | 1-(((1R,5R)-6-(6,8-difluoro-2-((tetrahydro-1H-pyrrolizin-7a(5H)-yl)methoxy))-7-(5,6,7,8-tetrahydroisoquinolin-4-yl)quinazolin-4-yl)-2,6-diazabicyclo[3.2.0]hept-2-yl)prop-2-en-1-one | 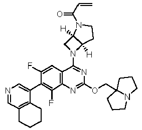 | 587 |
| 251 | 1-((1R,SR)-6-(6,8-difluoro-2-((tetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)-2-fluoroprop-2-en-1-one | 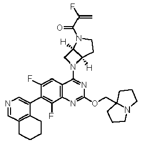 | 605 |
| 252 | 1-(((1R,SR)-6-(6,8-difluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)prop-2-en-1-one | 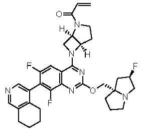 | 605 |
| 253 | 1-(((1R,SR)-6-(6,8-difluoro-2-((((2R,7aS)-2-fluorotetrahydro-1H-pyrrolizin-7a(5H)-yl)methoxy)-7-(5,6,7,8-tetrahydroisoquinolin-4-yl)quinazolin-4-yl)-2,6-diazabicyclo[3.2.0]hept-2-yl)-2-fluoroprop-2-en-1-one | 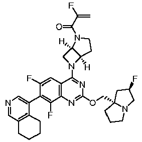 | 623 |
| 254 | 2-((S)-1-acryloyl-4-(7-(8-chloronaphthalen-1-yl)-2-(((S)-1-methylpyrrolidin-2-yl)methoxy)pyridino[2,3-d]pyrimidin-4-yl)piperazin-2-yl)acetonitrile | 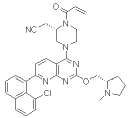 | 582 |
| 255 | 2-((S)-4-(7-(8-chloronaphthalen-1-yl)-2-((((S)-1-methylpyrrolidin-2-yl)methoxy)pyridino[2,3-d]pyrimidin-4-yl)-1-(2-fluoroacryloyl)piperazin-2-yl)acetonitrile | 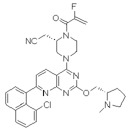 | 600 |
| 256 | 2-((S)-1-acryloyl-4-(7-(8-chloronaphthalen-1-yl)-2-((((2S,4R)-4-fluoro-1-methylpyrrolidin-2-yl]methoxy))pyridino[2,3-d]pyrimidin-4-yl)piperazin-2-yl)acetonitrile | 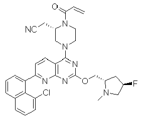 | 600 |
| 257 | 2-((S)-4-(7-(8-chloronaphthalen-1-yl)-2-((((2S,4R)-4-fluoro-1-methylpyrrolidin-2-yl)methoxy)pyridino [2,3- d]pyrimidin-4-yl)-1-(2-fluoroacryloyl)piperazin-2-yl)acetonitrile | 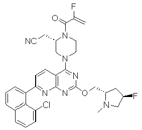 | 618.2 |
| 258 | (S)-2-(1-acryloyl-4-(7-(8-chloronaphthalen-1-yl)-2-((tetrahydro-1H-pyrrolizin-7a(5H)-yl)methoxy)pyridino [2,3 -d]pyrimidin-4-yl)piperazin-2-yl)acetonitrile | 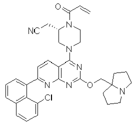 | 608 |
| 259 | (S)-2-(4-(7-(8-chloronaphthalen-1-yl)-2-((tetrahydro-1H-pyrrolizin-7a(5H)-yl)methoxy)pyridino[2,3-d]pyrimidin-4-yl)-1-(2-fluoroacryloyl)piperazin-2-yl)acetonitrile | 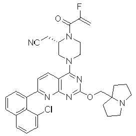 | 626 |
| 260 | 2-((S)-1-acryloyl-4-(7-(8-chloronaphthalen-1-yl)-2-(((((2R,7aS)-2-fluorotetrahydro-1H-pyrrolizin-7a(5H)-yl)methoxy)pyridino[2,3-d]pyrimidin-4-yl)piperazin-2-yl)acetonitrile | 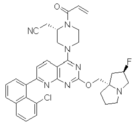 | 626 |
| 261 | 2-((S)-4-(7-(8-chloronaphthalen-1-yl)-2-(((((2R,7aS)-2-fluorotetrahydro-1H-pyrrolizin-7 a(5H)-yl)methoxy]pyridino[2,3-d]pyrimidin-4-yl)-1-(2-fluoroacryloyl)piperazin-2-yl)acetonitrile | 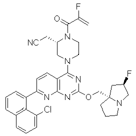 | 644 |
| 262 | 1-((1R,5R)-6-(7-(8-chloronaphthalen-1-yl)-2-((((S)-1-methylpyrrolidin-2--2-yl)methoxy)pyridino[2,3-d]pyrimidin-4-yl)-2,6-diazabicyclo[3.2.0]hept-2-yl)prop-2-en-1-one | 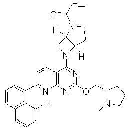 | 555 |
| 263 | 1-((1R,SR)-6-(7-(8-chloronaphthalen-1-yl)-2-((((S)-1-methylpyrrolidin-2--2-yl)methoxy)pyridino[2,3-d]pyrimidin-4-yl)-2,6-diazabicyclo[3.2.0]hept-2-yl)-2-fluoroprop-2-en-1-one | 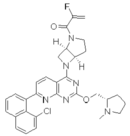 | 573.2 |
| 264 | 1-((1R,SR)-6-(7-(8-chloronaphthalen-1-yl)-2-((((2S,4R)-4-fluoro-1-methylpyrrolidin-2-yl)methoxy)pyridino[2,3-d]pyrimidin-4-yl)-2,6-diazabicyclo[3.2.0]hept-2-yl)prop-2-en-1-one | 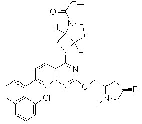 | 573.2 |
| 265 | 1-((1R,SR)-6-(7-(8-chloronaphthalen-1-yl)-2-((((2S,4R)-4-fluoro-1-methylpyrrolidin-2-yl)methoxy)pyridino[2,3-d]pyrimidin-4-yl)-2,6-diazabicyclo[3.2.0]hept-2-yl)-2-fluoroprop-2-en-1-one | 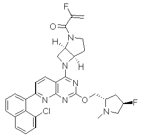 | 591 |
| 266 | 1-((1R,SR)-6-(7-(8-chloronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 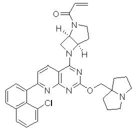 | 581 |
| 267 | 1-((1R,SR)-6-(7-(8-chloronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 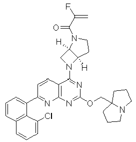 | 599 |
| 268 | 1-(((1R,5R)-6-(7-(8-chloronaphthalen-1-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 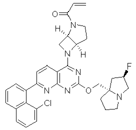 | 599 |
| 269 | 1-(((1R,5R)-6-(7-(8-chloronaphthalen-1-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 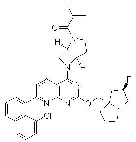 | 617 |
| 270 | 2-((S)-1-acryloyl-4-(7-(8-chloro-7-fluoronaphthalen-1-yl)-2-(((S)-1-methylpyrrolidin-2-yl)methoxy)pyridino [2,3 -d]pyrimidin-4-yl)piperazin-2-yl)acetonitrile | 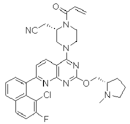 | 600.5 |
| 271 | 2-((S)-4-(7-(8-chloro-7-fluoronaphthalen-1-yl)-2-(((S)-1-methylpyrrolidin-2-yl)methoxy)pyridino[2,3-d]pyrimidin-4-yl)-1-(2-fluoroacryloyl)piperazin-2-yl)acetonitrile | 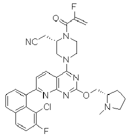 | 618 |
| 272 | 2-((S)-1-acryloyl-4-(7-(8-chloro-7-fluoronaphthalen-1-yl)-2-((((2S,4R)-4-fluoro-1-methylpyrrolidine-2-acyl)methoxy)pyridino [2, 3-d]pyrimidin-4-yl)piperazin-2-yl)acetonitrile | 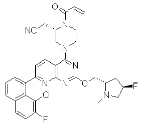 | 618 |
| 273 | 2-((S)-4-(7-(8-chloro-7-fluoronaphthalen-1-yl)-2-((((2S,4R)-4-fluoro-1-methylpyrrolidin-2-yl)methoxy)pyridino[2,3-d]pyrimidin-4-yl)-1 -(2-fluoroacryloyl)piperazin-2-yl)acetonitrile | 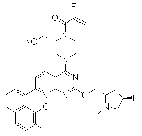 | 636 |
| 274 | (S)-2-(1-acryloyl-4-(7-(8-chloro-7-fluoronaphthalen-1-yl)-2-((tetrahydro-1H-pyrrolizin-7a(5H)-yl)methoxy)pyridino [2,3-d]pyrimidin-4-yl)piperazin-2-yl)acetonitrile | 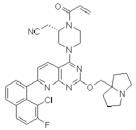 | 626 |
| 275 | (S)-2-(4-(7-(8-chloro-7-fluoronaphthalen-1-yl)-2-((tetrahydro-1H-pyrrolizin-7a(5H)-yl)methoxy)pyridino[2,3-d]pyrimidin-4-yl)-1 -(2-fluoroacryloyl)piperazin-2-yl)acetonitrile | 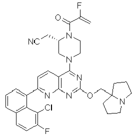 | 644 |
| 276 | 2-((S)-1-acryloyl-4-(7-(8-chloro-7-fluoronaphthalen-1-yl)-2-((((2R,7aS)-2-fluorotetrahydro-1H-pyrrolizin-7a(5H)-yl)methoxy)pyridino[2,3-d]pyrimidin-4-yl)piperazin-2-yl)acetonitrile | 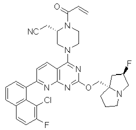 | 644 |
| 277 | 2-((S)-4-(7-(8-chloro-7-fluoronaphthalen-1-yl)-2-(((((2R,7aS)-2-fluorotetrahydro-1H-pyrrolizin-7a(5H)-yl)methoxy)pyridino[2,3-d]pyrimidin-4-yl)-1-(2-fluoroacryloyl)piperazin-2-yl)acetonitrile | 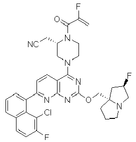 | 662 |
| 278 | 1-((1R,SR)-6-(7-(8-chloro-7-fluoronaphthalen-1-yl)-2-(((S)-1-methylpyrrolidin-2--2-yl)methoxy)pyridino[2,3-d]pyrimidin-4-yl)-2,6-diazabicyclo[3.2.0]hept-2-yl)prop-2-en-1-one | 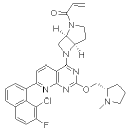 | 573 |
| 279 | 1-((1R,SR)-6-(7-(8-chloro-7-fluoronaphthalen-1-yl)-2-(((S)-1-methylpyrrolidin-2-yl)methoxy)pyridino[2,3-d]pyrimidin-4-yl)-2,6-diazabicyclo[3.2.0]hept-2-yl)-2-fluoroprop-2-en-1-one | 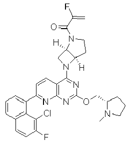 | 591 |
| 280 | 1-((1R,SR)-6-(7-(8-chloro-7-fluoronaphthalen-1-yl)-2-((((2S,4R)-4-fluoro-1-methylpyrrolidin-2-yl))methoxy)pyridino[2,3-d]pyrimidin-4-yl)-2,6-diazabicyclo[3.2.0]hept-2-yl)prop-2-en-1-one | 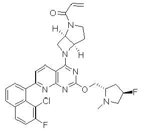 | 591 |
| 281 | 1-((1R,SR)-6-(7-(8-chloro-7-fluoronaphthalen-1-yl)-2-((((2S,4R)-4-fluoro-1-methylpyrrolidin-2-yl))methoxy)pyridino [2, 3-d]pyrimidin-4-yl)-2,6-diazabicyclo[3.2.0]hept-2-yl)-2-fluoroprop-2-en-1-one | 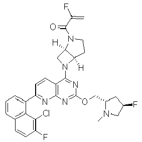 | 609 |
| 282 | 1-((1R,SR)-6-(7-(8-chloro-7-fluoronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 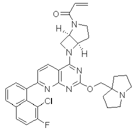 | 599 |
| 283 | 1-((1R,SR)-6-(7-(8-chloro-7-fluoronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 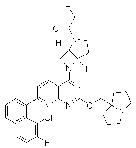 | 617 |
| 284 | 1-(((1R,SR)-6-(7-(8-chloro-7-fluoronaphthalen-1-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 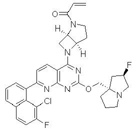 | 617 |
| 285 | 1-(((1R,SR)-6-(7-(8-chloro-7-fluoronaphthalen-1-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 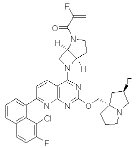 | 635 |
| 286 | 2-((S)-1-acryloyl-4-(2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile | 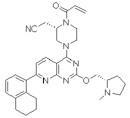 | 552 |
| 287 | 2-((S)-1-(2-fluoroacryloyl)-4-(2-((((S)-1-methylpyrrolidin-2-yl)methoxy)-7-(5,6,7,8-tetrahydronaphthalen-1-yl)pyridino [2,3 -d]pyrimidin-4-yl)piperazin-2-yl)acetonitrile | 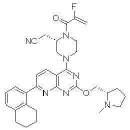 | 570 |
| 288 | 2-((S)-1-acryloyl-4-(2-((((2S,4R)-4-fluoro-1-methylpyrrolidin-2-yl)methoxy)-7-(5,6,7,8-tetrahydronaphthalen-1-yl)pyridino [2,3 -d]pyrimidin-4-yl)piperazin-2-yl)acetonitrile | 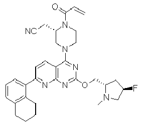 | 570 |
| 289 | 2-((S)-4-(2-(((((2S,4R)-4-fluoro-1-methylpyrrolidin-2-yl)methoxy)-7-(5,6,7,8-tetrahydronaphthalen-1)-yl)pyridino[2,3-d]pyrimidin-4-yl)-1-(2-fluoroacryloyl)piperazin-2-yl)acetonitrile | 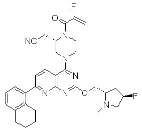 | 588 |
| 290 | (S)-2-(1-acryloyl-4-(2-(((tetrahydro-1H-pyrrolizin-7a(5H)-yl)methoxy))-7-(5,6,7,8-tetrahydronaphthalen-1-yl)pyridino [2,3 -d]pyrimidin-4-yl)piperazin-2-yl)acetonitrile | 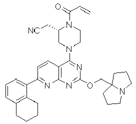 | 578 |
| 291 | (S)-2-(1-(2-fluoroacryloyl)-4-(2-((tetrahydro-1H-pyrrolizin-7a(5H)-yl)methoxy)-7-(5,6,7,8-tetrahydronaphthalen-1-yl)pyridino [2,3-d]pyrimidin-4-yl)piperazin-2-yl)acetonitrile | 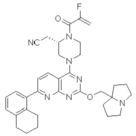 | 596 |
| 292 | 2-((2S)-1-acryloyl-4-(2-(((((2R,7aS)-2-fluorotetrahydro-1H-pyrrolizin-7a(5H)-yl)methoxy)-7-(5,6,7,8-tetrahydronaphthalen-1-yl)pyridino[2,3-d]pyrimidin-4-yl)piperazin-2-yl)acetonitrile | 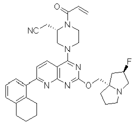 | 596 |
| 293 | 2-((2S)-1-(2-fluoroacryloyl)-4-(2-(((((2R,7aS)-2-fluorotetrahydro-1H-pyrrolizin-7a(5H)-yl)methoxy)-7-(5,6,7,8-tetrahydronaphthalen-1-yl)pyridino[2,3-d]pyrimidin-4-yl)piperazin-2-yl)acetonitrile | 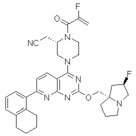 | 614 |
| 294 | 1-(((R),5R)-6-(2-((((S)-1-methylpyrrolidin-2-yl)methoxy)-7]-(5,6,7,8-tetrahydronaphthalen-1-yl)pyridino[2,3-d]pyrimidin-4-yl)-2,6-diazabicyclo[3.2.0]hept-2-yl)prop-2-en-1-one | 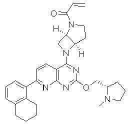 | 525 |
| 295 | 2-fluoro-1-(((1R,SR)-6-(2-((((S)-1-methylpyrrolidin-2-yl)methoxy)-7)-(5,6,7,8-tetrahydronaphthalen-1-yl)pyridino[2,3-d]pyrimidin-4-yl)-2,6-diazabicyclo[3.2.0]hept-2-yl)prop-2-en-1-one | 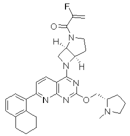 | 543 |
| 296 | 1-((1R,SR)-6-(2-((((2S,4R)-4-fluoro-1-methylpyrrolidin-2-yl)methoxy)-7-(5,6,7,8-tetrahydronaphthalen-1-yl)pyridino [2,3-d]pyrimidin-4-yl)-2,6-diazabicyclo[3.2.0]hept-2-yl)prop-2-en-1-one | 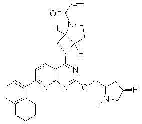 | 543 |
| 297 | 2-fluoro-1-(((1R,SR)-6-(2-((((2S,4R)-4-fluoro-1-methylpyrrolidin-2-yl)methoxy)-7-(5,6,7,8-tetrahydronaphthalen-1-yl)pyridino[2,3-d]pyrimidin-4-yl)-2,6-diazabicyclo[3.2.0]hept-2-yl)prop-2-en-1 -one | 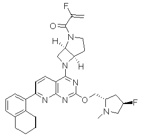 | 561 |
| 298 | 1-((1R,5R)-6-(2-(((tetrahydro-1H-pyrrolizin-7a(5H)-yl)methoxy)-7)((5,6,7,8-tetrahydronaphthalen-1-yl)pyridino[2,3-d]pyrimidin-4-yl)-2,6-diazabicyclo[3.2.0]hept-2-yl)prop-2-en-1-one | 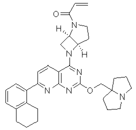 | 551 |
| 299 | 2-fluoro-1-(((1R,5R)-6-(2-((tetrahydro-1H-pyrrolizin-7a(5H)-yl)methoxy))-7-(5,6,7,8-tetrahydronaphthalen-1-yl)pyridino[2,3-d]pyrimidin-4-yl)-2,6-diazabicyclo[3.2.0]hept-2-yl)prop-2-en-1-one | 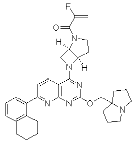 | 569 |
| 300 | 1-(((1R,SR)-6-(2-(((((2R,7aS)-2-fluorotetrahydro-1H-pyrrolizin-7a(5H)-yl)methoxy)-7-(5,6,7,8-tetrahydronaphthalen-1 -yl)pyridino [2,3 -d]pyrimidin-4-yl)-2,6-diazabicyclo[3.2.0]hept-2-yl)prop-2-en-1-one | 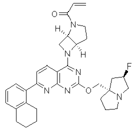 | 569 |
| 301 | 2-fluoro-1-(((1R,5R)-6-(2-((((2R,7aS)-2-fluorotetrahydro-1H-pyrrolizin-7a(5H)-yl)methoxy)-7-(5,6,7,8-tetrahydronaphthalen-1-yl)pyridino[2,3-d]pyrimidin-4-yl)-2,6-diazabicyclo[3.2.0]hept-2-yl)prop-2-en-1-one | 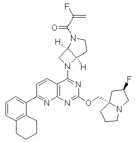 | 587 |
| 302 | 2-((S)-1-acryloyl-4-(7-(5-chloroisoquinolin-4-yl)-2-(((S)-1-methylpyrrolidin-2-yl)methoxy)pyridino[2,3-d]pyrimidin-4-yl)piperazin-2-yl)acetonitrile | 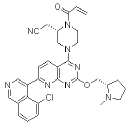 | 583 |
| 303 | 2-((S)-4-(7-(5-chloroisoquinolin-4-yl)-2-((((S)-1-methylpyrrolidin-2-yl)methoxy)pyridino[2,3- d]pyrimidin-4-yl)-1-(2-fluoroacryloyl)piperazin-2-yl)acetonitrile | 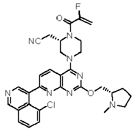 | 601 |
| 304 | 2-((S)-1-acryloyl-4-(7-(5-chloroisoquinolin-4-yl)-2-((((2S,4R)-4-fluoro-1-methylpyrrolidin-2-yl)methoxy))pyridino[2,3-d]pyrimidin-4-yl)piperazin-2-yl)acetonitrile | 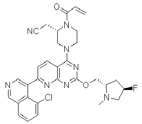 | 601 |
| 305 | 2-((S)-4-(7-(5-chloroisoquinolin-4-yl)-2-((((2S,4R)-4-fluoro-1-methylpyrrolidin-2-yl)methoxy)pyridino[2,3-d]pyrimidin-4-yl)-1-(2-fluoroacryloyl)piperazin-2-yl)acetonitrile | 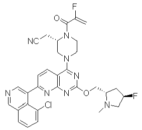 | 619 |
| 306 | (S)-2-(1-acryloyl-4-(7-(5-chloroisoquinolin-4-yl)-2-((tetrahydro-1H-pyrrolizin-7a(5H)-yl)methoxy)pyridino [2,3-d]pyrimidin-4-yl)piperazin-2-yl)acetonitrile | 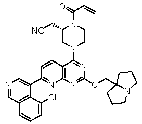 | 609 |
| 307 | (S)-2-(4-(7-(5-chloroisoquinolin-4-yl)-2-(((tetrahydro-1H-pyrrolizin-7a(5H)-yl)methoxy)pyridino[2,3-d]pyrimidin-4-yl)-1-(2-fluoroacryloyl)piperazin-2-yl)acetonitrile | 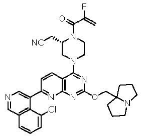 | 627 |
| 308 | 2-((2S)-1-acryloyl-4-(7-(5-chloroisoquinolin-4-yl)-2-((((2R,7aS)-2-fluorotetrahydro-1H-pyrrolizin-7a(5H)-yl)methoxy)pyridino[2,3-d]pyrimidin-4-yl)piperazin-2-yl)acetonitrile | 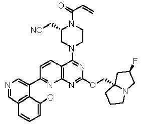 | 627 |
| 309 | 2-((2S)-4-(7-(5-chloroisoquinolin-4-yl)-2-((((2R,7aS)-2-fluorotetrahydro-1H-pyrrolizin-7a(5H)-yl)methoxy)pyridino[2,3-d]pyrimidin-4-yl)-1-(2-fluoroacryloyl)piperazin-2-yl)acetonitrile | 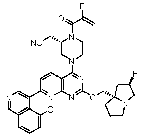 | 645 |
| 310 | 1-(((1R,5R)-6-(7-(5-chloroisoquinolin-4-yl)-2-(((S)-1-methylpyrrolidin-2--2-yl)methoxy)pyridino[2,3-d]pyrimidin-4-yl)-2,6-diazabicyclo[3.2.0]hept-2-yl)prop-2-en-1-one | 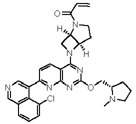 | 556 |
| 311 | 1-(((1R,5R)-6-(7-(5-chloroisoquinolin-4-yl)-2-(((S)-1-methylpyrrolidin-2--2-yl)methoxy)pyridino[2,3-d]pyrimidin-4-yl)-2,6-diazabicyclo[3.2.0]hept-2-yl)-2-fluoroprop-2-en-1-one | 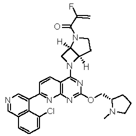 | 574 |
| 312 | 1-((1R,SR)-6-(7-(5-chloroisoquinolin-4-yl)-2-((((2S,4R)-4-fluoro-1-methylpyrrolidin-2-yl)methoxy)pyridino[2,3-d]pyrimidin-4-yl)-2,6-diazabicyclo[3.2.0]hept-2-yl)prop-2-en-1-one | 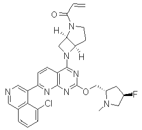 | 574 |
| 313 | 1-((1R,SR)-6-(7-(5-chloroisoquinolin-4-yl)-2-((((2S,4R)-4-fluoro-1-methylpyrrolidin-2-yl)methoxy)pyridino[2,3-d]pyrimidin-4-yl)-2,6-diazabicyclo[3.2.0]hept-2-yl)-2-fluoroprop-2-en-1-one | 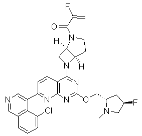 | 592 |
| 314 | 1-(((1R,SR)-6-(7-(5-chloroisoquinolin-4-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 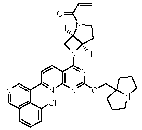 | 582 |
| 315 | 1-(((1R,SR)-6-(7-(5-chloroisoquinolin-4-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 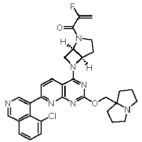 | 600 |
| 316 | 1-((1R,SR)-6-(7-(5-chloroisoquinolin-4-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 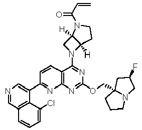 | 600 |
| 317 | 1-((1R,SR)-6-(7-(5-chloroisoquinolin-4-yl)-2-((((2R,7aS)-2-fluorotetrahydro-1H-pyrrolizin-7a(5H)-yl)methoxy)pyridino[2,3-d]pyrimidin-4-yl)-2,6-diazabicyclo[3.2.0]hept-2-yl)-2-fluoroprop-2-en-1-one | 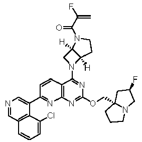 | 618 |
| 318 | 2-((S)-1-acryloyl-4-(2-((((S)-1-methylpyrrolidin-2-yl)methoxy)-7-(5,6,7,8-tetrahydroisoquinolin-4-yl)pyridino[2,3-d]pyrimidin-4-yl)piperazin-2-yl)acetonitrile | 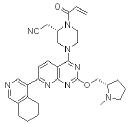 | 553 |
| 319 | 2-((S)-1-(2-fluoroacryloyl)-4-(2-((((S)-1-methylpyrrolidin-2-yl)methoxy)-7-(5,6,7,8-tetrahydroisoquinoline-4-yl)pyridino[2,3-d]pyrimidin-4-yl)piperazin-2-yl)acetonitrile | 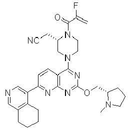 | 571 |
| 320 | 2-((S)-1-acryloyl-4-(2-((((2S,4R)-4-fluoro-1-methylpyrrolidin-2-yl)methoxy)-7-(5,6,7,8-tetrahydroisoquinolin-4-yl)pyridino[2,3-d] pyrimidin-4-yl)piperazin-2-yl)acetonitrile | 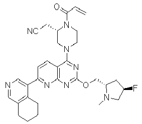 | 571 |
| 321 | 2-((S)-4-(2-(((((2S,4R)-4-fluoro-1-methylpyrrolidin-2-yl)methoxy)-7-(5,6,7,8-tetrahydroisoquinolin-4-yl)pyridino[2,3-d]pyrimidin-4-yl)-1-(2-fluoroacryloyl)piperazin-2-yl)acetonitrile | 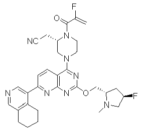 | 589 |
| 322 | (S)-2-(1-acryloyl-4-(2-(((tetrahydro-1H-pyrrolizin-7a(5H)-yl)methoxy))-7-(5,6,7,8-tetrahydroisoquinolin-4-yl)pyridino [2,3-d]pyrimidin-4-yl)piperazin-2-yl)acetonitrile | 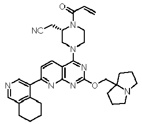 | 579 |
| 323 | (S)-2-(1-(2-fluoroacryloyl)-4-(2-((tetrahydro-1H-pyrrolizin-7a(5H)-yl)methoxy)-7-(5,6,7,8-tetrahydroisoquinolin-4-yl)pyridino[2,3-d]pyrimidin-4-yl)piperazin-2-yl)acetonitrile | 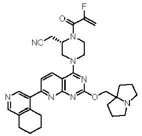 | 597 |
| 324 | 2-((2S)-1-acryloyl-4-(2-(((((2R,7aS)-2-fluorotetrahydro-1H-pyrrolizin-7a(5H)-yl)methoxy)-7-(5,6,7,8-tetrahydroisoquinolin-4-yl)pyridino[2,3-d]pyrimidin-4-yl)piperazin-2-yl)acetonitrile | 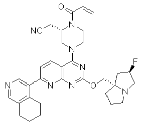 | 597 |
| 325 | 2-((2S)-1-(2-fluoroacryloyl)-4-(2-(((((2R,7aS)-2-fluorotetrahydro-1H-pyrrolizin-7a(5H)-yl)methoxy)-7-(5,6,7,8-tetrahydroisoquinolin-4-yl)pyridino[2,3-d]pyrimidin-4-yl)piperazin-2-yl)acetonitrile | 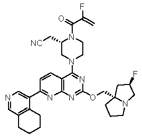 | 615 |
| 326 | 1-((1R,5R)-6-(2-((((S)-1-methylpyrrolidin-2-yl)methoxy)-7)-(5,6,7,8-tetrahydroisoquinolin-4-yl)pyridino[2,3-d]pyrimidin-4-yl)-2,6-diazabicyclo[3.2.0]hept-2-yl)prop-2-en-1-one | 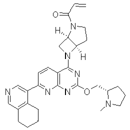 | 526 |
| 327 | 2-fluoro-1-(((1R,SR)-6-(2-((((S)-1-methylpyrrolidin-2-yl)methoxy)-7)-(5,6,7,8-tetrahydroisoquinolin-4-yl)pyridino[2,3-d]pyrimidin-4-yl)-2,6-diazabicyclo[3.2.0]hept-2-yl)prop-2-en-1-one | 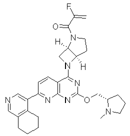 | 544 |
| 328 | 1-(((1R,5R)-6-(2-((((2S,4R)-4-fluoro-1-methylpyrrolidin-2-yl)methoxy)-7-(5,6,7,8-tetrahydroisoquinoline-4-yl)pyridino[2,3-d]pyrimidin-4-yl)-2,6-diazabicyclo[3.2.0]hept-2-yl)prop-2-en-1-one | 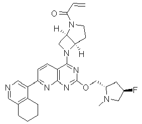 | 544 |
| 329 | 2-fluoro-1-(((1R,SR)-6-(2-((((2S,4R)-4-fluoro-1-methylpyrrolidin-2-yl)methoxy)-7-(5,6,7,8-tetrahydroisoquinolin-4-yl)pyridino[2,3-d]pyrimidin-4-yl)-2,6-diazabicyclo[3.2.0]hept-2-yl)prop-2-en-1-one | 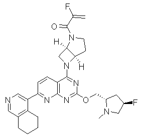 | 562 |
| 330 | 1-((1R,5R)-6-(2-(((tetrahydro-1H-pyrrolizin-7a(5H)-yl)methoxy)-7-(5,6,7,8-tetrahydroisoquinolin-4-yl)pyridino[2,3-d]pyrimidin-4-yl)-2,6-diazabicyclo[3.2.0]hept-2-yl)prop-2-en-1-one | 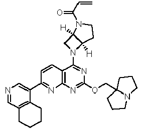 | 552 |
| 331 | 2-fluoro-1-(((1R,5R)-6-(2-((tetrahydro-1H-pyrrolizin-7a(5H)-yl)methoxy))-7-(5,6,7,8-tetrahydroisoquinolin-4-yl)pyridinyl [2, 3-d] pyrimidin-4-yl)-2,6-diazabicyclo[3.2.0]hept-2-yl)prop-2-en-1-one | 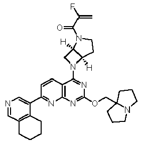 | 570 |
| 332 | 1-(((1R,SR)-6-(2-(((((2R)-2-fluorotetrahydro-1H-pyrrolizin-7a(5H)-yl)methoxy)-7-(5,6,7,8-tetrahydroisoquinolin-4-yl)pyridino[2,3-d]pyrimidin-4-yl)-2,6-diazabicyclor3.2.0]hept-2-yl)prop-2-en-1-one | 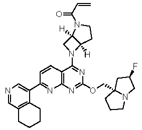 | 570 |
| 333 | 2-fluoro-1-(((1R,SR)-6-(2-((((2R)-2-fluorotetrahydro-1H-pyrrolizin-7a(5H)-yl)methoxy)-7-(5,6,7,8-tetrahydroisoquinolin-4-yl)pyridino[2,3-d]pyrimidin-4-yl)-2,6-diazabicyclo[3.2.0]hept-2-yl)prop-2-en-1-one | 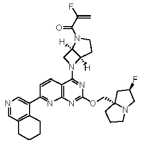 | 588 |
| 334 | 2-((S)-1-acryloyl-4-(7-(8-chloronaphthalen-1-yl)-6-fluoro-2-(((S)-1-methylpyrrolidin-2-yl)methoxy)pyridino [2, 3-d]pyrimidin-4-yl)piperazin-2-yl)acetonitrile | 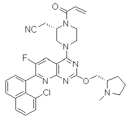 | 600 |
| 335 | (S)-2-(1-acryloyl-4-(7-(8-chloronaphthalen-1-yl)-6-fluoro-2-((tetrahydro-1H-pyrrolizin-7a(5H)-yl)methoxy)pyridino [2,3 -d]pyrimidin-4-yl)piperazin-2-yl)acetonitrile | 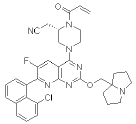 | 626 |
| 336 | 1-((1R,SR)-6-(7-(8-chloronaphthalen-1-yl)-6-fluoro-2-(((S)-1-methylpyrrolidin-2-yl)methoxy)pyridino[2,3-d]pyrimidin-4-yl)-2,6-diazabicyclo[3.2.0]hept-2-yl)prop-2-en-1-one | 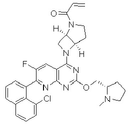 | 555 |
| 337 | 1-(((1R,5R)-6-(7-(8-chloronaphthalen-1-yl)-6-fluoro-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 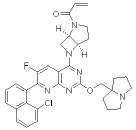 | 599 |
| 338 | 2-((S)-1-acryloyl-4-(7-(8-chloro-7-fluoronaphthalen-1-yl)-6-fluoro-2-(((S)-1-methylpyrrolidin-2-yl)methoxy)pyridino [2, 3-d]pyrimidin-4-yl)piperazin-2-yl)acetonitrile | 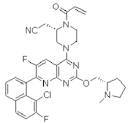 | 618 |
| 339 | (S)-2-(1-acryloyl-4-(7-(8-chloro-7-fluoronaphthalen-1-yl)-6-fluoro-2-((tetrahydro-1H-pyrrolizin-7a(5H)-yl)methoxy)pyridino [2, 3-d]pyrimidin-4-yl)piperazin-2-yl)acetonitrile | 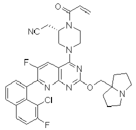 | 644 |
| 340 | 1-((1R,SR)-6-(7-(8-chloro-7-fluoronaphthalen-1-yl)-6-fluoro-2-(((S)-1-methylpyrrolidin-2-yl)methoxy)pyridino[2,3-d]pyrimidin-4-yl)-2,6-diazabicyclo[3.2.0]hept-2-yl)prop-2-en-1-one | 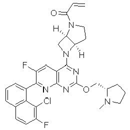 | 591 |
| 341 | 1-((1R,SR)-6-(7-(8-chloro-7-fluoronaphthalen-1-yl)-6-fluoro-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 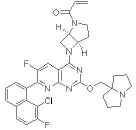 | 617 |
| 342 | 2-((S)-1-acryloyl-4-(6-fluoro-2-(((S)-1-methylpyrrolidin-2-yl)methoxy)-7-(5,6,7,8-tetrahydronaphthalen-1 -yl)pyridino [2,3 -d]pyrimidin-4-yl)piperazin-2-yl)acetonitrile | 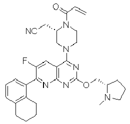 | 570 |
| 343 | (S)-2-(1-acryloyl-4-(6-fluoro-2-((tetrahydro-1H-pyrrolizin-7a(5H)-yl)methoxy)-7-(5,6,7,8-tetrahydronaphthalen-1-yl)pyridino[2,3-d]pyrimidin-4-yl)piperazin-2-yl)acetonitrile | 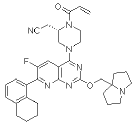 | 596 |
| 344 | 1-((1R,SR)-6-(6-fluoro-2-((((S)-1-methylpyrrolidin-2-yl)methoxy)-7]-(5,6,7,8-tetrahydronaphthalen-1-yl)pyridino[2,3-d]pyrimidin-4-yl)-2,6-diazabicyclo[3.2.0]hept-2-yl)prop-2-en-1-one | 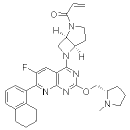 | 543 |
| 345 | 1-((1R,5R)-6-(6-fluoro-2-((tetrahydro-1H-pyrrolizin-7a(5H)-yl)methoxy)-7-(5,6,7,8-tetrahydronaphthalen-1-yl)pyridino[2,3-d]pyrimidin-4-yl)-2,6-diazabicyclo[3.2.0]hept-2-yl)prop-2-en-1-one | 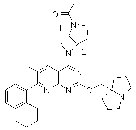 | 569 |
| 346 | 2-((S)-1-acryloyl-4-(6-chloro-7-(8-chloronaphthalen-1-yl)-2-(((S)-1-methylpyrrolidin-2-yl)methoxy)pyridino [2,3 -d]pyrimidin-4-yl)piperazin-2-yl)acetonitrile | 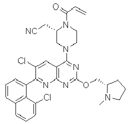 | 582 |
| 347 | (S)-2-(1-acryloyl-4-(6-chloro-7-(8-chloronaphthalen-1-yl)-2-((tetrahydro-1H-pyrrolizin-7a(5H)-yl)methoxy)pyridino [2,3 -d]pyrimidin-4-yl)piperazin-2-yl)acetonitrile | 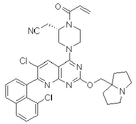 | 642 |
| 348 | 1-((1R,5R)-6-(6-chloro-7-(8-chloronaphthalen-1-yl)-2-((((S)-1-methylpyrrolidin-2--2-yl)methoxy)pyridino[2,3-d]pyrimidin-4-yl)-2,6-diazabicyclo[3.2.0]hept-2-yl)prop-2-en-1-one | 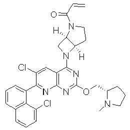 | 589 |
| 349 | 1-(((1R,5R)-6-(6-chloro-7-(8-chloronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 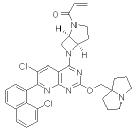 | 615 |
| 350 | 2-((S)-1-acryloyl-4-(6-chloro-7-(8-chloro-7 - fluoronaphthalen-1-yl)-2-(((S)-1-methylpyrrolidin-2-yl))methoxy)pyridino [2, 3-d]pyrimidin-4-yl)piperazin-2-yl)acetonitrile | 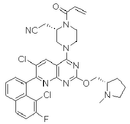 | 634 |
| 351 | (S)-2-(1-acryloyl-4-(6-chloro-7-(8-chloro-7- fluoronaphthalen-1-yl)-2-((tetrahydro-1H-pyrrolizin-7a(5H)-yl)methoxy)pyridino[2,3-d]pyrimidin-4-yl)piperazin-2-yl)acetonitrile | 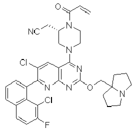 | 660 |
| 352 | 1-((1R,5R)-6-(6-chloro-7-(8-chloro-7-fluoronaphthalen-1-yl)-2-((((S)-1-methylpyrrolidin-2-yl)methoxy)pyridino[2,3-d]pyrimidin-4-yl)-2,6-diazabicyclo[3.2.0]hept-2-yl)prop-2-en-1-one | 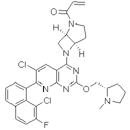 | 607 |
| 353 | 1-((1R,5R)-6-(6-chloro-7-(8-chloro-7-fluoronaphthalen-1-yl)-2-((tetrahydro-1H-pyrrolizin-7a(5H)-yl)methoxy)pyridino[2,3-d]pyrimidin-4-yl)-2,6-diazabicyclo[3.2.0]hept-2-yl)prop-2-en-1-one | 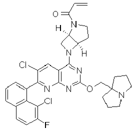 | 633 |
| 354 | 2-((S)-1-acryloyl-4-(6-chloro-2-(((S)-1-methylpyrrolidin-2-yl)methoxy)-7-(5,6,7,8-tetrahydronaphthalen-1-yl)pyridino[2,3-d]pyrimidin-4-yl)piperazin-2-yl)acetonitrile | 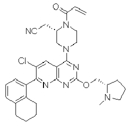 | 586 |
| 355 | (S)-2-(1-acryloyl-4-(6-chloro-2-((tetrahydro-1H-pyrrolizin-7a(5H)-yl)methoxy)-7-(5,6,7,8-tetrahydronaphthalen-1-yl)pyridino [2,3-d]pyrimidin-4-yl)piperazin-2-yl)acetonitrile | 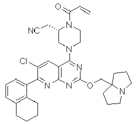 | 612 |
| 356 | 1-((1R,5R)-6-(6-chloro-2-(((S)-1-methylpyrrolidin-2-yl)methoxy)-7-(5,6,7,8-tetrahydronaphthalen-1-yl)pyridino[2,3-d]pyrimidin-4-yl)-2,6-diazabicyclo[3.2.0]hept-2-yl)prop-2-en-1-one | 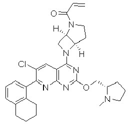 | 559 |
| 357 | 1-((1R,SR)-6-(6-chloro-2-((tetrahydro-1H-pyrrolizin-7a(5H)-yl)methoxy)-7-(5,6,7,8-tetrahydronaphthalen-1-yl)pyridino[2,3-d]pyrimidin-4-yl)-2,6-diazabicyclo[3.2.0]hept-2-yl)prop-2-en-1-one | 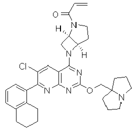 | 585 |
| 358 | 2-((S)-1-acryloyl-4-(7-(8-chloronaphthalen-1-yl)-8-fluoro-2-(((S)-1-methylpyrrolidin-2-yl)methoxy)quinazolin-4-yl)piperazin-2-yl)acetonitrile | 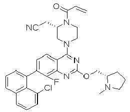 | 599.23 |
| 359 | 2-((S)-1-acryloyl-4-(7-(8-chloro-7-fluoronaphthalen-1-yl)-8-fluoro-2-(((S)-1-methylpyrrolidin-2-yl)methoxy)quinazolin-4-yl)piperazin-2-yl)acetonitrile | 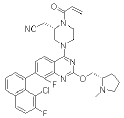 | 617.22 |
| 360 | 2-((S)-1-acryloyl-4-(8-fluoro-2-((((2S,4R)-4-fluoro-1-methylpyrrolidin-2-yl)methoxy)-7-(5,6,7,8-tetrahydronaphthalen-1 -ylquinazolin-4-ylpiperazin-2-ylacetonitrile | 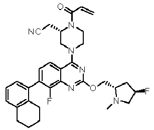 | 587.29 |
| 361 | 2-((S)-1-acryloyl-4-(7-(8-chloronaphthalen-1-yl)-8-fluoro-2-((((2S,4R)-4-fluoro-1-methylpyrrolidin-2-(yl)methoxy)quinazolin-4-yl)piperazin-2-yl)acetonitrile | 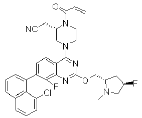 | 617.22 |
| 362 | 2-((S)-1-acryloyl-4-(7-(8-chloro-7-fluoronaphthalen-1-yl)-8-fluoro-2-((((2S,4R)-4-fluoro-1-methylpyrrolidin-2-yl)methoxy)quinazolin-4-ylpiperazin-2-yl)acetonitrile | 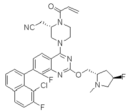 | 635.21 |
| 363 | (S)-2-(1-acryloyl-4-(8-fluoro-2-((tetrahydro-1H-pyrrolizin-7a(5H)-yl)methoxy)-7-(5,6,7,8-tetrahydronaphthalene)-1-yl)quinazolin-4-ylpiperazin-2-yl)acetonitrile | 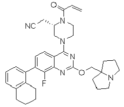 | 594.31 |
| 364 | (S)-2-(1-acryloyl-4-(7-(8-chloronaphthalen-1-yl)-8-fluoro-2-((tetrahydro-1H-pyrrolizin-7a(5H)-yl)methoxy)quinazolin-4-yl)piperazin-2-yl)acetonitrile | 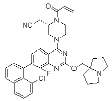 | 625.24 |
| 365 | (S)-2-(1-acryloyl-4-(7-(8-chloro-7-fluoronaphthalen-1-yl)-8-fluoro-2-((tetrahydro-1H-pyrrolizin-7a(5H)-yl)methoxy)quinazolin-4-yl)piperazin-2-yl)acetonitrile | 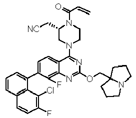 | 643.23 |
| 366 | 2-((2S)-1-acryloyl-4-(8-fluoro-2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)piperazin-2-yl)acetonitrile | 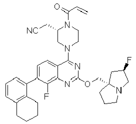 | 613.3 |
| 367 | 2-((2S)-1-acryloyl-4-(7-(8-chloronaphthalen-1-yl)-8-fluoro-2-((((2R,7aS)-2-fluorotetrahydro-1H-pyrrolizin-7a(5H))-yl)methoxy)quinazolin-4-yl)piperazin-2-yl)acetonitrile | 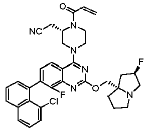 | 643.23 |
| 368 | 2-((2S)-1-acryloyl-4-(7-(8-chloro-7-fluoronaphthalen-1-yl)-8-fluoro-2-((((2R,7aS)-2-fluorotetrahydro-1H-pyrrolizin-7a(5H)-yl)methoxy)quinazolin-4-yl)piperazin-2-yl)acetonitrile | 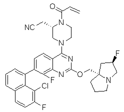 | 661.22 |
| 369 | 1-((1R,SS)-6-(8-fluoro-2-((((S)-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 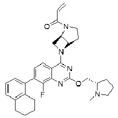 | 542.29 |
| 370 | 1-((1R,SS)-6-(7-(8-chloronaphthalen-1-yl)-8-fluoro-2-(((S)-1-methylpyrrolidin-2-yl)methoxy)quinazolin-4-yl)-2,6-diazabicyclo[3.2.0]hept-2-yl)prop-2-en-1-one | 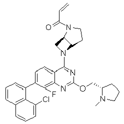 | 572.22 |
| 371 | 1-((1R,5S)-6-(7-(8-chloro-7-fluoronaphthalen-1-yl)-8-fluoro-2-((((S)-1-methylpyrrolidin-2-yl)methoxy)quinazolin-4-yl)-2,6-diazabicyclo[3.2.0]hept-2-yl)prop-2-en-1-one | 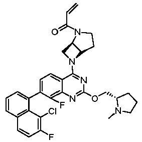 | 590.21 |
| 372 | 1-((1R,SS)-6-(8-fluoro-2-((((S)-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1 -one | 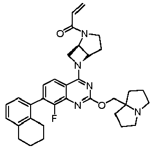 | 568.3 |
| 373 | 1-((1R,SS)-6-(7-(8-chloronaphthalen-1-yl)-8-fluoro-2-((tetrahydro-1H-pyrrolizin-7a(5H)-yl)methoxy)quinazolin-4-yl)-2,6-diazabicyclo[3.2.0]hept-2-yl)prop-2-en-1-one | 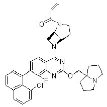 | 598.23 |
| 374 | 1-((1R,5S)-6-(7-(8-chloro-7-fluoronaphthalen-1-yl)-8-fluoro-2-((tetrahydro-1H-pyrrolizin-7a(5H)-yl)methoxy)quinazolin-4-yl)-2,6-diazabicyclo[3.2.0]hept-2-yl)prop-2-en-1-one | 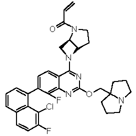 | 616.22 |
| 375 | 1-(((1R,5S)-6-(2-((((2R,7aS)-2-fluorotetrahydro-1H-pyrrolizin-7a(5H)-yl)methoxy]]-7-(5,6,7,8-tetrahydronaphthalen-1-yl)-8-fluoro-quinazolin-5-yl)-2,6-diazabicyclo[3.2.0]hept-2-yl)prop-2-en-1-one | 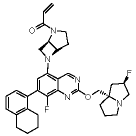 | 586.29 |
| 376 | 1-(((1R,SS)-6-(8-fluoro-7-(8-chloronaphthalen-1-yl)-8-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 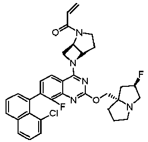 | 616.22 |
| 377 | 1-(((1R,SS)-6-(7-(8-chloro-7-fluoronaphthalen-1-yl)-8-fluoro-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 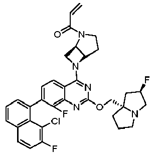 | 634.21 |
| 378 | 1-((1R,5S)-6-(2-((((2S,4R)-4-fluoro-1-methylpyrrolidin-2-yl)methoxy)-7-(5,6,7,8-tetrahydronaphthalen-1-yl)8-fluoro-quinazolin-4-yl)-2,6-diazabicyclo[3.2.0]hept-2-yl)prop-2-en-1-one | 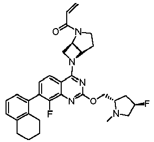 | 560.28 |
| 379 | 1-((1R,5S)-6-(8-fluoro-7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 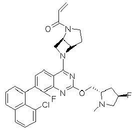 | 590.21 |
| 380 | 1-(((1R,5S)-6-(8-fluoro-7-(8-chloro-7-fluoronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 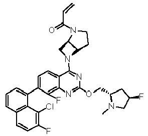 | 608.2 |
| 381 | 1-((S)-4-(8-fluoro-2-(((S)-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 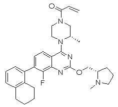 | 544.3 |
| 382 | 1-((S)-4-(8-fluoro-7-(8-chloronaphthalen-1-yl)-2-(((S)-1-methylpyrrolidin-2-yl)methoxy)quinazolin-4-yl)-3-methylpiperazin-1 -yl)prop-2-en-1 -one | 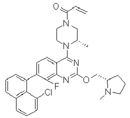 | 574.23 |
| 383 | 1-((S)-4-(8-fluoro-7-(8-chloro-7-fluoronaphthalen-1-yl)-2-(((S)-1-methylpyrrolidin-2-yl)methoxy)quinazolin-4-yl)-3-methylpiperazin-1-yl)prop-2-en-1-one | 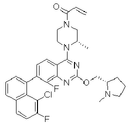 | 592.22 |
| 384 | (S)-1-(4-(8-fluoro-2-((tetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 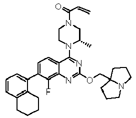 | 570.32 |
| 385 | (S)-1-(4-(8-fluoro-7-(8-chloronaphthalen-1-yl)-2-((tetrahydro-1H-pyrrolizin-7a(5H)-yl)methoxy)quinazolin-4-yl)-3-methylpiperazin-1-yl)prop-2-en-1-one | 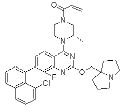 | 600.25 |
| 386 | (S)-1-(4-(8-fluoro-7-(8-chloro-7-fluoronaphthalen-1-yl)-2-((tetrahydro-1H-pyrrolizin-7a(5H)-yl)methoxy)quinazolin-4-yl)-3-methylpiperazin-1-yl)prop-2-en-1-one | 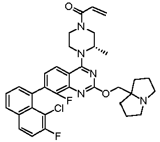 | 618.24 |
| 387 | 1-((3S)-4-(8-fluoro-2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 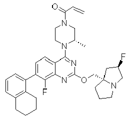 | 588.31 |
| 388 | 1-((3S)-4-(8-fluoro-7-(8-chloronaphthalen-1-yl)-2-((((2R,7aS)-2-fluorotetrahydro-1H-pyrrolizin-7a(5H)-yl]methoxy)quinazolin-4-yl)-3-methylpiperazin-1-yl)prop-2-en-1-one | 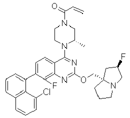 | 617.24 |
| 389 | 1-((3S)-4-(8-fluoro-7-(8-chloro-7-fluoronaphthalen-1-yl)-2-((((2R,7aS)-2-fluorotetrahydro-1H-pyrrolizine-7a(5H))-yl)methoxy)quinazolin-4-yl)-3-methylpiperazin-1-yl)prop-2-en-1-one | 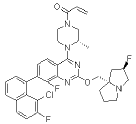 | 635.23 |
| 390 | 1-((S)-4-(8-fluoro--2-((((2S,4R)-4-fluoro-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 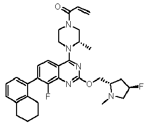 | 561.29 |
| 391 | 1-((S)-4-(8-fluoro-7-(8-chloronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 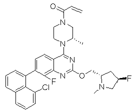 | 591.22 |
| 392 | 1-((S)-4-(8-fluoro-7-(8-chloro-7-fluoronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 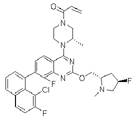 | 609.21 |
| 393 | 2-((S)-1-acryloyl-4-(6-fluoro-2-(((S)-1-methylpyrrolidin-2-yl)methoxy)-7-(5,6,7,8-tetrahydronaphthalen-1-yl)quinazolin-4-yl)piperazin-2-yl)acetonitrile | 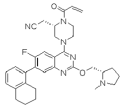 | 569.29 |
| 394 | 2-((S)-1-acryloyl-4-(6-fluoro-7-(8-chloronaphthalen-1-yl)-2-(((S)-1-methylpyrrolidin-2-yl)methoxy)quinazolin-4-yl)piperazin-2-yl)acetonitrile | 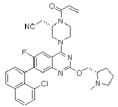 | 616.22 |
| 395 | 2-((S)-1-acryloyl-4-(6-fluoro-7-(8-chloro-7-fluoronaphthalen-1-yl)-2-(((S)-1-methylpyrrolidin-2-yl))methoxy)quinazolin-4-yl)piperazin-2-yl)acetonitrile | 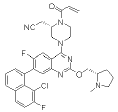 | 634.21 |
| 396 | 2-((S)-1-acryloyl-4-(6-fluoro-2-((((2S,4R)-4-fluoro-1-methylpyrrolidin-2-yl)methoxy)-7-(5,6,7,8-tetrahydronaphthalen-1 -ylquinazolin-4-ylpiperazin-2-ylacetonitrile | 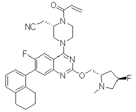 | 604.28 |
| 397 | 2-((S)-1-acryloyl-4-(6-fluoro-7-(8-chloronaphthalen-1-yl)-2-((((2S,4R)-4-fluoro-1-methylpyrrolidin-2-(yl)methoxy)quinazolin-4-yl)piperazin-2-yl)acetonitrile | 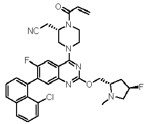 | 634.21 |
| 398 | 2-((S)-1-acryloyl-4-(6-fluoro-7-(8-chloro-7-fluoronaphthalen-1-yl)-2-((((2S,4R)-4-fluoro-1-methylpyrrolidin-2-yl)methoxy)quinazolin-4-ylpiperazin-2-yl)acetonitrile | 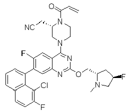 | 652.2 |
| 399 | (S)-2-(1-acryloyl-4-(6-fluoro-2-((tetrahydro-1H-pyrrolizin-7a(5H)-yl)methoxy)-7-(5,6,7,8-tetrahydronaphthalen-1-yl)quinazolin-4-ylpiperazin-2-yl)acetonitrile | 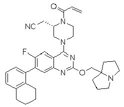 | 611.3 |
| 400 | 1-((1R,5S)-6-(6-fluoro-7-(8-chloronaphthalen-1-yl)-2-((tetrahydro-1H-pyrrolizin-7a(5H)-yl)methoxy)quinazolin-4-yl)--2,6-diazabicyclo[3.2.0]hept-2-yl)prop-2-en-1-one | 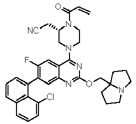 | 642.23 |
| 401 | 1-((1R,SS)-6-(6-fluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 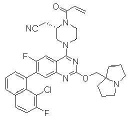 | 660.22 |
| 402 | 1-(((1R,5S)-6-(6-fluoro-2-((((2R,7aS)-2-fluorotetrahydro-1H-pyrrolizin-7a(5H)-yl)methoxy]]-7-(5,6,7,8-tetrahydronaphthalen-1-yl)quinazolin-5-yl)-2,6-diazabicyclo[3.2.0]hept-2-yl)prop-2-en-1-one | 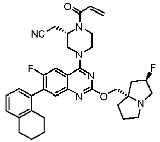 | 630.29 |
| 403 | 1-(((1R,SS)-6-(6-fluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 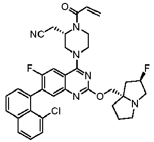 | 660.22 |
| 404 | 1-(((1R,5S)-6-(6-fluoro-7-(8-chloro-7-fluoronaphthalen-1-yl)-2-((((2R,7aS)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 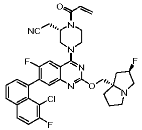 | 688.21 |
| 405 | 1-((1R,SS)-6-(6-fluoro-2-((((2S,4R)-4-fluoro-1-methylpyrrolidin-2-yl)methoxy)-7-(5,6,7,8-tetrahydronaphthalen-1 -yl)quinazolin-4-yl)-2,6-diazabicyclo[3.2.0]hept-2-yl)prop-2-en-1-one | 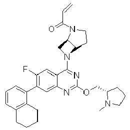 | 559.28 |
| 406 | 1-(((1R,SS)-6-(6-fluoro-7-(8-chloronaphthalen-1-yl)-2-((((2R)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 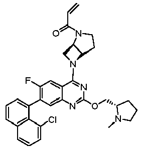 | 572.22 |
| 407 | 1-(((1R,5 S)-6-(6-fluoro-7-(8-chloro-7-fluoronaphthalen-1-yl)-2-((((2R)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 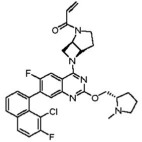 | 590.21 |
| 408 | 1-((1R,SS)-6-(6-fluoro-2-((tetrahydro-1H-pyrrolizin-7a(5H)-yl)methoxy)-7-(5,6,7,8-tetrahydronaphthalen-1-yl)quinazolin-4-yl)-2,6-diazabicyclo[3.2.0]hept-2-yl)prop-2-en-1-one | 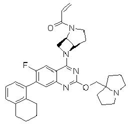 | 568.30 |
| 409 | 1-((1R,SS)-6-(6-fluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 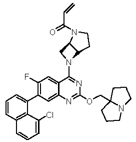 | 598.23 |
| 410 | 1-((1R,SS)-6-(6-fluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 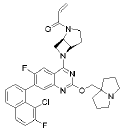 | 616.21 |
| 411 | 1-(((1R,5S)-6-(6-fluoro-2-((((2R,7aS)-2-fluorotetrahydro-1H-pyrrolizin-7a(5H)-yl)methoxy]]-7-(5,6,7,8-tetrahydronaphthalen-1-yl)quinazolin-5-yl)-2,6-diazabicyclo[3.2.0]hept-2-yl)prop-2-en-1-one | 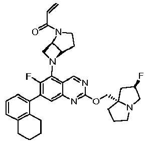 | 586.29 |
| 412 | 1-(((1R,SS)-6-(6-fluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 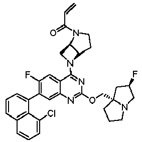 | 616.22 |
| 413 | 1-(((1R,5S)-6-(6-fluoro-7-(8-chloro-7-fluoronaphthalen-1-yl)-2-((((2R,7aS)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 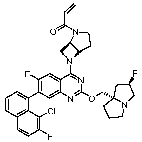 | 634.21 |
| 414 | 1-((1R,SS)-6-(6-fluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 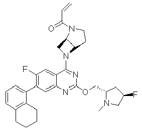 | 560.28 |
| 415 | 1-((1R,5S)-6-(6-fluoro-7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 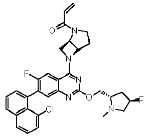 | 590.21 |
| 416 | 1-(((1R,5S)-6-(6-fluoro-7-(8-chloro-7-fluoronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 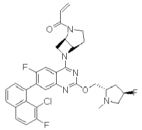 | 608.2 |
| 417 | 1-((S)-4-(6-fluoro-2-(((S)-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 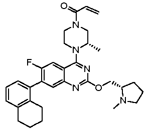 | 544.3 |
| 418 | 1-((S)-4-(6-fluoro-7-(8-chloronaphthalen-1-yl)-2-(((S)-1-methylpyrrolidin-2-yl)methoxy)quinazolin-4-yl)-3-methylpiperazin-1 -yl)prop-2-en-1 -one | 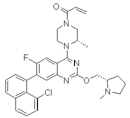 | 574.23 |
| 419 | 1-((S)-4-(6-fluoro-7-(8-chloro-7-fluoronaphthalen-1-yl)-2-(((S)-1-methylpyrrolidin-2-yl)methoxy)quinazolin-4-yl)-3-methylpiperazin-1-yl)prop-2-en-1-one | 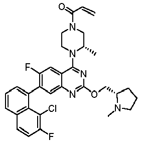 | 591.22 |
| 420 | (S)-1-(4-(6-fluoro-2-((tetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 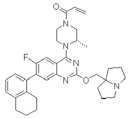 | 570.32 |
| 421 | (S)-1-(4-(6-fluoro-7-(8-chloronaphthalen-1-yl)-2-((tetrahydro-1H-pyrrolizin-7a(5H)-yl)methoxy)quinazolin-4-yl)-3-methylpiperazin-1-yl)prop-2-en-1-one | 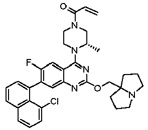 | 580.25 |
| 422 | (S)-1-(4-(6-fluoro-7-(8-chloro-7-fluoronaphthalen-1-yl)-2-((tetrahydro-1H-pyrrolizin-7a(5H)-yl)methoxy)quinazolin-4-yl)-3-methylpiperazin-1-yl)prop-2-en-1-one | 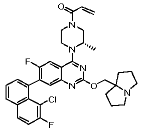 | 618.24 |
| 423 | 1-((3S)-4-(6-fluoro-2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 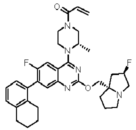 | 588.31 |
| 424 | 1-((3S)-4-(6-fluoro-7-(8-chloronaphthalen-1-yl)-2-((((2R,7aS)-2-fluorotetrahydro-1H-pyrrolizin-7a(5H)-yl]methoxy)quinazolin-4-yl)-3-methylpiperazin-1-yl)prop-2-en-1-one | 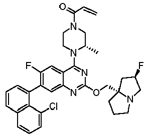 | 618.24 |
| 425 | 1-((3S)-4-(6-fluoro-7-(8-chloro-7-fluoronaphthalen-1-yl)-2-((((2R,7aS)-2-fluorotetrahydro-1H-pyrrolizine-7a(5H))-yl)methoxy)quinazolin-4-yl)-3-methylpiperazin-1-yl)prop-2-en-1-one | 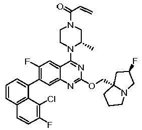 | 636.23 |
| 426 | 1-((S)-4-(6-fluoro-2-((((2S,4R)-4-fluoro-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 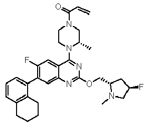 | 56229 |
| 427 | 1-((S)-4-(6-fluoro-7-(8-chloronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 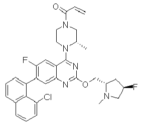 | 592.22 |
| 428 | 1-((S)-4-(6-fluoro-7-(8-chloro-7-fluoronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 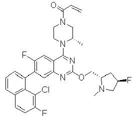 | 610.21 |
| 429 | 2-((2S)-1-acryloyl-4-(6,8-difluoro-2-((((S)-1-methylpyrrolidin-2-yl)methoxy)-7-(5,6,7,8-tetrahydronaphthalen-1 -yl)quinazolin-4-ylpiperazin-2-yl)acetonitrile | 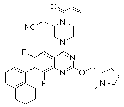 | 587.29 |
| 430 | 2-((S)-1-acryloyl-4-(6,8-difluoro-7-(8-chloronaphthalen-1-yl)-2-(((S)-1-methylpyrrolidin-2-yl)methoxy)quinazolin-4-yl)piperazin-2-yl)acetonitrile | 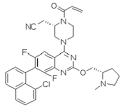 | 617.22 |
| 431 | 2-((S)-1-acryloyl-4-(6,8-difluoro-7-(8-chloro-7-fluoronaphthalen-1-yl)-2-(((S)-1-methylpyrrolidin-2-yl))methoxy)quinazolin-4-yl)piperazin-2-yl)acetonitrile | 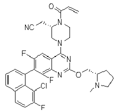 | 635.21 |
| 432 | 2-((S)-1-acryloyl-4-(6,8-difluoro-2-((((2S,4R)-4-fluoro-1-methylpyrrolidin-2-yl)methoxy)-7-(5,6,7,8-tetrahydronaphthalen-1 -ylquinazolin-4-ylpiperazin-2-ylacetonitrile | 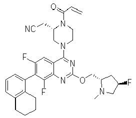 | 605.28 |
| 433 | 2-((S)-1-acryloyl-4-(6,8-difluoro-7-(8-chloronaphthalen-1-yl)-2-((((2S,4R)-4-fluoro-1-methylpyrrolidin-2-(yl)methoxy)quinazolin-4-yl)piperazin-2-yl)acetonitrile | 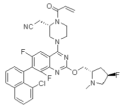 | 635.21 |
| 434 | 2-((S)-1-acryloyl-4-(6,8-difluoro-7-(8-chloro-7-fluoronaphthalen-1-yl)-2-((((2S,4R)-4-fluoro-1-methylpyrrolidin-2-yl)methoxy)quinazolin-4-ylpiperazin-2-yl)acetonitrile | 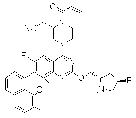 | 653.2 |
| 435 | (S)-2-(1-acryloyl-4-(6,8-difluoro-2-((tetrahydro-1H-pyrrolizin-7a(5H)-yl)methoxy)-7-(5,6,7,8-tetrahydronaphthalen-1 -yl)quinazolin-4-ylpiperazin-2-yl)acetonitrile | 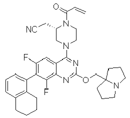 | 612.3 |
| 436 | 1-((1R,5S)-6-(6,8-difluoro-7-(8-chloronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 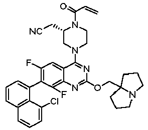 | 643.23 |
| 437 | 1-((1R,5S)-6-(6,8-difluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 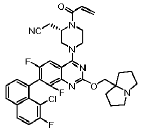 | 661.22 |
| 438 | 1-(((1R,5S)-6-(6,8-difluoro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 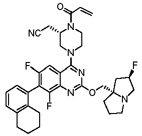 | 631.29 |
| 439 | 1-(((1R,SS)-6-(6,8-difluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 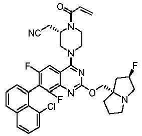 | 661.22 |
| 440 | 1-(((1R,5S)-6-(6,8-difluoro-7-(8-chloro-7-fluoronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 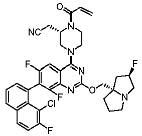 | 679.21 |
| 441 | 1-((1R,SS)-6-(6,8-difluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 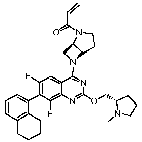 | 560.28 |
| 442 | 1-(((1R,SS)-6-(6,8-difluoro-7-(8-chloronaphthalen-1-yl)-2-((((2R)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 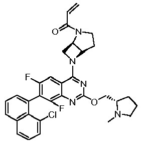 | 590.21 |
| 443 | 1-(((1R,5S)-6-(6,8-difluoro-7-(8-chloro-7-fluoronaphthalen-1-yl)-2-((((2R)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 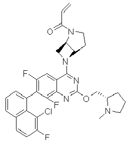 | 608.2 |
| 444 | 1-((1R,SS)-6-(6,8-difluoro-2-((tetrahydro-1H-pyrrolizin-7a(5H)-yl)methoxy)-7-(5,6,7,8-tetrahydronaphthalen-1-yl)quinazolin-4-yl)-2,6-diazabicyclo[3.2.0]hept-2-yl)prop-2-en-1-one | 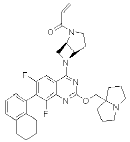 | 586.29 |
| 445 | 1-((1R,SS)-6-(6,8-difluoro-2-((((2S,4R)-4-fluoro-1-methylpyrrolidin-2-yl)methoxy)-7-(5,6,7,8-tetrahydronaphthalen-1 -yl)quinazolin-4-yl)-2,6-diazabicyclo[3.2.0]hept-2-yl)prop-2-en-1-one | 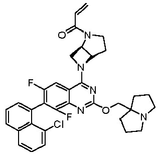 | 616.22 |
| 446 | 1-((1R,5S)-6-(6,8-difluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 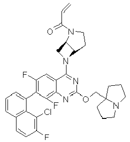 | 634.21 |
| 447 | 1-(((1R,5S)-6-(6,8-difluoro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 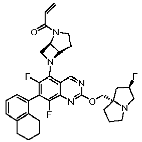 | 604.28 |
| 448 | 1-(((1R,SS)-6-(6,8-difluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 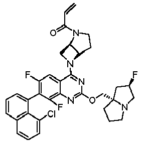 | 634.21 |
| 449 | 1-(((1R,5S)-6-(6,8-difluoro-7-(8-chloro-7-fluoronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 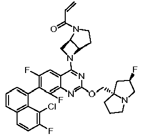 | 652.2 |
| 450 | 1-((1R,SS)-6-(6,8-difluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 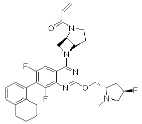 | 578.27 |
| 451 | 1-((1R,5S)-6-(6,8-difluoro-7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 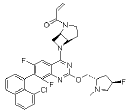 | 608.2 |
| 452 | 1-(((1R,5S)-6-(6,8-difluoro-7-(8-chloro-7-fluoronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 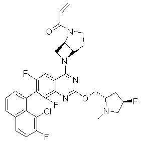 | 626.19 |
| 453 | 1-((S)-4-(6,8-difluoro-2-(((S)-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 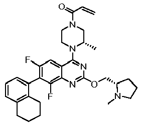 | 562.29 |
| 454 | 1-((S)-4-(6,8-difluoro-7-(8-chloronaphthalen-1-yl)-2-(((S)-1-methylpyrrolidin-2-yl)methoxy)quinazolin-4-yl)-3-methylpiperazin-1 -yl)prop-2-en-1 -one | 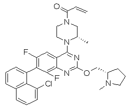 | 592.22 |
| 455 | 1-((S)-4-(6,8-difluoro-7-(8-chloro-7-fluoronaphthalen-1-yl)-2-(((S)-1-methylpyrrolidin-2-yl)methoxy)quinazolin-4-yl)-3-methylpiperazin-1-yl)prop-2-en-1-one | 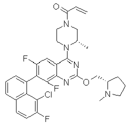 | 610.21 |
| 456 | (S)-1-(4-(6,8-difluoro-2-((tetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 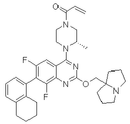 | 588.31 |
| 457 | (S)-1-(4-(6,8-difluoro-7-(8-chloronaphthalen-1-yl)-2-((tetrahydro-1H-pyrrolizin-7a(5H)-yl)methoxy)quinazolin-4-yl)-3-methylpiperazin-1-yl)prop-2-en-1-one | 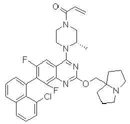 | 618.24 |
| 458 | (S)-1-(4-(6,8-difluoro-7-(8-chloro-7-fluoronaphthalen-1-yl)-2-((tetrahydro-1H-pyrrolizin-7a(5H)-yl)methoxy)quinazolin-4-yl)-3-methylpiperazin-1-yl)prop-2-en-1-one | 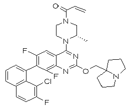 | 652.20 |
| 459 | 1-((3S)-4-(6,8-difluoro-2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 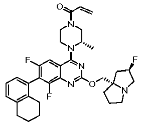 | 606.3 |
| 460 | 1-((3S)-4-(6,8-difluoro-7-(8-chloronaphthalen-1-yl)-2-((((2R,7aS)-2-fluorotetrahydro-1H-pyrrolizin-7a(5H)-yl]methoxy)quinazolin-4-yl)-3-methylpiperazin-1-yl)prop-2-en-1-one | 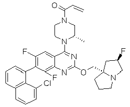 | 636.23 |
| 461 | 1-((3S)-4-(6,8-difluoro-7-(8-chloro-7-fluoronaphthalen-1-yl)-2-((((2R,7aS)-2-fluorotetrahydro-1H-pyrrolizin-7a(5H))-yl)methoxy)quinazolin-4-yl)-3-methylpiperazin-1-yl)prop-2-en-1-one | 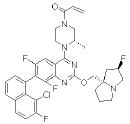 | 655.22 |
| 462 | 1-((S)-4-(6,8-difluoro-2-((((2S,4R)-4-fluoro-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 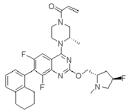 | 580.28 |
| 463 | 1-((S)-4-(6,8-difluoro-7-(8-chloronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 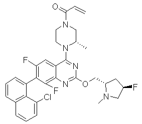 | 610.21 |
| 464 | 1-((S)-4-(6,8-difluoro-7-(8-chloro-7-fluoronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 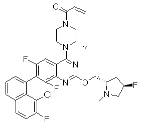 | 628.2 |
| 465 | 2-((S)-1-acryloyl-4-(6-chloro-7-(8-chloronaphthalen-1-yl)-2-(((S)-1-methylpyrrolidin-2-yl)methoxy)quinazolin-4-yl)piperazin-2-yl)acetonitrile | 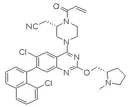 | 615.20 |
| 466 | 2-((S)-1-acryloyl-4-(6-chloro-7-(8-chloro-7 - fluoronaphthalen-1-yl)-2-(((S)-1-methylpyrrolidin-2-yl))methoxy)quinazolin-4-yl)piperazin-2-yl)acetonitrile | 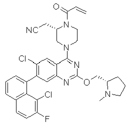 | 633.19 |
| 467 | 2-((S)-1-acryloyl-4-(6-chloro-2-((((2S,4R)-4-fluoro-1-methylpyrrolidin-2-yl)methoxy)-7-(5,6,7,8-tetrahydronaphthalen-1 -ylquinazolin-4-ylpiperazin-2-ylacetonitrile | 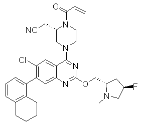 | 603.26 |
| 468 | 2-((S)-1-acryloyl-4-(6-chloro-7-(8-chloronaphthalen-1-yl)-2-((((2S,4R)-4-fluoro-1-methylpyrrolidin-2-(yl)methoxy)quinazolin-4-yl)piperazin-2-yl)acetonitrile | 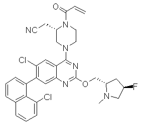 | 633.19 |
| 469 | 2-((S)-1-acryloyl-4-(6-chloro-7-(8-chloro-7-fluoronaphthalen-1-yl)-2-((((2S,4R)-4-fluoro-1-methylpyrrolidin-2-yl)methoxy)quinazolin-4-ylpiperazin-2-yl)acetonitrile | 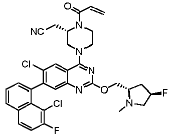 | 651.18 |
| 470 | (S)-2-(1-acryloyl-4-(6-chloro-2-((tetrahydro-1H-pyrrolizin-7a(5H)-yl)methoxy)-7-(5,6,7,8-tetrahydronaphthalen-1-yl)quinazolin-4-ylpiperazin-2-yl)acetonitrile | 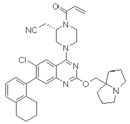 | 611.28 |
| 471 | 1-((1R,5S)-6-(6-chloro-7-(8-chloronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 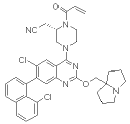 | 614.20 |
| 472 | 1-((1R,5S)-6-(6-chloro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 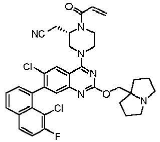 | 632.19 |
| 473 | 1-(((1R,5S)-6-(6-chloro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 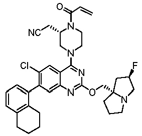 | 602.26 |
| 474 | 1-(((1R,5S)-6-(6-chloro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 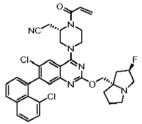 | 632.19 |
| 475 | 1-(((1R,5S)-6-(6-chloro-7-(8-chloro-7-fluoronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 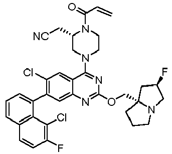 | 650.18 |
| 476 | 1-((1R,5S)-6-(6-chloro-2-((((2S,4R)-4-fluoro-1-methylpyrrolidin-2-yl)methoxy)-7-(5,6,7,8-tetrahydronaphthalen-1 -yl)quinazolin-4-yl)-2,6-diazabicyclo[3.2.0]hept-2-yl)prop-2-en-1-one | 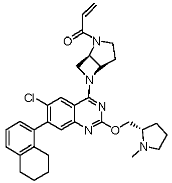 | 576.25 |
| 477 | 1-(((1R,5S)-6-(6-chloro-7-(8-chloronaphthalen-1-yl)-2-((((2R)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 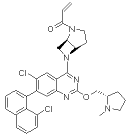 | 632.19 |
| 478 | 1-(((1R,5S)-6-(6-chloro-7-(8-chloro-7-fluoronaphthalen-1-yl)-2-((((2R)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 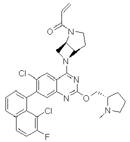 | 650.18 |
| 479 | 1-((1R,SS)-6-(6-chloro-2-((tetrahydro-1H-pyrrolizin-7a(5H)-yl)methoxy)-7-(5,6,7,8-tetrahydronaphthalen-1-yl)quinazolin-4-yl)-2,6-diazabicyclo[3.2.0]hept-2-yl)prop-2-en-1 -one | 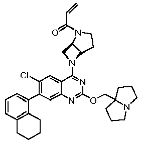 | 575.25 |
| 480 | 1-((1R,5S)-6-(6-chloro-2-((((2S,4R)-4-fluoro-1-methylpyrrolidin-2-yl)methoxy)-7-(5,6,7,8-tetrahydronaphthalen-1 -yl)quinazolin-4-yl)-2,6-diazabicyclo[3.2.0]hept-2-yl)prop-2-en-1-one | 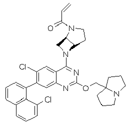 | 616.22 |
| 481 | 1-((1R,5S)-6-(6-chloro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 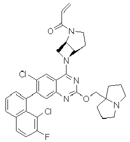 | 632.20 |
| 482 | 1-(((1R,5S)-6-(6-chloro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 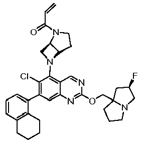 | 602.26 |
| 483 | 1-(((1R,5S)-6-(6-chloro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 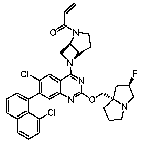 | 632.19 |
| 484 | 1-(((1R,5S)-6-(6-chloro-7-(8-chloro-7-fluoronaphthalen-1-yl)-2-((((2R,7aS)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 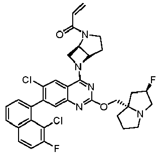 | 650.18 |
| 485 | 1-((1R,5S)-6-(6-chloro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 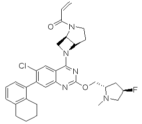 | 576.25 |
| 486 | 1-((1R,5S)-6-(6-chloro-7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 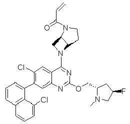 | 606.18 |
| 487 | 1-(((1R,5S)-6-(6-chloro-7-(8-chloro-7-fluoronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 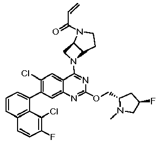 | 624.17 |
| 488 | 1-((S)-4-(6-chloro-2-(((S)-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 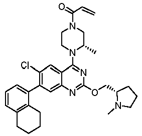 | 560.27 |
| 489 | 1-((S)-4-(6-chloro-7-(8-chloronaphthalen-1-yl)-2-(((S)-1-methylpyrrolidin-2-yl)methoxy)quinazolin-4-yl)-3-methylpiperazin-1-yl)prop-2-en-1 -one | 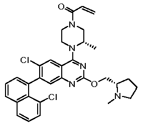 | 590.20 |
| 490 | 1-((S)-4-(6-chloro-7-(8-chloro-7-fluoronaphthalen-1-yl)-2-(((S)-1-methylpyrrolidin-2-yl)methoxy)quinazolin-4-yl)-3-methylpiperazin-1-yl)prop-2-en-1-one | 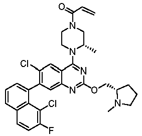 | 608.19 |
| 491 | (S)-1-(4-(6-chloro-2-((tetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 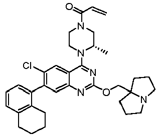 | 586.29 |
| 492 | (S)-1-(4-(6-chloro-7-(8-chloronaphthalen-1-yl)-2-((tetrahydro-1H-pyrrolizin-7a(5H)-yl)methoxy)quinazolin-4-yl)-3-methylpiperazin-1-yl)prop-2-en-1-one | 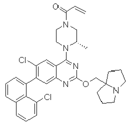 | 616.22 |
| 493 | (S)-1-(4-(6-chloro-7-(8-chloro-7-fluoronaphthalen-1-yl)-2-((tetrahydro-1H-pyrrolizin-7a(5H)-yl)methoxy)quinazolin-4-yl)-3-methylpiperazin-1-yl)prop-2-en-1-one | 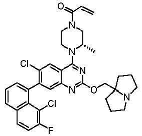 | 634.21 |
| 494 | 1-((3S)-4-(6-chloro-2-((((2R,7aS)-2-fluorotetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1 -one | 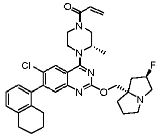 | 604.28 |
| 495 | 1-((3S)-4-(6-chloro-7-(8-chloronaphthalen-1-yl)-2-((((2R,7aS)-2-fluorotetrahydro-1H-pyrrolizin-7a(5H)-yl]methoxy)quinazolin-4-yl)-3-methylpiperazin-1-yl)prop-2-en-1 -one | 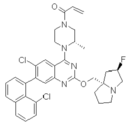 | 634.21 |
| 496 | 1-((3S)-4-(6-chloro-7-(8-chloro-7-fluoronaphthalen-1-yl)-2-((((2R,7aS)-2-fluorotetrahydro-1H-pyrrolizine-7a(5H))-yl)methoxy)quinazolin-4-yl)-3-methylpiperazin-1-yl)prop-2-en-1 -one | 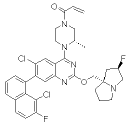 | 652.20 |
| 497 | 1-((S)-4-(6-chloro-2-((((2S,4R)-4-fluoro-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 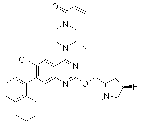 | 578.26 |
| 498 | 1-((S)-4-(6-chloro-7-(8-chloronaphthalen-1-yl)-2-((((2S,4R)-4-fluoro-1-methylpyrrolidin-2-yl)methoxy)quinazolin-4-yl)-3-methylpiperazin-1-yl)prop-2-en-1 -one | 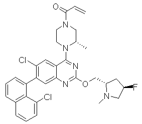 | 608.19 |
| 499 | 1-((S)-4-(6-chloro-7-(8-chloro-7-fluoronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 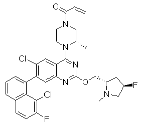 | 626.18 |
| 500 | 2-((2S)-1-acryloyl-4-(6-chloro-8-fluoro-2-((((S)-1-methylpyrrolidin-2-yl)methoxy)-7-(5,6,7,8-tetrahydronaphthalen-1 -ylquinazolin-4-ylpiperazin-2-ylacetonitrile | 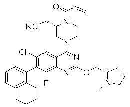 | 603.26 |
| 501 | 2-((S)-1-acryloyl-4-(6-chloro-8-fluoro-7-(8-chloronaphthalen-1-yl)-2-(((S)-1-methylpyrrolidin-2-yl)methoxy)quinazolin-4-yl)piperazin-2-yl)acetonitrile | 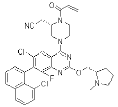 | 633.19 |
| 502 | 2-((S)-1-acryloyl-4-(6-chloro-8-fluoro-7-(8-chloro-7-fluoronaphthalen-1-yl)-2-(((S)-1-methylpyrrolidin-2-yl))methoxy)quinazolin-4-yl)piperazin-2-yl)acetonitrile | 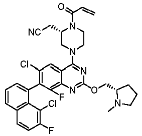 | 651.18 |
| 503 | 2-((S)-1-acryloyl-4-(6-chloro-8-fluoro-2-((((2S,4R)-4-fluoro-1-methylpyrrolidin-2-yl)methoxy)-7-(5,6,7,8-tetrahydronaphthalen-1 -ylquinazolin-4-ylpiperazin-2-ylacetonitrile | 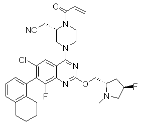 | 621.25 |
| 504 | 2-((S)-1-acryloyl-4-(6-chloro-8-fluoro-7-(8-chloronaphthalen-1-yl)-2-((((2S,4R)-4-fluoro-1-methylpyrrolidin-2-(yl)methoxy)quinazolin-4-yl)piperazin-2-yl)acetonitrile | 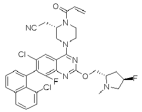 | 651.18 |
| 505 | 2-((S)-1-acryloyl-4-(6-chloro-8-fluoro-7-(8-chloro-7-fluoronaphthalen-1-yl)-2-((((2S,4R)-4-fluoro-1-methylpyrrolidin-2-yl)methoxy)quinazolin-4-ylpiperazin-2-yl)acetonitrile | 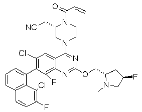 | 669.17 |
| 506 | (S)-2-(1-acryloyl-4-(6-chloro-8-fluoro-2-((tetrahydro-1H-pyrrolizin-7a(5H)-yl)methoxy)-7-(5,6,7,8-tetrahydronaphthalen-1 -yl)quinazolin-4-ylpiperazin-2-yl)acetonitrile | 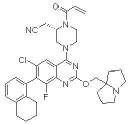 | 628.27 |
| 507 | 1-((1R,5S)-6-(6-chloro-8-fluoro-7-(8-chloronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 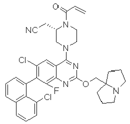 | 659.20 |
| 508 | 1-((1R,5S)-6-(6-chloro-8-fluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 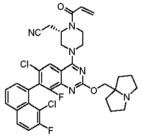 | 677.19 |
| 509 | 1-(((1R,5S)-6-(6-chloro-8-fluoro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 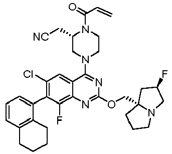 | 647.26 |
| 510 | 1-(((1R,5S)-6-(6-chloro-8-fluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 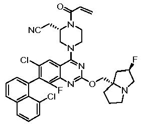 | 677.19 |
| 511 | 1-(((1R,5S)-6-(6-chloro-8-fluoro-7-(8-chloro-7-fluoronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 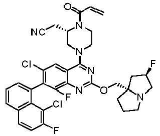 | 695.18 |
| 512 | 1-((1R,SS)-6-(6-chloro-8-fluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 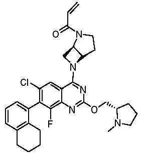 | 576.25 |
| 513 | 1-(((1R,5S)-6-(6-chloro-8-fluoro-7-(8-chloronaphthalen-1-yl)-2-((((2R)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 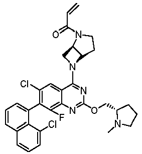 | 606.18 |
| 514 | 1-(((1R,5S)-6-(6-chloro-8-fluoro-7-(8-chloro-7-fluoronaphthalen-1-yl)-2-((((2R)-2-fluorotetrahydro-1H-pyrrolizine-7a(5H)-yl)methoxy)quinazolin-4-yl)-2,6-diazabicyclo[3.2.0]hept-2-yl)prop-2-en-1-one | 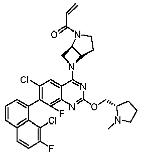 | 624.17 |
| 515 | 1-((1R,SS)-6-(6-chloro-8-fluoro-2-((tetrahydro-1H-pyrrolizin-7a(5H)-yl)methoxy)-7-(5,6,7,8-tetrahydronaphthalen-1-yl)quinazolin-4-yl)-2,6-diazabicyclo[3.2.0]hept-2-yl)prop-2-en-1-one | 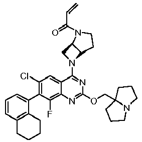 | 602.26 |
| 516 | 1-((1R,SS)-6-(6-chloro-8-fluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 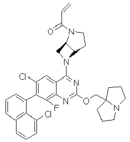 | 632.19 |
| 517 | 1-((1R,5S)-6-(6-chloro-8-fluoro-7-(8-chloro-7-fluoronaphthalen-1-yl)-2-((tetrahydro-1H-pyrrolizin-7a(5H)-yl)methoxy)quinazolin-4-yl)-2,6-diazabicyclo[3.2.0]hept-2-yl)prop-2-en-1-one | 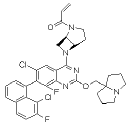 | 650.18 |
| 518 | 1-(((1R,5S)-6-(6-chloro-8-fluoro-2-((((2R,7aS)-2-fluorotetrahydro-1H-pyrrolizin-7a(5H)-yl)methoxy)-7-(5,6,7,8-tetrahydronaphthalen-1-yl)quinazolin-5-yl)-2,6-diazabicyclo[3.2.0]hept-2-yl)prop-2-en-1-one | 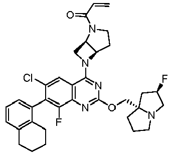 | 620.25 |
| 519 | 1-(((1R,5S)-6-(6-chloro-8-fluoro-7-(8-chloronaphthalen-1-yl)-2-((((2R,7aS)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 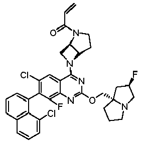 | 650.18 |
| 520 | 1-(((1R,5S)-6-(6-chloro-8-fluoro-7-(8-chloro-7-fluoronaphthalen-1-yl)-2-((((2R)-2-fluorotetrahydro-1H-pyrrolizin-7a(5H)-yl)methoxy)quinazolin-4-yl)-2,6-diazabicyclo[3.2.0]hept-2-yl)prop-2-en-1-one | 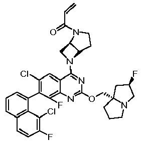 | 668.17 |
| 521 | 1-((1R,SS)-6-(6-chloro-8-fluoro-2-((((2S,4R)-4-fluoro-1-methylpyrrolidin-2-yl)methoxy)-7-(5,6,7,8-tetrahydronaphthalen-1-yl)quinazolin-4-yl)-2,6-diazabicyclo[3.2.0]hept-2-yl)prop-2-en-1-one | 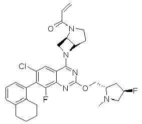 | 594.24 |
| 522 | 1-((1R,5S)-6-(6-chloro-8-fluoro-7-(8-chloronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 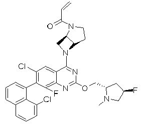 | 624.17 |
| 523 | 1-(((1R,5S)-6-(6-chloro-8-fluoro-7-(8-chloro-7-fluoronaphthalen-1-yl)-2-((((2S,4R)-4-fluoro-1-methylpyrrolidin-2-yl)methoxy)quinazolin-4-yl)-2,6-diazabicyclo[3.2.0]hept-2-yl)prop-2-en-1-one | 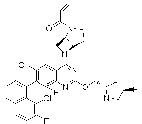 | 642.16 |
| 524 | 1-((S)-4-(6-chloro-8-fluoro-2-(((S)-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 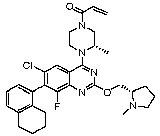 | 578.26 |
| 525 | 1-((S)-4-(6-chloro-8-fluoro-7-(8-chloronaphthalen-1-yl)-2-(((S)-1-methylpyrrolidin-2-yl)methoxy)quinazolin-4-yl)-3-methylpiperazin-1-yl)prop-2-en-1-one | 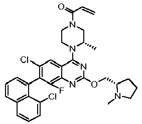 | 608.19 |
| 526 | 1-((S)-4-(6-chloro-8-fluoro-7-(8-chloro-7-fluoronaphthalen-1-yl)-2-(((S)-1-methylpyrrolidin-2-yl)methoxy)quinazolin-4-yl)-3-methylpiperazin-1-yl)prop-2-en-1-one | 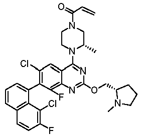 | 626.18 |
| 527 | (S)-1-(4-(6-chloro-8-fluoro-2-((tetrahydro-1H-pyrrolizin-7a(5H)-yl)methoxy)-7-(5,6,7,8-tetrahydronaphthalen-1-yl)quinazolin-4-yl)-3-methylpiperazin-1 -yl)prop-2-en-1 -one | 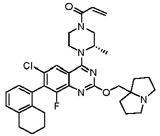 | 604.28 |
| 528 | (S)-1-(4-(6-chloro-8-fluoro-7-(8-chloronaphthalen-1-yl)-2-((tetrahydro-1H-pyrrolizin-7a(5H)-yl)methoxy)quinazolin-4-yl)-3-methylpiperazin-1-yl)prop-2-en-1-one | 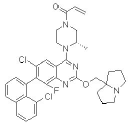 | 634.21 |
| 529 | (S)-1-(4-(6-chloro-8-fluoro-7-(8-chloro-7-fluoronaphthalen-1-yl)-2-((tetrahydro-1H-pyrrolizin-7a(5H)-yl)methoxy)quinazolin-4-yl)-3-methylpiperazin-1-yl)prop-2-en-1-one | 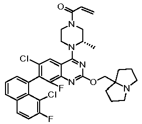 | 652.20 |
| 530 | 1-((3S)-4-(6-chloro-8-fluoro-2-((((2R)-2-fluorotetrahydro-1H-pyrrolizin-7a(5H)-yl)methoxy)-7-(5,6,7,8-tetrahydronaphthalen-1-yl)quinazolin-4-yl)-3-methylpiperazin-1-yl)prop-2-en-1-one | 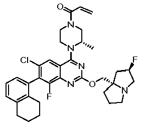 | 622.27 |
| 531 | 1-((3S)-4-(6-chloro-8-fluoro-7-(8-chloronaphthalen-1-yl)-2-((((2R,7aS)-2-fluorotetrahydro-1H-pyrrolizin-7a(5H)-yl)methoxy)quinazolin-4-yl)-3-methylpiperazin-1-yl)prop-2-en-1-one | 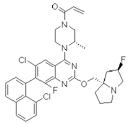 | 652.20 |
| 532 | 1-((3S)-4-(6-chloro-8-fluoro-7-(8-chloro-7-fluoronaphthalen-1-yl)-2-((((2R,7aS)-2-fluorotetrahydro-1H-pyrrolizin-7a(5H))-yl)methoxy)quinazolin-4-yl)-3-methylpiperazin-1-yl)prop-2-en-1-one | 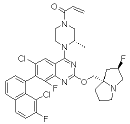 | 670.19 |
| 533 | 1-((S)-4-(6-chloro-8-fluoro-2-((((2S,4R)-4-fluoro-1-methylpyrrolidin-2-yl)methoxy)-7-(5,6,7,8-tetrahydronaphthalen-1-yl)quinazolin-4-yl)-3-methylpiperazin-1-yl)prop-2-en-1-one | 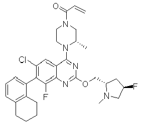 | 596.25 |
| 534 | 1-((S)-4-(6-chloro-8-fluoro-7-(8-chloronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 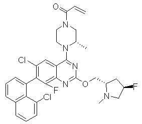 | 626.18 |
| 535 | 1-((S)-4-(6-chloro-8-fluoro-7-(8-chloro-7-fluoronaphthalen-1-yl)-2-((((2S,4R)-4-fluoro-1-methylpyrrolidin-2-yl)methoxy)quinazolin-4-yl)-3-methylpiperazin-1-yl)prop-2-en-1-one | 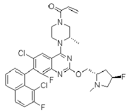 | 643.17 |
| 536 | 2-((2S)-1-acryloyl-4-(2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 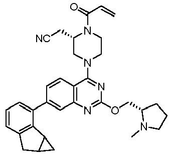 | 549.3 |
| 537 | 2-((2S)-1-(2-fluoroacryloyl)-4-(2-(((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 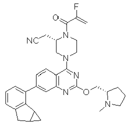 | 567.3 |
| 538 | 2-((S)-1-acryloyl-4-(2-((((S)-1-methylpyrrolidin-2-yl)methoxy)-7-(thiochroman-8-yl)quinazolin-4-yl)piperazin-2-yl)acetonitrile | 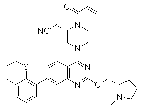 | 569.3 |
| 539 | 2-((S)-1-acryloyl-4-(7-(benzothien-7-yl)-2-(((S)-1-methylpyrrolidin-2-yl)methoxy)quinazolin-4-yl)piperazin-2-yl)acetonitrile | 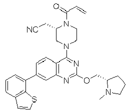 | 553.2 |
| 540 | 2-((S)-4-(7-(benzo[b]thien-7-yl)-2-((S)-1-methylpyrrolidin-2-yl)methoxy)quinazolin-4-yl)-1-(2-fluoroacryloyl)piperazin-2-yl)acetonitrile | 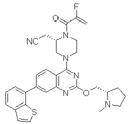 | 571.2 |
| 541 | 2-((S)-1-acryloyl-4-(7-(benzothien-4-yl)-2-(((S)-1-methylpyrrolidin-2-yl)methoxy)quinazolin-4-yl)piperazin-2-yl)acetonitrile | 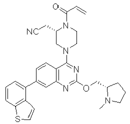 | 553.2 |
| 542 | 2-((S)-4-(7-(benzo[b]thien-4-yl)-2-((S)-1-methylpyrrolidin-2-yl)methoxy)quinazolin-4-yl)-1-(2-fluoroacryloyl)piperazin-2-yl)acetonitrile | 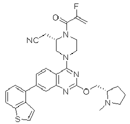 | 571.2 |
| 543 | 2-((2S)-1-acryloyl-4-(2-(((2R)-2-fluorotetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 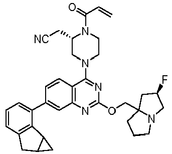 | 593.3 |
| 544 | 2-((2S)-1-(2-fluoroacryloyl)-4-(2-(((2R)-2-fluorotetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 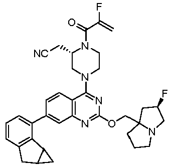 | 611.3 |
| 545 | 2-((2S)-1-acryloyl-4-(2-((tetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 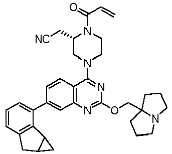 | 575.3 |
| 546 | 2-((2S)-1-(2-fluoroacryloyl)-4-(2-((tetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 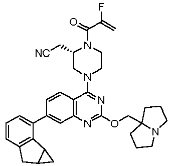 | 593.3 |
| 547 | 2-((S)-1-(2-fluoroacryloyl)-4-(2-((tetrahydro-1H-pyrrolizin-7a(5H)-yl)methoxy)-7-((1aS,6aS)-1,1a,6,6a-tetrahydrocyclopropa[a]inden-2-yl)quinazolin-4-yl)piperazin-2-yl)acetonitrile | 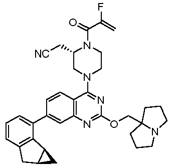 | 593.3 |
| 548 | 2-((S)-1-(2-fluoroacryloyl)-4-(2-((tetrahydro-1H-pyrrolizin-7a(5H)-yl)methoxy)-7-((1aR,6aR)-1,1a,6,6a-tetrahydrocyclopropa[a]inden-2-yl)quinazolin-4-yl)piperazin-2-yl)acetonitrile | 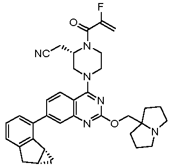 | 593.3 |
| 549 | 2-((2S)-1-(2-fluoroacryloyl)-4-(2-(((2R)-2-fluorotetrahydro-1H-pyrrolizin-7a(5H)-yl)methoxy)-7-((1aS,6aS)-1,1a,6,6a-tetrahydrocyclopropa[a]inden-2-yl)quinazolin-4-yl)piperazin-2-yl)acetonitrile | 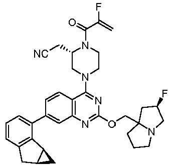 | 611.3 |
| 550 | 2-((2S)-1-(2-fluoroacryloyl)-4-(2-(((2R)-2-fluorotetrahydro-1H-pyrrolizin-7a(5H)-yl)methoxy)-7-((1aR,6aR)-1,1a,6,6a-tetrahydrocyclopropa[a]inden-2-yl)quinazolin-4-yl)piperazin-2-yl)acetonitrile | 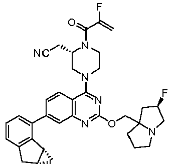 | 611.3 |
| 551 | 2-((2S)-4-(7-(benzo[b]thien-7-yl)-2-((2R)-2-fluorotetrahydro-1H-pyrrolizin-7a(5H)-yl)methoxy)quinazolin-4-yl)-1-(2-fluoroacryloyl)piperazin-2-yl)acetonitrile | 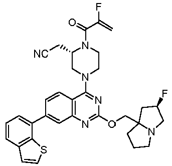 | 615.2 |
| 552 | (S)-2-(4-(7-(benzothien-7-yl)-2-((tetrahydro-1H-pyrrolizin-7a(5H)-yl)methoxy)quinazolin-4-yl)-1-(2-fluoroacryloyl)piperazin-2-yl)acetonitrile | 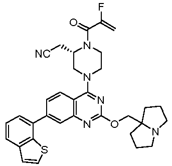 | 597.2 |
| 553 | 2-((2S)-4-(7-(benzo[b]thien-4-yl)-2-((2R)-2-fluorotetrahydro-1H-pyrrolizin-7a(5H)-yl)methoxy)quinazolin-4-yl)-1-(2-fluoroacryloyl)piperazin-2-yl)acetonitrile | 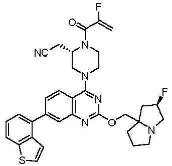 | 615.2 |
| 554 | (S)-2-(4-(7-(benzothien-4-yl)-2-((tetrahydro-1H-pyrrolizin-7a(5H)-yl)methoxy)quinazolin-4-yl)-1-(2-fluoroacryloyl)piperazin-2-yl)acetonitrile | 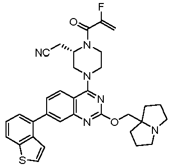 | 597.2 |
| 555 | 2-((2S)-1-acryloyl-4-(6-chloro-2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 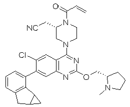 | 583.3 |
| 556 | 2-((S)-1-acryloyl-4-(6-chloro-2-((((S)-1-methylpyrrolidin-2-yl)methoxy)-7-(thiocyano-8)quinazolin-4-yl)piperazin-2-yl)acetonitrile | 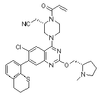 | 603.2 |
| 557 | 2-((S)-1-acryloyl-4-(7-(benzothien-7-yl)-6-chloro-2-(((S)-1-methylpyrrolidin-2-yl)methoxy)quinazolin-4-yl)piperazin-2-yl)acetonitrile | 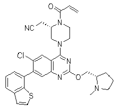 | 587.2 |
| 558 | 2-((S)-4-(7-(benzothien-4-yl)-6-chloro-2-(((S)- 1 - methylpyrrolidin-2-yl)methoxy)quinazolin-4-yl)-1-(2-fluoroacryloyl)piperazin-2-yl)acetonitrile | 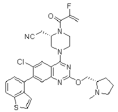 | 605.2 |
| 559 | 2-((2S)-1-acryloyl-4-(6-chloro-8-fluoro-2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 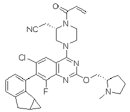 | 601.2 |
| 560 | 2-((2S)-1-acryloyl-4-(6-chloro-8-fluoro-2-((S)-1-methylpyrrolidin-2-yl)methoxy)-7-(thiochroman-8-yl)quinazolin-4-yl)piperazin-2-yl)acetonitrile | 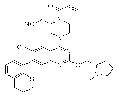 | 621.2 |
| 561 | 2-((2S)-1-acryloyl-4-(7-(benzothien-7-yl)-6-chloro-8-fluoro-2-(((S)-1-methylpyrrolidin-2-yl)methoxy)quinazolin-4-yl)piperazin-2-yl)acetonitrile | 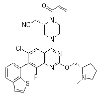 | 605.2 |
| 562 | 2-((2S)-4-(7-(benzothien-4-yl)-6-chloro-8-fluoro-2-(((S)-1 -methylpyrrolidin-2-yl)methoxy)quinazolin-4-yl)-1-(2-fluoroacryloyl)piperazin-2-yl)acetonitrile | 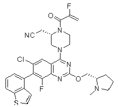 | 623.2 |
| 563 | 2-((2S)-1-acryloyl-4-(6,8-difluoro-2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 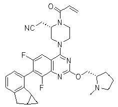 | 585.3 |
| 564 | 2-((2S)-1-acryloyl-4-(6,8-difluoro-2-((S)-1-methylpyrrolidin-2-yl)methoxy)-7-(thiocyano-8)quinazolin-4-yl)piperazin-2-yl)acetonitrile | 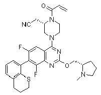 | 605.2 |
| 565 | 2-((2S)-1-acryloyl-4-(7-(benzothien-7-yl)-6,8-difluoro-2-(((S)-1-methylpyrrolidin-2-yl)methoxy)quinazolin-4-yl)piperazin-2-yl)acetonitrile | 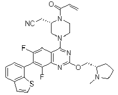 | 589.2 |
| 566 | 2-((2S)-4-(7-(benzothien-4-yl)-6,8-difluoro-2-(((S)-1-methylpyrrolidin-2-yl)methoxy)quinazolin-4-yl)-1-(2-fluoroacryloyl)piperazin-2-yl)acetonitrile | 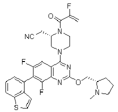 | 607.2 |
| 567 | 2-((2S)-1-acryloyl-4-(6-fluoro-2-(((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 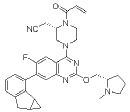 | 567.3 |
| 568 | 2-((2S)-4-(6-fluoro-2-(((S)-1-methylpyrrolidin-2-yl)methoxy)-7-(1,1a,6,6a-tetrahydrocyclopropa[a]inden-2-yl)quinazolin-4-yl)-1-(2-fluoroacryloyl)piperazin-2-yl)acetonitrile | 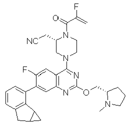 | 585.3 |
| 569 | 2-((S)-1-acryloyl-4-(6-fluoro-2-((((S)-1- methylpyrrolidin-2-yl)methoxy)-7-(thiochroman-8-yl)quinazolin-4-yl)piperazin-2-yl)acetonitrile | 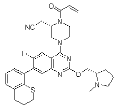 | 587.3 |
| 570 | 2-((S)-1-acryloyl-4-(7-(benzothien-7-yl)-6-fluoro-2-(((S)-1-methylpyrrolidin-2-yl)methoxy)quinazolin-4-yl)piperazin-2-yl)acetonitrile | 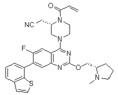 | 571.2 |
| 571 | 2-((S)-4-(7-(benzothien-7-yl)-6-fluoro-2-(((S)-1-methylpyrrolidin-2-yl)methoxy)quinazolin-4-yl)-1-(2-fluoroacryloyl)piperazin-2-yl)acetonitrile | 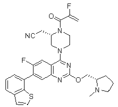 | 589.2 |
| 572 | 2-((S)-1-acryloyl-4-(7-(benzothien-4-yl)-6-fluoro-2-(((S)-1-methylpyrrolidin-2-yl)methoxy)quinazolin-4-yl)piperazin-2-yl)acetonitrile | 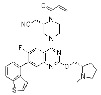 | 571.2 |
| 573 | 2-((S)-4-(7-(benzothien-4-yl)-6-fluoro-2-(((S)-1-methylpyrrolidin-2-yl)methoxy)quinazolin-4-yl)-1-(2-fluoroacryloyl)piperazin-2-yl)acetonitrile | 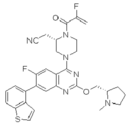 | 589.2 |
| 574 | 2-(((2S)-1-acryloyl-4-(6-fluoro-2-((((2R,7aS)-2-fluorotetrahydro-1H-pyrrolizin-7a(5H)-yl)methoxy)-7-(1,1a,6,6a-tetrahydrocyclopropa[a]indan-2-yl)quinazolin-4-yl)piperazin-2-yl)acetonitrile | 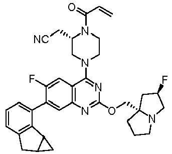 | 611.3 |
| 575 | 2-((2S)-4-(6-fluoro-2-((((2R,7aS)-2-fluorotetrahydro-1H-pyrrolizine-7a(5H)-yl)methoxy)-7-(1,1a,6,6a-tetrahydrocyclopropa[a]indan-2-yl)quinazolin-4-yl)-1-(2-fluoroacryloyl)piperazin-2-yl)acetonitrile | 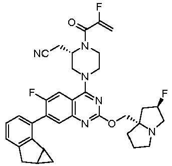 | 629.7 |
| 576 | 2-((2S)-1-acryloyl-4-(6-fluoro-2-((tetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 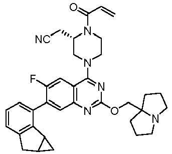 | 593.3 |
| 577 | 2-((2S)-4-(6-fluoro-2-((tetrahydro-1H-pyrrolizin-7a(5H)-yl)methoxy)-7-(1,1a,6,6a-tetrahydrocyclopropa[a]inden-2-yl)quinazolin-4-yl)-1-(2-fluoroacryloyl)piperazin-2-yl)acetonitrile | 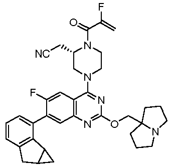 | 611.3 |
| 578 | 2-((S)-4-(6-fluoro-2-((tetrahydro-1H-pyrrolizin-7a(5H)-yl)methoxy)-7-((1aS,6aS)-1,1a,6,6a-tetrahydrocyclopropa[a]inden-2-yl)quinazolin-4-yl)-1-(2-fluoroacryloyl)piperazin-2-yl)acetonitrile | 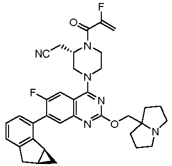 | 611.3 |
| 579 | 2-((S)-4-(6-fluoro-2-((tetrahydro-1H-pyrrolizin-7a(5H)-yl)methoxy)-7-((1aR,6aR)-1,1a,6,6a-tetrahydrocyclopropa[a]inden-2-yl)quinazolin-4-yl)-1-(2-fluoroacryloyl)piperazin-2-yl)acetonitrile | 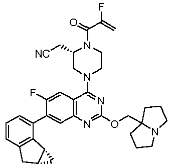 | 611.3 |
| 580 | 2-((S)-4-(6-fluoro-2-((((2R,7aS)-2-fluorotetrahydro-1H-pyrrolizine-7a(5H)-yl)methoxy)-7-((1aS,6aS)-1,1a,6,6a-tetrahydrocyclopropa[a]indan-2-yl)quinazolin-4-yl)-1-(2-fluoroacryloyl)piperazin-2-yl)acetonitrile | 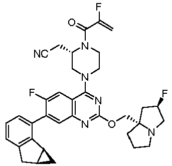 | 629.3 |
| 581 | 2-((S)-4-(6-fluoro-2-((((2R,7aS)-2-fluorotetrahydro-1H-pyrrolizine-7a(5H)-yl)methoxy)-7-((1aR,6aR)-1,1a,6,6a-tetrahydrocyclopropa[a]indan-2-yl)quinazolin-4-yl)-1-(2-fluoroacryloyl)piperazin-2-yl)acetonitrile | 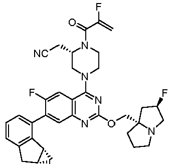 | 629.3 |
| 582 | (S)-2-(4-(7-(benzothien-4-yl)-6-fluoro-2-((tetrahydro-1H-pyrrolizin-7a(5H)-yl)methoxy)quinazolin-4-yl)-1-(2-fluoroacryloyl)piperazin-2-yl)acetonitrile | 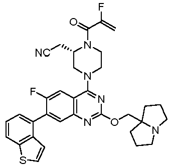 | 615.2 |
| 583 | 2-((2S)-4-(7-(benzo[b]thien-4-yl)-6-fluoro-2-(((2R)-2-fluorotetrahydro-1H-pyrrolizin-7a(5H)-yl)methoxy)quinazolin-4-yl)-1-(2-fluoroacryloyl)piperazin-2-yl)acetonitrile | 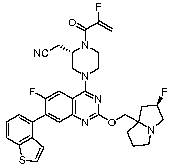 | 633.2 |
| 584 | 2-((2S)-1-acryloyl-4-(8-fluoro-2-(((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile | 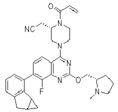 | 567.3 |
| 585 | 2-((2S)-4-(8-fluoro-2-(((S)-1-methylpyrrolidin-2-yl)methoxy)-7-(1,1a,6,6a-tetrahydrocyclopropa[a]inden-2-yl)quinazolin-4-yl)-1-(2-fluoroacryloyl)piperazin-2-yl)acetonitrile | 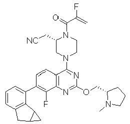 | 585.3 |
| 586 | 2-((S)-1-acryloyl-4-(8-fluoro-2-((((S)-1-methylpyrrolidin-2-yl)methoxy)-7-(thiocyano-8)quinazolin-4-yl)piperazin-2-yl)acetonitrile | 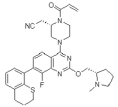 | 587.3 |
| 587 | 2-((S)-1-acryloyl-4-(7-(benzothien-7-yl)-8-fluoro-2-(((S)-1-methylpyrrolidin-2-yl)methoxy)quinazolin-4-yl)piperazin-2-yl)acetonitrile | 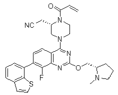 | 571.2 |
| 588 | 2-((S)-4-(7-(benzothien-7-yl)-8-fluoro-2-(((S)-1-methylpyrrolidin-2-yl)methoxy)quinazolin-4-yl)-1-(2-fluoroacryloyl)piperazin-2-yl)acetonitrile | 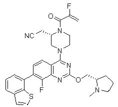 | 589.2 |
| 589 | 2-((S)-1-acryloyl-4-(7-(benzothien-4-yl)-8-fluoro-2-(((S)-1-methylpyrrolidin-2-yl)methoxy)quinazolin-4-yl)piperazin-2-yl)acetonitrile | 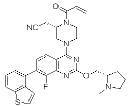 | 571.2 |
| 590 | 2-((S)-4-(7-(benzo[b]thien-4-yl)-8-fluoro-2-((S)-1-methylpyrrolidin-2-yl)methoxy)quinazolin-4-yl)-1-(2-fluoroacryloyl)piperazin-2-yl)acetonitrile | 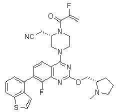 | 589.2 |
| 591 | 2-((2S)-1-acryloyl-4-(8-fluoro-2-((tetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile | 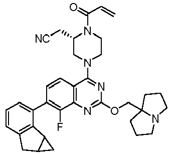 | 593.3 |
| 592 | 2-((2S)-4-(8-fluoro-2-((tetrahydro-1H-pyrrolizin-7a(5H)-yl)methoxy)-7-(1,1a,6,6a-tetrahydrocyclopropa[a]inden-2-yl)quinazolin-4-yl)-1-(2-fluoroacryloyl)piperazin-2-yl)acetonitrile | 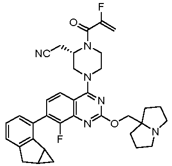 | 611.3 |
| 593 | 2-(((2S)-1-acryloyl-4-(8-fluoro-2-((((2R,7aS)-2-fluorotetrahydro-1H-pyrrolizin-7a(5H)-yl)methoxy)-7-(1,1a,6,6a-tetrahydrocyclopropa[a]indan-2-yl)quinazolin-4-yl)piperazin-2-yl)acetonitrile | 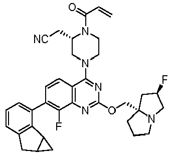 | 611.3 |
| 594 | 2-((2S)-4-(8-fluoro-2-((((2R,7aS)-2-fluorotetrahydro-1H-pyrrolizine-7a(5H)-yl)methoxy)-7-(1,1a,6,6a-tetrahydrocyclopropa[a]indan-2-yl)quinazolin-4-yl)-1-(2-fluoroacryloyl)piperazin-2-yl)acetonitrile | 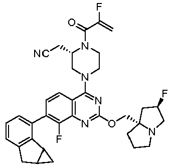 | 629.3 |
| 595 | 2-((S)-4-(8-fluoro-2-((tetrahydro-1H-pyrrolizin-7a(5H)-yl)methoxy)-7-((1aS,6aS)-1,1a,6,6a-tetrahydrocyclopropa[a]inden-2-yl)quinazolin-4-yl)-1-(2-fluoroacryloyl)piperazin-2-yl)acetonitrile | 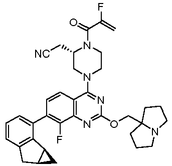 | 611.3 |
| 596 | 2-((S)-4-(8-fluoro-2-((tetrahydro-1H-pyrrolizin-7a(5H)-yl)methoxy)-7-((1aR,6aR)-1,1a,6,6a-tetrahydrocyclopropa[a]inden-2-yl)quinazolin-4-yl)-1-(2-fluoroacryloyl)piperazin-2-yl)acetonitrile | 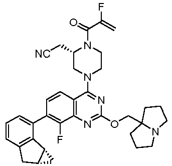 | 611.3 |
| 597 | 2-((S)-4-(8-fluoro-2-((((2R,7aS)-2-fluorotetrahydro-1H-pyrrolizin-7a(5H)-yl)methoxy)-7-((1aS,6aS)-1,1a,6,6a-tetrahydrocyclopropa[a]indan-2-yl)quinazolin-4-yl)-1-(2-fluoroacryloyl)piperazin-2-yl)acetonitrile | 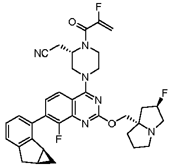 | 629.3 |
| 598 | 2-((S)-4-(8-fluoro-2-((((2R,7aS)-2-fluorotetrahydro-1H-pyrrolizine-7a(5H)-yl)methoxy)-7-((1aR,6aR)-1,1a,6,6a-tetrahydrocyclopropa[a]indan-2-yl)quinazolin-4-yl)-1-(2-fluoroacryloyl)piperazin-2-yl)acetonitrile | 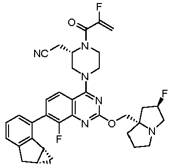 | 629.3 |
| 599 | 2-((2S)-4-(7-(benzo[b]thien-7-yl)-8-fluoro-2-((2R)-2-fluorotetrahydro-1H-pyrrolizin-7a(5H)-yl)methoxy)quinazolin-4-yl)-1-(2-fluoroacryloyl)piperazin-2-yl)acetonitrile | 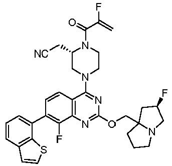 | 633.2 |
| 600 | (S)-2-(4-(7-(benzothien-7-yl)-8-fluoro-2-((tetrahydro-1H-pyrrolizin-7a(5H)-yl)methoxy)quinazolin-4-yl)-1-(2-fluoroacryloyl)piperazin-2-yl)acetonitrile | 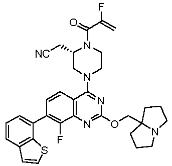 | 615.2 |
| 601 | 2-((2S)-4-(7-(benzothien-4-yl)-8-fluoro-2-((2R)-2-fluorotetrahydro-1H-pyrrolizin-7a(5H)-yl)methoxy)quinazolin-4-yl)-1-(2-fluoroacryloyl)piperazin-2-yl)acetonitrile | 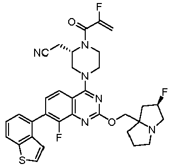 | 633.2 |
| 602 | (S)-2-(4-(7-(benzothien-4-yl)-8-fluoro-2-((tetrahydro-1H-pyrrolizin-7a(5H)-yl)methoxy)quinazolin-4-yl)-1-(2-fluoroacryloyl)piperazin-2-yl)acetonitrile | 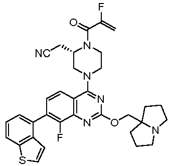 | 615.2 |
| 603 | 2-((2S)-1-acryloyl-4-(2-(((S)-1-methylpyrrolidin-2-yl)methoxy)-7-(1,1a,6,6a-tetrahydrocyclopropa[a]inden-2-yl)pyridino[2,3-d]pyrimidin-4-yl)piperazin-2-yl)acetonitrile | 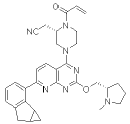 | 550.3 |
| 604 | 2-((S)-1-acryloyl-4-(2-((((S)-1-methylpyrrolidin-2-yl)methoxy)-7-(thiochroman-8-yl)pyridino[2,3-d]pyrimidin-4-yl)piperazin-2-yl)acetonitrile | 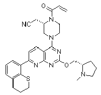 | 570.3 |
| 605 | 2-((S)-1-acryloyl-4-(7-(benzo[b]thien-7-yl)-2-(((S)-1-methylpyrrolidin-2-yl)methoxy)pyridino [2, 3-d]pyrimidin-4-yl)piperazin-2-yl)acetonitrile | 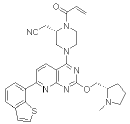 | 554.2 |
| 606 | 2-((S)-4-(7-(benzo[b]thien-4-yl)-2-((S)-1-methylpyrrolidin-2-yl)methoxy)pyridino[2,3-d]pyrimidin-4-yl)-1-(2-fluoroacryloyl)piperazin-2-yl)acetonitrile | 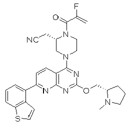 | 572.2 |
| 607 | 2-((2S)-1-acryloyl-4-(6-chloro-2-(((S)-1-methylpyrrolidin-2-yl)methoxy)-7-(1,1a,6,6a-tetrahydrocyclopropa[a]inden-2-yl)pyridino[2,3-d]pyrimidin-4-yl)piperazin-2-yl)acetonitrile | 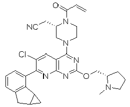 | 584.3 |
| 608 | 2-((S)-1-acryloyl-4-(6-chloro-2-((S)-1-methylpyrrolidin-2-yl)methoxy)-7-(thiochroman-8-yl)pyridino[2,3-d]pyrimidin-4-yl)piperazin-2-yl)acetonitrile | 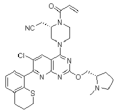 | 604.2 |
| 609 | 2-((S)-1-acryloyl-4-(7-(benzo[b]thien-7-yl)-6-chloro-2-(((S)-1-methylpyrrolidin-2-yl)methoxy)pyridino[2,3-d]pyrimidin-4-yl)piperazin-2-yl)acetonitrile | 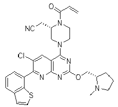 | 588.2 |
| 610 | 2-((S)-4-(7-(benzothien-4-yl)-6-chloro-2-(((S)-1-methylpyrrolidin-2-yl)methoxy)pyridino[2,3-d]pyrimidin-4-yl)-1-(2-fluoroacryloyl)piperazin-2-yl)acetonitrile | 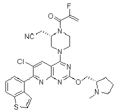 | 606.2 |
| 611 | 2-((2S)-1-acryloyl-4-(6-fluoro-2-(((S)-1-methylpyrrolidin-2-yl)methoxy)-7-(1,1a,6,6a-tetrahydrocyclopropa[a]inden-2-yl)pyridino[2,3-d]pyrimidin-4-yl)piperazin-2-yl)acetonitrile | 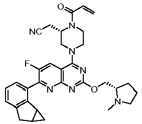 | 568.3 |
| 612 | 2-((S)-1-acryloyl-4-(6-fluoro-2-((S)-1-methylpyrrolidin-2-yl)methoxy)-7-(thiochroman-8-yl)pyridino[2,3-d]pyrimidin-4-yl)piperazin-2-yl)acetonitrile | 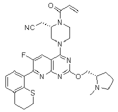 | 588.3 |
| 613 | 2-((S)-1-acryloyl-4-(7-(benzo[b]thien-7-yl)-6-fluoro-2-(((S)-1-methylpyrrolidin-2-yl)methoxy)pyridino[2,3-d]pyrimidin-4-yl)piperazin-2-yl)acetonitrile | 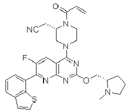 | 572.2 |
| 614 | 2-((S)-4-(7-(benzothien-4-yl)-6-fluoro-2-(((S)-1-methylpyrrolidin-2-yl)methoxy)pyridino [2, 3-d]pyrimidin-4-yl)-1-(2-fluoroacryloyl)piperazin-2-vl)acetonitrile | 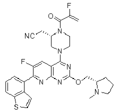 | 590.2 |

### Example 615: Synthesis of (S)-1-(4-(6-chloro-8-fluoro-7-(6-fluoro-3,4-dihydroquinoline-1(2H)-yl)-2-((1-methylpyrrolidin-2-yl)methoxy)quinazolin-4-yl)piperazin-1-yl)prop-2-en-1-one

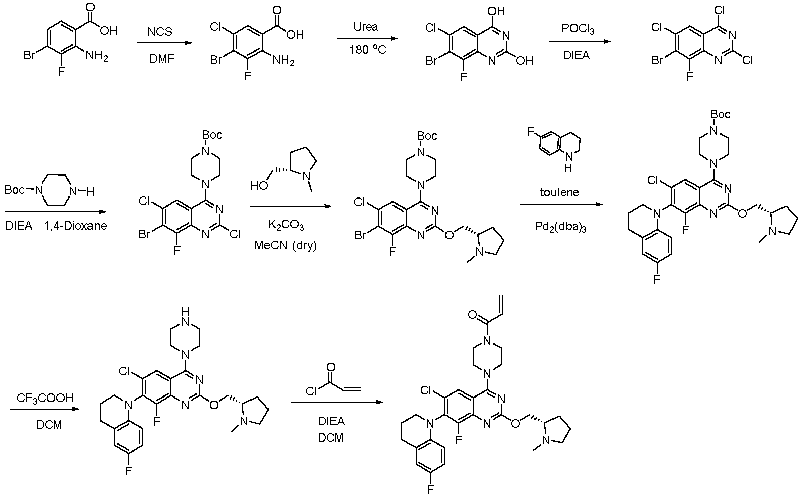

### Step 1: Synthesis of 2-amino-4-bromo-5-chloro-3-fluorobenzoic acid

2-amino-4-bromo-3-fluorobenzoic acid (2.30 g, 10 mmol) and NCS (1.60 g, 12 mmol) were dissolved in 30 ml of DMF, and the reaction system was stirred overnight at 70°C. After the reaction was complete, 300 ml of water was added to the reaction system, and a large amount of solid was precipitated. Suction filtration, drying and weighing were carried out to obtain a light yellow solid. (1.34 g, yield: 50%). ¹H NMR (400MHz, DMSO) δ 13.11 (s, 1H), 7.63 (s, 1H), 6.62 (s, 2H).

### Step 2: Synthesis of 7-bromo-6-chloro-8-fluoroquinazolin-2,4-diol

2-amino-4-bromo-5-chloro-3-fluorobenzoic acid (1.34 g, 5 mmol) and urea (1.5 g, 25 mmol) were placed in a 50 ml one-mouth flask, and the reaction system was heated to 180°C under nitrogen protection; and after three hours of reaction, 30 ml of 1 N/mol NaOH solution was added to the reaction system, the reaction system was stirred, whereby a large amount of insoluble solid precipitated out, and suction filtration was carried out to obtain a light yellow solid. (514 mg, 35%). ¹H NMR (400MHz, DMSO) δ 11.40 (s, 1H), 11.34 (s, 1H), 7.70 (s, 1H).

### Step 3: Synthesis of 7-bromo-2,4,6-trichloro-8-fluoroquinazoline

The compound 7-bromo-6-chloro-8-fluoroquinazolin-2,4-diol (500 mg, 1.73 mmol) was dissolved in 10 ml of phosphorus oxychloride, 1 ml of DMF was added, and the reaction system was heated to 110°C and stirred overnight. After the reaction was complete, phosphorus oxychloride was removed under reduced pressure to obtain a black pasty liquid. Under ice-water bath condition, ice water was added and stirred, whereby a large amount of solid precipitated out, and after suction filtration and drying, a yellow solid was obtained (300 mg, 54%). ¹H NMR (400MHz, DMSO) δ 7.70 (s, 1H).

### Step 4: Synthesis of tert-butyl 4-(7-bromo-2,6-dichloro-8-fluoroquinazolin-4-yl)piperazine-1-carboxylate

The compound tert-butyl 7-bromo-2,4,6-trichloro-8-fluoroquinazoline (300 mg, 0.9 mmol) and piperazine-1-carboxylate (167.4 mg, 0.9 mmol) was dissolved in 4 ml of 1,4-dioxane, and after DIEA (0.5 ml, 2.25 mmol) was added, the reaction system was heated at 55°C for three hours. After the reaction was complete, the solvent was removed under reduced pressure to obtain a crude product. After the crude product was dissolved in DCM, the organic phase was washed with 0.5 mol/L HCl three times. After extraction and separation, the organic phase was dried and then subjected to removal under reduced pressure to obtain a yellow solid. (414 mg, yield: 96%). ¹H NMR (400MHz, CDCl₃) δ 7.70 (s, 1H), 3.73 (m, 4H), 3.32 (m, 4H), 1.44 (s, 9H).

### Step 5: Synthesis of tert-butyl (S)-4-(7-bromo-6-chloro-8-fluoro-2-((1-methylpyrrolidin-2-yl)methoxy)quinazolin-4-yl)piperazine-1-carboxylate

The compound tert-butyl 4-(7-bromo-2,6-dichloro-8-fluoroquinazolin-4-yl)piperazine-1-carboxylate (414 mg, 0.86 mmol) and K₂CO₃ (240 mg, 1.72 mmol) were dissolved in 30 ml of ultradry acetonitrile, and (S)-(1-methylpyrrolidin-2-yl)methanol (96 mg, 0.86 mmol) was added. The reaction system was heated to 90°C under nitrogen protection and stirred for 6 h. After the reaction was complete, the solvent was removed under reduced pressure to obtain a crude product, which was separated by column chromatography (DCM : MeOH = 10 : 1) to obtain a brown solid. (170 mg, 35%)

### Step 6: Synthesis of tert-butyl (S)-4-(6-chloro-8-fluoro-7-(6-fluoro-3,4-dihydroquinoline-1(2H)-yl)-2-(((1-methylpyrrolidin-2-yl)methoxy)quinazolin-4-yl)piperazine-1-carboxylic acid

The compound tert-butyl (S)-4-(7-bromo-6-chloro-8-fluoro-2-((1-methylpyrrolidin-2-yl)methoxy)quinazolin-4-yl)piperazine-1-carboxylate (38 mg, 0.05 mmol), 6-fluoro-1,2,3,4-tetrahydroquinoline (26 mg, 0.18 mmol), t-BuONa (34 mg, 0.34 mmol), and RuPhos (38 mg, 0.08 mmol) were dissolved in 6 ml of toluene. After displacement with nitrogen, Pd2(dba)3 (38 mg, 0.04 mmol) was added, and after continued displacement with nitrogen, the reaction was carried out at 100°C overnight. After the reaction was complete, the solvent was removed under reduced pressure to obtain a crude product. The crude product was separated by column chromatography to obtain a light yellow target product. (70 mg, 65%).

### Step 7: Synthesis of (S)-6-chloro-8-fluoro-7-(6-fluoro-3,4-dihydroquinoline-1(2H)-yl)-2-((1-methylpyrrolidin-2-yl)methoxy)-4-(piperazin-1-yl)quinazoline

The compound tert-butyl (S)-4-(6-chloro-8-fluoro-7-(6-fluoro-3,4-dihydroquinoline-1(2H)-yl)-2-(((1-methylpyrrolidin-2-yl)methoxy)quinazolin-4-yl)piperazine-1-carboxylic acid (62 mg, 0.1 mmol) was dissolved in a hybrid solvent of DCM and CF3COOH (3 ml/1 ml) and stirred at room temperature for 1 h; and after the reaction was complete, the organic solvent was removed from the reaction system under reduced pressure, dichloromethane was added for dissolution, the system was then spin-dried again, and the same operation was repeated again. The resulting crude product was directly used for the next step of reaction. (53 mg, yield: 100 %)

### Step 8: Synthesis of (S)-1-(4-(6-chloro-8-fluoro-7-(6-fluoro-3,4-dihydroquinoline-1(2H)-yl)-2-((1-methylpyrrolidin-2-yl)methoxy)quinazolin-4-yl)piperazin-1-yl)prop-2-en-1-one

The compound (S)-6-chloro-8-fluoro-7-(6-fluoro-3,4-dihydroquinoline-1(2H)-yl)-2-((1-methylpyrrolidin-2-yl)methoxy)-4-(piperazin-1-yl)quinazoline (53 mg, 0.1 mmol) was dissolved in dichloromethane (3 mL), DIEA (80 mg, 0.60 mmol) was added under ice-water bath cooling condition, acryloyl chloride (18 mg, 0.13 mmol) was then added, and the mixture was reacted under stirring and ice-water bath conditions for 10 min. After the reaction was complete, the reaction was quenched with saturated sodium bicarbonate and extracted with dichloromethane, and the organic phase was dried with anhydrous sodium sulfate, concentrated and separated by PLC (dichloromethane/methanol = 1511) to obtain an off-white solid. (30 mg, yield: 50 %). ¹H NMR (400 MHz, CDCl₃) δ 7.47 (d, J = 9.1 Hz, 1H), 7.31 - 7.23 (m, 1H), 6.93 - 6.80 (m, 1H), 6.71 (s, 1H), 6.54 (dd, J = 16.7, 10.5 Hz, 1H), 6.30 (d, J = 16.7 Hz, 1H), 5.71 (d, J = 11.9 Hz, 1H), 5.12 (s, 1H), 4.67 (d, J = 10.9 Hz, 1H), 4.10 (t, J = 8.1 Hz, 2H), 3.80 (s, 7H), 3.13 (t, J = 8.2 Hz, 2H), 2.95 (s, 3H), 2.77 (s, 1H), 2.37 - 1.86 (m, 5H), 1.61 (s, 4H). MS m/z: 583.23 [M+H]+

The compounds of Examples 616-619 were prepared by the method for Example 615

| Exa mple | Compound name | Structural formula | m/z: |
|---|---|---|---|
| | | | ES⁺[M+H] |
| | | | |

| | | | |
|---|---|---|---|
| 616 | 2-((S)-1-acryloyl-4-(7-(3,4-dihydroquinoline-1 (2H)-yl)-8-fluoro-2-((((S)-1- methylpyrrolidin-2-yl)methoxy)quinazolin-4-yl)piperazin-2-yl)acetonitrile | 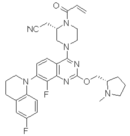 | 588.28 |
| 617 | (S)-1-(4-(8-fluoro-7-(indol-1-yl)-2-((1-methylpyrrolidin-2-yl)methoxy)quinazolin-4-yl)piperazin-1-yl)prop-2-en-1-one | 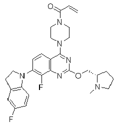 | 535.26 |
| 618 | 2-((S)-1-acryloyl-4-(7-(8-chloro-3,4-dihydroquinoline-1(2H)-yl)-2-((((S)-1- methylpyrrolidin-2-yl)methoxy)quinazolin-4-yl)piperazin-2-yl)acetonitrile | 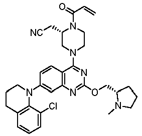 | 586 |
| 619 | 2-((S)-4-(7-(8-chloro-3,4-dihydroquinoline-1(2H)-yl)-2-(((S)-1-methylpyrrolidin-2-yl)methoxy)quinazolin-4-yl)-1-(2-fluoroacryloyl)piperazin-2-yl)acetonitrile | 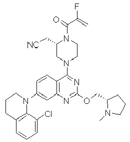 | 604 |

### Example 620; Synthesis of (S)-4-(4-acryloyl-3-(cyanomethyl)piperazin-1-yl)-7-(8-chloro-7-fluoronaphthalen-1-yl)-2-((tetrahydro-1H-pyrrolizin-7a(5H)-yl)methoxy)quinoline-3-acetonitrile

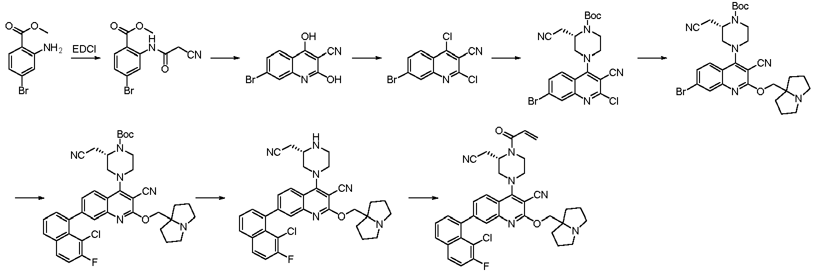

### Step 1: Synthesis of methyl 4-bromo-2-(2-cyanoacetamido)benzoate

The compound methyl 2-amino-4-bromobenzoate (10 g, 43.47 mmol) and cyanoacetic acid (4.44 g, 52.16 mmol) were dissolved in dichloromethane (100 mL), EDCI (12.5 g, 65.10 mmol) was added under ice-water bath cooling condition, and the mixture was reacted under stirring and ice-water bath cooling conditions for 1 h. After the reaction was complete, the reaction liquid was diluted by adding water and extracted with dichloromethane, and the organic phase was washed with a saturated aqueous NaCl solution, dried with anhydrous sodium sulfate and concentrated to obtain a target compound. (12.9 g, yield: 100%). ¹H NMR (400MHz, CDCl₃) δ 11.73 (s, 1H), 8.88 (d,J = 1.5Hz, 1H), 7.92 (d,J = 8.6Hz, 1H), 7.32 (dd,J = 8.6, 1.8Hz, 1H), 3.97 (s, 3H), 3.61 (s, 2H).

### Step 2: 7-bromo-2,4-dihydroxyquinoline-3-acetonitrile

The compound methyl 4-bromo-2-(2-cyanoacetamido)benzoate (12.9 g, 43.42 mmol) was dissolved in anhydrous methanol (100 mL), 30% sodium methoxide solution (11.73 g, 65.13 mmol) was added dropwise under ice-water bath cooling condition, and the mixture was reacted under stirring and ice-water bath cooling conditions for 30 min. After the reaction was complete, the reaction product was adjusted to pH 2-3 with 10% hydrochloric acid aqueous solution, the reaction liquid was diluted by adding 100 mL of water, whereby a solid precipitated out, and after filtration, the solid was dried in vacuo to obtain an off-white solid. (11.18 g, yield: 97%). ¹H NMR (400MHz, DMSO) δ 11.62 (s, 1H), 7.90 (d,J = 8.6Hz, 1H), 7.45 (d,J = 1.8Hz, 1H), 7.37 (dd, J = 8.6, 1.8Hz, 1H).

### Step 3: Synthesis of 7-bromo-2,4-dichloroquinoline-3-acetonitrile

The compound 7-bromo-2,4-dihydroxyquinoline-3-acetonitrile (11.0 g, 41.50 mmol) was dissolved in acetonitrile (10 mL) and POCl₃ (40 mL), and the mixture was heated to 90° C and reacted under stirring for 16 h. After the reaction was complete, the reaction product was cooled to room temperature and concentrated to obtain a light yellow solid, which was directly used for the next step. (12.53 g, yield: 100%).

### Step 4: Synthesis of tert-butyl (S)-4-(7-bromo-2-chloro-3-cyanoquinolin-4-yl)-2-(cyanomethyl)piperazine-1-carboxylate

The compound 7-bromo-2,4-dichloroquinoline-3-acetonitrile (5.00 g, 16.56 mmol) was dissolved in anhydrous DMF (50 mL), DIEA (12.84 g, 16.42 mL, 99.36 mmol) was added under ice-water bath cooling condition, (S)-2-(piperazin-2-yl)acetonitrile dihydrochloride (3.6 g, 18.22 mmol) was then added, the mixture was reacted under stirring and ice-water bath cooling conditions for 10 min; and di-tert butyl dicarbonate (7.25 g, 33.12 mmol) was then added, and the mixture was reacted at room temperature under stirring for 16 h. After the reaction was complete, the reaction liquid was diluted by adding 100 mL of cold water under stirring, whereby a solid precipitated out, and after filtration, the solid was washed with water and dried in vacuo to obtain a light yellow solid, which was directly used for the next step. (7.2 g, yield: 88.59%). ¹H NMR (400MHz, CDCl₃) δ 8.20 (d,J = 1.8Hz, 1H), 7.84 (d,J = 9.0Hz, 1H), 7.71 (dd,J = 9.0, 1.8Hz, 1H), 4.76 (s, 1H), 4.20 (s, 1H), 4.07 (dd,J = 12.5, 3.5Hz, 1H), 3.76 (d,J = 13.0Hz, 1H), 3.67 (d,J = 11.7 Hz, 1H), 3.56 (s, 1H), 3.44 (s, 1H), 2.83 (s, 2H).

### Step 5: Synthesis of tert-butyl (S)-4-(7-bromo-3-cyano-2-((tetrahydro-1H-pyrrolizin-7a(5H)-yl)methoxy)quinolin-4-yl)-2-(cyanomethyl)piperazine-1-carboxylate

The compound (tetrahydro-1H-pyrrolizin-7a(5H)-yl)methanol (44 mg, 0.31 mmol) was dissolved in anhydrous tetrahydrofuran (3 mL), 60% NaH (13 mg, 0.31 mmol) was added under ice-water bath cooling condition, the mixture was reacted under stirring and ice-water bath cooling conditions for 20 min, tert-butyl (S)-4-(7-bromo-2-chloro-3-cyanoquinolin-4-yl)-2-(cyanomethyl)piperazine-1-carboxylate (100 mg, 0.20 mmol) was then added to the reaction liquid, and the mixture was reacted at room temperature under stirring for 4 h. After the reaction was complete, the reaction was quenched with cold water and extracted with ethyl acetate, and the organic phase was washed with a saturated aqueous NaCl solution, dried with anhydrous sodium sulfate, concentrated, separated and purified by TLC to obtain a beige solid. (100 mg, yield: 82.7%). ¹H NMR (400 MHz, CDCl₃) δ 8.05 - 7.99 (m, 1H), 7.77 - 7.68 (m, 1H), 7.57 - 7.48 (m, 1H), 4.81 - 4.62 (m, 3H), 4.18 (s, 1H), 4.00 (dd, *J* = 12.4, 3.5 Hz, 1H), 3.82 (d, *J* = 42.0 Hz, 2H), 3.66 (dd, *J* = 29.7, 12.2 Hz, 2H), 3.49 (s, 1H), 3.35 (t, *J* = 11.2 Hz, 1H), 3.06 - 2.72 (m, 4H), 2.29 (d, *J* = 20.6 Hz, 4H), 2.11 (d, *J* = 5.8 Hz, 2H), 1.98 (s, 2H), 1.53 (s, 9H).

### Step 6: Synthesis of tert-butyl (S)-4-(7-(8-chloro-7-fluoronaphthalen-1-yl)-3-cyano-2-((tetrahydro-1H-pyrrolizin-7a(5H)-yl)methoxy)quinolin-4-yl)-2-(cyanomethyl)piperazine-1-carboxylate

The compound tert-butyl (S)-4-(7-bromo-3-cyano-2-((tetrahydro-1H-pyrrolizin-7a(5H)-yl)methoxy)quinolin-4-yl)-2-(cyanomethyl)piperazine-1-carboxylate (35 mg, 0.06 mmol) and 2-(8-chloro-7-fluoronaphthalen-1-yl)-4,4,5,5-tetramethyl-1,3,2-dioxaborane (22 mg, 0.07 mmol) was dissolved in dioxane/water = 5/1 (3 mL), cesium carbonate (58 mg, 0.18 mmol) and Pd(PPh₃)₄ (34 mg, 0.03 mmol) were then added, and after displacement with nitrogen, the mixture was heated to 90°C and reacted under stirring for 1 h. After the reaction was complete, the reaction product was cooled to room temperature, the reaction liquid was diluted by adding water and extracted with ethyl acetate, and the organic phase was dried with anhydrous sodium sulfate, concentrated, separated and purified by TLC to obtain a beige solid. (22 mg, yield: 53%). ¹H NMR (400MHz, CDCl₃) δ 7.93 (d,J = 8.2Hz, 1H), 7.87 (dd,J = 9.4, 3.7Hz, 1H), 7.83 (s, 1H), 7.57 - 7.50 (m, 2H), 7.48 - 7.36 (m, 4H), 4.93 (dd,J = 20.0, 11.4Hz, 1H), 4.84 - 4.65 (m, 2H), 4.20 (s, 1H), 4.11 - 3.97 (m, 3H), 3.91 - 3.65 (m, 2H), 3.46 (d,J = 39.4Hz, 3H), 3.00 (s, 3H), 2.93 - 2.70 (m, 1H), 2.58 - 2.28 (m, 4H), 2.22 - 1.95 (m, 4H), 1.54 (s, 9H).

### Step 7: Synthesis of (S)-7-(8-chloro-7-fluoronaphthalen-1-yl)-4-(3-(cyanomethyl)piperazin-1-yl)-2-((tetrahydro-1H-pyrrolizin-7a(5H)-yl)methoxy)quinoline-3-acetonitrile

The compound tert-butyl (S)-4-(7-(8-chloro-7-fluoronaphthalen-1-yl)-3-cyano-2-((tetrahydro-1H-pyrrolizin-7a(5H)-yl)methoxy)quinolin-4-yl)-2-(cyanomethyl)piperazine-1-carboxylate (22 mg, 0.03 mmol) was dissolved in dichloromethane (3 mL), trifluoroacetic acid (1 mL) was then added, and the mixture was reacted at room temperature under stirring for 1 h. After the reaction was complete, the reaction liquid was concentrated to remove excess trifluoroacetic acid, then dissolved in dichloromethane, washed with a saturated sodium carbonate aqueous solution and extracted with dichloromethane, and the organic phase was washed with a saturated aqueous NaCl solution, dried with anhydrous sodium sulfate and concentrated to obtain an off-white solid, which was directly used for the next step. (19 mg, yield: 100%).

### Step 8: Synthesis of (S)-4-(4-acryloyl-3-(cyanomethyl)piperazin-1-yl)-7-(8-chloro-7-fluoronaphthalen-1-yl)-2-((tetrahydro-1H-pyrrolizin-7a(5H)-yl)methoxy)quinoline-3-acetonitrile

The compound (S)-7-(8-chloro-7-fluoronaphthalen-1-yl)-4-(3-(cyanomethyl)piperazin-1-yl)-2-((tetrahydro-1H-pyrrolizin-7a(5H)-yl)methoxy)quinoline-3-acetonitrile (18 mg, 0.03 mmol) was dissolved in anhydrous dichloromethane (5 mL), DIEA (7 mg, 0.05 mmol) and acryloyl chloride (3.2 mg, 0.04 mmol) were added under ice-water bath cooling condition, and the mixture was reacted under stirring and ice-water bath conditions for 10 min. After the reaction was complete, the reaction was quenched with a saturated sodium carbonate aqueous solution and extracted with dichloromethane, and the organic phase was dried with anhydrous sodium sulfate, concentrated, separated and purified by TLC to obtain an off-white solid. (18 mg, yield: 91.7%). ¹H NMR (400 MHz, CDCl₃) δ 7.94- 7.84 (m, 4H), 7.55 - 7.38 (m, 4H), 6.67 - 6.64 (m, 1H), 6.42 (d, *J* = 16.7 Hz, 1H), 5.85 (d, *J* = 10.3 Hz, 1H), 4.92 (dd, *J* = 20.6, 11.8 Hz, 1H), 4.71 (d, *J* = 11.6 Hz, 1H), 4.16 - 3.75 (m, 6H), 3.49 (s, 1H), 3.01 (s, 4H), 2.58 - 2.25 (m, 4H), 2.15 - 2.12(m, 2H), 2.09 - 1.94 (m, 2H). MS m/z: 649.67 [M+H]⁺

### Example 621: Synthesis of (S)-4-(4-acryloyl-3-(cyanomethyl)piperazin-1-yl)-7-(8-chloro-7-fluoronaphthalen-1-yl)-6-fluoro-2-((tetrahydro-1H-pyrrolizin-7a(5H)-yl)methoxy)quinoline-3-acetonitrile

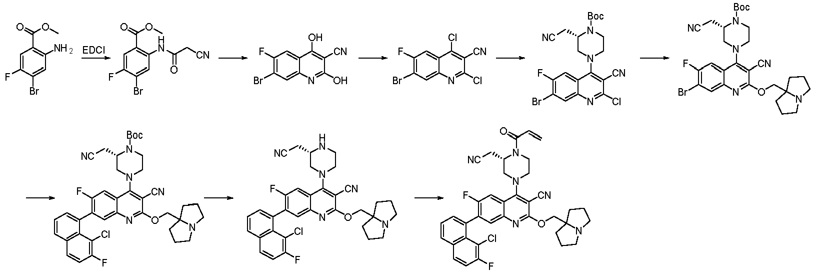

### Step 1: Synthesis of methyl 4-bromo-2-(2-cyanoacetamido)-5-fluorobenzoate

The compound methyl 2-amino-4-bromo-5-fluorobenzoate (1.2 g, 4.84 mmol) and cyanoacetic acid (0.49 g, 5.81 mmol) were dissolved in dichloromethane (15 mL), EDCI (1.39 g, 7.26 mmol) was added under ice-water bath cooling condition, and the mixture was reacted under stirring and ice-water bath cooling conditions for 1 h. After the reaction was complete, the reaction liquid was diluted by adding water and extracted with dichloromethane, and the organic phase was washed with a saturated aqueous NaCl solution, dried with anhydrous sodium sulfate and concentrated to obtain a target compound. (1.5 g, yield: 98%). ¹H NMR (400MHz, CDCl₃) δ 11.58 (s, 1H), 8.97 (d,J = 6.4Hz, 1H), 7.80 (d,J = 8.8Hz, 1H), 3.99 (s, 3H), 3.60 (s, 2H).

### Step 2: Synthesis of 7-bromo-6-fluoro-2,4-dihvdroxyquinoline-3-acetonitrile

The compound methyl 4-bromo-2-(2-cyanoacetamido)-5-fluorobenzoate (1.5 g, 4.76 mmol) was dissolved in anhydrous methanol (10 mL), 30% sodium methoxide solution (1.29 g, 7.14 mmol) was added dropwise under ice-water bath cooling condition, and the mixture was reacted under stirring and ice-water bath cooling conditions for 30 min. After the reaction was complete, the reaction product was adjusted to pH 2-3 with 10% hydrochloric acid aqueous solution, the reaction liquid was diluted by adding 50 mL of water, whereby a solid precipitated out, and after filtration, the solid was dried in vacuo to obtain an off-white solid. (1.35 g, yield: 100%). ¹H NMR (400MHz, DMSO) δ 11.47 (s, 1H), 7.82 (d,J = 9.3Hz, 1H), 7.53 (d,J = 5.9Hz, 1H).

### Step 3: Synthesis of 7-bromo-2,4-dichloro-6-fluoroquinoline-3-acetonitrile

The compound 7-bromo-6-fluoro-2,4-dihydroxyquinoline-3-acetonitrile (0.24 g, 0.85 mmol) was dissolved in acetonitrile (1 mL) and POCl₃ (4 mL), and the mixture was heated to 90°C and reacted under stirring for 16 h. After the reaction was complete, the reaction product was cooled to room temperature and concentrated to obtain a light yellow solid, which was directly used for the next step. (271 mg, yield: 100%). ¹H NMR (400MHz, CDCl₃) δ 8.38 (d,J = 6.3Hz, 1H), 7.94 (d,J = 8.3Hz, 1H).

### Step 4: Synthesis of tert-butyl (S)-4-(7-bromo-2-chloro-3-cyano-6-fluoroquinolin-4-yl)-2-(cyanomethyl)piperazine-1-carboxylate

The compound 7-bromo-2,4-dichloro-6-fluoroquinoline-3-acetonitrile (271 mg, 0.85 mmol) was dissolved in anhydrous DMF (5 mL), DIEA (659 mg, 5.09 mmol) was added under ice-water bath cooling condition, (S)-2-(piperazin-2-yl)acetonitrile dihydrochloride (185 mg, 0.93 mmol) was then added, the mixture was reacted under stirring and ice-water bath cooling conditions for 10 min; and di-tert butyl dicarbonate (372 mg, 1.70 mmol) was then added, and the mixture was reacted at room temperature under stirring for 16 h. After the reaction was complete, the reaction liquid was diluted by adding 30 mL of cold water under stirring, whereby a solid precipitated out, and after filtration, the solid was washed with water and dried in vacuo to obtain a light yellow solid, which was directly used for the next step. (400 mg, yield: 93%). ¹H NMR (400MHz, CDCl₃) δ 8.31 (d,J = 6.6Hz, 1H), 7.64 (d,J = 9.0Hz, 1H), 4.76 (s, 1H), 4.21 (s, 1H), 4.08 (dd,J = 12.4, 3.6Hz, 1H), 3.78 - 3.33 (m, 4H), 2.83 (qd,J = 16.9, 7.6Hz, 2H), 1.53 (s, 9H).

### Step 5: Synthesis of tert-butyl (S)-4-(7-bromo-3-cyano-6-fluoro-2-((tetrahydro-1H-pyrrolizin-7a(5H)-yl)methoxy)quinolin-4-yl)-2-(cyanomethyl)piperazine-1-carboxylate

The compound (tetrahydro-1H-pyrrolizin-7a(5H)-yl)methanol (44 mg, 0.31 mmol) was dissolved in anhydrous tetrahydrofuran (3 mL), 60% NaH (13 mg, 0.31 mmol) was added under ice-water bath cooling condition, the mixture was reacted under stirring and ice-water bath cooling conditions for 20 min, tert-butyl (S)-4-(7-bromo-2-chloro-3-cyano-6-fluoroquinolin-4-yl)-2-(cyanomethyl)piperazine-1-carboxylate (100 mg, 0.20 mmol) was then added to the reaction liquid, and the mixture was heated to 60°C and reacted under stirring for 2 h. After the reaction was complete, the reaction product was cooled to room temperature, the reaction was quenched with cold water and extracted with ethyl acetate, and the organic phase was washed with a saturated aqueous NaCl solution, dried with anhydrous sodium sulfate, concentrated, separated and purified by TLC to obtain an off-white solid. (100 mg, yield: 83%). ¹H NMR (400 MHz, CDCl₃) δ 8.10 (d, J = 6.6 Hz, 1H), 7.53 (d, J = 9.1 Hz, 1H), 4.75 (s, 3H), 4.19 (s, 1H), 4.01 (dd, *J* = 12.4, 3.5 Hz, 1H), 3.82 (d, *J* = 42.0 Hz, 2H), 3.66 (dd, *J* = 29.7, 12.2 Hz, 2H), 3.49 (s, 1H), 3.35 (t, *J* = 11.2 Hz, 1H), 3.06 - 2.72 (m, 4H), 2.29 (d, *J* = 20.6 Hz, 4H), 2.11 (d, *J* = 5.8 Hz, 2H), 1.98 (s, 2H), 1.53 (s, 9H).

### Step 6: Synthesis of tert-butyl (S)-4-(7-(8-chloro-7-fluoronaphthalen-1-yl)-3-cyano-6-fluoro-2-((tetrahvdro-1H-pyrrolizin-7a(5H)-yl)methoxylauinolin-4-yl)-2-(cyanomethyl)piperazine-1-carboxylate

The compound tert-butyl (S)-4-(7-bromo-3-cyano-6-fluoro-2-((tetrahydro-1H-pyrrolizin-7a(5H)-yl)methoxy)quinolin-4-yl)-2-(cyanomethyl)piperazine-1-carboxylate (30 mg, 0.05 mmol) and 2-(8-chloro-7-fluoronaphthalen-1-yl)-4,4,5,5-tetramethyl-1,3,2-dioxaborane (22 mg, 0.07 mmol) was dissolved in dioxane/water = 5/1 (3 mL), cesium carbonate (58 mg, 0.18 mmol) and Pd(PPh₃)₄ (34 mg, 0.03 mmol) were then added, and after displacement with nitrogen, the mixture was heated to 90°C and reacted under stirring for 1 h. After the reaction was complete, the reaction product was cooled to room temperature, the reaction liquid was diluted by adding water and extracted with ethyl acetate, and the organic phase was dried with anhydrous sodium sulfate, concentrated, separated and purified by TLC to obtain a beige solid. (20 mg, yield: 57%). MS m/z: 714 [M+H]⁺

### Step 7: Synthesis of (S)-7-(8-chloro-7-fluoronaphthalen-1-yl)-4-(3-(cyanomethyl)piperazin-1-yl)-6-fluoro-2-((tetrahydro-1H-pyrrolizin-7a(5H)-yl)methoxy)quinoline-3-acetonitrile

The compound tert-butyl (S)-4-(7-(8-chloro-7-fluoronaphthalen-1-yl)-3-cyano-6-fluoro-2-((tetrahydro-1H-pyrrolizin-7a(5H)-yl)methoxy)quinolin-4-yl)-2-(cyanomethyl)piperazine-1-carboxylate (20 mg, 0.03 mmol) was dissolved in dichloromethane (3 mL), trifluoroacetic acid (1 mL) was then added, and the mixture was reacted at room temperature under stirring for 1 h. After the reaction was complete, the reaction liquid was concentrated to remove excess trifluoroacetic acid, then dissolved in dichloromethane, washed with a saturated sodium carbonate aqueous solution and extracted with dichloromethane, and the organic phase was washed with a saturated aqueous NaCl solution, dried with anhydrous sodium sulfate and concentrated to obtain an off-white solid, which was directly used for the next step. (17 mg, yield: 100%).

### Step 8: Synthesis of (S)-4-(4-acryloyl-3-(cyanomethyl)piperazin-1-yl)-7-(8-chloro-7-fluoronaphthalen-1-yl)-6-fluoro-2-((tetrahydro-1H-pyrrolizin-7a(5H)-yl)methoxy)quinoline-3-acetonitrile

The compound (S)-7-(8-chloro-7-fluoronaphthalen-1-yl)-4-(3-(cyanomethyl)piperazin-1-yl)-6-fluoro-2-((tetrahydro-1H-pyrrolizin-7a(5H)-yl)methoxy)quinoline-3-acetonitrile (17 mg, 0.03 mmol) was dissolved in anhydrous dichloromethane (5 mL), DIEA (7 mg, 0.05 mmol) and acryloyl chloride (3.2 mg, 0.04 mmol) were added under ice-water bath cooling condition, and the mixture was reacted under stirring and ice-water bath conditions for 10 min. After the reaction was complete, the reaction was quenched with a saturated sodium carbonate aqueous solution and extracted with dichloromethane, and the organic phase was dried with anhydrous sodium sulfate, concentrated, separated and purified by TLC to obtain an off-white solid. (10 mg, yield: 54%). ¹H NMR (400 MHz, CDCl₃) δ 7.93 - 7.84 (m, 4H), 7.55 - 7.38 (m, 3H), 6.67 - 6.63 (m, 1H), 6.41 (d, *J* = 16.7 Hz, 1H), 5.84 (d, *J* = 10.3 Hz, 1H), 4.93 (dd, *J* = 20.6, 11.8 Hz, 1H), 4.72 (d, *J* = 11.6 Hz, 1H), 4.17 - 3.75 (m, 6H), 3.49 (s, 1H), 3.01 (s, 4H), 2.58 - 2.25 (m, 4H), 2.15 - 2.12(m, 2H), 2.09 - 1.94 (m, 2H). MS m/z: 667.6[M+H]⁺

### Example 622: Synthesis of 4-((S)-4-acryloyl-3-(cyanomethyl)piperazin-1-yl)-6-chloro-2-((((S)-1-methylpyrrolidin-2-yl)methoxy)-7-(5,6,7,8-tetrahydronaphthalen-1-yl)quinoline-3-carbonitrile

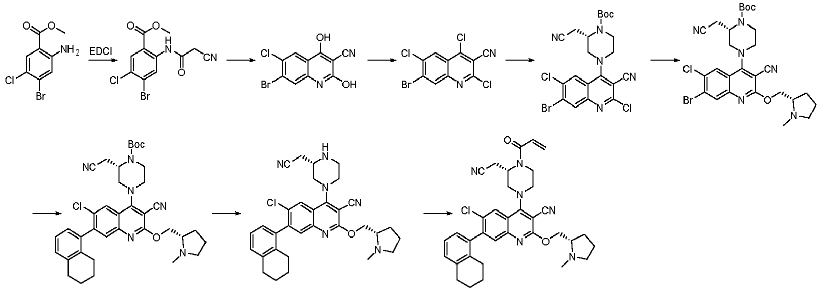

### Step 1: Synthesis of methyl 4-bromo-5-chloro-2-(2-cvanoacetamido)benzoate

The compound methyl 2-amino-4-bromo-5-chlorobenzoate (1.0 g, 3.78 mmol) and cyanoacetic acid (0.39 g, 4.53 mmol) were dissolved in dichloromethane (15 mL), EDCI (1.08 g, 5.67 mmol) was added under ice-water bath cooling condition, and the mixture was reacted under stirring and ice-water bath cooling conditions for 1 h. After the reaction was complete, the reaction liquid was diluted by adding water and extracted with dichloromethane, and the organic phase was washed with a saturated aqueous NaCl solution, dried with anhydrous sodium sulfate and concentrated to obtain a target compound. (1.19 g, yield: 95%). ¹H NMR (400MHz, CDCl₃) δ 11.63 (s, 1H), 9.03 (s, 1H), 8.13 (s, 1H), 3.99 (s, 3H), 3.61 (s, 2H).

### Step 2: Synthesis of 7-bromo-6-chloro-2,4-dihydroxyquinoline-3-acetonitrile

The compound methyl 4-bromo-2-(2-cyanoacetamido)-5-chlorobenzoate (1.19 g, 3.59 mmol) was dissolved in anhydrous methanol (10 mL), 30% sodium methoxide solution (0.97 g, 5.39 mmol) was added dropwise under ice-water bath cooling condition, and the mixture was reacted under stirring and ice-water bath cooling conditions for 30 min. After the reaction was complete, the reaction product was adjusted to pH 2-3 with 10% hydrochloric acid aqueous solution, the reaction liquid was diluted by adding 50 mL of water, whereby a solid precipitated out, and after filtration, the solid was dried in vacuo to obtain an off-white solid. (1.07 g, yield: 100%). ¹H NMR (400MHz, DMSO) δ 11.40 (s, 1H), 8.06 (s, 1H), 7.57 (s, 1H).

### Step 3: Synthesis of 7-bromo-2,4,6-trichloroquinoline-3-acetonitrile

The compound 7-bromo-6-chloro-2,4-dihydroxyquinoline-3-acetonitrile (0.20 g, 0.67 mmol) was dissolved in acetonitrile (1 mL) and POCl₃ (4 mL), and the mixture was heated to 90°C and reacted under stirring for 16 h. After the reaction was complete, the reaction product was cooled to room temperature and concentrated to obtain a light yellow solid, which was directly used for the next step. (225 mg, yield: 100%).

### Step 4: Synthesis of tert-butyl (S)-4-(7-bromo-2,6-dichloro-3-cyanoquinolin-4-yl)-2-(cyanomethyl)piperazine-1-carboxylate

The compound 7-bromo-2,4,6-trichloroquinoline-3-acetonitrile (225 mg, 0.67 mmol) was dissolved in anhydrous DMF (5 mL), DIEA (518 mg, 4.01 mmol) was added under ice-water bath cooling condition, (S)-2-(piperazin-2-yl)acetonitrile dihydrochloride (146 mg, 0.74 mmol) was then added, the mixture was reacted under stirring and ice-water bath cooling conditions for 10 min; and di-tert butyl dicarbonate (2922 mg, 1.34 mmol) was then added, and the mixture was reacted at room temperature under stirring for 4 h. After the reaction was complete, the reaction liquid was diluted by adding 30 mL of cold water under stirring, whereby a solid precipitated out, and after filtration, the solid was washed with water and dried in vacuo to obtain a light yellow solid, which was directly used for the next step. (230 mg, yield: 89%). ¹H NMR (300MHz, CDCl₃) δ 8.35 (s, 1H), 8.05 (s, 1H), 4.78 (s, 1H), 4.51 (s, 1H), 4.26 (d,J = 22.4Hz, 1H), 3.82 - 3.39 (m, 4H), 3.10 (d,J = 13.7Hz, 1H), 2.83 (qd,J = 16.9, 7.6Hz, 2H), 1.55 (s, 9H).

### Step 5: Synthesis of tert-butyl (S)-4-(7-bromo-6-chloro-3-cyano-2-((((S)-1-methylpyrrolidin-2-yl)methoxy)quinolin-4-yl)-2-(cyanomethyl)piperazine-1-carboxylate

The compound (S)-(1-methylpyrrolidin-2-yl)methanol (40 mg, 0.34 mmol) was dissolved in anhydrous tetrahydrofuran (3 mL), 60% NaH (14 mg, 0.34 mmol) was added under ice-water bath cooling condition, the mixture was reacted under stirring and ice-water bath cooling conditions for 20 min, tert-butyl (S)-4-(7-bromo-2,6-dichloro-3-cyanoquinolin-4-yl)-2-(cyanomethyl)piperazine-1-carboxylate (60 mg, 0.11 mmol) was then added to the reaction liquid, and the mixture was heated to 60°C and reacted under stirring for 2 h. After the reaction was complete, the reaction product was cooled to room temperature, the reaction was quenched with cold water and extracted with ethyl acetate, and the organic phase was washed with a saturated aqueous NaCl solution, dried with anhydrous sodium sulfate, concentrated, separated and purified by TLC to obtain an off-white solid. (50 mg, yield: 83%). ¹H NMR (400MHz, CDCl₃) δ 8.13 (s, 1H), 7.92 (s, 1H), 4.75 (s, 1H), 4.60 (s, 1H), 4.19 (s, 1H), 4.02 (dd, J = 12.4, 3.6Hz, 1H), 3.71 - 3.31 (m, 6H), 2.84 (s, 3H), 2.72 (s, 3H), 2.52 (s, 1H), 2.18 (s, 1H), 2.03 (s, 1H), 1.91 (s, 3H), 1.53 (s, 9H).

### Step 6: Synthesis of tert-butyl (S)-4-(6-chloro-3-cyano-2-(((S)-1-methylpyrrolidin-2-yl)methoxy)-7-(5,6,7,8-tetrahydronaphthalen-1-yl)quinolin-4-yl)-2-(cyanomethyl)piperazine-1-carboxylate

The compound tert-butyl (S)-4-(7-bromo-6-chloro-3-cyano-2-((((S)-1-methylpyrrolidin-2-yl)methoxy)quinolin-4-yl)-2-(cyanomethyl)piperazine-1-carboxylate (25 mg, 0.04 mmol) and 4,4,5,5-tetramethyl-2-(5,6,7,8-tetrahydronaphthalen-1-yl)-1,3,2-dioxaborane (13 mg, 0.05 mmol) were dissolved in dioxane/water = 5/1 (3 mL), cesium carbonate (40 mg, 0.12 mmol) and Pd(PPh₃)₄ (24 mg, 0.02 mmol) were then added, and after displacement with nitrogen, the mixture was heated to 90°C and reacted under stirring for 1 h. After the reaction was complete, the reaction product was cooled to room temperature, the reaction liquid was diluted by adding water and extracted with ethyl acetate, and the organic phase was dried with anhydrous sodium sulfate, concentrated, separated and purified by TLC to obtain a beige solid. (27 mg, yield: 100%). MS m/z: 655.7 [M+H]⁺

### Step 7: Synthesis of 6-chloro-4-((S)-3-(cyanomethyl)piperazin-1-yl)-2-((((S)-1-methylpyrrolidin-2-yl)methoxyl-7-(5,6,7,8-tetrahydronaphthalen-1-yl)quinoline-3-acetonitrile

The compound tert-butyl (S)-4-(6-chloro-3-cyano-2-(((S)-1-methylpyrrolidin-2-yl)methoxy)-7-(5,6,7,8-tetrahydronaphthalen-1-yl)quinolin-4-yl)-2-(cyanomethyl)piperazine-1-carboxylate (27 mg, 0.04 mmol) was dissolved in dichloromethane (3 mL), trifluoroacetic acid (1 mL) was then added, and the mixture was reacted at room temperature under stirring for 1 h. After the reaction was complete, the reaction liquid was concentrated to remove excess trifluoroacetic acid, then dissolved in dichloromethane, washed with a saturated sodium carbonate aqueous solution and extracted with dichloromethane, and the organic phase was washed with a saturated aqueous NaCl solution, dried with anhydrous sodium sulfate and concentrated to obtain an off-white solid, which was directly used for the next step. (23 mg, yield: 100%).

### Step 8: Synthesis of 4-((S)-4-acryloyl-3-(cyanomethyl)piperazin-1-yl)-6-chloro-2-((((S)-1-methylpyrrolidin-2-yl)methoxy)-7-(5,6,7,8-tetrahydroanphthalen-1-yl)quinoline-3-carbonitrile

Compound 6-chloro-4-((S)-3-(cyanomethyl)piperazin-1-yl)-2-((((S)-1-methylpyrrolidin-2-yl)methoxy)-7-(5,6,7,8-tetrahydronaphthalen-1-yl)quinoline-3-acetonitrile (23 mg, 0.04 mmol) was dissolved in anhydrous dichloromethane (5 mL), DIEA (7 mg, 0.05 mmol) and acryloyl chloride (5 mg, 0.05 mmol) were added under ice-water bath cooling condition, and the mixture was reacted under stirring and ice-water bath conditions for 10 min. After the reaction was complete, the reaction was quenched with a saturated sodium carbonate aqueous solution and extracted with dichloromethane, and the organic phase was dried with anhydrous sodium sulfate, concentrated, separated and purified by TLC to obtain an off-white solid. (8 mg, yield: 32%). ¹H NMR (400 MHz, CDCl₃) δ 7.94 (d, *J* = 2.5 Hz, 1H), 7.73 (t, *J* = 10.6 Hz, 1H), 7.19 (t, *J* = 5.1 Hz, 2H), 6.96 (dd, *J* = 10.4, 6.6 Hz, 1H), 6.61 (d, *J* = 10.6 Hz, 1H), 6.43 (d, *J* = 15.8 Hz, 1H), 5.87 (d, *J* = 10.9 Hz, 1H), 4.69 (s, 1H), 4.07 (d, *J* = 12.9 Hz, 1H), 3.93 - 3.69 (m, 3H), 3.48 (s, 2H), 3.01 (d, *J* = 8.4 Hz, 2H), 2.87 - 2.55(m, 6H), 2.54 - 2.14 (m, 4H), 1.88 - 1.74 (m, 10H). MS m/z: 609.67 [M+H]⁺.

The compounds of Examples 623-839 were prepared by preparation method 3.

| Ex. | Compound name | Structural formula | m/z: ES⁺[M+ H] |
|---|---|---|---|
| 623 | 4-((S)-4-acryloyl-3-(cyanomethyl)piperazin-1-yl)-7-(8-chloro-7-fluoronaphthalen-1-yl)-2-((((S)-1-methylpyrrolidon-2-yl)methoxy)-4a, 8a-dihydroquinoline-3-acetonitrile | 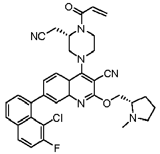 | 625 |
| 624 | 7-(8-chloro-7-fluoronaphthalen-1-yl)-4-((S)-3-(cyanomethyl)-4-(2-fluoroacryloyl)piperazin-1-yl)-2-(((S-1-methylpyrrolidin-2-yl)methoxy)-4a,8a-dihydroquinoline-3-acetonitrile | 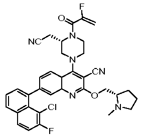 | 643 |
| 625 | 4-((S)-4-acryloyl-3-(cyanomethyl)piperazin-1-yl)-7-(8-chloro-7-fluoronaphthalen-1-yl)-2-((((2S,4R)-4-fluoro-1-methylpyrrolidin-2-yl)methoxy)-4a,8a-dihydroquinoline-3-acetonitrile | 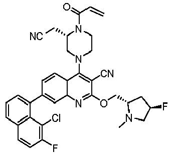 | 643 |
| 626 | 7-(8-chloro-7-fluoronaphthalen-1-yl)-4-((S)-3-(cyanomethyl)-4-(2-fluoroacryloyl)piperazin-1-yl)-2-((((4R)-4-fluoro-1-methylpyrrolidin-2-yl)methoxy)-4a,8a-dihydroquinoline-3-acetonitrile | 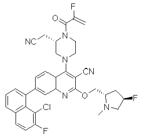 | 661 |
| 627 | 7-(8-chloro-7-fluoronaphthalen-1-yl)-4-((S)-3-(cyanomethyl)-4-(2-fluoroacryloyl)piperazin-1-yl)-2-((tetrahydro-1H-pyrrolizin-7a(5H)-yl)methoxy)-4a,8a-dihydroquinoline-3-acetonitrile | 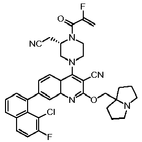 | 669 |
| 628 | 4-((S)-4-acryloyl-3-(cyanomethyl)piperazin-1-yl)-7-(8-chloro-7-fluoronaphthalen-1-yl)-2-((((2R,7aS)-2-fluorotetrahydro-1H-pyrrolizin-7a(5H)-yl)methoxy)-4a,8a-dihydroquinoline-3-acetonitrile | 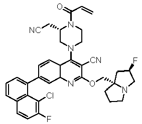 | 669 |
| 629 | 7-(8-chloro-7-fluoronaphthalen-1-yl)-4-((S)-3-(cyanomethyl)-4-(2-fluoroacryloyl)piperazin-1-yl)-2-((((2R,7aS)-2-fluorotetrahydro-1H-pyrrolizin-7a(5H)-yl)methoxy)-4a,8a-dihydroquinoline-3-acetonitrile | 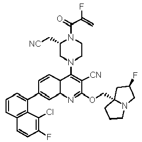 | 687 |
| 630 | 4-((lR,5R)-2-acryloyl-2,6-diazabicyclo[3.2.0]hept-6-yl)-7-(8-chloro-7-fluoronaphthalen-1-yl)-2-(((S)-1-methylpyrrolidin-2-yl)methoxy)quinoline-3-acetonitrile | 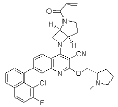 | 596 |
| 631 | 7-(8-chloro-7-fluoronaphthalen-1-yl)-4-((1R,5R)-2-(2-fluoroacryloyl)-2,6-diazabicyclo[3.2.0]hept-6-6yl)-2-((((S)-1-methylpyrrolidin-2-yl)methoxy)quinoline-3-acetonitrile | 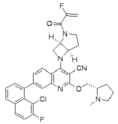 | 614 |
| 632 | 4-((lR,5R)-2-acryloyl-2,6-diazabicyclo[3.2.0]hept-6-yl)-7-(8-chloro-7-fluoronaphthalen-1-yl)-2-(((2S,4R)-4-fluoro-1-methylpyrrolidin-2-yl)methoxy)quinoline-3-acetonitrile | 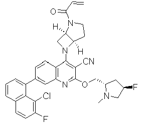 | 614 |
| 633 | 7-(8-chloro-7-fluoronaphthalen-1-yl)-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)quinoline-3-acetonitrile | 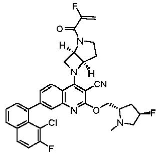 | 632 |
| 634 | 4-((lR,5R)-2-acryloyl-2,6-diazabicyclo[3.2.0]hept-6-yl)-7-(8-chloro-7-fluoronaphthalen-1-yl)-2-((tetrahydro-1H-pyrrolizin-7a(5H)-yl)methoxy)quinoline-3-acetonitrile | 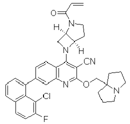 | 622 |
| 635 | 7-(8-chloro-7-fluoronaphthalen-1-yl)-4-((1R,SR)-2-(2-fluoroacryloyl)-2,6-diazabicyclo[3.2.0]hept-6-yl)-2-((tetrahydro-1H-pyrrolizin-7a(5H)-yl)methoxy)quinoline-3-acetonitrile | 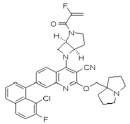 | 640 |
| 636 | 4-((lR,5R)-2-acryloyl-2,6-diazabicyclo[3.2.0]hept-6-yl)-7-(8-chloro-7-fluoronaphthalen-1-yl)-2-(((2R,7aS)-2-fluorotetrahydro-1H-pyrrolizin-7a(5H)-yl)methoxy)quinoline-3-acetonitrile | 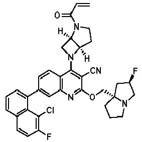 | 640 |
| 637 | 7-(8-chloro-7-fluoronaphthalen-1-yl)-4-((1R,SR)-2-(2-fluoroacryloyl)-2,6-diazabicyclo[3.2.0]hept-6-6yl)-2-((((2R,7aS)-2-fluorotetrahydro-1H-pyrrolizin-7a(5H)-yl)methoxy)quinoline-3-acetonitrile | 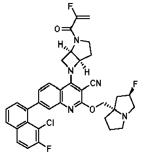 | 658 |
| 638 | 4-((S)-4-acryloyl-3-(cyanomethyl)piperazin-1-yl)-7-(8-chloronaphthalen-1-yl)-2-((((S)-1-methylpyrrolidin-2-yl)methoxy)-4a,8a-dihydroquinoline-3-acetonitrile | 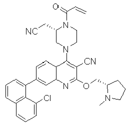 | 607 |
| 639 | 7-(8-chloronaphthalen-1-yl)-4-((S)-3-(cyanomethyl)-4-(2-fluoroacryloyl)piperazin-1-yl)-2-((((S)-1-methylpyrrolidone)-2-yl)methoxy)-4a, 8a-dihydroquinoline-3-acetonitrile | 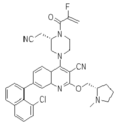 | 625 |
| 640 | 4-((S)-4-acryloyl-3-(cyanomethyl)piperazin-1-yl)-7-(8-chloronaphthalen-1-yl)-2-((((2S,4R)-4-fluoro-1-methylpyrrolidin-2-(yl)methoxy)-4a,8a-dihydroquinoline-3-acetonitrile | 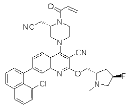 | 625 |
| 641 | 7-(8-chloronaphthalen-1-yl)-4-((S)-3-(cyanomethyl)-4-(2-fluoroacryloyl)piperazin-1-yl)-2-((((2S,4R)-4-fluoro-1-methylpyrrolidin-2-yl)methoxy)-4a,8a-dihydroquinoline-3-acetonitrile | 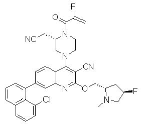 | 643 |
| 642 | 4-((S)-4-acryloyl-3-(cyanomethyl)piperazin-1-yl)-7-(8-chloronaphthalen-1-yl)-2-(((tetrahydro-1H-pyrrolizin-7a(5H)-yl)methoxy)-4a,8a-dihydroquinoline-3-acetonitrile | 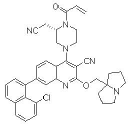 | 633 |
| 643 | 7-(8-chloronaphthalen-1-yl)-4-((S)-3-(cyanomethyl)-4-(2-fluoroacryloyl)piperazin-1-yl)-2-((tetrahydro-1H-pyrrolizin-7a(5H)-yl)methoxy)-4a,8a-dihydroquinoline-3-acetonitrile | 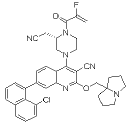 | 651 |
| 644 | 4-((S)-4-acryloyl-3-(cyanomethyl)piperazin-1-yl)-7-(8-chloronaphthalen-1-yl)-2-((((2R,7aS)-2-fluorotetrahydro-1H-pyrrolizin-7a(5H)-yl)methoxy)-4a,8a-dihydroquinoline-3-acetonitrile | 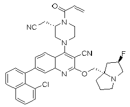 | 651 |
| 645 | 7-(8-chloronaphthalen-1-yl)-4-((S)-3-(cyanomethyl)-4-(2-fluoroacryloyl)piperazin-1-yl)-2-((((2R,7aS)-2-fluorotetrahydro-1H-pyrrolizin-7a(5H)-yl)methoxy)-4a,8a-dihydroquinoline-3-acetonitrile | 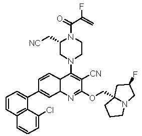 | 669 |
| 646 | 4-((lR,5R)-2-acryloyl-2,6-diazabicyclo[3.2.0]hept-6-yl)-7-(8-chloronaphthalen-1-yl)-2-(((S-1-methylpyrrolidin-2-(yl)methoxy)quinoline-3-acetonitrile | 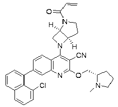 | 578 |
| 647 | 7-(8-chloronaphthalen-1-yl)-4-((1R,SR)-2-(2-fluoroacryloyl)-2,6-diazabicyclo[3.2.0]hept-6-yl)-2-((((S)-1-methylpyrrolidin-2-yl)methoxy)quinoline-3-acetonitrile | 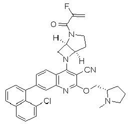 | 596 |
| 648 | 4-((lR,5R)-2-acryloyl-2,6-diazabicyclo[3.2.0]hept-6-yl)-7-(8-chloronaphthalen-1-yl)-2-(((2S,4R)-4-fluoro-1-methylpyrrolidin-2-yl)methoxy)quinoline-3-acetonitrile | 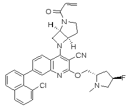 | 596 |
| 649 | 7-(8-chloronaphthalen-1-yl)-2-((((2S,4R)-4-fluoro-1-methylpyrrolidin-2-yl)methoxy)-4-((1R,SR)-2-(2-fluoroacryloyl)-2,6-diazabicyclo[3.2.0]hept-6-yl)quinoline-3-acetonitrile | 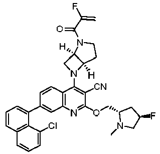 | 614 |
| 650 | 4-((lR,5R)-2-acryloyl-2,6-diazabicyclo[3.2.0]hept-6-yl)-7-(8-chloronaphthalen-1-yl)-2-((tetrahydro-1H-pyrrolizin-7a(5H)-yl)methoxy)quinoline-3-acetonitrile | 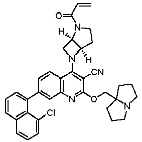 | 604 |
| 651 | 7-(8-chloronaphthalen-1-yl)-4-((1R,SR)-2-(2-fluoroacryloyl)-2,6-diazabicyclo[3.2.0]hept-6-yl)-2-(((tetrahydro-1H-pyrrolizin-7a(5H)-yl)methoxy)quinoline-3-acetonitrile | 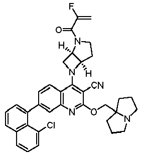 | 622 |
| 652 | 4-((lR,5R)-2-acryloyl-2,6-diazabicyclo[3.2.0]hept-6-yl)-7-(8-chloronaphthalen-1-yl)-2-(((2R,7aS)-2-fluorotetrahydro-1H-pyrrolizin-7a(5H)-yl)methoxy)quinoline-3-acetonitrile | 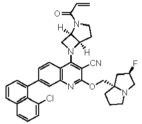 | 622 |
| 653 | 7-(8-chloronaphthalen-1-yl)-4-((1R,SR)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)quinoline-3-acetonitrile | 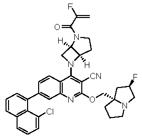 | 640 |
| 654 | 4-((S)-4-acryloyl-3-(cyanomethyl)piperazin-1-yl)-7-(8-chloronaphthalen-1-yl)-8-fluoro-2-((((S)-1-methylpyrrolidon-2-yl)methoxy)-4a, 8a-dihydroquinoline-3-acetonitrile | 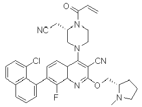 | 625 |
| 655 | 7-(8-chloronaphthalen-1-yl)-4-((S)-3-(cyanomethyl)-4-(2-fluoroacryloyl)piperazin-1-yl)-8-fluoro-2-(((S-1-methylpyrrolidin-2-yl)methoxy)-4a,8a-dihydroquinoline-3-acetonitrile | 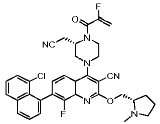 | 643 |
| 656 | 4-((S)-4-acryloyl-3-(cyanomethyl)piperazin-1-yl)-7-(8-chloronaphthalen-1-yl)-8-fluoro-2-((((2S,4R)-4-fluoro-1-methylpyrrolidin-2-yl)methoxy)-4a,8a-dihydroquinoline-3-acetonitrile | 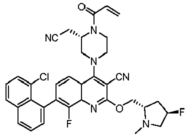 | 643 |
| 657 | 7-(8-chloronaphthalen-1-yl)-4-((S)-3-(cyanomethyl)-4-(2-fluoroacryloyl)piperazin-1-yl)-8-fluoro-2-(((4R)-4-fluoro-1-methylpyrrolidin-2-yl)methoxy)-4a,8a-dihydroquinoline-3-acetonitrile | 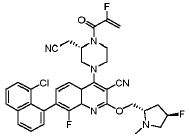 | 661 |
| 658 | 4-((S)-4-acryloyl-3-(cyanomethyl)piperazin-1-yl)-7-(8-chloronaphthalen-1-yl)-8-fluoro-2-(((tetrahydro-1H-pyrrolizin-7a(5H)-yl)methoxy)-4a,8a-dihydroquinoline-3-acetonitrile | 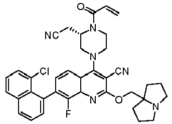 | 651 |
| 659 | 7-(8-chloronaphthalen-1-yl)-4-((S)-3-(cyanomethyl)-4-(2-fluoroacryloyl)piperazin-1 -yl)-8-fluoro-2-((tetrahydro-1H-pyrrolizin-7a(5H)-yl)methoxy)-4a,8a-dihydroquinoline-3-acetonitrile | 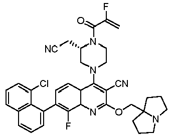 | 669 |
| 660 | 4-((S)-4-acryloyl-3-(cyanomethyl)piperazin-1-yl)-7-(8-chloronaphthalen-1-yl)-8-fluoro-2-((((2R,7aS)-2-fluorotetrahydro-1H-pyrrolizin-7a(5H)-yl)methoxy)-4a,8a-dihydroquinoline-3-acetonitrile | 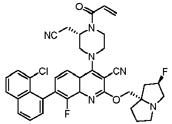 | 669 |
| 661 | 7-(8-chloronaphthalen-1-yl)-4-((S)-3-(cyanomethyl)-4-(2-fluoroacryloyl)piperazin-1-yl)-8-fluoro-2-((((2R,7aS)-2-fluorotetrahydro-1H-pyrrolizin-7a(5H)-yl)methoxy)-4a,8a-dihydroquinoline-3-acetonitrile | 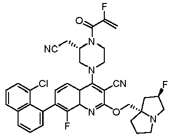 | 687 |
| 662 | 4-((1R,5R)-2-acryloyl-2,6-diazabicyclo[3.2.0]hept-6-yl)-7-(8-chloronaphthalen-1-yl)-8-fluoro-2-(((S)-1-methylpyrrolidin-2-yl)methoxy)quinoline-3-acetonitrile | 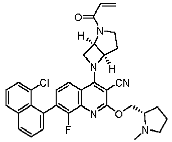 | 596 |
| 663 | 7-(8-chloronaphthalen-1-yl)-8-fluoro-4-((1R,SR)-2-(2-fluoroacryloyl)-2,6-diazabicyclo[3.2.0]hept-6-yl)-2-((((S)-1-methylpyrrolidin-2-yl)methoxy)quinoline-3-acetonitrile | 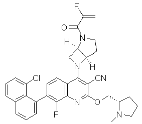 | 614 |
| 664 | 4-((1R,5R)-2-acryloyl-2,6-diazabicyclo[3.2.0]hept-6-yl)-7-(8-chloronaphthalen-1-yl)-8-fluoro-2-(((2S,4R)-4-fluoro-1-methylpyrrolidin-2-yl)methoxy)quinoline-3-acetonitrile | 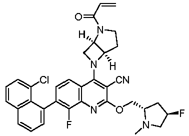 | 614 |
| 665 | (8-chloronaphthalen-1-yl)-8-fluoro-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)quinoline-3-acetonitrile | 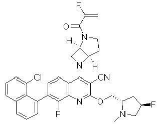 | 632 |
| 666 | 4-((lR,5R)-2-acryloyl-2,6-diazabicyclo[3.2.0]hept-6-yl)-7-(8-chloronaphthalen-1-yl)-8-fluoro-2-((tetrahydro-1H-pyrrolizin-7a(5H)-yl)methoxy)quinoline-3-acetonitrile | 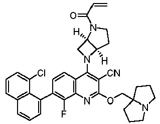 | 622 |
| 667 | 7-(8-chloronaphthalen-1-yl)-8-fluoro-4-((1R,SR)-2-(2-fluoroacryloyl)-2,6-diazabicyclo[3.2.0]hept-6-yl)-2-((tetrahydro-1H-pyrrolizin-7a(5H)-yl)methoxy)quinoline-3-acetonitrile | 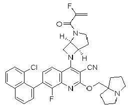 | 640 |
| 668 | 4-((lR,5R)-2-acryloyl-2,6-diazabicyclo[3.2.0]hept-6-yl)-7-(8-chloronaphthalen-1-yl)-8-fluoro-2-(((2R,7aS)-2-fluorotetrahydro-1H-pyrrolizin-7a(5H)-yl)methoxy)quinoline-3-carbonitrile | 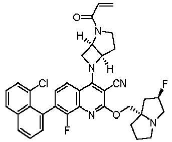 | 640 |
| 669 | 7-(8-chloronaphthalen-1-yl)-8-fluoro-4-((1R,SR)-2-(2-fluoroacryloyl)-2,6-diazabicyclo[3.2.0]hept-6-6yl)-2-((((2R,7aS)-2-fluorotetrahydro-1H-pyrrolizin-7a(5H)-yl)methoxy)quinoline-3-carbonitrile | 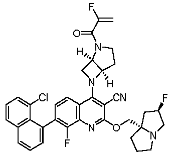 | 658 |
| 670 | 4-((S)-4-acryloyl-3-(cyanomethyl)piperazin-1-yl)-8-fluoro-2-((((S)-1-methylpyrrolidin-2-yl)methoxy)-7-(5,6,7,8-tetrahydronaphthalen-1-yl)-4a,8a-dihydroquinoline-3-acetonitrile | 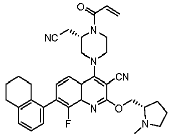 | 595 |
| 671 | 4-((S)-3-(cyanomethyl)-4-(2-fluoroacryloyl)piperazin-1-yl)-8-fluoro-2-(((S)-1-methylpyrrolidin-2-yl)methoxy)-7-(5,6,7,8-tetrahydronaphthalen-1-yl)-4a,8a-dihydroquinoline-3-acetonitrile | 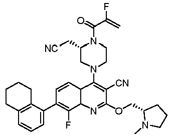 | 613 |
| 672 | 4-((S)-4-acryloyl-3-(cyanomethyl)piperazin-1-yl)-8-fluoro-2-((((2S,4R)-4-fluoro-1-methylpyrrolidin-2-yl)methoxy)-7-(5,6,7,8-tetrahydronaphthalen-1-yl)-4a,8a-dihydroquinoline-3-acetonitrile | 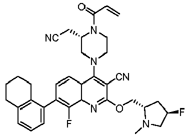 | 613 |
| 673 | 4-((S)-3-(cyanomethyl)-4-(2-fluoroacryloyl)piperazin-1-yl)-8-fluoro-2-((((2S,4R)-4-fluoro-1-methylpyrrolidin-2-yl)methoxy)-7-(5,6,7,8-tetrahydronaphthalen-1-yl)-4a,8a-dihydroquinoline-3-acetonitrile | 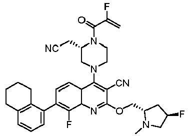 | 631 |
| 674 | 4-((S)-4-acryloyl-3-(cyanomethyl)piperazin-1-yl)-8-fluoro-2-((tetrahydro-1H-pyrrolizin-7a(5H)-yl)methoxy)-7-(5,6,7,8-tetrahydronaphthalen-1-yl)-4a,8a-dihydroquinoline-3-acetonitrile | 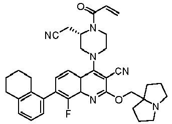 | 621 |
| 675 | 4-((S)-3-(cyanomethyl)-4-(2-fluoroacryloyl)piperazin-1-yl)-8-fluoro-2-((tetrahydro-1H-pyrrolizin-7a(5H)-yl)methoxy)-7-(5,6,7,8-tetrahydronaphthalen-1-yl)-4a,8a-dihydroquinoline-3-acetonitrile | 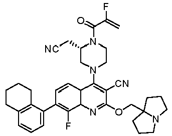 | 639 |
| 676 | 4-((S)-4-acryloyl-3-(cyanomethyl)piperazin-1-yl)-8-fluoro-2-((((2R,7aS)-2-fluorotetrahydro-1H-pyrrolizin-7a(5H)-yl))methoxy)-7-(5,6,7,8-tetrahydronaphthalen-1-y1)-4a,8a-dihydroquinoline-3-acetonitrile | 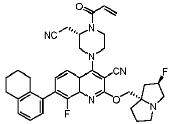 | 639 |
| 677 | 4-((S)-3-(cyanomethyl)-4-(2-fluoroacryloyl)piperazin-1-yl)-8-fluoro-2-((((2R,7aS)-2-fluorotetrahydro-1H-pyrrolizin-7a(5H)-yl)methoxy)-7-(5,6,7,8-tetrahydronaphthalen-1-yl)-4a,8a-dihydroquinoline-3-acetonitrile | 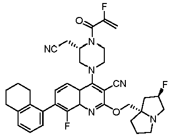 | 657 |
| 678 | 4-(((lR,5R)-2-acryloyl-2,6-diazabicyclo[3.2.0]hept-6-yl)-8-fluoro-2-((((S)-1-methylpyrrolidin-2-yl))methoxy)-7-(5,6,7,8-tetrahydronaphthalen-1-yl)quinoline-3-acetonitrile | 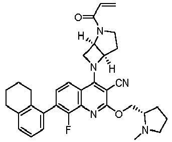 | 566 |
| 679 | 8-fluoro-4-((1R,SR)-2-(2-fluoroacryloyl)-2,6-diazabicyclo[3.2.0]hept-6-yl)-2-((((S)-1-methylpyrrolidon-2-yl)methoxy)-7-(5,6,7,8-tetrahydronaphthalen-1 -yl)quinoline-3 -acetonitrile | 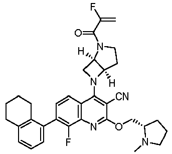 | 584 |
| 680 | 4-((lR,5R)-2-acryloyl-2,6-diazabicyclo[3.2.0]hept-6-yl)-8-fluoro-2-(((((2S,4R)-4-fluoro-1-methylpyrrolidin-2-yl)methoxy)-7-(5,6,7,8-tetrahydronaphthalen-1-yl)quinoline-3-acetonitrile | 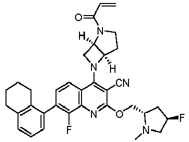 | 584 |
| 681 | 8-fluoro-2-((((2S,4R)-4-fluoro-1-methylpyrrolidin-2-yl)methoxy)-4-((lR,5R)-2-(2-fluoroacryloyl)-2,6-diazabicyclo[3.2.0]hept-6-yl)-7-(5,6,7,8-tetrahydronaphthalen-1-yl)quinoline-3-acetonitrile | 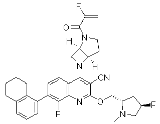 | 602 |
| 682 | 4-(((1R,5R)-2-acryloyl-2,6-diazabicyclo[3.2.0]hept-6-yl)-8-fluoro-2-((tetrahydro-1H-pyrrolizin-7a(5H)-yl)methoxy)-7-(5,6,7,8-tetrahydronaphthalen-1-yl)quinoline-3-acetonitrile | 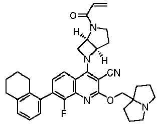 | 592 |
| 683 | 8-fluoro-4-((1R,SR)-2-(2-fluoroacryloyl)-2,6-diazabicyclo[3.2.0]hept-6-yl)-2-((tetrahydro-1H-pyrrolizin-7a(5H)-yl)methoxy)-7-(5,6,7,8-tetrahydronaphthalen-1-yl)quinoline-3-acetonitrile | 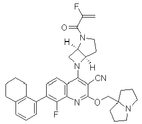 | 610 |
| 684 | 4-(((1R,5R)-2-acryloyl-2,6-diazabicyclo[3.2.0]hept-6-yl)-8-fluoro-2-(((((2R,7aS)-2-fluorotetrahydro-1H-pyrrolizin-7a(5H)-yl)methoxy)-7-(5,6,7,8-tetrahydronaphthalen-1-yl)quinoline-3-carbonitrile | 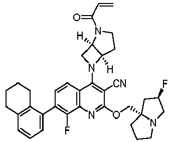 | 610 |
| 685 | 8-fluoro-4-(((1R,SR)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)-7-(5,6,7,8-tetrahydronaphthalen-1-yl)quinoline-3-acetonitrile | 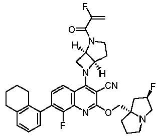 | 628 |
| 686 | 4-((S)-4-acryloyl-3-(cyanomethyl)piperazin-1-yl)-7-(8-chloronaphthalen-1-yl)-8-fluoro-2-((((S)-1-methylpyrrolidon-2-yl)methoxy)-4a, 8a-dihydro-1,6-naphthyridine-3-acetonitrile | 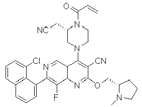 | 626 |
| 687 | 7-(8-chloronaphthalen-1-yl)-4-((S)-3-(cyanomethyl)-4-(2-fluoroacryloyl)piperazin-1-yl)-8-fluoro-2-(((S-1-methylpyrrolidin-2-yl)methoxy)-4a,8a-dihydro-1,6-naphthyridine-3-acetonitrile | 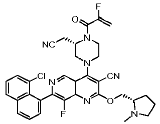 | 644 |
| 688 | 4-((S)-4-acryloyl-3-(cyanomethyl)piperazin-1-yl)-7-(8-chloronaphthalen-1-yl)-8-fluoro-2-((((2S,4R)-4-fluoro-1-methylpyrrolidin-2-yl)methoxy)-4a,8a-dihydro-1,6-naphthyridine-3-acetonitrile | 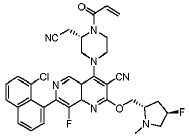 | 644 |
| 689 | 7-(8-chloronaphthalen-1-yl)-4-((S)-3-(cyanomethyl)-4-(2-fluoroacryloyl)piperazin-1-yl)-8-fluoro-2-(((4R)-4-fluoro-1-methylpyrrolidin-2-yl)methoxy)-4a,8a-dihydro-1,6-naphthyridine-3-acetonitrile | 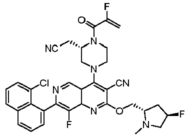 | 662 |
| 690 | 4-((S)-4-acryloyl-3-(cyanomethyl)piperazin-1-yl)-7-(8-chloronaphthalen-1-yl)-8-fluoro-2-(((tetrahydro-1H-pyrrolizin-7a(5H)-yl)methoxy)-4a,8a-dihydro-1,6-naphthyridine-3-acetonitrile | 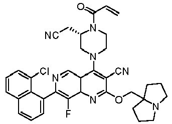 | 652 |
| 691 | 7-(8-chloronaphthalen-1-yl)-4-((S)-3-(cyanomethyl)-4-(2-fluoroacryloyl)piperazin-1-yl)-8-fluoro-2-((tetrahydro-1H-pyrrolizin-7a(5H)-yl)methoxy)-4a,8a-dihydro-1,6-naphthyridine-3-acetonitrile | 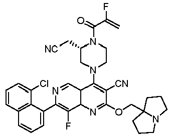 | 670 |
| 692 | 4-((S)-4-acryloyl-3-(cyanomethyl)piperazin-1-yl)-7-(8-chloronaphthalen-1-yl)-8-fluoro-2-((((2R,7aS)-2-fluorotetrahydro-1H-pyrrolizine-7a(5H)-yl)methoxy)-4a,8a-dihydro-1,6-naphthyridine-3-acetonitrile | 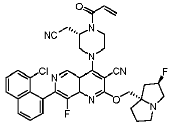 | 670 |
| 693 | 7-(8-chloronaphthalen-1-yl)-4-((S)-3-(cyanomethyl)-4-(2-fluoroacryloyl)piperazin-1-yl)-8-fluoro-2-((((2R,7aS)-2-fluorotetrahydro-1H-pyrrolizine-7a(5H)-yl)methoxy)-4a,8a-dihydro-1,6-naphthyridine-3-acetonitrile | 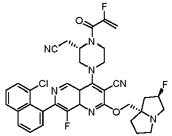 | 688 |
| 694 | 4-((1R,5R)-2-acryloyl-2,6-diazabicyclo[3.2.0]hept-6-yl)-7-(8-chloronaphthalen-1-yl)-8-fluoro-2-(((S)-1-methylpyrrolidin-2-yl)methoxy)-1,6-naphthyridine-3-acetonitrile | 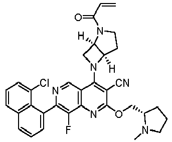 | 597 |
| 695 | 7-(8-chloronaphthalen-1-yl)-8-fluoro-4-((1R,SR)-2-(2-fluoroacryloyl)-2,6-diazabicyclo[3.2.0]hept-6-yl)-2-((((S)-1-methylpyrrolidin-2-yl)methoxy)-1,6-naphthyridine-3-acetonitrile | 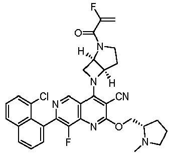 | 615 |
| 696 | 4-((1R,5R)-2-acryloyl-2,6-diazabicyclo[3.2.0]hept-6-6yl)-7-(8-chloronaphthalen-1-yl)-8-fluoro-2-(((2S,4R)-4-fluoro-1-methylpyrrolidin-2-yl)methoxy)-1,6-naphthyridine-3-acetonitrile | 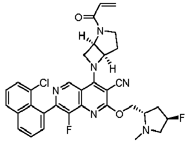 | 615 |
| 697 | 7-(8-chloronaphthalen-1-yl)-8-fluoro-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)-1,6-naphthyridine-3-acetonitrile | 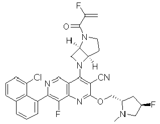 | 633 |
| 698 | 4-((1R,5R)-2-acryloyl-2,6-diazabicyclo[3.2.0]hept-6-yl)-7-(8-chloronaphthalen-1-yl)-8-fluoro-2-((tetrahydro-1H-pyrrolizin-7a(5H)-yl)methoxy)-1,6-naphthyridine-3-acetonitrile | 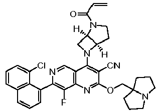 | 623 |
| 699 | 7-(8-chloronaphthalen-1-yl)-8-fluoro-4-((1R,SR)-2-(2-fluoroacryloyl)-2,6-diazabicyclo[3.2.0]hept-6-6yl)-2-((tetrahydro-1H-pyrrolizin-7a(5H)-yl)methoxy)-1,6-naphthyridine-3-acetonitrile | 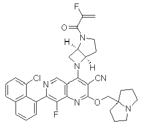 | 641 |
| 700 | 4-((1R,5R)-2-acryloyl-2,6-diazabicyclo[3.2.0]hept-6-yl)-7-(8-chloronaphthalen-1-yl)-8-fluoro-2-(((2R,7aS)-2-fluorotetrahydro-1H-pyrrolizin-7a(5H)-yl)methoxy)-1,6-naphthyridine-3-acetonitrile | 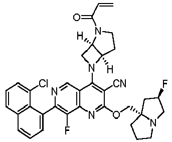 | 641 |
| 701 | 7-(8-chloronaphthalen-1-yl)-8-fluoro-4-((1R,SR)-2-(2-fluoroacryloyl)-2,6-diazabicyclo[3.2.0]hept-6-6yl)-2-((((2R,7aS)-2-fluorotetrahydro-1H-pyrrolizin-7a(5H)-yl)methoxy)-1,6-naphthyridine-3-acetonitrile | 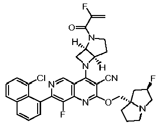 | 659 |
| 702 | 4-((S)-4-acryloyl-3-(cyanomethyl)piperazin-1-yl)-8-fluoro-2-((((S)-1-methylpyrrolidin-2-yl)methoxy)-7-(5,6,7,8-tetrahydronaphthalen-1-yl)-4a,8a-dihydro-1,6-naphthyridine-3-acetonitrile | 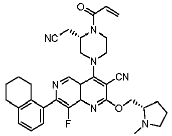 | 596 |
| 703 | 4-((S)-3-(cyanomethyl)-4-(2-fluoroacryloyl)piperazin-1-yl)-8-fluoro-2-(((S)-1-methylpyrrolidin-2-yl)methoxy)-7-(5,6,7,8-tetrahydronaphthalen-1-yl)-4a,8a-dihydro-1,6-naphthyridine-3-acetonitrile | 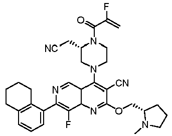 | 614 |
| 704 | 4-((S)-4-acryloyl-3-(cyanomethyl)piperazin-1-yl)-8-fluoro-2-((((2S,4R)-4-fluoro-1-methylpyrrolidin-2-yl)methoxy)-7-(5,6,7,8-tetrahydronaphthalen-1-yl)-4a,8a-dihydro-1,6-naphthyridine-3-acetonitrile | 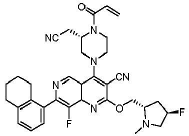 | 614 |
| 705 | 4-((S)-3-(cyanomethyl)-4-(2-fluoroacryloyl)piperazin-1-yl)-8-fluoro-2-((((2S,4R)-4-fluoro-1-methylpyrrolidin-2-yl)methoxy)-7-(5,6,7,8-tetrahydronaphthalen-1-yl)-4a,8a-dihydro-1,6-naphthyridine-3-acetonitrile | 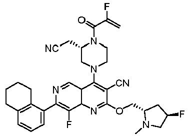 | 632 |
| 706 | 4-((S)-4-acryloyl-3-(cyanomethyl)piperazin-1-yl)-8-fluoro-2-((tetrahydro-1H-pyrrolizin-7a(5H)-yl)methoxy)-7-(5,6,7,8-tetrahydronaphthalen-1-yl)-4a,8a-dihydro-1,6-naphthyridine-3-acetonitrile | 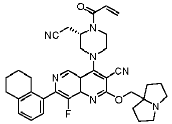 | 622 |
| 707 | 4-((S)-3-(cyanomethyl)-4-(2-fluoroacryloyl)piperazin-1-yl)-8-fluoro-2-((tetrahydro-1H-pyrrolizin-7a(5H)-yl)methoxy)-7-(5,6,7,8-tetrahydronaphthalen-1-yl)-4a,8a-dihydro-1,6-naphthyridine-3-acetonitrile | 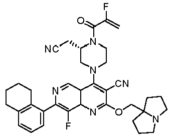 | 640 |
| 708 | 4-((S)-4-acryloyl-3-(cyanomethyl)piperazin-1-yl)-8-fluoro-2-((((2R,7aS)-2-fluorotetrahydro-1H-pyrrolizin-7a(5H)-yl))methoxy)-7-(5,6,7,8-tetrahydronaphthalen-1-yl)-4a,8a-dihydro-1,6-naphthyridine-3-acetonitrile | 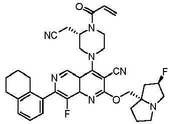 | 640 |
| 709 | 4-((S)-3-(cyanomethyl)-4-(2-fluoroacryloyl)piperazin-1-yl)-8-fluoro-2-((((2R,7aS)-2-fluorotetrahydro-1H-pyrrolizin-7a(5H)-yl)methoxy)-7-(5,6,7,8-tetrahydronaphthalen-1-yl)-4a,8a-dihydro-1,6-naphthyridine-3-acetonitrile | 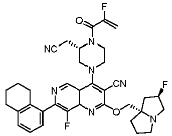 | 658 |
| 710 | 4-((1R,5R)-2-acryloyl-2,6-diazabicyclo[3.2.0]hept-6-yl)-8-fluoro-2-((((S)-1-methylpyrrolidin-2-yl)methoxy)-7-(5,6,7,8-tetrahydronaphthalen-1-yl)-1,6-naphthyridine-3-acetonitrile | 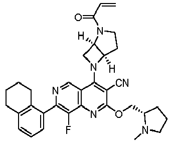 | 567 |
| 711 | 8-fluoro-4-((1R,SR)-2-(2-fluoroacryloyl)-2,6-diazabicyclo[3.2.0]hept-6-yl)-2-((((S)-1-methylpyrrolidon-2-yl)methoxy)-7-(5,6,7,8-tetrahydronaphthalen-1-yl)-1,6-naphthyridine-3-acetonitrile | 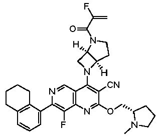 | 585 |
| 712 | 4-((1R,5R)-2-acryloyl-2,6-diazabicyclo[3.2.0]hept-6-yl)-8-fluoro-2-(((2S,4R)-4-fluoro-1-methylpyrrolidin-2-yl)methoxy)-7-(5,6,7,8-tetrahydronaphthalen-1-yl)-1,6-naphthyridine-3-acetonitrile | 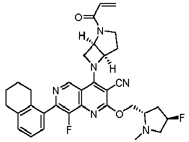 | 585 |
| 713 | 8-fluoro-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)-7-(5,6,7,8-tetrahydronaphthalen-1-yl)-1,6-naphthyridine-3-acetonitrile | 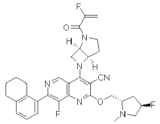 | 603 |
| 714 | 4-(((1R,5R)-2-acryloyl-2,6-diazabicyclo[3.2.0]hept-6-yl)-8-fluoro-2-((tetrahydro-1H-pyrrolizin-7a(5H)-yl)methoxy)-7-(5,6,7,8-tetrahydronaphthalen-1-yl)-1,6-naphthyridine-3-acetonitrile | 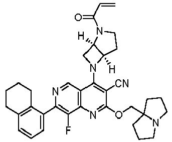 | 593 |
| 715 | 8-fluoro-4-((1R,SR)-2-(2-fluoroacryloyl)-2,6-diazabicyclo[3.2.0]hept-6-yl)-2-((tetrahydro-1H-pyrrolizin-7a(5H)-yl)methoxy)-7-(5,6,7,8-tetrahydronaphthalen-1-yl)-1,6-naphthyridine-3-acetonitrile | 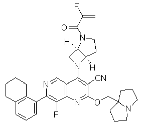 | 611 |
| 716 | 4-(((1R,5R)-2-acryloyl-2,6-diazabicyclo[3.2.0]hept-6-yl)-8-fluoro-2-(((((2R,7aS)-2-fluorotetrahydro-1H-pyrrolizin-7a(5H)-yl)methoxy)-7-(5,6,7,8-tetrahydronaphthalen-1-yl)-1,6-naphthyridine-3-acetonitrile | 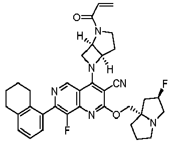 | 611 |
| 717 | 8-fluoro-4-(((1R,SR)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)-7-(5,6,7,8-tetrahydronaphthalen-1-yl)-1,6-naphthyridine-3-acetonitrile | 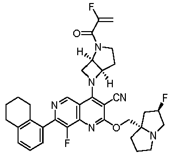 | 629 |
| 718 | 4-((S)-4-acryloyl-3-(cyanomethyl)piperazin-1-yl)-6-chloro-7-(8-chloro-7-fluoronaphthalen-1-yl)-2-((((S)-1-methylpyrrolidin-2-yl)methoxy)-4a,8a-dihydroquinoline-3-acetonitrile | 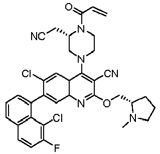 | 659 |
| 719 | 6-chloro-7-(8-chloro-7-fluoronaphthalen- 1-yl)-4-((S)-3-(cyanomethyl)-4-(2-fluoroacryloyl)piperazin-1-yl)-2-(((S)-1-methylpyrrolidin-2-yl)methoxy)-4a,8a-dihydroquinoline-3-acetonitrile | 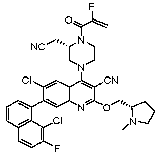 | 677 |
| 720 | 4-((S)-4-acryloyl-3-(cyanomethyl)piperazin-1-yl)-6-chloro-7-(8-chloro-7-fluoronaphthalen-1-yl)-2-((((4R)-4-fluoro-1-methylpyrrolidin-2-yl)methoxy)-4a,8a-dihydroquinoline-3-acetonitrile | 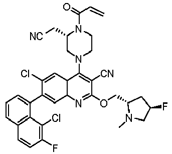 | 677 |
| 721 | 6-chloro-7-(8-chloro-7-fluoronaphthalen- 1-yl)-4-((S)-3-(cyanomethyl)-4-(2-fluoroacryloyl)piperazin-1-yl)-2-((((2S,4R)-4-fluoro-1-methylpyrrolidin-2-yl)methoxy)-4a,8a-dihydroquinoline-3-acetonitrile | 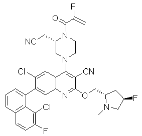 | 695 |
| 722 | 4-((S)-4-acryloyl-3-(cyanomethyl)piperazin-1-yl)-6-chloro-7-(8-chloro-7-fluoronaphthalen-1-yl)-2-((tetrahydro-1H-pyrrolizin-7a(5H)-yl)methoxy)-4a,8a-dihydroquinoline-3-acetonitrile | 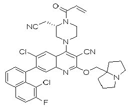 | 685 |
| 723 | 6-chloro-7-(8-chloro-7-fluoronaphthalen- 1-yl)-4-((S)-3-(cyanomethyl)-4-(2-fluoroacryloyl)piperazin-1-yl)-2-((tetrahydro-1H-pyrrolizin-7a(5H)-yl)methoxy)-4a,8a-dihydroquinoline-3-acetonitrile | 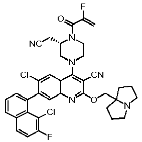 | 703 |
| 724 | 4-((S)-4-acryloyl-3-(cyanomethyl)piperazin-1-yl)-6-chloro-7-(8-chloro-7-fluoronaphthalen-1 -yl)-2-((((2R,7aS)-2-fluorotetrahydro-1H-pyrrolizin-7a(5H)-yl)methoxy)-4a,8a-dihydroquinoline-3-acetonitrile | 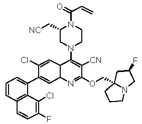 | 703 |
| 725 | 6-chloro-7-(8-chloro-7-fluoronaphthalen- 1-yl)-4-((S)-3-(cyanomethyl)-4-(2-fluoroacryloyl)piperazin-1-yl)-2-(((2R,7aS)-2-fluorotetrahydro-1H-pyrrolizin-7a(5H)-yl)methoxy)-4a,8a-dihydroquinoline-3-acetonitrile | 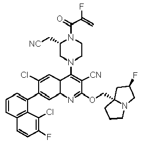 | 721 |
| 726 | 4-((1R,5R)-2-acryloyl-2,6-diazabicyclo[3.2.0]hept-6-yl)-6-chloro-7-(8-chloro-7-fluoronaphthalen-1-yl)-2-((((S)-1-methylpyrrolidin-2-yl)methoxy)quinoline-3-acetonitrile | 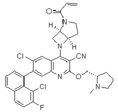 | 630 |
| 727 | 6-chloro-7-(8-chloro-7-fluoronaphthalen-1-yl)-4-((1R,5R)-2-(2-fluoroacryloyl)-2,6-diazabicyclo[3.2.0]hept-6-yl)-2-((((S)-1-methylpyrrolidin-2-yl)methoxy)quinoline-3-acetonitrile | 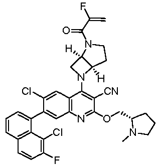 | 648 |
| 728 | 4-((1R,5R)-2-acryloyl-2,6-diazabicyclo[3.2.0]hept-6-yl)-6-chloro-7-(8-chloro-7-fluoronaphthalen-1-yl)-2-((((2S,4R)-4-fluoro-1-methylpyrrolidin-2-yl)methoxy)quinoline-3-acetonitrile | 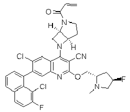 | 648 |
| 729 | 6-chloro-7-(8-chloro-7-fluoronaphthalen-1-yl)-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)quinoline-3-acetonitrile | 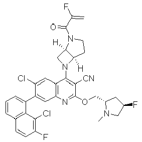 | 666 |
| 730 | 4-((1R,5R)-2-acryloyl-2,6-diazabicyclo[3.2.0]hept-6-yl)-6-chloro-7-(8-chloro-7-fluoronaphthalen-1-yl)-2-((tetrahydro-1H-pyrrolizin-7a(5H)-yl)methoxy)quinoline-3-acetonitrile | 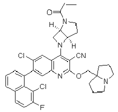 | 656 |
| 731 | 6-chloro-7-(8-chloro-7-fluoronaphthalen-1-yl)-4-((1R,5R)-2-(2-fluoroacryloyl)-2,6-diazabicyclo[3.2.0]hept-6-yl)-2-(((tetrahydro-1H-pyrrolizin-7a(5H)-yl)methoxy)quinoline-3-acetonitrile | 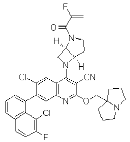 | 674 |
| 732 | 4-((1R,5R)-2-acryloyl-2,6-diazabicyclo[3.2.0]hept-6-yl)-6-chloro-7-(8-chloro-7-fluoronaphthalen-1-yl)-2-(((((2R,7aS)-2-fluorotetrahydro-1H-pyrrolizin-7a(5H)-yl)methoxy)quinoline-3-acetonitrile | 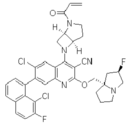 | 674 |
| 733 | 6-chloro-7-(8-chloro-7-fluoronaphthalen-1-yl)-4-((lR,5R)-2-(2-fluoroacryloyl)-2,6-diazabicyclo[3.2.0]hept-6-yl)-2-(((((2R,7aS)-2-fluorotetrahydro-1H-pyrrolizin-7a(5H)-yl)methoxy)quinoline-3-acetonitrile | 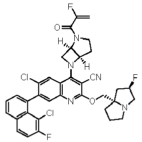 | 692 |
| 734 | 4-((S)-4-acryloyl-3-(cyanomethyl)piperazin-1-yl)-6-chloro-7-(8-chloronaphthalen-1-yl)-2-((((S)-1-methylpyrrolidon-2-yl)methoxy)-4a, 8a-dihydroquinoline-3-acetonitrile | 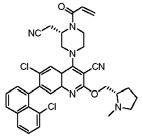 | 641 |
| 735 | 6-chloro-7-(8-chloronaphthalen-1-yl)-4-((S)-3-(cyanomethyl)-4-(2-fluoroacryloyl)piperazin-1-yl)-2-(((S-1-methylpyrrolidin-2-yl)methoxy)-4a,8a-dihydroquinoline-3-acetonitrile | 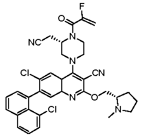 | 659 |
| 736 | 4-((S)-4-acryloyl-3-(cyanomethyl)piperazin-1-yl)-6-chloro-7-(8-chloronaphthalen-1-yl)-2-((((2S,4R)-4-fluoro-1-methylpyrrolidin-2-yl)methoxy)-4a,8a-dihydroquinoline-3-acetonitrile | 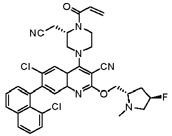 | 659 |
| 737 | 6-chloro-7-(8-chloronaphthalen-1-yl)-4-((S)-3-(cyanomethyl)-4-(2-fluoroacryloyl)piperazin-1-yl)-2-(((( 4R)-4-fluoro-1 -methylpyrrolidin-2-yl)methoxy)-4a,8a-dihydroquinoline-3-acetonitrile | 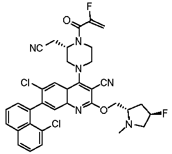 | 677 |
| 738 | 4-((S)-4-acryloyl-3-(cyanomethyl)piperazin-1-yl)-6-chloro-7-(8-chloronaphthalen-1-yl)-2-((tetrahydro-1H-pyrrolizin-7a(5H)-yl)methoxy)-4a,8a-dihydroquinoline-3-acetonitrile | 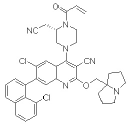 | 667 |
| 739 | 6-chloro-7-(8-chloronaphthalen-1-yl)-4-((S)-3-(cyanomethyl)-4-(2-fluoroacryloyl)piperazin-1-yl)-2-((tetrahydro-1H-pyrrolizin-7a(5H)-yl)methoxy)-4a,8a-dihydroquinoline-3-acetonitrile | 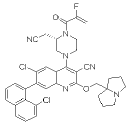 | 685 |
| 740 | 4-((S)-4-acryloyl-3-(cyanomethyl)piperazin-1-yl)-6-chloro-7-(8-chloronaphthalen-1-yl)-2-((((2R,7aS)-2-fluorotetrahydro-1H-pyrrolizine-7a(5H)-yl)methoxy)-4a,8a-dihydroquinoline-3-acetonitrile | 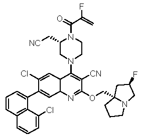 | 685 |
| 741 | 6-chloro-7-(8-chloronaphthalen-1-yl)-4-((S)-3-(cyanomethyl)-4-(2-fluoroacryloyl)piperazin-1-yl)-2-((((2R,7aS)-2-fluorotetrahydro-1H-pyrrolizin-7a(5H)-yl)methoxy)-4a,8a-dihydroquinoline-3-acetonitrile | 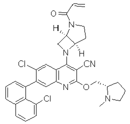 | 703 |
| 742 | 4-((1R,5R)-2-acryloyl-2,6-diazabicyclo[3.2.0]hept-6-yl)-6-chloro-7-(8-chloronaphthalen-1-yl)-2-(((S)-1-methylpyrrolidin-2-yl)methoxy)quinoline-3-acetonitrile | 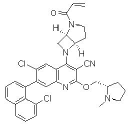 | 612 |
| 743 | 6-chloro-7-(8-chloronaphthalen-1-yl)-4-((1R,5R)-2-(2-fluoroacryloyl)-2,6-diazabicyclo[3.2.0]hept-6-yl)-2-((((S)-1-methylpyrrolidin-2-yl)methoxy)quinoline-3-acetonitrile | 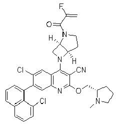 | 630 |
| 744 | 4-((1R,5R)-2-acryloyl-2,6-diazabicyclo[3.2.0]hept-6-yl)-6-chloro-7-(8-chloronaphthalen-1-yl)-2-(((2S,4R)-4-fluoro-1-methylpyrrolidin-2-yl)methoxy)quinoline-3-acetonitrile | 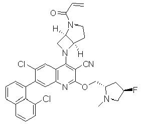 | 630 |
| 745 | 6-chloro-7-(8-chloronaphthalen-1-yl)-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)quinoline-3-acetonitrile | 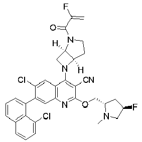 | 648 |
| 746 | 4-((1R,5R)-2-acryloyl-2,6-diazabicyclo[3.2.0]hept-6-yl)-6-chloro-7-(8-chloronaphthalen-1-yl)-2-((tetrahydro-1H-pyrrolizin-7a(5H)-yl)methoxy)quinoline-3-acetonitrile | 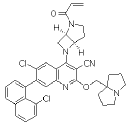 | 638 |
| 747 | 6-chloro-7-(8-chloronaphthalen-1-yl)-4-((1R,5R)-2-(2-fluoroacryloyl)-2,6-diazabicyclo[3.2.0]hept-6-yl)-2-((tetrahydro-1H-pyrrolizin-7a(5H)-yl)methoxy)quinoline-3-acetonitrile | 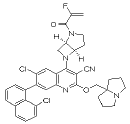 | 656 |
| 748 | 4-((1R,5R)-2-acryloyl-2,6-diazabicyclo[3.2.0]hept-6-yl)-6-chloro-7-(8-chloronaphthalen-1-yl)-2-(((2R,7aS)-2-fluorotetrahydro-1H-pyrrolizin-7a(5H)-yl)methoxy)quinoline-3-acetonitrile | 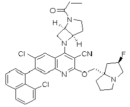 | 656 |
| 749 | 6-chloro-7-(8-chloronaphthalen-1-yl)-4-((1R,5R)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)quinoline-3-acetonitrile | 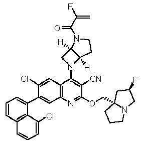 | 674 |
| 750 | 6-chloro-4-((S)-3-(cyanomethyl)-4-(2-fluoroacryloyl)piperazin-1-yl)-2-((((S-1--1-methylpyrrolidin-2-yl)methoxy)-7-(5,6,7,8-tetrahydronaphthalen-1-yl)-4a,8a-dihydroquinoline-3-acetonitrile | 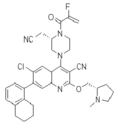 | 629 |
| 751 | 4-((S)-4-acryloyl-3-(cyanomethyl)piperazin-1-yl)-6-chloro-2-((((2S,4R)-4-fluoro-1-methylpyrrolidin-2-yl)methoxy)-7-(5,6,7,8-tetrahydronaphthalen-1-yl)-4a,8a-dihydroquinoline-3-acetonitrile | 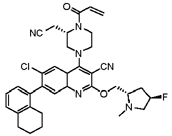 | 629 |
| 752 | 6-chloro-4-((S)-3-(cyanomethyl)-4-(2-fluoroacryloyl)piperazin-1-yl)-2-((((2S,4R)-4-fluoro-1- methylpyrrolidin-2-yl)methoxy)-7-(5,6,7,8-tetrahydronaphthalen-1-yl)-4a,8a-dihydroquinoline-3-acetonitrile | 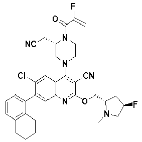 | 647 |
| 753 | 4-((S)-4-acryloyl-3-(cyanomethyl)piperazin-1-yl)-6-chloro-2-((tetrahydro-1H-pyrrolizin-7a(5H)-yl)methoxy)-7-(5,6,7,8-tetrahydronaphthalen-1-yl)-4a,8a-dihydroquinoline-3-acetonitrile | 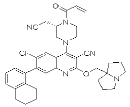 | 637 |
| 754 | 6-chloro-4-((S)-3-(cyanomethyl)-4-(2-fluoroacryloyl)piperazin-1-yl)-2-((tetrahydro-1H-pyrrolizin-7a(5H)-yl)methoxy)-7-(5,6,7,8-tetrahydronaphthalen-1-yl)-4a,8a-dihydroquinoline-3-acetonitrile | 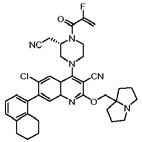 | 655 |
| 755 | 4-((S)-4-acryloyl-3-(cyanomethyl)piperazin-1-yl)-6-chloro-2-((((2R,7aS)-2-fluorotetrahydro-1H-pyrrolizin-7a(5H)-yl))methoxy)-7-(5,6,7,8-tetrahydronaphthalen-1-yl)-4a,8a-dihydroquinoline-3-acetonitrile | 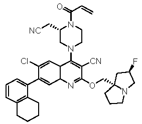 | 655 |
| 756 | 6-chloro-4-((S)-3-(cyanomethyl)-4-(2-fluoroacryloyl)piperazin-1-yl)-2-((((2R,7aS)-2-fluorotetrahydro-1H-pyrrolizin-7a(5H))-yl)methoxy)-7-(5,6,7,8-tetrahydronaphthalen-1-yl)-4a,8a-dihydroquinoline-3-acetonitrile | 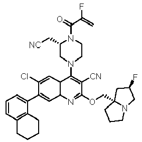 | 673 |
| 757 | 4-((1R,5R)-2-acryloyl-2,6-diazabicyclo[3.2.0]hept-6-yl)-6-chloro-2-(((S)-1-methylpyrrolidin-2-yl)methoxy)-7-(5,6,7,8-tetrahydronaphthalen-1-yl)quinoline-3-acetonitrile | 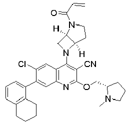 | 582 |
| 758 | 6-chloro-4-((1R,5R)-2-(2-fluoroacryloyl)-2,6-diazabicyclo[3.2.0]hept-6-yl)-2-((((S)-1-methylpyrrolidin-2-yl)methoxy)-7-(5,6,7,8-tetrahydronaphthalen-1-yl)quinoline-3-acetonitrile | 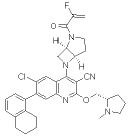 | 600 |
| 759 | 4-(((1R,5R)-2-acryloyl-2,6-diazabicyclo[3.2.0]hept-6-yl)-6-chloro-2-((((2S,4R)-4-fluoro-1-methylpyrrolidin-2-yl)methoxy)-7-(5,6,7,8-tetrahydronaphthalen-1-yl)quinoline-3-acetonitrile | 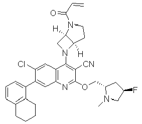 | 600 |
| 760 | 6-chloro-2-(((2S,4R)-4-fluoro-1-methylpyrrolidin-2-yl)methoxy)-4-((lR,5R)-2-(2-fluoroacryloyl)-2,6-diazabicyclo[3.2.0]hept-6-yl)-7-(5,6,7,8-tetrahydronaphthalen-1-yl)quinoline-3-acetonitrile | 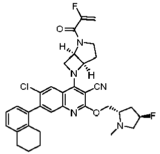 | 618 |
| 761 | 4-((1R,5R)-2-acryloyl-2,6-diazabicyclo[3.2.0]hept-6-yl)-6-chloro-2-((tetrahydro-1H-pyrrolizin-7a(5H)-yl)methoxy)-7-(5,6,7,8-tetrahydronaphthalen-1-yl)quinoline-3-acetonitrile | 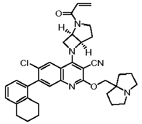 | 608 |
| 762 | 6-chloro-4-((1R,5R)-2-(2-fluoroacryloyl)-2,6-diazabicyclo[3.2.0]hept-6-yl)-2-((tetrahydro-1H-pyrrolizin-7a(5H)-yl)methoxy)-7-(5,6,7,8-tetrahydronaphthalen-1-yl)quinoline-3-acetonitrile | 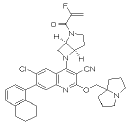 | 626 |
| 763 | 4-(((1R,5R)-2-acryloyl-2,6-diazabicyclo[3.2.0]hept-6-yl)-6-chloro-2-(((((2R,7aS)-2-fluorotetrahydro-1H-pyrrolizin-7a(5H)-yl)methoxy)-7-(5,6,7,8-tetrahydronaphthalen-1-yl)quinoline-3-acetonitrile | 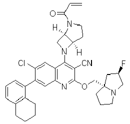 | 626 |
| 764 | 6-chloro-4-((1R,5R)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)-7-(5,6,7,8-tetrahydronaphthalen-1-yl)quinoline-3-acetonitrile | 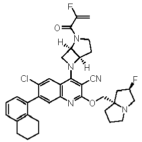 | 644 |
| 765 | 4-((S)-4-acryloyl-3-(cyanomethyl)piperazin-1-yl)-7-(8-chloro-7-fluoronaphthalen-1-yl)-6-fluoro-2-((((S)-1-methylpyrrolidin-2-yl)methoxy)-4a,8a-dihydroquinoline-3-acetonitrile | 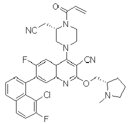 | 643 |
| 766 | 7-(8-chloro-7-fluoronaphthalen-1-yl)-4-((S)-3-(cyanomethyl)-4-(2-fluoroacryloyl)piperazin-1-yl)-6-fluoro-2-(((S)-1-methylpyrrolidin-2-yl)methoxy)-4a,8a-dihydroquinoline-3-acetonitrile | 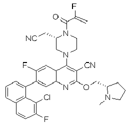 | 661 |
| 767 | 4-((S)-4-acryloyl-3-(cyanomethyl)piperazin-1-yl)-7-(8-chloro-7-fluoronaphthalen-1-yl)-6-fluoro-2-((((4R)-4-fluoro-1-methylpyrrolidin-2-yl)methoxy)-4a,8a-dihydroquinoline-3-acetonitrile | 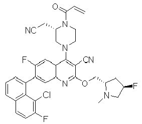 | 661 |
| 768 | 7-(8-chloro-7-fluoronaphthalen-1-yl)-4-((S)-3-(cyanomethyl)-4-(2-fluoroacryloyl)piperazin-1-yl)-6-fluoro-2-(((2S,4R)-4-fluoro-1-methylpyrrolidin-2-yl)methoxy)-4a,8a-dihydroquinoline-3-acetonitrile | 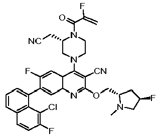 | 679 |
| 769 | 7-(8-chloro-7-fluoronaphthalen-1-yl)-4-((S)-3-(cyanomethyl)-4-(2-fluoroacryloyl)piperazin-1-yl)-6-fluoro-2-((tetrahydro-1H-pyrrolizin-7a(5H)-yl)methoxy)-4a,8a-dihydroquinoline-3-acetonitrile | 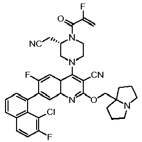 | 687 |
| 770 | 4-((S)-4-acryloyl-3-(cyanomethyl)piperazin-1-yl)-7-(8-chloro-7-fluoronaphthalen-1-yl)-6-fluoro-2-((((2R,7aS)-2-fluorotetrahydro-1H-pyrrolizine-7a(5H)-yl)methoxy)-4a,8a-dihydroquinoline-3-acetonitrile | 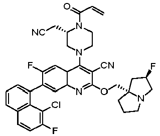 | 687 |
| 771 | 7-(8-chloro-7-fluoronaphthalen-1-yl)-4-((S)-3-(cyanomethyl)-4-(2-fluoroacryloyl)piperazin-1-yl)-6-fluoro-2-(((2R,7aS)-2-fluorotetrahydro-1H-pyrrolizin-7a(5H)-yl)methoxy)-4a,8a-dihydroquinoline-3-acetonitrile | 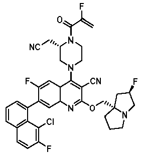 | 705 |
| 772 | 4-((1,5R)-2-acryloyl-2,6-diazabicyclo[3.2.0]hept-6-yl)-7-(8-chloro-7-fluoronaphthalen-1-yl)-6-fluoro-2-((((S)-1-methylpyrrolidin-2-yl)methoxy)quinoline-3-acetonitrile | 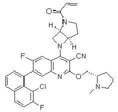 | 614 |
| 773 | 7-(8-chloro-7-fluoronaphthalen-1-yl)-6-fluoro-4-((1R,5R)-2-(2-fluoroacryloyl)-2,6-diazabicyclo[3.2.0]hept-6-yl)-2-((((S)-1-methylpyrrolidin-2-yl)methoxy)quinoline-3-acetonitrile | 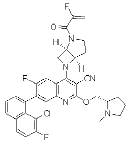 | 632 |
| 774 | 4-((1R,5R)-2-acryloyl-2,6-diazabicyclo[3.2.0]hept-6-yl)-7-(8-chloro-7-fluoronaphthalen-1-yl)-6-fluoro-2-((((2S,4R)-4-fluoro-1-methylpyrrolidin-2-yl)methoxy)quinoline-3-acetonitrile | 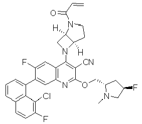 | 632 |
| 775 | 7-(8-chloro-7-fluoronaphthalen-1-yl)-6-fluoro-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)quinoline-3-acetonitrile | 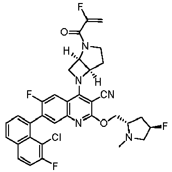 | 650 |
| 776 | 4-((1R,5R)-2-acryloyl-2,6-diazabicyclo[3.2.0]hept-6-yl)-7-(8-chloro-7-fluoronaphthalen-1-yl)-6-fluoro-2-((tetrahydro-1H-pyrrolizin-7a(5H)-yl)methoxy)quinoline-3-acetonitrile | 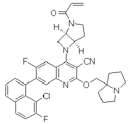 | 640 |
| 777 | 7-(8-chloro-7-fluoronaphthalen-1-yl)-6-fluoro-4-((1R,5R)-2-(2-fluoroacryloyl)-2,6-diazabicyclo[3.2.0]hept-6-yl)-2-(((tetrahydro-1H-pyrrolizin-7a(5H)-yl)methoxy)quinoline-3-acetonitrile | 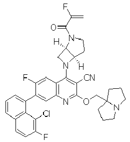 | 658 |
| 778 | 4-((1R,5R)-2-acryloyl-2,6-diazabicyclo[3.2.0]hept-6-yl)-7-(8-chloro-7-fluoronaphthalen-1-yl)-6-fluoro-2-((((2R,7aS)-2-fluorotetrahydro-1H-pyrrolizin-7a(5H)-yl)methoxy)quinoline-3-carbonitrile | 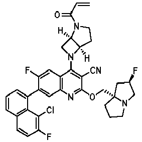 | 658 |
| 779 | 7-(8-chloro-7-fluoronaphthalen-1-yl)-6-fluoro-4-((1R,5R)-2-(2-fluoroacryloyl)-2,6-diazabicyclo[3.2.0]hept-6-yl)-2-(((((2R,7aS)-2-fluorotetrahydro-1H-pyrrolizin-7a(5H)-yl)methoxy)quinoline-3-acetonitrile | 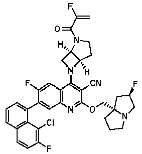 | 676 |
| 780 | 4-((S)-4-acryloyl-3-(cyanomethyl)piperazin-1-yl)-7-(8-chloronaphthalen-1-yl)-6-fluoro-2-((((S)-1-methylpyrrolidin-2-yl)methoxy)-4a,8a-dihydroquinoline-3-acetonitrile | 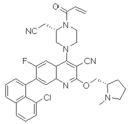 | 625 |
| 781 | 7-(8-chloronaphthalen-1-yl)-4-((S)-3-(cyanomethyl)-4-(2-fluoroacryloyl)piperazin-1-yl)-6-fluoro-2-(((S)-1- methylpyrrolidin-2-yl)methoxy)-4a,8a-dihydroquinoline-3-acetonitrile | 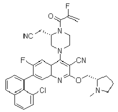 | 643 |
| 782 | 4-((S)-4-acryloyl-3-(cyanomethyl)piperazin-1-yl)-7-(8-chloronaphthalen-1-yl)-6-fluoro-2-((((2S,4R)-4-fluoro-1-methylpyrrolidin-2-yl)methoxy)-4a,8a-dihydroquinoline-3-acetonitrile | 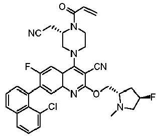 | 643 |
| 783 | 7-(8-chloronaphthalen-1-yl)-4-((S)-3-(cyanomethyl)-4-(2-fluoroacryloyl)piperazin-1-yl)-6-fluoro-2-((((4R)-4-fluoro-1-methylpyrrolidin-2-yl)methoxy)-4a,8a-dihydroquinoline-3-acetonitrile | 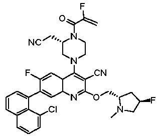 | 661 |
| 784 | 4-((S)-4-acryloyl-3-(cyanomethyl)piperazin-1-yl)-7-(8-chloronaphthalen-1-yl)-6-fluoro-2-(((tetrahydro-1H-pyrrolizin-7a(5H)-yl)methoxy)-4a,8a-dihydroquinoline-3-acetonitrile | 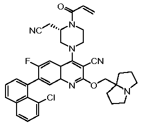 | 651 |
| 785 | 7-(8-chloronaphthalen-1-yl)-4-((S)-3-(cyanomethyl)-4-(2-fluoroacryloyl)piperazin-1-yl)-6-fluoro-2-((tetrahydro-1H-pyrrolizin-7a(5H)-yl)methoxy)-4a,8a-dihydroquinoline-3-acetonitrile | 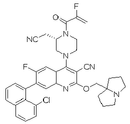 | 669 |
| 786 | 4-((S)-4-acryloyl-3-(cyanomethyl)piperazin-1-yl)-7-(8-chloronaphthalen-1-yl)-6-fluoro-2-((((2R,7aS)-2-fluorotetrahydro-1H-pyrrolizin-7a(5H)-yl)methoxy)-4a,8a-dihydroquinoline-3-acetonitrile | 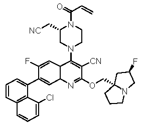 | 669 |
| 787 | 7-(8-chloronaphthalen-1-yl)-4-((S)-3-(cyanomethyl)-4-(2-fluoroacryloyl)piperazin-1-yl)-6-fluoro-2-((((2R,7aS)-2-fluorotetrahydro-1H-pyrrolizin-7a(5H)-yl)methoxy)-4a,8a-dihydroquinoline-3-acetonitrile | 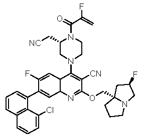 | 687 |
| 788 | 4-((1R,5R)-2-acryloyl-2,6-diazabicyclo[3.2.0]hept-6-yl)-7-(8-chloronaphthalen-1-yl)-6-fluoro-2-((((S)-1-methylpyrrolidin-2-yl)methoxy)quinoline-3-acetonitrile | 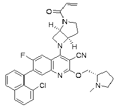 | 596 |
| 789 | 7-(8-chloronaphthalen-1-yl)-6-fluoro-4-((1R,SR)-2-(2-fluoroacryloyl)-2,6-diazabicyclo[3.2.0]hept-6-yl)-2-((((S)-1-methylpyrrolidin-2-yl)methoxy)quinoline-3-acetonitrile | 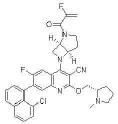 | 614 |
| 790 | 4-((1R,5R)-2-acryloyl-2,6-diazabicyclo[3.2.0]hept-6-yl)-7-(8-chloronaphthalen-1-yl)-6-fluoro-2-((((2S,4R)-4-fluoro-1-methylpyrrolidin-2-yl)methoxy)quinoline-3-acetonitrile | 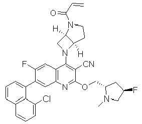 | 614 |
| 791 | 7-(8-chloronaphthalen-1-yl)-6-fluoro-2-((((2S,4R)-4-fluoro- 1-methylpyrrolidin-2-yl)methoxy)-4-((1R,SR)-2-(2-fluoroacryloyl)-2,6-diazabicyclo[3.2.0]hept-6-yl)quinoline-3-acetonitrile | 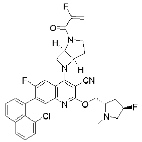 | 632 |
| 792 | 4-((1R,5R)-2-acryloyl-2,6-diazabicyclo[3.2.0]hept-6-yl)-7-(8-chloronaphthalen-1-yl)-6-fluoro-2-(((tetrahydro-1H-pyrrolizin-7a(5H)-yl)methoxy)quinoline-3-acetonitrile | 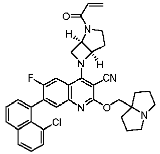 | 622 |
| 793 | 7-(8-chloronaphthalen-1-yl)-6-fluoro-4-((1R,SR)-2-(2-fluoroacryloyl)-2,6-diazabicyclo[3.2.0]hept-6-yl)-2-((tetrahydro-1H-pyrrolizin-7a(5H)-yl)methoxy)quinoline-3-acetonitrile | 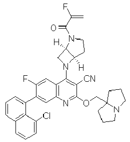 | 640 |
| 794 | 4-((1R,5R)-2-acryloyl-2,6-diazabicyclo[3.2.0]hept-6-yl)-7-(8-chloronaphthalen-1-yl)-6-fluoro-2-((((2R,7aS)-2-fluorotetrahydro-1H-pyrrolizin-7a(5H)-yl)methoxy)quinoline-3-acetonitrile | 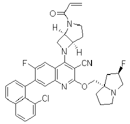 | 640 |
| 795 | 7-(8-chloronaphthalen-1-yl)-6-fluoro-4-((1R,SR)-2-(2-fluoroacryloyl)-2,6-diazabicyclo[3.2.0]hept-6-yl)-2-((((2R,7aS)-2-fluorotetrahydro-1H-pyrrolizin-7a(5H)-yl)methoxy)quinoline-3-acetonitrile | 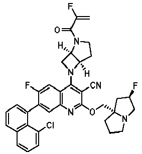 | 658 |
| 796 | 4-((S)-4-acryloyl-3-(cyanomethyl)piperazin-1-yl)-6-fluoro-2-((((S)-1-methylpyrrolidin-2-yl)methoxy)-7-(5,6,7,8-tetrahydronaphthalen-1-yl)-4a,8a-dihydroquinoline-3-acetonitrile | 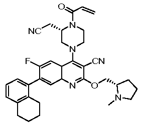 | 595 |
| 797 | 4-((S)-3-(cyanomethyl)-4-(2-fluoroacryloyl)piperazin-1-yl)-6-fluoro-2-(((S)-1-methylpyrrolidin-2-yl)methoxy)-7-(5,6,7,8-tetrahydronaphthalen-1-yl)-4a,8a-dihydroquinoline-3-acetonitrile | 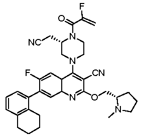 | 613 |
| 798 | 4-((S)-4-acryloyl-3-(cyanomethyl)piperazin-1-yl)-6-fluoro-2-((((2S,4R)-4-fluoro-1-methylpyrrolidin-2-yl)methoxy)-7-(5,6,7,8-tetrahydronaphthalen-1-yl)-4a,8a-dihydroquinoline-3-acetonitrile | 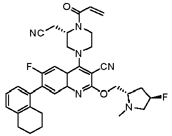 | 613 |
| 799 | 4-((S)-3-(cyanomethyl)-4-(2-fluoroacryloyl)piperazin-1-yl)-6-fluoro-2-((((2S,4R)-4-fluoro-1-methylpyrrol-2-yl)methoxy)-7-(5,6,7,8-tetrahydronaphthalen-1-yl)-4a,8a-dihydroquinoline-3-acetonitrile | 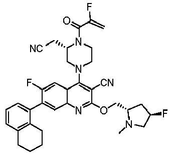 | 631 |
| 800 | 4-((S)-4-acryloyl-3-(cyanomethyl)piperazin-1-yl)-6-fluoro-2-(((tetrahydro-1H-pyrrolizin-7a(5H)-yl)methoxy)-7-(5,6,7,8-tetrahydronaphthalen-1-yl)-4a,8a-dihydroquinoline-3-acetonitrile | 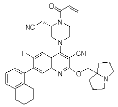 | 621 |
| 801 | 4-((S)-3-(cyanomethyl)-4-(2-fluoroacryloyl)piperazin-1-yl)-6-fluoro-2-((tetrahydro-1H-pyrrolizin-7a(5H)-yl)methoxy)-7-(5,6,7,8-tetrahydronaphthalen-1-yl)-4a,8a-dihydroquinoline-3-acetonitrile | 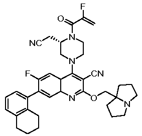 | 639 |
| 802 | 4-((S)-4-acryloyl-3-(cyanomethyl)piperazin-1-yl)-6-fluoro-2-((((2R,7aS)-2-fluorotetrahydro-1H-pyrrolidin-7a(5H)-yl))methoxy)-7-(5,6,7,8-tetrahydronaphthalen-1-yl)-4a,8a-dihydroquinoline-3-acetonitrile | 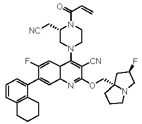 | 639 |
| 803 | 4-((S)-3-(cyanomethyl)-4-(2-fluoroacryloyl)piperazin-1-yl)-6-fluoro-2-((((2R,7aS)-2-fluorotetrahydro-1H-pyrrolizin-7a(5H)-yl)methoxy)-7-(5,6,7,8-tetrahydronaphthalen-1-yl)-4a,8a-dihydroquinoline-3-acetonitrile | 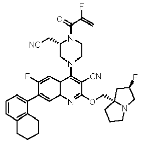 | 657 |
| 804 | 4-((1R,5R)-2-acryloyl-2,6-diazabicyclo[3.2.0]hept-6-yl)-6-fluoro-2-(((S)-1-methylpyrrolidin-2-yl)methoxy)-7-(5,6,7,8-tetrahydronaphthalen-1-yl)quinoline-3-acetonitrile | 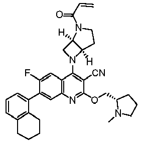 | 566 |
| 805 | 6-fluoro-4-((1R,SR)-2-(2-fluoroacryloyl)-2,6-diazabicyclo[3.2.0]hept-6-yl)-2-((((S)-1-methylpyrrolidin-2-yl)methoxy)-7-(5,6,7,8-tetrahydronaphthalen-1-yl)quinoline-3-acetonitrile | 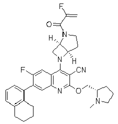 | 584 |
| 806 | 4-(((1R,5R)-2-acryloyl-2,6-diazabicyclo[3.2.0]hept-6-yl)-6-fluoro-2-(((((2S,4R)-4-fluoro-1-methylpyrrolidin-2-yl)methoxy)-7-(5,6,7,8-tetrahydronaphthalen-1-yl)quinoline-3-acetonitrile | 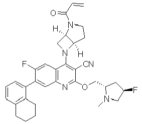 | 584 |
| 807 | 6-fluoro-2-((((2S,4R)-4-fluoro-1-methylpyrrolidin-2-yl)methoxy)-4-((1R,5R)-2-(2-fluoroacryloyl)-2,6-diazabicyclo[3.2.0]hept-6-yl)-7-(5,6,7,8-tetrahydronaphthalen-1-yl)quinoline-3-acetonitrile | 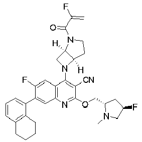 | 602 |
| 808 | 4-((1R,5R)-2-acryloyl-2,6-diazabicyclo[3.2.0]hept-6-yl)-6-fluoro-2-((tetrahydro-1H-pyrrolizin-7a(5H)-yl)methoxy)-7-(5,6,7,8-tetrahydronaphthalen-1-yl)quinoline-3-acetonitrile | 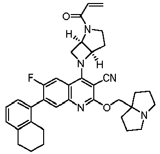 | 592 |
| 809 | 6-fluoro-4-((1R,SR)-2-(2-fluoroacryloyl)-2,6-diazabicyclo[3.2.0]hept-6-yl)-2-((tetrahydro-1H-pyrrolidon-7a(5H)-yl)methoxy)-7-(5,6,7,8-tetrahydronaphthalen-1-yl)quinoline-3-acetonitrile | 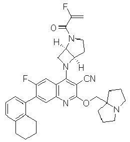 | 610 |
| 810 | 4-(((1R,5R)-2-acryloyl-2,6-diazabicyclo[3.2.0]hept-6-yl)-6-fluoro-2-(((((2R,7aS)-2-fluorotetrahydro-1H-pyrrolizin-7a(5H)-yl)methoxy)-7-(5,6,7,8-tetrahydronaphthalen-1-yl)quinoline-3-acetonitrile | 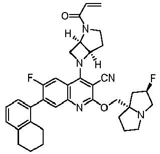 | 610 |
| 811 | 6-fluoro-4-((1R,SR)-2-(2-fluoroacryloyl)-2,6-diazabicyclo[3.2.0]hept-6-6yl)-2-((((2R,7aS)-2-fluorotetrahydro-1H-pyrrolizin-7a(5H)-yl)methoxy)-7-(5,6,7,8-tetrahydronaphthalen-1-yl)quinoline-3-acetonitrile | 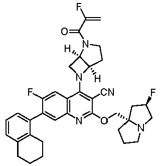 | 628 |
| 812 | 4-((1R,5R)-2-acryloyl-2,6-diazabicyclo[3.2.0]hept-6-yl)-6-fluoro-2-((tetrahydro-1H-pyrrolizin-7a(5H)-yl)methoxy)-7-(5,6,7,8-tetrahydroisoquinolin-4-yl)quinoline-3-acetonitrile | 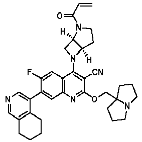 | 593 |
| 813 | 4-((1R,5R)-2-acryloyl-2,6-diazabicyclo[3.2.0]hept-6-yl)-6-fluoro-2-(((S)-1-methylpyrrolidin-2-yl)methoxy)-7-(5,6,7,8-tetrahydroisoquinolin-4-yl)quinoline-3-acetonitrile | 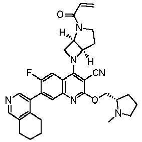 | 567 |
| 814 | 4-((S)-4-acryloyl-3-(cyanomethyl)piperazin-1-yl)-6-fluoro-2-(((tetrahydro-1H-pyrrolizin-7a(5H)-yl)methoxy)-7-(5,6,7,8-tetrahydroisoquinolin-4-yl)-4a,8a-dihydroquinoline-3-acetonitrile | 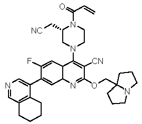 | 622 |
| 815 | 4-((S)-4-acryloyl-3-(cyanomethyl)piperazin-1-yl)-6-fluoro-2-((((S)-1-methylpyrrolidin-2-yl)methoxy)-7-(5,6,7,8-tetrahydroisoquinolin-4-yl)-4a,8a-dihydroquinoline-3-acetonitrile | 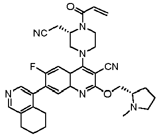 | 596 |
| 816 | 4-((1R,5R)-2-acryloyl-2,6-diazabicyclo[3.2.0]hept-6-yl)-7-(5-chloroisoquinolin-4-yl)-6-fluoro-2-((tetrahydro-1H-pyrrolizin-7a(5H)-yl)methoxy)quinoline-3-acetonitrile | 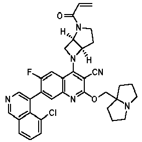 | 623 |
| 817 | 4-((1R,5R)-2-acryloyl-2,6-diazabicyclo[3.2.0]hept-6-yl)-7-(5-chloroisoquinolin-4-yl)-6-fluoro-2-(((S)-1-methylpyrrolidin-2-yl)methoxy)quinoline-3-acetonitrile | 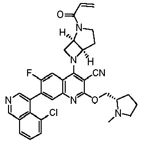 | 597 |
| 818 | 4-((S)-4-acryloyl-3-(cyanomethyl)piperazin-1-yl)-7-(5-chloroisoquinolin-4-yl)-6-fluoro-2-(((tetrahydro-1H-pyrrolizin-7a(5H)-yl)methoxy)-4a,8a-dihydroquinoline-3-acetonitrile | 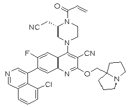 | 652 |
| 819 | 4-((S)-4-acryloyl-3-(cyanomethyl)piperazin-1-yl)-7-(5-chloroisoquinolin-4-yl)-6-fluoro-2-((((S)-1-methylpyrrolidin-2-yl)methoxy)-4a,8a-dihydroquinoline-3-acetonitrile | 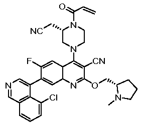 | 626 |
| 820 | 4-((S)-4-acryloyl-3-(cyanomethyl)piperazin-1-yl)-2-(((S)-1-methylpyrrolidin-2-yl)methoxy)-7-(1,1a,6,6a-tetrahydrocyclopropa[a]inden-2-yl)quinoline-3-acetonitrile | 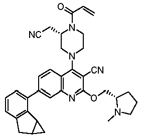 | 573.3 |
| 821 | 4-((S)-4-acryloyl-3-(cyanomethyl)piperazin-1-yl)-2-(((S)-1-methylpyrrolidin-2-yl)methoxy)-7-(thiochroman-8-yl)quinoline-3-acetonitrile | 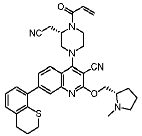 | 593.3 |
| 822 | 4-((S)-4-acryloyl-3-(cyanomethyl)piperazin-1-yl)-7-(benzothien-7-yl)-2-((((S)-1-methylpyrrolidin-2-yl)methoxy)quinoline-3-acetonitrile | 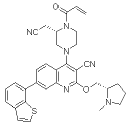 | 577.2 |
| 823 | 7-(benzothien-4-yl)-4-((S)-3-(cyanomethyl)-4-(2-fluoroacryloyl)piperazin-1-yl)-2-(((S)-1-methylpyrrolidin-2-yl)methoxy)quinoline-3-acetonitrile | 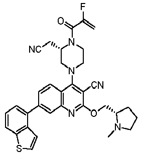 | 595.2 |
| 824 | 4-((S)-4-acryloyl-3-(cyanomethyl)piperazin-1-yl)-6-chloro-2-((S)-1-methylpyrrolidin-2-yl)methoxy)-7-(1,1a,6,6a-tetrahydrocyclopropa[a]inden-2-yl)quinoline-3-acetonitrile | 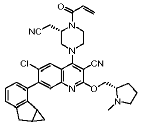 | 607.3 |
| 825 | 4-((S)-4-acryloyl-3-(cyanomethyl)piperazin-1-yl)-6-chloro-2-((((S)-1-methylpyrrolidin-2-yl)methoxy)-7-(thiochroman-8-yl)quinoline-3-acetonitrile | 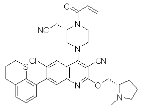 | 627.2 |
| 826 | 4-((S)-4-acryloyl-3-(cyanomethyl)piperazin-1-yl)-7-(benzo[b]thien-7-yl)-6-chloro-2-((((S)-1-methylpyrrolidin-2-yl)methoxy)quinoline-3-acetonitrile | 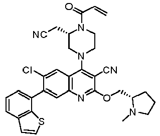 | 611.2 |
| 827 | 7-(benzothien-4-yl)-6-chloro-4-((S)-3-(cyanomethyl)-4-(2-fluoroacryloyl)piperazin-1-yl)-2-(((S)-1-methylpyrrolidin-2-yl)methoxy)quinoline-3-acetonitrile | 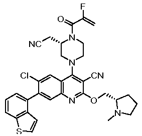 | 629.2 |
| 828 | 4-((S)-4-acryloyl-3-(cyanomethyl)piperazin-1-yl)-8-fluoro-2-(((S)-1-methylpyrrolidin-2-yl)methoxy)-7-(1,1a,6,6a-tetrahydrocyclopropa[a]inden-2-yl)quinoline-3-acetonitrile | 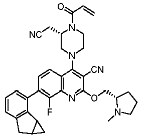 | 591.3 |
| 829 | 4-((S)-4-acryloyl-3-(cyanomethyl)piperazin-1-yl)-8-fluoro-2-((((S)-1-methylpyrrolidin-2-yl)methoxy)-7-(thiochroman-8-yl)quinoline-3-carbonitrile | 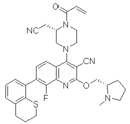 | 611.3 |
| 830 | 4-((S)-4-acryloyl-3-(cyanomethyl)piperazin-1-yl)-7-(benzo[b]thien-7-yl)-8-fluoro-2-((((S)-1-methylpyrrolidin-2-yl)methoxy)quinoline-3-carbonitrile | 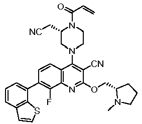 | 595.2 |
| 831 | 7-(benzothien-4-yl)-4-((S)-3-(cyanomethyl)-4-(2-fluoroacryloyl)piperazin-1-yl)-8-fluoro-2-((((S)-1-methylpyrrolidin-2-yl)methoxy)quinoline-3-carbonitrile | 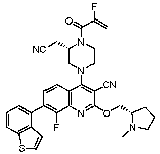 | 613.2 |
| 832 | 4-((S)-4-acryloyl-3-(cyanomethyl)piperazin-1-yl)-6-fluoro-2-(((S)-1-methylpyrrolidin-2-yl)methoxy)-7-(1,1a,6,6a-tetrahydrocyclopropa[a]inden-2-yl)quinoline-3-carbonitrile | 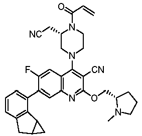 | 591.3 |
| 833 | 4-((S)-4-acryloyl-3-(cyanomethyl)piperazin-1-yl)-6-fluoro-2-((((S)-1-methylpyrrolidin-2-yl)methoxy)-7-(thiochroman-8-yl)quinoline-3-acetonitrile | 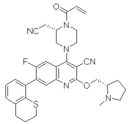 | 611.3 |
| 834 | 4-((S)-4-acryloyl-3-(cyanomethyl)piperazin-1-yl)-7-(benzo[b]thien-7-yl)-6-fluoro-2-(((S)-1-methylpyrrolidin-2-yl)methoxy)quinoline-3-acetonitrile | 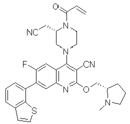 | 595.2 |
| 835 | 7-(benzothien-4-yl)-4-((S)-3-(cyanomethyl)-4-(2-fluoroacryloyl)piperazin-1-yl)-6-fluoro-2-((((S)-1-methylpyrrolidin-2-yl)methoxy)quinoline-3-acetonitrile | 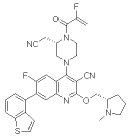 | 613.2 |
| 836 | 4-((S)-4-acryloyl-3-(cyanomethyl)piperazin-1-yl)-8-fluoro-2-(((S)-1-methylpyrrolidin-2-yl)methoxy)-7-(1,1a,6,6a-tetrahydrocyclopropa[a]inden-2-yl)-1,6-naphthyridine-3-acetonitrile | 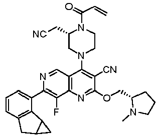 | 592.3 |
| 837 | 4-((S)-3-(cyanomethyl)-4-(2-fluoroacryloyl)piperazin-1-yl)-8-fluoro-2-(((S)-1-methylpyrrolidin-2-yl)methoxy)-7-(thiochroman-8-yl)-1,6-naphthyridine-3-acetonitrile | 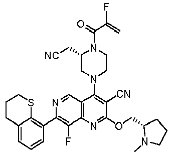 | 630.2 |
| 838 | 4-((S)-4-acryloyl-3-(cyanomethyl)piperazin-1-yl)-7-(benzo[b]thien-7-yl)-8-fluoro-2-((((S)-1-methylpyrrolidin-2-yl)methoxy)-1,6-naphthyridine-3-acetonitrile | 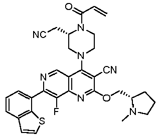 | 596.2 |
| 839 | 7-(benzothien-4-yl)-4-((S)-3-(cyanomethyl)-4-(2-fluoroacryloyl)piperazin-1-yl)-8-fluoro-2-((((S)-1-methylpyrrolidin-2-yl)methoxy)-1,6-naphthyridine-3-acetonitrile | 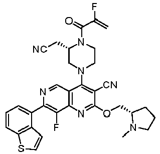 | 614.2 |

### Example 840: 2-((S)-1-acryloyl-4-(7-(8-chloro-7-fluoronaphthalen-1-yl)-2-(((S)-1-methylpyrrolidin-2-yl)methoxy)pyridino[3,2-d]pyrimidin-4-yl)piperazin-2-yl)acetonitrile

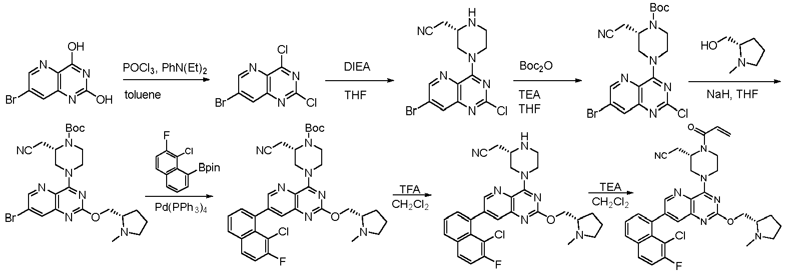

### Step 1: Synthesis of 7-bromo-2,4-dichloropyridino[3,2-d]pyrimidine

N,N-diethylaniline (1.41 g, 9.45 mmol) was added to a mixture of 7-bromopyridino[3,2-d]pyrimidine-2,4-diphenol (1.04 g, 4.3 mmol) and phosphorus oxychloride (10 mL) at room temperature. The resulting light yellow suspension was stirred for 5 min, and then heated and stirred in a 110°C oil bath for 16 h. After cooling to room temperature, concentration under reduced pressure was performed. The residue was mixed with toluene (20 mL × 2) and subjected to concentration under reduced pressure. The resulting light brown solid was directly used for the next step of reaction.

### Step 2: Synthesis of (S)-2-(4-(7-bromo-2-chloropyridino[3,2-d]pyrimidin-4-yl)piperazin-2-yl)acetonitrile

N,N-diisopropylethylamine (4.27 mL, 25.8 mmol) was added to a mixture of 7-bromo-2,4-dichloropyridino[3,2-d]pyrimidine and tetrahydrofuran (30 mL) in one portion in an ice-water bath. After 2 minutes of stirring, (S)-2-(piperazin-2-yl)acetonitrile hydrochloride (0.85 g, 4.3 mmol) was added. The reaction liquid was gradually warmed to room temperature and stirred for 5 h. The reaction liquid was directly used for the next step of reaction without treatment.

### Step 3: Synthesis of tert-butyl (S)-4-(7-bromo-2-chloropyridino[3,2-d]pyrimidin-4-yl)-2-(cyanomethyl)piperazine-1-carboxylate

At room temperature, triethylamine (0.6 mL, 4.3 mmol) and di-tert-butyl dicarbonate (1.31 g, 6 mmol) were added to the reaction liquid from the previous step. The reaction liquid was stirred at room temperature for 17 h. Ethyl acetate (100 mL) and water (100 mL) were added for extraction and separation. The aqueous phase was extracted with ethyl acetate (100 mL). The organic phases were combined, washed with a saturated aqueous NaCl solution (50 mL), dried with sodium sulfate and concentrated under reduced pressure to obtain a dark brown viscous material. Purification by column chromatography (silica gel, ethyl acetate : petroleum ether = 1 : 20 to ethyl acetate : petroleum ether = 1 : 5) gave a yellow solid. (210 mg, 0.449 mmol, overall yield over three steps: 10%). MS m/z: 467.4 [M+H]⁺.

### Step 4: Synthesis of tert-butyl (S)-4-(7-bromo-2-((((S)-1-methylpyrrolidin-2-yl)methoxy)pyridino[3,2-d]pyrimidin-4-yl)-2-(cyanomethyl)piperazine-1-carboxylate

dissolved in (S)-(1-methylpyrrolidin-2-yl)methanol (77 mg, 0.67 mmol) tetrahydrofuran (2 mL), sodium hydride (16 mg, 0.67 mmol) was added at 0°C, and the mixture was reacted at room temperature for 0.5 h. A solution of the compound tert-butyl (S)-4-(7-bromo-2-chloropyridino[3,2-d]pyrimidin-4-yl)-2-(cyanomethyl)piperazine-1-carboxylate (100 mg, 0.22 mmol) in tetrahydrofuran (2 mL) was then added and the reaction continued at room temperature for 1 h. The mixed reaction liquid was concentrated under reduced pressure and purified by PLC to obtain the compound tert-butyl (S)-4-(7-bromo-2-((((S)-1-methylpyrrolidin-2-yl)methoxy)pyridino[3,2-d]pyrimidin-4-yl)-2-(cyanomethyl)piperazine-1-carboxylate (82 mg, 67%). MS m/z: [M+H]⁺ = 546.6.

### Step 5: Synthesis of tert-butyl (S)-4-(7-(8-chloro-7-fluoronaphthalen-1-yl)-2-((((S)-1-methylpyrrolidin-2-yl)methoxy)pyridino [3,2-d]pyrimidin-4-yl)-2-(cyanomethyl)piperazine-1-carboxylate

The compound tert-butyl (S)-4-(7-bromo-2-((((S)-1-methylpyrrolidin-2-yl)methoxy)pyridino[3,2-d]pyrimidin-4-yl)-2-(cyanomethyl)piperazine-1-carboxylate (50 mg, 0.091 mmol) and 2-(8-chloro-7-fluoronaphthalen-1-yl)-4,4,5,5-tetramethyl-1,3,2-dioxaborane (56.1 mg, 0.18 mmol) were dissolved in 1,4-dioxane (2.0 mL). Pd(PPh₃)₄ (31.7 mg, 0.027 mmol), cesium carbonate (89.4 mg, 0.27 mmol) and water (0.1 mL) were added in order in a nitrogen atmosphere, and the mixture was then heated to 100°C and reacted at this temperature under stirring for 5 h. The mixed reaction liquid was concentrated under reduced pressure and purified by PLC to obtain the compound tert-butyl (S)-4-(7-(8-chloro-7-fluoronaphthalen-1-yl)-2-((((S)-1-methylpyrrolidin-2-yl)methoxy)pyridino[3,2-d]pyrimidin-4-yl)-2-(cyanomethyl)piperazine-1-carboxylate (35.5 mg, 60%). MS m/z: [M+H]⁺=646.7.

### Step 6: Synthesis of 2-((S)-4-(7-(8-chloro-7-fluoronaphthalen-1-yl)-2-(((S)-1-methylpyrrolidin-2-yl)methoxy)pyridinyl[3,2-d]pyrimidin-4-yl)piperazin-2-yl)acetonitrile

The compound tert-butyl (S)-4-(7-(8-chloro-7-fluoronaphthalen-1-yl)-2-((((S)-1-methylpyrrolidin-2-yl)methoxy)pyridino[3,2-d]pyrimidin-4-yl)-2-(cyanomethyl)piperazine-1-carboxylate (34 mg, 0.053 mmol) was dissolved in CH₂Cl₂ (1.5 mL), and TFA (0.5 mL) was added dropwise. The reaction liquid was stirred at room temperature until the reaction was complete. Subsequently, the reaction liquid was adjusted to pH = 10 with 10% NaOH aqueous solution, separated, extracted with CH₂Cl₂, washed with a saturated aqueous NaCl solution, dried (Na₂SO₄), filtered and concentrated under reduced pressure to obtain the compound 2-((S)-4-(7-(8-chloro-7-fluoronaphthalen-1-yl)-2-(((S)-1-methylpyrrolidin-2-yl)methoxy)pyridinyl[3,2-d]pyrimidin-4-yl)piperazin-2-yl)acetonitrile (24.3 mg, 84%). MS m/z: [M+H]⁺ = 546.6.

### Step 7: Synthesis of 2-((S)-1-acryloyl-4-(7-(8-chloro-7-fluoronaphthalen-1-yl)-2-(((S)-1-methylpyrrolidin-2-yl)methoxy)pyridino[3,2-d]pyrimidin-4-yl)piperazin-2-yl)acetonitrile

The compound 2-((S)-4-(7-(8-chloro-7-fluoronaphthalen-1-yl)-2-(((S)-1-methylpyrrolidin-2-yl)methoxy)pyridino[3,2-d]pyrimidin-4-yl)piperazin-2-yl)acetonitrile (23 mg, 0.042 mmol) and triethylamine (13 mg, 0.13 mmol) were dissolved in CH₂Cl₂ (1.0 mL), acryloyl chloride (7.6 mg, 0.08 mmol) was then added dropwise to the solution, and the mixture was stirred at room temperature for 1 h. The reaction liquid was concentrated under pressurized condition and purified by PLC to obtain the compound 2-((S)-1 -acryloyl-4-(7-(8-chloro-7-fluoronaphthalen-1 -yl)-2-(((S)-1 -methylpyrrolidin-2-yl)methoxy)pyridino[3,2-d]pyrimidin-4-yl)piperazin-2-yl)acetonitrile (24 mg, 94%). ¹H NMR (400 MHz, CDCl₃) δ 8.59 (s, 1H), 7.98 (d, *J* = 8.0 Hz, 1H), 7.91 (dd, *J* = 8.9, 5.5 Hz, 2H), 7.56 (dd, *J* = 15.5, 7.4 Hz, 1H), 7.50 - 7.38 (m, 2H), 7.05 - 6.81 (m, 1H), 6.45 - 6.30 (m, 1H), 5.81 (dd, *J* = 21.5, 10.7 Hz, 1H), 4.76 - 4.68 (m, 1H), 4.43 (s, 1H), 4.25 - 3.28 (m, 5H), 3.05 - 2.91 (m, 4H), 2.49 - 1.87 (m, 10H). MS m/z: [M+H]⁺=600.6

The compounds of Examples 841-853 were prepared by the preparation method for Example 840

| Ex. | Compound name | Structural formula | m/z: ES⁺[M+H] |
|---|---|---|---|
| 841 | 2-((S)-4-(7-(8-chloro-7-fluoronaphthalen-1-yl)-2-((S)-1-methylpyrrolidin-2-yl)methoxy)pyridino[3,2-d]pyrimidin-4-yl)-1-(2-fluoroacryloyl)piperazin-2-yl)acetonitrile | 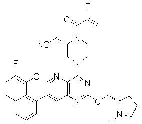 | 618.2 |
| 842 | 2-((S)-1-acryloyl-4-(7-(8-chloronaphthalen-1-yl)-2-((S)-1-methylpyrrolidin-2-yl)methoxy)pyridino[3,2-d]pyrimidin-4-yl)piperazin-2-yl)acetonitrile | 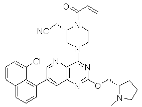 | 582.2 |
| 843 | 2-((S)-4-(7-(8-chloronaphthalen-1-yl)-2-(((S)-1-methylpyrrolidin-2-yl)methoxy)pyridino [3,2-d]pyrimidin-4-yl)-1-(2-fluoroacryloyl)piperazin-2-yl)acetonitrile | 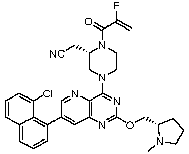 | 600.2 |
| 844 | 2-((S)-1-acryloyl-4-(2-(((S)-1-methylpyrrolidin-2-yl)methoxy)-7-(5,6,7,8-tetrahydronaphthalen-1-yl)pyridino[3,2-d]pyrimidin-4-yl)piperazin-2-yl)acetonitrile | 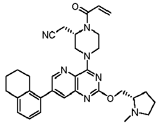 | 552.3 |
| 845 | 2-((S)-1-(2-fluoroacryloyl)-4-(2-((S)-1-methylpyrrolidin-2-yl)methoxy)-7-(5,6,7,8-tetrahydronaphthalen-1-yl)pyridino[3,2-d]pyrimidin-4-yl)piperazin-2-yl)acetonitrile | 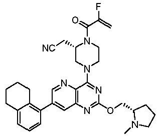 | 570.3 |
| 846 | 2-((2S)-1 -acryloyl-4-(2-(((S)-1 -methylpyrrolidin-2-yl)methoxy)-7-(1,1a,6,6a-tetrahydrocyclopropa[a]inden-2-yl)pyridino[3,2-d]pyrimidin-4-yl)piperazin-2-yl)acetonitrile | 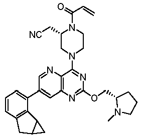 | 550.3 |
| 847 | 2-((2S)-1-(2-fluoroacryloyl)-4-(2-(((S)-1-methylpyrrolidin-2-yl)methoxy)-7-(1,1a,6,6a-tetrahydrocyclopropa[a]inden-2-yl)pyridino[3,2-d]pyrimidin-4-yl)piperazin-2-yl)acetonitrile | 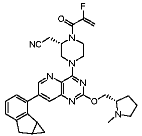 | 568.3 |
| 848 | 2-((2S)-1-acryloyl-4-(2-((tetrahydro-1H-pyrrolizin-7a(5H)-yl)methoxy)-7-(1,1a,6,6a-tetrahydrocyclopropa[a]inden-2-yl)pyridino[3,2-d] pyrimidin-4-yl)piperazin-2-yl)acetonitrile | 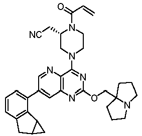 | 576.3 |
| 849 | 2-((2S)-1-(2-fluoroacryloyl)-4-(2-((tetrahydro-1H-pyrrolizin-7a(5H)-yl)methoxy)-7-(1,1a,6,6a-tetrahydrocyclopropa[a]inden-2-yl)pyridino[3,2-d] pyrimidin-4-yl)piperazin-2-yl)acetonitrile | 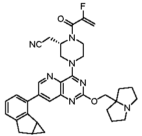 | 594.3 |
| 850 | (S)-2-(1-acryloyl-4-(7-(8-chloro-7-fluoronaphthalen-1-yl)-2-((tetrahydro-1H-pyrrolizin-7a(5H)-yl)methoxy)pyridino[3,2-d]pyrimidin-4-yl)piperazin-2-yl)acetonitrile | 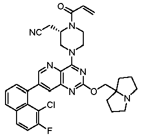 | 626.2 |
| 851 | (S)-2-(4-(7-(8-chloro-7-fluoronaphthalen-1-yl)-2-((tetrahydro-1H-pyrrolizin-7a(5H)-yl)methoxy)pyridino[3,2-d]pyrimidin-4-yl)-1-(2-fluoroacryloyl)piperazin-2-yl)acetonitrile | 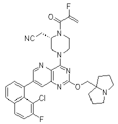 | 644.2 |
| 852 | 2-((2S)-4-(7-(benzo[b]thien-4-yl)-2-((2R)-2-fluorotetrahydro-1H-pyrrolizin-7a(5H)-yl)methoxy)pyridino[3,2-d]pyrimidin-4-yl)-1-(2-fluoroacryloyl)piperazin-2-yl)acetonitrile | 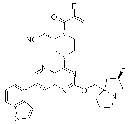 | 616.2 |
| 853 | 2-((2S)-4-(7-(benzo[b]thien-7-yl)-2-((2R)-2-fluorotetrahydro-1H-pyrrolizin-7a(5H)-yl)methoxy)pyridino[3,2-d]pyrimidin-4-yl)-1-(2-fluoroacryloyl)piperazin-2-yl)acetonitrile | 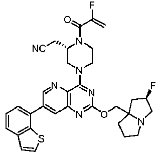 | 616.2 |

### Example 854: Synthesis of (S)-2-(1-acryloyl-4-(7-(8-chloronaphthalen-1-yl)-8-fluoro-2-((1-(pyrrolidin-1-ylmethyl)cyclopropyl)methoxy)pyridino[4.3-d]pyrimidin-4-yl)piperazin-2-yl)acetonitrile

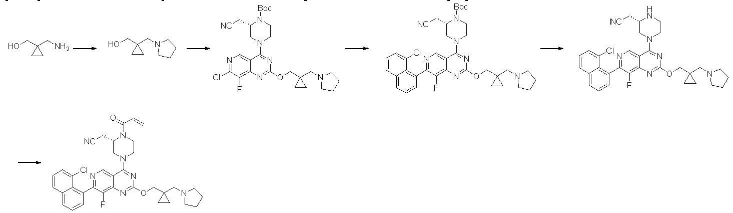

### Step 1: Synthesis of (1-(pyrrolidin-1-ylmethyl)cyclopropyl)methanol

The compound (1-(aminomethyl)cyclopropyl)methanol (500 mg, 4.94 mmol) and 1,4-dibromobutane (1.12 g, 5.19 mmol) were dissolved in acetonitrile (20 mL), potassium carbonate (1.78 g, 12.85 mmol) was then added, and the mixture was reacted at room temperature under stirring for 12 h. After the reaction was complete, filtration was carried out to remove a solid. The organic phase was concentrated and separated by column chromatography (DCM/7M NH3 in MeOH = 100/1) to obtain a colorless oil. (390 mg, yield: 50.8%). ¹H NMR (400 MHz, CDCl₃) δ 3.55 (s, 2H), 2.70 - 2.53 (m, 6H), 1.83 - 1.69 (m, 4H), 0.49 (q, *J*= 4.6 Hz, 2H), 0.36 (t, *J* = 5.2 Hz, 2H).

### Step 2: Synthesis of tert-butyl (S)-4-(7-chloro-8-fluoro-2-((1-(pyrrolidin-1-ylmethyl)cyclopropyl)methoxy)pyridino[4,3-d]pyrimidin-4-yl)-2-(cyanomethyl)piperazine-1-carboxylate

The compound tert-butyl (2S)-4-(7-chloro-8-fluoro-2-(methylsulfinyl)pyridino[4,3-d]pyrimidin-4-yl-2-(cyanomethyl)piperazine-1-carboxylate (100 mg, 0.21 mmol) and (1-(pyrrolidin-1-ylmethyl)cyclopropyl)methanol (37 mg, 0.23 mmol) were dissolved in anhydrous toluene (3 mL), sodium tert-butoxide (25 mg, 0.25 mmol) was added under ice-water bath cooling condition, and the mixture was reacted under stirring for 30 min. After the reaction liquid was complete, the reaction was quenched with cold water and extracted with ethyl acetate, and the organic phase was dried with anhydrous sodium sulfate, concentrated, separated and purified by prep-TLC to obtain an off-white solid. (50 mg, yield: 42%). ¹H NMR (400 MHz, CDCl₃) δ 8.78 (s, 1H), 4.62 (s, 1H), 4.42 (q, *J* = 10.9 Hz, 3H), 4.29 (d, *J* = 12.8 Hz, 1H), 4.08 (s, 1H), 3.88 (s, 1H), 3.66 (s, 1H), 3.46 (s, 1H), 2.87 - 2.77 (m, 1H), 2.73 (d, *J* = 5.7 Hz, 1H), 2.53 (s, 6H), 1.74 (s, 4H), 1.51 (s, 9H), 0.68 (s, 2H), 0.51 (s, 2H).

### Step 3: Synthesis of tert-butyl (S)-4-(7-(8-chloronaphthalen-1-vl)-8-tluoro-2-((1-(pvrrolidin-1-ylmethyl)cyclopropyl)methoxy)pyridino[4,3-d]pyrimidin-4-yl)-2-(cyanomethyl)piperazine-1-carboxylate

The compound tert-butyl (S)-4-(7-chloro-8-fluoro-2-((1-(pyrrolidin-1-ylmethyl)cyclopropyl)methoxy)pyridino[4,3-d]pyrimidin-4-yl)-2-(cyanomethyl)piperazine-1-carboxylate (50 mg, 0.09 mmol), 2-(8-chloronaphthalen-1-yl)-4,4,5,5-tetramethyl-1,3,2-dioxaborane (34 mg, 0.12 mmol) and cesium carbonate (87 mg, 0.27 mmol) were dissolved in 1,4-dioxane/water = 5/1 (3 mL), Pd(PPh₃)₄ (52 mg, 0.04 mmol) was then added, and after displacement with nitrogen three times, the mixture was heated to 100°C and reacted under stirring for 2 h. After the reaction was complete, the reaction liquid was cooled to room temperature, diluted with water and extracted with ethyl acetate, and the organic phase was dried with anhydrous sodium sulfate, concentrated, separated and purified by prep-TLC to obtain an off-white solid. (20 mg, yield: 33%).

### Step 4: Synthesis of (S)-2-(4-(7-(8-chloronaphthalen-1-yl)-8-fluoro-2-((1-(pyrrolidin-1-ylmethyl)cyclopropyl)methoxy)pyridino[4,3-d]pyrimidin-4-yl)piperazin-2-yl)acetonitrile

The compound tert-butyl (S)-4-(7-(8-chloronaphthalen-1-yl)-8-fluoro-2-((1-(pyrrolidin-1-ylmethyl)cyclopropyl)methoxy)pyridino[4,3-d]pyrimidin-4-yl)-2-(cyanomethyl)piperazine-1-carboxylate (20 mg, 0.03 mmol) was dissolved in dichloromethane (3 mL), trifluoroacetic acid (1 mL) was then added, and the mixture was reacted at room temperature under stirring for 30 min. After the reaction was complete, the reaction liquid was concentrated to obtain a crude product, which was directly used for the next step.

### Step 5: Synthesis of (S)-2-(1-acryloyl-4-(7-(8-chloronaphthalen-1-yl)-8-fluoro-2-((1-(pyrrolidin-1-ylmethyl)cyclopropyl)methoxy)pyridino[4,3-d]pyrimidin-4-yl)piperazin-2-yl)acetonitrile

The compound (S)-2-(4-(7-(8-chloronaphthalen-1-yl)-8-fluoro-2-((1-(pyrrolidin-1-ylmethyl)cyclopropyl)methoxy)pyridino[4,3-d]pyrimidin-4-yl)piperazin-2-yl)acetonitrile (17 mg, 0.03 mmol) was dissolved in dichloromethane (3 mL), DIEA (5 mg, 0.03 mmol) and acryloyl chloride (3 mg, 0.03 mmol) were added under ice-water bath cooling condition, and the mixture was reacted under stirring and ice-water bath cooling conditions for 5 min. After the reaction was complete, the reaction was quenched with a saturated sodium bicarbonate aqueous solution and extracted with dichloromethane, and the organic phase was dried with anhydrous sodium sulfate, concentrated, separated and purified by prep-TLC to obtain an off-white solid. (10 mg, yield: 50%). ¹H NMR (400 MHz, CDCl₃) δ 9.05 (s, 1H), 8.01 (dd, *J*= 7.9, 1.5 Hz, 1H), 7.88 (d, *J* = 8.1 Hz, 1H), 7.66 - 7.50 (m, 3H), 7.42 (td, *J* = 7.8, 2.4 Hz, 1H), 6.59 (s, 1H), 6.42 (d, *J* = 16.8 Hz, 1H), 5.85 (d, *J* = 10.8 Hz, 1H), 5.08 (s, 1H), 4.46 (d, *J* = 10.5 Hz, 4H), 3.90 (d, *J* = 103.6 Hz, 5H), 3.06 (s, 1H), 2.82 (d, *J* = 17.1 Hz, 2H), 2.55 (s, 4H), 1.78 (s, 4H), 0.73 (s, 2H), 0.57 (s, 2H). MS m/z: [M+H]⁺=640.59

### Example 855: Synthesis of (S)-2-(1-acryloyl-4-(7-(8-chloronaphthalen-1-yl)-8-fluoro-2-((1-(pyrrolidin-1-ylmethyl)cyclopropyl)methoxy)quinazolin-4-yl)piperazin-2-yl)acetonitrile

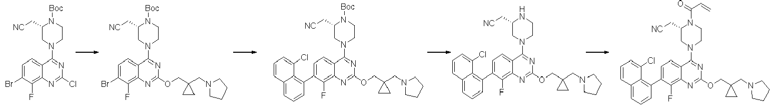

### Step 1: Synthesis of tert-butyl (S)-4-(7-bromo-8-fluoro-2-((1-(pyrrolidin-1-ylmethyl)cyclopropyl)methoxy)quinazolin-4-yl-2-(cyanomethyl)piperazine-1-carboxylate

The compound (1-(pyrrolidin-1-ylmethyl)cyclopropyl)methanol (84 mg, 0.54 mmol) was dissolved in anhydrous THF (5 mL), 60% NaH (20 mg, 0.50 mmol) was added under ice-water bath cooling condition, the mixture was reacted under stirring and ice-water bath cooling conditions for 20 min, the compound tert-butyl (S)-4-(7-bromo-2-chloro-8-fluoroquinazolin-4-yl)-2-(cyanomethyl)piperazine-1-carboxylate (200 mg, 0.41 mmol) was added, and the mixture was then reacted at room temperature under stirring for 8 h. After the reaction was complete, the reaction was quenched with cold water and extracted with ethyl acetate, and the organic phase was dried with anhydrous sodium sulfate, concentrated and separated by column chromatography to obtain a light yellow solid. (150 mg, yield: 61%). ¹H NMR (400 MHz, CDCl₃) δ 7.46 (d, *J* = 9.1 Hz, 1H), 7.38 (dd, *J* = 9.0, 6.0 Hz, 1H), 4.65 (s, 1H), 4.42 (s, 2H), 4.24 (d, *J* = 12.2 Hz, 1H), 4.16 (d, *J* = 10.8 Hz, 2H), 3.54 (dd, *J* = 13.8, 3.6 Hz, 1H), 3.32 (d, *J* = 10.4 Hz, 2H), 2.87 - 2.69 (m, 2H), 2.50 (s, 6H), 1.71 (s, 4H), 1.52 (s, 11H), 0.67 (s, 2H), 0.48 (s, 2H).

### Step 2: Synthesis of tert-butyl (S)-4-(7-(8-chloronaphthalene)-8-fluoro-2-((1-(pyrrolidin-1-ylmethyl)cyclopropyl)methoxy)quinazolin-4-yl)-2-(cyanomethyl)piperazine-1-carboxylate

The compound tert-butyl (S)-4-(7-bromo-8-fluoro-2-((1-(pyrrolidin-1-ylmethyl)cyclopropyl)methoxy)quinazolin-4-yl-2-(cyanomethyl)piperazine-1-carboxylate (130 mg, 0.22 mmol), 2-(8-chloronaphthalen-1-yl)-4,4,5,5-tetramethyl-1,3,2-dioxaborane (93 mg, 0.0.32 mmol) and potassium carbonate (60 mg, 0.43 mmol) were dissolved in 1,4-dioxane/water = 5/1 (3 mL), Pd(dppf)Cl₂ (31 mg, 0.04 mmol) was then added, and after displacement with nitrogen three times, the mixture was heated to 100°C and reacted under stirring for 2 h. After the reaction was complete, the reaction liquid was cooled to room temperature, diluted with water and extracted with ethyl acetate, and the organic phase was dried with anhydrous sodium sulfate, concentrated, separated and purified by prep-TLC to obtain an off-white solid. (80 mg, yield: 54.3%). ¹H NMR (400 MHz, CDCl₃) δ 7.97 (d, *J* = 8.1 Hz, 1H), 7.88 (d, *J* = 7.4 Hz, 1H), 7.54 (dd, *J* = 14.2, 4.9 Hz, 3H), 7.45 - 7.38 (m, 2H), 7.23 (d, *J* = 6.6 Hz, 1H), 4.70 (s, 1H), 4.36 (dd, *J* = 30.7, 19.9 Hz, 4H), 4.14 (s, 1H), 3.55 (d, *J* = 11.7 Hz, 1H), 3.36 (s, 2H), 2.87 (s, 2H), 2.51 (s, 4H), 1.71 (s, 4H), 1.53 (s, 9H), 0.67 (s, 2H), 0.47 (s, 2H).

### Step 3: Synthesis of (S)-2-(4-(7-(8-chloronaphthalen-1-yl)-8-fluoro-2-((1-(pyrrolidin-1-ylmethyl)cyclopropyl)methoxy)quinazolin-4-yl)piperazin-2-yl)acetonitrile

The compound tert-butyl (S)-4-(7-(8-chloronaphthalene)-8-fluoro-2-((1-(pyrrolidin-1-ylmethyl)cyclopropyl)methoxy)quinazolin-4-yl)-2-(cyanomethyl)piperazine-1-carboxylate (80 mg, 0.12 mmol) was dissolved in dichloromethane (3 mL), trifluoroacetic acid (1 mL) was then added, and the mixture was reacted at room temperature under stirring for 1 h. After the reaction was complete, the reaction liquid was concentrated to obtain a crude product, which was directly used for the next step.

### Step 4: Synthesis of (S)-2-(1-acryloyl-4-(7-(8-chloronaphthalen-1-yl)-8-fluoro-2-((1-(pyrrolidin-1-ylmethyl)cyclopropyl)methoxy)quinazolin-4-yl)piperazin-2-yl)acetonitrile

The compound (S)-2-(4-(7-(8-chloronaphthalen-1-yl)-8-fluoro-2-((1-(pyrrolidin-1-ylmethyl)cyclopropyl)methoxy)quinazolin-4-yl)piperazin-2-yl)acetonitrile (68 mg, 0.12 mmol) was dissolved in dichloromethane (10 mL), DIEA (18 mg, 0.14 mmol) and acryloyl chloride (12 mg, 0.13 mmol) were added under ice-water bath cooling condition, and the mixture was reacted under stirring and ice-water bath cooling conditions for 5 min. After the reaction was complete, the reaction was quenched with a saturated sodium bicarbonate aqueous solution and extracted with dichloromethane, and the organic phase was dried with anhydrous sodium sulfate, concentrated, separated and purified by prep-TLC to obtain an off-white solid. (42 mg, yield: 56%). ¹H NMR (400 MHz, CDCl₃) δ 7.97 (d, *J* = 7.3 Hz, 1H), 7.88 (d, *J* = 7.4 Hz, 1H), 7.68 - 7.60 (m, 1H), 7.55 (dd, *J* = 9.7, 8.0 Hz, 2H), 7.43 (dd, *J* = 7.8, 2.3 Hz, 2H), 7.26 (m, 1H), 6.61 (s, 1H), 6.42 (d, *J* = 16.8 Hz, 1H), 5.84 (d, *J* = 10.5 Hz, 1H), 5.16 (s, 1H), 4.54 - 4.26 (m, 4H), 4.02 (s, 1H), 3.53 (d, *J* = 89.6 Hz, 4H), 3.01 (s, 1H), 2.89 (d, *J* = 34.6 Hz, 1H), 2.51 (s, 6H), 1.72 (s, 4H), 0.69 (s, 2H), 0.48 (s, 2H). MS m/z: [M+H]⁺=639.61.

The compounds of Examples 856-871 were prepared by the preparation method for Examples 854 and 855

| Ex. | Compound name | Structural formula | m/z: ES⁺[M+H] |
|---|---|---|---|
| 856 | (S)-2-(4-(7-(8-chloronaphthalen-1-yl)-8-fluoro-2-((1-(pyrrolidin-1-ylmethyl)cyclopropyl)methoxy)pyridino[4,3-d]pyrimidin-4-yl)-1-(2-fluoroacryloyl)piperazin-2-vl)acetonitrile | 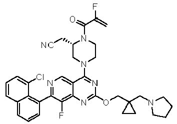 | 658.2 |
| 857 | (S)-2-(4-(7-(8-chloro-7-fluoronaphthalen-1-yl)-8-fluoro-2-((1-(pyrrolidin-1-ylmethyl)cyclopropyl)methoxy)pyridino[4,3-d]pyrimidin-4-yl)-1-(2-fluoroacryloyl)piperazin-2-vl)acetonitrile | 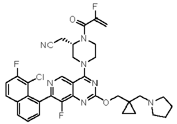 | 676.2 |
| 858 | (S)-2-(1-acryloyl-4-(7-(8-chloro-7-fluoronaphthalen-1-yl)-8-fluoro-2-((1-(pyrrolidin-1-ylmethyl)cyclopropyl)methoxy)pyridino[4,3-d]pyrimidin-4-yl)piperazin-2-yl)acetonitrile | 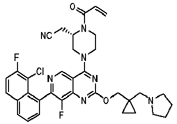 | 658 |
| 859 | (S)-2-(4-(7-(8-acetenylnaphthalen-1-yl)-8-fluoro-2-((1-(pyrrolidin-1-ylmethyl)cyclopropyl)methoxy)pyridino[4,3-d]pyrimidin-4-yl)-1-(2-fluoroacryloyl)piperazin-2-vl)acetonitrile | 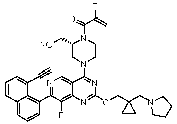 | 648 |
| 860 | (S)-2-(1-acryloyl-4-(7-(8-acetenylnaphthalen-1-yl)-8-fluoro-2-((1-(pyrrolidin-1-ylmethyl)cyclopropyl)methoxy)pyridino[4,3-d]pyrimidin-4-yl)piperazin-2-yl)acetonitrile | 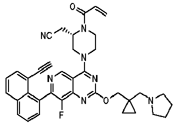 | 630 |
| 861 | (S)-2-(4-(7-(8-chloronaphthalen-1-yl)-8-fluoro-2-((1-(pyrrolidin-1-ylmethyl)cyclopropyl)methoxy)quinazolin-4-yl)-1-(2-fluoroacryloyl)piperazin-2-yl)acetonitrile | 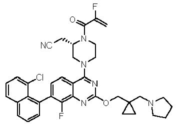 | 657 |
| 862 | (S)-2-(4-(7-(8-chloro-7-fluoronaphthalen-1-yl)-8-fluoro-2-((1-(pyrrolidin-1-ylmethyl)cyclopropyl)methoxy)quinazolin-4-yl)-1-(2-fluoroacryloyl)piperazin-2-yl)acetonitrile | 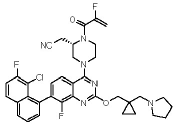 | 675 |
| 863 | (S)-2-(1-acryloyl-4-(7-(8-chloro-7-fluoronaphthalen-1-yl)-8-fluoro-2-((1-(pyrrolidin-1-ylmethyl)cyclopropyl)methoxy)quinazolin-4-yl)piperazin-2-yl(acetonitrile | 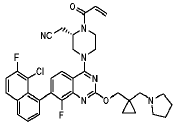 | 657 |
| 864 | (S)-2-(4-(7-(8-acetenylnaphthalen-1-yl)-8-fluoro-2-((1-(pyrrolidin-1-ylmethyl)cyclopropyl)methoxy)quinazolin-4-yl)-1-(2-fluoroacryloyl)piperazin-2-yl)acetonitrile | 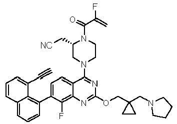 | 647 |
| 865 | (S)-2-(1-acryloyl-4-(7-(8-acetenylnaphthalen-1-yl)-8-fluoro-2-(((1-(pyrrolidin-1-ylmethyl)cyclopropyl)methoxy)quinazolin-4-yl)piperazin-2-yl)acetonitrile | 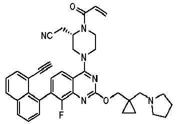 | 629 |
| 866 | 2-((2S)-4-(8-fluoro-2-((1-(pyrrolidin-1-ylmethyl)cyclopropyl)methoxy)-7-(1,1a,6,6a-tetrahydrocyclopropa[a]inden-2-yl)pyridinyl[4,3-d]pyrimidin-4-yl)-1-(2-fluoroacryloyl)piperazin-2-yl)acetonitrile | 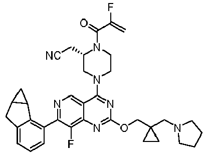 | 626.5 |
| 867 | 2-((2S)-4-(8-fluoro-2-((1-(pyrrolidin-1-ylmethyl)cyclopropyl)methoxy)-7-(1,la,6,6a-tetrahydrocyclopropa[a]inden-2-yl)pyridinyl[4,3-d]pyrimidin-4-yl)-1-(2-fluoroacryloyl)piperazin-2-yl)acetonitrile, isomer 1 | 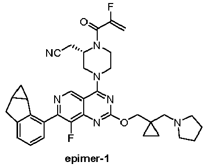 | 626.5 |
| 868 | 2-((2S)-4-(8-fluoro-2-((1-(pyrrolidin-1-ylmethyl)cyclopropyl)methoxy)-7-(1,la,6,6a-tetrahydrocyclopropa[a]inden-2-yl)pyridinyl[4,3-d]pyrimidin-4-yl)-1-(2-fluoroacryloyl)piperazin-2-yl)acetonitrile, isomer 2 | 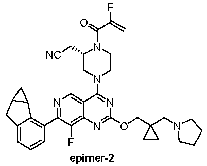 | 626.5 |
| 869 | 2-((2S)-4-(8-fluoro-2-((1-(pyrrolidin-1-ylmethyl)cyclopropyl)methoxy)-7-(1,1a,6,6a-tetrahydrocyclopropa[a]inden-2-yl)quinazolin-4-yl)-1-(2-fluoroacryloyl)piperazin-2-yl)acetonitrile | 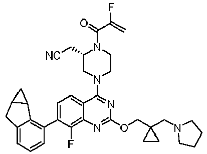 | 625.5 |
| 870 | 2-((2S)-4-(8-fluoro-2-((1-(pyrrolidin-1-ylmethyl)cyclopropyl)methoxy)-7-(1,1a,6,6a-tetrahydrocyclopropa[a]inden-2-yl)quinazolin-4-yl)-1-(2-fluoroacryloyl)piperazin-2-yl)acetonitrile, isomer 1 | 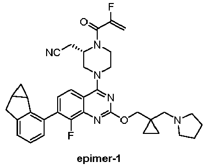 | 625.5 |
| 871 | 2-((2S)-4-(8-fluoro-2-((1-(pyrrolidin-1-ylmethyl)cyclopropyl)methoxy)-7-(1,1a,6,6a-tetrahydrocyclopropa[a]inden-2-yl)quinazolin-4-yl)-1-(2-fluoroacryloyl)piperazin-2-yl)acetonitrile, isomer 2 | 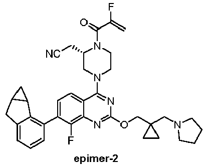 | 625.5 |

### Example 872: Synthesis of 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile

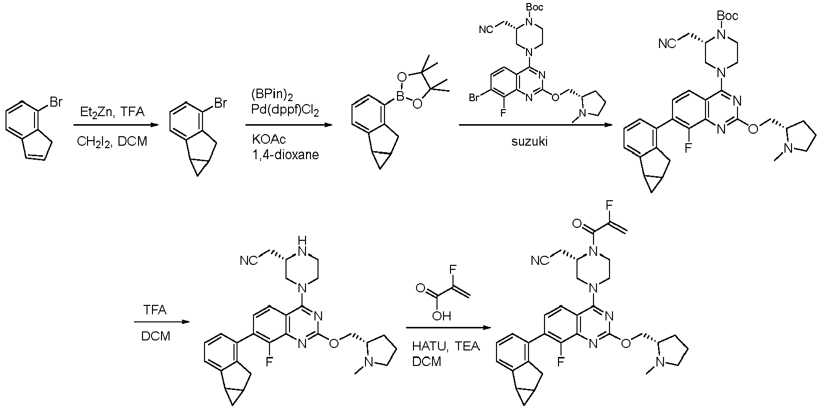

### Step 1: Synthesis of 5-bromo-1,1a,6,6a-tetrahydrocyclopropa[a]indene

A solution of diethyl zinc/n-hexane (1M, 423 mL) in anhydrous dichloromethane (200 mL) was cooled in an ice-water bath. A solution of trifluoroacetic acid (31 mL, 423 mmol) in anhydrous dichloromethane (200 mL) was added dropwise. After stirring at this temperature for 20 min, a solution of diiodomethane (34.3 mL, 423 mmol) in anhydrous dichloromethane (100 mL) was added. After continued stirring for 20 min, a solution of 7-bromo-1H-indene (22 g, 113 mmol) in anhydrous dichloromethane (100 mL) was slowly added dropwise. The ice-water bath was removed, and after stirring for 16 h, a white suspension was obtained. Dilute hydrochloric acid (0.1 M, 500 mL) was slowly added under stirring. The resulting mixture was extracted with petroleum ether (500 mL + 200 mL). The organic phases were combined, washed with a saturated sodium bicarbonate solution (300 mL) and with a saturated aqueous NaCl solution (300 mL), dried with sodium sulfate and concentrated in vacuo to obtain a yellow oil. The product 5-bromo-1,1a,6,6a-tetrahydrocyclopropa[a]indene was obtained by column chromatography (silica gel, petroleum ether) as a light yellow oil (22 g, 105 mmol, yield 93%). ¹H NMR (400 MHz, CDCl₃) δ 7.24-7.20 (m, 2H), 7.00-6.96 (m, 1H), 3.13 (dd, *J* = 17.6, 6.7 Hz, 1H), 2.97 (d, *J* = 17.6 Hz, 1H), 2.46-2.41 (m, 1H), 1.91-1.85 (m, 1H), 1.12-1.06 (m, 1H), 0.13-0.10 (m, 1H)

### Step 2: Synthesis of 4,4,5,5-tetramethyl-2-(1,1a,6,6a-tetrahydrocyclopropa[a]inden-5-yl)-1,3,2-dioxolane

5-bromo-1,1a,6,6a-tetrahydrocyclopropa[a]indene (30 g, 144 mmol), bis(pinacolato)diboron (72.9 g, 287 mmol), Pd(dppf)Cl₂ (21 g, 28.7 mmol) and potassium acetate (42 g, 431 mmol) were added to a round-bottomed flask. After displacement with nitrogen, anhydrous 1,4-dioxane (400 mL) which had been deoxidized in advance was added. The resulting suspension was stirred at 100°C for 16 h. After cooling to room temperature, the reaction mixture was extracted with water (500 mL) and methyl tert-butyl ether (500 mL). After layering, the aqueous phase was extracted with methyl tert-butyl ether (300 mL). The combined organic phases were washed with a saturated aqueous NaCl solution (300 mL), dried with sodium sulfate and spin-dried in vacuo. The remaining dark brown oil was purified by column chromatography (silica gel, ethyl acetate : petroleum ether = 1 : 40) to obtain a colorless oil, which was left to stand to slowly become a light yellow solid (26 g, 101 mmol, yield: 70%). ¹H NMR (400 MHz, CDCl₃) δ 7.54 (dd, *J* = 8.0, 2.0 Hz, 1H), 7.38 (dd, *J* = 8.0, 0.2 Hz, 1H), 7.11 (t, *J* = 8.0 Hz, 1H), 3.29 (dd, *J* = 17.6, 6.7 Hz, 1H), 3.20 (d, *J =* 17.6 Hz, 1H), 2.36-2.31 (m, 1H), 1.88-1.81 (m, 1H), 1.55 (s, 12H), 1.05-1.00 (m, 1H), 0.06-0.01 (m, 1H)

### Step 3: Synthesis of tert-butyl (2S)-2-(cyanomethyl)-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazine-1-carboxylate

Tert-butyl (S)-4-(7-bromo-8-fluoro-2-(((S)-1-methylpyrrolidin-2-yl)methoxy)quinazolin-4-yl)-2-(cyanomethyl)piperazine-1-carboxylate (300 mg, 0.53 mmol), 4,4,5,5-tetramethyl-2-(1,1a,6,6a-tetrahydrocyclopropa[a]inden-2-yl)-1,3,2-dioxolane (244 mg, 0.954 mmol), Pd(dppf)Cl₂ (78 mg, 0.106 mmol) and potassium carbonate (146 mg, 1.06 mmol) were added to a round-bottomed flask. After displacement with nitrogen, 1,4-dioxane (5 mL) and water (1 mL) which had been deoxidized in advance was added. The reaction liquid was stirred at 100°C for 4 h. Ethyl acetate (30 mL) and water (20 mL) were added. After shaking and separation, the aqueous phase was extracted with ethyl acetate (20 mL). The combined organic phases were washed with a saturated aqueous NaCl solution (20 mL), dried with sodium sulfate and spin-dried in vacuo. The remaining brown viscous material was purified by prep-TLC (silica gel, methanol : dichloromethane = 1 : 9) to obtain a light yellow solid. (140 mg, 0.228 mmol, yield: 43%) MS m/z: 613.4 [M+H]⁺.

### Step 4: Synthesis of 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile

Trifluoroacetic acid (0.8 mL) was added dropwise to a solution of tert-butyl (2S)-2-(cyanomethyl)-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazine-1-carboxylate (29 mg, 0.047 mmol) in dichloromethane (4 mL) at room temperature. The resulting solution was stirred at room temperature for 4 h. Dichloromethane (10 mL) was added and concentration in vacuo was performed. Dichloromethane (5 mL) was added to the resulting residue, concentration was performed again, and this process was repeated once. The resulting yellow solid was directly used for the next step.

### Step 5: Synthesis of 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile

A solution of 2-fluoroacrylic acid (8.5 mg, 0.095 mmol) and HATU (27 mg, 0.071 mmol) in dichloromethane (3 mL) was cooled in an ice-water bath. Triethylamine (0.026 mL, 0.189 mmol) was added dropwise. The ice-water bath was removed and stirring was performed for 20 min. A solution of 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile in dichloromethane (2 mL) was added in one portion. Stirring was performed at room temperature for 6 h. Dichloromethane (17 mL), water (15 mL) and a saturated sodium carbonate solution (5 mL) were added, and after shaking and separation, the aqueous phase was extracted with dichloromethane (20 mL). The organic phases were combined, washed with a saturated aqueous NaCl solution (10 mL), dried with sodium sulfate and subjected to rotary evaporation under reduced pressure to obtain a yellow solid. Purification by prep-TLC (methanol: dichloromethane = 1 : 8) gave 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile (60 mg, 0.103 mmol, overall yield over two steps: 63%). ¹H NMR (400 MHz, CDCl₃) δ 7.64 (d, *J* = 7.8 Hz, 1H), 7.39 (d, *J* = 7.8 Hz, 1H), 7.26 - 7.21 (m, 2H), 7.09 (d, *J* = 7.8 Hz, 1H), 5.46 (d, *J* = 16 Hz, 1H), 5.27 (dd, *J* = 16.4, 4.0 Hz, 1H), 4.99 - 4.94 (m, 1H), 4.64 (dd, *J* = 8.0, 4.0 Hz, 1H), 4.44 (d, J = 16 Hz, 1H), 4.34 (d, J = 16 Hz, 1H), 3.73 - 3.40 (m, 6H), 3.16 - 3.05 (m, 2H), 2.86 (s, 3H), 2.77-2.73 (m, 1H), 2.45-2.41 (m, 1H), 2.30-2.25 (m, 1H), 2.30-2.25 (m, 1H), 2.16-2.00 (m, 3H), 1.88-1.83 (m, 1H), 1.11-1.06 (m, 1H), 0.14-0.12 (m, 1H). MS m/z: [M+H]⁺=585.4.

The compounds of Examples 873-902 were prepared by the preparation method for Example 872

| Ex. | Compound name | Structural formula | m/z: ES⁺[M+H] |
|---|---|---|---|
| 873 | 2-((2S)-1-acryloyl-4-(8-fluoro-2-((S)-1-methylpyrrolidin-2-yl)methoxy)-7-(1,1a,6,6a-tetrahydrocyclopropa[a]inden-5-yl)pyridinyl [4, 3-d]pyrimidin-4-yl)piperazin-2-yl)acetonitrile, 1st eluting isomer | 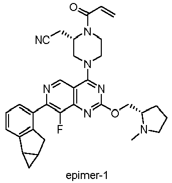 | 568.4 |
| | | | SFC(Chiralpak AD-3, 50x4.6 mm I.D., 3µm, 50% EtOH (0.1%IPAm)/CO₂, 3.4 mL/min, 1800psi |
| 874 | 2-((2S)-1-acryloyl-4-(8-fluoro-2-((S)-1-methylpyrrolidin-2-yl)methoxy)-7-(1,1a,6,6a-tetrahydrocyclopropa[a]inden-5-yl)pyridinyl[4,3-d]pyrimidin-4-yl)piperazin-2-yl)acetonitrile, 2nd eluting isomer | 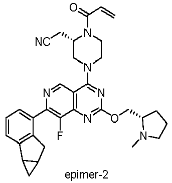 | 568.4 |
| | | | SFC(Chiralpak AD-3, 50x4.6 mm I.D., 3µm, 50% EtOH (0.1%IPAm)/CO₂, 3.4 mL/min, 1800psi |
| 875 | 2-((2S)-4-(8-fluoro-2-((S)-1-methylpyrrolidin-2-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, 1st eluting isomer | 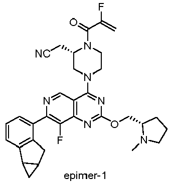 | 586.4 |
| | | | SFC(Chiralpak AD-3, 50x4.6 mm I.D., 3µm, 50% EtOH (0.1%IPAm)/CO₂, 3.4 mL/min, 1800psi |
| 876 | 2-((2S)-4-(8-fluoro-2-((S)-1-methylpyrrolidin-2-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, 2nd eluting isomer | 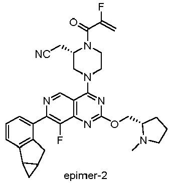 | 586.4 |
| | | | SFC(Chiralpak AD-3, 50x4.6 mm I.D., 3µm, 50% EtOH (0.1%IPAm)/CO₂, 3.4 mL/min, 1800psi |
| 877 | 2-((2S)-1-acryloyl-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridino[4,3-d]pyrimidin-4-yl)piperazin-2-yl)acetonitrile, 1st eluting isomer | 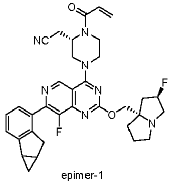 | 612.4 |
| | | | SFC(Chiralpak AD-3, 50x4.6 mm I.D., 3µm, 50% EtOH (0.1%IPAm)/CO₂, 3.4 mL/min, 1800psi |
| 878 | 2-((2S)-1-acryloyl-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridino[4,3-d]pyrimidin-4-yl)piperazin-2-yl)acetonitrile, 2nd eluting isomer | 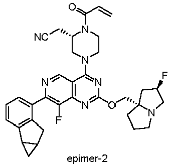 | 612.4 |
| | | | SFC(Chiralpak AD-3, 50x4.6 mm I.D., 3µm, 50% EtOH (0.1%IPAm)/CO₂, 3.4 mL/min, 1800psi |
| 879 | 2-((2S)-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, 1st eluting isomer | 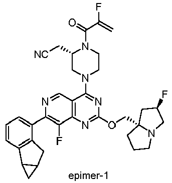 | 630.4 |
| | | | SFC(Chiralpak AD-3, 50x4.6 mm I.D., 3µm, 50% EtOH (0.1%IPAm)/CO₂, 3.4 mL/min, 1800psi |
| 880 | 2-((2S)-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, 2nd eluting isomer | 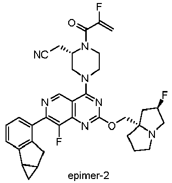 | 630.4 |
| | | | SFC(Chiralpak AD-3, 50x4.6 mm I.D., 3µm, 50% EtOH (0.1%IPAm)/CO₂, 3.4 mL/min, 1800psi |
| 881 | 2-((2S)-1-acryloyl-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, 1st eluting isomer | 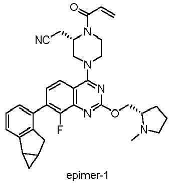 | 567.4 |
| | | | SFC(Chiralcel OD-3, 50x4.6 mm I.D., 3µm, 50% EtOH (0.1%IPAm)/CO₂, 4 mL/min, 1800psi |
| 882 | 2-((2S)-1-acryloyl-4-(8-fluoro-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, 2nd eluting isomer | 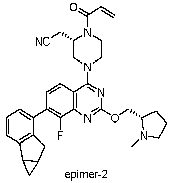 | 567.4 |
| | | | SFC(Chiralcel OD-3, 50x4.6 mm I.D., 3µm, 50% EtOH (0.1%IPAm)/CO₂, 4 mL/min, 1800psi |
| 883 | 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile, 1st eluting isomer | 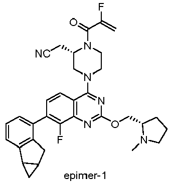 | 585.4 |
| | | | SFC(Chiralcel OD-3, 50x4.6 mm I.D., 3µm, 50% EtOH (0.1%IPAm)/CO₂, 4 mL/min, 1800psi |
| 884 | 2-((2S)-4-(8-fluoro-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile, 2nd eluting isomer | 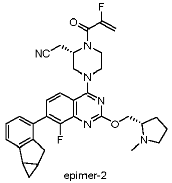 | 585.4 |
| | | | SFC(Chiralcel OD-3, 50x4.6 mm I.D., 3µm, 50% EtOH (0.1%IPAm)/CO₂, 4 mL/min, 1800psi |
| 885 | 2-((2S)-1-acryloyl-4-(8-fluoro-2-((2R,7aS)-2-fluorotetrahydro-1H-pyrrolizin-7a(5H)-yl)methoxy)-7-(1,la,6,6a-tetrahydrocyclopropa[a]inden-5-yl)quinazolin-4-yl)piperazin-2-yl)acetonitrile, 1st eluting isomer | 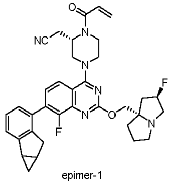 | 611.4 |
| | | | SFC(Chiralcel OD-3, 50x4.6 mm I.D., 3µm, 50% EtOH (0.1%IPAm)/CO₂, 4 mL/min, 1800psi |
| 886 | 2-((2S)-1-acryloyl-4-(8-fluoro-2-((2R,7aS)-2-fluorotetrahydro-1H-pyrrolizin-7a(5H)-yl)methoxy)-7-(1,la,6,6a-tetrahydrocyclopropa[a]inden-5-yl)quinazolin-4-yl)piperazin-2-yl)acetonitrile, 2nd eluting isomer | 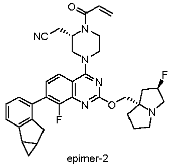 | 611.4 |
| | | | SFC(Chiralcel OD-3, 50x4.6 mm I.D., 3µm, 50% EtOH (0.1%IPAm)/CO₂, 4 mL/min, 1800psi |
| 887 | 2-((2S)-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)-1-(2-fluoroacryloyl)piperazin-2-yl)acetonitrile, 1st eluting isomer | 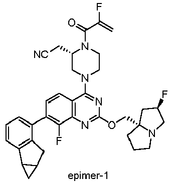 | 629.4 |
| | | | SFC(Chiralcel OD-3, 50x4.6 mm I.D., 3µm, 50% EtOH (0.1%IPAm)/CO₂, 4 mL/min, 1800psi |
| 888 | 2-((2S)-4-(8-fluoro-2-((2R,7aS)-2-fluorotetrahydro-1H-pyrrolizin-7a(5H)-yl)methoxy)-7-(1,la,6,6a-tetrahydrocyclopropa[a]inden-5-yl)quinazolin-4-yl)-1-(2-fluoroacryloyl)piperazin-2-yl)acetonitrile, 2nd eluting isomer | 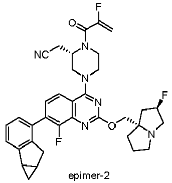 | 629.4 |
| | | | SFC(Chiralcel OD-3, 50x4.6 mm I.D., 3µm, 50% EtOH (0.1%IPAm)/CO₂, 4 mL/min, 1800psi |
| 889 | 2-((2S)-1-acryloyl-4-(8-fluoro-2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile, isomer 1 | 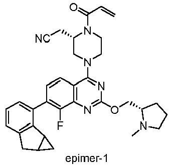 | 567.4 |
| 890 | 2-((2S)-1-acryloyl-4-(8-fluoro-2-((S)-1-methylpyrrolidin-2-yl)methoxy)-7-(1,1a,6,6a-tetrahydrocyclopropa[a]inden-2-yl)quinazolin-4-yl)piperazin-2-yl)acetonitrile, isomer 2 | 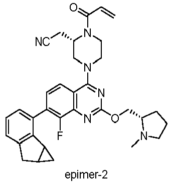 | 567.4 |
| 891 | 2-((2S)-4-(8-fluoro-2-((S)-1-methylpyrrolidin-2-yl)methoxy)-7-(1,1a,6,6a-tetrahydrocyclopropa[a]inden-2-yl)quinazolin-4-yl)-1-(2-fluoroacryloyl)piperazin-2-yl)acetonitrile, isomer 1 | 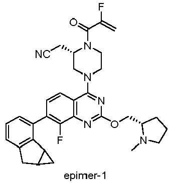 | 585.4 |
| 892 | 2-((2S)-4-(8-fluoro-2-((S)-1-methylpyrrolidin-2-yl)methoxy)-7-(1,1a,6,6a-tetrahydrocyclopropa[a]inden-2-yl)quinazolin-4-yl)-1-(2-fluoroacryloyl)piperazin-2-yl)acetonitrile, isomer 2 | 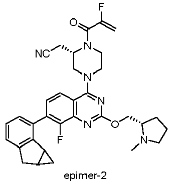 | 585.4 |
| 893 | 2-((2S)-1-acryloyl-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile, isomer 1 | 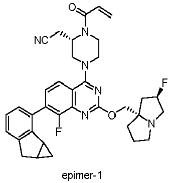 | 611.4 |
| 894 | 2-((2S)-1-acryloyl-4-(8-fluoro-2-((2R,7aS)-2-fluorotetrahydro-1H-pyrrolizin-7a(5H)-yl)methoxy)-7-(1,1a,6,6a-tetrahydrocyclopropa[a]inden-2-yl)quinazolin-4-yl)piperazin-2-yl)acetonitrile, isomer 2 | 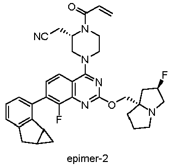 | 611.4 |
| 895 | 2-((2S)-1-acryloyl-4-(2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 1 | 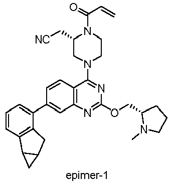 | 549.4 |
| 896 | 2-((2S)-1-acryloyl-4-(2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 2 | 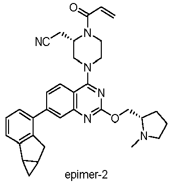 | 549.4 |
| 897 | 2-((2S)-1-(2-fluoroacryloyl)-4-(2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 1 | 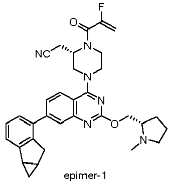 | 567.4 |
| 898 | 2-((2S)-1-(2-fluoroacryloyl)-4-(2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 2 | 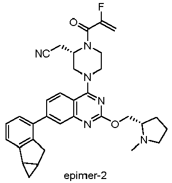 | 567.4 |
| 899 | 2-((2S)-1-acryloyl-4-(2-((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 1 | 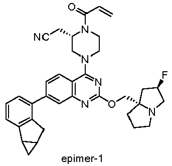 | 593.4 |
| 900 | 2-((2S)-1-acryloyl-4-(2-((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 2 | 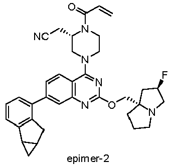 | 593.4 |
| 901 | 2-((2S)-1-(2-fluoroacryloyl)-4-(2-(((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 1 | 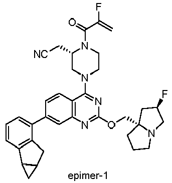 | 611.4 |
| 902 | 2-((2S)- 1-(2-fluoroacryloyl)-4-(2-(((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 2 | 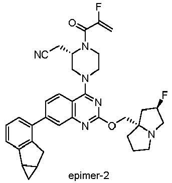 | 611.4 |
| 903 | 2-((S)-4-(7-(8-chloronaphthalen-1-yl)-8-fluoro-2-((S)-1-methylpyrrolidin-2-yl)methoxy)quinazolin-4-yl)-1-(2-fluoroacryloyl)piperazin-2-yl)acetonitrile | 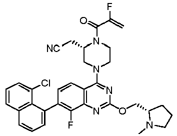 | 617.3 |
| 904 | 2-((S)-4-(7-(8-chloronaphthalen-1-yl)-8-fluoro-2-((2R,7aS)-2-fluorotetrahydro-1H-pyrrolizin-7a(5H)-methoxy)quinazolin-4-yl)-1-(2-fluoroacryloyl)piperazin-2-yl)acetonitrile | 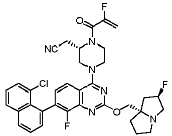 | 661.3 |
| 905 | 2-((2S)-1-acryloyl-4-(8-fluoro-2-((1-(pyrrolidin-1-ylmethyl)cyclopropyl)methoxy)-7-(1,1a,6,6a-tetrahydrocyclopropa[a]inden-5-yl)pyridinyl [4,3-d]pyrimidin-4-yl)piperazin-2-yl)acetonitrile, isomer 1 | 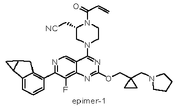 | 608.4 |
| 906 | 2-((2S)-1-acryloyl-4-(8-fluoro-2-((1-(pyrrolidin-1-ylmethyl)cyclopropyl)methoxy)-7-(1,1a,6,6a-tetrahydrocyclopropa[a]inden-5-yl)pyridinyl[4,3-d]pyrimidin-4-yl)piperazin-2-yl)acetonitrile, isomer 2 | 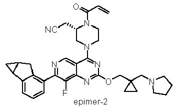 | 608.4 |
| 907 | 2-((2S)-4-(8-fluoro-2-((1-(pyrrolidin-1-ylmethyl)cyclopropyl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, isomer 1 | 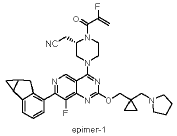 | 626.4 |
| 908 | 2-((2S)-4-(8-fluoro-2-((1-(pyrrolidin-1-ylmethyl)cyclopropyl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, isomer 2 | 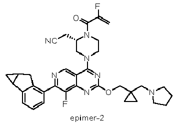 | 626.4 |
| 909 | 2-((2S)-1-acryloyl-4-(8-fluoro-2-((1-(pyrrolidin-1-ylmethyl)cyclopropyl)methoxy)-7-(1,1a,6,6a-tetrahydrocyclopropa[a]inden-5-yl)quinazolin-4-yl)piperazin-2-yl)acetonitrile, isomer 1 | 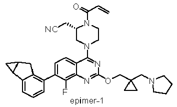 | 607.4 |
| 910 | 2-((2S)-1-acryloyl-4-(8-fluoro-2-((1-(pyrrolidin-1-ylmethyl)cyclopropyl)methoxy)-7-(1,1a,6,6a-tetrahydrocyclopropa[a]inden-5-yl)quinazolin-4-yl)piperazin-2-yl)acetonitrile, isomer 2 | 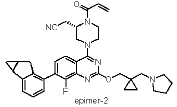 | 607.4 |
| 911 | 2-((2S)-4-(8-fluoro-2-((1-(pyrrolidin-1-ylmethyl)cyclopropyl)methoxy)-7-(1, 1a,6,6a-tetrahydrocyclopropa[a]inden-5-yl)quinazolin-4-yl)-1-(2-fluoroacryloyl)piperazin-2-yl)acetonitrile, isomer 1 | 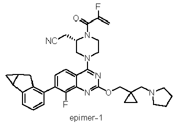 | 625.4 |
| 912 | 2-((2S)-4-(8-fluoro-2-((1-(pyrrolidin-1-ylmethyl)cyclopropyl)methoxy)-7-(1,1a,6,6a-tetrahydrocyclopropa[a]inden-5-yl)quinazolin-4-yl)-1-(2-fluoroacryloyl)piperazin-2-yl)acetonitrile, isomer 2 | 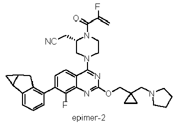 | 625.4 |
| 913 | 2-((S)-4-(7-(8-chloro-7-fluoronaphthalen-1-yl)-8-fluoro-2-((S)-1-methylpyrrolidin-2-yl)methoxy)quinazolin-4-yl)-1-(2-fluoroacryloyl)piperazin-2-yl)acetonitrile | 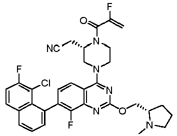 | 635.4 |
| 914 | 2-((S)-4-(7-(8-chloro-7-fluoronaphthalen-1-yl)-8-fluoro-2-((2R,7aS)-2-fluorotetrahydro-1H-pyrrolizin-7a(5H)-ylmethoxy)quinazolin-4-yl)-1-(2-fluoroacryloyl)piperazin-2-yl)acetonitrile | 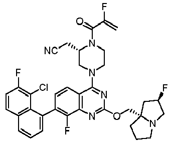 | 679.4 |

### Example 915: Synthesis of 2-((2S)-4-(6-chloro-7-(8-chloro-7-fluoronaphthalen-1-yl)-8-cyclopropoxy-2-((2R,7aS)-2-fluorotetrahydro-1H-pyrrolin-7a(5H)-ylmethoxy)quinazolin-4-yl)-1-(2-fluoroacryloyl)piperazin-2-yl)acetonitrile

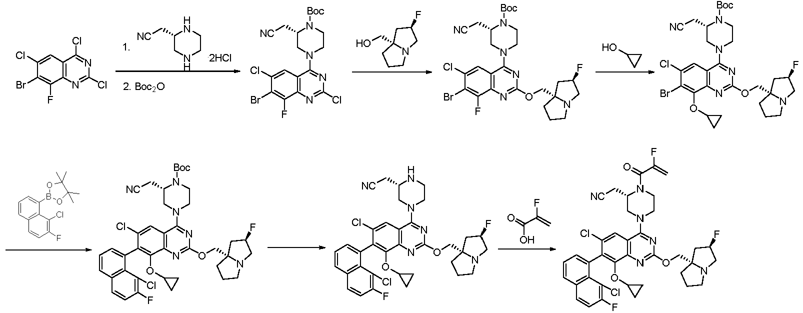

### Step 1: Synthesis of tert-butyl (S)-4-(7-bromo-2,6-dichloro-8-fluoroquinazolin-4-yl)-2-(cyanomethyl)piperazine-1-carboxylate

N,N-diisopropylethylamine (4.27 mL, 25.8 mmol) was added to a mixture of 7-bromo-2,4,6-trichloro-8-fluoroquinazoline (2.5 g, 7.58 mmol) and N,N-dimethylformamide (30 mL) in an ice-water bath. After 2 minutes of stirring, (S)-2-(piperazin-2-yl)acetonitrile hydrochloride (1.50 g, 7.60 mmol) was added. The reaction was carried out in an ice-water bath at room temperature and stirred for 1 h.

N,N-diisopropylethylamine (1.42 mL, 8.6 mmol) and di-tert butyl dicarbonate (1.96 g, 9 mmol) were added to the above reaction liquid at room temperature. The reaction liquid was stirred at room temperature for 4 h. Ethyl acetate (200 mL) and water (200 mL) were added for exaction and separation. The aqueous phase was extracted with ethyl acetate (100 mL). The organic phases were combined, washed with water (50 mL × 3) and with a saturated aqueous NaCl solution (50 mL), dried with sodium sulfate and concentrated under reduced pressure to obtain a dark brown viscous material. Purification by column chromatography (silica gel, ethyl acetate : petroleum ether = 1 : 10 to ethyl acetate : petroleum ether = 1 : 5) gave a yellow solid. (3.8 g, 7.32 mmol, yield: 96%). MS m/z: 518.2 [M+H]⁺.

### Step 2: Synthesis of tert-butyl (S)-4-(7-bromo-6-chloro-8-fluoro-2-((2R,7aS)-2-fluorotetrahydro-1H-pyrrolizin-7a(5H)-yl)methoxy)quinazolin-4-yl)-2-(cyanomethyl)piperazine-1-carboxylate

A solution of ((2R,7aS)-2-fluorotetrahydro-1H-pyrrolizin-7a(5H)-yl)methanol (1.4 g, 8.78 mmol) in tetrahydrofuran (30 mL) was cooled in an ice-water bath. Sodium hydride (0.32 g, 8.05 mmol) was added, and a reaction was carried out at room temperature for 0.5 h. A solution of tert-butyl (S)-4-(7-bromo-2,6-dichloro-8-fluoroquinazolin-4-yl)-2-(cyanomethyl)piperazine-1-carboxylate (3.8 g, 7.32 mmol) in tetrahydrofuran (15 mL) was added, and the reaction continued at room temperature for 1 h. Ethyl acetate (100 mL) and a diluted sodium carbonate aqueous solution (100 mL) were added for extraction, and the aqueous phase was extracted by ethyl acetate (50 mL). The organic phases were combined, washed with a saturated aqueous NaCl solution (50 mL), dried with sodium sulfate and concentrated under reduced pressure to obtain a light brown solid. Purification by column chromatography (silica gel, methanol : dichloromethane = 1 : 40) gave tert-butyl (S)-4-(7-bromo-6-chloro-8-fluoro-2-((2R,7aS)-2-fluorotetrahydro-1H-pyrrolizin-7a(5H)-yl)methoxy)quinazolin-4-yl)-2-(cyanomethyl)piperazine-1-carboxylate (2.1 g, 3.21 mmol, yield: 44%). MS m/z: [M+H]⁺=641.3.

### Step 3: Synthesis of tert-butyl (S)-4-(7-bromo-6-chloro-8-cyclopropoxy-2-((2R,7aS)-2-fluorotetrahydro-1H-pyrrolizin-7a(5H)-yl)methoxy)quinazolin-4-yl)-2-(cyanomethyl)piperazine-1-carboxylate

Tert-butyl (S)-4-(7-bromo-6-chloro-8-fluoro-2-((2R,7aS)-2-fluorotetrahydro-1H-pyrrolizin-7a(5H)-yl)methoxy)quinazolin-4-yl)-2-(cyanomethyl)piperazine-1-carboxylate (1.9 g, 2.96 mmol) and cyclopropanol (0.34 g, 6.0 mmol) were dissolved in tetrahydrofuran (20 mL). Sodium hydrogen (0.14 g, 3.5 mmol) was added in a nitrogen atmosphere, and the mixture was heated to 60°C and reacted for 2 h. Ethyl acetate (100 mL) and water (100 mL) were added for extraction. The organic phase was washed with a saturated aqueous NaCl solution (30 mL), dried with sodium sulfate and then concentrated under reduced pressure. The remaining viscous material was purified by column chromatography (silica gel, methanol : dichloromethane = 1 : 40) to obtain tert-butyl (S)-4-(7-bromo-6-chloro-8-cyclopropoxy-2-((2R,7aS)-2-fluorotetrahydro-1H-pyrrolizin-7a(5H)-yl)methoxy)quinazolin-4-yl)-2-(cyanomethyl)piperazine-1-carboxylate (0.8 g, 1.18 mmol, yield: 40%). MS m/z: [M+H]⁺=679.4.

### Step 4: Synthesis of tert-butyl (2S)-4-(6-chloro-7-(8-chloro-7-fluoronaphthalen-1-yl)-8-cyclopropoxy-2-(((2R,7aS)-2-fluorotetrahydro-1H-pyrrolizin-7a(5H)-yl)methoxy)quinazolin-4-yl)-2-(cyanomethyl)piperazine-1-carboxylate

Tert-butyl (S)-4-(7-bromo-6-chloro-8-cyclopropoxy-2-((2R,7aS)-2-fluorotetrahydro-1H-pyrrolizin-7a(5H)-yl)methoxy)quinazolin-4-yl)-2-(cyanomethyl)piperazine-1-carboxylate (200 mg, 0.294 mmol), 2-(8-chloro-7-fluoronaphthalen-1-yl)-4,4,5,5-tetramethyl-1,3,2-dioxaborane (144 mg, 0.47 mmol), Pd(dppf)Cl₂ (22 mg, 0.0294 mmol) and potassium carbonate (81 mg, 0.59 mmol) were added to an eggplant-shaped flask. Displacement with nitrogen was carried out three times. A mixed solvent of 1,4-dioxane (10 mL) and water (2 mL) which had been deoxidized in advance was added. Displacement with nitrogen was carried out three times. Then, after heating to 100°C, a reaction was carried out under stirring for 4 h. After cooling to room temperature, ethyl acetate (50 mL) and a diluted sodium carbonate aqueous solution (50 mL) were added for extraction. The organic phase was washed with a saturated aqueous NaCl solution (20 mL), dried with sodium sulfate and then concentrated under reduced pressure. The resulting brown viscous material was subjected to column chromatography (silica gel, triethylamine : methanol : dichloromethane = 0.04 : 1 : 40) to obtain the compound tert-butyl (2S)-4-(6-chloro-7-(8-chloro-7-fluoronaphthalen-1-yl)-8-cyclopropoxy-2-(((2R,7aS)-2-fluorotetrahydro-1H-pyrrolizin-7a(5H)-yl)methoxy)quinazolin-4-yl)-2-(cyanomethyl)piperazine-1-carboxylate (41 mg, 0.0525 mmol, yield: 18%). MS m/z: [M+H]⁺ = 779.5.

### Step 5: Synthesis of 2-((2S)-4-(6-chloro-7-(8-chloro-7-fluoronaphthalen-1-yl)-8-cyclonronoxy-2-((2R,7aS)-2-fluorotetrahydro-1H-pyrrolizin-7a(5H)-ylmethoxy)quinazolin-4-yl)piperazin-2-yl)acetonitrile

The compound tert-butyl (2S)-4-(6-chloro-7-(8-chloro-7-fluoronaphthalen-1-yl)-8-cyclopropoxy-2-(((2R,7aS)-2-fluorotetrahydro-1H-pyrrolizin-7a(5H)-yl)methoxy)quinazolin-4-yl)-2-(cyanomethyl)piperazine-1-carboxylate (40 mg, 0.0513 mmol) was dissolved in dichloromethane (3 mL), and trifluoroacetic acid (1 mL) was added dropwise. The reaction liquid was stirred at room temperature for 4 h. Dichloromethane (30 mL) and a saturated sodium carbonate aqueous solution (20 mL) were added for extraction. The aqueous phase was extracted with dichloromethane (10 mL × 2). The organic phases were combined, washed with a saturated aqueous NaCl solution (10 mL), dried with sodium sulfate and concentrated under reduced pressure to obtain 2-((2S)-4-(6-chloro-7-(8-chloro-7-fluoronaphthalen-1-yl)-8-cyclopropoxy-2-((2R,7aS)-2-fluorotetrahydro-1H-pyrrolizin-7a(5H)-ylmethoxy)quinazolin-4-yl)piperazin-2-yl)acetonitrile. The product was directly used for the next step of reaction without further purification. MS m/z: [M+H]+=679.4.

### Step 6: Synthesis of 2-((2S)-4-(6-chloro-7-(8-chloro-7-fluoronanhthalen-1-yl)-8-cyclopropoxy-2-((2R,7aS)-2-fluorotetrahydro-1H-pyrrolizin-7a(5H)-ylmethoxy)quinazolin-4-yl)-1-(2-fluoroacryloyl)piperazin-2-yl)acetonitrile

A solution of 2-fluoroacrylic acid (8.5 mg, 0.095 mmol) and HATU (27 mg, 0.071 mmol) in dichloromethane (3 mL) was cooled in an ice-water bath. Triethylamine (0.026 mL, 0.189 mmol) was added dropwise. The ice-water bath was removed and stirring was performed for 20 min. A solution of 2-((2S)-4-(6-chloro-7-(8-chloro-7-fluoronaphthalen-1-yl)-8-cyclopropoxy-2-((2R,7aS)-2-fluorotetrahydro-1H-pyrrolizin-7a(5H)-ylmethoxy)quinazolin-4-yl)piperazin-2-yl)acetonitrile in dichloromethane (2 mL) was added in one portion. Stirring was performed at room temperature for 6 h. Dichloromethane (17 mL), water (15 mL) and a saturated sodium carbonate solution (5 mL) were added, and after shaking and separation, the aqueous phase was extracted with dichloromethane (20 mL). The organic phases were combined, washed with a saturated aqueous NaCl solution (10 mL), dried with sodium sulfate and subjected to rotary evaporation under reduced pressure to obtain a yellow solid. Purification by prep-TLC (methanol : dichloromethane = 1 : 10) gave 2-((2S)-4-(6-chloro-7-(8-chloro-7-fluoronaphthalen-1-yl)-8-cyclopropoxy-2-((2R,7aS)-2-fluorotetrahydro-1H-pyrrolizin-7a(5H)-ylmethoxy)quinazolin-4-yl)-1-(2-fluoroacryloyl)piperazin-2-yl)acetonitrile (19 mg, 0.0253 mmol, overall yield over two steps: 48%). ¹H NMR (400 MHz, CDCl₃) δ 7.96 (d, *J* = 7.8 Hz, 1H), 7.88-7.86 (m, 1H), 7.65-7.63 (m, 1H), 7.55 (t, *J* = 7.8 Hz, 1H), 7.33 (s, 1H), 7.27 - 7.24 (m, 1H), 5.45 (d, *J* = 48 Hz, 1H), 5.46 (d, *J* = 16 Hz, 1H), 5.27 (dd, *J =* 16.4, 4.0 Hz, 1H), 4.89 (brs, 1H), 4.45 - 4.24 (m, 4H), 3.67-3.64 (m, 2H), 3.48-3.44 (m, 2H), 3.33-3.31 (m, 3H), 3.24-3.19 (m, 2H), 3.10-2.88 (m, 3H), 2.29-2.16 (m, 2H), 1.96-1.92 (m, 3H), 0.30-0.10 (m, 4H). MS m/z: [M+H]⁺=751.4

The compounds of Examples 916-938 were prepared by the preparation method for Example 915

| Ex. | Compound name | Structural formula | m/z: ES⁺[M+H] |
|---|---|---|---|
| 916 | 2-((2S)-4-(6-chloro-7-(8-chloronaphthalen-1-yl)-8-cyclopropoxy-2-((2R,7aS)-2-fluorotetrahydro-1H-pyrrolizin-7a(5H)-yl)methoxy)quinazolin-4-yl)-1-(2-fluoroacryloyl)piperazin-2-yl)acetonitrile | 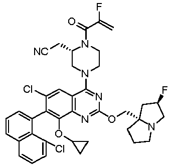 | 733.4 |
| 917 | 2-((2S)-4-(6-chloro-8-cyclopropoxy-2-((2R,7aS)-2-fluorotetrahydro-1H-pyrrolizin-7a(5H)-ylmethoxy)-7-(1,1a,6,6a-tetrahydrocyclopropa[a]inden-5-yl)quinazolin-4-yl)-1-(2-fluoroacryloyl)piperazin-2-yl)acetonitrile | 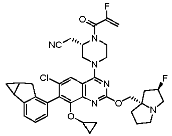 | 701.5 |
| 918 | 2-((2S)-4-(6-chloro-8-cyclopropoxy-2-((2R,7aS)-2-fluorotetrahydro-1H-pyrrolizin-7a(5H)-ylmethoxy)-7-(1,1a,6,6a-tetrahydrocyclopropa[a]inden-5-yl)quinazolin-4-yl)-1-(2-fluoroacryloyl)piperazin-2-yl)acetonitrile, isomer 1 | 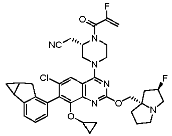 | 701.5 |
| 919 | 2-((2S)-4-(6-chloro-8-cyclopropoxy-2-((2R,7aS)-2-fluorotetrahydro-1H-pyrrolizin-7a(5H)-ylmethoxy)-7-(1,1a,6,6a-tetrahydrocyclopropa[a]inden-5-yl)quinazolin-4-yl)-1-(2-fluoroacryloyl)piperazin-2-yl)acetonitrile, isomer 2 | 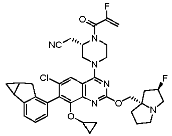 | 701.5 |
| 920 | 2-((2S)-1-acryloyl-4-(6-chloro-7-(8-chloro-7- fluoronaphthalen-1-yl)-8-cyclopropoxy-2-((2R,7aS)-2-fluorotetrahydro-1H-pyrrolizin-7a(5H-yl)methoxy)quinazolin-4-yl)piperazin-2-yl)acetonitrile | 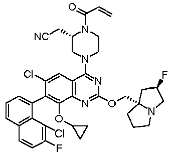 | 733.4 |
| 921 | 2-((2S)-1-acryloyl-4-(6-chloro-7-(8-chloronaphthalen-1-yl)-8-cyclopropoxy-2-((2R,7aS)-2-fluorotetrahydro-1H-pyrrolizin-7a(5H)-ylmethoxy)quinazolin-4-yl)piperazin-2-yl)acetonitrile | 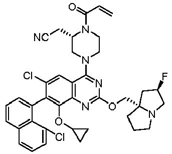 | 715.4 |
| 922 | 2-((2S)-1-acryloyl-4-(6-chloro-8-cyclopropoxy-2-((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 1 | 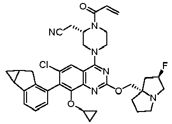 | 683.5 |
| 923 | 2-((2S)-1-acryloyl-4-(6-chloro-8-cyclopropoxy-2-((2R,7aS)-2-fluorotetrahydro-1H-pyrrolizin-7a(5H)-yl)methoxy)-7-(1,1a,6,6a-tetrahydrocyclopropa[a]inden-5-yl)quinazolin-4-yl)piperazin-2-yl)acetonitrile, isomer 2 | 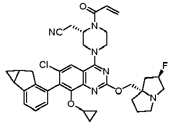 | 683.5 |
| 924 | 2-((2S)-4-(6-chloro-7-(8-chloro-7-fluoronaphthalen-1-yl)-8-cyclopropoxy-2-((S)-1-methylpyrrolidin-2-yl)methoxy)quinazolin-4-yl)-1-(2-fluoroacryloyl)piperazin-2-yl)acetonitrile | 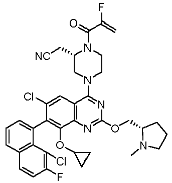 | 707.4 |
| 925 | 2-((2S)-4-(6-chloro-7-(8-chloronaphthalen-1-yl)-8-cyclopropoxy-2-((S)-1 -methylpyrrolidin-2-yl)methoxy)quinazolin-4-yl)-1-(2-fluoroacryloyl)piperazin-2-yl)acetonitrile | 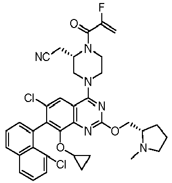 | 689.4 |
| 926 | 2-((2S)-4-(6-chloro-8-cyclopropoxy-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile, isomer 1 | 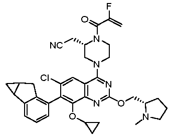 | 657.5 |
| 927 | 2-((2S)-4-(6-chloro-8-cyclopropoxy-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile, isomer 2 | 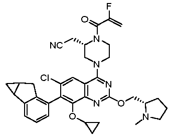 | 657.5 |
| 928 | 2-((2S)-1 -acryloyl-4-(6-chloro-7-(8-chloro-7 - fluoronaphthalen-1-yl)-8-cyclopropoxy-2-((S)-1-methylpyrrolidin-2-yl)methoxy)quinazolin-4-yl)piperazin-2-yl)acetonitrile | 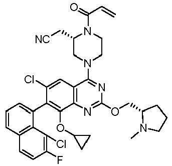 | 689.4 |
| 929 | 2-((2S)-1-acryloyl-4-(6-chloro-7-(8-chloronaphthalen-1-yl)-8-cyclopropoxy-2-((S)-1-methylpyrrolidin-2-yl)methoxy)quinazolin-4-yl)piperazin-2-yl)acetonitrile | 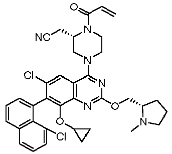 | 671.4 |
| 930 | 2-((2S)-1-acryloyl-4-(6-chloro-8-cyclopropoxy-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 1 | 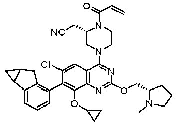 | 639.3 |
| 931 | 2-((2S)-1-acryloyl-4-(6-chloro-8-cyclopropoxy-2-((S)-1-methylpyrrolidin-2-yl)methoxy)-7-(1,1a,6,6a-tetrahydrocyclopropa[a]inden-5-yl)quinazolin-4-yl)piperazin-2-yl)acetonitrile, isomer 2 | 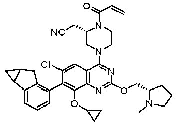 | 639.3 |
| 932 | 2-((2S)-4-(6-chloro-8-cyclopropoxy-2-((2R,7aS)-2-fluorotetrahydro-1H-pyrrolizin-7a(5H)-ylmethoxy)-7-(5-methyl-1H-indazol-4-yl)quinazolin-4-yl)-1-(2-fluoroacryloyl)piperazin-2-yl)acetonitrile | 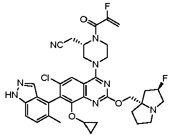 | 703.5 |
| 933 | 2-((2S)-1-acryloyl-4-(6-chloro-8-cyclopropoxy-2-((2R,7aS)-2-fluorotetrahydro-1H-pyrrolizin-7a(5H)-yl)methoxy)-7-(5-methyl-1H-indazol-4-yl)quinazolin-4-yl)piperazin-2-yl)acetonitrile | 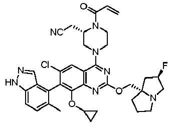 | 685.5 |
| 934 | 2-((2S)-4-(8-cyclopropoxy-2-((2R,7aS)-2-fluorotetrahydro-1H-pyrrolizin-7a(5H)-yl)methoxy)-7-(5-methyl-1H-indazol-4-yl)pyridinyl[4,3-d]pyrimidin-4-yl)-1-(2-fluoroacryloyl)piperazin-2-yl)acetonitrile | 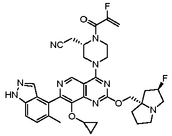 | 670.5 |
| 935 | 2-((S)-4-(7-(8-chloronaphthalen-1-yl)-8-cyclopropoxy-2-((2R,7aS)-2-fluorotetrahydro-1H-pyrrolizin-7a(5H)-yl)methoxy)pyridinyl[4,3-d]pyrimidin-4-yl)-1-(2-fluoroacryloyl)piperazin-2-yl)acetonitrile | 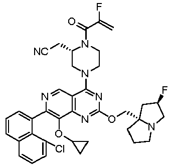 | 700.5 |
| 936 | 2-((S)-4-(7-(8-chloro-7-fluoronaphthalen-1-yl)-8-cyclopropoxy-2-((2R,7aS)-2-fluorotetrahydro-1H-pyrrolizin-7a(5H)-yl)methoxy)pyridino[4,3-d]pyrimidin-4-yl)-1-(2-fluoroacryloyl)piperazin-2-yl)acetonitrile | 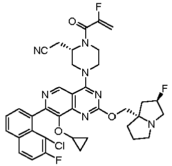 | 718.4 |
| 937 | 2-((2S)-4-(8-cyclopropoxy-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, isomer 1 | 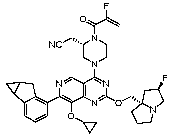 | 668.5 |
| 938 | 2-((2S)-4-(8-cyclopropoxy-2-((2R,7aS)-2-fluorotetrahydro-1H-pyrrolizin-7a(5H)-yl)methoxy)-7-(1,1a,6,6a-tetrahydrocyclopropa[a]inden-5-yl)pyridinyl[4,3-d]pyrimidin-4-yl)-1-(2-fluoroacryloyl)piperazin-2-yl)acetonitrile, isomer 2 | 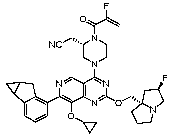 | 668.5 |

### Example 939: Synthesis of 2-((2S)-4-(7-(8-chloro-7-fluoronaphthalen-1-yl)-8-fluoro-2-((2R,7aS)-2-fluorotetrahydro-1H-pyrrolizin-7a(5H)-ylmethoy)-6-hydroxyquinazolin-4-yl)-1-(2-fluoroacryloyl)piperazin-2-yl)acetonitrile

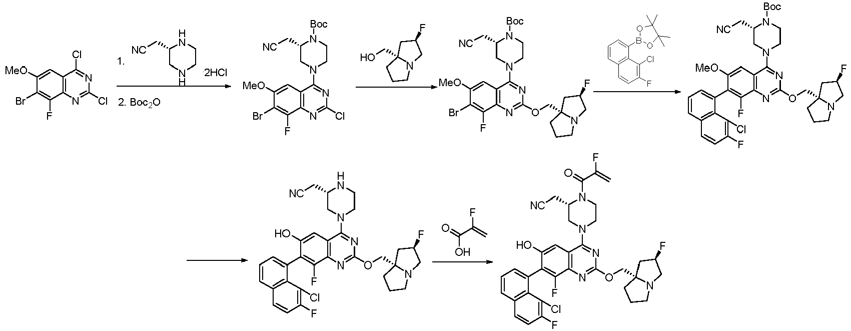

### Step 1: Synthesis of tert-butyl (S)-4-(7-bromo-2-chloro-8-fluoro-6-methoxyquinazolin-4-vl)-2-(cyanomethyl)piperazine-1-carboxylate

N,N-diisopropylethylamine (6.6 mL, 40 mmol) was added to a solution of 7-bromo-2,4-dichloro-8-fluoro-6-methoxyquinazoline (4.0 g, 12.3 mmol) in N,N-dimethylformamide (40 mL) in one portion in an ice-water bath. After one minute of stirring, (S)-2-(piperazin-2-yl)acetonitrile hydrochloride (2.55 g, 12.9 mmol) was added. The reaction was carried out in an ice-water bath at room temperature and stirred for 1 h.

N,N-diisopropylethylamine (2.2 mL, 13.3 mmol) and di-tert butyl dicarbonate (3.27 g, 15 mmol) were added to the above reaction liquid at room temperature. The reaction liquid was stirred at room temperature for 4 h. Ethyl acetate (200 mL) and water (200 mL) were added for exaction and separation. The aqueous phase was extracted with ethyl acetate (100 mL). The organic phases were combined, washed with water (60 mL × 3) and with a saturated aqueous NaCl solution (50 mL), dried with sodium sulfate and concentrated under reduced pressure to obtain a dark brown viscous material. Purification by column chromatography (silica gel, ethyl acetate : petroleum ether = 1 : 10 to ethyl acetate : petroleum ether = 1 : 5) gave a yellow solid. (5.4 g, 10.5 mmol, yield: 85%). MS m/z: 514.2 [M+H]+.

### Step 2: Synthesis of tert-butyl (S)-4-(7-bromo-8-fluoro-2-((2R,7aS)-2-fluorotetrahydro-1H-pyrrolizin-7a(5H)-ylmethoxy)-6-methoxyquinazolin-4-yl)-2-(cyanomethyl)piperazine-1-carboxylate

A solution of ((2R,7aS)-2-fluorotetrahydro-1H-pyrrolizin-7a(SH)-yl)methanol (1.9 g, 12 mmol) in tetrahydrofuran (40 mL) was cooled in an ice-water bath. Sodium hydride (0.44 g, 11 mmol) was added, and a reaction was carried out at room temperature for 0.5 h. Tert-butyl (S)-4-(7-bromo-2-chloro-8-fluoro-6-methoxyquinazolin-4-yl)-2-(cyanomethyl)piperazine-1-carboxylate (5.4 g, 10.5 mmol) was added in one portion, and the reaction continued at room temperature for 1 h. Ethyl acetate (100 mL) and a diluted sodium carbonate aqueous solution (100 mL) were added for extraction, and the aqueous phase was extracted by ethyl acetate (50 mL). The organic phases were combined, washed with a saturated aqueous NaCl solution (50 mL), dried with sodium sulfate and concentrated under reduced pressure to obtain a light brown solid. Purification by column chromatography (silica gel, methanol: dichloromethane = 1 : 40) gave tert-butyl (S)-4-(7-bromo-6-chloro-8-fluoro-2-((2R,7aS)-2-fluorotetrahydro-1H-pyrrolizin-7a(5H)-yl)methoxy)quinazolin-4-yl)-2-(cyanomethyl)piperazine-1-carboxylate (4.0 g, 6.27 mmol, yield: 60%). MS m/z: [M+H]⁺ = 637.4.

### Step 3: Synthesis of tert-butyl (2S)-4-(7-(8-chloro-7-fluoronaphthalen-1-yl)-8-fluoro-2-((2R,7aS)-2-fluorotetrahydro-1H-pyrrolizin-7a(5H)-yl)methoxy)-6-methoxyquinazolin-4-yl)-2-(cyanomethyl)piperazine-1-carboxylate

Tert-butyl (S)-4-(7-bromo-8-fluoro-2-((2R,7aS)-2-fluorotetrahydro-1H-pyrrolizin-7a(SH)-ylmethoxy)-6-methoxyquinazolin-4-yl)-2-(cyanomethyl)piperazine-1-carboxylate (300 mg, 0.47 mmol), 2-(8-chloro-7-fluoronaphthalen-1-yl)-4,4,5,5-tetramethyl-1,3,2-dioxaborane (230 mg, 0.75 mmol), Pd(dppf)Cl₂ (34 mg, 0.047 mmol), and potassium carbonate (127 mg, 0.94 mmol) were added to an eggplant-shaped flask. Displacement with nitrogen was carried out three times. A mixed solvent of 1,4-dioxane (10 mL) and water (2 mL) which had been deoxidized in advance was added. Displacement with nitrogen was carried out three times. Then, after heating to 100°C, a reaction was carried out under stirring for 4 h. After cooling to room temperature, ethyl acetate (50 mL) and a diluted sodium carbonate aqueous solution (50 mL) were added for extraction. The organic phase was washed with a saturated aqueous NaCl solution (20 mL), dried with sodium sulfate and then concentrated under reduced pressure. The resulting brown viscous material was subjected to column chromatography (silica gel, triethylamine : methanol: dichloromethane = 0.04 : 1 : 50) to obtain the compound tert-butyl (2S)-4-(7-(8-chloro-7-fluoronaphthalen-1-yl)-8-fluoro-2-((2R,7aS)-2-fluorotetrahydro-1H-pyrrolizin-7a(5H)-yl)methoxy)-6-methoxyquinazolin-4-yl)-2-(cyanomethyl)piperazine-1-carboxylate (71 mg, 0.096 mmol, yield: 20%). MS m/z: [M+H]⁺ = 737.5.

### Step 4: Synthesis of 2-((2S)-4-(7-(8-chloro-7-fluoronaphthalen-1-yl)-8-fluoro-2-((2R,7aS)-2-fluorotetrahydro-1H-pyrrolizin-7a(5H)-vlmethoxy)-6-hydroxyguinazolin-4-yl)piperazin-2-yl)acetonitrile

The compound tert-butyl (2S)-4-(7-(8-chloro-7-fluoronaphthalen-1-yl)-8-fluoro-2-((2R,7aS)-2-fluorotetrahydro-1H-pyrrolizin-7a(5H)-yl)methoxy)-6-methoxyquinazolin-4-yl)-2-(cyanomethyl)piperazine-1-carboxylate (70 mg, 0.096 mmol) was dissolved in dichloromethane (6 mL), the mixture was cooled in an ice-water bath, and a solution of boron tribromide (0.057 mL, 0.77 mmol) in dichloromethane (3 mL) was added dropwise. The reaction was carried out in an ice-water bath for 1. Dichloromethane (30 mL) was added and methanol (10 mL) was slowly added dropwise. Rotary evaporation under reduced pressure was carried out, and dichloromethane (20 mL) and a saturated sodium carbonate aqueous solution (20 mL) was added to the residue for extraction. The aqueous phase was extracted with dichloromethane (10 mL × 2). The organic phases were combined, washed with a saturated aqueous NaCl solution (10 mL), dried with sodium sulfate and concentrated under reduced pressure to obtain a yellow viscous material. Purification by prep-TLC (methanol: dichloromethane = 1 : 7) gave 2- ((2S)-4-(7-(8-chloro-7-fluoronaphthalen-1-yl)-8-fluoro-2-((2R,7aS)-2-fluorotetrahydro-1H-pyrrolizin- 7a(5H)-ylmethoxy)-6-hydroxyquinazolin-4-yl)piperazin-2-yl)acetonitrile (15 mg, 0.024 mmol, yield: 25%). MS m/z: [M+H]⁺ = 623.4.

### Step 5: Synthesis of 2-((2S)-4-(7-(8-chloro-7-fluoronaphthalen-1-yl)-8-fluoro-2-((2R,7aS)-2-fluorotetrahvdro-1H-pyrrolizin-7a(5H)-ylmethoxy)-6-hydroxyquinazolin-4-yl)-1-(2-fluoroacryloyl)piperazin-2-yl)acetonitrile

A solution of 2-fluoroacrylic acid (4.3 mg, 0.048 mmol) and HATU (11 mg, 0.03 mmol) in dichloromethane (3 mL) was cooled in an ice-water bath. Triethylamine (0.014 mL, 0.1 mmol) was added dropwise. The ice-water bath was removed and stirring was performed for 20 min. A solution of 2-((2S)-4- (7-(8-chloro-7-fluoronaphthalen-1-yl)-8-fluoro-2-((2R,7aS)-2-fluorotetrahydro-1H-pyrrolizin-7a(SH)- ylmethoxy)-6-hydroxyquinazolin-4-yl)piperazin-2-yl)acetonitrile (15 mg, 0.024 mmol) in dichloromethane (2 mL) was added in one portiton. Stirring was performed at room temperature for 2 h. Dichloromethane (17 mL), water (15 mL) and a saturated sodium carbonate solution (5 mL) were added, and after shaking and separation, the aqueous phase was extracted with dichloromethane (20 mL). The organic phases were combined, washed with a saturated aqueous NaCl solution (10 mL), dried with sodium sulfate and subjected to rotary evaporation under reduced pressure to obtain a yellow solid. Purification by prep-TLC (methanol : dichloromethane = 1 : 10) gave 2-((2S)-4-(7-(8-chloro-7-fluoronaphthalen-1-yl)-8-fluoro-2- ((2R,7aS)-2-fluorotetrahydro-1H-pyrrolizin-7a(5H)-ylmethoxy)-6-hydroxyquinazolin-4-yl)-1-(2- fluoroacryloyl)piperazin-2-yl)acetonitrile (5 mg, 0.0072 mmol, yield: 30%). ¹H NMR (400 MHz, CDCl₃) δ 7.88 (d, *J* = 7.8 Hz, 1H), 7.88-7.86 (m, 1H), 7.65-7.63 (m, 1H), 7.55 (t, *J* = 7.8 Hz, 1H), 7.27 - 7.24 (m, 1H), 7.01 (s, 1H), 5.45 (d, *J* = 48 Hz, 1H), 5.46 (d, *J* = 16 Hz, 1H), 5.27 (dd, *J* = 16.4, 4.0 Hz, 1H), 4.90 (brs, 1H), 4.45 - 4.29 (m, 4H), 3.68-3.64 (m, 2H), 3.46-3.44 (m, 2H), 3.33-3.31 (m, 2H), 3.24-3.19 (m, 2H), 3.10-2.88 (m, 3H), 2.29-2.16 (m, 2H), 1.96-1.92 (m, 3H). MS m/z: [M+H]⁺=695.4

The compounds of Examples 940-945 were prepared by the preparation method for Example 939

| Ex. | Compound name | Structural formula | m/z: ES⁺[M+H] |
|---|---|---|---|
| 940 | 2-((2S)-4-(7-(8-chloronaphthalen-1-yl)-8-fluoro-2-((2R,7aS)-2-fluorotetrahydro-1H-pyrrolizin-7a(5H)-yl)methoxy)-6-hydroxyquinazolin-4-yl)-1-(2-fluoroacryloyl)piperazin-2-yl)acetonitrile | 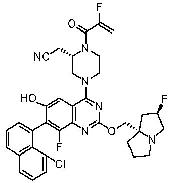 | 677.4 |
| 941 | 2-((2S)-4-(8-fluoro-2-((2R,7aS)-2-fluorotetrahydro-1H-pyrrolizin-7a(5H)-yl)methoxy)-6-hydroxy-7-(1,1a,6,6a-tetrahydrocyclopropa[a]inden-5-yl)quinazolin-4-yl)-1-(2-fluoroacryloyl)piperazin-2-yl)acetonitrile, isomer 1 | 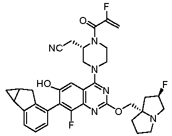 | 645.3 |
| 942 | 2-((2S)-4-(8-fluoro-2-((2R,7aS)-2-fluorotetrahydro-1H-pyrrolizin-7a(5H)-yl)methoxy)-6-hydroxy-7-(1,1a,6,6a-tetrahydrocyclopropa[a]inden-5-yl)quinazolin-4-yl)-1-(2-fluoroacryloyl)piperazin-2-yl)acetonitrile, isomer 2 | 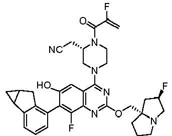 | 645.3 |
| 943 | 2-((2S)-4-(7-(8-chloro-7-fluoronaphthalen-1-yl)-8-fluoro-6-hydroxy-2-((S)-1-methylpyrrolidin-2-yl)methoxy)quinazolin-4-yl)-1-(2-fluoroacryloyl)piperazin-2-yl)acetonitrile | 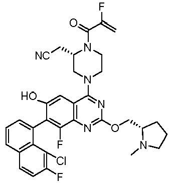 | 651.4 |
| 944 | 2-((2S)-4-(7-(8-chloronaphthalen-1-yl)-8-fluoro-6-hydroxy-2-((S)-1-methylpyrrolidin-2-yl)methoxy)quinazolin-4-yl)-1-(2-fluoroacryloyl)piperazin-2-yl)acetonitrile | 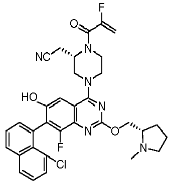 | 633.4 |
| 945 | 2-((2S)-4-(8-fluoro-6-hydroxy-2-((S)-1-methylpyrrolidin-2-yl)methoxy)-7-(1,1a,6,6a-tetrahydrocyclopropa[a]inden-5-yl)quinazolin-4-yl)-1-(2-fluoroacryloyl)piperazin-2-yl)acetonitrile | 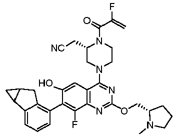 | 601.5 |

Example: Cell activity experiment
1. Cells: H358 purchased from Shanghai EK-Bioscience Biotechnology Co., Ltd.
2. Reagents: RPMI 1640 medium, Tryple, MTT (5 mg/ mL), DMSO, and DPBS.
3. Instruments: an incubator at 37°C and 5% CO2, a UTRAO microplate reader, a biosafety cabinet, a cell counting plate, and an Optec optical microscope.
4. Experimental consumables: 96-well plate Item No.: 3599, and 96-well round-bottomed dispensing plate.

Experimental steps of activity test of H358 cells:
1. Plating: Cells in the logarithmic growth phase were digested with Tryple and terminated with a fresh medium, and the cells were counted; and the cell concentration was adjusted to 55555 cells/mL with a fresh medium, wherein 90 µL was added to each well, and those on edges were filled with sterile DPBS.
2. The plate was incubated in the incubator at 37°C and 5% CO2 for 24 h, so that the cells cover the bottom of the well by about 50%.
3. Drug preparation for experimental group: The drug was dissolved in DMSO to prepare a 20 mmol/L stock solution; the stock solution was further diluted with DMSO to 2 mmol/L, which was serially 3-fold diluted to form an 8-concentration gradient, resulting in a 200× serial compound solution; 10 µL of the serial compound solution was taken and added to 190 µLµL of RPMI1640 medium to obtain a 10× serial compound solution; and 10 µL of the 10× compound solution was taken and added to 90 µL 96-well cell culture plate, with three replicates per grade. The concentration gradient of the compound in the 96-well cell culture plate was 0.05080526 nM, 1.524158 nM, 4.572474 nM, 13.717420 nM, 41.152260 nM, 123.456800 nM, 370.370400 nM, 1111.111000 nM, 3333.333000 nM, and 10000.000000 nM, with 100 µL per well, and the final concentration of DMSO was 0.5%.

The control group contained the same volume of solvent as the experimental group and was diluted with a complete medium, with 100 µL per well.
4. Incubation was carried out for 5 days in an incubator with 5% CO2 at 37°C.
5. After 5 days, 10 µL of an MTT solution (5 mg/ mL) was added to each well, and the culture was continued for 4 h.
6. The culture was terminated, and the culture solution was carefully sucked out of the well.
7. 150 µL of dimethyl sulfoxide (DMSO) was added to each of the null wells in the experimental group and the control group; and after shaking at a medium speed for 10 s, the crystal was fully dissolved, and the optical absorbance value thereof was measured at a wavelength of 492 nm.

The IC₅₀ values of some compounds were as shown in Table 1:

| Ex No. | H358 IC₅₀ (nM) | Ex. No. | H358 IC₅₀ (nM) |
|---|---|---|---|
| 1 | 15 | 261 | 24 |
| 2 | 1 | 286 | 355 |
| 3 | 3.5 | 358 | 3.4 |
| 4 | 70 | 359 | 3 |
| 5 | 20 | 360 | 10 |
| 6 | 90 | 361 | 5 |
| 7 | 0.07 | 362 | 4.6 |
| 8 | 0.64 | 363 | 5 |
| 9 | 10 | 364 | 2 |
| 10 | 60 | 365 | 2 |
| 11 | 26 | 366 | 3 |
| 12 | 80 | 367 | 1.5 |
| 13 | 5 | 368 | 0.11 |
| 14 | 30 | 369 | 20 |
| 15 | 3 | 370 | 10 |
| 16 | 5 | 371 | 6 |
| 17 | 30 | 372 | 5 |
| 18 | 100 | 394 | 158 |
| 19 | 35 | 395 | 61 |
| 20 | 120 | 429 | 15 |
| 21 | 7 | 430 | 140 |
| 22 | 20 | 431 | 148 |
| 23 | 3 | 432 | 2.4 |
| 24 | 15 | 131 | 16 |
| 25 | 10 | 132 | 3 |
| 26 | 40 | 133 | 8 |
| 27 | 15 | 142 | 20 |
| 28 | 80 | 143 | 80 |
| 29 | 10 | 144 | 25 |
| 30 | 60 | 145 | 102 |
| 31 | 8 | 146 | 10 |
| 32 | 35 | 147 | 18 |
| 33 | 25 | 148 | 5 |
| 34 | 80 | 149 | 10 |
| 35 | 15 | 158 | 4 |
| 36 | 120 | 159 | 18 |
| 37 | 15 | 160 | 5 |
| 38 | 36 | 161 | 20 |
| 39 | 8 | 162 | 2 |
| 40 | 30 | 163 | 5 |
| 41 | 15 | 164 | 1.5 |
| 42 | 35 | 165 | 4 |
| 43 | 16 | 254 | 30 |
| 44 | 64 | 258 | 15 |
| 45 | 10 | 259 | 31 |
| 46 | 30 | 260 | 10 |
| 47 | 6.5 | 465 | 70 |
| 48 | 20 | 466 | 65 |
| 49 | 57 | 467 | 80 |
| 50 | 140 | 468 | 75 |
| 51 | 148 | 469 | 70 |
| 52 | 0.22 | 470 | 50 |
| 53 | 180 | 471 | 45 |
| 54 | 70 | 472 | 40 |
| 55 | 160 | 473 | 35 |
| 56 | 2.2 | 474 | 30 |
| 57 | 150 | 475 | 25 |
| 58 | 0.81 | 510 | 130 |
| 59 | 140 | 536 | 30 |
| 60 | 70 | 537 | 150 |
| 61 | 0.71 | 615 | 1007 |
| 62 | 8 | 616 | 473 |
| 63 | 2.2 | 617 | 884 |
| 64 | 10 | 618 | 200 |
| 65 | 3.2 | 619 | 496 |
| 66 | 9 | 620 | 138 |
| 67 | 0.07 | 621 | 330 |
| 68 | 0.33 | 622 | 231 |
| 69 | 1.8 | 623 | 200 |
| 70 | 7 | 624 | 800 |
| 71 | 0.2 | 625 | 850 |
| 72 | 8 | 626 | 1500 |
| 73 | 15 | 627 | 120 |
| 74 | 25 | 628 | 60 |
| 75 | 86 | 629 | 120 |
| 76 | 160 | 840 | 809 |
| 77 | 55 | 854 | 4.4 |
| 78 | 60 | 855 | 5.1 |
| 79 | 20 | 867 | 5.9 |
| 80 | 65 | 868 | 6.6 |
| 81 | 5.5 | 870 | 7.2 |
| 82 | 12 | 871 | 9.0 |
| 83 | 3.2 | 872 | 8.4 |
| 84 | 10 | 873 | 12 |
| 85 | 6 | 874 | 429 |
| 93 | 6.5 | 877 | 9.5 |
| 94 | 15 | 878 | 236 |
| 95 | 8 | 879 | 4.0 |
| 96 | 25 | 880 | 433 |
| 97 | 0.49 | 881 | 3.4 |
| 98 | 8 | 882 | 550 |
| 99 | 0.34 | 883 | 10.6 |
| 100 | 6 | 884 | 1256 |
| 101 | 5 | 887 | 2.65 |
| 109 | 287 | 888 | 292 |
| 110 | 35 | 903 | 0.7 |
| 111 | 140 | 904 | 0.4 |
| 112 | 38 | 913 | 3.6 |
| 113 | 150 | 914 | 0.4 |
| 114 | 20 | 915 | 33 |
| 115 | 45 | 916 | 37 |
| 116 | 15 | 918 | 40 |
| 117 | 30 | 919 | 1679 |
| 126 | 18 | 920 | 31 |
| 127 | 72 | 924 | 49 |
| 128 | 20 | 932 | 0.4 |
| 129 | 50 | 933 | 0.3 |
| 130 | 8 | 945 | 155 |

### KRAS G12C-GDP Exchange Test:

1. Serially 4x-diluted compounds (10 concentration points in total) were separately premixed with KRAS G12C-GDP (ICE, Kras 20191018) in a reaction buffer (25 mM Hepes PH7.4, 125 mM NaCl, 5 mM MgCl2, 0.01% Tween20, and 0.1% BSA) in the reaction wells for 1 h.
2. A mixture of SOS (Pharmaron, ZZY-20190823), cRAF (Pharmaron, ZZY-20190823), GTP (Sigma, A6885-100MG), MAb Anti 6HIS-d2/MAb Anti GST-Eu (Cisbio, 61HISDLB/61GSTKLB) was added for a catalyzed reaction for 2 h.
3. The fluorescence signals of the emitted light at 615 nm and 665 nm under 320 nm excitation light were read by Biotek Microplate Reader (Synergy4).
4. The IC50 (median inhibitory concentration) of the compound was obtained by the following nonlinear fitting formula: Y=Bottom + (Top-Bottom)/(1+10^((LogIC50-X)*Hill Slope)), and the data were analyzed by Graphpad 6.0 software.

| Ex. No. | IC₅₀ (nM) | Ex. No. | IC₅₀ (nM) |
|---|---|---|---|
| 1 | 20.31 | 840 | 4.52 |
| 2 | 10.59 | 854 | 0.59 |
| 3 | 13.85 | 873 | 0.79 |
| 10 | 8.0 | 877 | 0.71 |
| 17 | 1.65 | 879 | 0.59 |
| 73 | 1.34 | 881 | 0.71 |
| 254 | 8.90 | 883 | 0.49 |
| 286 | 9.91 | 887 | 1.02 |
| 358 | 2.40 | 895 | 1.29 |
| 394 | 1.44 | 903 | 0.89 |
| 395 | 1.32 | 904 | 0.93 |
| 465 | 2.23 | | |

### Pharmacokinetic experiment in SD rats:

1. SPF male rats were randomly divided into groups. The compounds to be tested were separately administered by intravenous injection and oral gavage, with 3 animals for each compound to be tested in each mode of administration. The administration solvent was 5% DMSO + 10% Solutol + 85% normal saline or 85% PBS, and the substances to be tested were dissolved in the solvent to obtain clear solutions. Administration concentration and volume: 1) a single intravenous injection of 0.6 mg/mL of the compound to be tested, with an administration volume of 5 mL/kg and an administration dosage of 3 mg/kg; and 2) a single oral gavage of 1 mg/mL of the compound to be tested, with an administration volume of 10 mL/kg and an administration dosage of 10 mg/kg. The rats were fasted overnight (10-14 h) before administration and fed 4 h after administration.
2. Blood was collected via the jugular vein or by other appropriate methods at 200 µL per time point and anticoagulated with K2-EDTA, and after collection, the blood was placed on ice and centrifuged for plasma within 1 h (centrifugation conditions: 6800 g, 6 min, 2-8°C). The points for blood sampling from the animals in the intravenous injection group were respectively: before administration, and 5 min, 15 min, 30 min, 1 h, 2 h, 4 h, 8 h, and 24 h after administration; and the points for blood sampling from the animals in the oral administration group were respectively: before administration, and 15 min, 30 min, 1 h, 2 h, 4 h, 6 h, 8 h, and 24 h after administration.
3. The blood concentration was detected, and the pharmacokinetic parameter AUC(0-t) was calculated by Phoenix WinNonlin based on the blood concentration data at various time points. When the pharmacokinetic parameters were calculated, the BLQ before Cmax (including "No peak") was calculated as 0; and BLQ after Cmax (including "No peak") was not involved in the calculation. The formula of calculating the oral bioavailability was: F%=AUC0-tₒᵣₐₗ/AUC0-t_{IV}^{∗}(10/3)^{∗}100%.

| Ex. No. | Oral bioavailability, F% |
|---|---|
| 358 | 2.35% |
| 367 | 11.57% |
| 879 | 16.9% |
| 887 | 25.85% |
| 903 | 28.6% |
| 904 | 30.28% |
| 914 | 28.49% |
| ***Example 229 of WO 2020*/*146613 A1*** | 22.69% |

## Claims

1. A compound of formula (I) or a pharmaceutically acceptable salt, tautomer, prodrug or stereoisomer thereof, wherein the compound of formula (I) is: 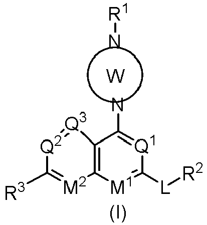 wherein:
ring W is a 4- to 12-membered saturated or partially saturated monocyclic, bridged cyclic, or spirocyclic ring, wherein the saturated or partially saturated monocyclic ring is optionally additionally substituted with one or more R⁴,
wherein
R⁴ is selected from: oxo, alkyl, alkenyl, alkynyl, cycloalkyl, aryl, heteroaryl, heterocyclyl, cyano, nitro, -C(O)OR⁵, or -C(O)N(R⁵)₂, among which the alkyl is unsubstituted or substituted with one or more of cyano, halo, -OR⁵, -N(R⁵)₂, or heteroaryl, wherein each R⁵ is independently hydrogen or alkyl;
R¹ is -L'-T,
wherein
L¹ is -O-, -S-, -NR^{a}-, -C(O)-, -SO₂-, -SO-, -C(=NR^{a})-, -C(O)-O-, -OC(O)-, -C(O)-NR^{a}-, or -NR^{a}C(O)-,
T is -CR^{a}=CR^{b}R^{c}, -C=CR^{b}, alkyl, cycloalkyl, aryl, heteroaryl, or heterocyclyl, and each of the alkyl, cycloalkyl, aryl, heteroaryl, or heterocyclyl is unsubstituted or substituted with one or more of oxo, halogen, hydroxyl, alkyl, haloalkyl, hydroxyalkyl, alkoxy, CN, nitro, or NR^{x}R^{y};
wherein
R^{a} is hydrogen, deuterium, cyano, halogen, hydroxyl, alkyl, haloalkyl, hydroxyalkyl, aryl, heteroaryl, or heterocyclyl;
R^{b} and R^{c} are each independently hydrogen, deuterium, cyano, halogen, -C(O)OR^{x}, alkyl, cycloalkyl, aryl, heteroaryl, or heterocyclyl, among which each of the alkyl, cycloalkyl, aryl, heteroaryl, or heterocyclyl is unsubstituted or substituted with one or two of oxo; halogen; hydroxyl; alkyl; haloalkyl; hydroxyalkyl; alkoxy; CN; nitro; NR^{x}R^{y}; aryl which is unsubstituted or substituted with alkyl, hydroxyl, or halogen; heteroaryl which is unsubstituted or substituted with alkyl, hydroxyl, or halogen; or heterocyclyl which is unsubstituted or substituted with alkyl, hydroxyl, or halogen,
or when T is -CR^{a}=CR^{b}R^{c}, R^{a} and R^{b}, or R^{a} and R^{c}, together with the carbon atom to which they are attached, form an unsaturated 5- to 8-membered ring which is unsubstituted or substituted with one or two of oxo, hydroxyl, halogen, alkyl, hydroxyalkyl, haloalkyl, or alkoxy;
R^{x} and R^{y} are each independently hydrogen or alkyl;
Q', Q², and Q³ are each independently N or CR¹¹, and M¹ and M² are each independently N or CR¹², provided that at least one of Q¹ and M¹ is N;
wherein
R¹¹ and R¹² are each independently hydrogen, halogen, cyano, nitro, alkyl, alkenyl, alkynyl, cycloalkyl, aryl, heteroaryl or heterocyclyl, -OR^{d}, -C(O)R^{d}, -CO₂R^{d}, -CONR^{d}R^{e}, or -NR^{d}R^{e}, wherein the alkyl, alkenyl, alkynyl, cycloalkyl, aryl, heteroaryl, and heterocyclyl are each independently substituted with one or more of oxo, halogen, hydroxyl, alkoxy, alkyl, cycloalkyl, nitro, cyano, and -NR^{d}R^{e}, wherein R^{d} and R^{e} are each independently hydrogen, alkyl, C₃-C₆ cycloalkyl, hydroxyalkyl, haloalkyl, and alkoxyalkyl;
L is a single bond, -O-, -S-, -NR^{a}-, -O-CH₂-, -S-CH₂-, -NR^{a}-CH₂-, -CH₂-O-, -CH₂-S-, -CH₂-NR^{a}-, - C(O)-, -SO₂-, -SO-, -C(O)-O-, -OC(O)-, -C(O)-NR^{a}-, or -NR^{a}C(O)-;
R² is alkyl, alkenyl, alkynyl, cycloalkyl, aryl, heteroaryl, or heterocyclyl, wherein the alkyl, alkenyl, alkynyl, cycloalkyl, aryl, heteroaryl, and heterocyclyl are each independently unsubstituted or substituted with one or more of halogen, cyano, alkyl, haloalkyl, hydroxyalkyl, alkoxyalkyl, oxo, -OR^{d}, -C(O)R^{d}, - CO₂R^{d}, -CONR^{d}R^{e}, -NR^{d}R^{e}, -CH₂ NR^{d}R^{e}, cycloalkyl, cycloalkylalkyl, aryl, heteroaryl, and heterocyclyl, wherein R^{d} and R^{e} are each independently hydrogen, alkyl, hydroxyalkyl, haloalkyl, and alkoxyalkyl;
R³ is cycloalkyl, heterocyclyl, aryl, or heteroaryl, provided that when M¹, Q', and Q² are all N, R³ is a non-aromatic fused bicyclic group, non-aromatic fused bicyclic heterocyclyl, or bicyclic heteroaryl, R³ is unsubstituted or substituted with one or more of the following groups: oxo, halogen, cyano, -OR^{d}, -C(O)R^{d}, -CO₂R^{d}, -CONR^{d}R^{e}, -NR^{d}COR^{e}, -NR^{d}R^{e}, -S(O)₂NR^{d}R^{e}, alkyl, alkenyl, alkynyl, cycloalkyl, aryl, heteroaryl, and heterocyclyl, wherein the alkyl, alkenyl, alkynyl, cycloalkyl, aryl, heteroaryl, and heterocyclyl are each independently substituted with halogen, alkyl, cyano, carbamoyl, alkoxy, hydroxyl, cycloalkyl, and heteroaryl, wherein R^{d} and R^{e} are each independently hydrogen, alkyl, C₃-C₆ cycloalkyl, hydroxyalkyl, haloalkyl, alkoxyalkyl, alkenyl, or cycloalkyl.

2. The compound or the pharmaceutically acceptable salt, tautomer, prodrug or stereoisomer thereof according to claim 1, wherein L¹ is -C(O)- or -SO₂-, and T is -CR^{a}=CR^{b}R^{c} or -C=CR^{b}, wherein R^{a} is hydrogen, deuterium, cyano, halogen, hydroxyl, or alkyl, R^{b} and R^{c} are each independently hydrogen; halogen; unsubstituted alkyl; alkyl substituted with hydroxyl, halogen, NR^{x}R^{y} or heterocyclyl; unsubstituted aryl or heteroaryl; and aryl or heteroaryl substituted with alkyl, hydroxyl or halogen, wherein R^{x} and R^{y} are each independently hydrogen or alkyl.

3. The compound or the pharmaceutically acceptable salt, tautomer, prodrug or stereoisomer thereof according to claim 1, wherein L is -O-CH₂- or -O-.

4. The compound or the pharmaceutically acceptable salt, tautomer, prodrug or stereoisomer thereof according to claim 3, wherein R² is heterocyclyl, and the heterocyclyl is unsubstituted or substituted with one or more of halogen and alkyl.

5. The compound or the pharmaceutically acceptable salt, tautomer, prodrug or stereoisomer thereof according to claim 4, wherein L-R² is 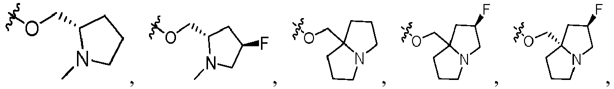 or

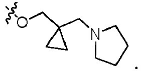

6. The compound or the pharmaceutically acceptable salt, tautomer, prodrug or stereoisomer thereof according to any one of claims 1-5, wherein R³ is aryl, and the aryl is phenyl or naphthyl which is unsubstituted or substituted with 1, 2, or 3 substituents of halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; - NR^{d}COR^{e} in which R^{d} and R^{e} are each independently hydrogen or alkyl; alkyl which is unsubstituted or substituted with halogen, cycloalkyl, hydroxyl or alkoxy; cycloalkyl which is unsubstituted or substituted with alkyl, cyano or carbamoyl; alkynyl; -NR^{d}R^{e} in which R^{d} and R^{e} are each independently hydrogen or alkyl; or heteroaryl.

7. The compound or the pharmaceutically acceptable salt, tautomer, prodrug or stereoisomer thereof according to any one of claims 1-5, wherein R³ is partially hydrogenated naphthyl which is unsubstituted or substituted with hydroxyl, alkyl, hydroxyalkyl, haloalkyl or halogen.

8. The compound or the pharmaceutically acceptable salt, tautomer, prodrug or stereoisomer thereof according to any one of claims 1-5, wherein R³ is heteroaryl which is unsubstituted or substituted with 1, 2, or 3 substituents of oxo, halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; -NR^{d}COR^{e} in which R^{d} and R^{e} are each independently hydrogen, alkyl, or alkenyl; alkyl which is unsubstituted or substituted with halogen, cycloalkyl, hydroxyl, or alkoxy; cycloalkyl which is unsubstituted or substituted with alkyl, cyano or carbamoyl; alkynyl; or -NR^{d}R^{e} in which R^{d} and R^{e} are each independently hydrogen or alkyl.

9. The compound or the pharmaceutically acceptable salt, tautomer, prodrug or stereoisomer thereof according to claim 8, wherein the heteroaryl is monocyclic heteroaryl selected from thiophene, thiazole, pyrazole, pyridine, or pyrimidine; or bicyclic heteroaryl selected from 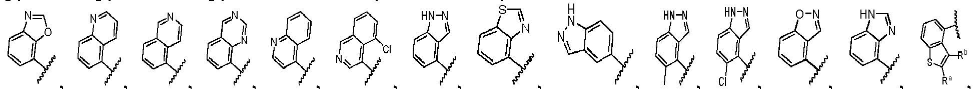 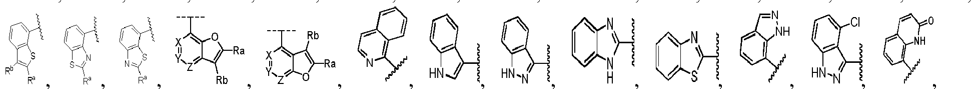 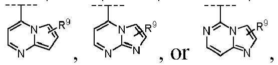 in which R^{a} and R^{b} are independently hydrogen, halogen, or alkyl, or R^{a} and R^{b} are connected to form a substituted or unsubstituted C₃-C₆ cycloalkyl, wherein the heteroaryl is unsubstituted or substituted as described above.

10. The compound or the pharmaceutically acceptable salt, tautomer, prodrug or stereoisomer thereof according to any one of claims 1-5, wherein R³ is non-aromatic fused bicyclic heterocyclyl which is unsubstituted or substituted with 1, 2, or 3 substituents of oxo, halogen; cyano; -OR^{d} in which R^{d} is hydrogen, alkyl, or haloalkyl; -CONR^{d}R^{e} in which R^{d} and R^{e} are each independently hydrogen, alkyl, or cycloalkyl; -NR^{d}COR^{e} in which R^{d} and R^{e} are each independently hydrogen, alkyl, or alkenyl; alkyl which is unsubstituted or substituted with halogen, cycloalkyl, hydroxyl, or alkoxy; cycloalkyl which is unsubstituted or substituted with alkyl, cyano or carbamoyl; alkynyl; or -NR^{d}R^{e} in which R^{d} and R^{e} are each independently hydrogen or alkyl.

11. The compound or the pharmaceutically acceptable salt, tautomer, prodrug or stereoisomer thereof according to claim 10, wherein R³ is 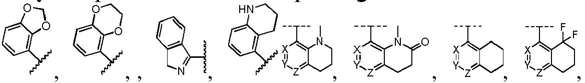 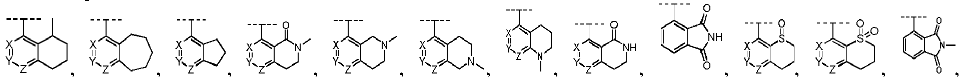 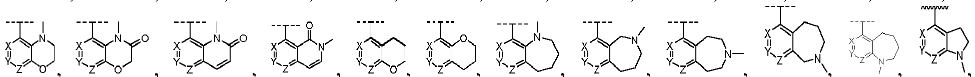 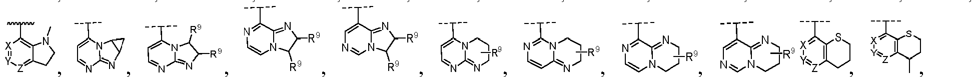 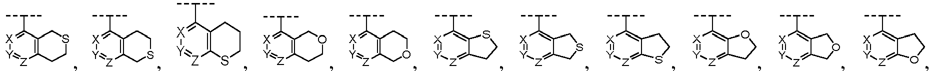 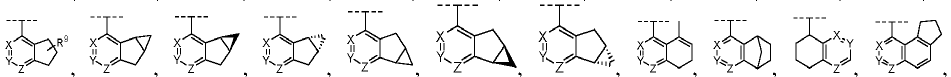 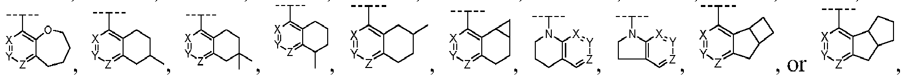 which is unsubstituted or substituted with 1, 2, or 3 substituents of oxo, halogen; hydroxyl, alkoxy, and alkyl; preferably, the substituent is oxo, halogen, hydroxyl, methoxy, or methyl, wherein X, Y, and Z are each independently N or CR⁹ in which R⁹ is hydrogen, hydroxyl, cyano, alkyl, haloalkyl, halogen, hydroxyalkyl, alkoxyalkyl, or alkylsulfonyl.

12. The compound or the pharmaceutically acceptable salt, tautomer, prodrug or stereoisomer thereof according to claim 1, wherein the compound is 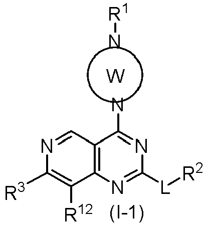 wherein:
R³ is 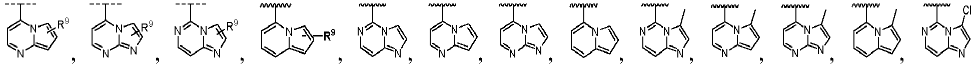 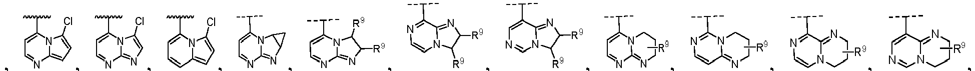 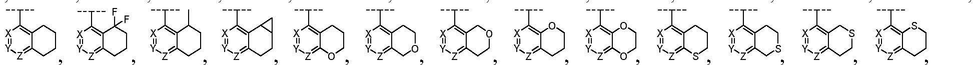 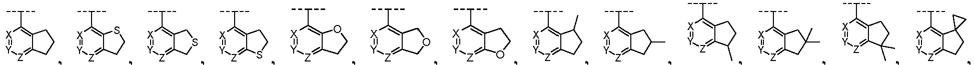 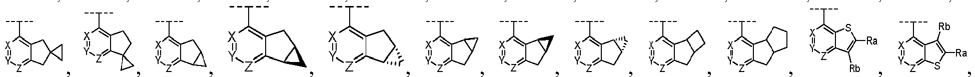 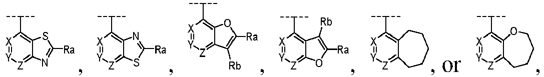 wherein X, Y, and Z are selected from N or CR⁹, and R^{a} and R^{b} are independently hydrogen, halogen, or alkyl, or R^{a} and R^{b} are connected to form a substituted or unsubstituted C₃-C₆ cycloalkyl, and the remaining variables are as defined for formula (I).

13. The compound or the pharmaceutically acceptable salt, tautomer, prodrug or stereoisomer thereof according to claim 1, wherein the compound is 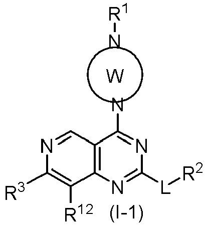 wherein L-R² is 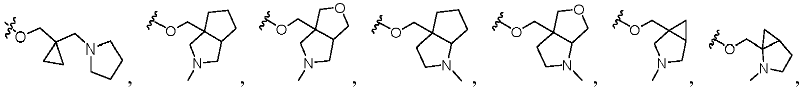 or 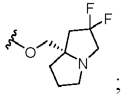 and R³ is preferably

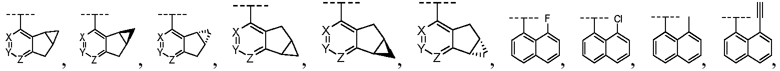

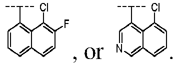

14. The compound or the pharmaceutically acceptable salt, tautomer, prodrug or stereoisomer thereof according to claim 1, wherein the compound is 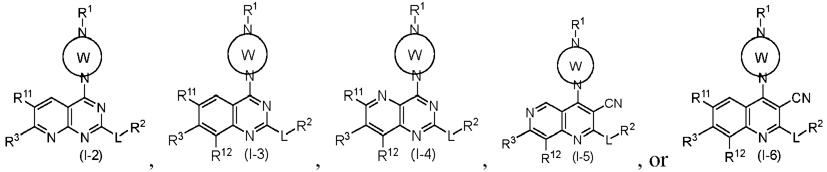 wherein:
R³ is 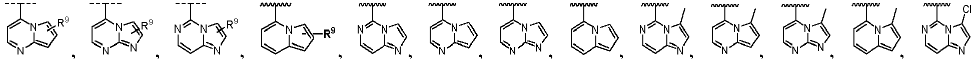 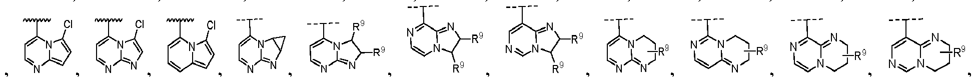 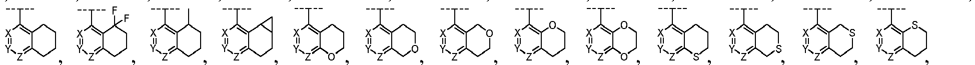 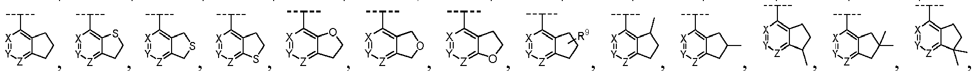 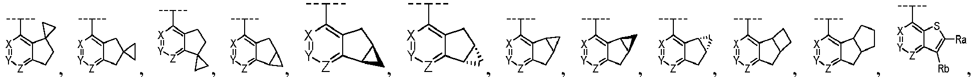 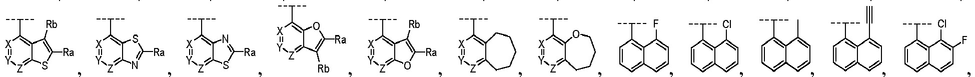 or 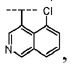 in which R^{a} and R^{b} are independently hydrogen, halogen, or alkyl, or R^{a} and R^{b} are connected to form a substituted or unsubstituted C₃-C₆ cycloalkyl.

15. The compound or the pharmaceutically acceptable salt, tautomer, prodrug or stereoisomer thereof according to any one of claims 1-14, wherein R¹-W is 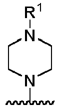 in which the piperazine ring is optionally additionally substituted with one or more R⁴.

16. The compound or the pharmaceutically acceptable salt, tautomer, prodrug or stereoisomer thereof according to any one of claims 1-14, wherein R¹-W is

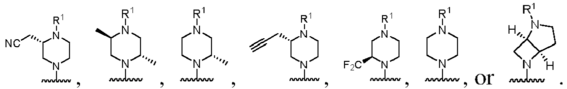

17. The compound or the pharmaceutically acceptable salt, tautomer, prodrug or stereoisomer thereof according to claim 15 or 16, wherein R¹ is 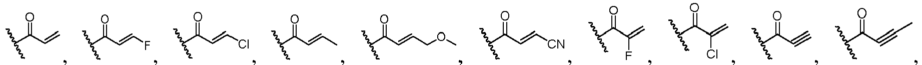 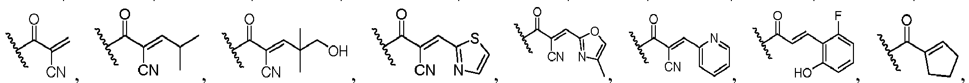 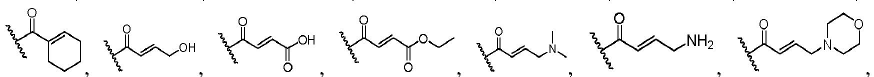 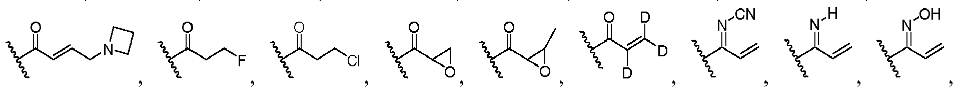 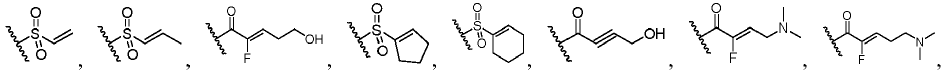 or

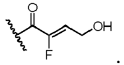

18. The compound or the pharmaceutically acceptable salt, tautomer, prodrug or stereoisomer thereof according to claim 1, wherein R³ is

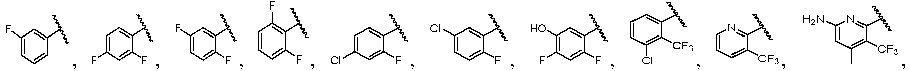

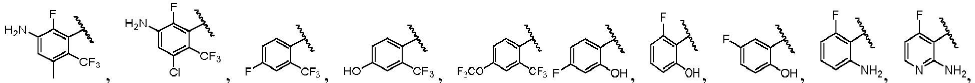

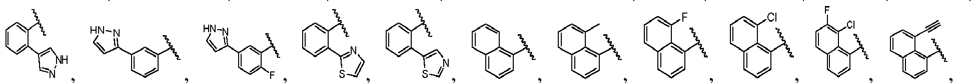

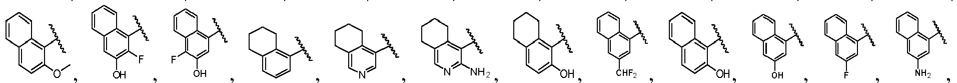

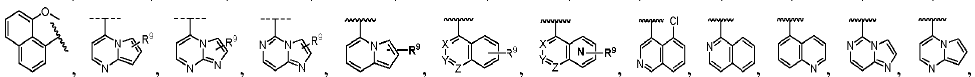

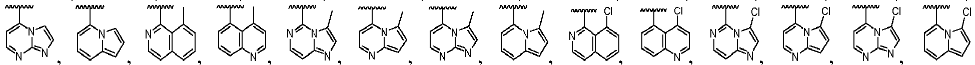

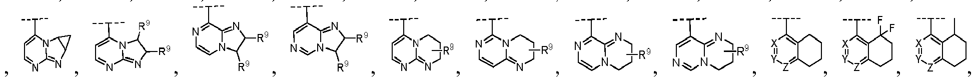

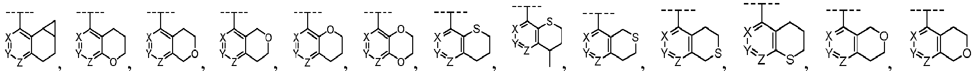

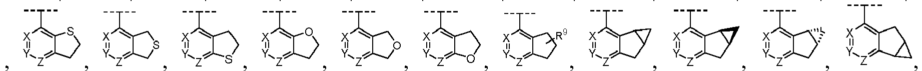

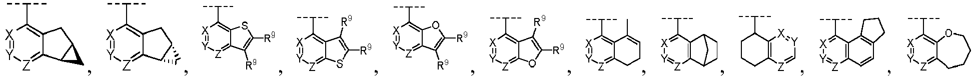

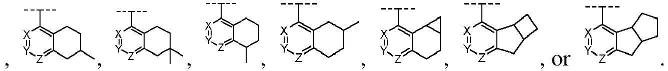

19. The compound or the pharmaceutically acceptable salt, tautomer, prodrug or stereoisomer thereof according to claim 1, wherein R¹¹ is hydrogen, nitro, hydroxyl, halogen, cyano, alkyl, haloalkyl, alkoxy, or alkoxyalkyl.

20. The compound or the pharmaceutically acceptable salt, tautomer, prodrug or stereoisomer thereof according to claim 1, wherein R¹² is hydrogen, halogen, C₁-C₆ alkyl, C₃-C₆ cycloalkyl, heterocyclyl, -OR^{d}, C₁-C₆ haloalkyl, aryl, or heteroaryl, wherein the aryl and heteroaryl are each unsubstituted or substituted with one or more of C₁-C₃ alkyl, halogen, C₁-C₃ haloalkyl, and C₃-C₆ cycloalkyl, and R^{d} is hydrogen, alkyl, C₃-C₆ cycloalkyl, hydroxyalkyl, or haloalkyl.

21. The compound or the pharmaceutically acceptable salt, tautomer, prodrug or stereoisomer thereof according to any one of claims 1-20, wherein the compound is

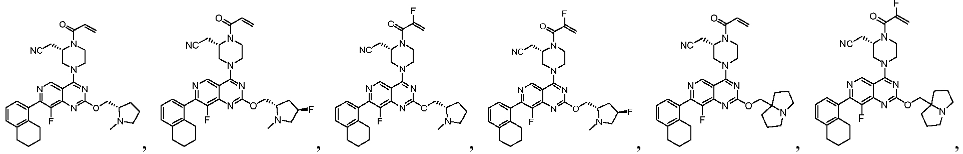

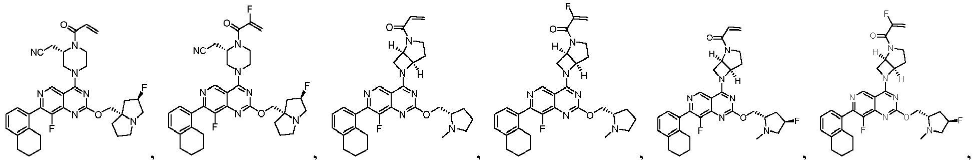

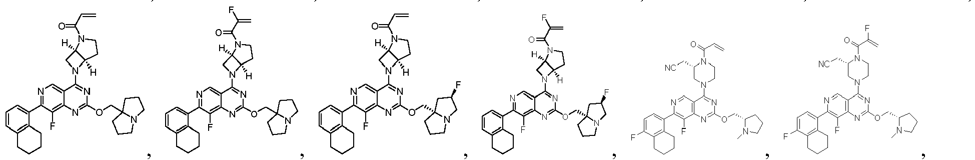

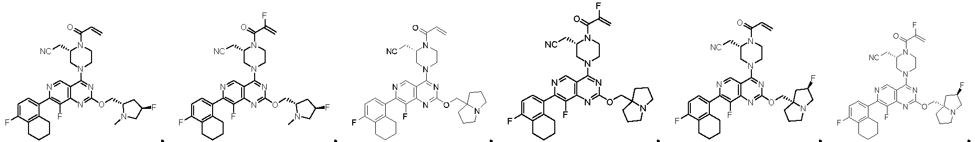

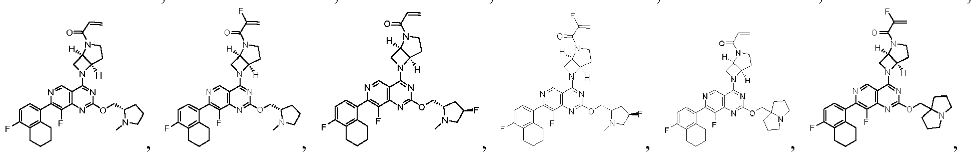

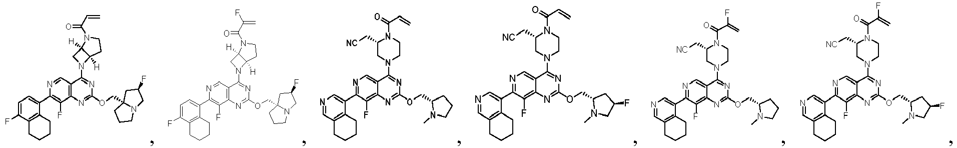

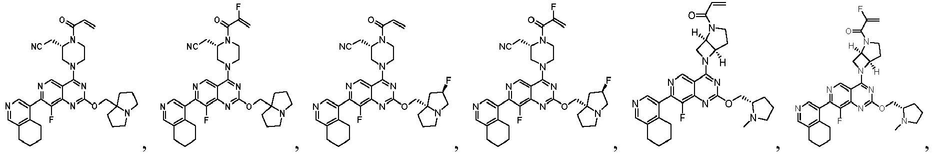

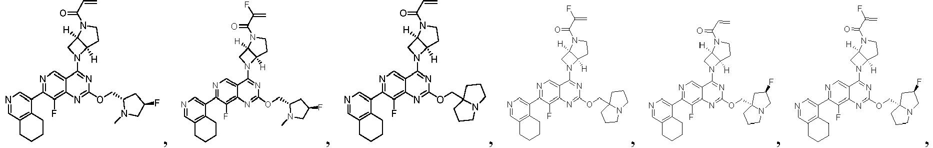

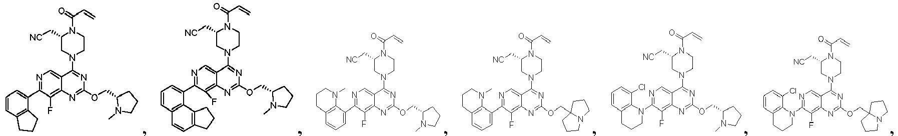

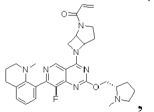

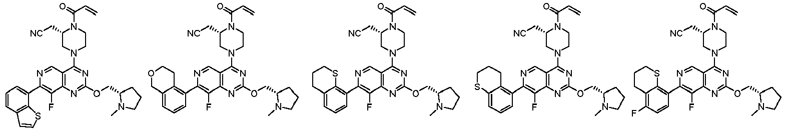

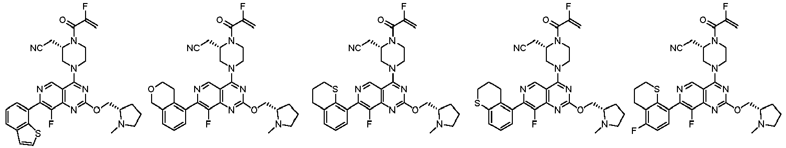

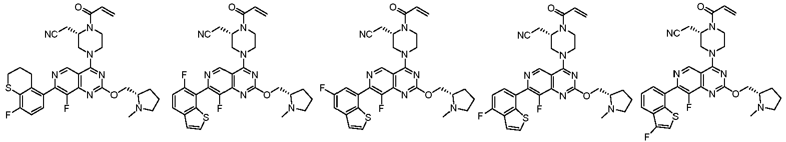

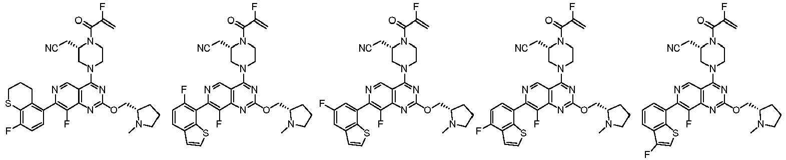

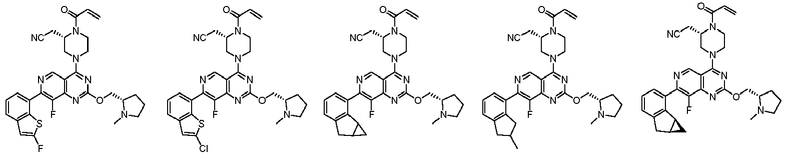

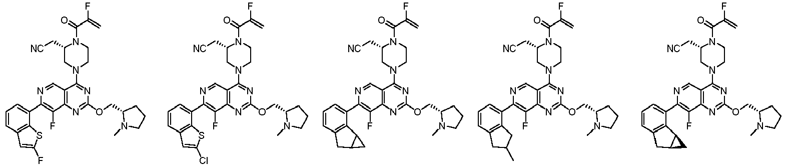

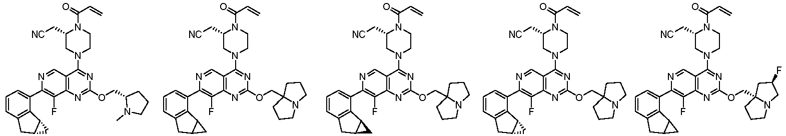

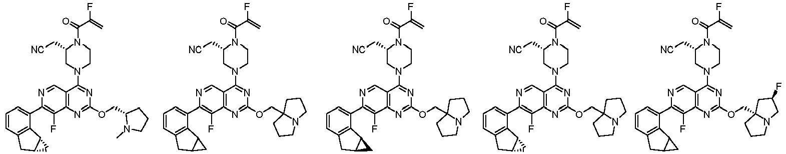

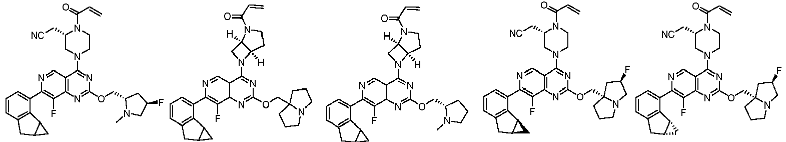

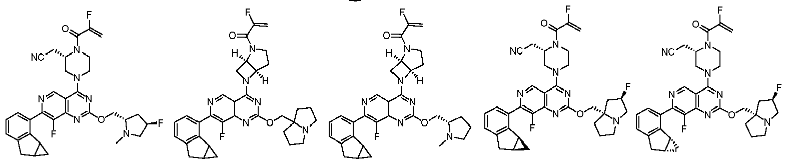

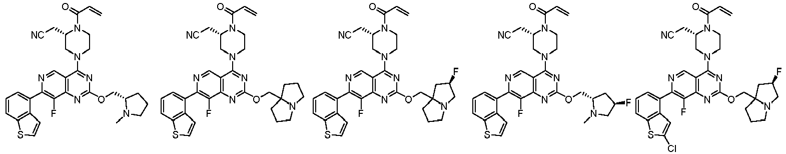

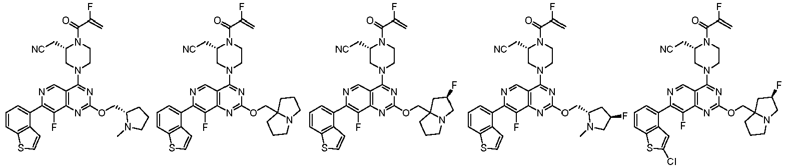

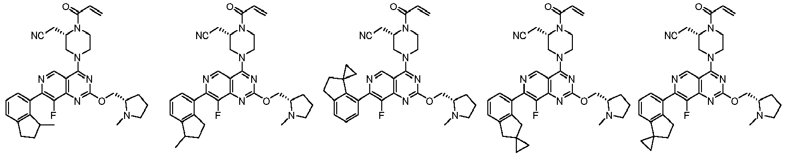

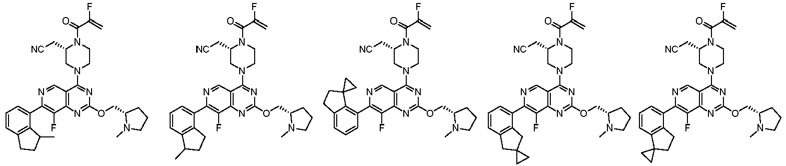

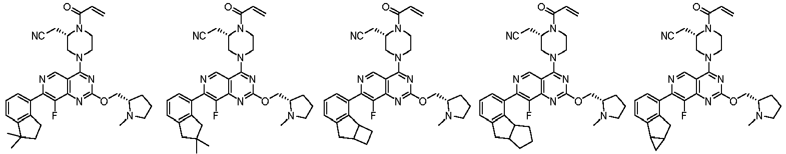

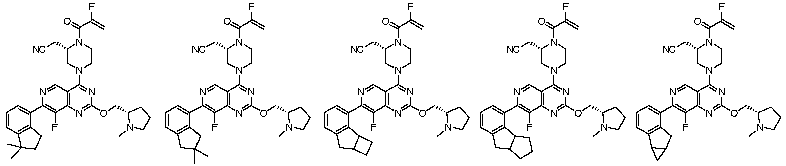

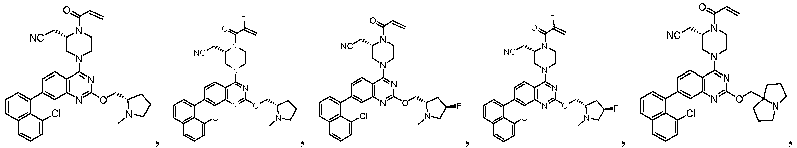

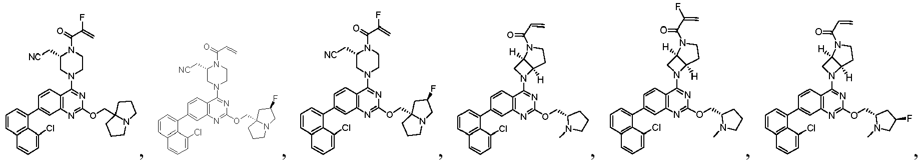

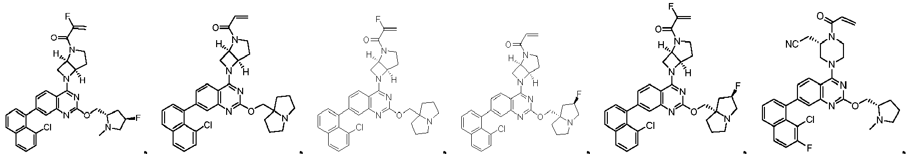

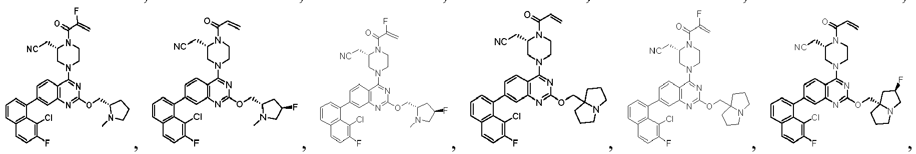

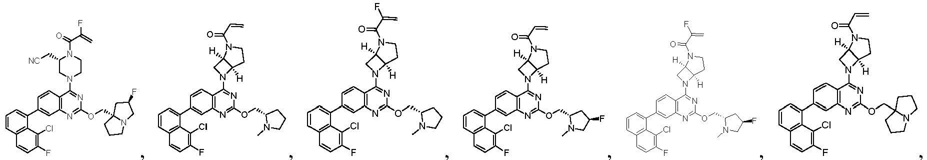

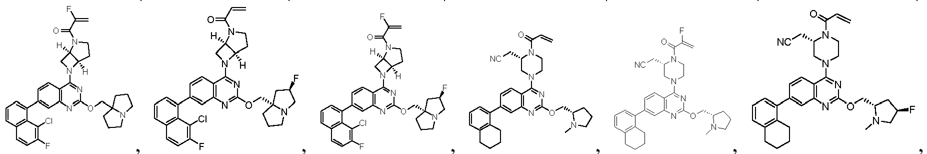

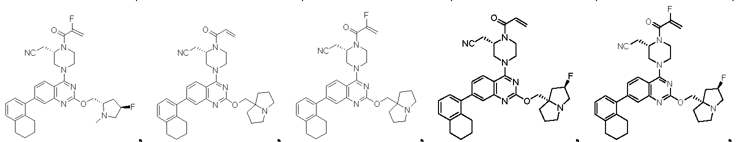

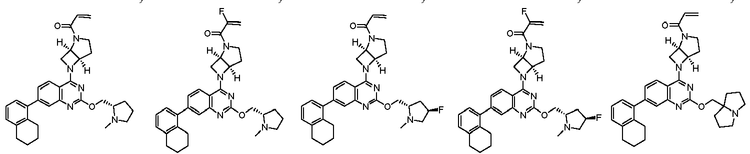

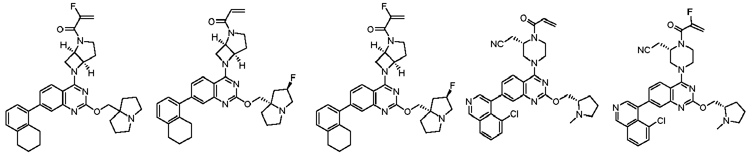

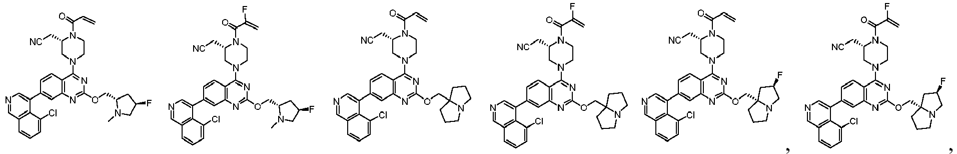

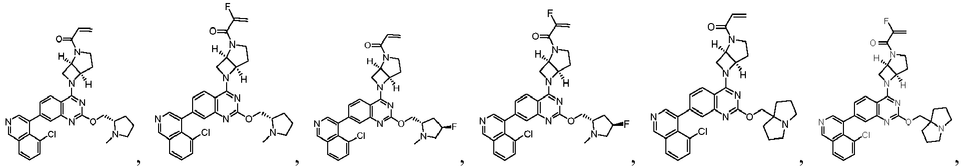

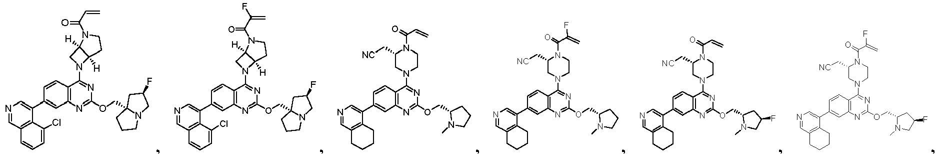

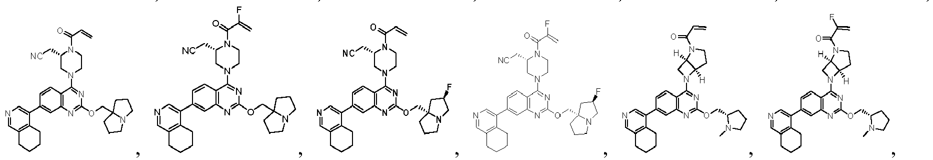

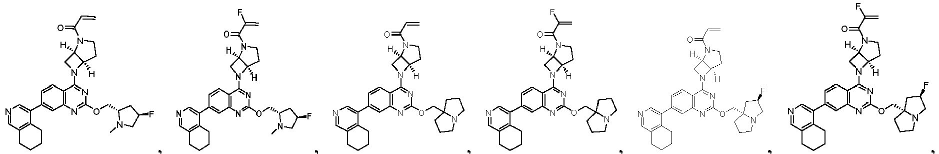

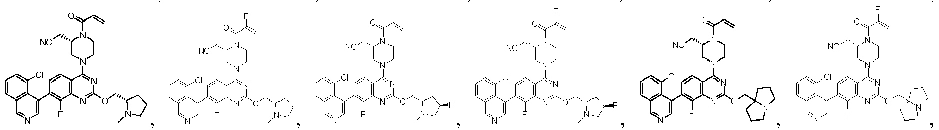

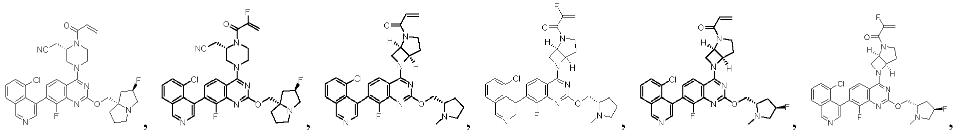

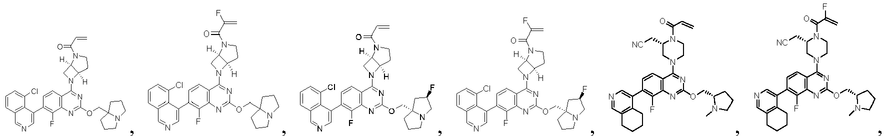

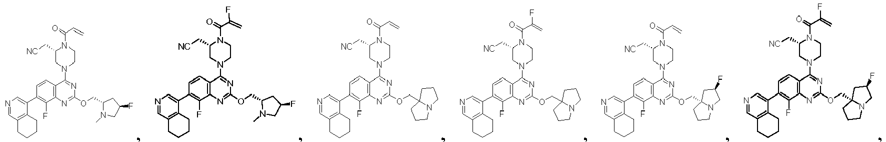

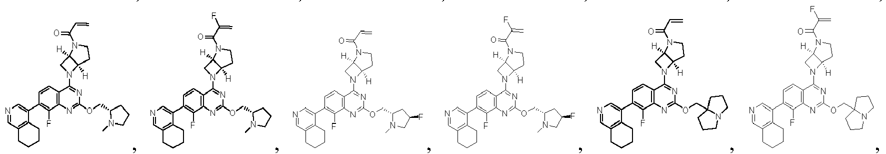

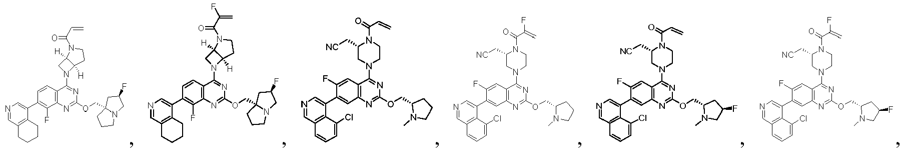

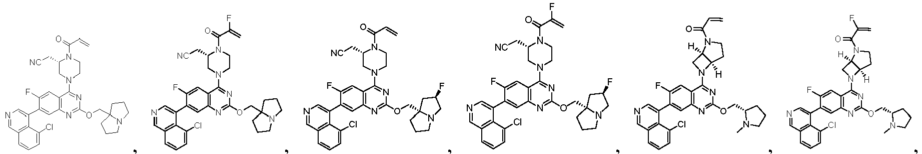

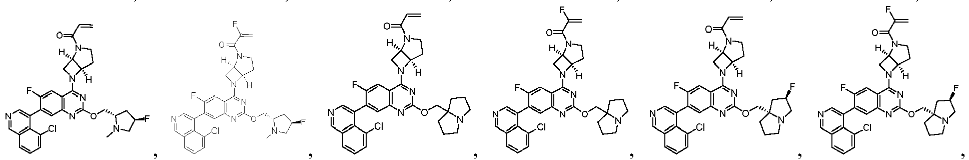

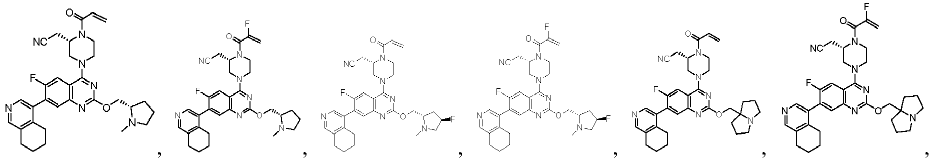

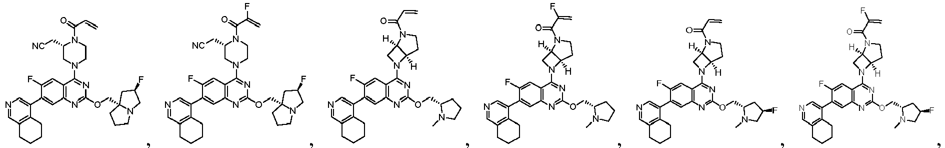

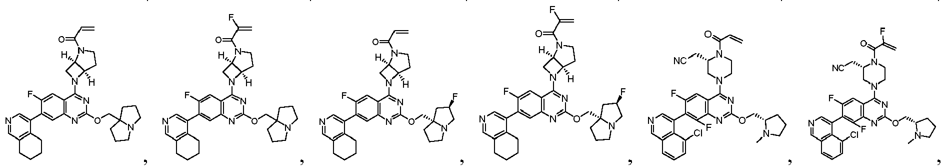

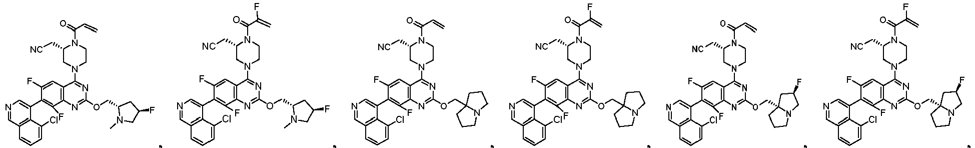

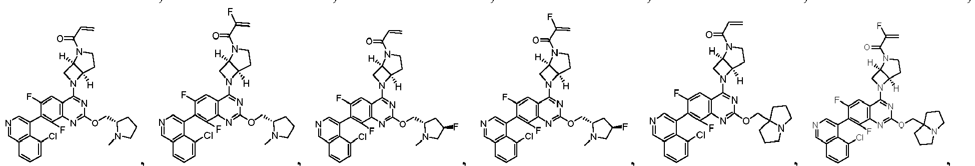

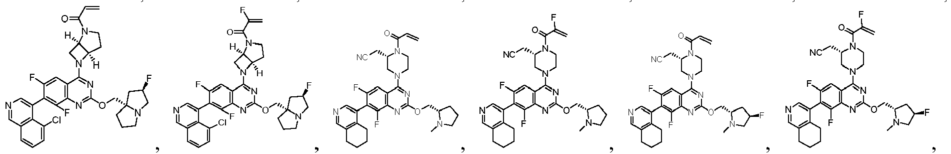

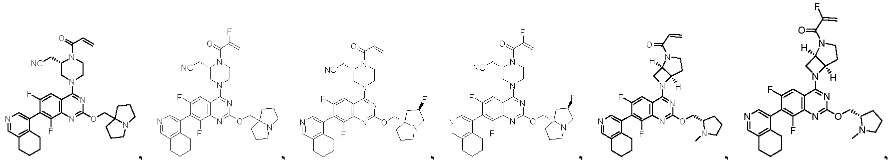

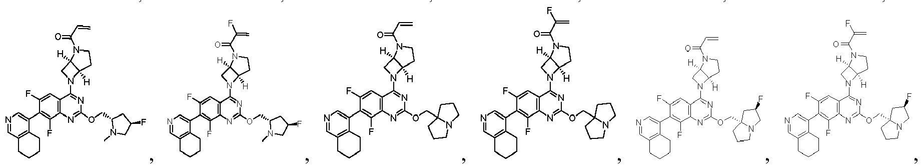

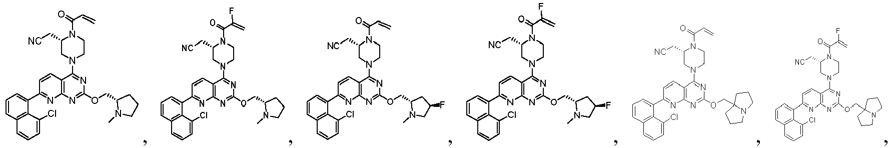

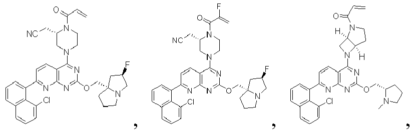

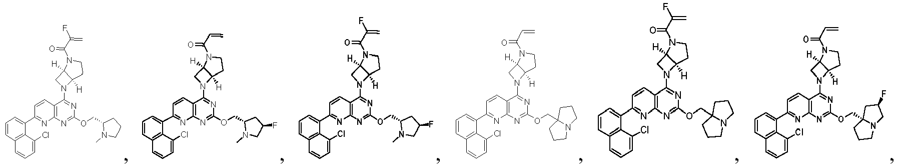

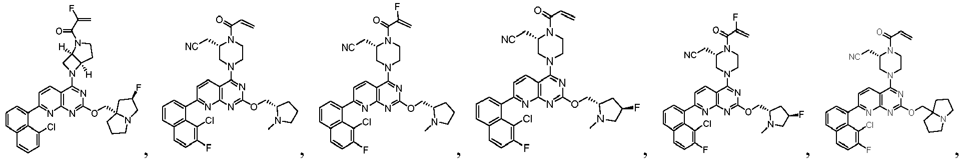

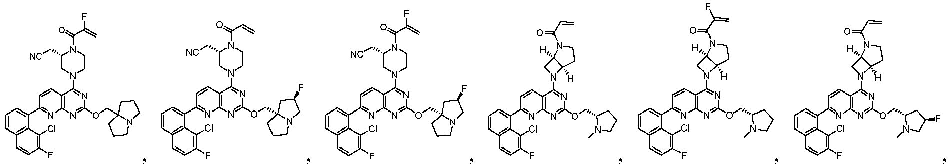

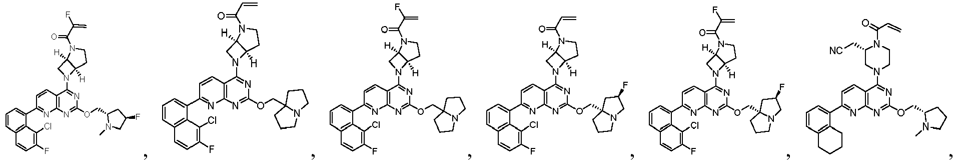

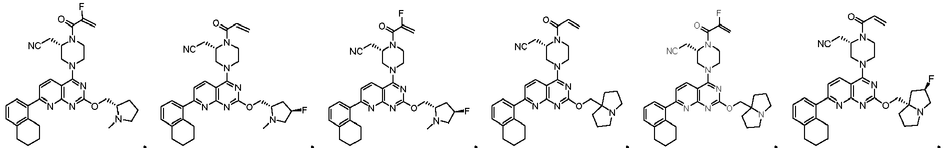

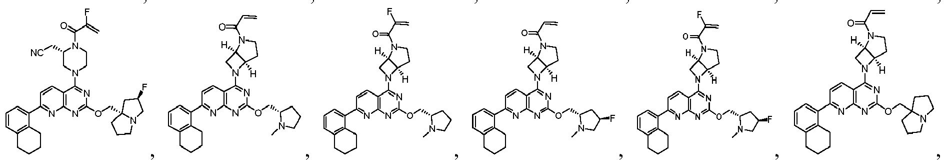

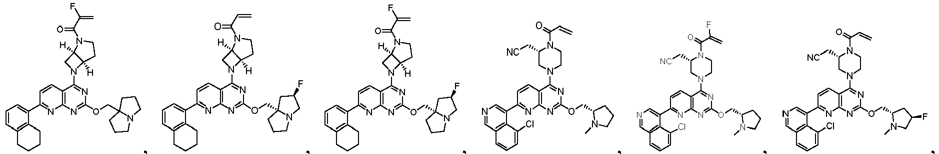

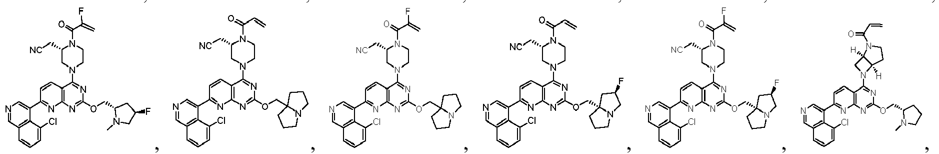

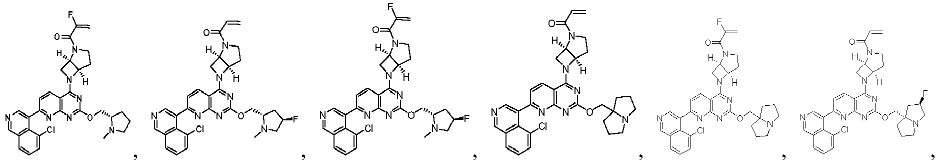

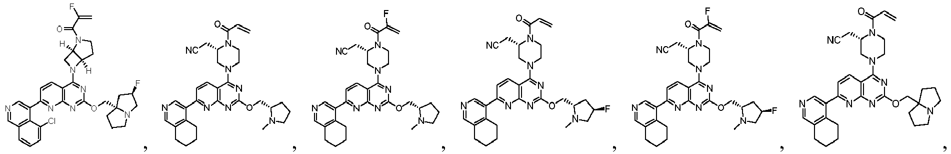

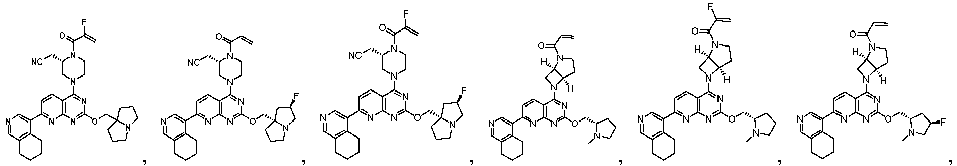

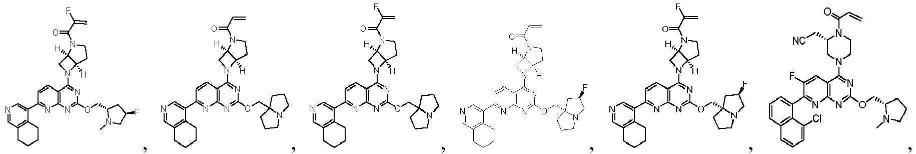

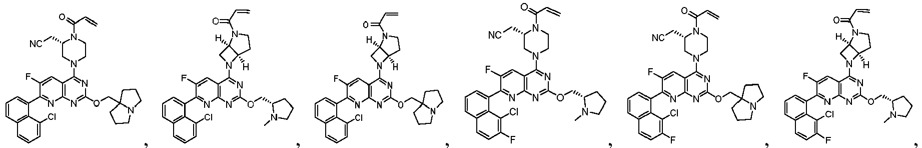

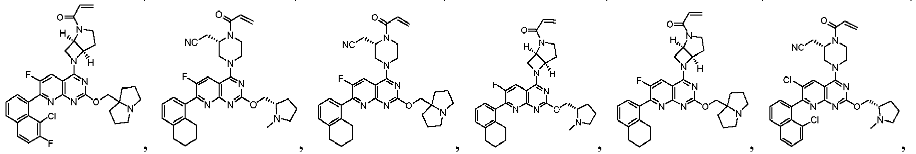

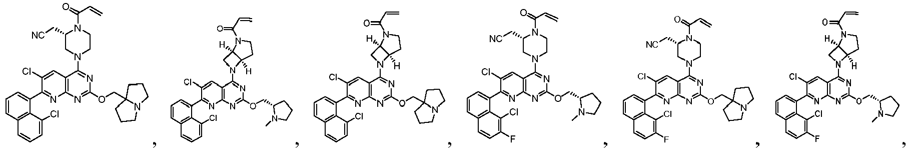

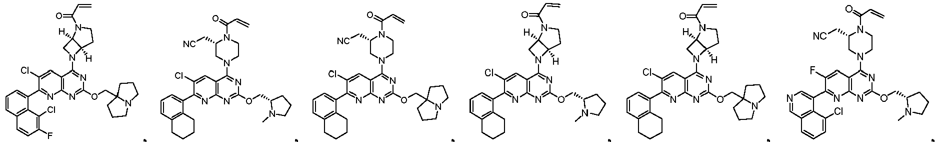

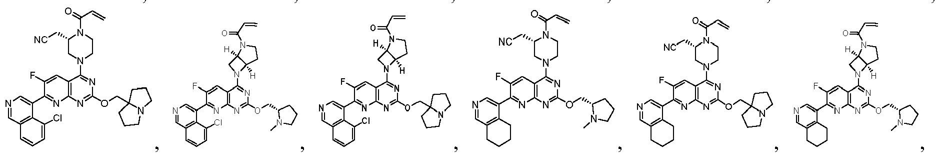

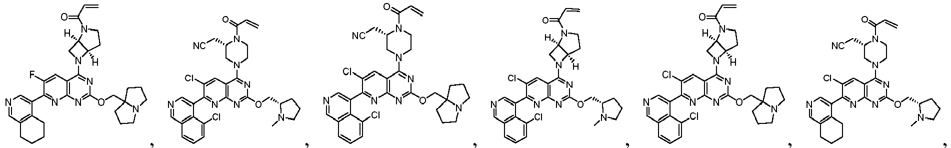

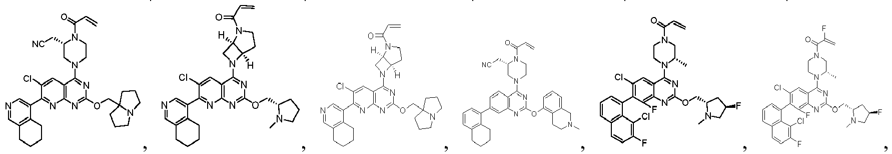

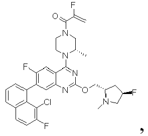

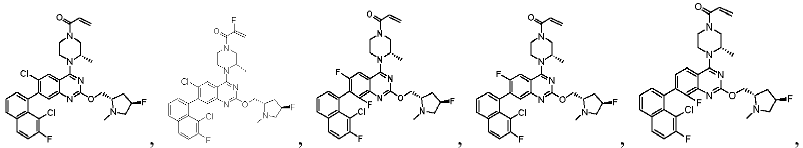

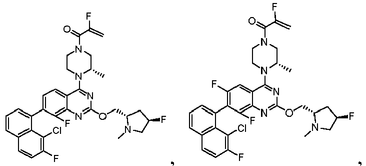

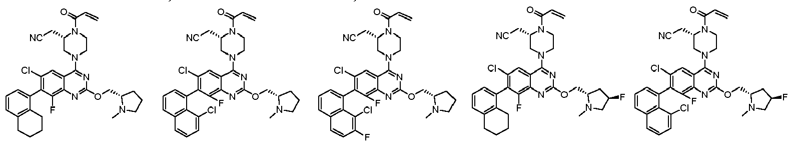

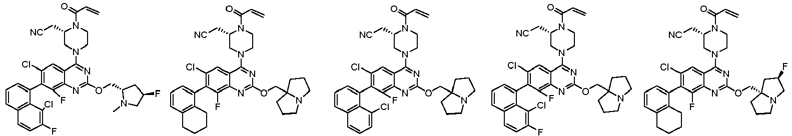

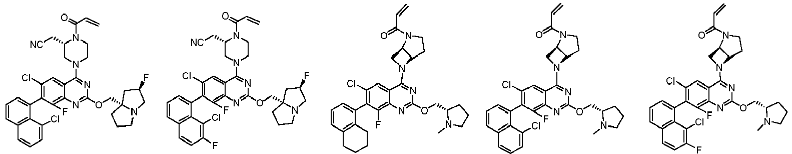

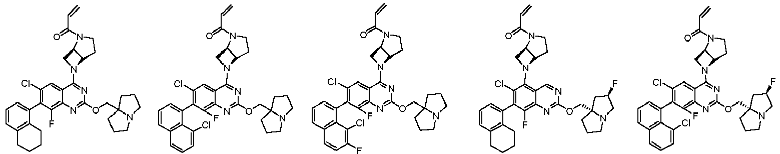

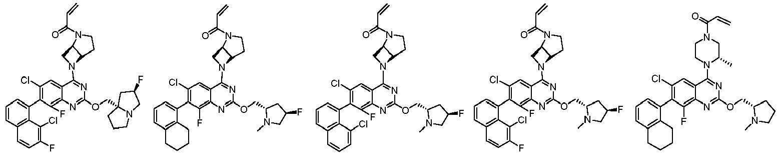

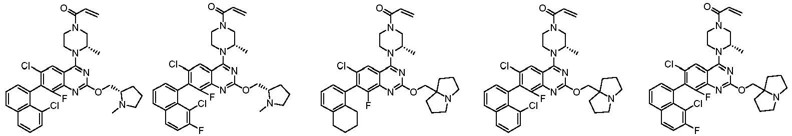

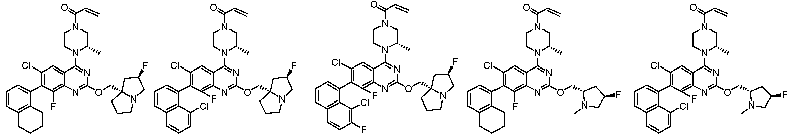

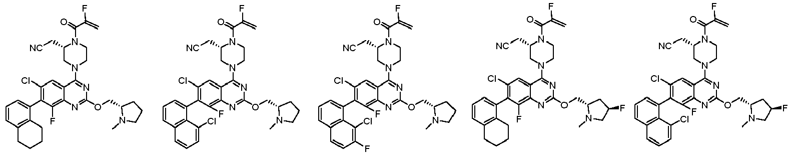

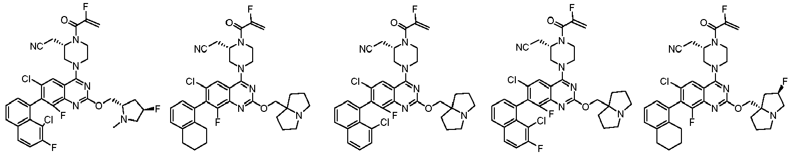

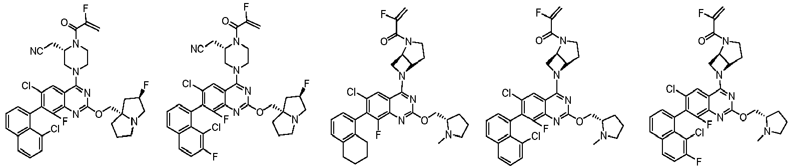

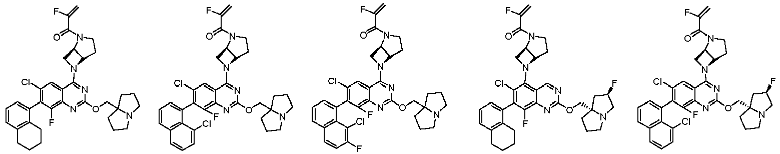

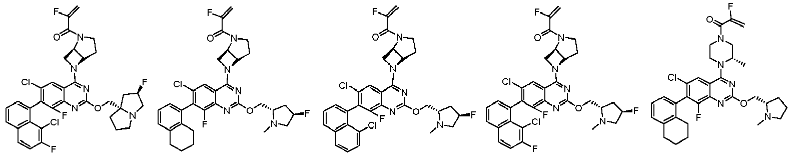

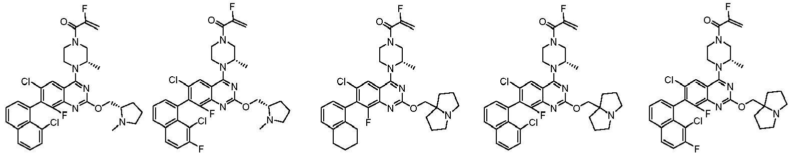

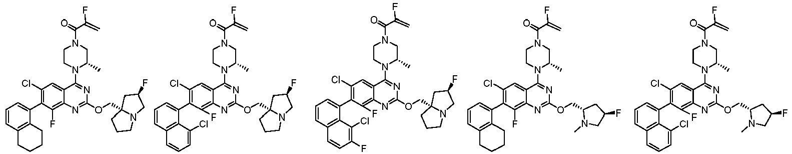

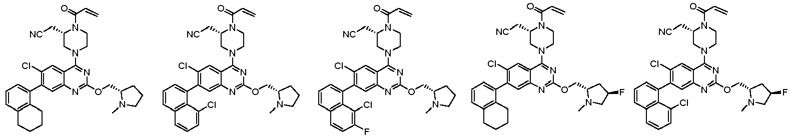

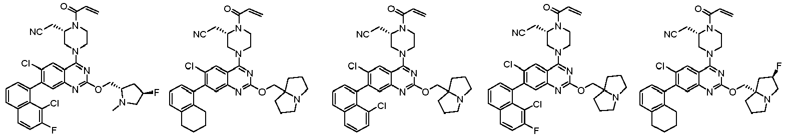

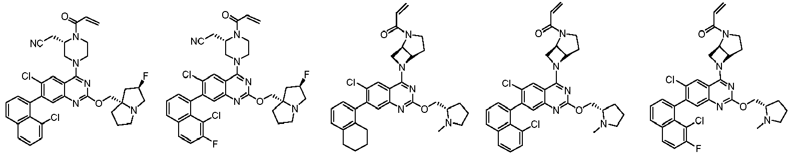

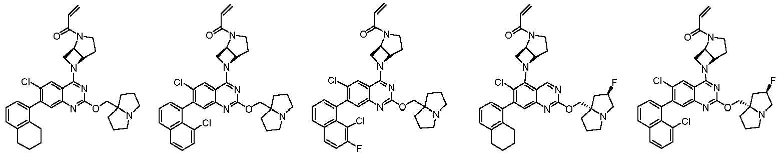

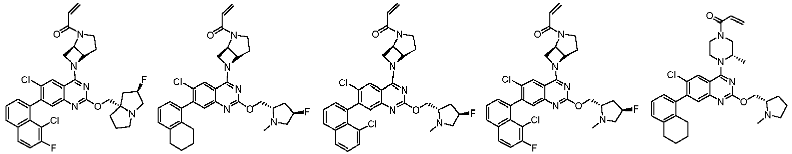

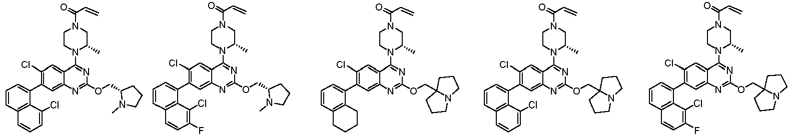

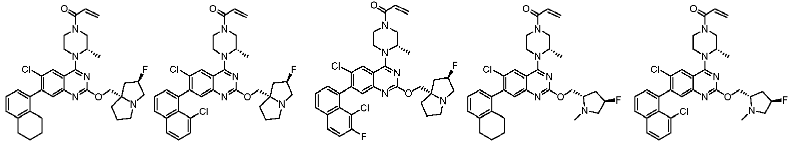

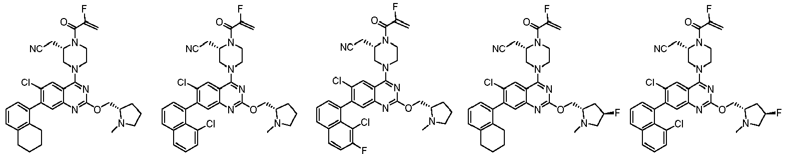

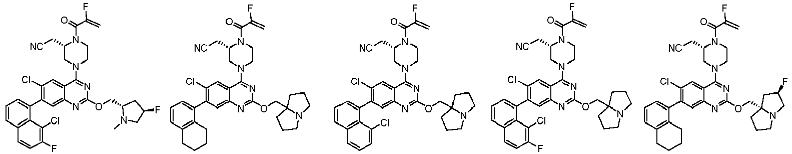

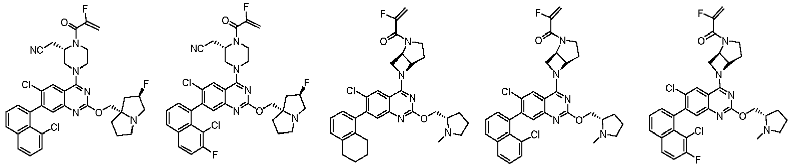

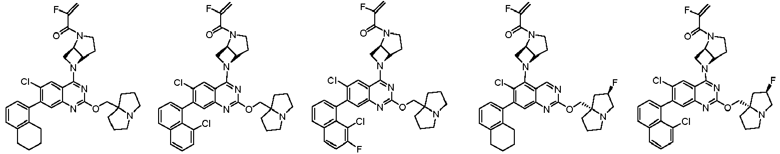

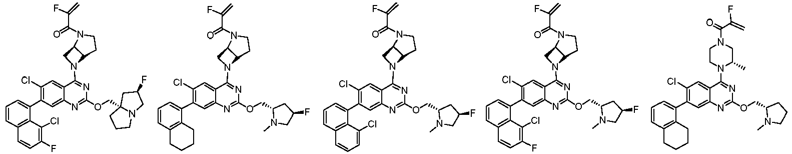

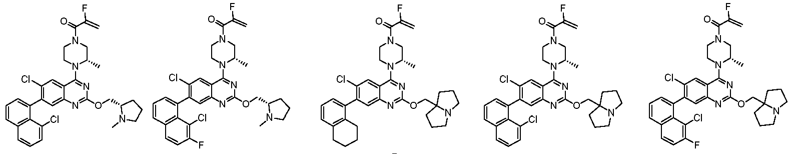

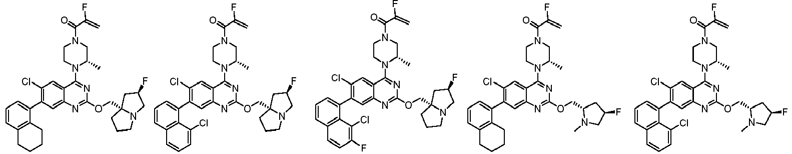

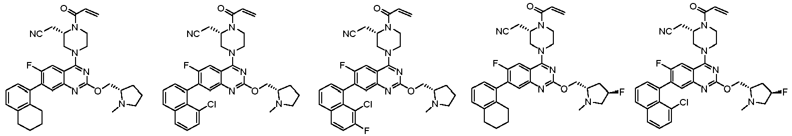

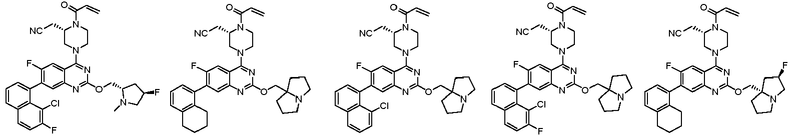

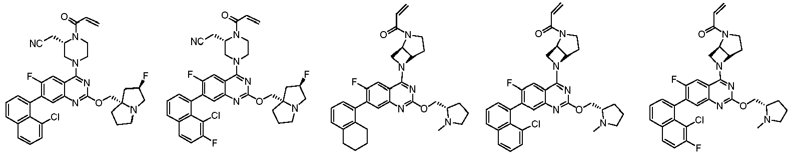

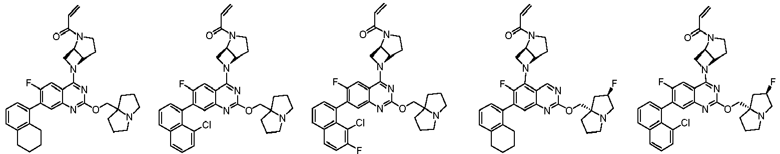

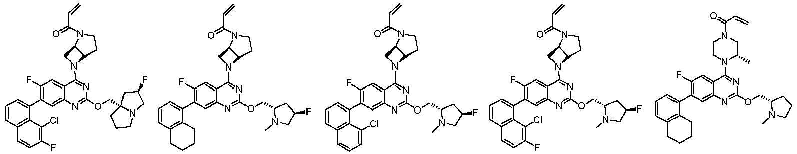

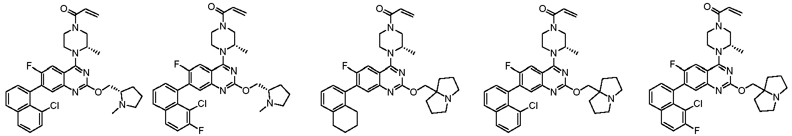

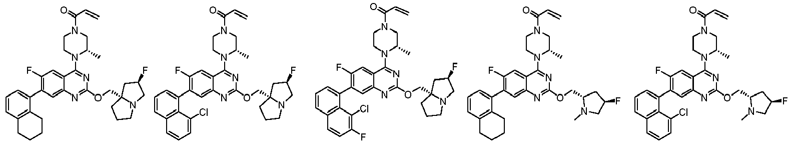

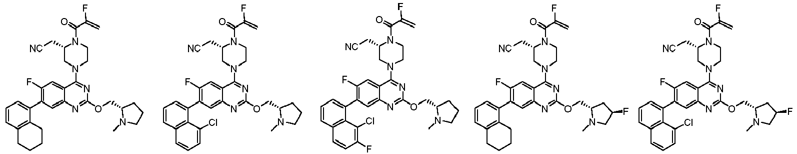

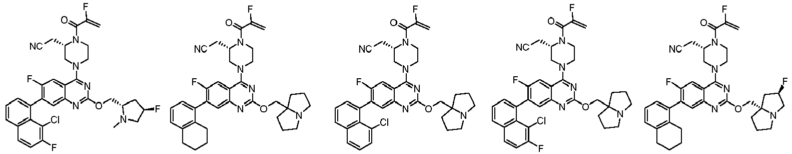

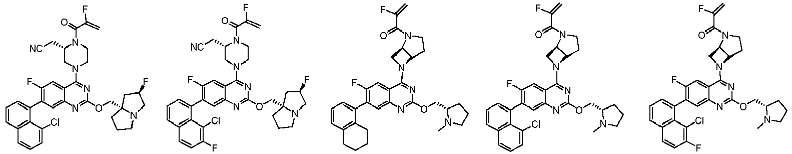

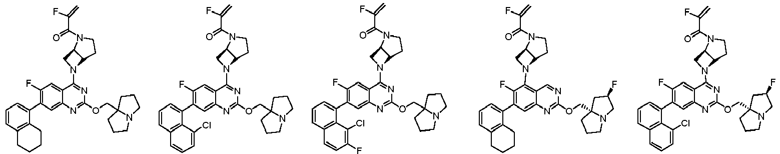

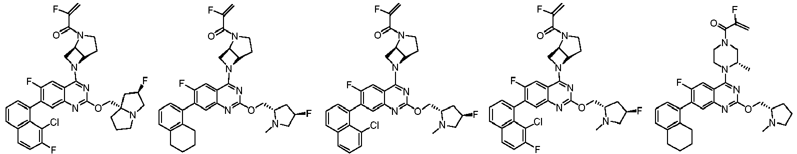

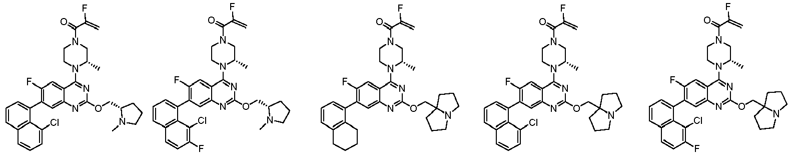

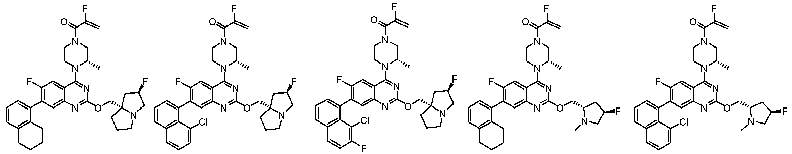

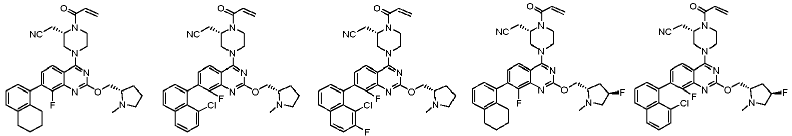

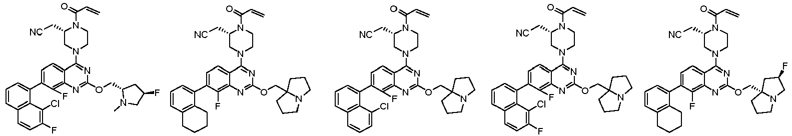

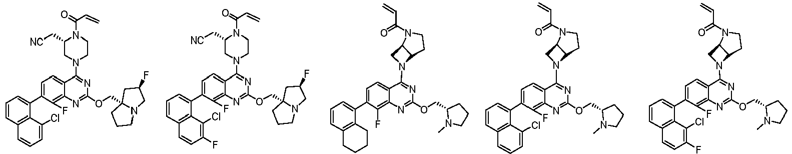

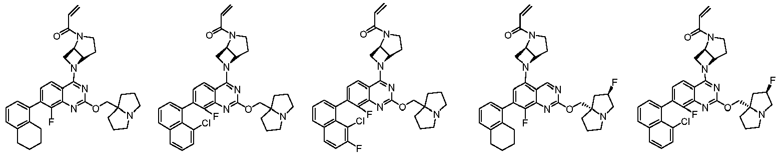

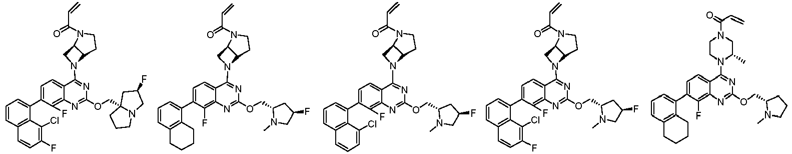

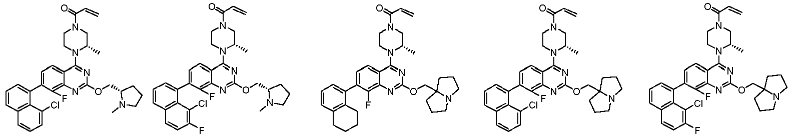

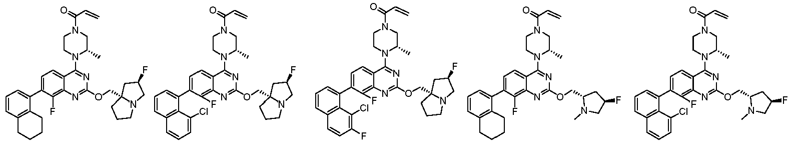

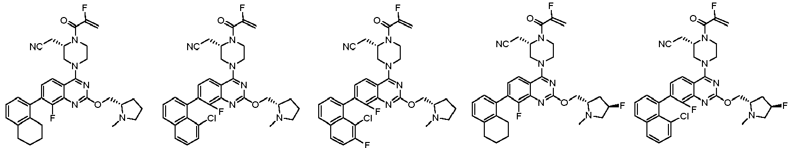

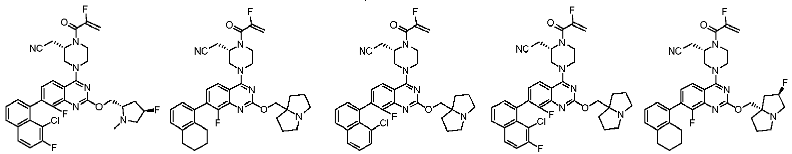

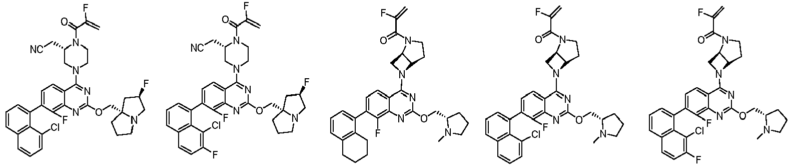

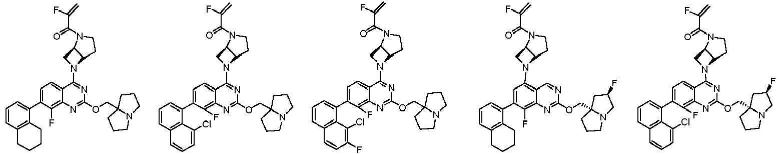

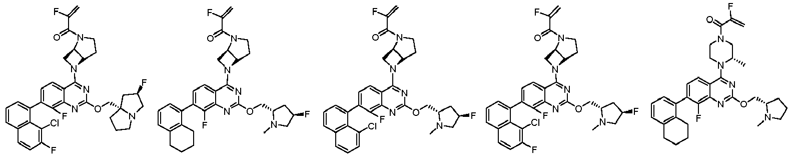

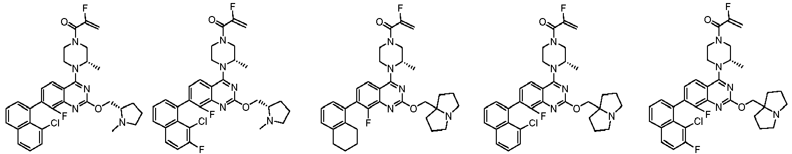

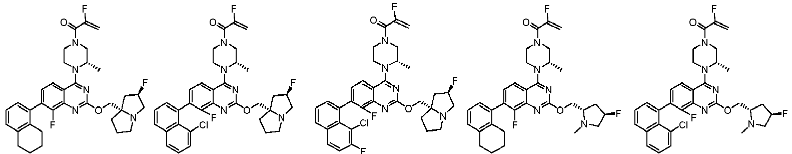

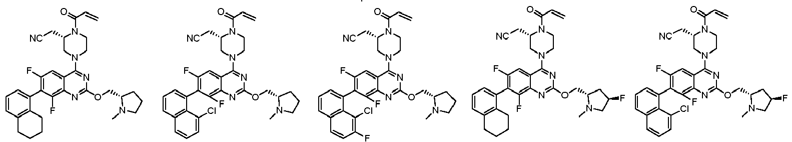

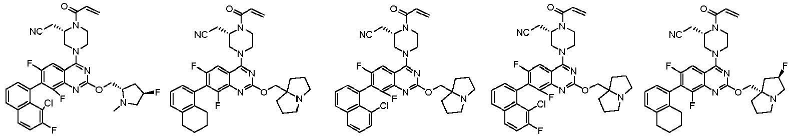

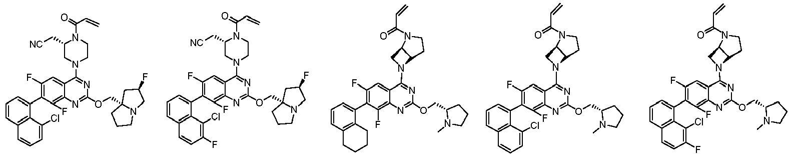

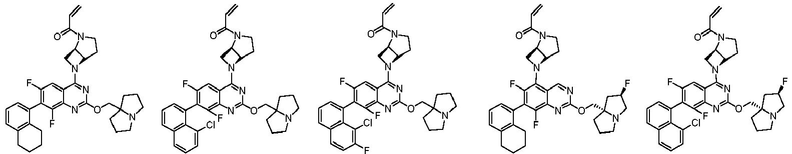

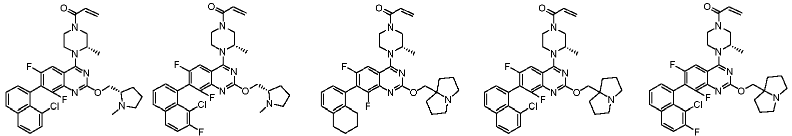

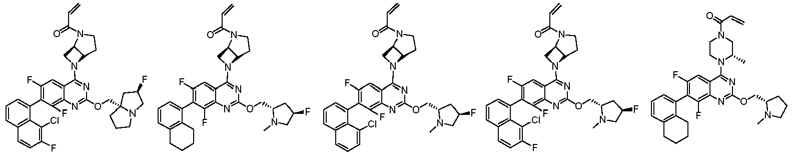

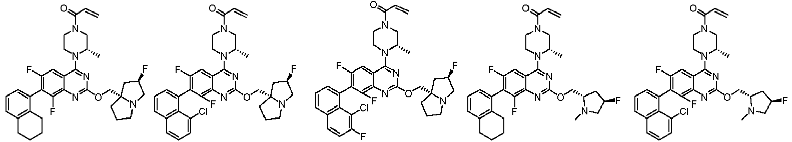

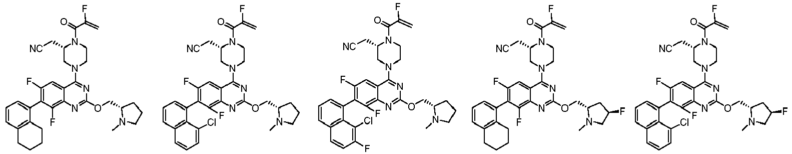

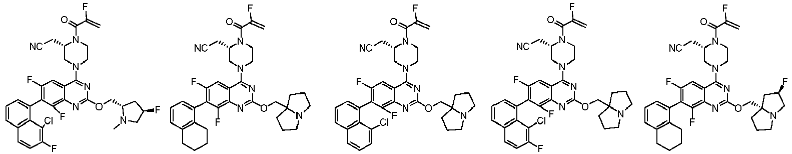

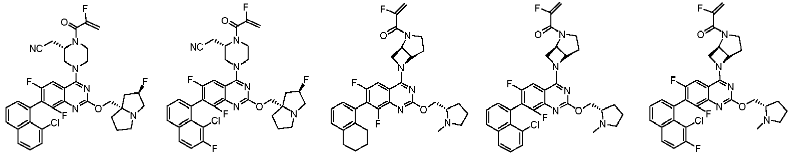

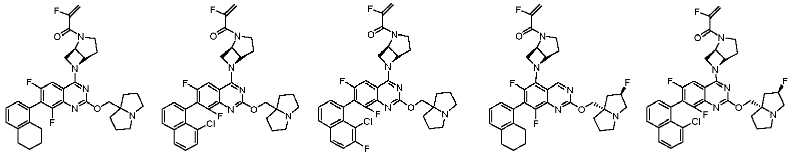

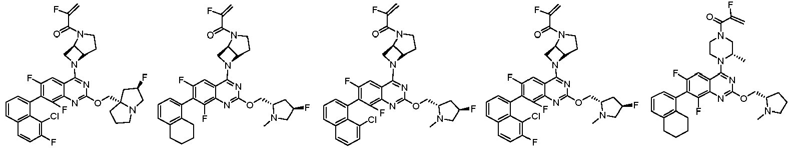

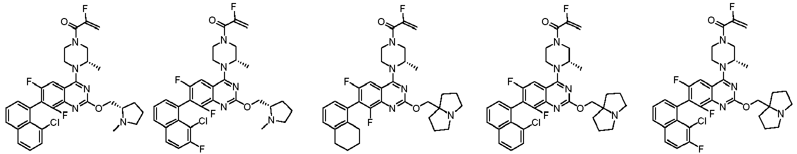

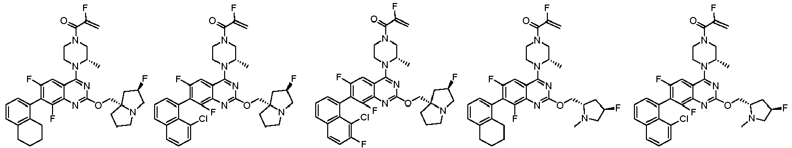

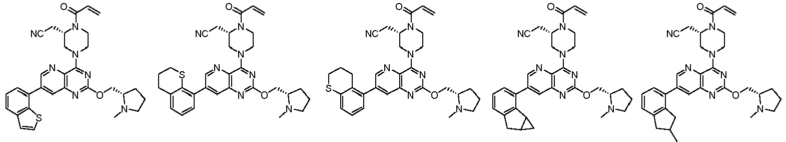

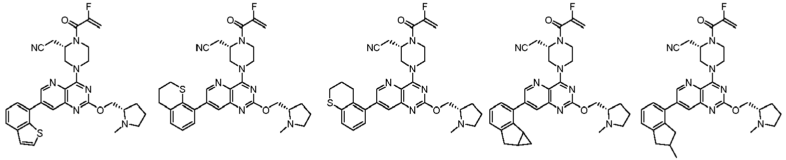

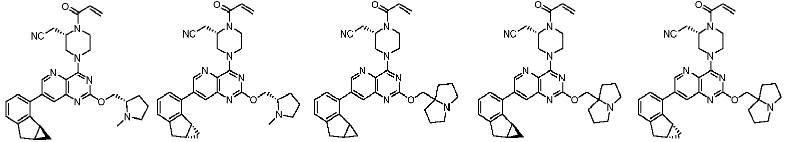

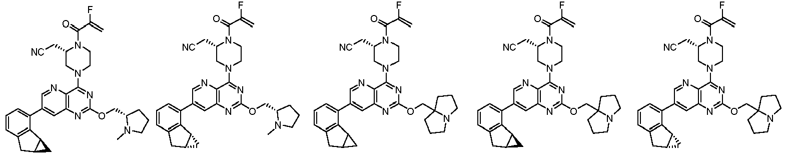

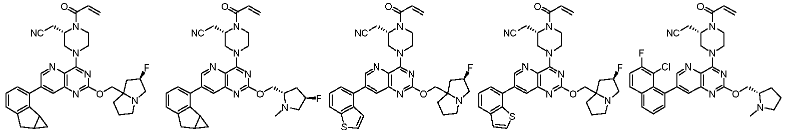

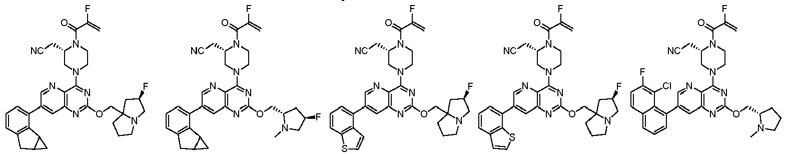

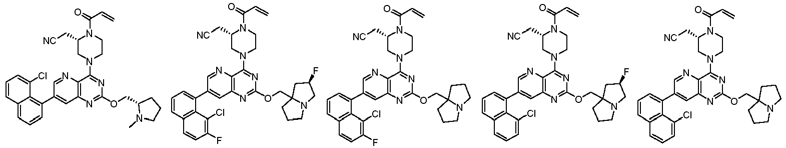

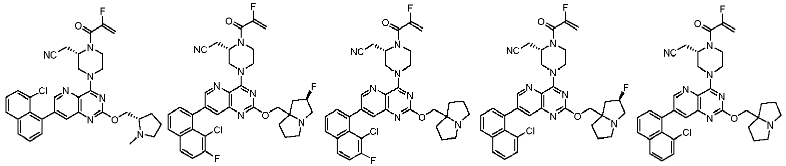

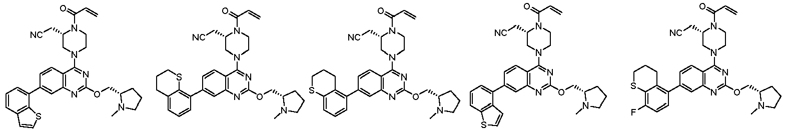

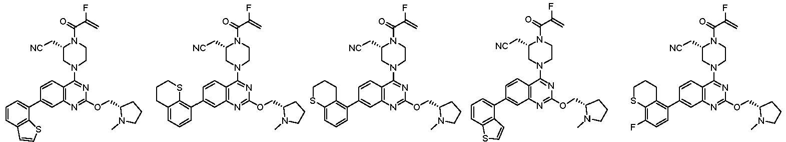

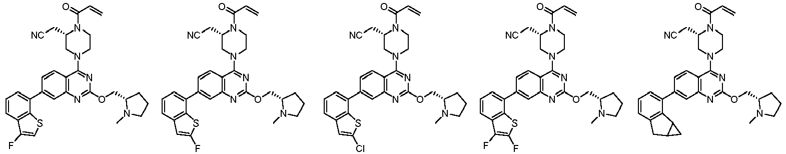

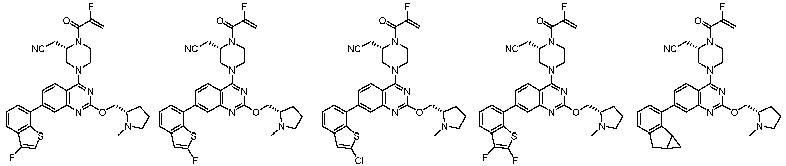

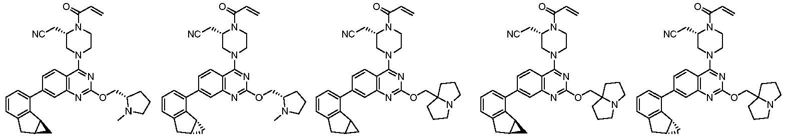

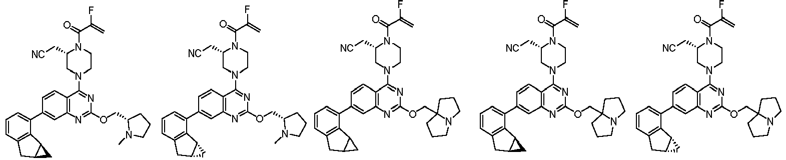

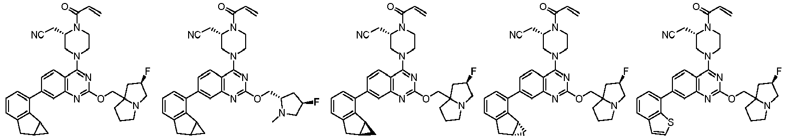

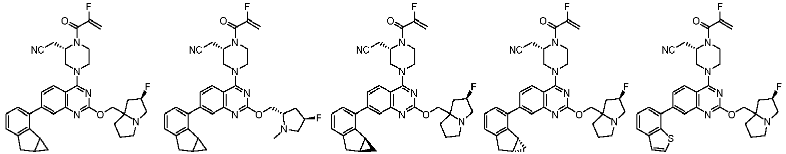

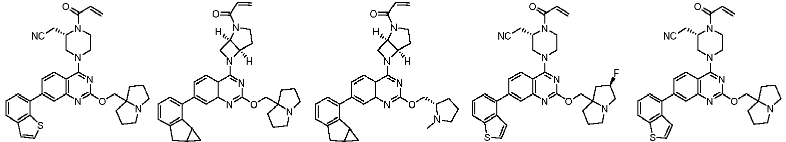

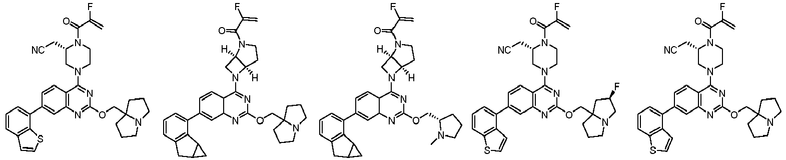

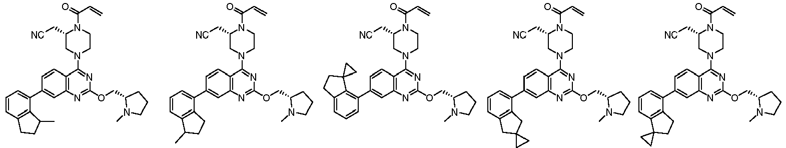

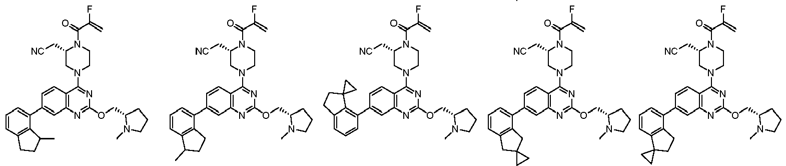

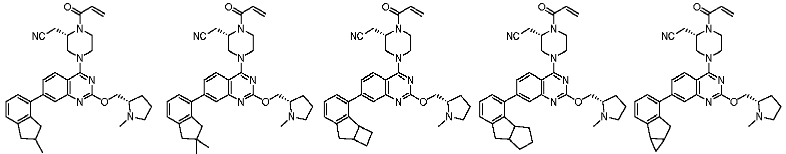

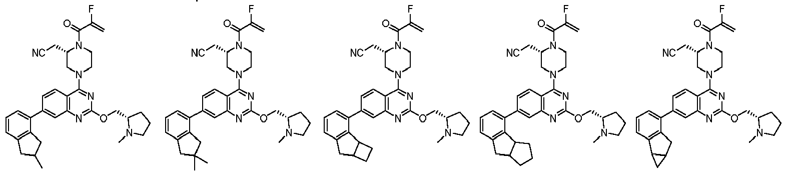

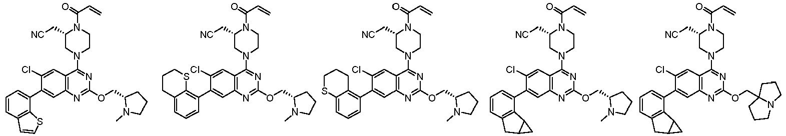

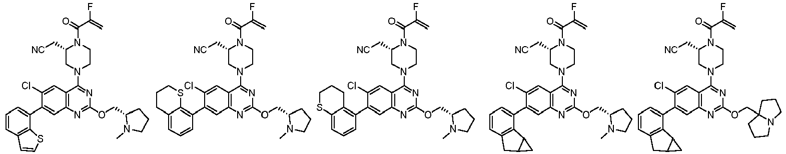

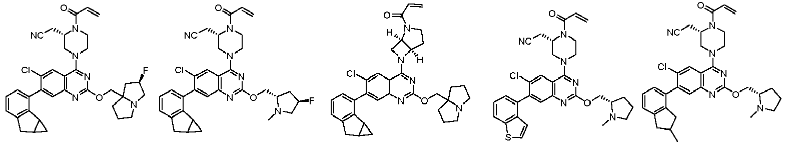

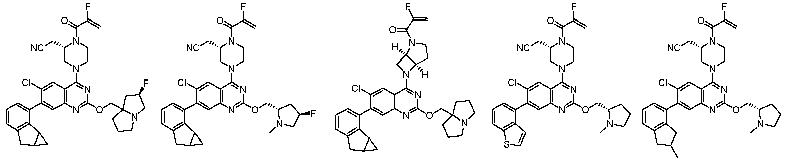

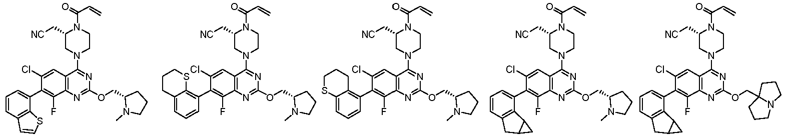

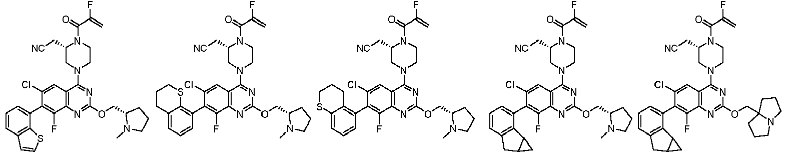

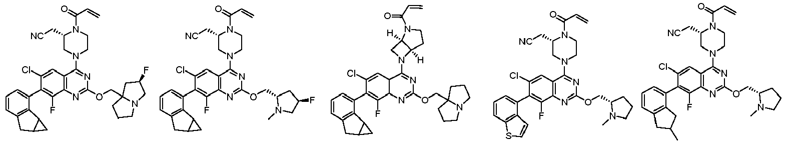

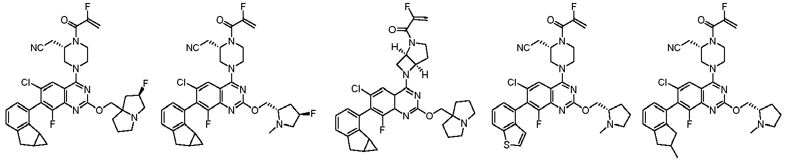

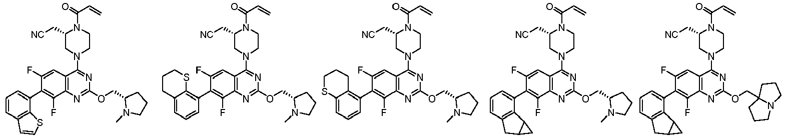

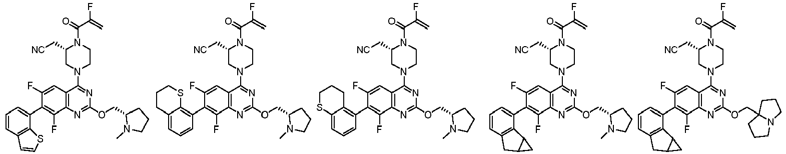

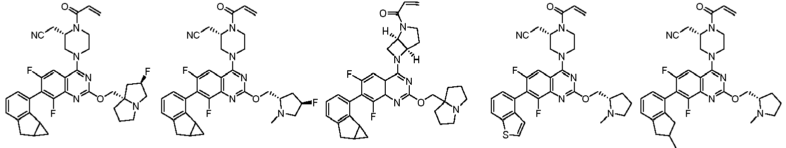

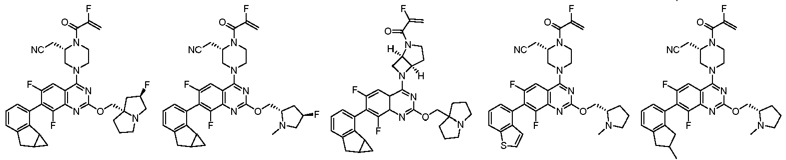

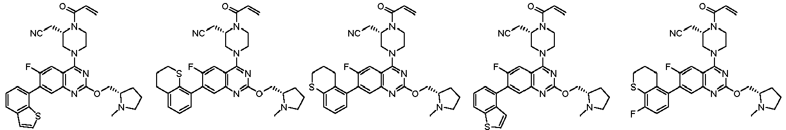

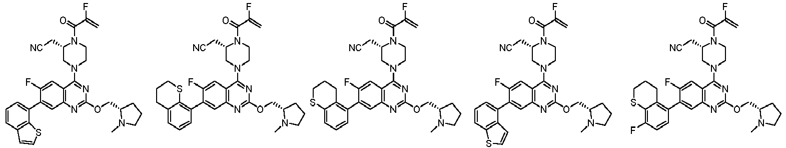

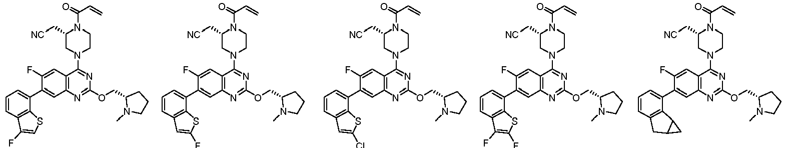

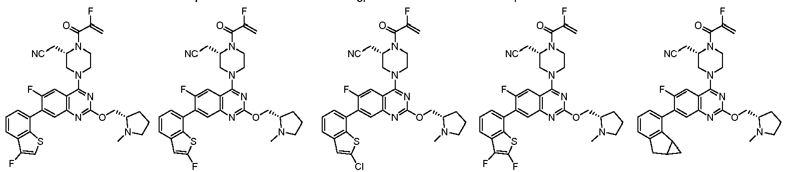

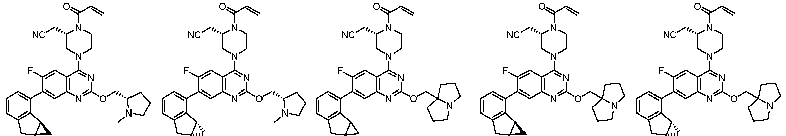

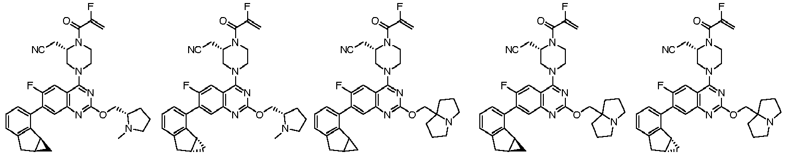

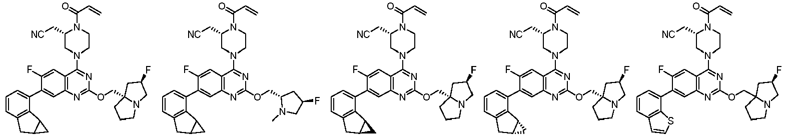

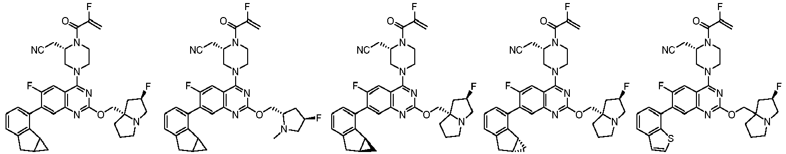

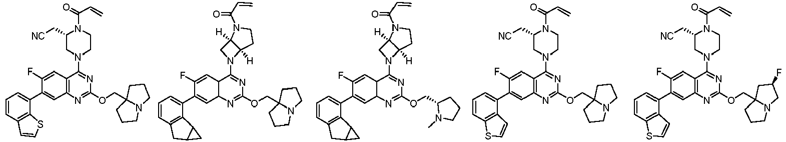

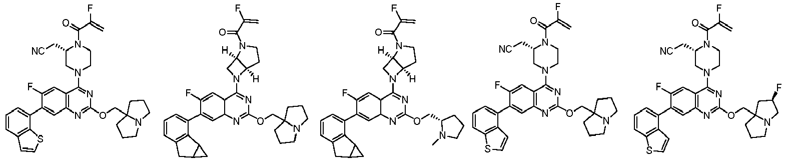

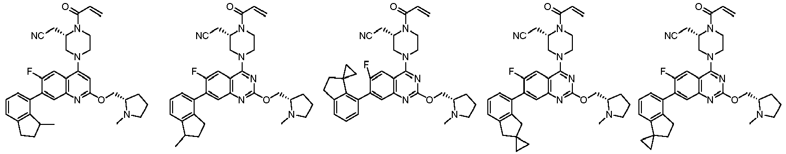

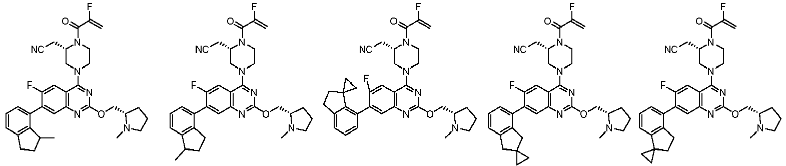

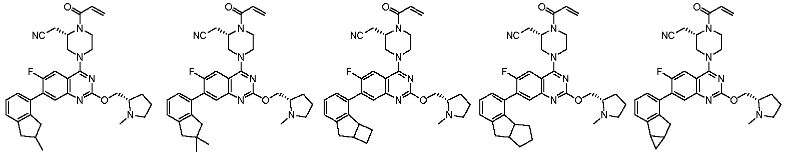

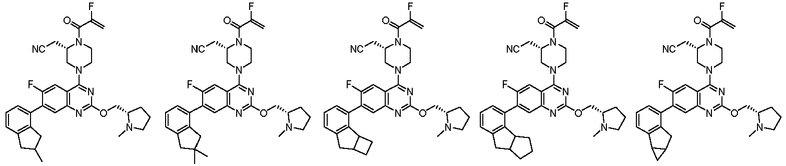

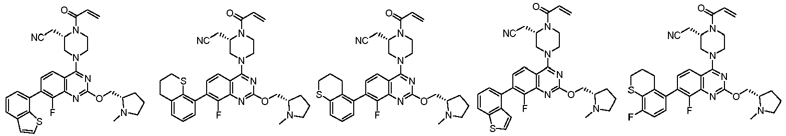

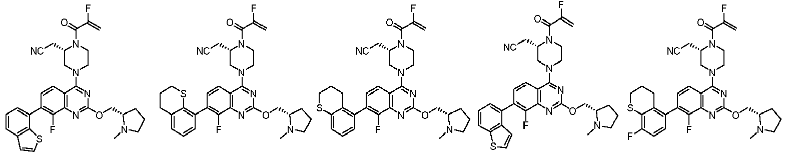

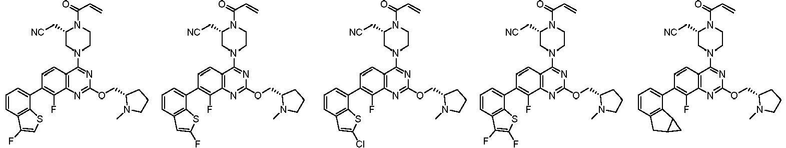

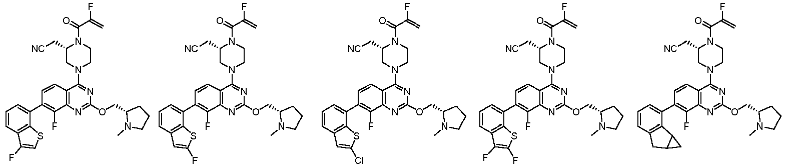

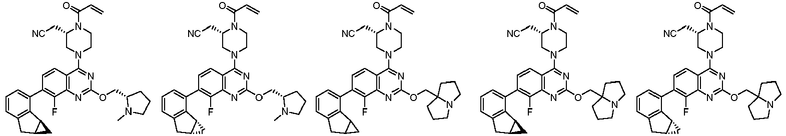

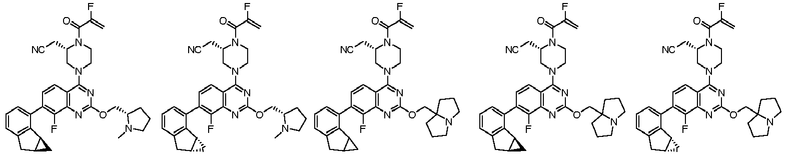

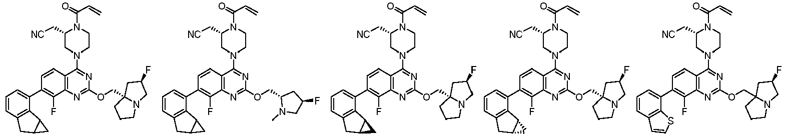

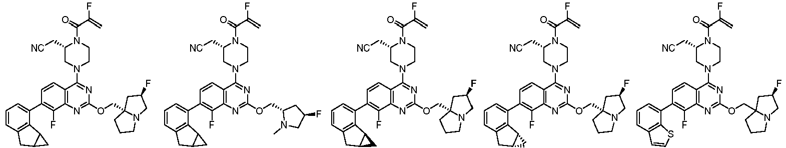

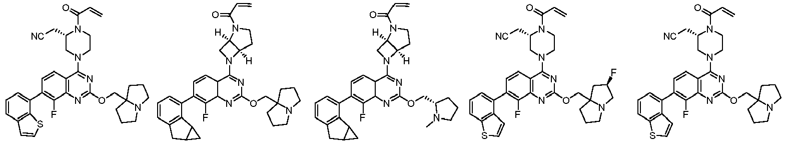

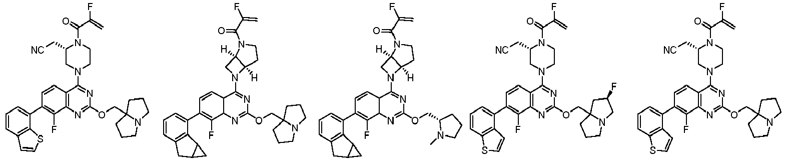

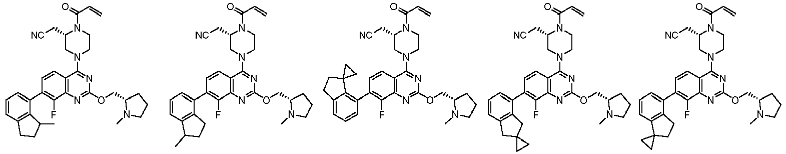

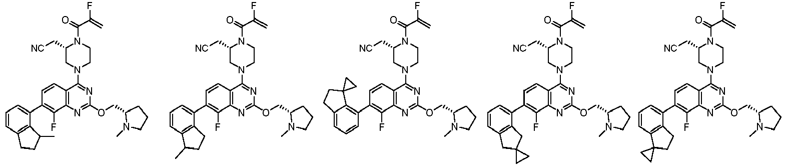

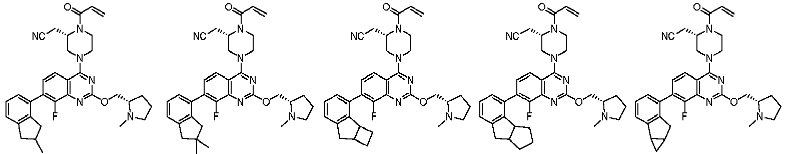

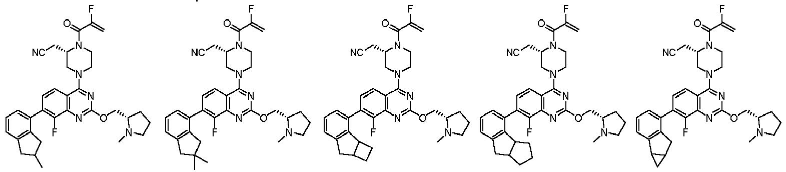

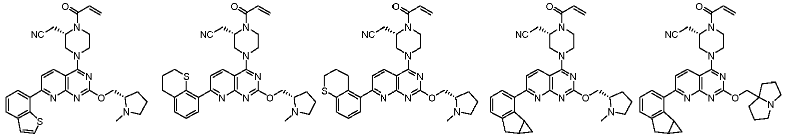

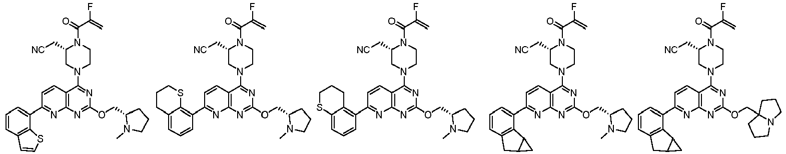

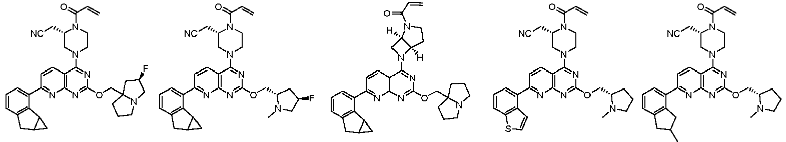

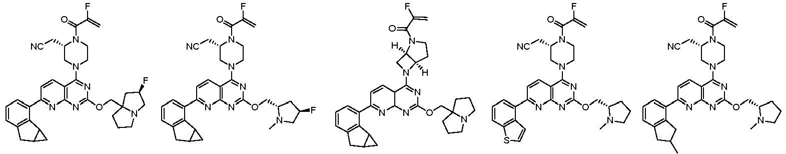

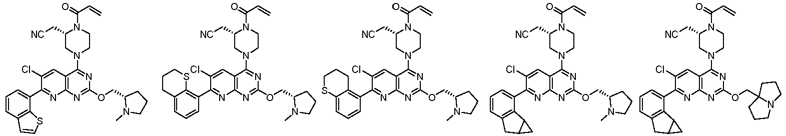

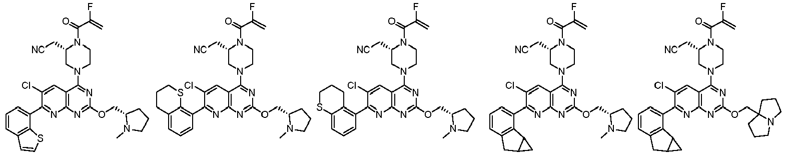

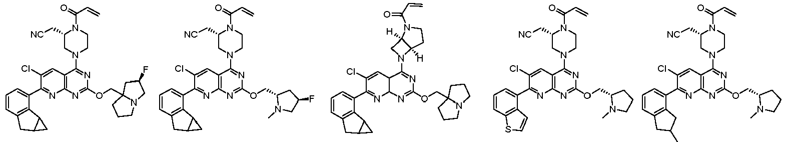

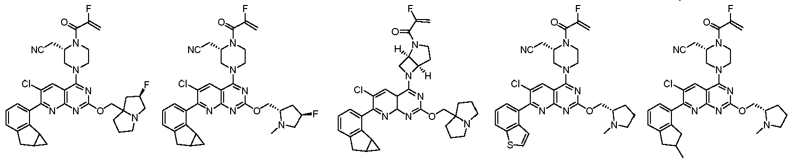

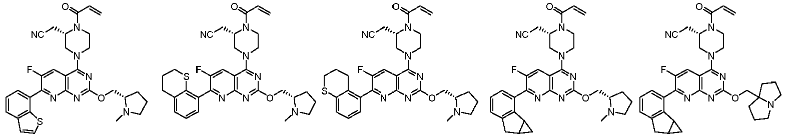

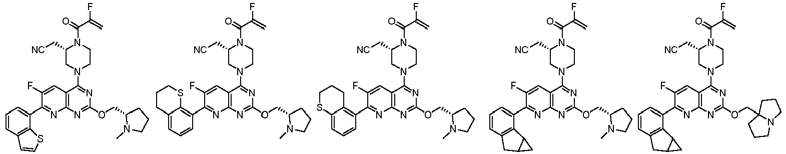

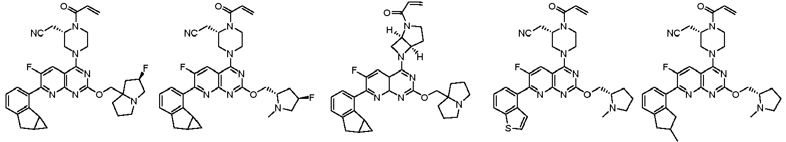

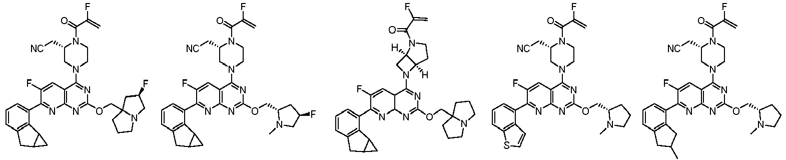

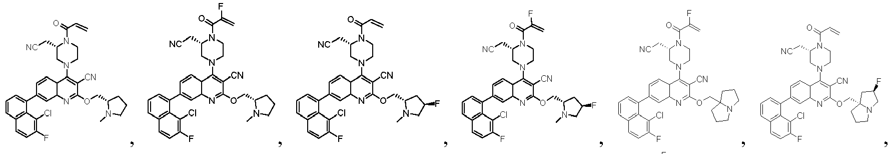

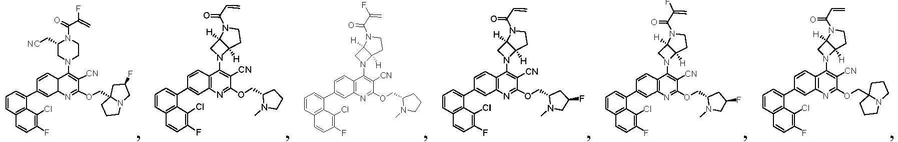

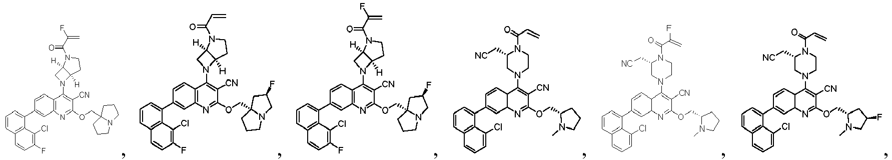

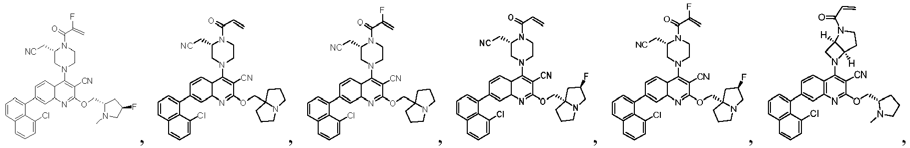

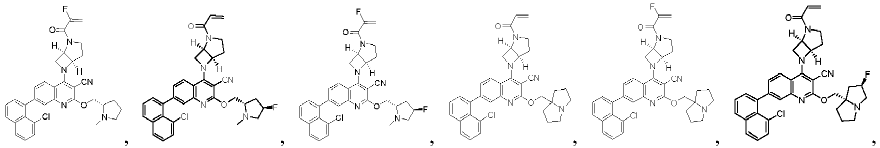

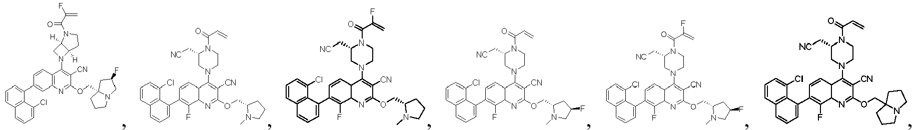

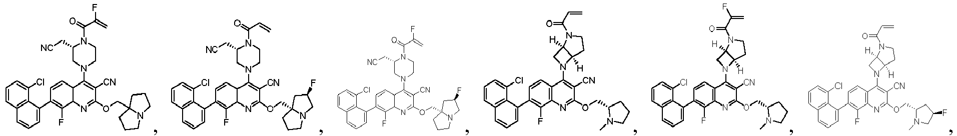

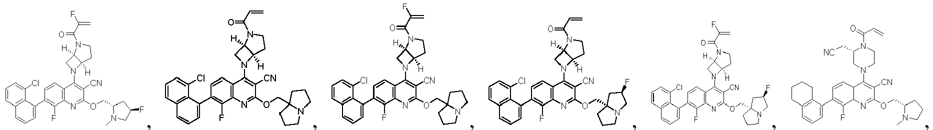

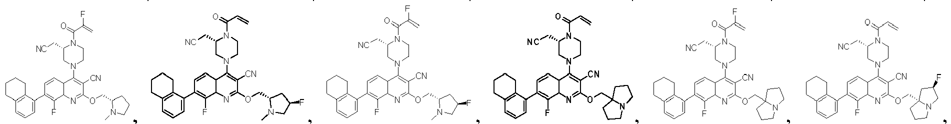

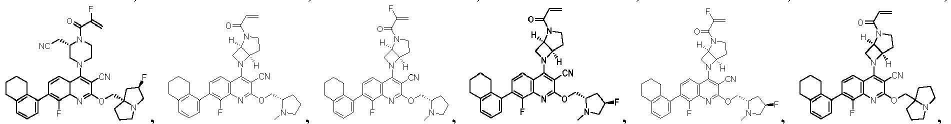

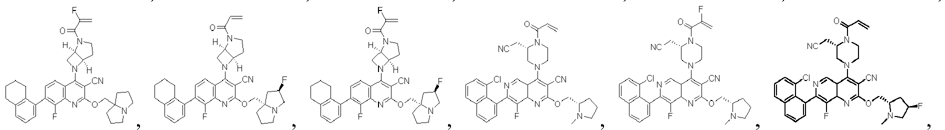

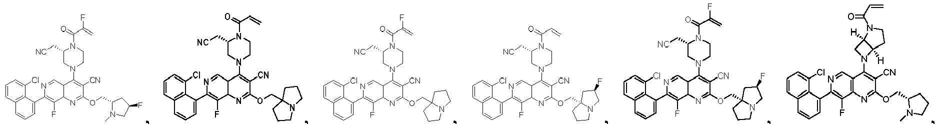

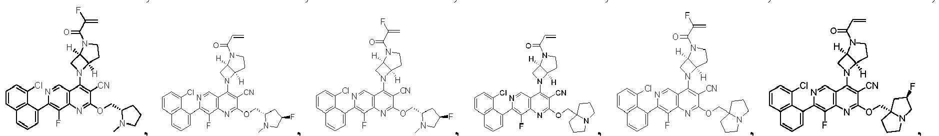

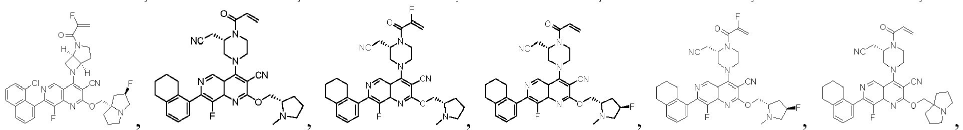

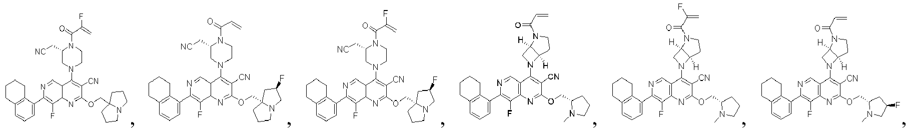

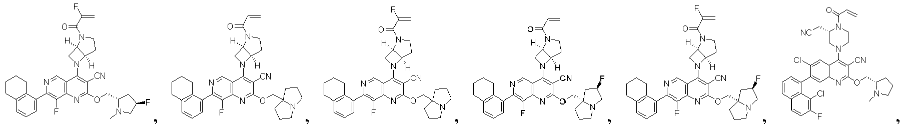

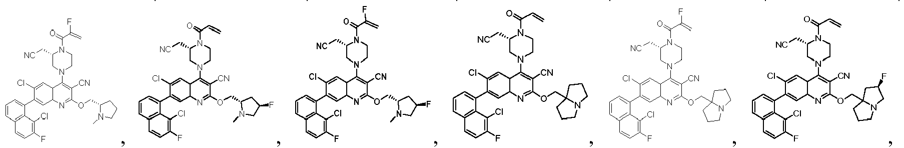

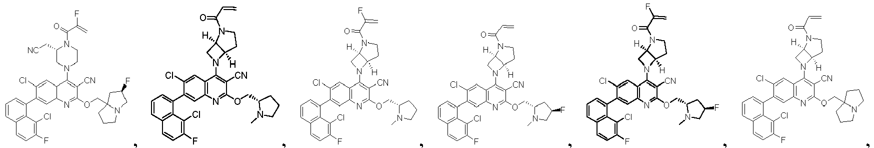

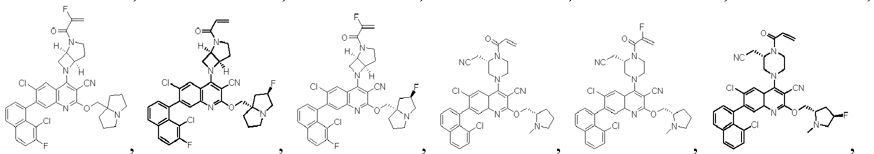

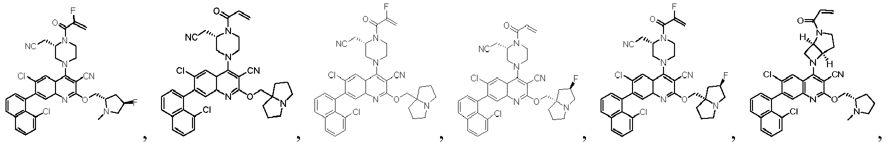

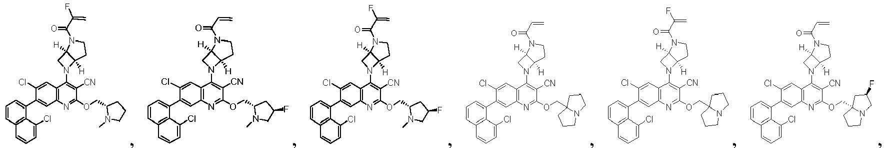

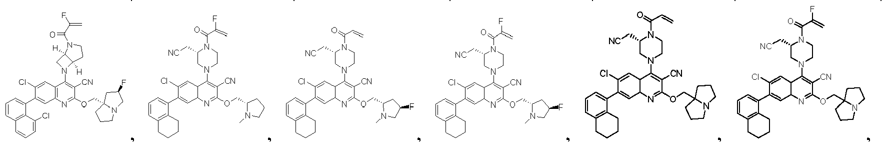

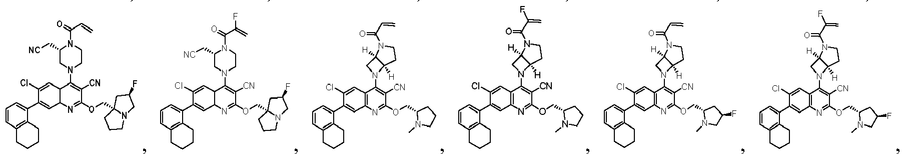

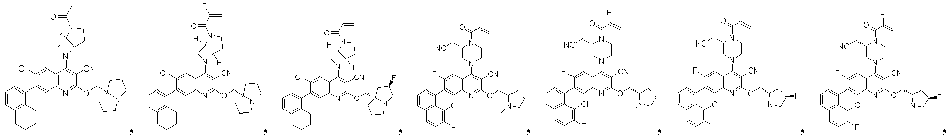

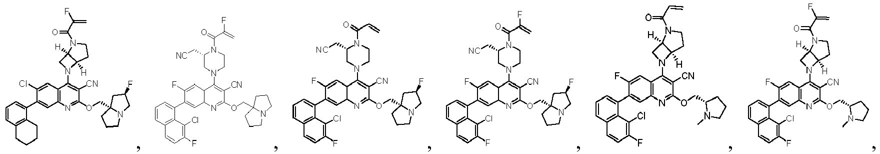

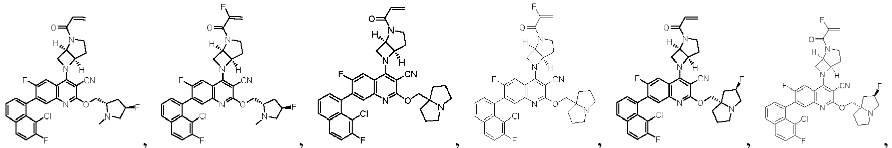

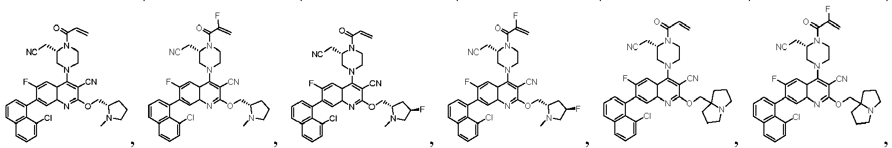

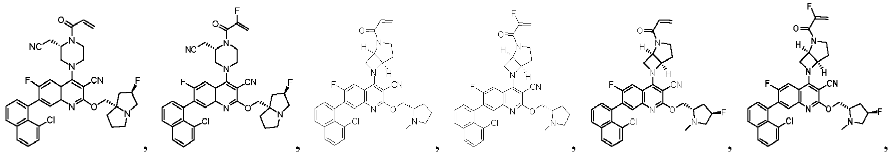

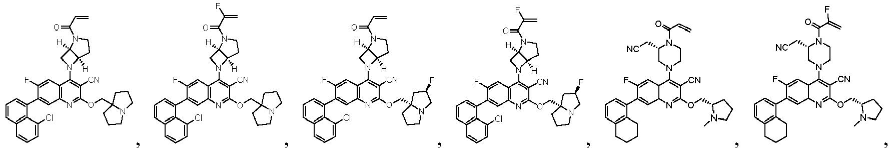

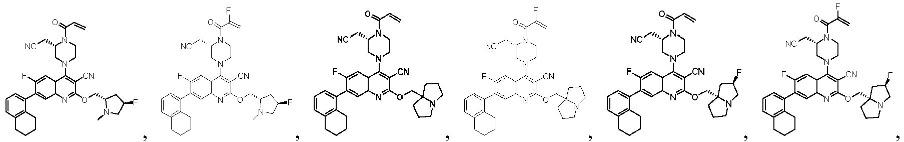

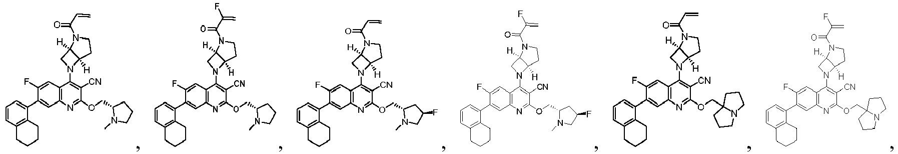

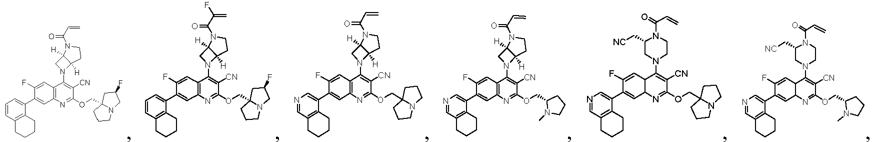

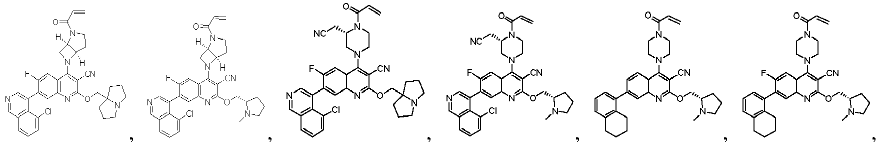

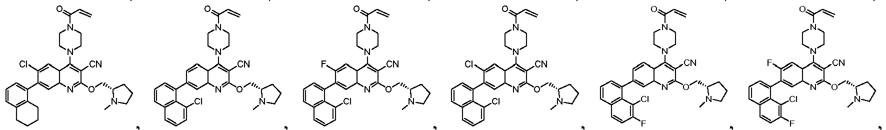

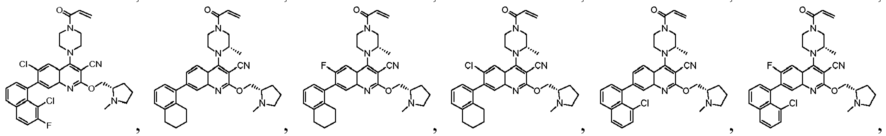

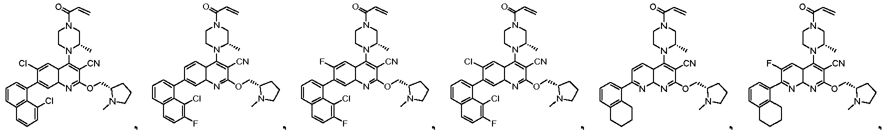

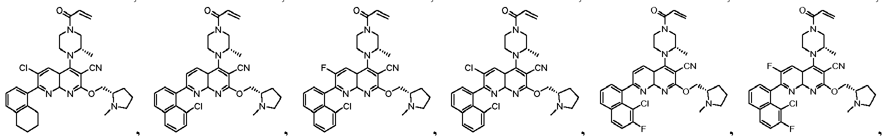

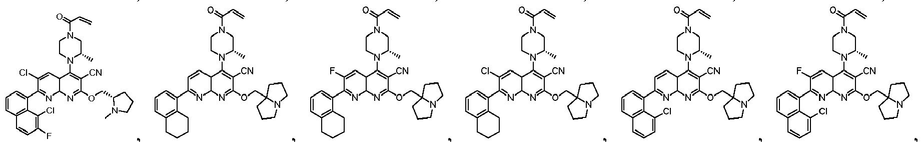

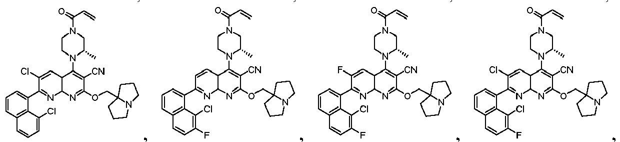

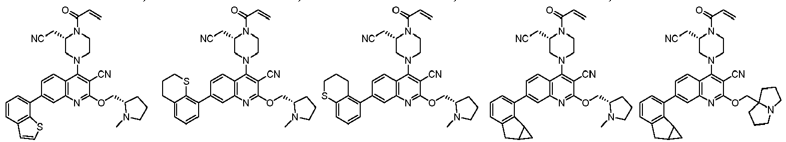

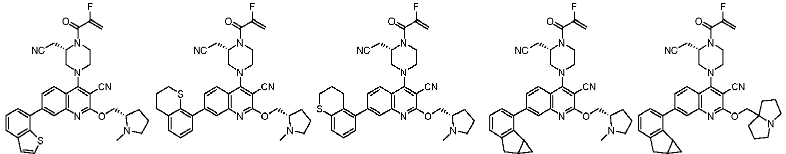

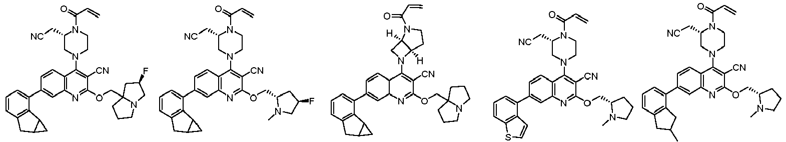

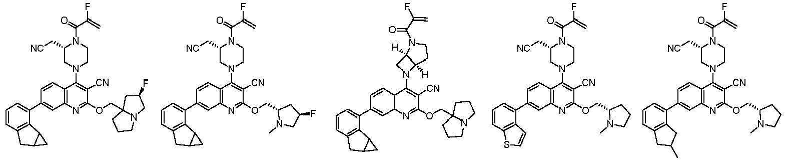

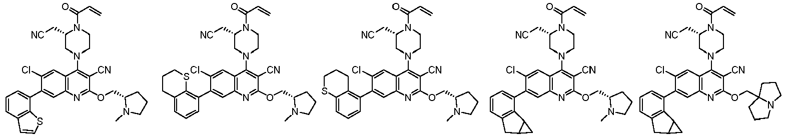

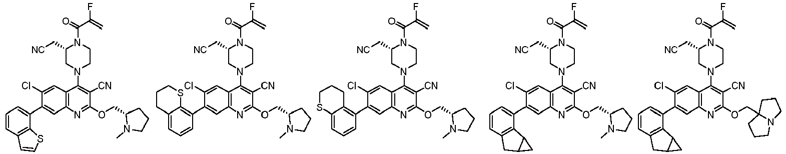

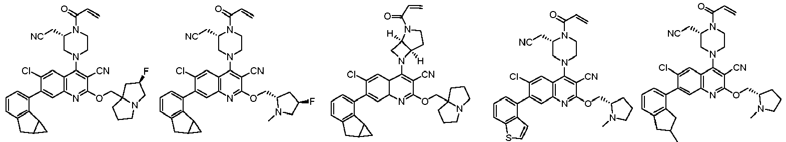

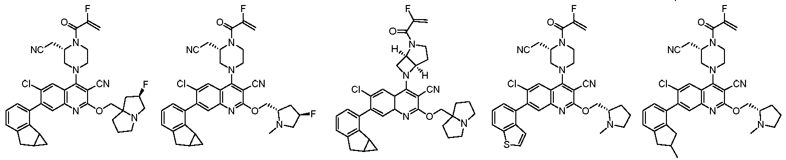

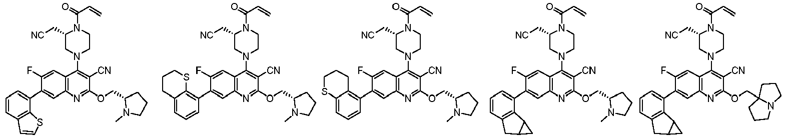

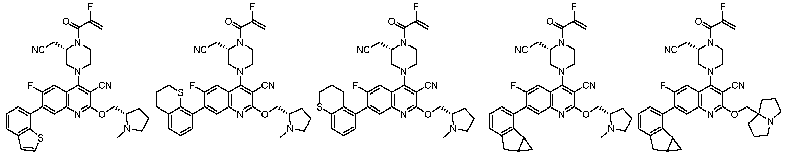

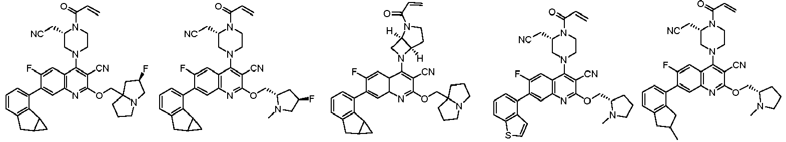

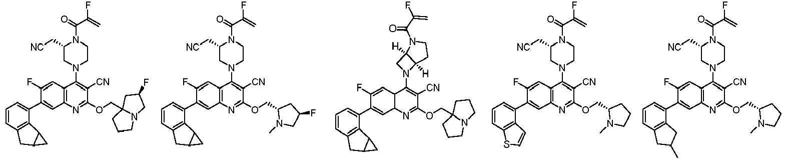

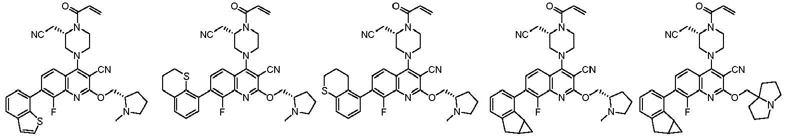

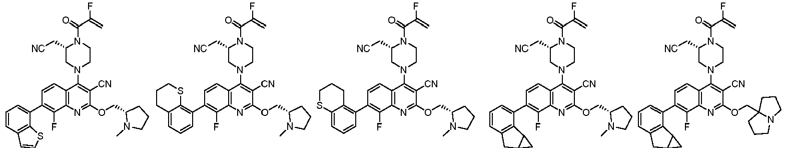

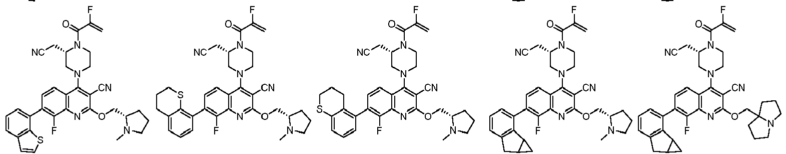

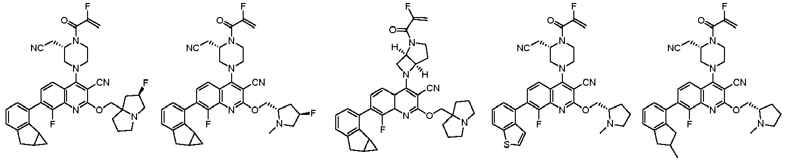

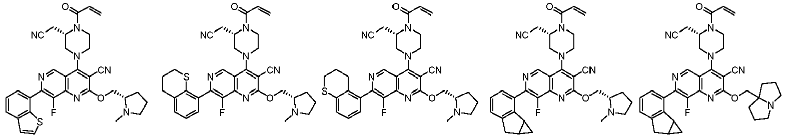

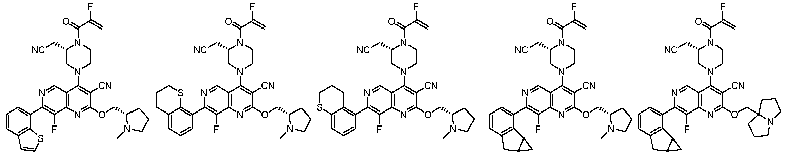

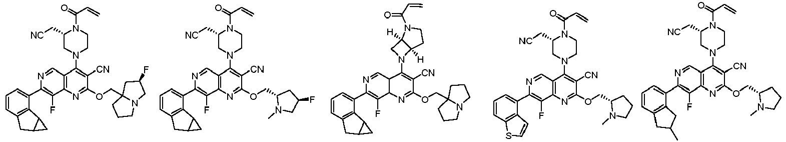

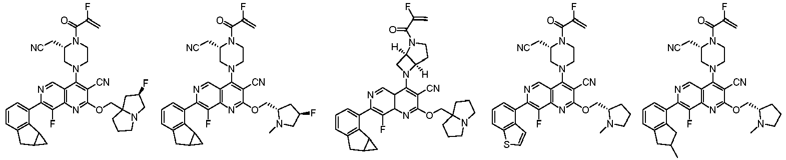

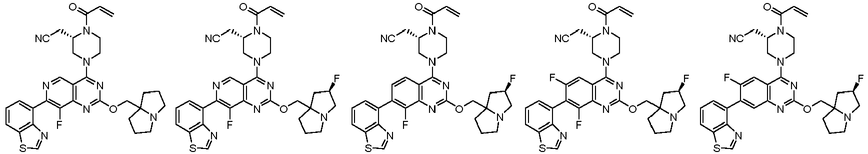

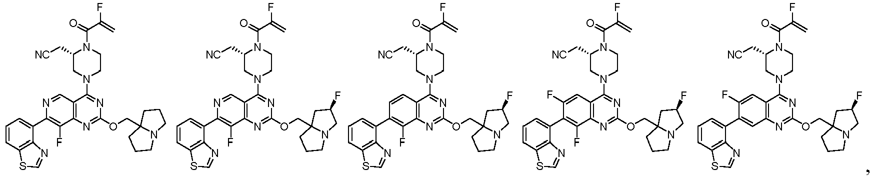

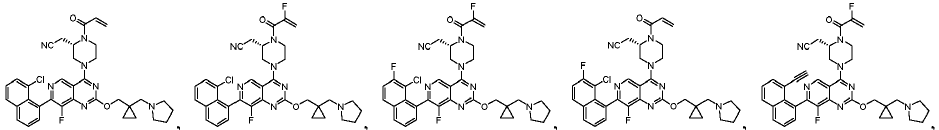

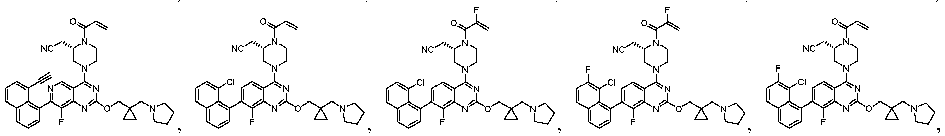

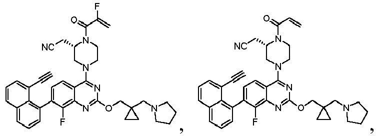

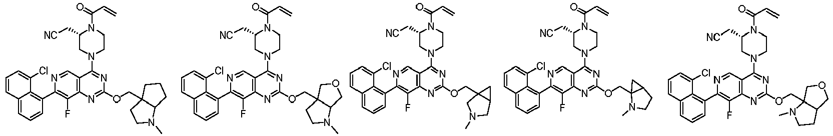

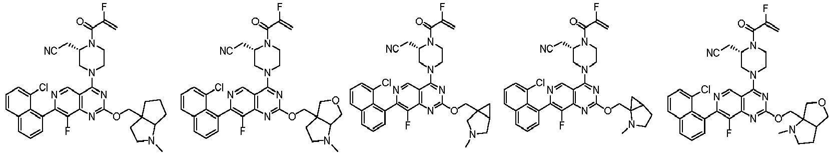

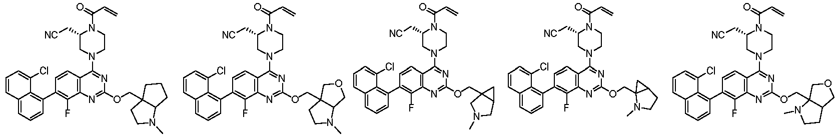

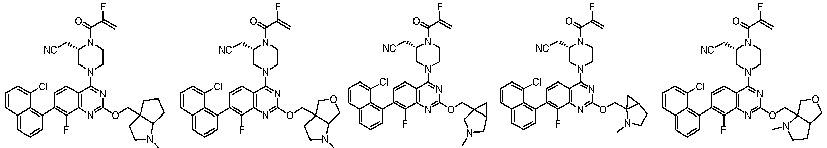

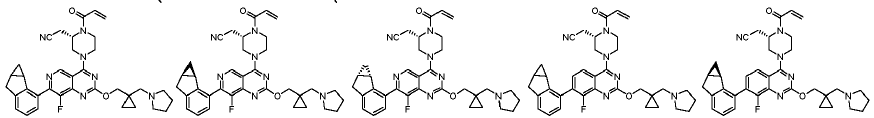

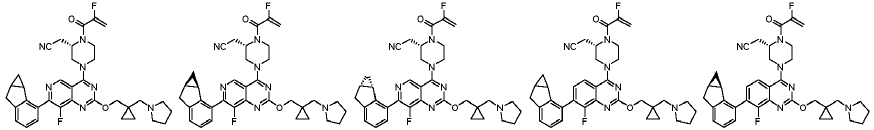

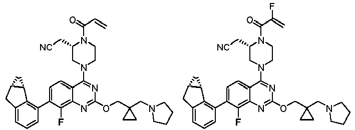

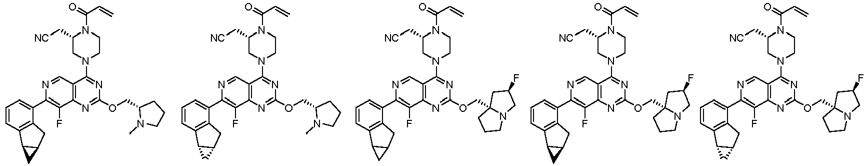

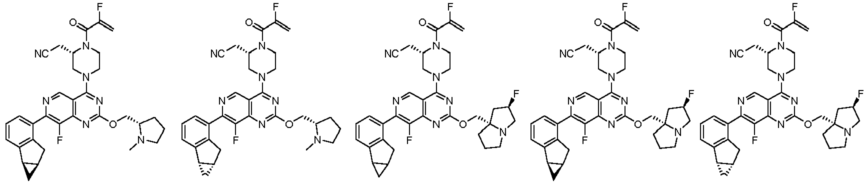

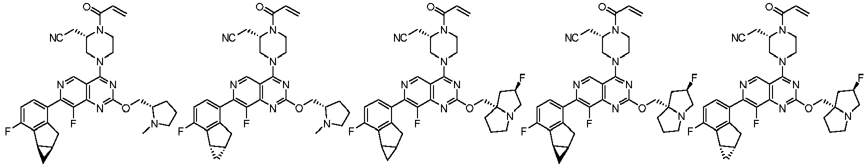

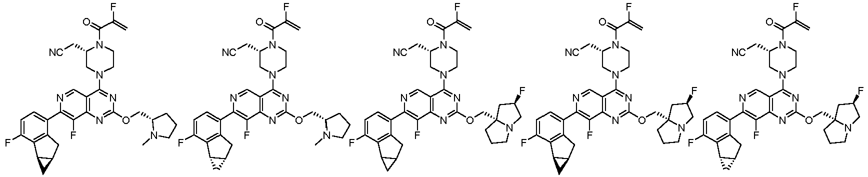

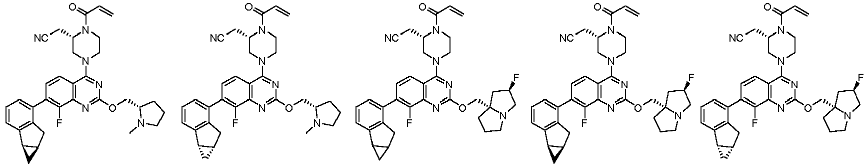

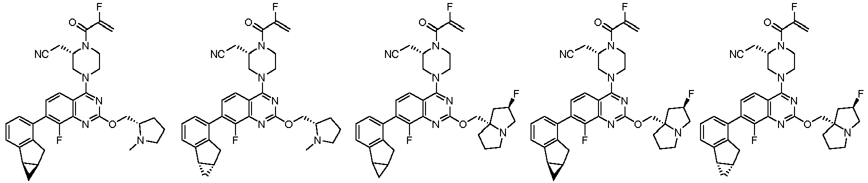

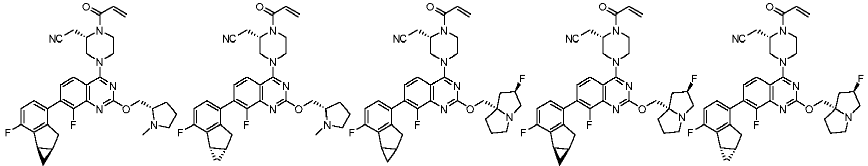

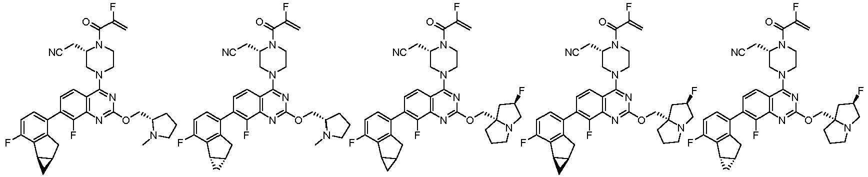

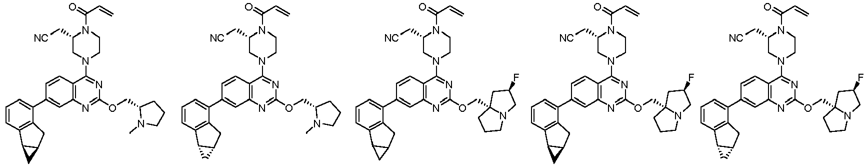

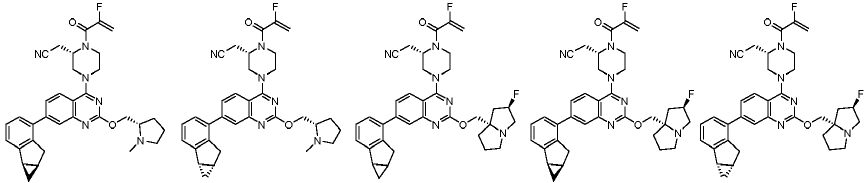

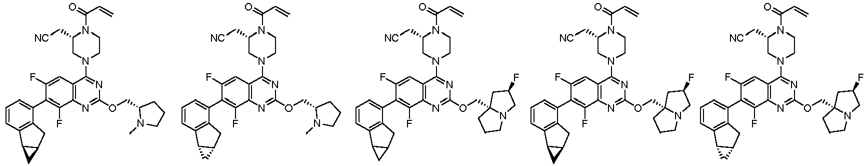

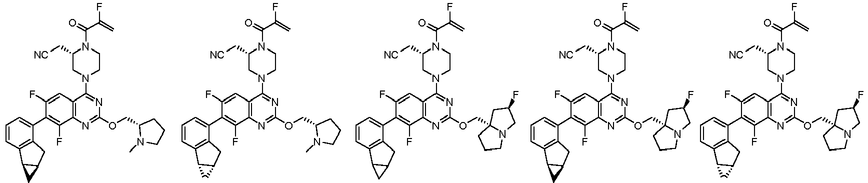

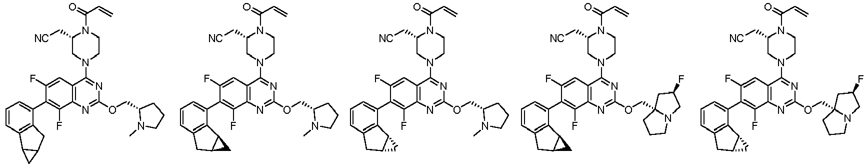

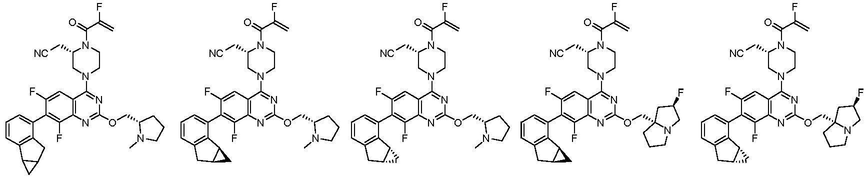

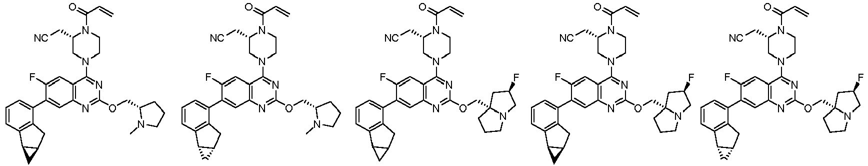

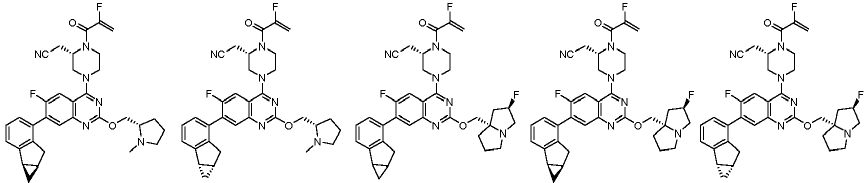

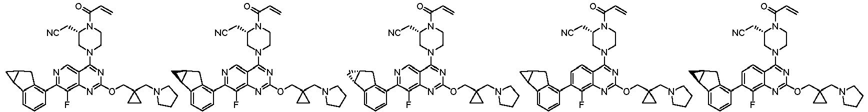

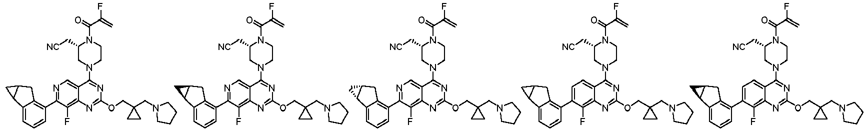

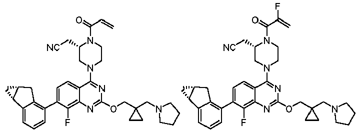

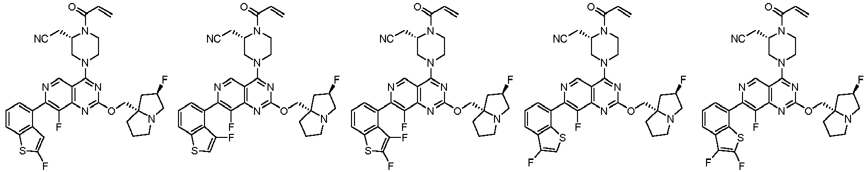

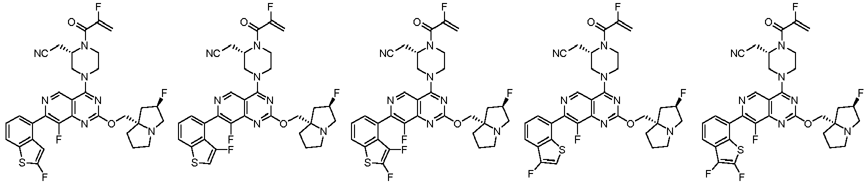

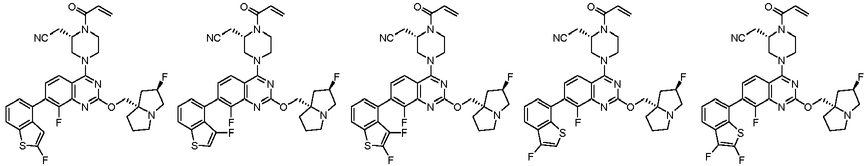

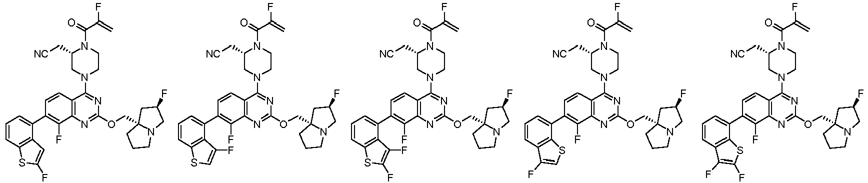

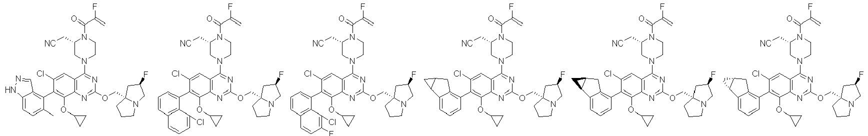

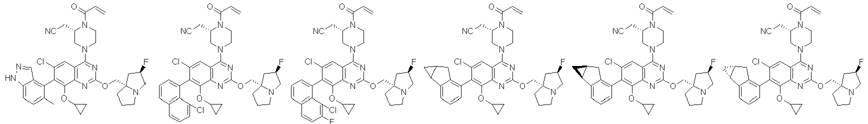

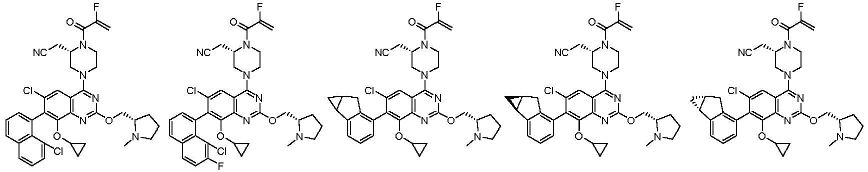

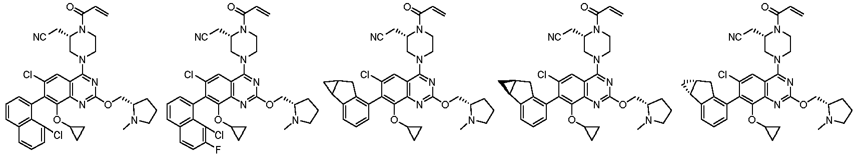

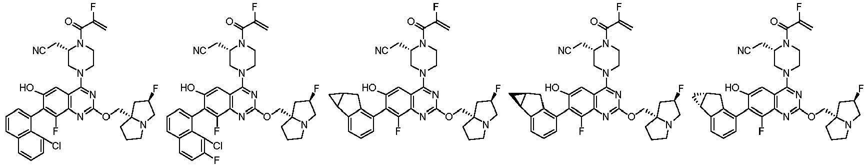

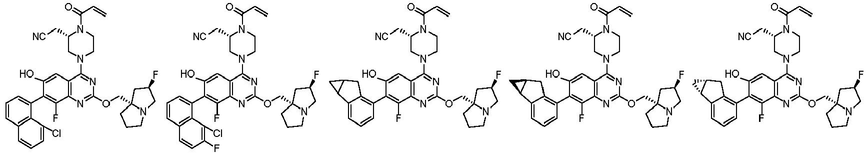

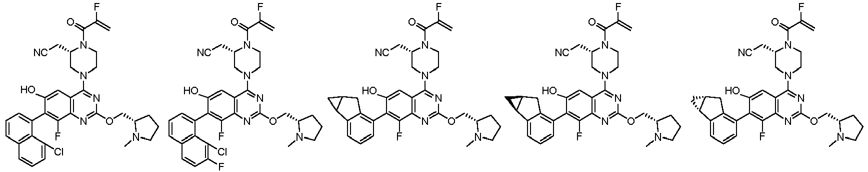

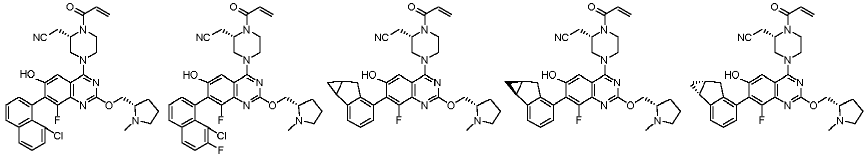

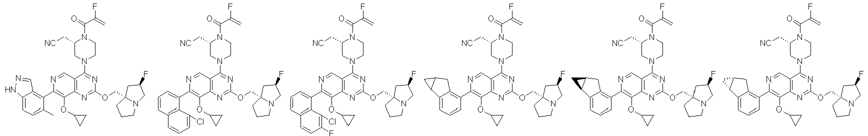

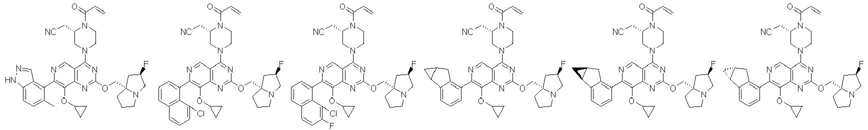

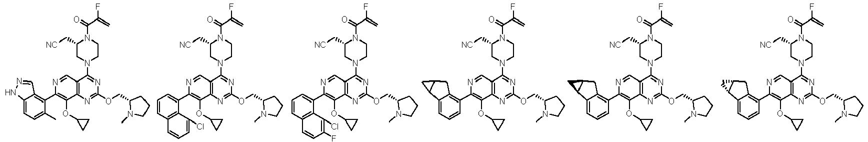

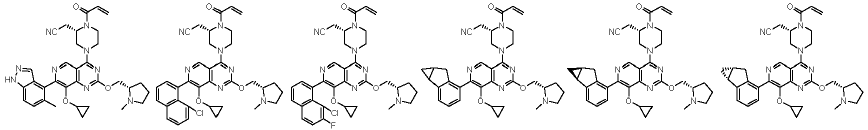

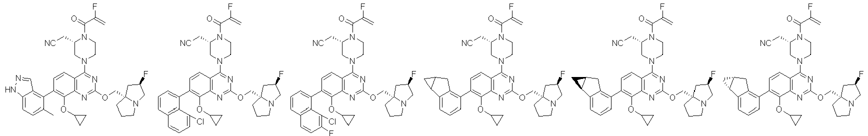

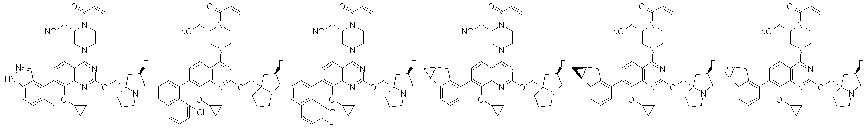

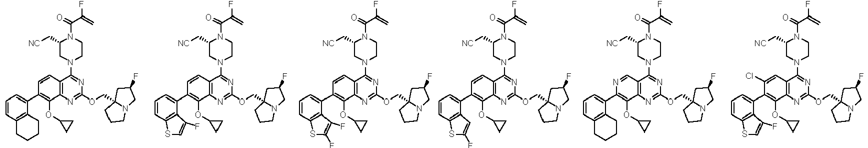

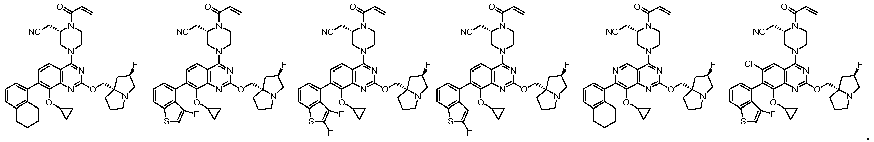

22. A pharmaceutical composition comprising the compound or the pharmaceutically acceptable salt, tautomer, prodrug or stereoisomer thereof according to any one of claims 1-21.

23. Use of the compound or the pharmaceutically acceptable salt, tautomer, prodrug or stereoisomer thereof according to any one of claims 1-21 and the pharmaceutical composition according to claim 21 in the preparation of a medicament for treating a cancer mediated by KRAS G12C, HRAS G12C, or NRAS G12 mutation.
